(19) Europäisches Patentamt
European Patent Office
Office européen des brevets

(11) EP 4 729 521 A1

(12) **EUROPEAN PATENT APPLICATION**
published in accordance with Art. 153(4) EPC

(43) Date of publication:
**22.04.2026 Bulletin 2026/17**

(21) Application number: **24822690.4**

(22) Date of filing: **11.06.2024**

(51) International Patent Classification (IPC):
***C07D 491/22*** *(2006.01)* ***A61K 47/68*** *(2017.01)*
***A61K 31/541*** *(2006.01)* ***A61P 35/00*** *(2006.01)*

(52) Cooperative Patent Classification (CPC):
**A61K 31/541; A61K 47/68; A61P 35/00; C07D 491/22**

(86) International application number:
**PCT/CN2024/098491**

(87) International publication number:
**WO 2024/255740 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.06.2023 CN 202310696385**
**21.12.2023 CN 202311768933**

(71) Applicant: **Chengdu Brilliant Pharmaceutical Co., Ltd.**
**Chengdu, Sichuan 610094 (CN)**

(72) Inventors:
- **CHEN, Tonghui**
  **Chengdu, Sichuan 610094 (CN)**
- **XU, Jun**
  **Chengdu, Sichuan 610094 (CN)**
- **WANG, Tong**
  **Chengdu, Sichuan 610094 (CN)**
- **FANG, Xuerong**
  **Chengdu, Sichuan 610094 (CN)**
- **LIAO, Jianyu**
  **Chengdu, Sichuan 610094 (CN)**
- **HUANG, Haoxi**
  **Chengdu, Sichuan 610094 (CN)**

(74) Representative: **Vossius & Partner Patentanwälte Rechtsanwälte mbB**
**Siebertstraße 3**
**81675 München (DE)**

# (54) CAMPTOTHECIN DERIVATIVE FOR TREATING OR PREVENTING CANCER, AND ANTIBODY-DRUG CONJUGATE THEREOF

(57) The present invention relates to camptothecin derivatives, their linker-payload conjugates, and antibody-drug conjugates, as well as pharmaceutically acceptable salts or esters, solvates, tautomers, stereoisomers, prodrugs, or isotopically labeled forms thereof, for use in the treatment or prevention of cancer. The invention further relates to a pharmaceutical composition comprising the camptothecin derivatives, their linker-payload conjugates, or antibody-drug conjugates; methods for the preparation of the same; and the use thereof.

EP 4 729 521 A1

## Description

## Technical Field

The present invention relates, in general, to camptothecin derivatives for the treatment or prevention of cancer as well as their linker-payload conjugates and antibody-drug conjugates, or pharmaceutically acceptable salts or esters, solvates, tautomers, stereoisomers, prodrugs, or isotopically labeled forms thereof. The present invention further relates to a pharmaceutical composition comprising said camptothecin derivatives or their linker-payload conjugates or antibody-drug conjugates, their preparation methods as well as the uses thereof.

## Background of the Invention

Antibody-drug conjugates (ADCs) are a highly promising class of targeted therapeutics, primarily composed of three parts: an antibody (Ab) responsible for selectively recognizing a tumor cell surface antigen, a small-molecule cytotoxic drug (payload) responsible for killing tumor cells, and a linker that attaches the payload to the antibody, wherein the antibody serves as a carrier to deliver the small-molecule cytotoxic drug specifically to target cells.

Upon the binding of an antibody to an antigen on the surface of tumor cells, ADC drugs are internalized into the cells via endocytosis. Subsequently, the small-molecule cytotoxic drug is released from the ADC and exerts its biological function. As a result, ADCs combine the potent cytotoxic effects of traditional small-molecule cytotoxic drugs with the tumor-targeting specificity of antibodies. To date, several ADC drugs have been used for the treatment of liquid tumors and solid tumors, such as POLIVY (Polatuzumab vedotin-piiq, CD79b-MMAE) approved by the FDA for the treatment of relapsed or refractory diffuse large B-cell lymphoma (R/R DLBCL); PADCEV (Enfortumab vedotin-ejfv, Nectin4-MMAE) approved by the FDA for the treatment of locally advanced or metastatic urothelial carcinoma (UC); Enhertu (Fam-trastuzumab deruxtecan-nxki, Her2-Dxd) for the treatment of breast cancer, etc.; Trodelvy (Sacituzumab govitecan-hziy, TROP2-SN38) for the treatment of patients with metastatic triple-negative breast cancer.

Camptothecin (CPT) is a small-molecule, plant-derived anticancer agent that functions as a DNA topoisomerase I inhibitor and has demonstrated significant efficacy against gastrointestinal cancer and head and neck cancers. Several camptothecin derivatives, such as irinotecan and exatecan, have been developed and are clinically used for treating various tumors, including intestinal, pancreatic, and lung cancers, etc. Camptothecin derivatives have also been utilized in ADC development, e.g., Enhertu which employs Dxd as the payload and Trodelvy which employs SN-38 as the payload. However, a substantial number of solid tumors remain poorly responsive to camptothecins (Joshua Z Drago, Shanu Modi, Sarat Chandarlapaty et al. Unlocking the potential of antibody-drug conjugates for cancer therapy. Nat Rev Clin Oncol. 2021 Jun;18(6): 327-344. doi: 10.1038/s41571-021-00470-8. Epub 2021 Feb 8.). Tumor cells can develop resistance to camptothecins and their corresponding ADCs through multiple mechanisms, such as reduced intracellular drug accumulation (G L Beretta, L Gatti, P Perego et al. Camptothecin resistance in cancer: insights into the molecular mechanisms of a DNA-damaging drug. Curr Med Chem. 2013;20(12):1541-65. doi: 10.2174/0929867311320120006.), which is associated with the fact that SN-38 is a substrate of P-glycoprotein (Michael Tagen, Yanli Zhuang, Fan Zhang et al. P-glycoprotein, but not multidrug resistance protein 4, plays a role in the systemic clearance of irinotecan and SN-38 in mice. Drug Metab Lett. 2010 Dec;4(4):195-201.).

Therefore, there remains a need to develop novel camptothecin derivatives with potent cytotoxic activity and ADC drugs with high efficacy and low toxicity. Notably, the camptothecin derivatives and ADC drugs of the present invention exhibt excellent therapeutic efficacy. Moreover, the camptothecin derivatives and ADC drugs of the present invention exhibit favorable tolerability, less advents toxic and side-effects and a wide therapeutic window. The ADC drugs of the present invention also exhibit favorable tumor tissue targeting and strong bystander effects.

## Content of the Invention

Summary of the Invention

In a first aspect, the present application provides an antibody-drug conjugate of formula (I), or a pharmaceutically acceptable salt, ester, solvate, tautomer, stereoisomer, prodrug, or isotopically labeled form thereof.

In a second aspect, the present application provides a compound of formula (II), or a pharmaceutically acceptable salt, ester, solvate, tautomer, stereoisomer, prodrug, or isotopically labeled form thereof.

In a third aspect, the present application provides a linker-payload conjugate of formula (III), or a pharmaceutically acceptable salt, ester, solvate, tautomer, stereoisomer, prodrug, or isotopically labeled form thereof.

In a fourth aspect, the present application provides a pharmaceutical composition comprising the agent of the present invention as defined herein, together with a pharmaceutically acceptable carrier, diluent, or excipient.

In a fifth aspect, the present application provides the agent of the present invention as defined herein for use in

treating or preventing cancer.

In a sixth aspect, the present application provides a method for preventing or treating cancer in a patient, comprising administering to the patient a therapeutically effective amount of the agent of the present invention as defined herein.

In a seventh aspect, the present application provides the use of the agent of the present invention as defined herein in the manufacture of a medicament for treating or preventing cancer.

In an eighth aspect, the present application provides a kit for treating or preventing cancer, comprising the agent of the present invention as defined herein and other co-administered drugs. Said co-administered drugs may have therapeutic effects identical to or different from those of the agent of the present invention.

In a ninth aspect, the present application provides a method for preparing the agent of the present invention as defined herein.

The agent of the present invention, including the camptothecin derivatives, linker-payload conjugates, and antibody-drug conjugates thereof in the present invention, exhibit high cytotoxic activity and are useful for treating or preventing tumors such as cancers, including but not limited to solid tumors, particularly lung cancer (e.g., small cell lung cancer, non-small cell lung cancer), liver cancer, digestive system cancers (e.g., intestinal cancer, gastric cancer, cardia cancer, esophageal cancer, appendiceal adenocarcinoma, colon cancer, rectal cancer, colorectal cancer, pancreatic cancer), bladder cancer, melanoma, breast cancer, ovarian cancer, cervical cancer, endometrial cancer, prostate cancer, basal cell carcinoma, cholangiocarcinoma, squamous cell carcinoma, thyroid cancer, brain cancer, head and neck cancer, peritoneal cancer, kidney cancer, urothelial carcinoma, testicular cancer, central nervous system tumors (e.g., glioma, glioblastoma such as glioblastoma multiforme, glioma, or sarcoma), choriocarcinoma, and oral epidermoid carcinoma; or hematological malignancies, particularly leukemias (e.g., acute or chronic myeloid leukemia, acute or chronic granulocytic leukemia), lymphomas (e.g., Hodgkin's lymphoma, non-Hodgkin's lymphoma, diffuse large B-cell lymphoma, acute B-cell lymphoma, follicular lymphoma), and multiple myeloma. Preferably, the cancer is ovarian cancer, colon cancer, lung adenocarcinoma, or oral epidermoid carcinoma. Compared with Dxd and SN-38, the agent of the present invention demonstrates superior tumor-killing activity.

Detailed Description of the Invention

In one aspect, the present invention provides an antibody-drug conjugate of formula (I)

$$Ab-\left[L_1-L_2-L_3-L_4-D\right]_n \qquad (I)$$

or a pharmaceutically acceptable salt or ester, solvate, tautomer, stereoisomer, prodrug, or isotopically labeled form thereof,

wherein:

Ab is an antibody or antigen-binding fragment thereof;

$L_1$ is each independently a linker unit;

$L_2$ is each independently a linking unit selected from the group consisting of:

$-(CR_mR_n)_t-(L_M)_{0-1}-(CH_2CH_2O)_s-(CR_mR_n)_t-CO-$,

$-(CR_mR_n)_t-L_M-(CR_mR_n)_t-(L_M)_{0-1}-L_N-L_M-(CH_2O)_s-(CR_mR_n)_t-CO-$,

$-(CR_mR_n)_t-L_M-(CR_mR_n)_t-N(R_p)-(CR_mR_n)_t-N(R_p)-(CR_mR_n)_t-CO-$,

and

$-(CR_mR_n)_t-L_M-CH(R_p)-CO-$, wherein the -CO- terminus of $L_2$ is connected to $L_3$, and the other terminus of $L_2$ is connected to $L_1$, and

wherein,

$R_m$ and $R_n$ are each independently H or $C_{1-6}$ alkyl,

$L_M$ is each independently -NH-CO- or -CO-NH-,

$L_N$ is each independently $-(CH_2CH_2O)_m-(CH_2)_t-$, $-(CH_2)_t-(OCH_2CH_2)_m-$, -(3- to 8-membered heteroarylene)-$(CH_2)_t-(OCH_2CH_2)_m$, or $-(OCH_2CH_2)_m-(CH_2)_t-$(3- to 8-membered heteroarylene)-,

$R_p$ is each independently $-CO-(CH_2CH_2O)_m-CH_3$ or $-(CH_2)_t-L_M-(CH_2CH_2O)_m-CH_3$,
s and t are each independently 0, 1, 2, 3, 4, 5, 6, 7, or 8, and
m is each independently 2, 3, 4, 5, 6, 7, or 8;

$L_3$ is each independently an amino acid residue, a short peptide chain consisting of 2-10 amino acid residues, or $-NH-(CH_2)_p-CO-$;
$L_4$ is each independently a bond or a spacer unit;
D is each independently a small molecule drug moiety derived from a compound of formula (II);

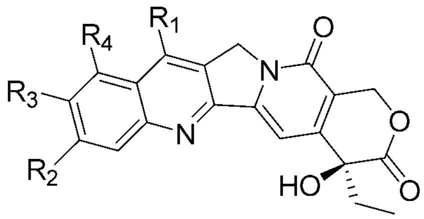

(II)

wherein,

$R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from the group consisting of halogen, CN, OH, SH and amino; $-(CH_2)_p-(C_{3-8}$ cycloalkyl) or $-(CH_2)_p-(C_{3-8}$ heterocycloalkyl), wherein the cycloalkyl and heterocycloalkyl are each optionally substituted with one or more substituents independently selected from the group consisting of halogen, CN, OH, SH, amino, $C_{1-6}$ alkyl, $C_{1-6}$ haloalkyl, $C_{1-6}$ hydroxyalkyl, $C_{1-6}$ cyanoalkyl and $C_{1-6}$ aminoalkyl; $-(CH_2)_p-(C_{1-6}$ alkoxy); $-(CH_2)_p-(C_{1-6}$ alkylthio); $-(CH_2)_p-NR_aR_b$; $-(CH_2)_p-CH=NR_a$; $-NH-CO-R_a$; $-NH-CO-(CH_2)_p-CH(OH)-R_a$; $-NH-(CH_2)_p-(CH=CH)-R_a$; or -COOH;
$R_2$ and $R_3$ are each independently halogen, cyano, $NO_2$, $C_{1-6}$ alkyl, $C_{1-6}$ haloalkyl, $C_{1-6}$ hydroxyalkyl, $C_{1-6}$ alkoxy, $-NH-CO-(C_{1-6}$ hydroxyalkyl), or $NR_aR_b$; or $R_2$ and $R_3$, together with the atoms to which they are attached, form a 4- to 8-membered heterocyclic group;
$R_4$ is H or $C_{3-8}$ cycloalkyl, preferably H;
$R_a$ and $R_b$ are each independently H, $C_{1-6}$ alkyl, $-CO-(C_{1-6}$ alkyl), $-SO-(C_{1-6}$ alkyl), $-SO_2-(C_{1-6}$ alkyl), or $-(CH_2)_q-(C_{3-8}$ cycloalkyl), wherein the alkyl and cycloalkyl are each optionally substituted with one or more substituents independently selected from the group consisting of halogen, CN, OH, SH and amino, and wherein the cycloalkyl is further optionally substituted with one or more substituents independently selected from the group consisting of $C_{1-6}$ alkyl, $C_{1-6}$ haloalkyl, $C_{1-6}$ hydroxyalkyl, $C_{1-6}$ cyanoalkyl and $C_{1-6}$ aminoalkyl; and
p and q are each independently 0, 1, 2, 3, 4, 5, or 6;
wherein D is linked to $L_4$ via $R_1$ or $R_3$, or via the oxygen from the hydroxy shown in formula (II); and
n is an integer from 0 to 10, preferably an integer from 0 to 8.

In another aspect, the present application provides an antibody-drug conjugate of formula (I):

$$Ab\left[L_1-L_2-L_3-L_4-D\right]_n \quad (I)$$

or a pharmaceutically acceptable salt or ester, solvate, tautomer, stereoisomer, prodrug, or isotopically labeled form thereof,
wherein:

Ab is an antibody or antigen-binding fragment thereof;
$L_1$ is each independently a linker unit;
$L_2$ is each independently a linking unit of the formula $-(CH_2CH_2O)_x-(CR_mR_n)_y-CO-$, wherein the -CO- terminus is connected to $L_3$ and the other terminus is connected to $L_1$, and wherein $R_m$ and $R_n$ are each independently H or $C_{1-6}$ alkyl, x is an integer from 0 to 5, y is an integer from 1 to 5; and the sum of x and y is ≤ 6;
$L_3$ is each independently an amino acid residue, a short peptide chain consisting of 2-10 amino acid residues, or $-NH-(CH_2)_p-CO-$;

$L_4$ is each independently a bond or a spacer unit;
D is each independently a small molecule drug moiety derived from a compound of formula (II);

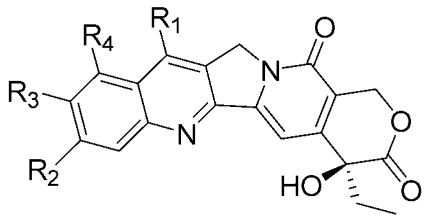

(II)

wherein,

$R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from the group consisting of halogen, CN, OH, SH and amino; $-(CH_2)_p-(C_{3-8}$ cycloalkyl) or $-(CH_2)_p-(C_{3-8}$ heterocycloalkyl), wherein the cycloalkyl and heterocycloalkyl are each optionally substituted with one or more substituents independently selected from the group consisting of halogen, CN, OH, SH, amino, $C_{1-6}$ alkyl, $C_{1-6}$ haloalkyl, $C_{1-6}$ hydroxyalkyl, $C_{1-6}$ cyanoalkyl and $C_{1-6}$ aminoalkyl; $-(CH_2)_p-(C_{1-6}$ alkoxy); $-(CH_2)_p-(C_{1-6}$ alkylthio); $-(CH_2)_p-NR_aR_b$; $-(CH_2)_p-CH=NR_a$; $-NH-CO-R_a$; $-NH-CO-(CH_2)_p-CH(OH)-R_a$; $-NH-(CH_2)_p-(CH=CH)-R_a$; or -COOH;
$R_2$ and $R_3$ are each independently halogen, cyano, $NO_2$, $C_{1-6}$ alkyl, $C_{1-6}$ haloalkyl, $C_{1-6}$ hydroxyalkyl, $C_{1-6}$ alkoxy, $-NH-CO-(C_{1-6}$ hydroxyalkyl), or $NR_aR_b$; or $R_2$ and $R_3$, together with the atoms to which they are attached, form a 4- to 8-membered heterocyclic group;
$R_4$ is H or $C_{3-8}$ cycloalkyl, preferably H;
$R_a$ and $R_b$ are each independently H, $C_{1-6}$ alkyl, $-CO-(C_{1-6}$ alkyl), $-SO-(C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino, and wherein the cycloalkyl is further optionally substituted with one or more substituents independently selected from the group consisting of $C_{1-6}$ alkyl, $C_{1-6}$ haloalkyl, $C_{1-6}$ hydroxyalkyl, $C_{1-6}$ cyanoalkyl and $C_{1-6}$ aminoalkyl; and
p and q are each independently 0, 1, 2, 3, 4, 5, or 6;
wherein D is linked to $L_4$ via $R_1$ or $R_3$, or via the oxygen from the hydroxy shown in formula (II); and
n is an integer from 0 to 10, preferably an integer from 0 to 8.

In another aspect, the present invention provides an antibody-drug conjugate of formula (I):

$$Ab-\left[L_1-L_2-L_3-L_4-D\right]_n \qquad (I)$$

or a pharmaceutically acceptable salt or ester, solvate, tautomer, stereoisomer, prodrug, or isotopically labeled form thereof,
wherein:

Ab is an antibody or antigen-binding fragment thereof;
$L_1$ is each independently a linker unit;
$L_2$ is each independently a linking unit of the formula $-(CH_2CH_2O)_x-(CR_mR_n)_y-CO-$, wherein the -CO- terminus is connected to $L_3$ and the other terminus is connected to $L_1$, and wherein $R_m$ and $R_n$ are each independently H or $C_{1-6}$ alkyl, x is an integer from 0 to 5, y is an integer from 1 to 5; and the sum of x and y is $\leq 6$;
$L_3$ is each independently an amino acid residue, a short peptide chain consisting of 2-10 amino acid residues, or $-NH-(CH_2)_p-CO-$;
$L_4$ is each independently a bond or a spacer unit;
D is each independently a small molecule drug moiety derived from a compound of formula (II):

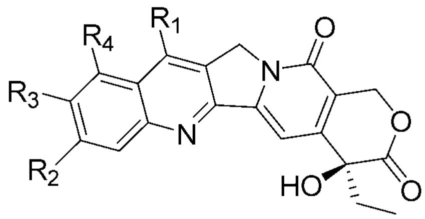

(II)

wherein,

$R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from halogen, CN, OH, SH, and amino; $-(CH_2)_p-(C_{3-8}$ cycloalkyl), wherein the cycloalkyl is optionally substituted with one or more substituents independently selected from the group consisting of halogen, CN, OH, SH and amino; $-(CH_2)_p-(C_{1-6}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH;

$R_2$ and $R_3$ are each independently halogen, cyano, $NO_2$, $C_{1-6}$ alkyl, $C_{1-6}$ haloalkyl, $C_{1-6}$ hydroxyalkyl, $C_{1-6}$ alkoxy, or $NR_aR_b$; or $R_2$ and $R_3$ together with the atoms to which they are attached form a 4- to 8-membered heterocyclic group;

$R_4$ is H or $C_{3-8}$ cycloalkyl, preferably H;

$R_a$ and $R_b$ are each independently H, $C_{1-6}$ alkyl, $-SO-(C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino;

p and q are each independently 0, 1, 2, 3, 4, 5, or 6;

wherein D is linked to $L_4$ via $R_1$ or $R_3$, or via the oxygen from the hydroxy in formula (II); and

n is an integer from 0 to 10, preferably an integer from 0 to 8.

In another aspect, the present invention provides a camptothecin derivative of formula (II):

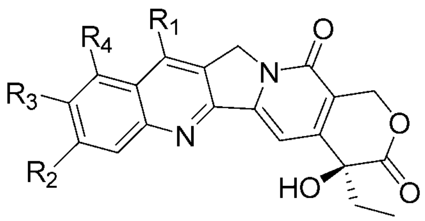

(II)

or a pharmaceutically acceptable salt or ester, solvate, tautomer, stereoisomer, prodrug, or isotopically labeled form thereof,
wherein,

$R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from the group consisting of halogen, CN, OH, SH and amino; $-(CH_2)_p-(C_{3-8}$ cycloalkyl) or $-(CH_2)_p-(C_{3-8}$ heterocycloalkyl), wherein the cycloalkyl and heterocycloalkyl are each optionally substituted with one or more substituents independently selected from the group consisting of halogen, CN, OH, SH, amino, $C_{1-6}$ alkyl, $C_{1-6}$ haloalkyl, $C_{1-6}$ hydroxyalkyl, $C_{1-6}$ cyanoalkyl, and $C_{1-6}$ aminoalkyl; $-(CH_2)_p-(C_{1-6}$ alkoxy); $-(CH_2)_p-(C_{1-6}$ alkylthio); $-(CH_2)_p-NR_aR_b$; $-(CH_2)_p-CH=NR_a$; $-NH-CO-R_a$; $-NH-CO-(CH_2)_p-CH(OH)-R_a$; $-NH-(CH_2)_p-(CH=CH)-R_a$; or -COOH;

$R_2$ and $R_3$ are each independently halogen, cyano, $NO_2$, $C_{1-6}$ alkyl, $C_{1-6}$ haloalkyl, $C_{1-6}$ hydroxyalkyl, $C_{1-6}$ alkoxy, $-NH-CO-(C_{1-6}$ hydroxyalkyl), or $NR_aR_b$; or $R_2$ and $R_3$, together with the atoms to which they are attached, form a 4- to 8-membered heterocyclic group;

$R_4$ is H or $C_{3-8}$ cycloalkyl, preferably H;

$R_a$ and $R_b$ are each independently H, $C_{1-6}$ alkyl, $-CO-(C_{1-6}$ alkyl), $-SO-(C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino, and wherein the cycloalkyl is further optionally substituted with one or more substituents independently selected from the

group consisting of $C_{1-6}$ alkyl, $C_{1-6}$ haloalkyl, $C_{1-6}$ hydroxyalkyl, $C_{1-6}$ cyanoalkyl and $C_{1-6}$ aminoalkyl; and p and q are each independently 0, 1, 2, 3, 4, 5, or 6.

In another aspect, the present invention provides a compound of formula (II):

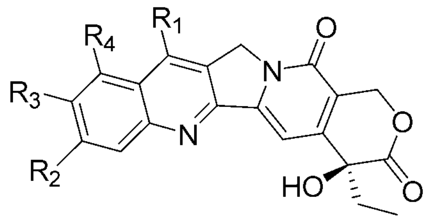

(II)

or a pharmaceutically acceptable salt or ester, solvate, tautomer, stereoisomer, prodrug, or isotopically labeled form thereof,
wherein,

$R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from the group consisting of halogen, CN, OH, SH and amino; $-(CH_2)_p-(C_{3-8}$ cycloalkyl), wherein the cycloalkyl is optionally substituted with one or more substituents independently selected from the group consisting of halogen, CN, OH, SH and amino; $-(CH_2)_p-(C_{1-6}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH;
$R_2$ and $R_3$ are each independently halogen, cyano, $NO_2$, $C_{1-6}$ alkyl, $C_{1-6}$ haloalkyl, $C_{1-6}$ hydroxyalkyl, $C_{1-6}$ alkoxy, or $NR_aR_b$; or $R_2$ and $R_3$ together with the atoms to which they are attached form a 4- to 8-membered heterocyclic group;
$R_4$ is H or $C_{3-8}$ cycloalkyl, preferably H;
$R_a$ and $R_b$ are each independently H, $C_{1-6}$ alkyl, $-SO-(C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino; and
p and q are each independently 0, 1, 2, 3, 4, 5, or 6.

In another aspect, the present invention provides a linker-payload conjugate of formula (III):

$$Lg-L_1-L_2-L_3-L_4-D \qquad (III)$$

or a pharmaceutically acceptable salt or ester, solvate, tautomer, stereoisomer, prodrug, or isotopically labeled form thereof,
wherein $L_1$, $L_2$, $L_3$, $L_4$ and D are as defined herein and wherein

when $L_1$ is

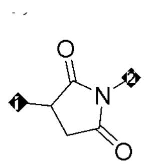,

Lg and $L_1$ together form

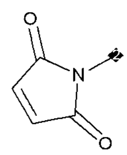;

when $L_1$ is not

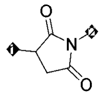,

Lg is a leaving group. Preferably, Lg is halogen, sulfonyl (e.g., methanesulfonyl, p-toluenesulfonyl), sulfonyloxy (e.g., methanesulfonyloxy, $CF_3SO_3$-, p-toluenesulfonyloxy), a tertiary-ammonium group (e.g., $Me_3N^+$ or $Et_3N^+$), or a diazonium group. More preferably, Lg is F, Cl, Br, or methanesulfonyl. Especially preferably, Lg is methanesulfonyl.

In some embodiments, $R_1$ is: H; halogen; CN; OH; SH; $C_{1-6}$ alkyl; $C_{1-6}$ haloalkyl; $C_{1-6}$ hydroxyalkyl; -$(CH_2)_p$-($C_{3-8}$ cycloalkyl) or -$(CH_2)_p$-($C_{3-8}$ heterocycloalkyl), wherein the cycloalkyl and the heterocycloalkyl are each optionally substituted with one or more substituents independently selected from the group consisting of OH, SH, $C_{1-6}$ alkyl, and $C_{1-6}$ hydroxyalkyl; -$(CH_2)_p$-($C_{1-6}$ alkoxy); -$(CH_2)_p$-($C_{1-6}$ alkylthio); -$(CH_2)_p$-$NR_aR_b$; -$(CH_2)_p$-CH=$NR_a$; -NH-CO-$R_a$; -NH-CO-$(CH_2)_p$-CH(OH)-$R_a$; -NH-$(CH_2)_p$-(CH=CH)-$R_a$; or -COOH. Further, $R_a$ and $R_b$ are each independently H, $C_{1-6}$ alkyl, $C_{1-6}$ hydroxyalkyl, $C_{1-6}$ aminoalkyl, -$SO_2$-($C_{1-6}$ alkyl), or -$(CH_2)_q$-($C_{3-8}$ cycloalkyl optionally substituted with one or more substituents independently selected from the group consisting of hydroxy and $C_{1-6}$ hydroxyalkyl).

In other embodiments, $R_1$ is: H; $C_{1-6}$ alkyl; $C_{1-6}$ haloalkyl; $C_{1-6}$ hydroxyalkyl; -$(CH_2)_p$-($C_{3-8}$ cycloalkyl); -$(CH_2)_p$-($C_{3-8}$ hydroxycycloalkyl); -$(CH_2)_p$-($C_{3-8}$ heterocycloalkyl), wherein the heterocycloalkyl is optionally substituted with one or more substituents independently selected from the group consisting of OH, $C_{1-6}$ alkyl and $C_{1-6}$ hydroxyalkyl, preferably selected from the group consisting of OH and $C_{1-6}$ hydroxyalkyl, more preferably selected from $C_{1-6}$ hydroxyalkyl; -$(CH_2)_p$-($C_{1-6}$ alkoxy); -$(CH_2)_p$-($C_{1-6}$ alkylthio); -$(CH_2)_p$-$NR_aR_b$; -$(CH_2)_p$-CH=$NR_a$; -NH-CO-$R_a$; -NH-CO-$(CH_2)_p$-CH(OH)-$R_a$; -NH-$(CH_2)_p$-(CH=CH)-$R_a$; or -COOH. Further, $R_a$ and $R_b$ are each independently H, $C_{1-6}$ alkyl, $C_{1-6}$ hydroxyalkyl, $C_{1-6}$ aminoalkyl, -$SO_2$-($C_{1-6}$ alkyl), -$(CH_2)_q$-($C_{3-8}$ cycloalkyl), -$(CH_2)_q$-($C_{3-8}$ hydroxycycloalkyl), -$(CH_2)_q$-($C_{3-8}$ cycloalkyl substituted with one or more amino groups), or -$(CH_2)_q$-($C_{3-8}$ cycloalkyl substituted with one or more $C_{1-6}$ hydroxyalkyl).

In further embodiments, $R_1$ is: H; $C_{1-6}$ alkyl; $C_{1-6}$ haloalkyl; $C_{1-6}$ hydroxyalkyl; -$(CH_2)_p$-($C_{3-8}$ heterocycloalkyl), wherein the heterocycloalkyl is optionally substituted with one or more substituents independently selected from the group consisting of OH and $C_{1-6}$ hydroxyalkyl, more preferably selected from $C_{1-6}$ hydroxyalkyl; -$(CH_2)_p$-($C_{1-6}$ alkoxy); -$(CH_2)_p$-($C_{1-6}$ alkylthio); -$(CH_2)_p$-$NH_2$; -$(CH_2)_p$-NH($C_{1-6}$ alkyl); -$(CH_2)_p$-NH($C_{1-6}$ hydroxyalkyl); -$(CH_2)_p$-NH($C_{1-6}$ aminoalkyl); -$(CH_2)_p$-NH-$(CH_2)_q$-($C_{3-8}$ cycloalkyl optionally substituted with one or more $C_{1-6}$ hydroxyalkyl); -$(CH_2)_p$-N($C_{1-6}$ alkyl)(-$SO_2$-$C_{1-6}$ alkyl); -$(CH_2)_p$-CH=N($C_{1-6}$ alkyl); -NH-CO-($C_{1-6}$ hydroxyalkyl); -NH-CO-($C_{3-8}$ hydroxycycloalkyl); -NH-CO-$(CH_2)_p$-CH(OH)-($C_{1-6}$ alkyl); -NH-CO-$(CH_2)_p$-CH(OH)-($C_{3-8}$ cycloalkyl); -NH-$(CH_2)_p$-(CH=CH)-($C_{1-6}$ alkyl); or -COOH.

In still other embodiments, $R_1$ is H; $C_{1-6}$ alkyl; $C_{1-6}$ haloalkyl; $C_{1-6}$ hydroxyalkyl; -$(CH_2)_p$-($C_{3-8}$ heterocycloalkyl), wherein the heterocycloalkyl is optionally substituted with one or more $C_{1-6}$ hydroxyalkyl; -$(CH_2)_p$-($C_{1-6}$ alkoxy); $(CH_2)_p$-($C_{1-6}$ alkylthio); -$(CH_2)_p$-$NH_2$; -$(CH_2)_p$-NH($C_{1-6}$ alkyl); -$(CH_2)_p$-NH($C_{1-6}$ hydroxyalkyl); -$(CH_2)_p$-NH($C_{1-6}$ aminoalkyl); -$(CH_2)_p$-NH-$(CH_2)_q$-($C_{3-8}$ cycloalkyl), wherein the cycloalkyl is optionally substituted with one or more substituents selected from $C_{1-6}$ hydroxyalkyl; -$(CH_2)_p$-N($C_{1-6}$ alkyl)(-$SO_2$-$C_{1-6}$ alkyl); -CH=N($C_{1-6}$ alkyl); -NH-CO-($C_{1-6}$ hydroxyalkyl); -NH-CO-($C_{3-8}$ hydroxycycloalkyl); -NH-CO-$(CH_2)_p$-CH(OH)-($C_{1-6}$ alkyl); -NH-CO-$(CH_2)_p$-CH(OH)-($C_{3-8}$ cycloalkyl); -NH-$(CH_2)_p$-(CH=CH)-($C_{1-6}$ hydroxyalkyl); or -COOH.

In yet other embodiments, $R_1$ is H; $C_{1-6}$ alkyl; $C_{1-6}$ haloalkyl; $C_{1-6}$ hydroxyalkyl; -$(CH_2)_p$-($C_{3-8}$ heterocycloalkyl), wherein the heterocycloalkyl is optionally substituted with one or more $C_{1-6}$ hydroxyalkyl; -$(CH_2)_p$-($C_{1-6}$ alkylthio); -$(CH_2)_p$-$NH_2$; -$(CH_2)_p$-NH($C_{1-6}$ alkyl); -$(CH_2)_p$-NH($C_{1-6}$ hydroxyalkyl); -$(CH_2)_p$-NH($C_{1-6}$ aminoalkyl); -$(CH_2)_p$-NH-$(CH_2)_q$-($C_{3-8}$ cycloalkyl), wherein the cycloalkyl is optionally substituted with one or more substituents selected from $C_{1-6}$ hydroxyalkyl; -$(CH_2)_p$-N($C_{1-6}$ alkyl)(-$SO_2$-$C_{1-6}$ alkyl); -CH=N($C_{1-6}$ alkyl); -NH-CO-($C_{1-6}$ hydroxyalkyl); -NH-CO-($C_{3-8}$ hydroxycycloalkyl); -NH-CO-$(CH_2)_p$-CH(OH)-($C_{3-8}$ cycloalkyl); -NH-$(CH_2)_p$-(CH=CH)-($C_{1-6}$ hydroxyalkyl); or -COOH.

In some embodiments, $R_1$ is: H; $C_{1-6}$ alkyl; -$(CH_2)_p$-($C_{3-8}$ heterocycloalkyl optionally substituted with one or more $C_{1-6}$ hydroxyalkyl); -$(CH_2)_p$-($C_{1-6}$ alkylthio); -$(CH_2)_p$-($NH_2$); -$(CH_2)_p$-NH($C_{1-6}$ alkyl); -$(CH_2)_p$-NH($C_{1-6}$ hydroxyalkyl); -$(CH_2)_p$-NH-$(CH_2)_q$-($C_{3-8}$ cycloalkyl optionally substituted with one or more $C_{1-6}$ hydroxyalkyl); -NH-CO-$(CH_2)_p$-CH(OH)-($C_{3-8}$ cycloalkyl); or -NH-$(CH_2)_p$-(CH=CH)-($C_{1-6}$ alkyl).

In some embodiments, $R_1$ is: H, $C_{1-6}$ alkyl, -$(CH_2)_p$-($C_{3-8}$ nitrogen-containing heterocycloalkyl optionally substituted with one or more $C_{1-6}$ hydroxyalkyl), -$(CH_2)_p$-($C_{1-6}$alkylthio), -$NH_2$, -$(CH_2)_p$-NH($C_{1-6}$ alkyl), -NH($C_{1-6}$ hydroxyalkyl), -$(CH_2)_p$-NH-$(CH_2)_q$-($C_{3-8}$ cycloalkyl optionally substituted with one or more $C_{1-6}$ hydroxyalkyl), -NH-CO-CH(OH)-($C_{3-8}$ cycloalkyl), or -NH-(CH=CH)-($C_{1-6}$ alkyl).

In further embodiments, $R_1$ is: H, $C_{1-6}$ alkyl, $C_{3-8}$ nitrogen-containing heterocycloalkyl optionally substituted with one or more $C_{1-6}$ hydroxyalkyl, -$CH_2$-($C_{1-6}$ alkylthio), -$NH_2$, -$CH_2$-NH($C_{1-6}$ alkyl), -NH($C_{1-6}$ hydroxyalkyl), -NH-($C_{3-8}$

cycloalkyl optionally substituted with one or more $C_{1-6}$ hydroxyalkyl), $-CH_2-NH-CH_2-(C_{3-8}$ cycloalkyl), -NH-CO-CH(OH)-($C_{3-8}$ cycloalkyl), and -NH-(CH=CH)-($C_{1-6}$ alkyl).

In some embodiments, $R_1$ is $C_{1-6}$ alkyl; $-(CH_2)_p-(C_{3-8}$ nitrogen-containing heterocycloalkyl optionally substituted with one or more $C_{1-6}$ hydroxyalkyl); $-(CH_2)_p-(C_{1-6}$ alkylthio); $-(CH_2)_p-NH(C_{1-6}$ alkyl); $-(CH_2)_p-NH-(CH_2)_q-(C_{3-8}$ cycloalkyl optionally substituted with one or more $C_{1-6}$ hydroxyalkyl); or $-(CH_2)_p-NH(C_{1-6}$ hydroxyalkyl); or $-NH-(CH_2)_p-(CH=CH)-(C_{1-}$ alkyl). Preferably, $R_1$ is $C_{1-6}$ alkyl; $-(CH_2)_p-(C_{3-8}$ nitrogen-containing heterocycloalkyl optionally substituted with one $C_{1-6}$ hydroxyalkyl); $-CH_2-(C_{1-6}$ alkylthio); -NH($C_{1-6}$ alkyl); -NH($C_{1-6}$ hydroxyalkyl); $-(CH_2)_p-NH-(CH_2)_q-(C_{3-8}$ cycloalkyl optionally substituted with one $C_{1-6}$ hydroxyalkyl); or -NH-(CH=CH)-($C_{1-6}$ alkyl).

In some embodiments, $R_1$ is $C_{1-6}$ alkyl; $-(CH_2)_p-(C_{3-8}$ nitrogen-containing heterocycloalkyl optionally substituted with one $C_{1-6}$ hydroxyalkyl); $-(CH_2)_p-(C_{1-6}$ alkylthio); $-NH_2$; $-(CH_2)_p-NH(C_{1-6}$ alkyl); $-(CH_2)_p-NH(C_{1-6}$ hydroxyalkyl); $-(CH_2)_p-NH-(CH_2)_q-(C_{3-8}$ cycloalkyl optionally substituted with one $C_{1-6}$ hydroxyalkyl); -NH-CO-($C_{1-6}$ hydroxyalkyl); $-NH-CO-(CH_2)_p-CH(OH)-(C_{3-8}$ cycloalkyl); or NH-(CH=CH)-($C_{1-6}$ alkyl). Preferably, $R_1$ is $C_{1-6}$ alkyl; a $C_{3-8}$ nitrogen-containing heterocycloalkyl substituted with one $C_{1-6}$ hydroxyalkyl; $-NH_2$; $-(CH_2)_p-NH(C_{1-6}$ alkyl); $-(CH_2)_p-NH(C_{1-6}$ hydroxyalkyl); -NH-($C_{3-8}$ cycloalkyl substituted with one $C_{1-6}$ hydroxyalkyl); -NH-CO-($C_{1-6}$ hydroxyalkyl); or -NH-(CH=CH)-($C_{1-6}$ alkyl).

In some embodiments, $R_1$ is selected from the group consisting of: H; methyl, ethyl, propyl, butyl, pentyl, hexyl; chloromethyl, fluoromethyl, bromomethyl, chloroethyl, fluoroethyl, bromoethyl, chloropropyl, fluoropropyl, bromopropyl, chlorobutyl, fluorobutyl, bromobutyl; hydroxymethyl, hydroxyethyl, hydroxypropyl, hydroxybutyl, hydroxypentyl, hydroxyhexyl; -(piperidinyl)-$CH_2OH$; $-CH_2$-(methylthio), $-CH_2$-(ethylthio), $-CH_2$-(propylthio), $-CH_2$-(butylthio), $-CH_2$-(pentylthio), $-CH_2$-(hexylthio); $-NH_2$, -NHMe, -NHEt, -NHPr, -NHBu, -NH(pentyl), -NH(hexyl); -NH(hydroxymethyl), -NH(hydroxyethyl), -NH(hydroxypropyl), -NH(hydroxybutyl), -NH(hydroxypentyl), -NH(hydroxyhexyl); -NH(aminomethyl), -NH(aminoethyl), -NH(aminopropyl), -NH(aminobutyl), -NH(aminopentyl), -NH(aminohexyl);

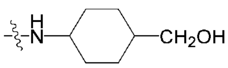

;

$-CH_2$-NHMe, $-CH_2$-NHEt, $-CH_2$-NHPr, $-CH_2$-NHBu, $-CH_2$-NH(pentyl), $-CH_2$-NH(hexyl); $-CH_2-NH-CH_2$-(cyclopropyl), $-CH_2-NH-CH_2$-(cyclobutyl), $-CH_2-NH-CH_2$-(cyclopentyl), $-CH_2-NH-CH_2$-(cyclohexyl); $-(CH_2)_2-N(Pr)(-SO_2Me)$; -CH=NMe, -CH=NEt, -CH=NPr, -CH=NBu, -CH=N(pentyl), -CH=N(hexyl); -NH-CO-hydroxymethyl, -NH-CO-hydroxyethyl, -NH-CO-hydroxypropyl, -NH-CO-hydroxybutyl, -NH-CO-hydroxypentyl, -NH-CO-hydroxyhexyl; -NH-CO-(hydroxycyclopropyl), -NH-CO-(hydroxycyclobutyl), -NH-CO-(hydroxycyclopentyl), -NH-CO-(hydroxycyclohexyl); -NH-CO-CH(OH)-(cyclopropyl), -NH-CO-CH(OH)-(cyclobutyl), -NH-CO-CH(OH)-(cyclopentyl), -NH-CO-CH(OH)-(cyclohexyl); $-NH-CO-CH_2$-CH(OH)(Me), $-NH-CO-CH_2$-CH(OH)(Et), $-NH-CO-CH_2$-CH(OH)(Pr), $-NH-CO-CH_2$-CH(OH)(Bu), $-NH-CO-CH_2$-CH(OH)(pentyl), $-NH-CO-CH_2$-CH(OH)(hexyl); $-NH-(CH_2)-(CH=CH)-(CH_2OH)$; or -COOH.

In some embodiments, $R_1$ is H; pentyl; chloromethyl; hydroxybutyl; -(piperidinyl)-$CH_2OH$; $-CH_2$-(propylthio); $-NH_2$; -NH(pentyl); $-CH_2$-NH(pentyl); -NH(hydroxybutyl); -NH(aminobutyl);

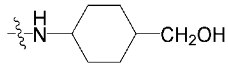

;

$-CH_2-NH-CH_2$-(cyclopentyl); $-(CH_2)_2-N(Pr)(-SO_2Me)$; -CH=N(pentyl); -NH-CO-(hydroxymethyl); -NH-CO-(hydroxycyclohexyl); -NH-CO-CH(OH)-(cyclopropyl); $-NH-CO-CH_2$-CH(OH)(Me); $-NH-(CH_2)-(CH=CH)-(CH_2OH)$; or -COOH.

In some embodiments, $R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from the group consisting of halogen, CN, OH, SH and amino; $-(CH_2)_p-(C_{3-8}$ cycloalkyl), wherein the cycloalkyl is optionally substituted with one or more substituents independently selected from the group consisting of halogen, CN, OH, SH and amino; $-(CH_2)_p-(C_{1-6}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH; wherein $R_a$ and $R_b$ are each independently H, $C_{1-6}$ alkyl, -SO-($C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino.

In some embodiments, $R_1$ is: H; halogen; CN; OH; SH; $C_{1-6}$ alkyl; $C_{1-6}$ haloalkyl; $C_{1-6}$ hydroxyalkyl; $-(CH_2)_p-(C_{3-8}$ cycloalkyl); $-(CH_2)_p-(C_{3-8}$ hydroxycycloalkyl); $-(CH_2)_p-(C_{1-6}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH.

In further embodiments, $R_1$ is H; $C_{1-6}$ alkyl; $C_{1-6}$ haloalkyl; $C_{1-6}$ hydroxyalkyl; $-(CH_2)_p-(C_{1-6}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH; preferably wherein $R_a$ and $R_b$ are each independently H, $C_{1-6}$ alkyl, $C_{1-6}$ hydroxyalkyl, $C_{1-6}$ aminoalkyl, $-SO_2-(C_{1-6}$ alkyl), $-(CH_2)_q-(C_{3-8}$ cycloalkyl), or $-(CH_2)_q-(C_{3-8}$ hydroxycycloalkyl).

In further embodiments, $R_1$ is H; $C_{1-6}$ alkyl; $C_{1-6}$ haloalkyl; $C_{1-6}$ hydroxyalkyl; $-(CH_2)_p-(C_{1-6}$ alkoxy); $-(CH_2)_p-(C_{1-6}$ alkylthio); $-(CH_2)_p-NH_2$; $-(CH_2)_p-NH(C_{1-6}$ alkyl); $-(CH_2)_p-NH(C_{1-6}$ hydroxyalkyl); $-(CH_2)_p-NH(C_{1-6}$ aminoalkyl); $-(CH_2)_p-NH-(CH_2)_q-(C_{3-8}$ cycloalkyl); $-(CH_2)_p-N(C_{1-6}$ alkyl)($-SO_2-C_{1-6}$ alkyl); $-CH=N(C_{1-6}$ alkyl); $-NH-CO-(C_{1-6}$ hydroxyalkyl); $-NH-CO-(C_{3-8}$ hydroxycycloalkyl); $-NH-CO-(CH_2)_p-CH(OH)-(C_{1-6}$ alkyl); $-NH-CO-(CH_2)_p-CH(OH)-(C_{3-8}$ cycloalkyl); or -COOH.

In some embodiments, $R_1$ is H, $C_{1-6}$ alkyl, $-(CH_2)_p-(C_{1-6}$ alkylthio), $-(CH_2)_p-(NH_2)$, $-(CH_2)_p-NH(C_{1-6}$ alkyl), $-(CH_2)_p-NH(C_{1-6}$ hydroxyalkyl), $-(CH_2)_p-NH-(CH_2)_q-(C_{3-8}$ cycloalkyl), or $-NH-CO-(CH_2)_p-CH(OH)-(C_{3-8}$ cycloalkyl).

In further embodiments, $R_1$ is H, $C_{1-6}$ alkyl, $-(CH_2)_p-(C_{1-6}$ alkylthio), $-NH_2$, $-(CH_2)_p-NH(C_{1-6}$ alkyl), $-NH(C_{1-6}$ hydroxyalkyl), $-(CH_2)_p-NH-(CH_2)_q-(C_{3-8}$ cycloalkyl), or $-NH-CO-CH(OH)-(C_{3-8}$ cycloalkyl).

In further embodiments, $R_1$ is H, $C_{1-6}$ alkyl, $-CH_2-(C_{1-6}$ alkylthio), $-NH_2$, $-CH_2-NH(C_{1-6}$ alkyl), $-NH(C_{1-6}$ hydroxyalkyl), $-CH_2-NH-CH_2-(C_{3-8}$ cycloalkyl), or $-NH-CO-CH(OH)-(C_{3-8}$ cycloalkyl).

In some embodiments, $R_1$ is $C_{1-6}$ alkyl, $-(CH_2)_p-(C_{1-6}$ alkylthio), $-(CH_2)_p-NH(C_{1-6}$ alkyl), or $-(CH_2)_p-NH(C_{1-6}$ hydroxyalkyl). Preferably, $R_1$ is $C_{1-6}$ alkyl, $-CH_2-(C_{1-6}$ alkylthio), $-NH(C_{1-6}$ alkyl), or $-NH(C_{1-6}$ hydroxyalkyl).

In some embodiments, $R_1$ is $C_{1-6}$ alkyl, $-(CH_2)_p-(C_{1-6}$ alkylthio), $-NH_2$, $-(CH_2)_p-NH(C_{1-6}$ alkyl), $-(CH_2)_p-NH(C_{1-6}$ hydroxyalkyl), $-NH-CO-(C_{1-6}$ hydroxyalkyl), or $-NH-CO-(CH_2)_p-CH(OH)-(C_{3-8}$ cycloalkyl). Preferably, $R_1$ is $C_{1-6}$ alkyl, $-NH_2$, $-(CH_2)_p-NH(C_{1-6}$ alkyl), $-(CH_2)_p-NH(C_{1-6}$ hydroxyalkyl), or $-NH-CO-(C_{1-6}$ hydroxyalkyl).

In some embodiments, $R_1$ is: $C_{1-6}$ alkyl; $-(CH_2)_p-(C_{3-8}$ heterocycloalkyl), wherein the heterocycloalkyl is optionally substituted with one or more substituents independently selected from OH, $C_{1-6}$ alkyl or $C_{1-6}$ hydroxyalkyl; $-(CH_2)_p-NR_aR_b$, wherein $R_a$ and $R_b$ are each independently H, $C_{1-6}$ alkyl, $C_{1-6}$ hydroxyalkyl, or $-(CH_2)_q-(C_{3-8}$ cycloalkyl optionally substituted with one or more substituents independently selected from hydroxy and $C_{1-6}$ hydroxyalkyl); or $NH-(CH_2)_p-(CH=CH)-R_a$, wherein $R_a$ is $C_{1-6}$ hydroxyalkyl, and p is 0, 1, 2, 3, or 4. Preferably, $R_1$ is: $C_{1-6}$ alkyl; $-(CH_2)_p-(C_{3-8}$ heterocycloalkyl optionally substituted with one $C_{1-6}$ hydroxyalkyl); $-(CH_2)_p-NR_aR_b$, wherein $R_a$ is H and $R_b$ is $C_{1-6}$ hydroxyalkyl or $-(CH_2)_q-(C_{3-8}$ cycloalkyl optionally substituted with one $C_{1-6}$ hydroxyalkyl); or $-NH-(CH_2)_p-(CH=CH)-R_a$, wherein $R_a$ is $C_{1-6}$ hydroxyalkyl, and p is 0, 1, 2, 3 or 4.

In other embodiments, $R_1$ is $C_{1-6}$ alkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-NH(C_{1-6}$ hydroxyalkyl); $-(CH_2)_p-NH-(CH_2)_q-(C_{3-8}$ cycloalkyl), wherein the cycloalkyl is optionally substituted with one or more substituents selected from $C_{1-6}$ hydroxyalkyl; or $-NH-(CH_2)_p-(CH=CH)-(C_{1-6}$ hydroxyalkyl).

In further embodiments, $R_1$ is $C_{1-6}$ alkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-(C_{1-6}$ alkylthio); $-(CH_2)_p-NH_2$; $-(CH_2)_p-NH(C_{1-6}$ alkyl); $-(CH_2)_p-NH(C_{1-6}$ hydroxyalkyl); $-(CH_2)_p-NH-(CH_2)_q-(C_{3-8}$ cycloalkyl), wherein the cycloalkyl is optionally substituted with one or more substituents selected from $C_{1-6}$ hydroxyalkyl; $-NH-CO-(C_{1-6}$ hydroxyalkyl); or $-NH-CO-(CH_2)_p-CH(OH)-(C_{3-8}$ cycloalkyl).

In still other embodiments, $R_1$ is $C_{1-6}$ alkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-NH_2$; $-(CH_2)_p-NH(C_{1-6}$ alkyl); $-(CH_2)_p-NH(C_{1-6}$ hydroxyalkyl); $-(CH_2)_p-NH-(CH_2)_q-(C_{3-8}$ cycloalkyl), wherein the cycloalkyl is optionally substituted with one or more substituents selected from $C_{1-6}$ hydroxyalkyl; $-NH-CO-(C_{1-6}$ hydroxyalkyl); or $-NH-CO-(CH_2)_p-CH(OH)-(C_{3-8}$ cycloalkyl).

In yet other embodiments, $R_1$ is $C_{1-6}$ alkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-NH(C_{1-6}$ hydroxyalkyl); $-(CH_2)_p-NH-(CH_2)_q-(C_{3-8}$ cycloalkyl), wherein the cycloalkyl is optionally substituted with one or more substituents selected from $C_{1-6}$ hydroxyalkyl; or $-NH-CO-(CH_2)_p-CH(OH)-(C_{3-8}$ cycloalkyl).

In other embodiments, $R_1$ is $C_{1-6}$ alkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-NH(C_{1-6}$ hydroxyalkyl); or $-(CH_2)_p-NH-(CH_2)_q-(C_{3-8}$ cycloalkyl), wherein the cycloalkyl is optionally substituted with one or more substituents selected from $C_{1-6}$ hydroxyalkyl.

In further embodiments, p is 0, 1, 2, 3, or 4. In some further embodiments, p is 0. In other further embodiments, p is 1. In yet other further embodiments, p is 2. In still other further embodiments, p is 3. In still further embodiments, p is 4.

In some embodiments, $R_1$ is selected from: H; methyl, ethyl, propyl, butyl, pentyl, hexyl; chloromethyl, fluoromethyl, bromomethyl, chloroethyl, fluoroethyl, bromoethyl, chloropropyl, fluoropropyl, bromopropyl, chlorobutyl, fluorobutyl, bromobutyl; hydroxymethyl, hydroxyethyl, hydroxypropyl, hydroxybutyl, hydroxypentyl, hydroxyhexyl; $-CH_2-$(methylthio), $-CH_2-$(ethylthio), $-CH_2-$(propylthio), $-CH_2-$(butylthio), $-CH_2-$(pentylthio), $-CH_2-$(hexylthio); $-NH_2$, -NHMe, -NHEt, -NHPr, -NHBu, -NH(pentyl), -NH(hexyl); -NH(hydroxymethyl), -NH(hydroxyethyl), -NH(hydroxypropyl), -NH(hydroxybutyl), -NH(hydroxypentyl), -NH(hydroxyhexyl); -NH(aminomethyl), -NH(aminoethyl), -NH(aminopropyl), -NH(aminobutyl), -NH(aminopentyl), -NH(aminohexyl); $-CH_2-NHMe$, $-CH_2-NHEt$, $-CH_2-NHPr$, $-CH_2-NHBu$, $-CH_2-NH$(pentyl), $-CH_2-NH$(hexyl); $-CH_2-NH-CH_2-$(cyclopropyl), $-CH_2-NH-CH_2-$(cyclobutyl), $-CH_2-NH-CH_2-$(cyclopentyl), $-CH_2-NH-CH_2-$(cyclohexyl); $-(CH_2)_2-N(Pr)(-SO_2Me)$; -CH=NMe, -CH=NEt, -CH=NPr, -CH=NBu, -CH=N(pentyl), -CH=N(hexyl); -NH-CO-hydroxymethyl, -NH-CO-hydroxyethyl, -NH-CO-hydroxypropyl, -NH-CO-hydroxybutyl, -NH-CO-hydroxypentyl, -NH-CO-hydroxyhexyl; -NH-CO-(hydroxycyclopropyl), -NH-CO-(hydroxycyclobutyl), -NH-CO-(hydroxycyclopentyl), -NH-CO-(hydroxycyclohexyl); -NH-CO-CH(OH)-(cyclopropyl), -NH-CO-CH(OH)-(cyclobutyl), -NH-CO-CH(OH)-(cyclopentyl), -NH-CO-CH(OH)-(cyclohexyl); -NH-CO-$CH_2$-CH(OH)(Me), -NH-CO-$CH_2$-CH(OH)(Et), -NH-CO-$CH_2$-CH(OH)(Pr), -NH-CO-$CH_2$-CH(OH)(Bu), -NH-CO-$CH_2$-CH(OH)(pentyl), -NH-CO-$CH_2$-CH(OH)(hexyl); or -COOH.

In some embodiments, $R_1$ is H; pentyl; chloromethyl; hydroxybutyl; -$CH_2$-(propylthio); -$NH_2$; -NH(pentyl); -$CH_2$-NH(pentyl); -NH(hydroxybutyl); -NH(aminobutyl); -$CH_2$-NH-$CH_2$-(cyclopentyl); -$(CH_2)_2$-N(Pr)(-$SO_2$Me); -CH=N(pentyl); -NH-CO-(hydroxymethyl); -NH-CO-(hydroxycyclohexyl); -NH-CO-CH(OH)-(cyclopropyl); -NH-CO-$CH_2$-CH(OH)(Me); or -COOH.

In some embodiments, $R_1$ is -NH(hydroxymethyl), -NH(hydroxyethyl), -NH(hydroxypropyl), -NH(hydroxybutyl), -NH(hydroxypentyl), -NH(hydroxyhexyl); -(azolidinyl)-($CH_2$OH), -(piperidinyl)-($CH_2$OH), -(azepanyl)-($CH_2$OH); or -NH-cyclopentyl-($CH_2$OH), -NH-cyclohexyl-($CH_2$OH), -NH-cycloheptyl-($CH_2$OH). Preferably, $R_1$ is -NH(hydroxybutyl), -(piperidinyl)-($CH_2$OH), or -NH-cyclohexyl-($CH_2$OH).

In some embodiments, $R_1$ is: pentyl; -NH(hydroxyethyl), -NH(hydroxypropyl), -NH(hydroxybutyl), -NH(hydroxypentyl); -(azolidinyl)-($CH_2$OH); -NH-cyclohexyl-($CH_2$OH); or -NH-($CH_2$)-(CH=CH)-($CH_2$OH).

In other embodiments, $R_1$ is: pentyl, -$CH_2$-(propylthio), -$NH_2$, -NH(pentyl), -NH(hydroxybutyl), -$CH_2$-NH(pentyl), -NH-CO-hydroxymethyl, -NH-CO-CH(OH)-(cyclopropyl), -(piperidinyl)-(hydroxymethyl), or -NH-(cyclohexane)-(hydroxymethyl).

In further embodiments, $R_1$ is pentyl, -$NH_2$, -NH(hydroxybutyl), -$CH_2$-NH(pentyl), -NH-CO-hydroxymethyl, -NH-CO-CH(OH)-(cyclopropyl), -(piperidinyl)-(hydroxymethyl), or -NH-(cyclohexyl)-(hydroxymethyl).

In yet other embodiments, $R_1$ is pentyl, -NH(hydroxybutyl), -NH-CO-CH(OH)-(cyclopropyl), -(piperidinyl)-(hydroxymethyl), or -NH-(cyclohexyl)-(hydroxymethyl).

In still further embodiments, $R_1$ is pentyl, -NH(hydroxybutyl), -(piperidinyl)-(hydroxymethyl), or -NH-(cyclohexyl)-(hydroxymethyl).

In some embodiments, $R_2$ is halogen, such as fluorine, chlorine, bromine, or iodine. In further embodiments, $R_2$ is fluorine.

In some embodiments, $R_3$ is $C_{1-6}$ alkyl, $C_{1-6}$ alkoxy, halogen, cyano, -$NH_2$, -NH-CO-($C_{1-6}$ hydroxyalkyl), or $NO_2$. Preferably, $R_3$ is $C_{1-6}$ alkyl, halogen, -$NH_2$, -NH-CO-($C_{1-6}$ hydroxyalkyl), or $NO_2$. In particular, $R_3$ is methyl, ethyl, propyl, butyl, fluorine, chlorine, bromine, -$NH_2$, -NH-CO-($CH_2$OH), or $NO_2$. More particularly, $R_3$ is methyl, bromine, -$NH_2$, -NH-CO-($CH_2$OH), or $NO_2$.

In some embodiments, $R_3$ is halogen, cyano, $NO_2$, $C_{1-6}$ alkyl, $C_{1-6}$ haloalkyl, $C_{1-6}$ hydroxyalkyl, $C_{1-6}$ alkoxy, or $NR_aR_b$, wherein $R_a$ and $R_b$ are each independently H, $C_{1-6}$ alkyl, -SO-($C_{1-6}$ alkyl), -$SO_2$-($C_{1-6}$ alkyl), or -$(CH_2)_q$-($C_{3-8}$ cycloalkyl), wherein the alkyl and the cycloalkyl are each optionally substituted with one or more substituents independently selected from the group consisting of halogen, CN, OH, SH and amino.

In some embodiments, $R_3$ is $C_{1-6}$ alkyl, $C_{1-6}$ alkoxy, halogen, cyano, -$NH_2$ or $NO_2$. In further embodiments, $R_3$ is $C_{1-6}$ alkyl, halogen, -$NH_2$ or $NO_2$. In particular, $R_3$ is methyl, ethyl, propyl, butyl, fluorine, chlorine, bromine, -$NH_2$ or $NO_2$. More particularly, $R_3$ is methyl, bromine, -$NH_2$ or $NO_2$.

In some embodiments, $R_3$ is $C_{1-6}$ alkyl or $NR_aR_b$, wherein $R_a$ and $R_b$ are each independently H or -CO-($C_{1-6}$ alkyl), wherein the alkyl is optionally substituted with one or more OH groups. Further, $R_3$ is $C_{1-6}$ alkyl, -$NH_2$ or -NH-CO-($CH_2$OH). Even further, $R_3$ is $C_{1-6}$ alkyl or -NH-CO-($CH_2$OH).

In some embodiments, $R_3$ is $C_{1-6}$ alkyl, such as methyl.

In some embodiments, $R_2$ and $R_3$, together with the atoms to which they are attached, form a 4-to 8-membered heterocyclic group containing N, O or S as a heteroatom, such as dioxolane, for example, in the compounds of Example 6 or 7.

In some embodiments, D is connected to $L_4$ via a nitrogen or oxygen atom in $R_1$ or $R_3$, or via the oxygen from the hydroxy shown in formula (II), for example, through an amide bond, an aminomethyl ether bond, or a carbamate bond.

In some embodiments, D is a small molecule drug moiety derived from a compound of formula (II) by removing an atom (e.g., a hydrogen atom) or a group of atoms, such as by removing a hydrogen atom attached to a nitrogen or oxygen atom in $R_1$ or $R_3$, or by removing a hydrogen atom from the hydroxy group shown in formula (II). In particular, D is a moiety selected from the group consisting of:

| No. | Structure | No. | Structure |
|---|---|---|---|
| 1' | 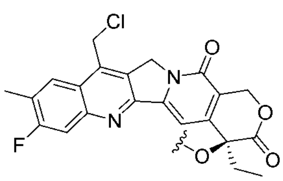 STI-A-4' | 2' | 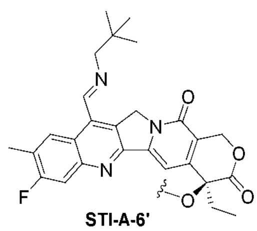 STI-A-6' |

(continued)

| No. | Structure | No. | Structure |
|---|---|---|---|
| 3’ | 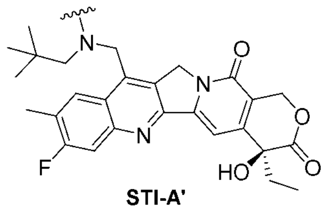 STI-A' | 4’ | 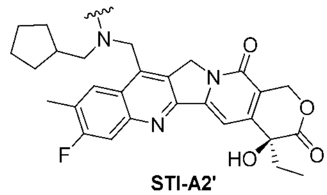 STI-A2' |
| 5’ | 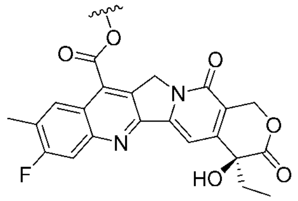 STI-F-01' | 6’ | 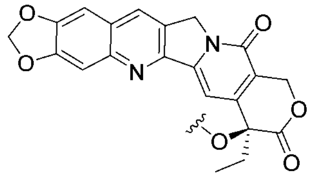 STI-E-05' |
| 7’ | 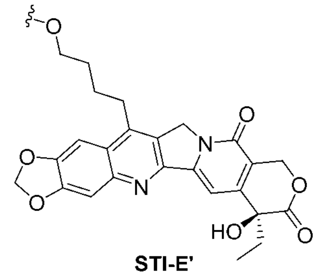 STI-E' | 8’ | 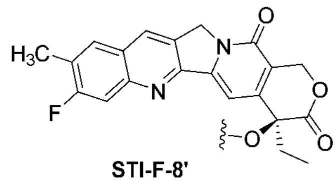 STI-F-8' |
| 9’ | 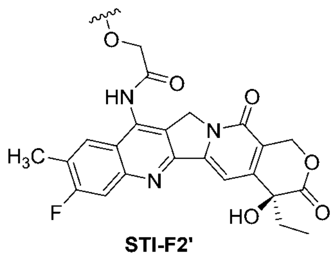 STI-F2' | 10’ | 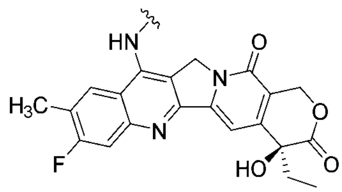 STI-F-03c-1' |
| 11’ | 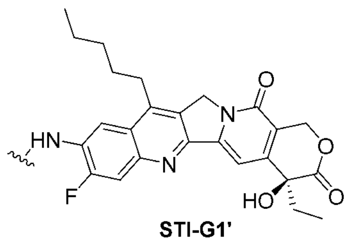 STI-G1' | 12’ | 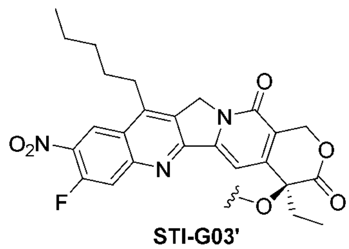 STI-G03' |
| 13’ | 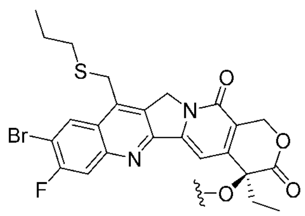 STI-G2-06c' | 14’ | 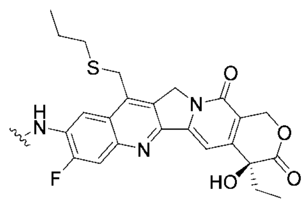 STI-G2' |

(continued)

| No. | Structure | No. | Structure |
|---|---|---|---|
| 15' | 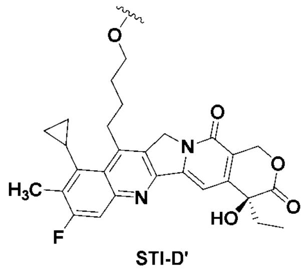 STI-D' | 16' | 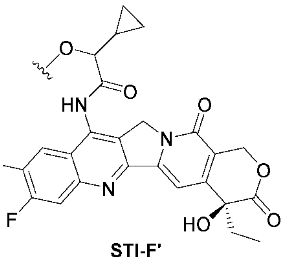 STI-F' |
| 17' | 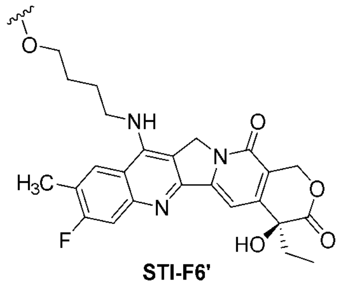 STI-F6' | 18' | 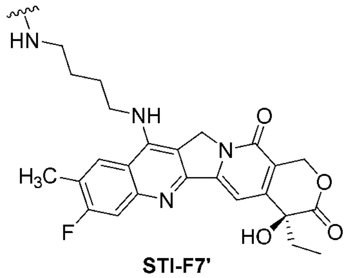 STI-F7' |
| 19' | 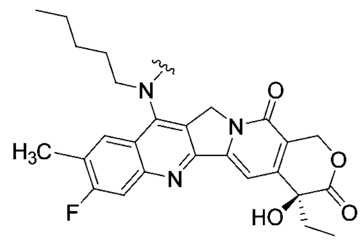 STI-F13' | 20' | 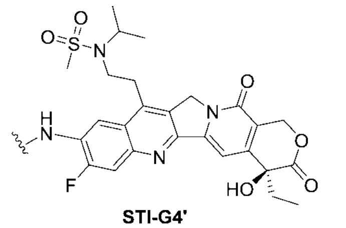 STI-G4' |
| 21' | 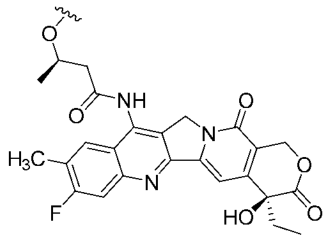 SIT-F12R' | 22' | 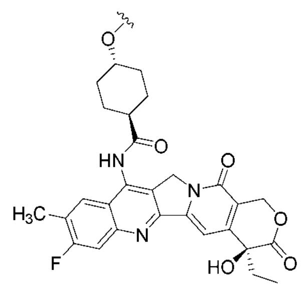 STI-F10' |
| P1' | 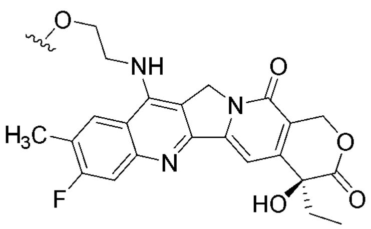 STI-F5' | P2' | 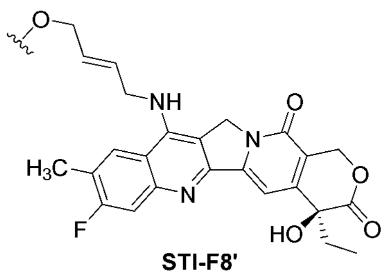 STI-F8' |

(continued)

| No. | Structure | No. | Structure |
|---|---|---|---|
| P3' | 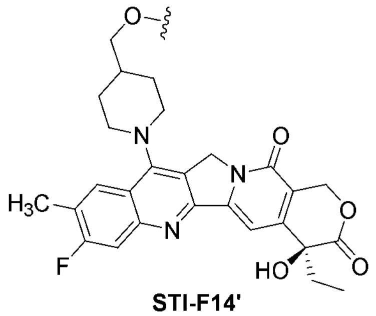 STI-F14' | P4' | 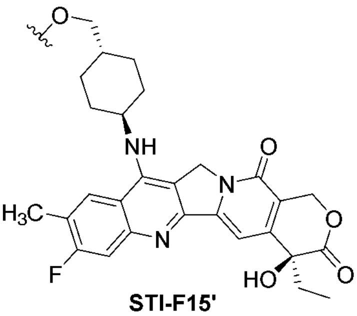 STI-F15' |
| P5' | 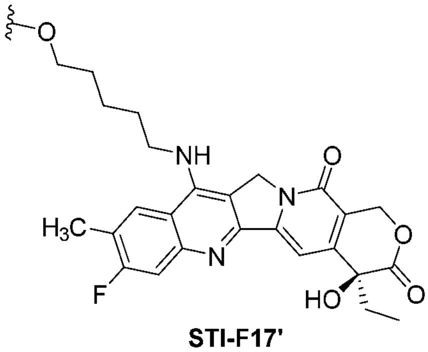 STI-F17' | P6' | 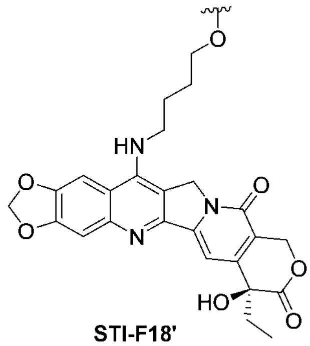 STI-F18' |
| P7' | 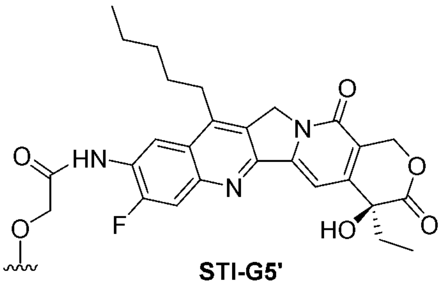 STI-G5' | | |

In some embodiments, $L_1$ is each independently:

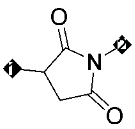 , 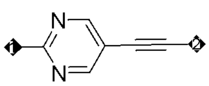 , 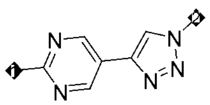 , 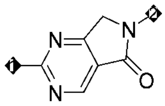 , or 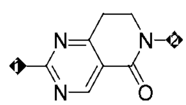 ,

preferably

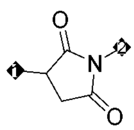 or 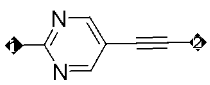 ,

more preferably

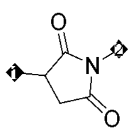 ,

wherein position 1 is connected to Ab, and position 2 is connected to $L_2$.

In some embodiments, $L_2$ is each independently a linking unit selected from the group consisting of: $-(CH_2)_t-(L_M)_{0-1}-(CH_2CH_2O)_s-(CH_2)_t-CO-$, $-(CH_2)_t-L_M-(CH_2)_t-(L_M)_{0-1}-L_N-L_M-(CH_2O)_s-(CH_2)_t-CO-$, $-(CH_2)_t-L_M-(CH_2)_t-N(R_p)-(CH_2)_t-N(R_p)-(CH_2)_t-CO-$, and $(CH_2)_t-L_M-CH(R_p)-CO-$, wherein the -CO-terminus of $L_2$ is connected to $L_3$, and the other terminus of $L_2$ is connected to $L_1$.

In some embodiments, $L_2$ is each independently $-(CR_mR_n)_t-(L_M)_{0-1}-(CH_2CH_2O)_s-(CR_mR_n)_t-CO-$, such as $-(CR_mR_n)_t-(CH_2CH_2O)_s-(CR_mR_n)_t-CO-$ or $-(CR_mR_n)_t-L_M-(CH_2CH_2O)_s-(CR_mR_n)_t-CO-$, wherein the -CO- terminus of $L_2$ is connected to $L_3$, and the other terminus of $L_2$ is connected to $L_1$. In further embodiments, $L_2$ is each independently $-(CH_2)_t-(L_M)_{0-1}-(CH_2CH_2O)_s-(CH_2)_t-CO-$, such as $-(CH_2)_t-(CH_2CH_2O)_s-(CH_2)_t-CO-$ or $-(CH_2)_t-L_M-(CH_2CH_2O)_s-(CH_2)_t-CO-$, wherein the -CO-terminus of $L_2$ is connected to $L_3$, and the other terminus of $L_2$ is connected to $L_1$. In further embodiments, $L_2$ is each independently $-(CH_2)_t-(CH_2CH_2O)_s-(CH_2)_t-CO-$, $-(CH_2)_t-CO-NH-(CH_2CH_2O)_s-(CH_2)_t-CO-$, or $-(CH_2)_t-NH-CO-(CH_2CH_2O)_s-(CH_2)_t-CO-$, preferably $-(CH_2)_t-(CH_2CH_2O)_s-(CH_2)_t-CO-$, or $-(CH_2)_t-CO-NH-(CH_2CH_2O)_s-(CH_2)_t-CO-$, wherein the -CO-terminus of $L_2$ is connected to $L_3$, and the other terminus of $L_2$ is connected to $L_1$.

In some embodiments, $L_2$ is each independently $-(CR_mR_n)_t-L_M-(CR_mR_n)_t-(L_M)_{0-1}-L_N-L_M-(CH_2O)_s-(CR_mR_n)_t-CO-$, such as $-(CR_mR_n)_t-L_M-(CR_mR_n)_t-L_N-L_M-(CH_2O)_s-(CR_mR_n)_t-CO-$, or $-(CR_mR_n)_t-L_M-(CR_mR_n)_t-L_M-L_N-L_M-(CH_2O)_s-(CR_mR_n)_t-CO-$, wherein the -CO- terminus of $L_2$ is connected to $L_3$, and the other terminus of $L_2$ is connected to $L_1$. In further embodiments, $L_2$ is each independently $-(CH_2)_t-L_M-(CH_2)_t-L_N-L_M-(CH_2O)_s-(CH_2)_t-CO-$, or $-(CH_2)_t-L_M-(CH_2)_t-L_M-L_N-L_M-(CH_2O)_s-(CH_2)_t-CO-$, wherein the -CO- terminus of $L_2$ is connected to $L_3$, and the other terminus of $L_2$ is connected to $L_1$. In further embodiments, $L_2$ is each independently $-(CH_2)_t-CO-NH-(CH_2)_t-$(3- to 8-membered heteroarylene)$-(CH_2)_t-(OCH_2CH_2)_m-NH-CO-(CH_2O)-(CH_2)_t-CO-$(preferably $-(CH_2)_t-CO-NH-(CH_2)_t-$(triazolylene)$-(CH_2)_t-(OCH_2CH_2)_m-NH-CO-(CH_2O)-(CH_2)_t-CO-$), $-(CH_2)_t-CO-NH-(CH_2)_t-NH-CO-(CH_2CH_2O)_m-(CH_2)_t-NH-CO-(CH_2O)-(CH_2)_t-CO-$, or $-(CH_2)_t-CO-NH-(CH_2)_t-NH-CO-(CH_2)_t-(OCH_2CH_2)_m-NH-CO-(CH_2O)-(CH_2)_t-CO-$, wherein the -CO- terminus of $L_2$ is connected to $L_3$, and the other terminus of $L_2$ is connected to $L_1$.

In some embodiments, $L_2$ is each independently $-(CR_mR_n)_t-L_M-(CR_mR_n)_t-N(R_p)-(CR_mR_n)_t-N(R_p)-(CR_mR_n)_t-CO-$, such as $-(CH_2)_t-L_M-(CH_2)_t-N(R_p)-(CH_2)_t-N(R_p)-(CH_2)_t-CO-$, wherein the -CO- terminus of $L_2$ is connected to $L_3$, and the other terminus of $L_2$ is connected to $L_1$. In further embodiments, $L_2$ is each independently $-(CH_2)_t-NH-CO-(CH_2)_t-N(R_p)-(CH_2)_t-N(R_p)-(CH_2)_t-CO-$, or $-(CH_2)_t-CO-NH-(CH_2)_t-N(R_p)-(CH_2)_t-N(R_p)-(CH_2)_t-CO-$, preferably $-(CH_2)_t-NH-CO-(CH_2)_t-N(R_p)-(CH_2)_t-N(R_p)-(CH_2)_t-CO-$, wherein the -CO- terminus of $L_2$ is connected to $L_3$, and the other terminus of $L_2$ is connected to $L_1$. In further embodiments, each $R_p$ is independently $-CO-(CH_2CH_2O)_m-CH_3$, $-(CH_2)_t-NH-CO-(CH_2CH_2O)_m-CH_3$, or $-(CH_2)_t-CO-NH-(CH_2CH_2O)_m-CH_3$, preferably $-CO-(CH_2CH_2O)_m-CH_3$.

In some embodiments, $L_2$ is each independently $-(CR_mR_n)_t-L_m-CH(R_p)-CO-$, such as $-(CH_2)_t-L_M-CH(R_p)-CO-$, and further such as $-(CH_2)_t-CO-NH-CH(R_p)-CO-$ or $-(CH_2)_t-NH-CO-CH(R_p)-CO-$, wherein the -CO- terminus of $L_2$ is connected to $L_3$, and the other terminus of $L_2$ is connected to $L_1$. In further embodiments, each $R_p$ is independently $-CO-(CH_2CH_2O)_m-CH_3$, $-(CH_2)_t-NH-CO-(CH_2CH_2O)_m-CH_3$, or $-(CH_2)_t-CO-NH-(CH_2CH_2O)_m-CH_3$, preferably $-(CH_2)_t-NH-CO-(CH_2CH_2O)_m-CH_3$.

Preferably, $L_2$ is each independently: $-(CH_2)_t-(CH_2CH_2O)_s-(CH_2)_t-CO-$, $-(CH_2)_t-CO-NH-(CH_2CH_2O),-(CH_2)_t-CO-$, $-(CH_2)_t-NH-CO-(CH_2CH_2O)_s-(CH_2)_t-CO-$, $-(CH_2)_t-CO-NH-(CH_2)_t-$(3- to 8-membered heteroarylene)$-(CH_2)_t-(OCH_2CH_2)_m-NH-CO-(CH_2O)-(CH_2)_t-CO-$, $-(CH_2)_t-CO-NH-(CH_2)_t-NH-CO-(CH_2CH_2O)_m-(CH_2)_t-NH-CO-(CH_2O)-(CH_2)_t-CO-$, $-(CH_2)_t-CO-NH-(CH_2)_t-NH-CO-(CH_2)_t-(OCH_2CH_2)_m-NH-CO-(CH_2O)-(CH_2)_t-CO-$, $-(CH_2)_t-NH-CO-(CH_2)_t-N(R_p)-(CH_2)_t-N(R_p)-(CH_2)_t-CO-$, $-(CH_2)_t-CO-NH-(CH_2)_t-N(R_p)-(CH_2)_t-N(R_p)-(CH_2)_t-CO-$, or $-(CH_2)_t-CO-NH-CH(R_p)-CO-$ or $-(CH_2)_t-NH-CO-CH(R_p)-CO-$, wherein the -CO- terminus of $L_2$ is connected to $L_3$, and the other terminus of $L_2$ is connected to $L_1$.

It is understood that the structural units of $L_M$ (e.g., -NH-CO- or -CO-NH-) are arranged in the structural formula of $L_2$ from left to right. Similarly, the structural units of $L_N$ are also arranged in the structural formula of $L_2$ from left to right.

In some embodiments, each s is independently 0, 1, 2, 3, 4, 5, 6, 7, or 8. In further embodiments, each s is independently 0. In some embodiments, each s is independently 3. In other embodiments, each s is independently 8.

In some embodiments, each t is independently 0, 1, 2, 3, 4, 5, 6, 7, or 8. In further embodiments, each s is independently 0, 1, 2, 3, 4, 5 or 6, preferably each is independently 0, 1, 2, 3, 4, or 5.

In some embodiments, each m is independently 3, 4, 5, 6, 7 or 8. In further embodiments, each m is independently 3. In other embodiments, each m is independently 8.

In some embodiments, $L_2$ is each independently a linking unit of the formula $-(CH_2CH_2O)_x-(CH_2)_y-CO-$, wherein the -CO- terminus is connected to $L_3$ and the other terminus is connected to $L_1$, and wherein x is an integer from 0 to 5, y is an integer from 1 to 5, and $x + y \leq 6$; preferably, $L_2$ is $-(CH_2)-CO-$, $-(CH_2)_2-CO-$, $-(CH_2)_3-CO-$, $-(CH_2)_4-CO-$, $-(CH_2)_5-CO-$, $-(CH_2)_6-CO-$, $-(CH_2CH_2O)-(CH_2)-CO-$, $-(CH_2CH_2O)-(CH_2)_2-CO-$, $-(CH_2CH_2O)-(CH_2)_3-CO-$, $-(CH_2CH_2O)-(CH_2)_4-CO-$, $-(CH_2CH_2O)-(CH_2)_5-CO-$, $-(CH_2CH_2O)_2-(CH_2)-CO-$, $-(CH_2CH_2O)_2-(CH_2)_2-CO-$, $-(CH_2CH_2O)_2-(CH_2)_3-CO-$,

$-(CH_2CH_2O)_2-(CH_2)_4-CO-$, $-(CH_2CH_2O)_3-(CH_2)-CO-$, $-(CH_2CH_2O)_3-(CH_2)_2-CO-$, $-(CH_2CH_2O)_3-(CH_2)_3-CO-$. More preferably, $L_2$ is each independently $-(CH_2)_5-CO-$ or $-(CH_2CH_2O)_3-(CH_2)_2-CO-$.

In other embodiments, $L_2$ is each independently:

- ♦ $-(CH_2)_5-CO-$;
- ♦ $-(CH_2CH_2O)_3-(CH_2)_2-CO-$;
- ♦ $-(CH_2)_5-CO-NH-(CH_2CH_2O)_8-(CH_2)_2-CO-$;
- ♦ $-(CH_2)_3-CO-NH-(CH_2)-$(triazolylene)$-(CH_2)_2-(OCH_2CH_2)_8-NH-CO-(CH_2)-O-(CH_2)-CO-$;
- ♦ $-(CH_2)_3-CO-NH-(CH_2)_2-NH-CO-(CH_2CH_2O)_8-(CH_2)_2-NH-CO-(CH_2)-O-(CH_2)-CO-$;
- ♦ $-(CH_2)_3-CO-NH-(CH_2)_2-NH-CO-(CH_2)_2-(OCH_2CH_2)_8-NH-CO-(CH_2)-O-(CH_2)-CO-$;

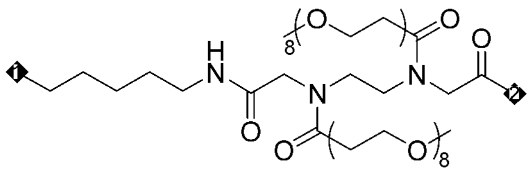

;

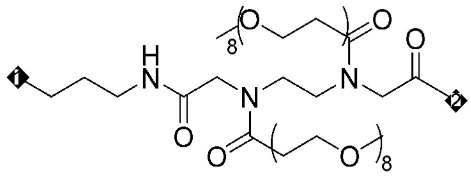

;

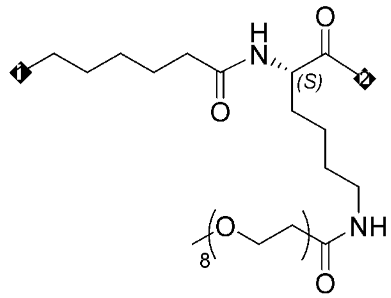

;

wherein the -CO- terminus of $L_2$ is connected to $L_3$ and the other terminus of $L_2$ is connected to $L_1$, or wherein position 1 is connected to $L_1$ and position 2 is connected to $L_3$.

In some embodiments, $L_3$ is each independently an amino acid residue, a short peptide chain consisting of 2-6 amino acid residues, or $-NH-(CH_2)_p-CO-$, wherein p is 0, 1, 2, 3, 4, 5, or 6. In particular, the amino acid is a natural or unnatural amino acid, preferably selected from the group consisting of glycine (Gly), alanine (Ala), valine (Val), phenylalanine (Phe), citrulline (Cit), lysine (Lys) and asparagine (Asn). In some embodiments, $L_3$ is Gly-Gly-Phe-Gly, Val-Cit, Val-Ala, Phe-Lys, Val-Lys, Lys, Ala-Ala-Ala, Ala-Ala-Asn, or $-NH-(CH_2)_2-CO-$. In particular, $L_3$ is Gly-Gly-Phe-Gly, Val-Cit, Val-Ala, Phe-Lys, Val-Lys, Ala-Ala-Ala, Ala-Ala-Asn, or $-NH-(CH_2)_2-CO-$. In some embodiments, $L_3$ is Gly-Gly-Phe-Gly, Val-Cit, Val-Ala, Phe-Lys, Lys, or $-NH-(CH_2)_2-CO-$. In particular, $L_3$ is Gly-Gly-Phe-Gly, Val-Ala, Phe-Lys, or Lys.

In some embodiments, $L_4$ is each independently a bond,

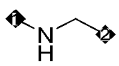

,

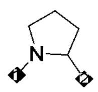

,

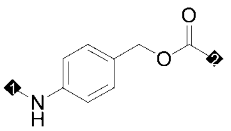, 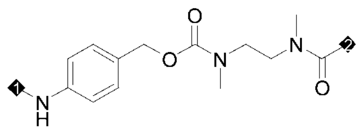 and 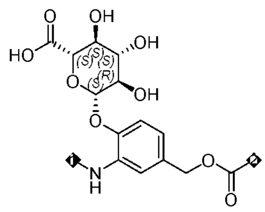,

wherein position 1 is connected to $L_3$ and position 2 is connected to D.

In further embodiments, $L_4$ is each independently a bond,

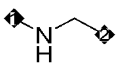, 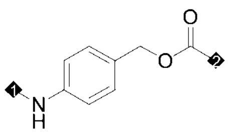

or

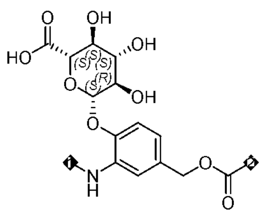,

wherein position 1 is connected to $L_3$ and position 2 is connected to D.

In some embodiments, $L_4$ is each independently

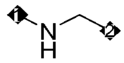,

wherein position 1 is connected to $L_3$ and position 2 is connected to D.

In some embodiments, the $-L_1-L_2-L_3-L_4-$ moiety is each independently selected from:

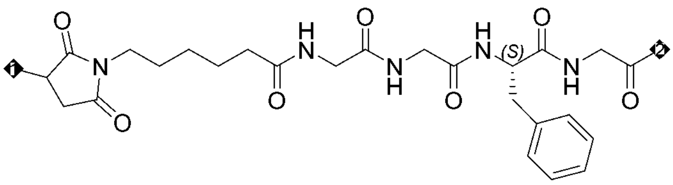 (MC-GGFG-);

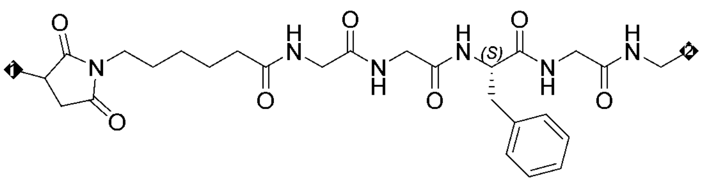;

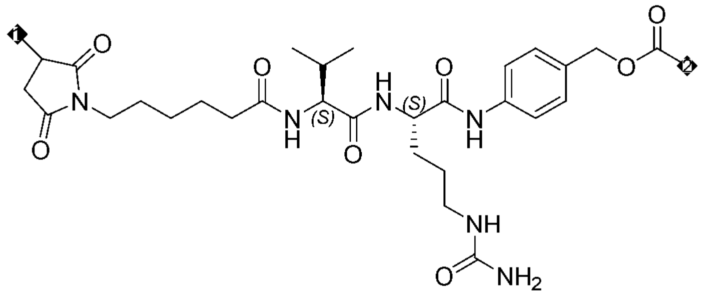

(MC-VC-PAB-);

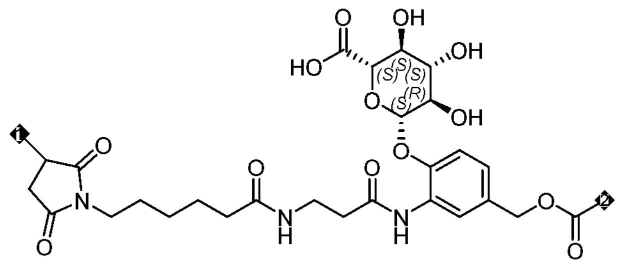

;

and

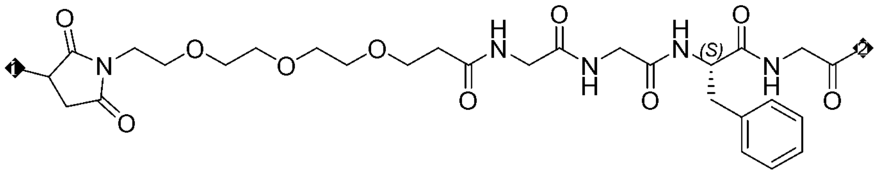

(MC-PEG3-GGFG);

wherein position 1 is connected to Ab, and position 2 is connected to D.

In some embodiments, the -$L_1$-$L_2$-$L_3$-$L_4$- moiety is each independently selected from:

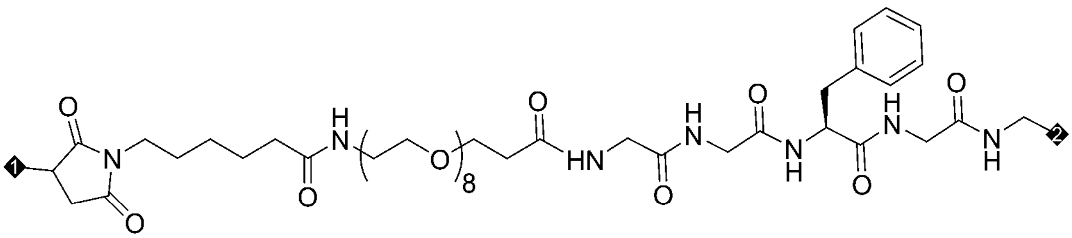

,

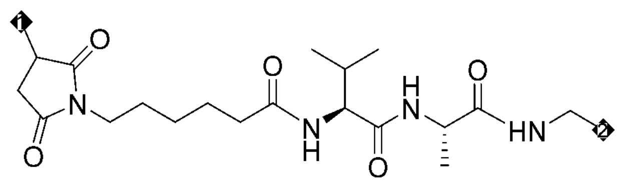

,

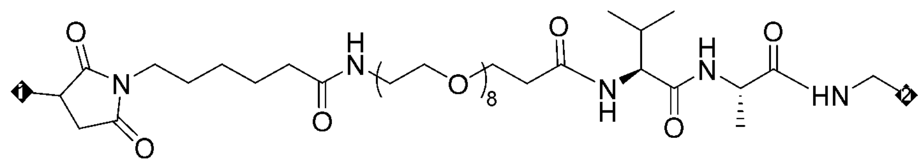

,

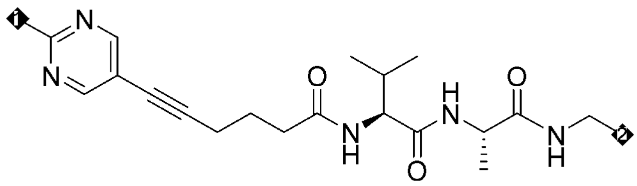

,

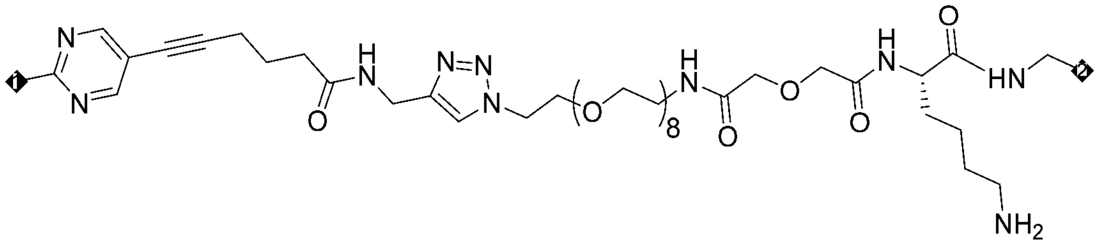

,

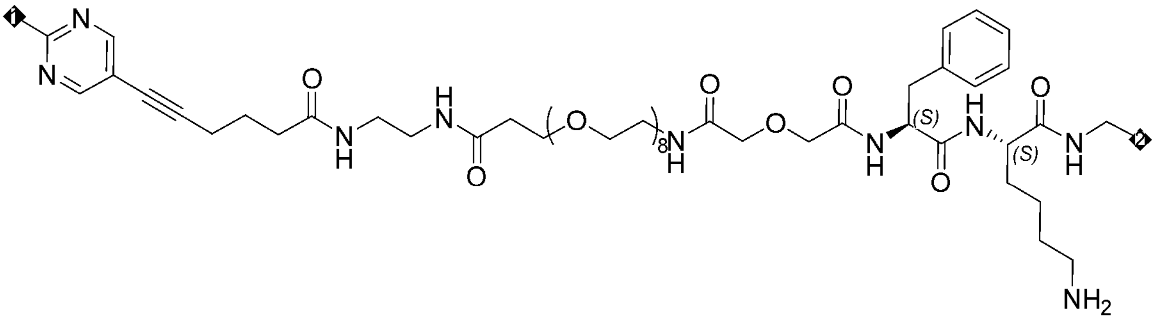

,

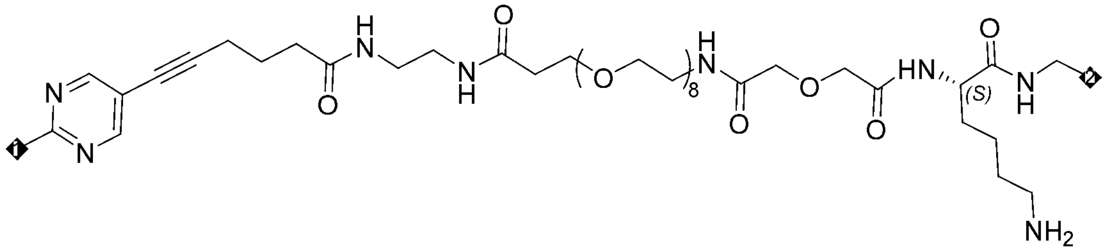

,

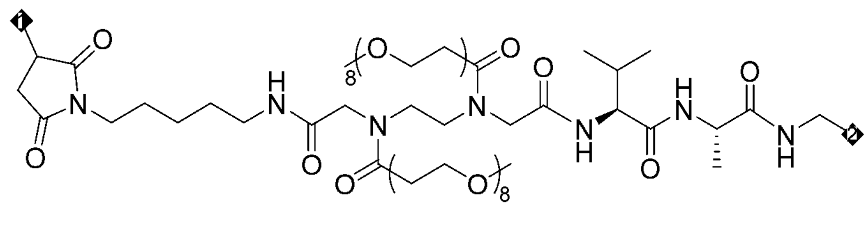

,

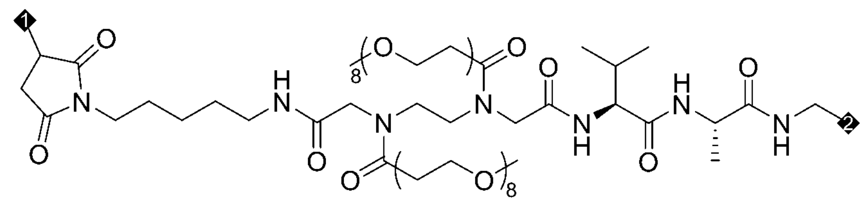

,

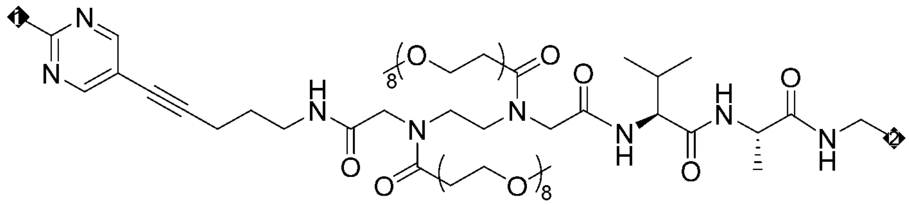

,

and

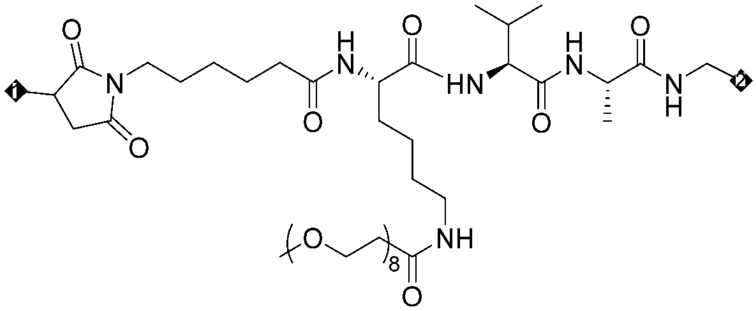

,

wherein position 1 is connected to Ab and position 2 is connected to D.

In some embodiments, the -$L_1$-$L_2$-$L_3$-$L_4$-D moiety is each independently selected from:

| No. | Structure |
|---|---|
| 23' | 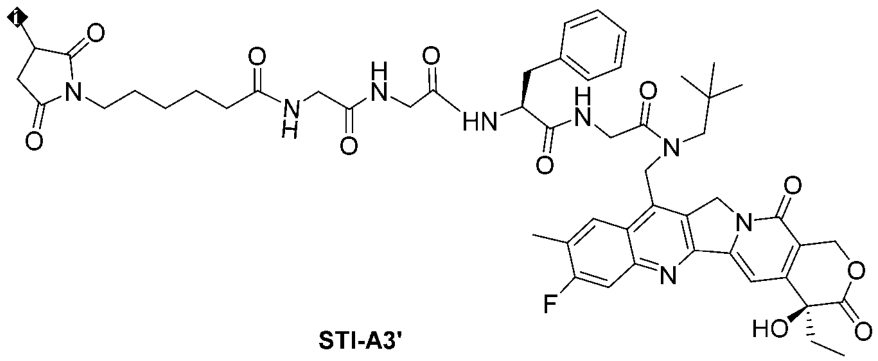 **STI-A3'** |
| 24' | 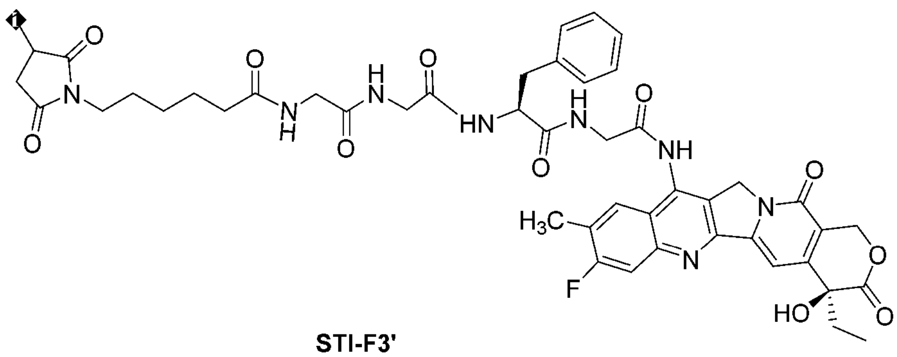 **STI-F3'** |

(continued)

| No. | Structure |
|---|---|
| 25' | 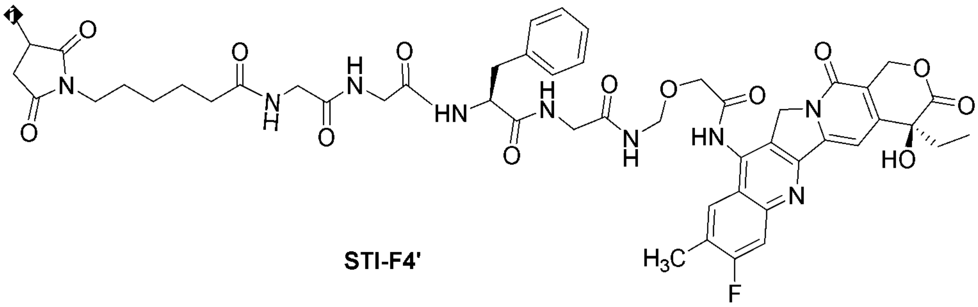 STI-F4' |
| 26' | 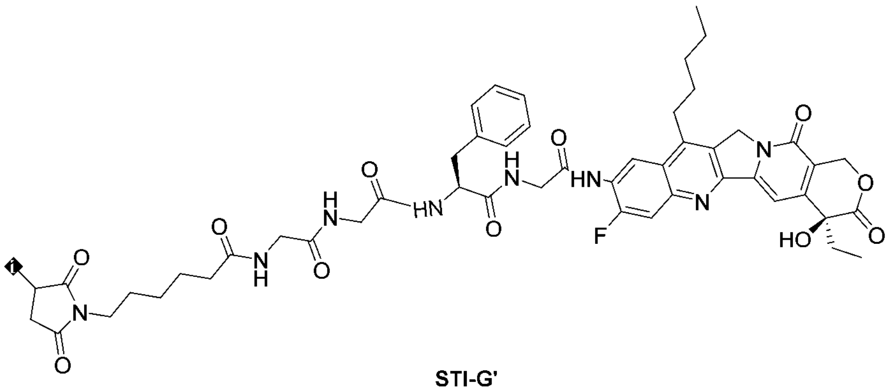 STI-G' |
| 27' | 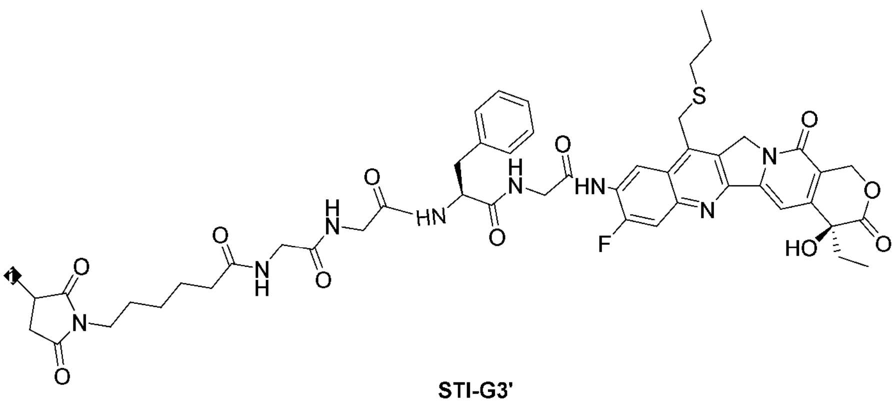 STI-G3' |
| 28' | 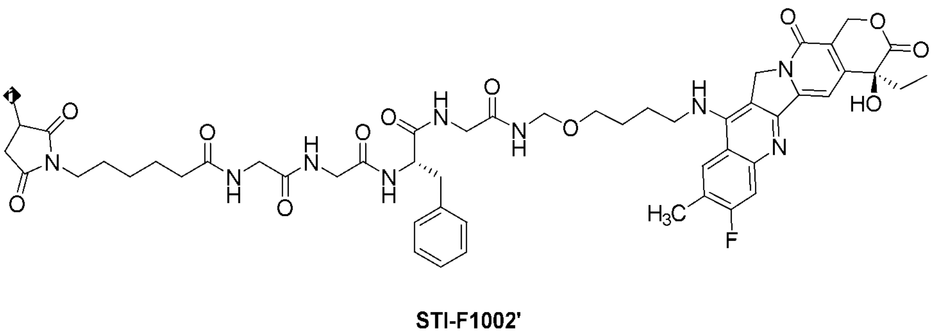 STI-F1002' |

(continued)

| No. | Structure |
|---|---|
| 29' | 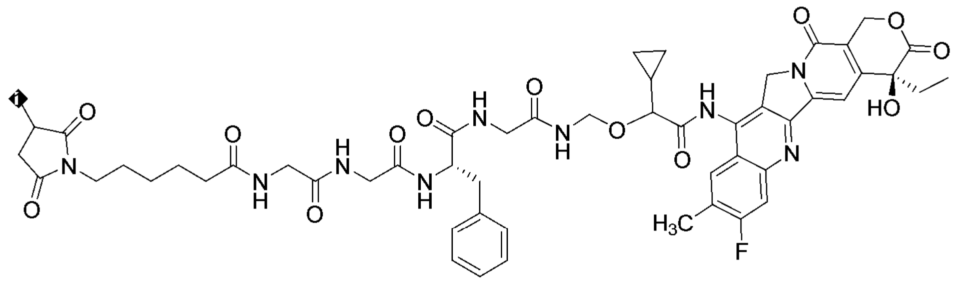 STI-F1001' |
| 30' | 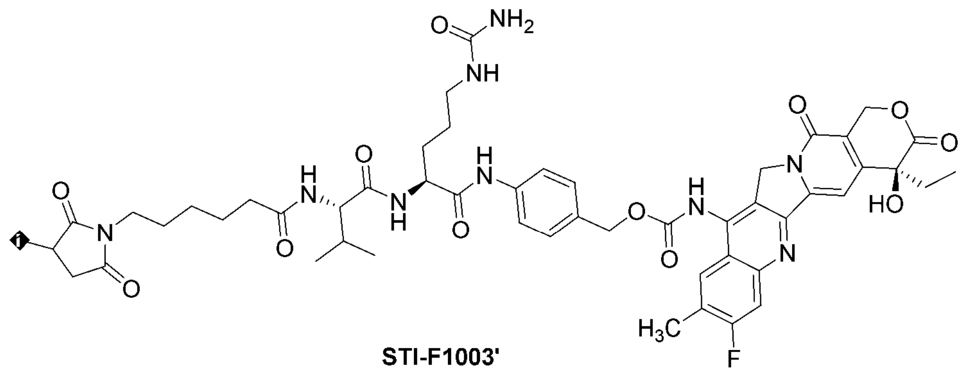 STI-F1003' |
| 31' | 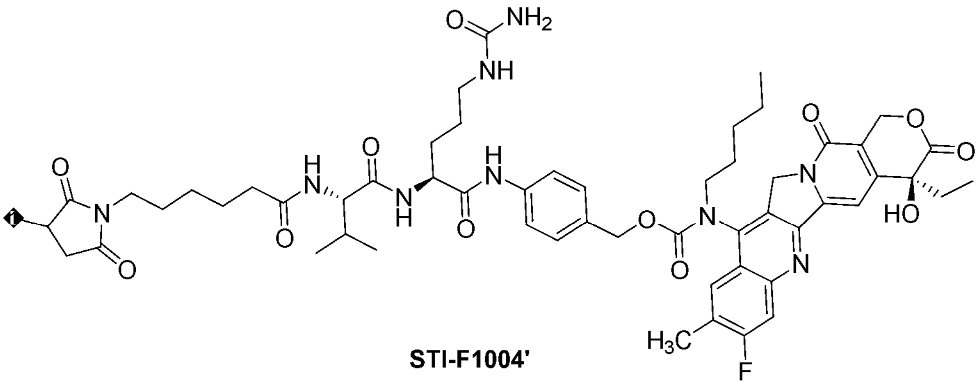 STI-F1004' |
| 32' | 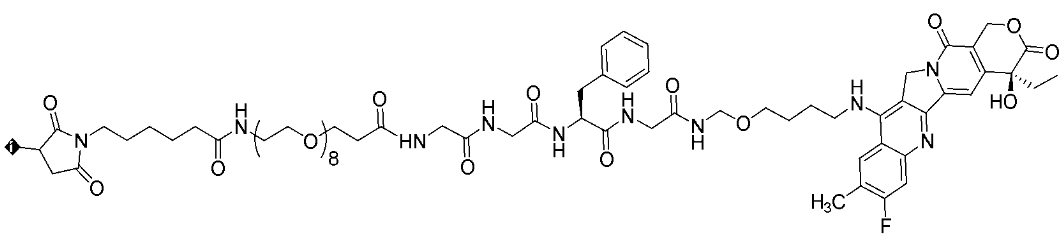 STI-F1006 |

(continued)

| No. | Structure |
|---|---|
| 33' | 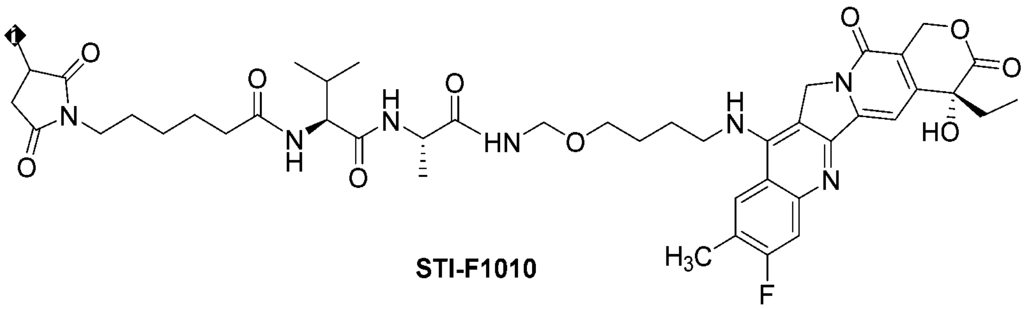 STI-F1010 |
| 34' | 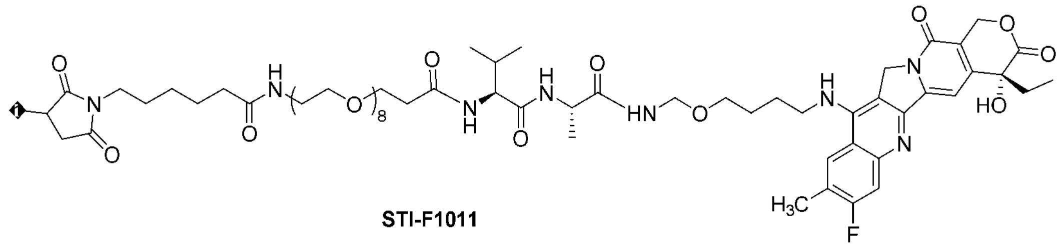 STI-F1011 |
| 35' | 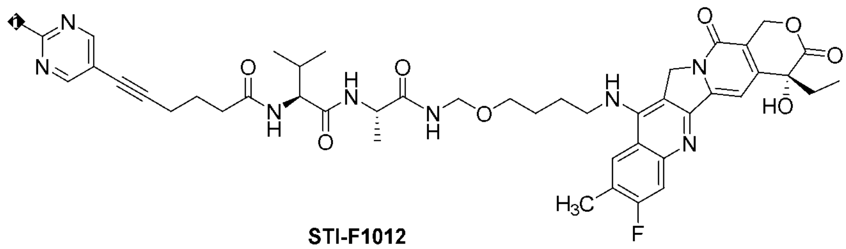 STI-F1012 |
| 36' | 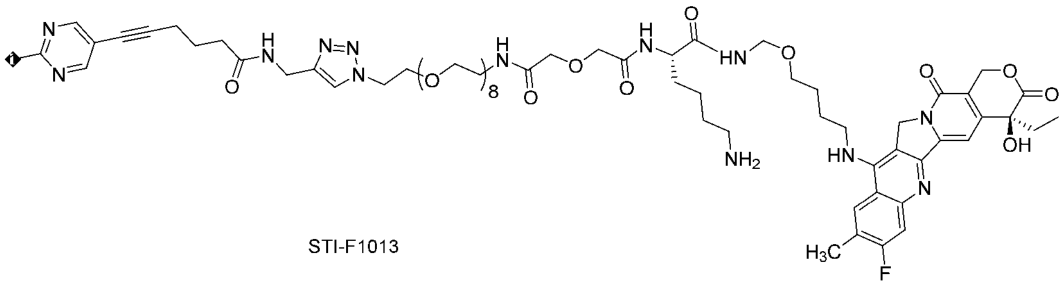 STI-F1013 |
| 37' | 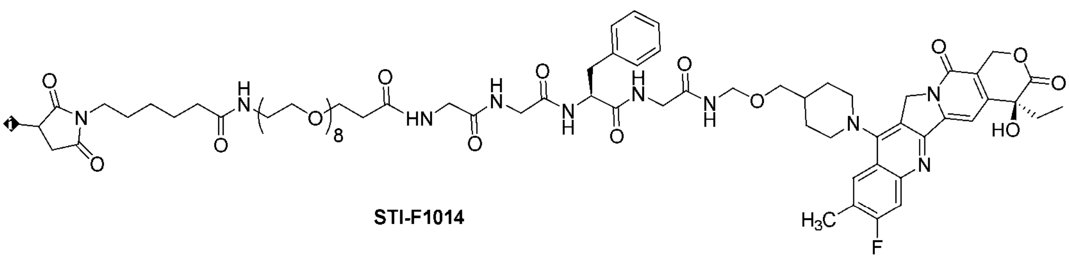 STI-F1014 |
| 38' | 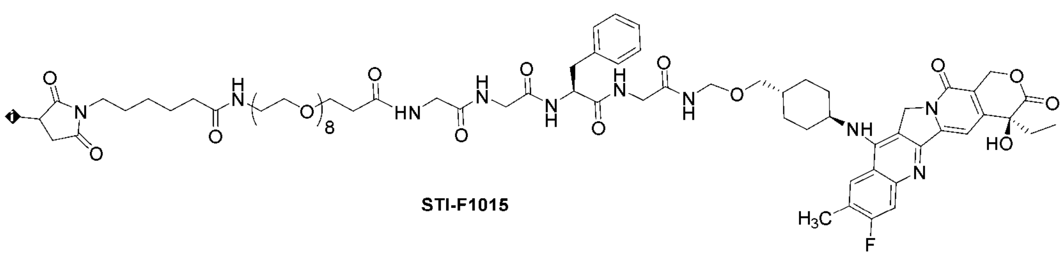 STI-F1015 |

(continued)

| No. | Structure |
|---|---|
| 39' | 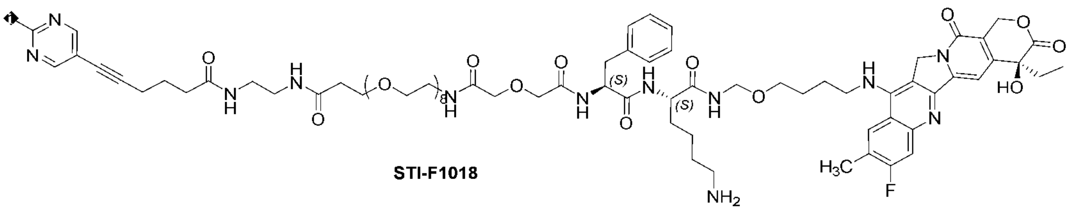 STI-F1018 |
| 40' | 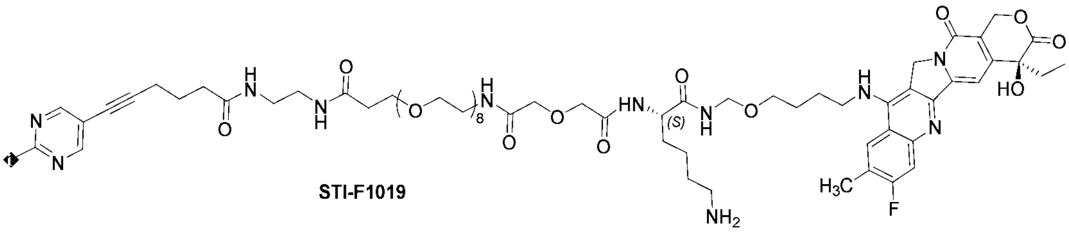 STI-F1019 |
| 41' | 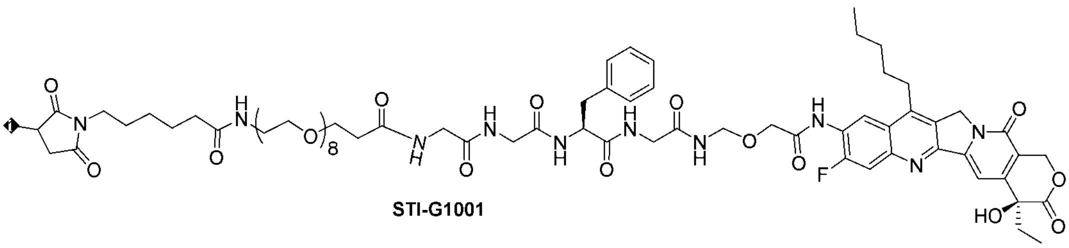 STI-G1001 |
| 42' | 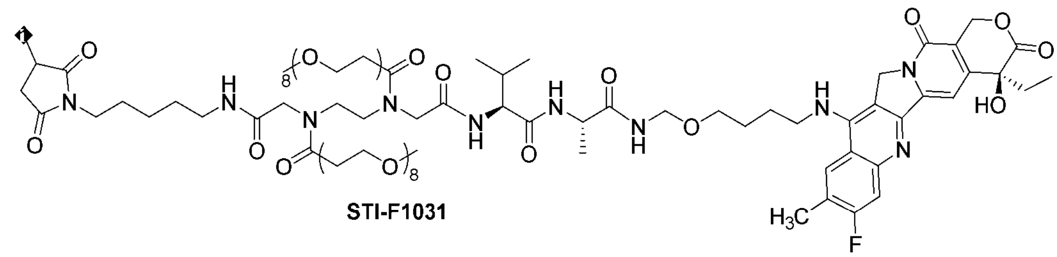 STI-F1031 |
| 43' | 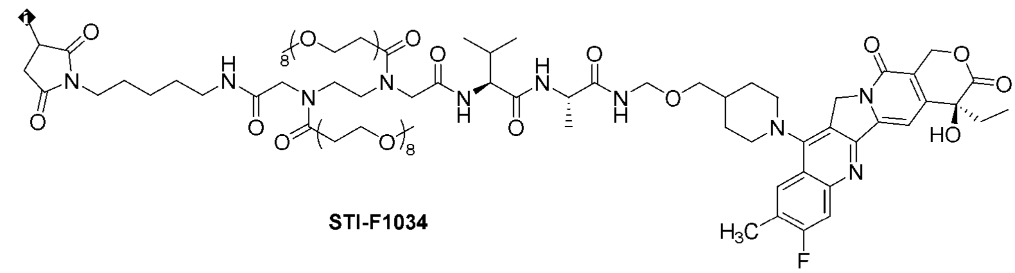 STI-F1034 |
| 44' | 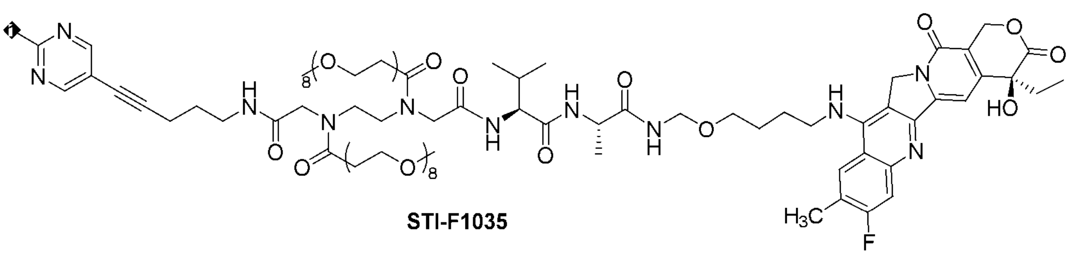 STI-F1035 |

(continued)

| No. | Structure |
|---|---|
| 45' | 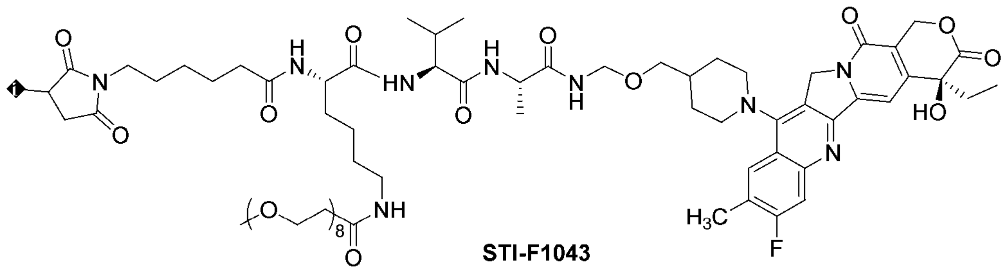 STI-F1043 |

wherein position 1 represents the point of attachment to Ab.

In some embodiments, Ab is an antibody or an antigen-binding fragment thereof that binds to a tumor cell surface antigen, e.g., an anti-Her2 antibody or an antigen-binding fragment thereof and/or an anti-FRα antibody or an antigen-binding fragment thereof. In some embodiments, Ab is farletuzumab or an antigen-binding fragment thereof and/or trastuzumab or an antigen-binding fragment thereof.

In some embodiments, when $L_1$ is

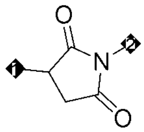

,

Lg and $L_1$ together form

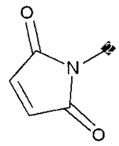

.

In other embodiments, when $L_1$ is not

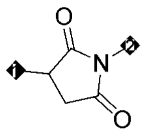

,

Lg is a leaving group. The leaving group is readily cleavable upon reaction with an antibody or antigen-binding fragment thereof. In some embodiments, Lg is halogen, sulfonyl (e.g. methanesulfonyl, p-toluenesulfonyl), sulfonyloxy (e.g. methanesulfonyloxy, $CF_3SO_3$-, p-toluenesulfonyloxy), a tertiary-ammonium group (e.g. $Me_3N^+$ or $Et_3N^+$), or a diazonium group. Preferably, Lg is F, Cl, Br, or methanesulfonyl. More preferably, Lg is methanesulfonyl.

In some embodiments, the antibody-drug conjugate of formula (I) in the present invention is selected from the group consisting of:

| name | Structure |
|---|---|
| Fa-ADC1 | 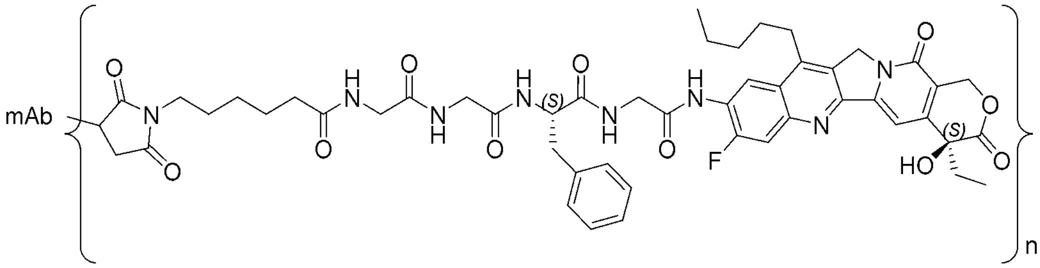 |
| Fa-ADC2 | 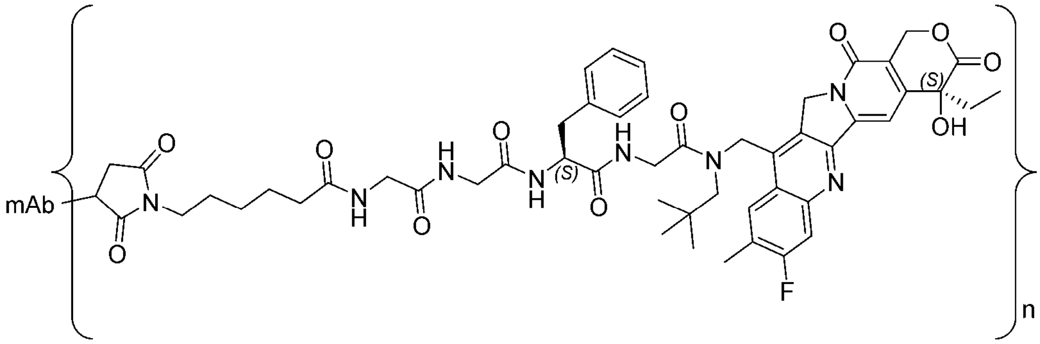 |
| Fa-ADC3 | 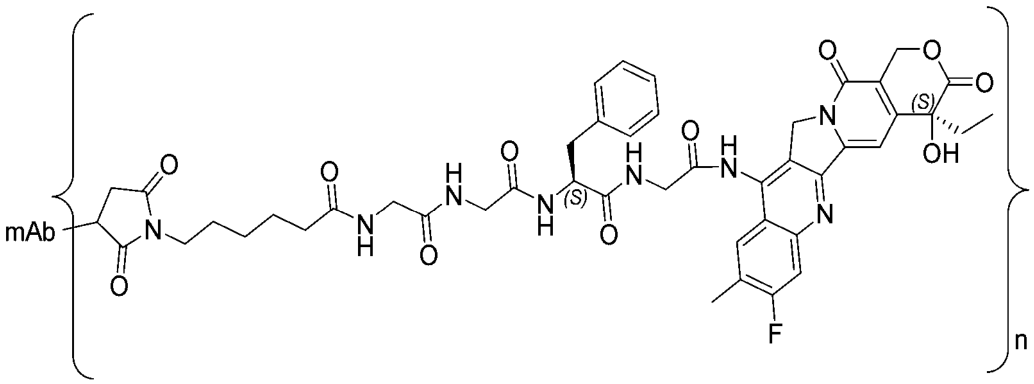 |
| Fa-ADC4 | 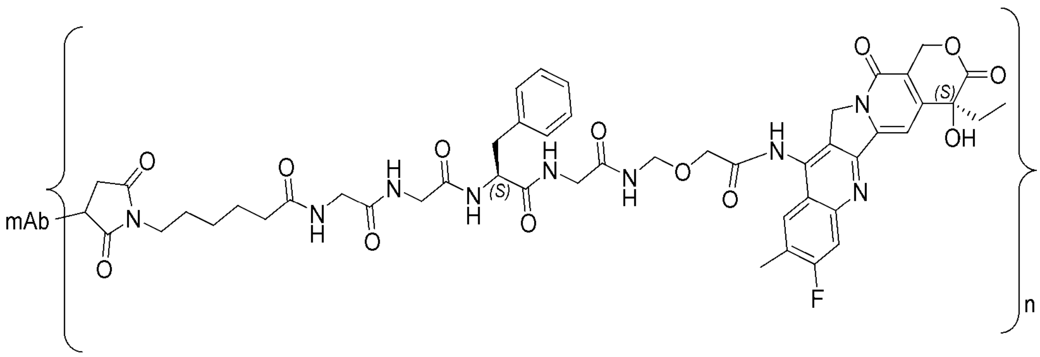 |

(continued)

| name | Structure |
|---|---|
| Fa-ADC5 | 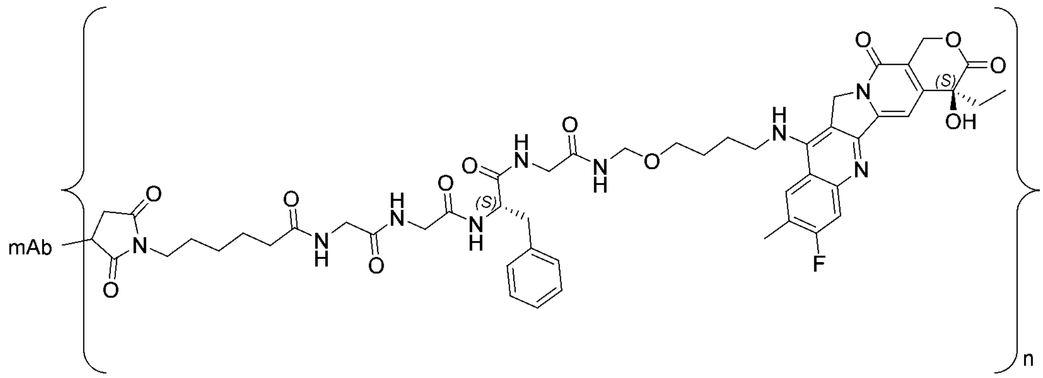 |
| Fa-ADC6 | 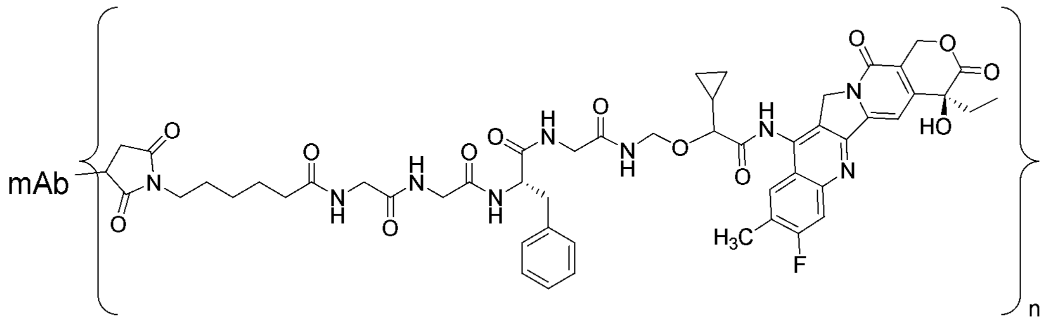 |
| Tra-ADC7 | 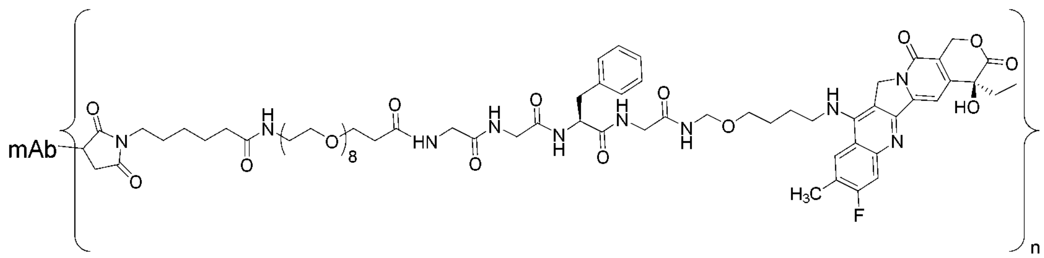 |
| Tra-ADC8 | 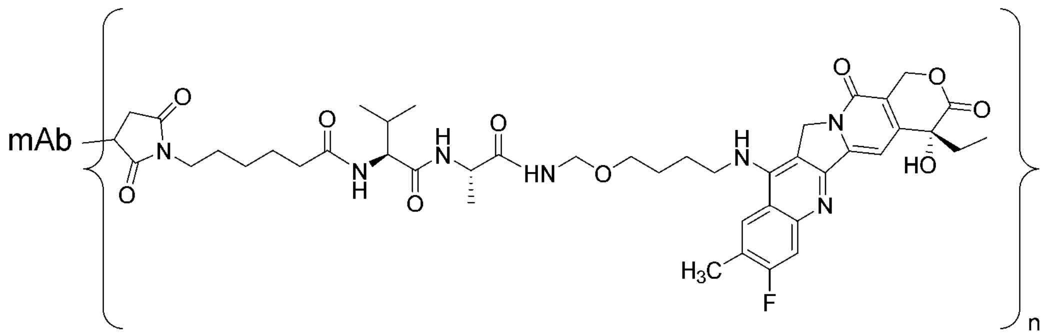 |
| Tra-ADC9 | 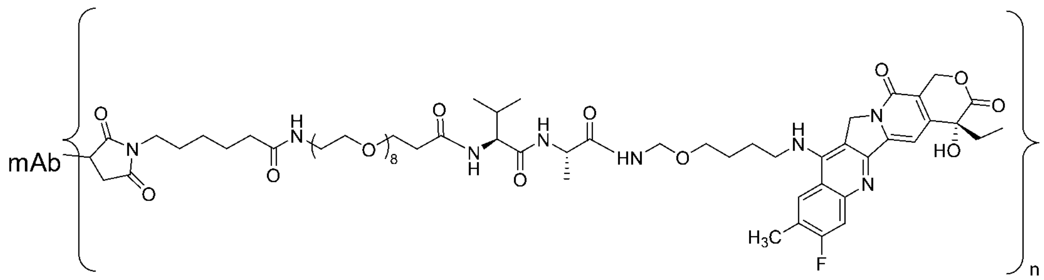 |

(continued)

| name | Structure |
| --- | --- |
| Tra-ADC10 | 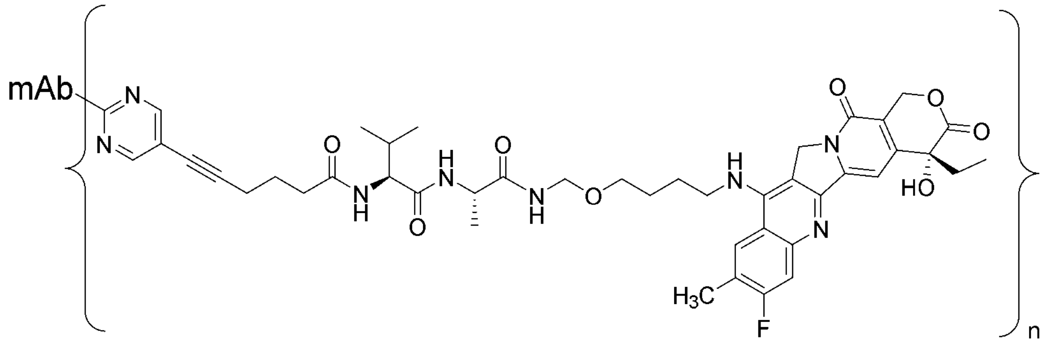 |
| Tra-ADC 11 | 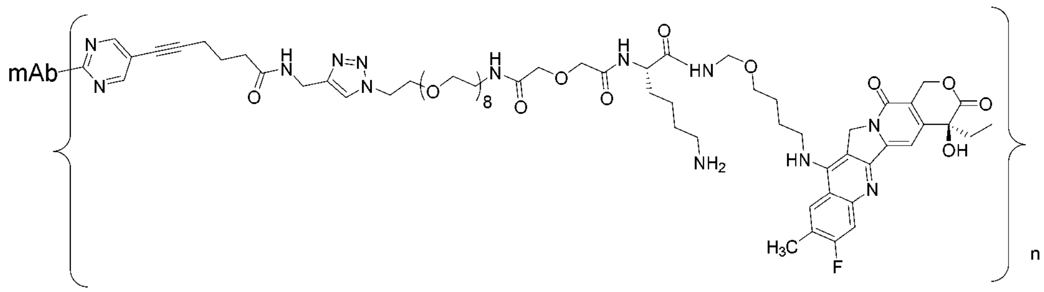 |
| Tra-ADC12 | 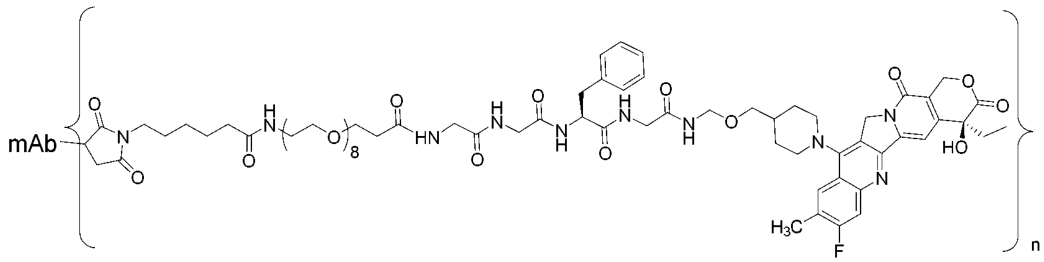 |
| Tra-ADC13 | 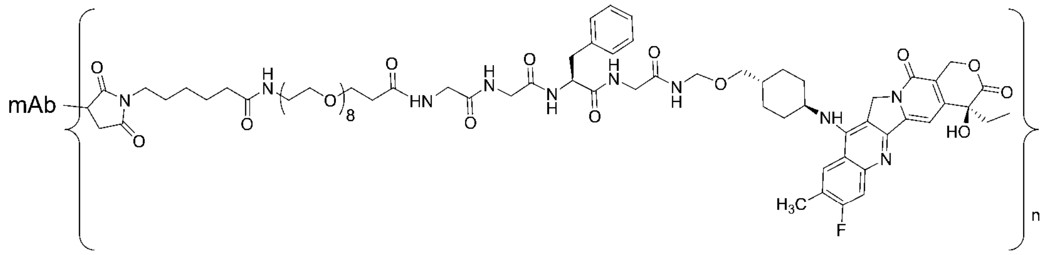 |
| Tra-ADC14 | 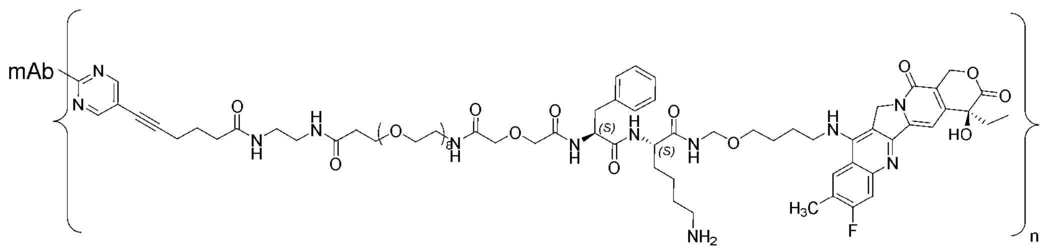 |

(continued)

| name | Structure |
|---|---|
| Tra-ADC15 | 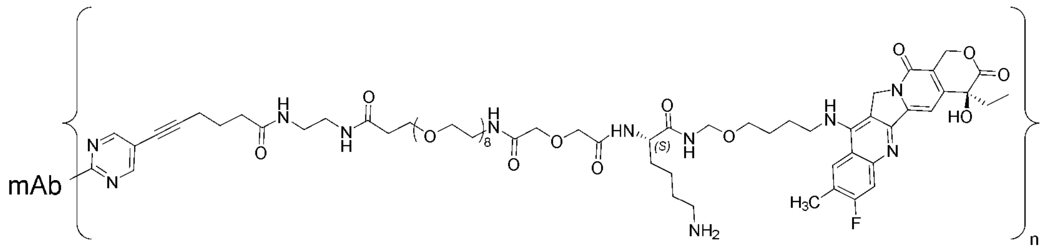 |
| Tra-ADC16 | 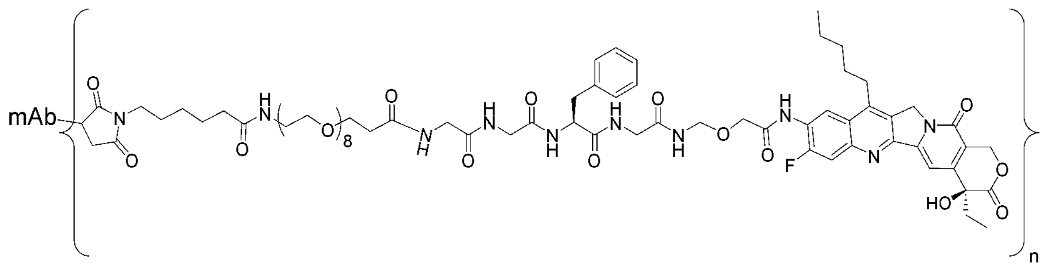 |
| Tra-ADC17 | 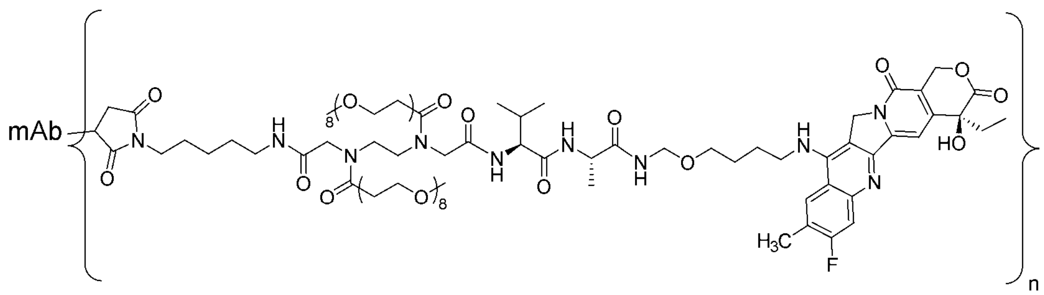 |
| Tra-ADC18 | 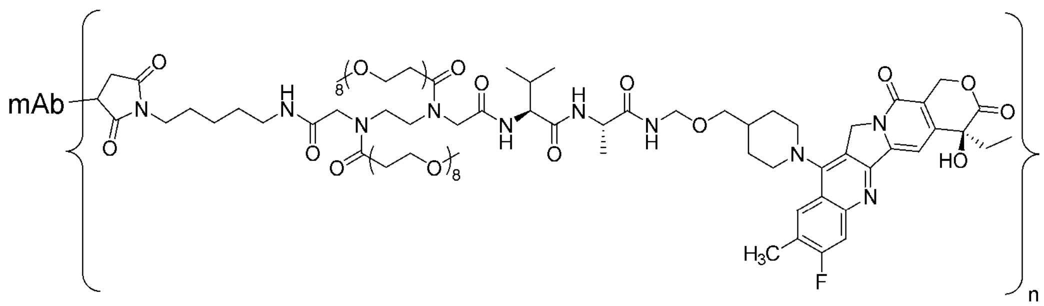 |
| Tra-ADC19 | 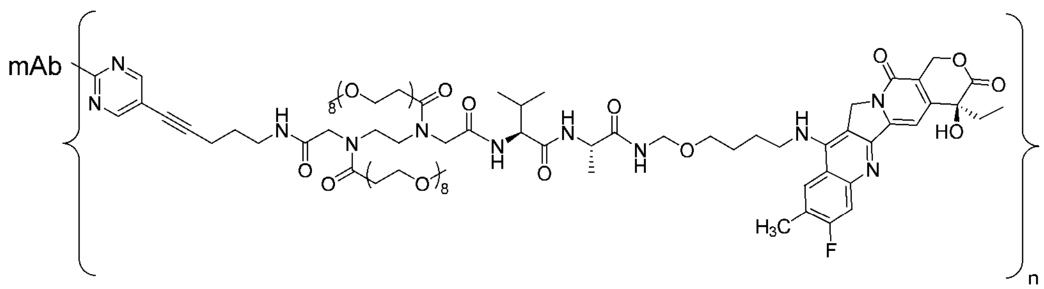 |

(continued)

| name | Structure |
|---|---|
| Tra-ADC20 | 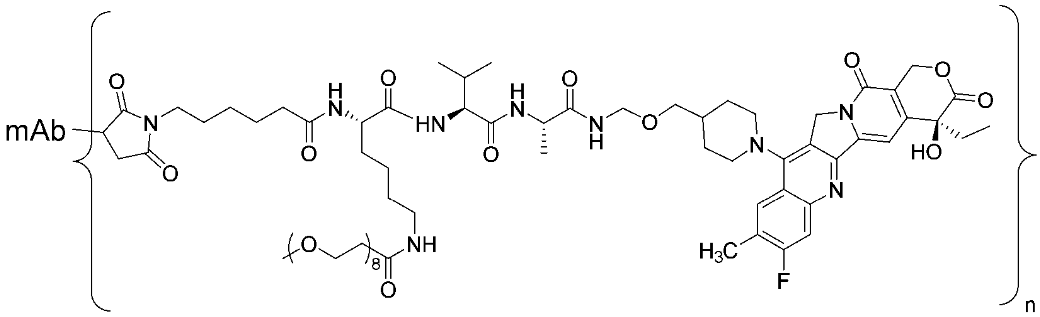 |

wherein, in Fa-ADCs, mAb represents farletuzumab (Fa); in Tra-ADCs, mAb represents trastuzumab (Tra); and n is an integer from 0 to 8, e.g., 0, 1, 2, 3, 4, 5, 6, 7, or 8.

In some embodiments, the antibody-drug conjugate of the present invention has an average DAR selected from any value within the range from 1.0 to 10.0, preferably has an average DAR selected from any value within the range from 2.0 to 8.0, and more preferably has an average DAR selected from any value within the range from 6.0 to 8.0.

In some embodiments, the compound of formula (II) in the present invention is selected from the group consisting of:

| No. | Structure | No. | Structure |
|---|---|---|---|
| 1 | 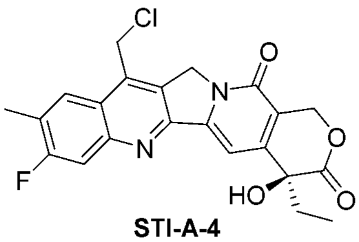 **STI-A-4** | 2 | 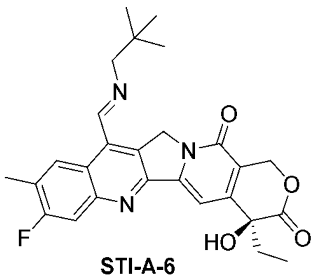 **STI-A-6** |
| 3 | 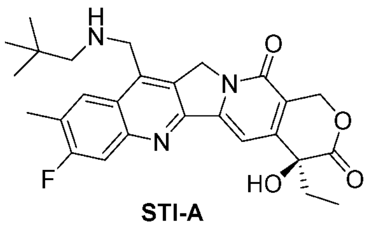 **STI-A** | 4 | 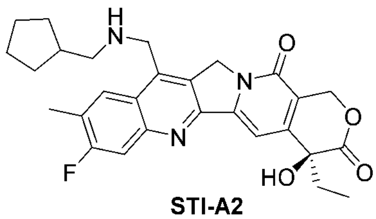 **STI-A2** |
| 5 | 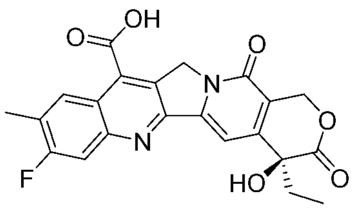 **STI-F-01** | 6 | 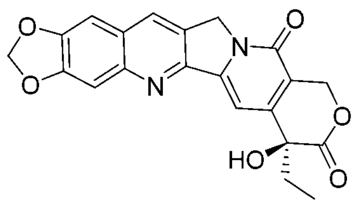 **STI-E05** |

(continued)

| No. | Structure | No. | Structure |
|---|---|---|---|
| 7 | 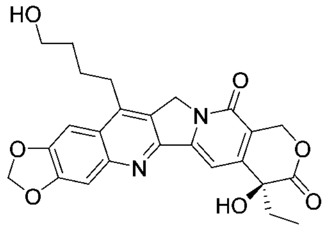 STI-E | 8 | 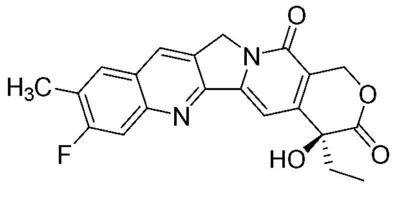 STI-F-8 |
| 9 | 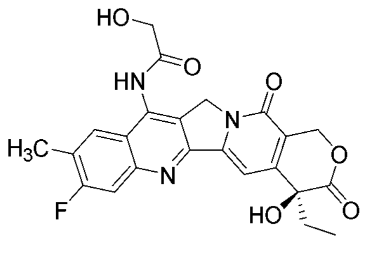 STI-F2 | 10 | 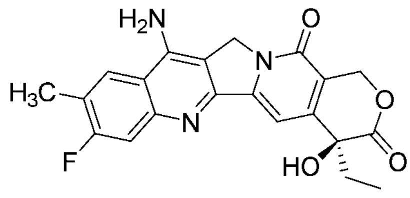 STI-F-03c-1 |
| 11 | 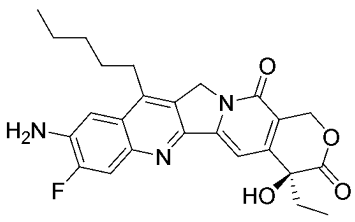 STI-G1 | 12 | 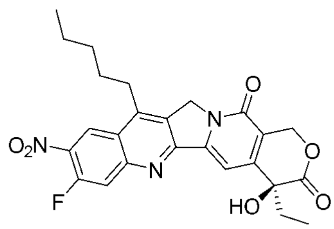 STI-G03 |
| 13 | 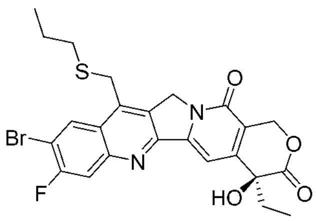 STI-G2-06c | 14 | 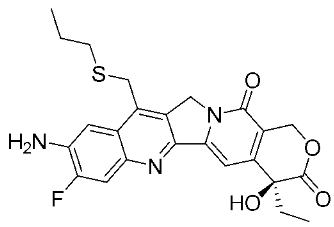 STI-G2 |
| 15 | 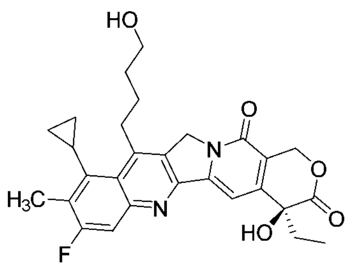 STI-D | 16 | 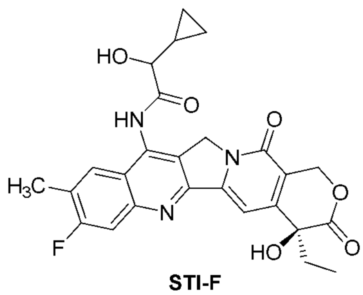 STI-F |

(continued)

| No. | Structure | No. | Structure |
|---|---|---|---|
| 17 | 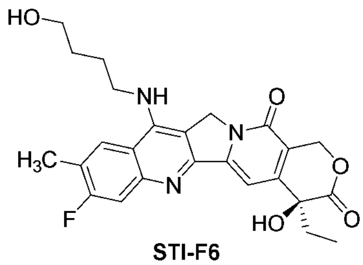 STI-F6 | 18 | 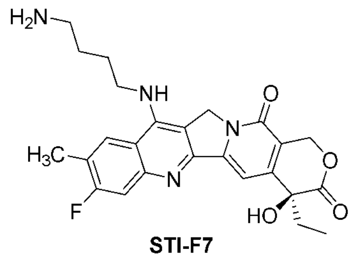 STI-F7 |
| 19 | 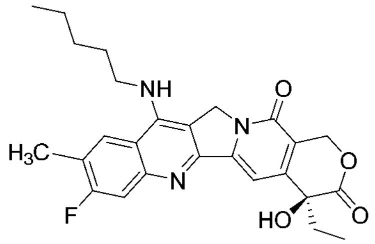 STI-F13 | 20 | 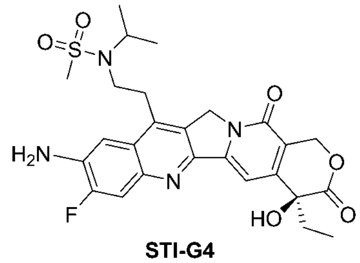 STI-G4 |
| 21 | 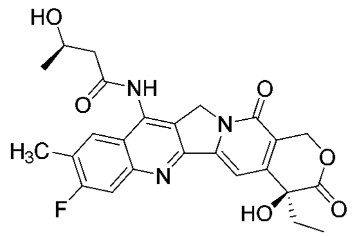 STI-F12R | 22 | 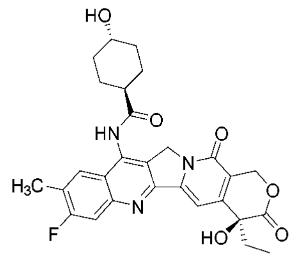 STI-F10 |
| P1 | 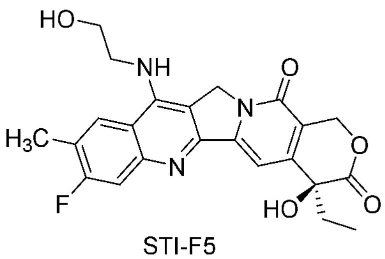 STI-F5 | P2 | 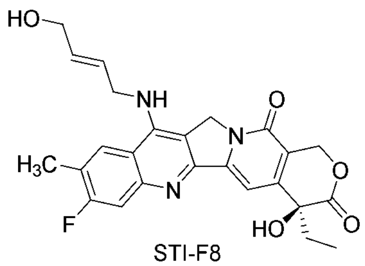 STI-F8 |
| P3 | 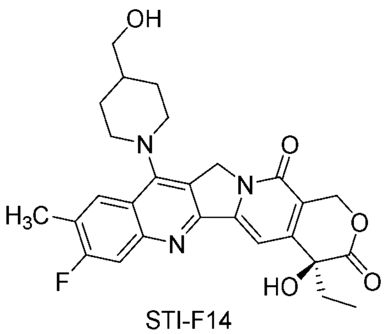 STI-F14 | P4 | 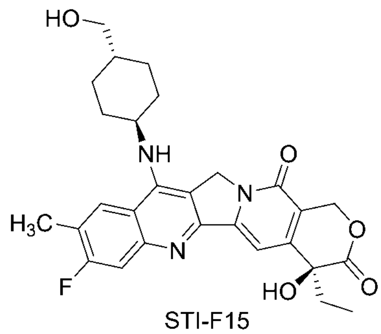 STI-F15 |

(continued)

| No. | Structure | No. | Structure |
|---|---|---|---|
| P5 | 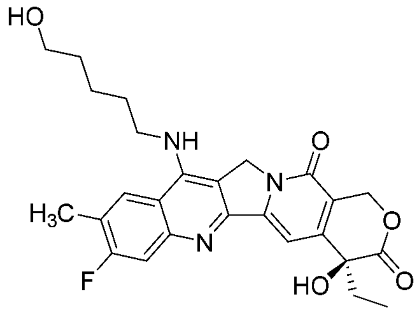 STI-F17 | P6 | 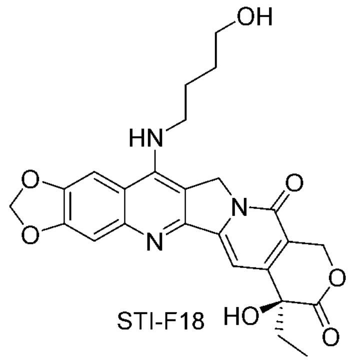 STI-F18 |
| P7 | 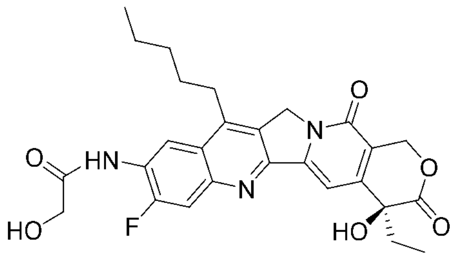 STI-G5 | | |

In some embodiments, the linker-payload conjugate of formula (III) in the present invention is selected from the group consisting of:

| No. | Structure |
|---|---|
| 23 | 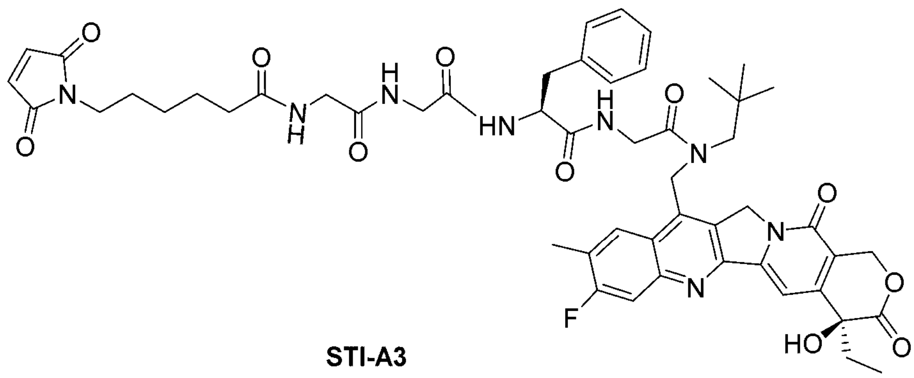 **STI-A3** |
| 24 | 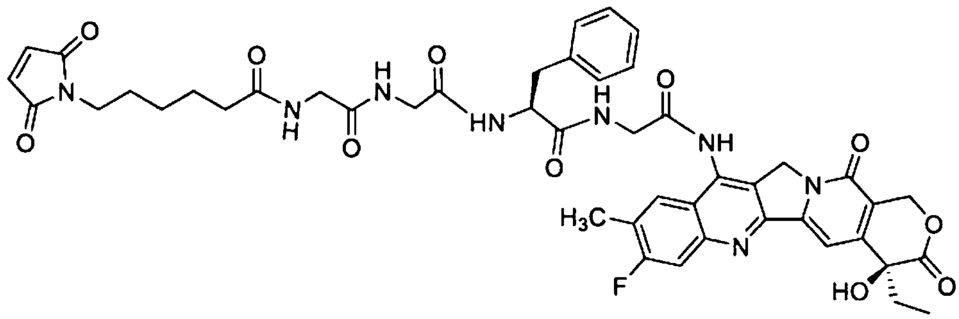 **STI-F3** |

(continued)

| No. | Structure |
|---|---|
| 25 | 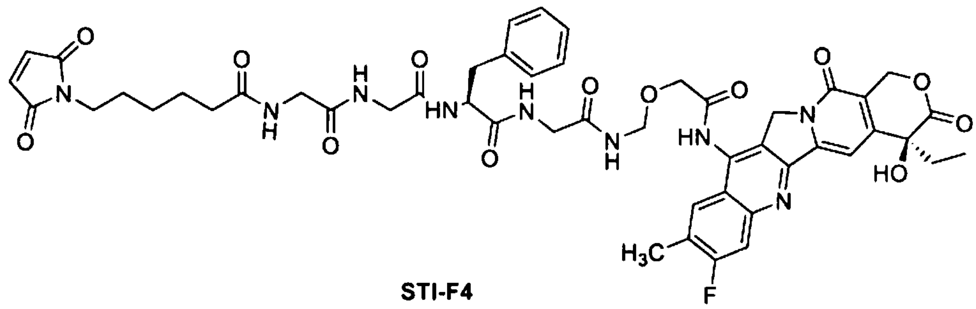 **STI-F4** |
| 26 | 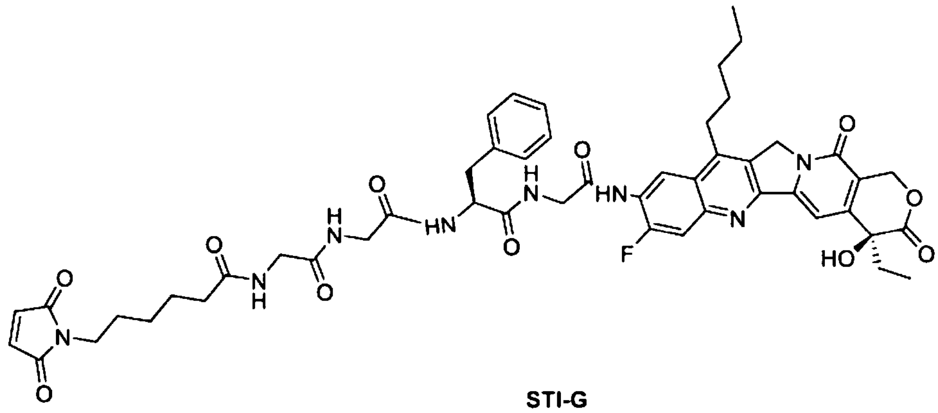 **STI-G** |
| 27 | 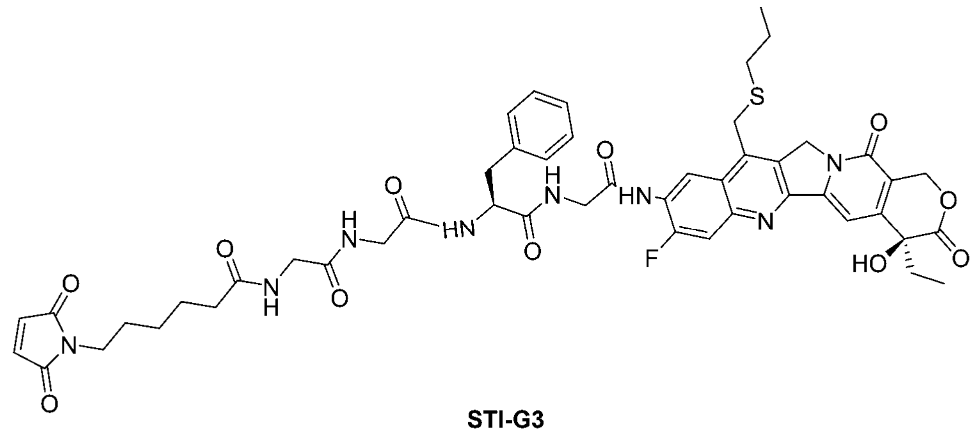 **STI-G3** |
| 28 | 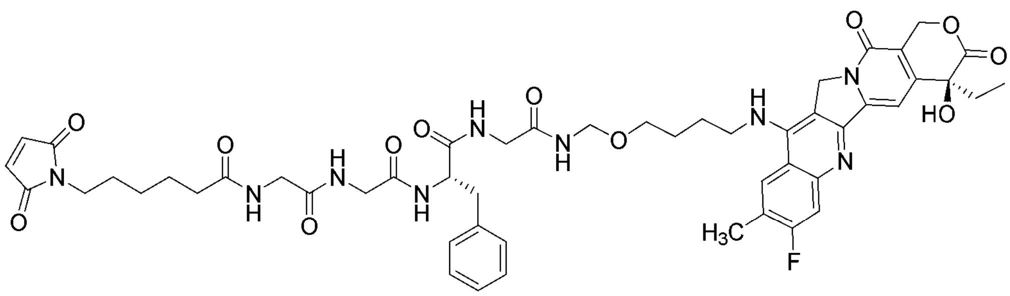 STI-F1002 |

(continued)

| No. | Structure |
|---|---|
| 29 | 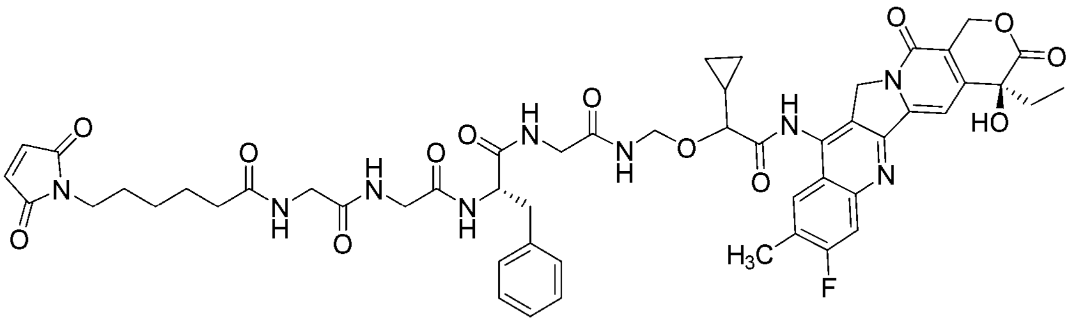 STI-F1001 |
| 30 | 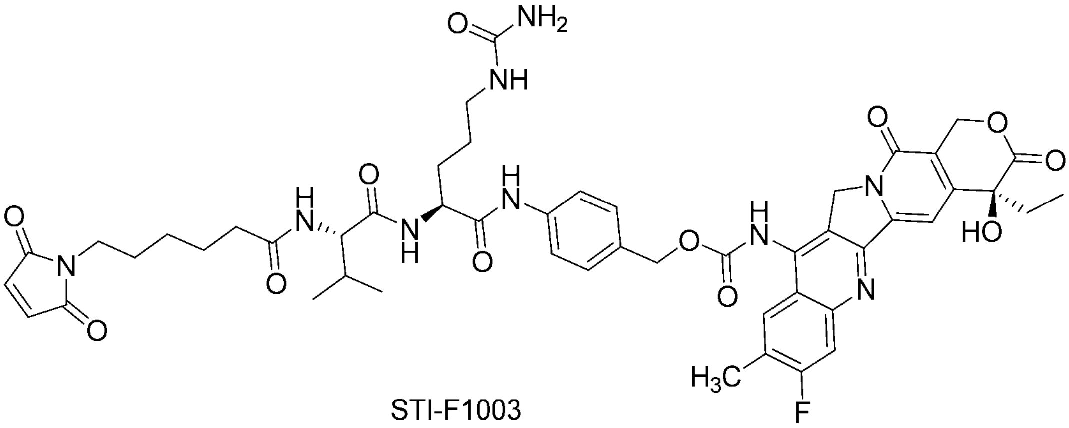 STI-F1003 |
| 31 | 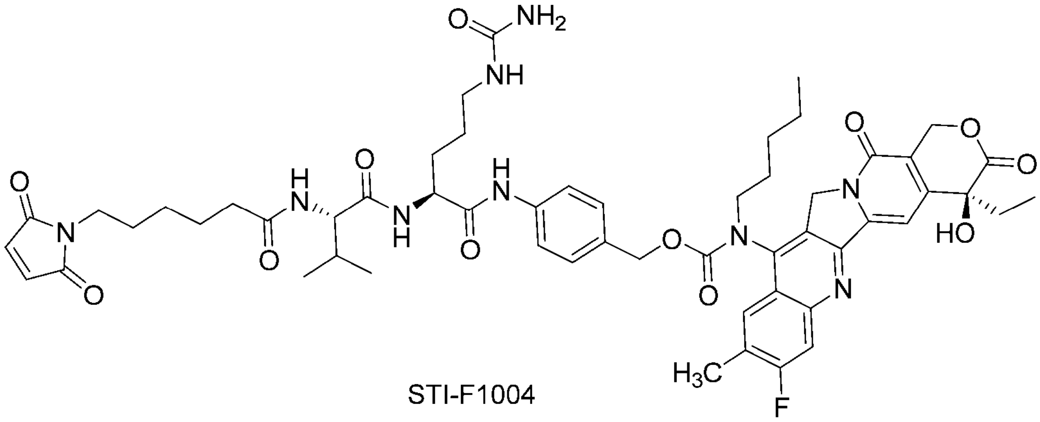 STI-F1004 |
| 32 | 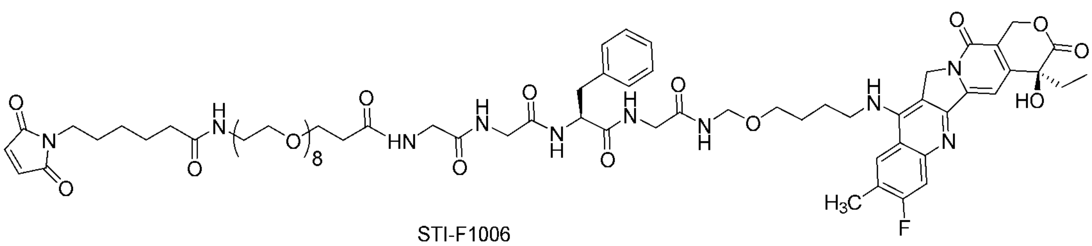 STI-F1006 |

(continued)

| No. | Structure |
|---|---|
| 33 | 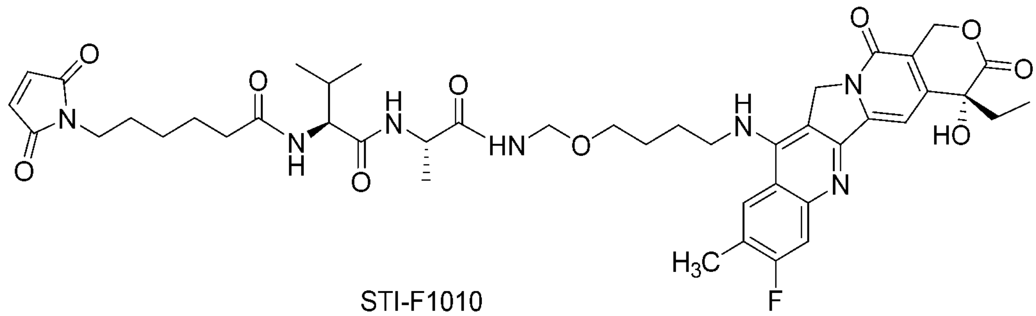 STI-F1010 |
| 34 | 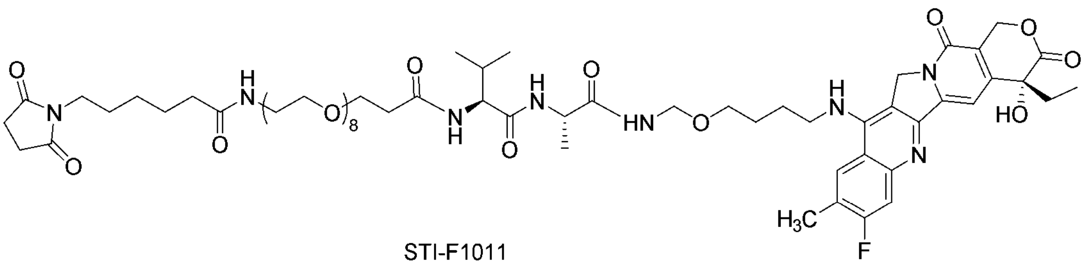 STI-F1011 |
| 35 | 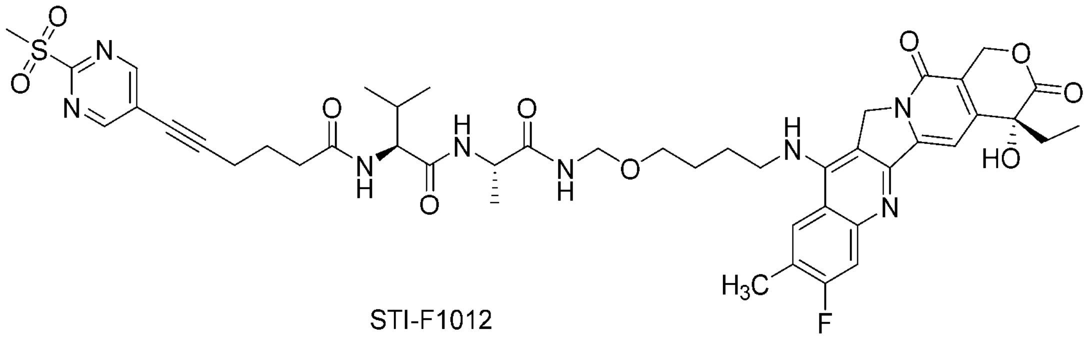 STI-F1012 |
| 36 | 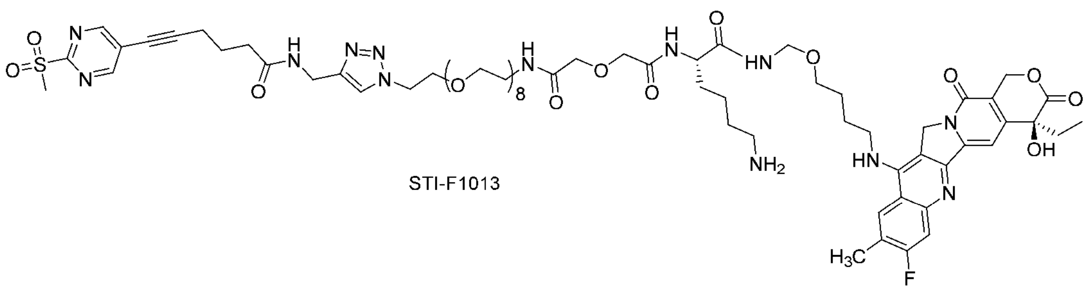 STI-F1013 |
| 37 | 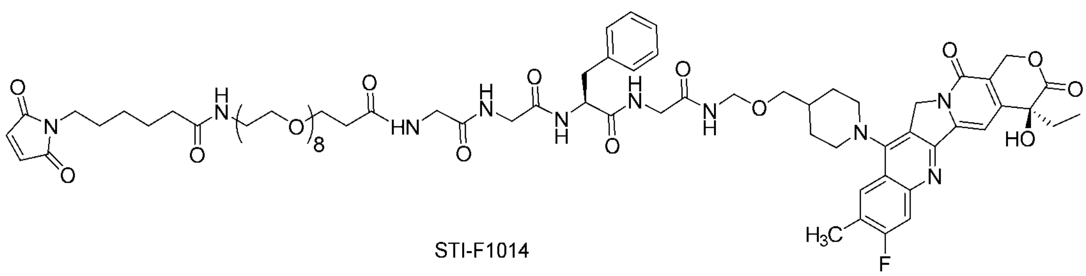 STI-F1014 |

(continued)

| No. | Structure |
|---|---|
| 38 | 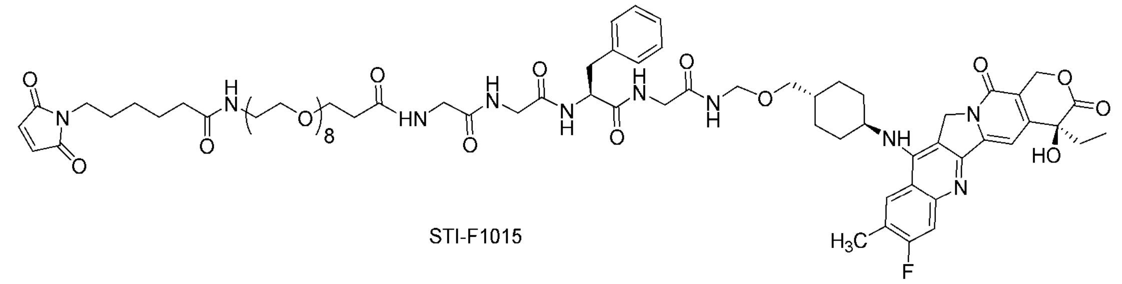 STI-F1015 |
| 39 | 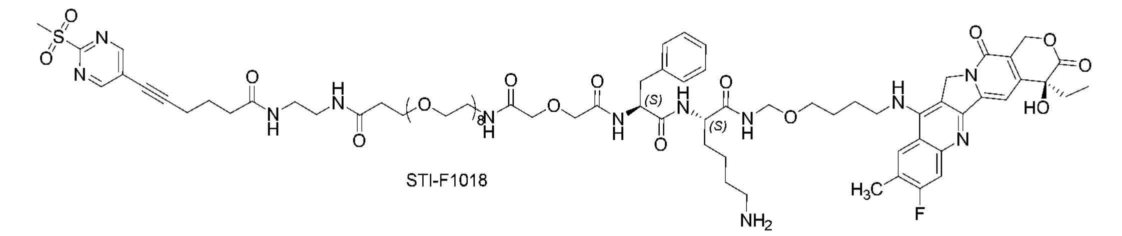 STI-F1018 |
| 40 | 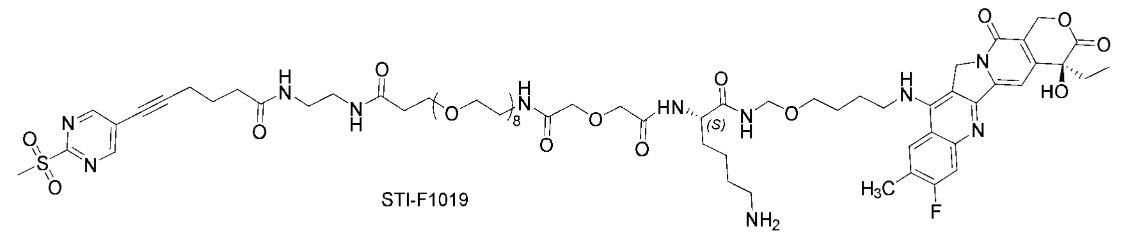 STI-F1019 |
| 41 | 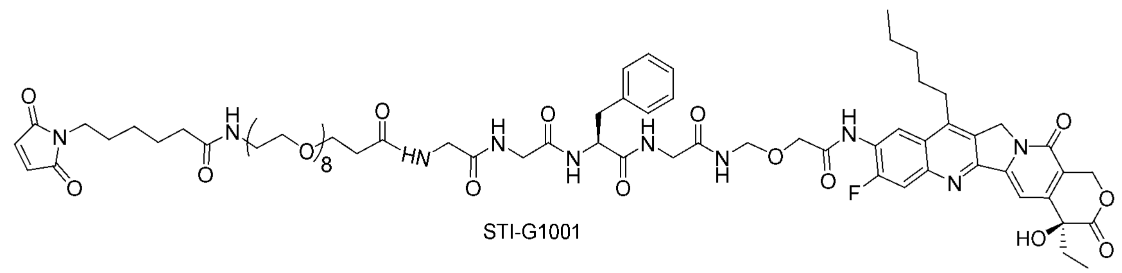 STI-G1001 |
| 42 | 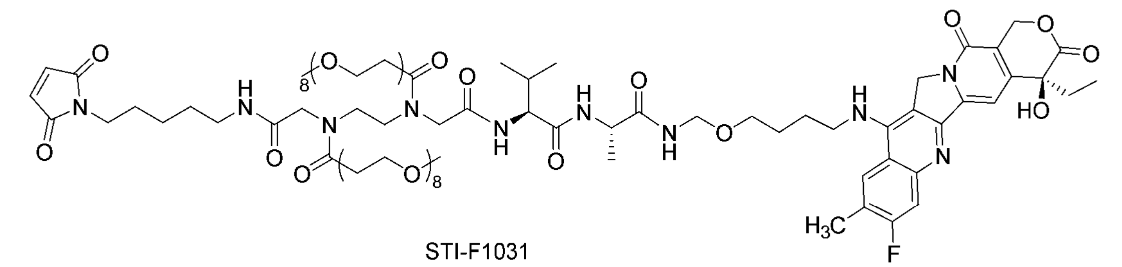 STI-F1031 |
| 43 | 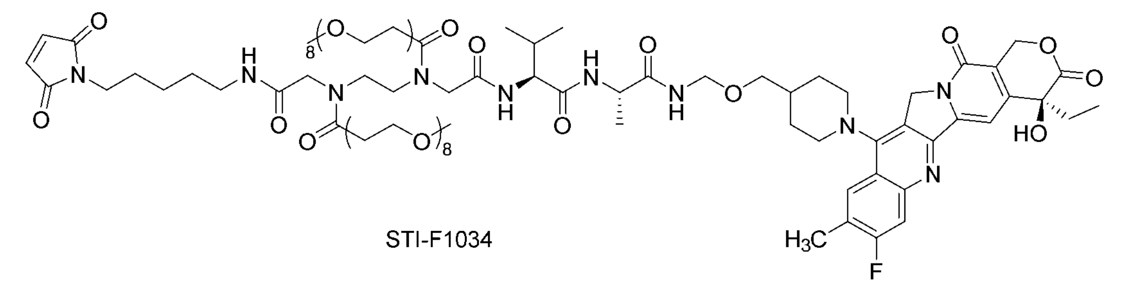 STI-F1034 |

(continued)

| No. | Structure |
| --- | --- |
| 44 | 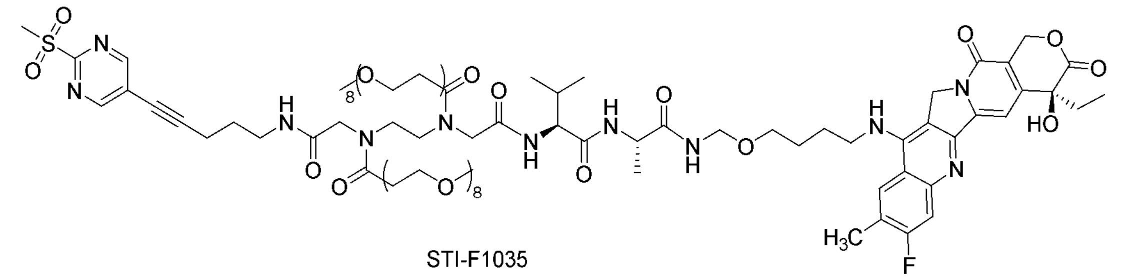 STI-F1035 |
| 45 | 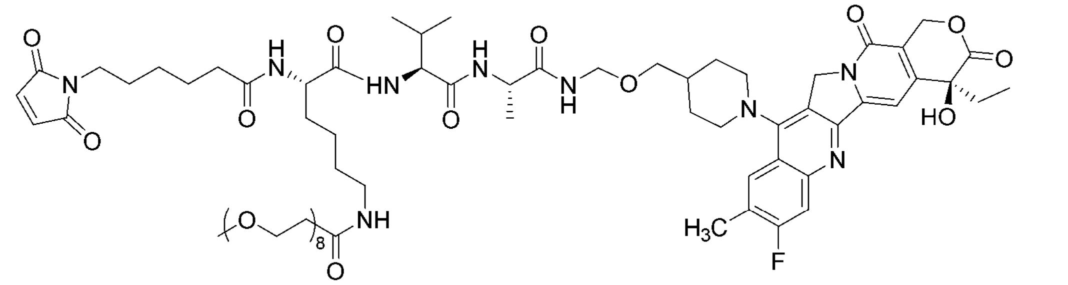 STI-F1043 . |

In another aspect, the present invention provides a pharmaceutical composition comprising the antibody-drug conjugate of the present invention, or a pharmaceutically acceptable salt or ester, solvate, tautomer, stereoisomer, prodrug, or isotopically labeled form thereof; or the compound of the present invention, or a pharmaceutically acceptable salt or ester, solvate, tautomer, stereoisomer, prodrug, or isotopically labeled form thereof; or the linker-payload conjugate of the present invention, or a pharmaceutically acceptable salt or ester, solvate, tautomer, stereoisomer, prodrug, or isotopically labeled form thereof; as well as a pharmaceutically acceptable carrier, diluent or excipient thereof.

In another aspect, the present invention provides the antibody-drug conjugate of the present invention, or a pharmaceutically acceptable salt or ester, solvate, tautomer, stereoisomer, prodrug, or isotopically labeled form thereof; or the compound of the present invention, or a pharmaceutically acceptable salt or ester, solvate, tautomer, stereoisomer, prodrug, or isotopically labeled form thereof; or the linker-payload conjugate of the present invention, or a pharmaceutically acceptable salt or ester, solvate, tautomer, stereoisomer, prodrug, or isotopically labeled form thereof, for use in the treatment or prevention of cancer.

In another aspect, the present invention provides a method for preventing or treating cancer in a patient, comprising administering to the patient a therapeutically effective amount of the antibody-drug conjugate of the present invention, or a pharmaceutically acceptable salt or ester, solvate, tautomer, stereoisomer, prodrug, or isotopically labeled form thereof; or the compound of the present invention, or a pharmaceutically acceptable salt or ester, solvate, tautomer, stereoisomer, prodrug, or isotopically labeled form thereof; or the linker-payload conjugate of the present invention, or a pharmaceutically acceptable salt or ester, solvate, tautomer, stereoisomer, prodrug, or isotopically labeled form thereof.

In another aspect, the present invention provides the use of the antibody-drug conjugate of the present invention, or a pharmaceutically acceptable salt or ester, solvate, tautomer, stereoisomer, prodrug, or isotopically labeled form thereof; or the compound of the present invention, or a pharmaceutically acceptable salt or ester, solvate, tautomer, stereoisomer, prodrug, or isotopically labeled form thereof; or the linker-payload conjugate of the present invention, or a pharmaceutically acceptable salt or ester, solvate, tautomer, stereoisomer, prodrug, or isotopically labeled form thereof, in the manufacture of a medicament for the treatment or prevention of cancer.

In some embodiments, the cancer is a solid tumor, particularly lung cancer (e.g., small cell lung cancer, non-small cell lung cancer), liver cancer, digestive system cancers (e.g., intestinal cancer, gastric cancer, cardia cancer, esophageal cancer, appendiceal adenocarcinoma, colon cancer, rectal cancer, colorectal cancer, pancreatic cancer), bladder cancer, melanoma, breast cancer, ovarian cancer, cervical cancer, endometrial cancer, prostate cancer, basal cell carcinoma, cholangiocarcinoma, squamous cell carcinoma, thyroid cancer, brain cancer, head and neck cancer, peritoneal cancer, kidney cancer, urothelial carcinoma, testicular cancer, central nervous system tumors (e.g., glioma, glioblastoma such as glioblastoma multiforme, glioma, or sarcoma), choriocarcinoma, and oral epidermoid carcinoma; or hematological malignancies, particularly leukemias (e.g., acute or chronic myeloid leukemia, acute or chronic granulocytic leukemia), lymphomas (e.g., Hodgkin's lymphoma, non-Hodgkin's lymphoma, diffuse large B-cell lymphoma, acute B-cell lymphoma, follicular lymphoma), and multiple myeloma. Preferably, the cancer is ovarian cancer, colon cancer, lung adenocarcinoma, or oral epidermoid carcinoma.

The terms and scientific/technical nomenclature used in the present application shall have the meanings generally understood by a person skilled in the art, unless otherwise specified. In particular, the terms in the present application have the meanings shown below.

The terms "a", "an", "the" and the entity itself, as used herein, are to be understood to encompass both the singular and the plural, unless otherwise specifically indicated or clearly contradicted by the context.

A hyphen ("-") that does not appear between two letters or symbols indicates a point of attachment for a substituent. For example, $-NR_aR_b$ indicates that the group is attached to the rest of the molecule through the nitrogen atom, and $-(CH_2)_p-NR_aR_b$ indicates that the group is attached to the rest of the molecule through $-(CH_2)_p-$. The "-" may also be omitted when the point of attachment for the substituent is obvious to a person skilled in the art (e.g., for halogen, CN, OH, $NH_2$, etc.).

The term "halogen" or "halo" refers to fluorine (F), chlorine (Cl), bromine (Br), or iodine (I), preferably fluorine, chlorine, or bromine.

The term "alkyl", alone or as part of another group, represents a fully saturated straight- or branched-chain hydrocarbon group consisting of carbon and hydrogen atoms. Preferably, the alkyl group has 1-6 carbon atoms ($C_{1-6}$ alkyl), 1-4 carbon atoms ($C_{1-4}$ alkyl), or 1-3 carbon atoms ($C_{1-3}$ alkyl). Examples of alkyl groups include, but are not limited to, methyl (Me), ethyl (Et), propyl (Pr) (including n-propyl and isopropyl), butyl (Bu) (including n-butyl, isobutyl, sec-butyl, and tert-butyl), pentyl (including n-pentyl, isopentyl, neopentyl, etc.), hexyl, heptyl, octyl, and the like.

The term "alkenyl", alone or as part of another group, represents a straight- or branched-chain hydrocarbon group consisting of carbon and hydrogen atoms and containing at least one double bond. Preferably, the alkenyl group has 2-6 carbon atoms ($C_{2-6}$ alkenyl), 2-4 carbon atoms ($C_{2-4}$ alkenyl), or 2-3 carbon atoms ($C_{2-3}$ alkenyl). Examples of alkenyl groups include, but are not limited to, vinyl, propenyl, allyl, butenyl, isobutenyl, pentenyl, isopentenyl, hexenyl, and the like.

The term "alkynyl", alone or as part of another group, represents a straight- or branched-chain hydrocarbon group consisting of carbon and hydrogen atoms and containing at least one triple bond. Preferably, the alkynyl group has 2-6 carbon atoms ($C_{2-6}$ alkynyl), 2-4 carbon atoms ($C_{2-4}$ alkynyl), or 2-3 carbon atoms ($C_{2-3}$ alkynyl). Representative examples of alkynyl groups include, but are not limited to, ethynyl, propynyl, propargyl, butynyl, isobutynyl, pentynyl, isopentynyl, hexynyl, and the like.

The terms "alkoxy"and "alkyl-O-" are used interchangeably and represent an alkyl group as defined above that is attached via an oxygen atom. Preferably, the alkoxy group has 1-6 carbon atoms ($C_{1-6}$ alkoxy), 1-4 carbon atoms ($C_{1-4}$ alkoxy), or 1-3 carbon atoms ($C_{1-3}$ alkoxy). Examples of alkoxy groups include, but are not limited to, methoxy, ethoxy, propoxy (including n-propoxy and isopropoxy), butoxy (including n-butoxy, sec-butoxy, isobutoxy, tert-butoxy, etc.), pentoxy (including n-pentoxy, isopentoxy, neopentoxy, etc.), hexoxy, heptoxy, octoxy, and the like.

The terms "alkylthio" and "alkyl-S-" are used interchangeably and represent an alkyl group as defined above that is attached via a sulfur atom. Preferably, the alkylthio group has 1-6 carbon atoms ($C_{1-6}$ alkylthio), 1-4 carbon atoms ($C_{1-4}$ alkylthio), or 1-3 carbon atoms ($C_{1-3}$ alkylthio). Examples of alkylthio groups include, but are not limited to, methylthio, ethylthio, propylthio (including n-propylthio and isopropylthio), butylthio (including n-butylthio, sec-butylthio, isobutylthio, tert-butylthio, etc.), pentylthio (including n-pentylthio, isopentylthio, neopentylthio, etc.), hexylthio, heptylthio, octylthio, and the like.

The term "haloalkyl" refers to an alkyl group, as defined herein, which is substituted with one or more (e.g., 1, 2, 3, 4, 5, 6, or 7) halogen atoms. It is understood that when more than one halogen substituent is present, the halogen substituents may be identical or different and may be located on the same or different carbon atoms. Preferably, the haloalkyl is $C_{1-6}$ haloalkyl, $C_{1-4}$ haloalkyl, or $C_{1-3}$ haloalkyl. Examples of haloalkyl include, but are not limited to, fluoromethyl, chloromethyl, difluoromethyl, dichloromethyl, fluorochloromethyl, trifluoromethyl, trichloromethyl, dichlorofluoromethyl, difluoroethyl, trifluoroethyl, trichloroethyl, chlorodifluoroethyl, difluoropropyl, and trifluoropropyl, and the like.

The term "hydroxyalkyl" refers to an alkyl group, as defined herein, which is substituted with one or more (e.g., 1, 2, 3, 4, 5, 6, or 7) hydroxyl groups. It is understood that when more than one hydroxyl substituent is present, the hydroxyl substituents may be located on the same or different carbon atoms. Preferably, the hydroxyalkyl is $C_{1-6}$ hydroxyalkyl, $C_{1-4}$ hydroxyalkyl, or $C_{1-3}$ hydroxyalkyl. Examples of hydroxyalkyl include, but are not limited to, hydroxymethyl, hydroxyethyl, hydroxypropyl, hydroxybutyl, and the like.

The term "cyanoalkyl" refers to an alkyl group, as defined herein, which is substituted with one or more (e.g., 1, 2, 3, 4, 5, 6, or 7) cyano groups. It is understood that when more than one cyano substituent is present, the cyano substituents may be located on the same or different carbon atoms. Preferably, the cyanoalkyl is $C_{1-6}$ cyanoalkyl, $C_{1-4}$ cyanoalkyl or $C_{1-3}$ cyanoalkyl. Examples of cyanoalkyl include, but are not limited to, cyanomethyl, cyanoethyl, cyanopropyl, and the like.

The term "aminoalkyl" refers to an alkyl group, as defined herein, which is substituted with one or more (e.g., 1, 2, 3, 4, 5, 6, or 7) amino groups. It is understood that when more than one amino substituent is present, the amino substituents may be located on the same or different carbon atoms. Preferably, the aminoalkyl is $C_{1-6}$ aminoalkyl, $C_{1-4}$ aminoalkyl, or

$C_{1-3}$ aminoalkyl. Examples of aminoalkyl include, but are not limited to, aminomethyl, aminoethyl, aminopropyl, and the like.

The term "cycloalkyl", alone or as part of another group, represents a fully or partially saturated non-aromatic monocyclic or bicyclic hydrocarbon group consisting of carbon and hydrogen atoms, preferably fully saturated. Preferably, the cycloalkyl has 3-8 ring carbon atoms ($C_{3-8}$ cycloalkyl) or 3-6 ring carbon atoms ($C_{3-6}$ cycloalkyl). Examples of cycloalkyl include, but are not limited to, cyclopropyl, cyclobutyl, cyclopentyl, cyclopentenyl, cyclohexyl, cyclohexenyl, cycloheptyl, cyclooctyl, and the like. When the cycloalkyl is substituted with one or more hydroxyl groups, it may be referred to as "hydroxycycloalkyl", including $C_{3-8}$ hydroxycycloalkyl, or $C_{3-6}$ hydroxycycloalkyl.

The term "heterocyclic group" refers to a fully saturated (i.e., heterocycloalkyl) or partially saturated (e.g., heterocycloalkenyl containing one or more double bonds, heterocycloalkynyl containing one or more triple bonds, etc.) non-aromatic monocyclic or bicyclic group containing one or more, for example 1, 2, 3, or 4 heteroatoms independently selected from N, O, or S, with the remaining ring members being carbon. The carbon member(s) of the heterocyclic group may optionally be replaced by -CO-, and the optional N and S heteroatoms may optionally be oxidized (e.g., in NO, SO, $SO_2$) and the optional N heteroatoms may optionally be quaternized (e.g., in $[NR]^+Cl^-$, $[NR]^+OH^-$). Preferably, the heterocyclic group is a 3- to 8-membered heterocyclic group, 4-8 membered heterocyclic group, 4-6 membered heterocyclic group, or 4-5 membered heterocyclic group. A nitrogen-containing heterocyclic group represents a heterocyclic group containing one or more nitrogen heteroatoms with the remaining ring members being carbon, for example, pyrrole, dihydropyrrole, pyrrolidine, pyridine, dihydropyridine, tetrahydropyridine, piperidine, etc. Examples of heterocyclic groups include, but are not limited to: aziridinyl, oxiranyl, azetidinyl, oxetanyl, thietanyl, dihydropyrrolyl, pyrrolidinyl, pyrrolidinonyl, dihydrofuranyl, tetrahydrofuranyl, dihydrothienyl, tetrahydrothienyl, tetrahydrothienyl 1-oxide, tetrahydrothienyl 1,1-dioxide, dioxolanyl, dioxolenyl, dihydropyrazolyl, pyrazolidinyl, imidazolinyl, imidazolidinyl, imidazolonyl, dihydroimidazolonyl, tetrahydroimidazolonyl, dihydrooxazolyl, oxazolidinyl, dihydroisoxazolyl, isoxazolidinyl, dihydrothiazolyl, thiazolidinyl, dihydroisothiazolyl, isothiazolidinyl, dihydropyridinyl, tetrahydropyridinyl, piperidinyl, piperidinonyl, dihydropyridazinyl, tetrahydropyridazinyl, hexahydropyridazinyl, dihydropyrimidinyl, tetrahydropyrimidinyl, hexahydropyrimidinyl, dihydropyrazinyl, tetrahydropyrazinyl, piperazinyl, pyranyl, dihydropyranyl, tetrahydropyranyl, thiopyranyl, dihydrothiopyranyl, tetrahydrothiopyranyl, oxazinyl, isoxazinyl, dihydrooxazinyl, oxazinane, morpholinyl, thiomorpholinyl, thiomorpholinyl 1-oxide, thiomorpholinyl 1,1-dioxide, dihydroindolyl, dihydrobenzofuranyl, dihydroisobenzofuranyl, dihydrobenzothienyl, dihydrobenzothiazolyl, chromenyl, dihydrobenzopyranyl, tetrahydroquinolinyl, tetrahydroisoquinolinyl, and the like. The heterocyclic group may be attached to the remainder of the molecule via a carbon atom or a heteroatom, provided it is chemically feasible. The heterocyclic group may optionally be fused with other cyclic groups, which may be aromatic or non-aromatic and may optionally contain one or more heteroatoms selected from N, O, or S, for example, as in

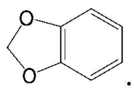
.

The term "aryl" refers to a monovalent carbocycle having one or more rings, preferably 1 or 2 rings, wherein at least one ring is aromatic and the other ring(s), if present, may be aromatic or non-aromatic. The aryl preferably has 6-10 ring carbon atoms (i.e., $C_{6-10}$ aryl), more preferably 6 ring carbon atoms (i.e., $C_6$ aryl or phenyl). Representative examples of aryl include, but are not limited to, phenyl, naphthyl, tetrahydronaphthyl, indenyl, dihydroindenyl, or tetrahydronaphthyl, and the like.

The term "heteroaryl" refers to a monocyclic or bicyclic cyclic group having one or more, preferably 1, 2, 3, 4, 5, or 6 heteroatoms independently selected from N, O, or S, with the remaining ring members being carbon, wherein at least one ring is aromatic, and the other ring(s), if present, may be aromatic or non-aromatic. Any N and S heteroatoms in the heteroaryl may optionally be oxidized (e.g., as in NO, SO, $SO_2$) and any N heteroatoms may optionally be quaternized (e.g., as in $[NR]^+Cl^-$, $[NR]^+OH^-$). The heteroaryl is preferably a 3-8 membered, more preferably a 5-8 membered heteroaryl (e.g., monocyclic, such as nitrogen-containing). Representative examples of heteroaryl include, but are not limited to: pyrrolyl, furanyl, thienyl, pyrazolyl, imidazolyl, oxazolyl, isoxazolyl, thiazolyl, isothiazolyl, triazolyl, pyridinyl, pyridazinyl, pyrimidinyl, pyrazinyl, pyranyl, thiopyranyl, oxazinyl, oxadiazinyl, indolyl, isoindolyl, aza-indolyl (e.g., 7-aza-indolyl, 6-aza-indolyl, 5-aza-indolyl, 4-aza-indolyl), benzofuranyl, isobenzofuranyl, benzothienyl, benzothiazolyl, indazolyl, benzimidazolyl, benzoxazolyl, quinolinyl, isoquinolinyl, benzopyranyl, cinnolinyl, quinazolinyl, quinoxalinyl, benzoxazinyl, benzotriazolyl, purinyl, indolizinyl, pyrrolopyridinyl, pyrrolopyrimidinyl, pyrrolopyrazinyl, imidazopyridinyl, imidazopyrimidinyl, pyrazolopyridinyl, pyrazolopyridazinyl, pyrazolopyrimidinyl, pyrazolopyrazinyl, pyridopyrazinyl, pyridopyrimidinyl, pyrimidopyrimidinyl, pyrazinopyrazinyl, phthalazinyl, naphthyridinyl, and the like. The heteroaryl may be attached to the remainder of the compound via a carbon atom or a heteroatom, provided it is chemically feasible.

The terms "heteroarylene" and "heteroaryldiyl" are used interchangeably and refer to the divalent heteroaryl group as defined herein.

The term "-ylene" or "-diyl" represents a bivalent group derived by removing two hydrogen atoms from a molecule.

The term "CN" represents cyano.

The term "OH" represents hydroxy.

The term "SH" represents mercapto.

The term "$NH_2$" represents amino.

The term "-CO-" or "-C(=O)-" represents carbonyl.

The term "-SO-" represents sulfinyl.

The term "-$SO_2$-" represents sulfonyl.

The term "-COOH" represents carboxy.

The term "$NO_2$" represents nitro.

The term "leaving group" refers to an atom or functional group that readily dissociates from a molecule in a chemical reaction. Examples thereof include, but are not limited to: halogens, e.g., F, Cl, or Br; sulfonyl, e.g., methanesulfonyl or p-toluenesulfonyl; sulfonyloxy, e.g., alkylsulfonyloxy (e.g., mesyloxy), trifluoromethylsulfonyloxy or arylsulfonyloxy (e.g., p-toluenesulfonyloxy); tertiary-ammonium group (e.g., $Me_3N^+$ or $Et_3N^+$); or diazonium groups.

The expression "optional" or "optionally" means that the subsequently described event may or may not occur, and that the description includes circumstances where said event occurs and circumstances where it does not. For example, "optionally substituted with..." includes both the unsubstituted circumstance and the substituted circumstance. An "optional substituent" indicates that the substituent may or may not be present.

When any variable occurs more than once in a structural formula, it is defined independently at each occurrence. For example, the expression "optionally substituted with one or more substituents independently selected from ..." indicates substitution with one or more independently selected substituents, each of which may be identical to or different from each other. Combinations of substituents and/or variables are permitted, provided that the combination results in a stable compound.

The term "comprise" or "include" means to include the stated elements, integers, or steps, but does not exclude the inclusion of any other elements, integers, or steps. As used herein, the term "comprise" or "include" also encompasses the circumstance consisting of the recited elements, integers, or steps, unless otherwise indicated.

The term "agent of the present invention" includes the antibody-drug conjugate of formula (I) of the present invention, the small molecule camptothecin derivative of formula (II) of the present invention, the linker-payload conjugate of formula (III) of the present invention, or a salt or ester, solvate (e.g., hydrate), tautomer, stereoisomer, prodrug, metabolite, or isotopically labeled form (e.g., deuterated form) thereof. In some embodiments, the "agent of the present invention" specifically refers to a compound or conjugate of the Examples, or a salt or ester, solvate (e.g., hydrate), tautomer, stereoisomer, prodrug, metabolite, or isotopically labeled form (e.g., deuterated form) thereof.

The term "pharmaceutically acceptable" refers to a substance or composition that, when administered to an animal such as a human, does not produce adverse effects, allergic reactions, or other undesirable reactions.

The term "pharmaceutically acceptable salt" refers to those salts that retain the biological effectiveness of the agent of the present invention and are not biologically or otherwise undesirable. The agent of the present invention may be in the form of a salt, preferably a pharmaceutically acceptable salt, including acid addition salts and base addition salts. Acid addition salts may be formed with inorganic acids, e.g., hydrochloric acid, hydrobromic acid, hydroiodic acid, sulfuric acid, phosphoric acid, nitric acid, carbonic acid, perchloric acid, etc., or with organic acids, e.g., formic acid, acetic acid, trifluoroacetic acid, propionic acid, glycolic acid, gluconic acid, lactic acid, pyruvic acid, oxalic acid, malic acid, malonic acid, glutaric acid, adipic acid, succinic acid, fumaric acid, maleic acid, tartaric acid, citric acid, aspartic acid, ascorbic acid, glutamic acid, anthranilic acid, benzoic acid, cinnamic acid, mandelic acid, pamoic acid, phenylacetic acid, methanesulfonic acid, ethanesulfonic acid, ethanedisulfonic acid, benzenesulfonic acid, p-toluenesulfonic acid, xylenesulfonic acid, isethionic acid, naphthalenesulfonic acid, naphthalenedisulfonic acid, camphorsulfonic acid, salicylic acid, oleic acid, nicotinic acid, palmitic acid, stearic acid, furoic acid, hippuric acid, orotic acid, and pamoic acid, etc. Base addition salts may be formed with organic or inorganic bases, including but not limited to alkali metal salts, such as lithium, sodium, or potassium salts; alkaline earth metal salts, such as calcium or magnesium salts; organic base salts, such as ammonium salts formed with organic bases containing N-groups. The salts can be synthesized from the parent compound by conventional methods.

Pharmaceutically acceptable salts are preferred. However, other salts may also be useful, for example, in isolation or purification steps, and may be used during preparation, thus they are also encompassed within the scope of the present application.

The term "stereoisomer" as used herein represents an isomer resulting from the presence of at least one asymmetric center. In compounds having one or more (e.g., one, two, three, or four) asymmetric centers, this may result in racemic mixtures, single enantiomers, mixtures of diastereomers, and individual diastereomers. Certain individual molecules may also exist as geometric isomers (cis/trans).

The term "tautomer" refers to structural isomers having different energies, which are interconvertible with each other via a low energy barrier. For example, proton tautomers (also known as prototropic tautomers) include interconversions via proton migration, such as keto-enol tautomerization and imine-enamine tautomerization. Valence tautomers include interconversions via reorganization of some bonding electrons.

Solid lines, solid wedges, or dashed wedges may be used in the present application to depict bonds in the agent of the present invention. A solid line bond attached to an asymmetric carbon atom is intended to indicate that all possible stereoisomers at that carbon atom are included (e.g., a specific enantiomer, a racemic mixture, etc.). A solid or dashed wedge attached to an asymmetric carbon atom is intended to indicate the presence of the depicted stereoisomer. When in a racemic mixture, solid and dashed wedges are used to define relative stereochemistry, not absolute stereochemistry. Unless otherwise indicated, the agent of the present invention may exist as stereoisomers, including cis and trans isomers, optical isomers (e.g., R and S enantiomers), diastereomers, geometric isomers, rotamers, conformational isomers, atropisomers, and mixtures thereof. The agent of the present invention may exhibit more than one type of isomerism and consist of the mixtures thereof (e.g., racemic mixtures and diastereomeric pairs).

The present invention also includes prodrugs of the agent of the present invention. The term "prodrug" refers to a chemically modified active or inactive compound that, after administration to an individual, is converted into the agent of the present invention through physiological processes *in vivo* (e.g., hydrolysis, metabolism, etc.). Accordingly, in the present application, the term "administration" includes administering a prodrug of the agent of the present invention to treat various diseases or disorders, wherein said prodrug is converted into the agent of the present invention *in vivo.* Techniques for the production and use of prodrugs are well known to those skilled in the art.

The present invention also includes all pharmaceutically acceptable isotopically labeled forms, which are identical to the agent of the present invention except that one or more atoms are replaced by atoms having the same atomic number but an atomic mass or mass number different from the atomic mass or mass number predominantly found in nature. Examples of isotopes suitable for incorporation into the agent of the present invention include, but are not limited to: isotopes of hydrogen (e.g., $^{2}H$, $^{3}H$); isotopes of carbon (e.g., $^{11}C$, $^{13}C$ and $^{14}C$); isotopes of chlorine (e.g., $^{36}Cl$); isotopes of fluorine (e.g., $^{18}F$); isotopes of iodine (e.g., $^{123}I$ and $^{125}I$); isotopes of nitrogen (e.g., $^{13}N$ and $^{15}N$); isotopes of oxygen (e.g., $^{15}O$, $^{17}O$ and $^{18}O$); isotopes of phosphorus (e.g., $^{32}P$); and isotopes of sulfur (e.g., $^{35}S$).

The present invention also encompasses metabolites of the agent of the present invention, i.e., compounds formed *in vivo* following administration of the agent of the present invention. These metabolites may be produced through, for example, oxidation, reduction, hydrolysis, amidation, deamidation, esterification, de-esterification, enzymatic cleavage, and the like. Accordingly, the present invention includes metabolites of the agent of the present invention, including compounds produced by methods involving contact of the agent of the present invention with a mammal for a period sufficient to yield its metabolite products.

Some agents of the present invention may exist in both unsolvated and solvated forms, including hydrated forms. The term "solvate" refers to a complex formed by the agent of the present invention with solvent molecules. Examples of solvents capable of forming solvates include water, isopropanol, ethanol, methanol, DMSO, ethyl acetate, acetic acid, and ethanolamine. The term "hydrate" refers to a complex wherein the solvent molecule is water. Methods of solvation are well known in the art.

The term "individual" or "patient" refers to an animal, preferably a mammal. Examples of individuals include, but are not limited to, primates (e.g., humans and non-human primates such as monkeys), equines, bovines, ovines, felines, canines, lagomorphs, and rodents (e.g., mice and rats). In some embodiments, the individual is a human, including children, adolescents, or adults.

The term "treatment" refers to (i) treating or preventing a particular disease, disorder, or condition, (ii) alleviating, ameliorating, or eliminating one or more symptoms of a particular disease, disorder, or condition, and optionally (iii) preventing or delaying the onset of one or more symptoms of the specific disease, disorder, or condition described herein. In some embodiments, "treatment" refers to improving at least one physical parameter, which may not be perceptible to the patient. In other embodiments, "treatment" refers to modulating a disease or disorder physically (e.g., stabilizing perceptible symptoms), physiologically (e.g., stabilizing physical parameters), or both.

The term "prevention" refers to the administration of one or more pharmaceutical substances, particularly the compound of the present invention and/or a pharmaceutically acceptable salt thereof, to an individual predisposed to a disease or disorder, in order to prevent the individual from developing the disease.

The terms "inhibition", "alleviation" and the like refer to the reduction or suppression of a specific ailment, symptom, or condition, or a significant decrease in biological activity or baseline activity of a process.

The term "effective amount" refers to an amount sufficient to achieve a desired therapeutic or preventive effect at the required dosage and for the required duration. It may be determined by the attending physician or veterinary practitioner and will vary depending on such factors as the compound, the disease state being treated, the severity of the disease, the age and related health status of the individual, the route and form of administration, and the judgment of the attending physician or veterinary practitioner. Generally, a "preventive effective amount" is less than a "therapeutic effective amount".

The terms "formulation" or "pharmaceutical composition" refer to a composition comprising at least one active ingredient and at least one inactive ingredient, e.g., a pharmaceutically acceptable excipient, which is suitable for

administration to an animal, preferably a mammal (including humans). The formulation of the present invention may be any formulation applicable in the field, such as tablets, capsules, liquid formulations, etc.

The term "pharmaceutically acceptable excipient" refers to a component in a pharmaceutical formulation other than the active ingredient, and is non-toxic to the individual. Examples of pharmaceutically acceptable excipients include, but are not limited to, binders, disintegrants, lubricants, solvents, dispersion media, buffers, vehicles, antioxidants, preservatives, or flavoring agents.

When referring to chemical reactions, "treating", "contacting" and "reacting" mean adding or mixing two or more reagents under appropriate conditions to produce the indicated and/or desired product. It is understandable that the reaction producing the indicated and/or desired product may not necessarily result directly from the combination of the initially added reagents, i.e., there may be one or more intermediates generated in the mixture that ultimately lead to the formation of the indicated and/or desired product.

The term "about", when used in conjunction with a numerical value, represents a range of ±20%, preferably ±10%, and more preferably ±5% of the stated value.

Antibody-Drug Conjugate

The term "antibody-drug conjugate" or "ADC" as used herein refers to a substance formed by conjugating a small-molecule drug moiety (Payload) to an antibody or an antigen-binding fragment thereof (Ab, which is responsible for the targeting and sometimes also has biological activity) via a linker.

The method of conjugating a linker to an antibody or antigen-binding fragment thereof is known to a person skilled in the art, including non-site-specific conjugation and site-specific conjugation, for example but not limited to, lysine conjugation, cysteine conjugation, Thiomab technology, incorporation of unnatural amino acids, and enzymatic catalysis. For example, for a maleimide linker, cysteine residues on the antibody are utilized as attachment sites for the payload. Reduction of the interchain disulfide bonds on the antibody generates eight free thiol groups, which undergo Michael addition with maleimide to attach the payload, thereby producing an ADC product with a DAR value close to 8.

Antibody-drug conjugates can be characterized by the Drug-to-Antibody Ratio (DAR). The term "Drug-to-Antibody Ratio" or "DAR" as used herein refers to the ratio of the number of small molecule drug moieties (D) conjugated to the antibody or antigen-binding fragment thereof (Ab) to the number of antibody or antigen-binding fragment moieties (Ab). The average Drug-to-Antibody Ratio (average DAR) represents the ratio of the molar concentration of total drug molecules to the molar concentration of total antibody molecules in the assay system. The DAR can range from 1 to 20, although higher number of payloads are also possible, which depends on the number of conjugation sites on the antibody. Methods for determining DAR are known in the art, such as reversed-phase high-performance liquid chromatography (RP-HPLC), size-exclusion chromatography (SEC-HPLC), mass spectrometry, and hydrophobic interaction chromatography (HIC-HPLC).

In some embodiments, the antibody-drug conjugate of the present invention has an average DAR selected from any value within the following ranges: 1.0-20.0, 1.0-18.0, 1.0-16.0, 1.0-10.0, 2.0-14.0, 3.0-12.0, 4.0-10.0, 5.0-9.0, 6.0-8.0, or 2.0-8.0. In some embodiments, the antibody-drug conjugate of the present invention has an average DAR selected from any value within the following ranges: 2±0.4, 4±0.4, 6±0.4, or 8±0.4. In some embodiments, the antibody-drug conjugate of the present invention has an average DAR of about 2.0, 4.0, 6.0, 8.0, 10.0, or 12.0.

In some embodiments, the antibody-drug conjugate of the present invention has an average DAR value of about 1.0 to about 20.0, for example about 1.0-18.0, about 1.0-16.0, about 2.0-14.0, about 3.0-12.0, about 4.0-10.0, about 5.0-9.0, about 6.0-8.0, about 1.0-8.0, about 2.0-6.0, for example about 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, 10.0, 12.0, and 16.0, as well as ranges having any two of these values as endpoints.

Antibody or Antigen-Binding Fragment Thereof

The terms "full antibody", "whole antibody" or "full-length antibody" are used interchangeably herein and refer to an antibody molecule having the structure of a natural immunoglobulin molecule. For a conventional four-chain IgG antibody, the full-length antibody comprises two heavy chains (H) and two light chains (L) interconnected by disulfide bonds. For a heavy-chain antibody that only has heavy chains and lacks light chains, the full-length antibody comprises two heavy chains (H) interconnected by disulfide bonds.

The term "antibody fragment" includes a portion of an intact antibody. Preferably, the antibody fragment is an antigen-binding fragment.

The term "antigen-binding fragment" refers to a molecule that differs from an intact antibody. It comprises a portion of the intact antibody, and binds to the antigen to which the intact antibody binds. Examples of antibody fragments

include, but are not limited to, Fv, Fab, Fab', Fab'-SH, $F(ab')_2$; dAb (domain antibody); linear antibodies; single-chain antibodies (e.g., scFv); single-domain antibodies such as VHH; diabodies or fragments thereof; or camelid antibodies.

The term "antigen" refers to a molecule that elicits an immune response. This immune response may involve the production of antibodies, the activation of specific immune cells, or both. A person skilled in the art will understand that any macromolecule, including nearly all proteins or peptides, can serve as an antigen. Further, an antigen may be derived from recombinant or genomic DNA. In some embodiments, the antigen is a tumor cell surface antigen, such as Her2 or FRα.

The terms "complementarity determining region", "CDR region", or "CDR" refer to regions within the antibody variable domain that are hypervariable in sequence and form structurally determined loops ("hypervariable loops") and/or contain antigen-contact residues ("antigen contact points"). CDRs are primarily responsible for binding to an antigenic epitope. The CDRs of the heavy and light chains are generally designated CDR1, CDR2, and CDR3, numbered sequentially from the N-terminus. CDRs located within the variable domain of an antibody heavy chain are referred to as HCDR1, HCDR2, and HCDR3, whereas those located within the variable domain of an antibody light chain are referred to as LCDR1, LCDR2, and LCDR3. For a given light or heavy chain variable region amino acid sequence, the precise amino acid sequence boundaries of each CDR can be determined using any one of, or a combination of, many well-known antibody CDR designation systems. These designation systems include, for example: Chothia based on antibody three-dimensional structure and the topology of CDR loops (Chothia et al., 1989, Nature 342: 877-883; Al-Lazikani et al., "Standard conformations for the canonical structures of immunoglobulins", Journal of Molecular Biology, 273, 927-948, 1997); Kabat based on antibody sequence variability (Kabat et al., Sequences of Proteins of Immunological Interest, 4th ed., U.S. Department of Health and Human Services, National Institutes of Health (1987)); AbM (University of Bath); Contact (University College London); the international ImMunoGeneTics database (IMGT) (available on the World Wide Web at imgt.cines.fr/); and the North CDR definition based on affinity propagation clustering utilizing a large set of crystal structures (North et al., "A New Clustering of Antibody CDR Loop Conformations", Journal of Molecular Biology, 406, 228-256, 2011).

The term "antibody in IgG form" refers to an antibody whose heavy chain constant region belongs to the IgG isotype. For example, an antibody in IgG4 form indicates that its heavy chain constant region is derived from IgG4, and an antibody in IgG1 form indicates that its heavy chain constant region is derived from IgG1.

Antibodies or antigen-binding fragments thereof applicable for the present application may bind to any antigen suitable for an antibody, preferably a tumor cell surface antigen, such as Her2 or FRα.

In some embodiments, the antibody or antigen-binding fragment thereof applicable for the present application is an antibody or antigen-binding fragment thereof that binds to Her2, particularly human Her2. Any Her2 antibody or antigen-binding fragment thereof known to a person skilled in the art is suitable for the present invention, including, but not limited to, previously disclosed antibodies or commercially available antibodies, such as trastuzumab.

In some embodiments, the antibody or antigen-binding fragment thereof applicable for the present application is an antibody or antigen-binding fragment thereof that binds to FRα, particularly human FRα. Any FRα antibody or antigen-binding fragment thereof known to a person skilled in the art is suitable for the present invention, including, but not limited to, previously disclosed antibodies or commercially available antibodies, such as farletuzumab.

In some embodiments, the antibody applicable for the present application is an antibody in IgG1 form, an antibody in IgG2 form, an antibody in IgG3 form, or an antibody in IgG4 form. Preferably, the antibody suitable for the ADCs of the present application is an antibody in IgG1 form or an antigen-binding fragment thereof.

In some embodiments, the antibody applicable for the present application is a monoclonal antibody. In some embodiments, the antibody suitable for the present invention is humanized. In some embodiments, the antibody suitable for the present invention is a human antibody. In some embodiments, the antibody suitable for the present invention is a chimeric antibody.

In one embodiment, the antibody applicable for the present application is a full-length antibody.

In one embodiment, the antigen-binding fragment of the antibody applicable for the present application is an antibody fragment selected from the group consisting of: Fab, Fab', Fab'-SH, Fv, single-chain antibodies (e.g., scFv), $(Fab')_2$, single-domain antibodies such as VHH, dAb (domain antibody), linear antibodies, half-antibodies, or bispecific, trispecific, tetraspecific, and other multispecific antibodies. In some embodiments, the antigen-binding fragment suitable for the ADCs of the present application includes, but is not limited to, VHH, Fv molecules, scFv molecules, Fab molecules, and $F(ab')_2$ molecules.

In one embodiment of the present invention, the antibody or antigen-binding fragment thereof suitable for use in the present application is trastuzumab or farletuzumab, or an antigen-binding fragment thereof. In one embodiment, the antibody or antigen-binding fragment thereof comprises one or more CDRs (preferably 3 CDRs, i.e., HCDR1, HCDR2, and HCDR3; or LCDR1, LCDR2, and LCDR3; more preferably 6 CDRs, i.e., HCDR1, HCDR2, HCDR3, LCDR1, LCDR2, and LCDR3) of trastuzumab or farletuzumab, or an antigen-binding fragment thereof; or comprises the VH and/or VL of trastuzumab or farletuzumab, or an antigen-binding fragment thereof; or comprises the heavy chain and/or light chain of said antibody.

In some embodiments, the anti-Her2 antibody or antigen-binding fragment thereof in the present invention

comprises heavy chain variable regions CDR1, CDR2, and CDR3, and light chain variable regions CDR1, CDR2, and CDR3 from trastuzumab. In some embodiments, the anti-Her2 antibody or antigen-binding fragment thereof in the present invention comprises the heavy chain variable regions and the light chain variable regions from trastuzumab. In some embodiments, the anti-Her2 antibody or antigen-binding fragment thereof in the present invention comprises the heavy chains and the light chains from trastuzumab.

In some embodiments, the anti-FRα antibody or antigen-binding fragment thereof in the present invention comprises heavy chain variable regions CDR1, CDR2, and CDR3, and light chain variable regions CDR1, CDR2, and CDR3 from farletuzumab. In some embodiments, the anti-FRα antibody or antigen-binding fragment thereof in the present invention comprises the heavy chain variable regions and the light chain variable regions from farletuzumab. In some embodiments, the anti-FRα antibody or antigen-binding fragment thereof in the present invention comprises the heavy chains and the light chains from farletuzumab.

Linker

The term "linker" as used herein refers to a connecting moiety used to link a small-molecule cytotoxic drug (Payload) to an antibody or an antigen-binding fragment thereof (Ab), and may be represented as the $-L_1-L_2-L_3-L_4$ moiety in the present application. Linkers generally include cleavable linkers and non-cleavable linkers. Cleavable linkers utilize the difference in conditions between the bloodstream and the tumor-cell interior (e.g., pH, proteolytic activity, or glutathione concentration) or take advantage of the particular action of antibody in the lysosome (e.g., lysosomal proteases) to cleave and release the payload, thereby exerting drug activity. Non-cleavable linkers rely on degradation of the antibody in the lysosome to release the payload and produce pharmacological activity.

Linkers suitable for the present invention include cleavable linkers and non-cleavable linkers, such as acid-sensitive linkers (e.g., hydrazone linkers), glutathione-sensitive linkers (e.g., disulfide linkers), enzymatically cleavable linkers (e.g., short peptide linkers, β-glucuronide linkers), amide bond linkers, thioether linkers, and the like. In particular, linkers suitable for the present application include, but are not limited to, those provided above, especially those used in the Examples.

The term "linker unit" refers to a component ($L_1$) of the linker ($-L_1-L_2-L_3-L_4$-moiety), which serves to connect the linker, or the linker as part of a linker-payload conjugate, to the antibody or an antigen-binding fragment thereof. Examples thereof include, but are not limited to:

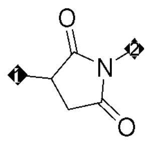 , 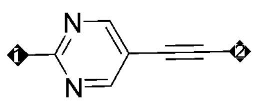 , 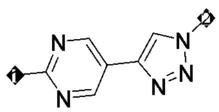 , 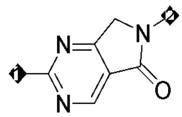 , or 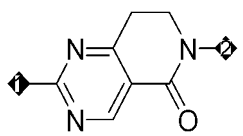 ,

wherein position 1 is connected to Ab, and position 2 is connected to $L_2$.

The term "linking unit" refers to a component ($L_2$) of the linker ($-L_1-L_2-L_3-L_4$-moiety), which serves to connect the linker unit ($L_1$) to the $L_3$ moiety that are amino acid residue, short peptide chain, or $-NH-(CH_2)_p-CO-$.

$L_3$, a component of the linker in the present application, is an amino acid residue, a short peptide chain consisting of 2-10 (preferably 2-6) amino acid residues, or $-NH-(CH_2)_p-CO-$, e.g., $-NH-(CH_2)_2-CO-$. The amino acid may be natural or unnatural, e.g., glycine (Gly), alanine (Ala), valine (Val), phenylalanine (Phe), citrulline (Cit), lysine (Lys), and asparagine (Asn). $L_3$ may be connected to the small-molecule drug moiety directly or via a spacer unit.

The term "spacer unit" refers to a component ($L_4$) of the linker ($-L_1-L_2-L_3-L_4$-moiety). When present, it serves to connect the linker to the small-molecule drug moiety (D), or may provide an additional structural moiety to facilitate the release of the small-molecule drug moiety from the ADC. Examples thereof include, but are not limited to:

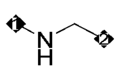 , 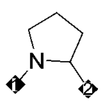 , 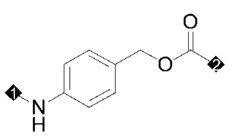 , 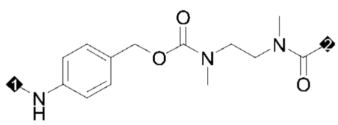 and 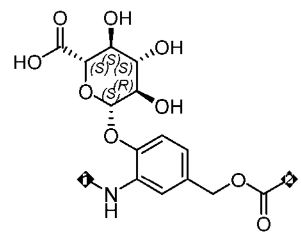 ,

wherein position 1 is connected to $L_3$, and position 2 is connected to D.

Small-Molecule Drug Moiety

The terms "small-molecule drug moiety", "small-molecule cytotoxic drug moiety" and "Payload" are used interchangeably herein, and refer to the component responsible for killing tumor cells in an antibody-drug conjugate or a linker-payload conjugate. After administration, the ADC undergoes cleavage between or within target cells, releasing the small-molecule drug or a derivative thereof, which then exerts its biological activity. To avoid ambiguity, it should be noted that the "drug" refers to not only a "pharmaceutical product" approved by medicinal regulatory authorities but also any compound with potential biological activity in clinical, research, or academic studies.

In the present invention, the small-molecule drug moiety is derived from a camptothecin derivative of formula (II), particularly from the camptothecin derivatives described in the Examples herein. It is a moiety obtained by the loss of one atom (e.g., a hydrogen atom) or a group of atoms from the camptothecin derivative of formula (II). For example, the small-molecule drug moiety is obtained by the loss of one hydrogen atom attached to the nitrogen or oxygen atom in $R_1$ or $R_3$, or by the loss of one hydrogen atom from the hydroxyl group shown in formula (II). The hydroxyl group shown in formula (II) refers to the hydroxyl group indicated by the arrow in the following structure:

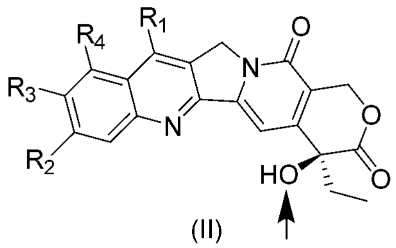

(II)

The small-molecule drug moiety of the present invention may be connected to the linker via any chemically feasible bonding mode, including but not limited to amide bonds, aminomethyl ether bonds, and carbamate bonds.

*Efficacy and Beneficial Effects*

The agent of the present invention, including the antibody-drug conjugate of formula (I), the camptothecin derivative of formula (II), and the linker-payload conjugate of formula (III), or a pharmaceutically acceptable salt or ester, solvate, tautomer, stereoisomer, or isotopically labeled form thereof, exhibit high cytotoxic activity and are useful for treating or preventing tumors such as cancer, including but not limited to solid tumors, particularly lung cancer (e.g., small cell lung cancer, non-small cell lung cancer), liver cancer, digestive system cancers (e.g., intestinal cancer, gastric cancer, cardia cancer, esophageal cancer, appendiceal adenocarcinoma, colon cancer, rectal cancer, colorectal cancer, pancreatic cancer), bladder cancer, melanoma, breast cancer, ovarian cancer, cervical cancer, endometrial cancer, prostate cancer, basal cell carcinoma, cholangiocarcinoma, squamous cell carcinoma, thyroid cancer, brain cancer, head and neck cancer, peritoneal cancer, kidney cancer, urothelial carcinoma, testicular cancer, central nervous system tumors (e.g., glioma, glioblastoma such as glioblastoma multiforme, glioma, or sarcoma), choriocarcinoma, and oral epidermoid carcinoma; or hematological malignancies, particularly leukemias (e.g., acute or chronic myeloid leukemia, acute or chronic granulocytic leukemia), lymphomas (e.g., Hodgkin's lymphoma, non-Hodgkin's lymphoma, diffuse large B-cell lymphoma, acute B-cell lymphoma, follicular lymphoma), and multiple myeloma. Preferably, the agent of the present invention is usefule for treating and/or preventing ovarian cancer, colon cancer, lung adenocarcinoma, or oral epidermoid carcinoma. Compared with known camptothecin derivatives such as Dxd and SN-38, the agent of the present invention demonstrates superior tumor-killing activity.

The agent of the present invention exhibits good tolerability, and does not cause significant behavioral abnormalities or body weight fluctuations (e.g., decrease) while effectively killing tumor cells.

The agent of the present invention possesses good solubility and physicochemical stability, allowing them to be readily formulated into pharmaceutical preparations suitable for administration.

The ADCs of the present invention have a suitable drug-to-antibody ratio and a uniform distribution of the drug-to-antibody ratio.

The ADCs of the present inventionare are stable in blood circulation, reducing the loss of drug molecules in non-target tissues and mitigating off-target toxicity. Moreover, the ADCs of the present invention exhibit excellent tumor tissue targeting, accumulating in the tumor microenvironment, which increases the concentration ratio of the active drug molecule within the tumor relative to the blood and reduces mechanism-related toxicity of the conjugate, leading to a favorable therapeutic index. The small-molecule drug released from the ADCs of the present invention can also exert a bystander effect, further killing tumor cells in the vicinity of the target tumor cells that do not express or express low levels of the antigen.

The ADCs of the present invention demonstrate superior therapeutic or preventive efficacy and/or reduced side effects compared to the corresponding small-molecule camptothecin derivatives, providing a wider therapeutic window.

*Pharmaceutical Compositions and Administration*

The agent of the present invention may be administered in the form of pharmaceutical compositions via any applicable route, for example but not limited to, oral (e.g., in the form of tablets, capsules, or liquid formulations such as solutions, emulsions, or suspensions), inhalation (e.g., as aerosols), rectal (e.g., as suppositories), or parenteral administration (e.g., as injectable solutions, such as intravenous, intramuscular, subcutaneous, intraperitoneal, intracranial, etc.). Parenteral administration, such as intravenous injection, is particularly preferred.

Techniques for formulating the agent of the present invention into pharmaceutical compositions are well known in the art. For example, the agent of the present invention may be processed into forms of pharmaceutical compositions, such as tablets, capsules, liquid formulations (e.g., injections, infusions, syrups, emulsions, suspensions, etc.), lyophilized powders for injection, dispersions, sprays, suppositories, liposomes, etc., using one or more pharmaceutically acceptable carriers, diluents, or excipients. Pharmaceutically acceptable carriers, diluents, or excipients are well known in the art, including, for example, fillers, disintegrants, solvents, solubilizers, stabilizers, wetting agents, emulsifiers, preservatives, sweeteners, colorants, flavoring agents, salts for adjusting osmotic pressure, buffers, masking agents, or antioxidants, etc.

The dosage may vary within a wide range and must, of course, be adjusted according to the individual needs in each specific case. Determination of an appropriate dosage may be made by the attending physician based on considerations such as the type and severity of the disease to be treated, the individual's health condition and medical history, the specific compound to be administered, the route of administration, and concomitant medications. The daily dosage for a 70 kg adult is typically from about 0.01 mg to about 1000 mg of the agent of the present invention, or a corresponding amount of a pharmaceutically acceptable salt or ester thereof. If necessary, the dosage of the agent of the present invention may exceed this range. The daily dosage may be administered as a single dose or in divided doses.

The agent of the present invention may be used alone or in combination with one or more other active agents or therapies whose pharmacological effects may be the same as, or different from, those of the agent of the present invention. The agent of the present invention may be administered simultaneously, separately, or sequentially with other concomitant other active agents via the same or different routes of administration. They may be included in the same pharmaceutical composition (fixed combination) or provided as separate forms, such as a combination product in the form of a kit. They may be formulated and/or supplied by the same or different manufacturers. When the agent of the present invention is administered in combination with other active agents, the dosage of the co-administered active agents will, of course, vary depending on such factors as the concomitant medication, the condition to be treated, the individual's general health conditions, and the judgment of the physician or veterinarian. To facilitate patient compliance, the kit of the present application may typically include instructions for administration.

General Preparation Methods

The agent of the present invention may be prepared by various methods, including the method described in the schemes below, the method provided in the Examples, or these analogous thereto. For each reaction step, appropriate reaction conditions are known to or can be readily determined by a person skilled in the art. Starting materials are generally commercially available or can be readily prepared using methods known in the art or described herein. Each variable in the general formula has the meaning defined herein, unless otherwise specified.

In the preparation of the compounds of the present invention, the protection of groups (e.g., amino protecting groups, hydroxy protecting groups) may be necessary, which can be readily determined by a person skilled in the art. For a general description of protecting groups and their use, reference may be made to T. W. Greene, Protective Groups in Organic Synthesis, John Wiley & Sons, New York, 1991.

If necessary, starting materials and intermediates in the synthetic reaction schemes may be isolated and purified through conventional techniques, including but not limited to filtration, distillation, crystallization, chromatography, etc. These materials may be characterized by conventional methods, including physical constants and spectral data.

For illustrative purposes only, the following scheme provides an exemplary route for synthesizing the agent of the present invention. It is understood by a person skilled in the art that other synthetic routes may also be utilized, and compounds prepared by the methods described below may be further modified based on the content of the present application using conventional compounds well known to a person skilled in the art.

Scheme: Synthesis of Camptothecin Derivatives

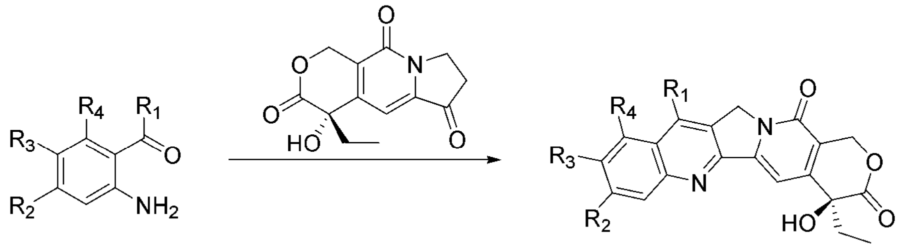

wherein each variable is as defined herein.

This reaction can be conducted via Friedländer quinoline synthesis, for example, under catalysis by an acid such as p-toluenesulfonic acid, glacial acetic acid, or other Lewis acids to afford the reaction product. Subsequently, the resulting product can be further modified, for example, through substitution reactions, condensation reactions (forming amide bonds), Buchwald-Hartwig coupling reactions, or amino-deprotection, to yield other camptothecin derivatives. The reaction conditions can be readily determined by those skilled in the field of organic synthesis.

The synthesis of linker-payload conjugates falls within the capability of a person skilled in the field of organic synthesis. For example, linker-payload conjugates can be prepared via Williamson ether synthesis, condensation reactions, and the like.

Methods for linking an antibody to a linker-payload conjugate are well konw in the ADC field, such as lysine conjugation, cysteine conjugation, Thiomab technology, unnatural amino acid technology, enzymatic conjugation, etc. Specifically, the antibody may be reduced and then conjugated with the linker-payload conjugate, and the resulting antibody-drug conjugate optionally purified.

**Brief Description of the Drawings**

Figs. 1a to 1d show the cytotoxic efficacy of the ADCs of the present invention against Capan-1 cells; RLU denotes relative light units (Example B).

Figs. 2a to 2d show the cytotoxic efficacy of the ADCs of the present invention against NCI-N87 cells; RLU denotes relative light units (Example B).

Figs. 3a to 3e show that the ADCs of the present invention exhibit a bystander effect (Example C).

Figure 4 shows that the ADCs of the present invention inhibit the growth of human ovarian cancer cell SKOV-3 tumors in a mouse model (Example D.2).

Figure 5 shows that the ADCs of the present invention do not cause body weight loss and are well tolerated in the SKOV-3 immunodeficient mouse model (Example D.2).

Figs. 6a and 6b show the tumor growth curve (6a) and body weight change rate curve (6b), respectively, after administration of the ADCs of the present invention in a JIMT-1 tumor-bearing mouse model (Example D.3).

Figs. 7a and 7b show the tumor growth curve (7a) and body weight change rate curve (7b), respectively, after administration of the ADCs of the present invention in a Capan-1 tumor-bearing mouse model (Example D.3).

Figs. 8a and 8b show the tumor growth curve (8a) and body weight change rate curve (8b), respectively, after administration of the ADCs of the present invention in an NCI-N87 tumor-bearing mouse model (Example D.3).

Figs. 9a and 9b show the tumor growth curve (9a) and body weight change rate curve (9b), respectively, after administration of the ADCs of the present invention in a JIMT-1 tumor-bearing mouse model (Example D.4).

Figs. 10a and 10b show the tumor growth curve (10a) and body weight change rate curve (10b), respectively, after administration of the ADCs of the present invention in a Capan-1 tumor-bearing mouse model (Example D.4).

Figs. 11a and 11b show the tumor growth curve (11a) and body weight change rate curve (11b), respectively, after administration of the ADCs of the present invention in an NCI-N87 tumor-bearing mouse model (Example D.4).

Figs. 12a and 12b show the tumor growth curve (12a) and body weight change rate curve (12b), respectively, after administration of the ADCs of the present invention in a Calu-3 tumor-bearing mouse model (Example D.5).

Figs. 13a and 13b show the tumor growth curve (13a) and body weight change rate curve (13b), respectively, after administration of the ADCs of the present invention in an OE-19 tumor-bearing mouse model (Example D.5).

Figs. 14a and 14b show the tumor growth curve (14a) and body weight change rate curve (14b), respectively, after administration of the ADCs of the present invention in a Capan-1 tumor-bearing mouse model (Example D.6).

## Examples

The following examples are provided to further illustrate the present invention. It should be understood that the examples are provided solely for the purpose of facilitating a better understanding of the invention and in no way limit the scope thereof.

In the present application, where a chemical name and a structural formula are inconsistent, the structural formula shall prevail, unless the context indicates that the chemical name, rather than the structural formula, is correct. For simplicity, not all hydrogen atoms are explicitly shown in some of the compound structural formulas provided herein. When a compound has vacant valencies, it indicates the presence of hydrogen atoms that are not explicitly labeled.

Experimental materials and reagents used in the Examples are commercially available, or can be readily prepared using methods known to a person skilled in the art.

## Abbreviations

The abbreviations used herein (e.g., for chemical groups and compounds) generally have the meanings known in the art, unless otherwise indicated. For example, the following abbreviations are used herein: acetyl (Ac), boron trichloride ($BCl_3$), tert-butoxycarbonyl (Boc), deuterated chloroform ($CDCl_3$), N,N'-dicyclohexylcarbodiimide (DCC), 1,2-dichloroethane (DCE), dichloromethane (DCM), N,N-diisopropylethylamine (DIPEA/DPEA), 4-dimethylaminopyridine (DMAP), N,N-dimethylformamide (DMF), dimethyl sulfoxide (DMSO), deuterated dimethyl sulfoxide (DMSO-$d_6$), ethyl acetate (EA), 6-(maleimido)hexanoic acid succinimide ester (EMCS), triethylamine ($Et_3N$), 9-fluorenylmethoxycarbonyl (Fmoc), glycine (Gly or G), 2-(7-azobenzotriazole)-N,N,N',N'-tetramethyluronium hexafluorophosphate (HATU), lysine (Lys or K), mole/liter (M), meta-chloroperoxybenzoic acid (mCPBA), acetonitrile (MeCN), N-hydroxysuccinimide (NHS), triflate (OTf), tris(dibenzylideneacetone)dipalladium(0) [$Pd_2(dba)_3$], 1,1-bis(diphenylphosphine)ferrocene palladium dichloride [$Pd(dppf)Cl_2$], petroleum ether (PE), pentafluorophenol (PFP-OH), phenylalanine (Phe or F), pyridinium p-toluenesulfonate (PPTS), preparative thin-layer chromatography (Prep-TLC), pyridine (Py), trifluoroacetic acid (TFA), trifluoromethanesulfonic anhydride ($Tf_2O$), thin-layer chromatography (TLC), p-tosyl (Ts), p-toluenesulfonic acid (TsOH), 4,5-bis(diphenylphosphino)-9,9-dimethylxanthene (Xantphos), and sodium triacetoxyborohydride (STAB).

## Testing and Preparation Basic Information

### Liquid Chromatography-Mass Spectrometry (LCMS)

Test Instrument Model: Agilent 1260II G6125B
Test Method: Electrospray ionization source, positive/negative ion mode ($ESI^{\pm}$)
Chromatography Column: YMC-TRIART C18 (4.6×50mm×5μm)
Mobile Phase: A (10Mm $NH_4HCO_3$) - B (MeCN): 0-1.5min (5%B-100%B), 1.5-2.5min (100%B-100%B), 2.5-2.6min (100%B-5%B), 2.6-3.75min (5%B-5%B); Flow Rate: 2.0 mL/min;
Column Temperature: 40°C
LCMS data were generated under the following conditions: chlorine isotopes are reported as $^{35}Cl$, and bromine isotopes are reported as $^{79}Br$.

### Nuclear Magnetic Resonance Spectroscopy ($^1H$ NMR)

Test Instrument: Bruker AVANCE NEO 400 NMR
Test Method: Solvent DMSO-$d_6$ (+$D_2O$) or $CDCl_3$; Internal Standard: Tetramethylsilane (TMS)

The multiplicity of signals is denoted by the following abbreviations: s, singlet; d, doublet; t, triplet; q, quartet; dd, doublet of doublets; dt, doublet of triplets; tt, triplet of triplets; td, triplet of doublets; ddd, doublet of doublet of doublets; br, broad; hept, heptet; m, multiplet. All observed coupling constants *J* are reported in Hertz. Exchangeable protons, including but not limited to active hydrogens such as hydroxyl hydrogen, carboxyl hydrogen, and amino hydrogen, are not always observed.

**Medium-to-High Pressure Reverse-Phase prep-LC**

Preparation Instrument: SHIMADZU LC-20AP
Chromatography Column: SHIMADZU C18 (50 × 250 mm, 10 μm)
Acidic Mobile Phase: 0.05% TFA aqueous solution-MeCN; Basic Mobile Phase: 0.1% $NH_4HCO_3$ aqueous solution-MeCN; Neutral Mobile Phase: $H_2O$/MeCN;
Flow Rate: 15 mL/min; Detection Wavelength: 214 nm, 254 nm

**Low-to-Medium Pressure Reverse-Phase prep-LC**

Preparation Instrument: ISOLERA PRIME
Chromatography Column: Spherical C18 (40-750 μm, 100 Å)
Basic Mobile Phase: 0.1% $NH_4HCO_3$ aqueous solution-MeCN; Acidic Mobile Phase: 0.05% TFA aqueous solution-MeCN;
Flow Rate: 25 mL/min; Detection Wavelength: 214 nm, 254 nm

**Example 1: Synthesis of (S)-4-ethyl-4-hydroxy-8-fluoro-9-methyl-11-(chloromethyl)-1,12-dihydro-14H-pyrano [3',4':6,7 ]indolizino[1,2-b]quinoline-3,14(4H)-dione (STI-A-4)**

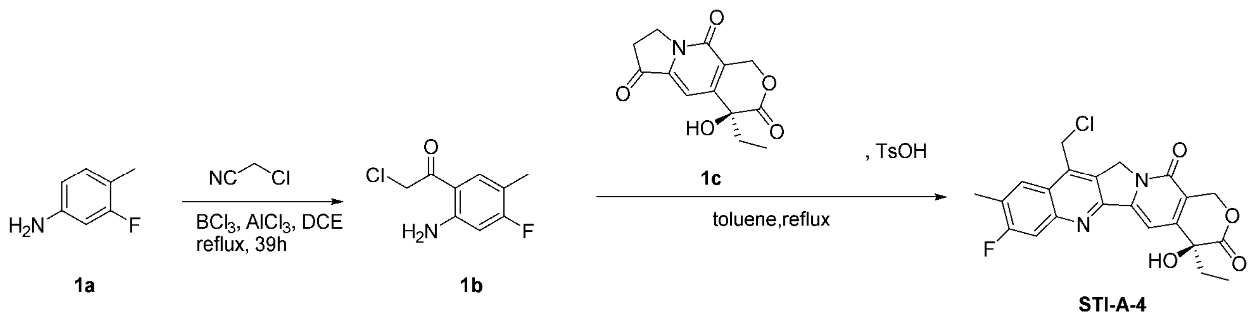


1a 1b 1c STI-A-4

Step 1: Under a nitrogen atmosphere and in an ice bath, 160 mL DCE and a 1M solution of boron trichloride in DCM (32.0 mL, 32.0 mmol) were added to a reaction flask, followed by the addition of compound 1a (5.0 g, 40.0 mmol), with stirring at 0°C for 10 minutes. With the ice bath maintained, chloroacetonitrile (5.4 mL, 47.0 mmol) and aluminum trichloride (7.0 g, 52.0 mmol) were added, then the reaction system was warmed slowly to room temperature (RT). The system was stirred at RT for 10 min, and then stirred under reflux for 39 hours. After the completion of reaction, the system was added into 80 mL ice water, followed by the addition of 40 mL 4 M HCl, with stirring at RT for 30 minutes. The reaction was extracted with 80 mL dichloromethane. The organic phase was dried over anhydrous $Na_2SO_4$ and concentrated under reduced pressure. The residue was subjected to medium-to-high pressure reverse-phase prep-LC (neutral mobile phase) and lyophilized to afford 1.0 g of compound 1b as a yellow solid powder; yield 12.4%. LCMS (254 nm) purity 97.2%, Rt = 1.789 min; MS Calculated: 201.0; MS Found: 202.0 $[M+H]^+$. $^1H$ NMR (400 MHz, DMSO-$d_6$) δ 7.68 (d, *J* = 8.7 Hz, 1H), 7.30 (s, 2H), 6.53 (d, *J* = 12.5 Hz, 1H), 4.97 (s, 2H), 2.08 (s, *J* = 8.6 Hz, 3H).

Step 2: Compound 1b (500 mg, 2.48 mmol) and compound 1c (490 mg, 1.86 mmol) were dissolved in 30 mL anhydrous toluene under nitrogen atmosphere and refluxed for 30 minutes using a water separator. TsOH (48 mg, 0.25 mmol) was added, and the mixture was stirred under reflux for 3.5 hours. After the completion of the reaction confirmed by TLC (DCM:MeOH = 30:1, product Rf = 0.4) and LCMS, the reaction system was cooled to RT, filtered, and washed with acetone. The filter cake was dried under vacuum at RT to give 719 mg of compound STI-A-4 as a yellow solid; yield 90.2%. LCMS (254 nm) purity 96.8%, Rt = 1.711 min; MS Calculated: 428.1; MS Found: 429.2 $[M+H]^+$. $^1H$ NMR (400 MHz, DMSO-$d_6$) δ 8.36 (d, *J* = 8.1 Hz, 1H), 7.94 (d, *J* = 10.7 Hz, 1H), 7.32 (s, 1H), 5.44 (d, *J* = 5.1 Hz, 4H), 5.35 (s, 2H), 2.54 (s, 3H), 1.92 - 1.78 (m, 2H), 0.88 (t, *J* = 7.3 Hz, 3H).

**Example 2: Synthesis of (S,E)-4-ethyl-4-hydroxy-8-fluoro-9-methyl-11-((neopentylimino)methyl)-1,12-dihydro-14H-py rano[3',4':6,7]indolizino[1,2-b]quinoline-3,14(4H)-dione (STI-A-6)**

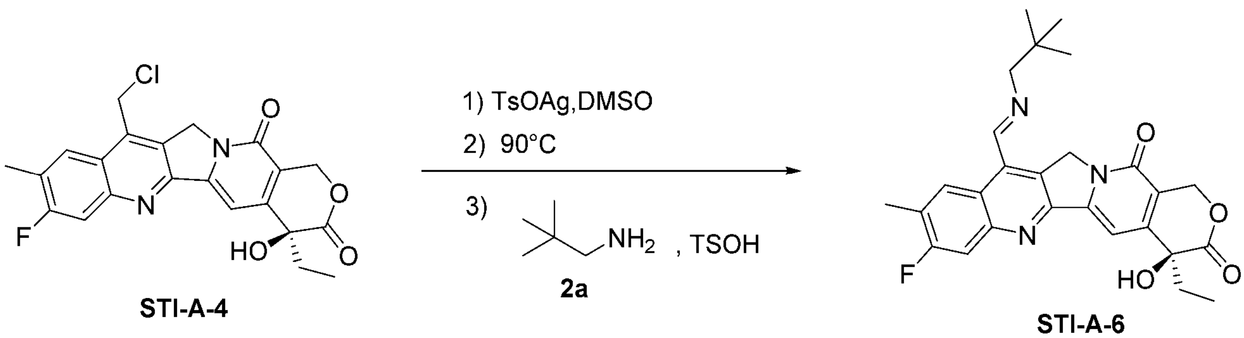


STI-A-4

STI-A-6

Compound STI-A-4 (500 mg, 1.17 mmol) from Example 1 and silver p-toluenesulfonate (390 mg, 1.40 mmol) were dissolved in 30 mL DMSO. The mixture was stirred at RT overnight under a nitrogen atmosphere, and then heated to 90°C with stirring for 3 hours. After completion of reaction confirmed by LCMS monitoring, the mixture was cooled to RT, and TsOH (21 mg, 0.12 mmol) and compound 2a (234 mg, 2.69 mmol) were added with stirring at RT for 3.5 hours. After completion of reaction confirmed by LCMS monitoring, the mixture was subjected to low-to-medium pressure reverse-phase prep-LC (basic mobile phase) and lyophilized to afford 152 mg of the title compound (STI-A-6) as a yellow solid in 27.3% yield. LCMS (254 nm) purity 94.8%, $R_t$ = 2.234 min; MS Calculated: 477.2; MS Found: 478.1 $[M+H]^+$. $^1$H NMR (400 MHz, DMSO-$d_6$) δ 9.29 (s, 1H), 8.56 (d, *J* = 8.1 Hz, 1H), 7.78 (d, *J* = 10.6 Hz, 1H), 7.22 (s, 1H), 6.45 (s, 1H), 5.34 (s, 2H), 5.17 (s, 2H), 3.56 (s, 2H), 2.42 (s, 3H), 1.87 - 1.76 (m, 2H), 1.00 (s, 9H), 0.83 (t, *J* = 7.4 Hz, 3H).

**Example 3: Synthesis of (S)-4-ethyl-4-hydroxy-8-fluoro-9-methyl-11-((neopentylamino)methyl)-1,12-dihydro-14H-pyra no[3',4':6,7]indolizino[1,2-b]quinoline-3,14(4H)-dione (STI-A)**

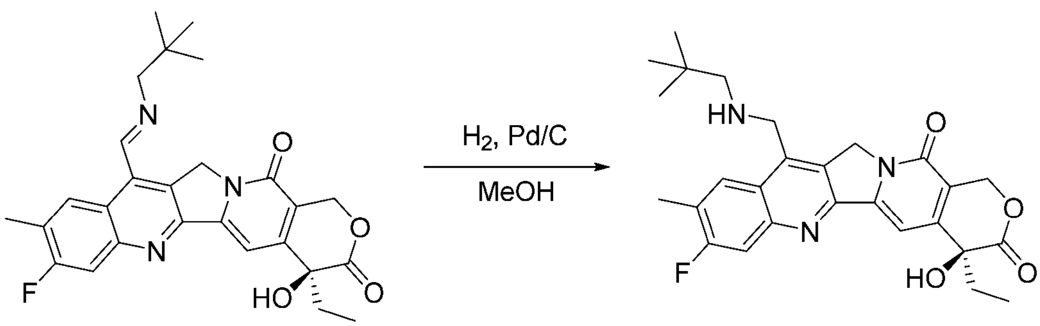


STI-A-6

STI-A

Compound STI-A-6 (152 mg, 0.32 mmol) from Example 2 and 10% Pd/C (51 mg, 0.05 mmol) were suspended in 10 mL methanol, and stirred under a hydrogen atmosphere (1 atm) at RT for 4 hours. After completion of reaction confirmed by LCMS monitoring, the system was filtered. The filtrate was subjected to low-to-medium pressure reverse-phase prep-LC (basic mobile phase) and lyophilized to afford 37 mg of the title compound (STI-A) as a yellow solid in 24.2% yield. LCMS (254 nm) purity 99.0%, Rt = 2.147 min; MS Calculated: 479.2; MS Found: 480.3 $[M+H]^+$. $^1$H NMR (400 MHz, DMSO-$d_6$) δ 8.36 (d, *J* = 8.5 Hz, 1H), 7.85 (d, *J* = 10.8 Hz, 1H), 7.31 (s, 1H), 6.51 (s, 1H), 5.43 (s, 2H), 5.38 (s, 2H), 4.30 (s, 2H), 2.49 (s, 3H), 2.43 (s, 2H), 1.93 - 1.81 (m, 2H), 0.88 (s, 12H).

**Example 4: Synthesis of (S)-4-ethyl-4-hydroxy-8-fluoro-9-methyl-11-(((cyclopentylmethyl)amino)methyl)-1,12-dihydro -14H-pyrano[3',4':6,7]indolizino[1,2-b]quinoline-3,14(4H)-dione (STI-A2)**

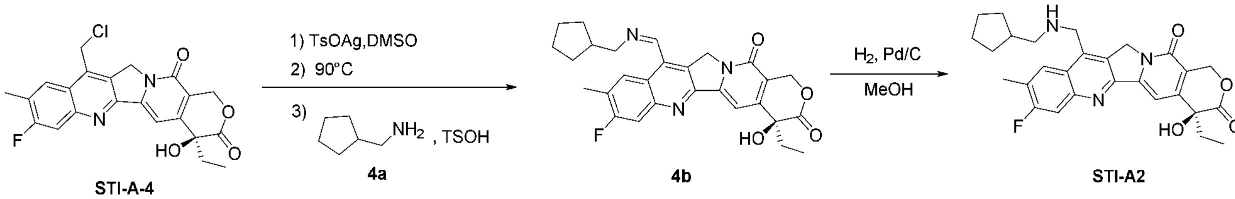


STI-A-4 4a 4b STI-A2

Step 1: Compound STI-A-4 (150 mg, 0.35 mmol) from Example 1 and silver p-toluenesulfonate (117 mg, 0.42 mmol) were dissolved in 10 mL DMSO. The mixture was stirred at RT overnight under nitrogen atmosphere, and then heated to 90°C with stirring for 3 hours. After completion of reaction confirmed by LCMS monitoring, the mixture was cooled to RT, andn TsOH (3 mg, 0.02 mmol) and compound 4a (73 mg, 0.74 mmol) were added. The mixture was stirred at RT for 1 hour. After completion of reaction confirmed by LCMS monitoring, the mixture was subjected to low-to-medium pressure reverse-phase prep-LC (basic mobile phase) and lyophilized to afford 16 mg of compound 4b as a yellow solid in 10.4% yield. LCMS (254 nm) purity 95.5%, Rt = 2.084 min; MS Calculated: 489.2; MS Found: 490.2 $[M+H]^+$. $^1H$ NMR (400 MHz, DMSO-$d_6$) δ 9.45 (s, 1H), 8.73 (d, *J* = 8.1 Hz, 1H), 7.94 (d, *J* = 10.9 Hz, 1H), 7.32 (s, 1H), 6.52 (s, 1H), 5.42 (s, 2H), 5.33 (s, 2H), 2.52 (s, 3H), 1.89 - 1.78 (m, 4H), 1.72 - 1.62 (m, 2H), 1.63 - 1.48 (m, 3H), 1.49 - 1.37 (m, 4H), 0.88 (t, *J* = 7.3 Hz, 3H).

Step 2: Compound 4b (10 mg, 0.02 mmol) and 10% Pd/C (3 mg, 0.003 mmol) were suspended in 3 mL methanol. The mixture was stirred under a hydrogen atmosphere (1 atm) at RT for 6 hours. After completion of reaction confirmed by LCMS monitoring, the system was filtered. The filtrate was subjected to low-to-medium pressure reverse-phase prep-LC (basic mobile phase) and lyophilized to afford 3 mg of the title compound (STI-A2) as a yellow solid in 24.2% yield. LCMS (254 nm) purity 95.7%, Rt = 1.959 min; MS Calculated: 491.2; MS Found: 492.3 $[M+H]^+$. $^1H$ NMR (400 MHz, DMSO-$d_6$) δ 8.32 (d, *J* = 8.5 Hz, 1H), 7.88 (d, *J* = 10.7 Hz, 1H), 7.57 (s, 1H), 5.32 (s, 2H), 4.80 - 4.61 (m, 2H), 4.35 (s, 2H), 4.19 (d, *J* = 13.5 Hz, 1H), 2.65 (d, *J* = 7.5 Hz, 2H), 2.47 (s, 3H), 2.09 - 2.01 (m, 2H), 1.72 (d, *J* = 14.2 Hz, 3H), 1.52 (d, *J* = 22.0 Hz, 6H), 0.86 (d, *J* = 7.1 Hz, 3H).

**Example 5: Synthesis of (S)-4-ethyl-8-fluoro-4-hydroxy-9-methyl-3,14-dioxo-3,4,12,14-tetrahydro-1H-pyrano [3',4':6,7] indolizino[1,2-b]quinoline-11-carboxylic acid (STI-F-01)**

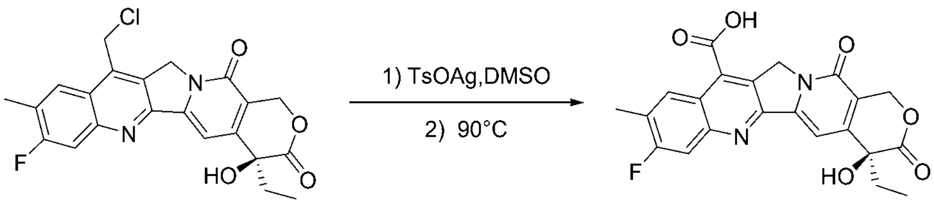


STI-A-4 STI-F-01

Compound STI-A-4 (250 mg, 0.58 mmol) from Example 1 and silver p-toluenesulfonate (200 mg, 0.70 mmol) were dissolved in 20 mL DMSO. The mixture was stirred at RT overnight under a nitrogen atmosphere, and then heated to 90°C with stirring for 3 hours. After formation of aldehyde confirmed by LCMS monitoring, a portion of the reaction liquid was subjected to low-to-medium pressure reverse-phase prep-LC (basic mobile phase), air-oxidised, and lyophilized to afford 50 mg of the title compound (STI-F-01) as a white solid. LCMS: Rt = 1.25 min; MS Calculated: 424.1; MS Found: 425.2 $[M+H]^+$. $^1H$ NMR (400 MHz, DMSO-$d_6$) δ 8.85 (d, *J* = 8.5 Hz, 1H), 7.84 (d, *J* = 11.0 Hz, 1H), 7.29 (s, 1H), 6.50 (s, 1H), 5.42 (s, 2H), 5.27 (s, 2H), 2.47 (s, 3H), 1.92 - 1.80 (m, 2H), 0.88 (t, *J* = 7.4 Hz, 3H).

**Example 6: Synthesis of (S)-7-ethyl-7-hydroxy-10,13-dihydro-11H-[1,3]dioxolo[4,5-g]pyrano [3',4':6,7]indolizino[1,2-b]quinoline-8,11(7H)-dione (STI-E05), and**

**Example 7: Synthesis of (S)-7-ethyl-7-hydroxy-14-(4-hydroxybutyl)-10,13-dihydro-11H-[1,3] dioxolo[4,5-g]pyrano[3',4':6,7]indolizino[1,2-b]quinoline-8,11(7H)-dione (STI-E)**

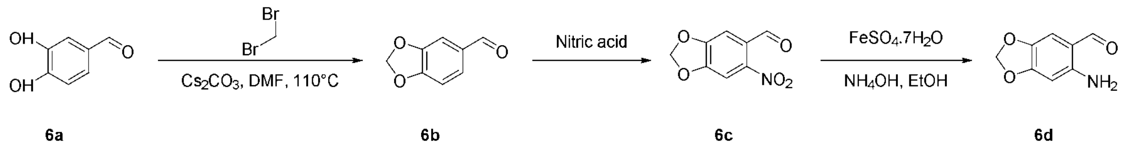


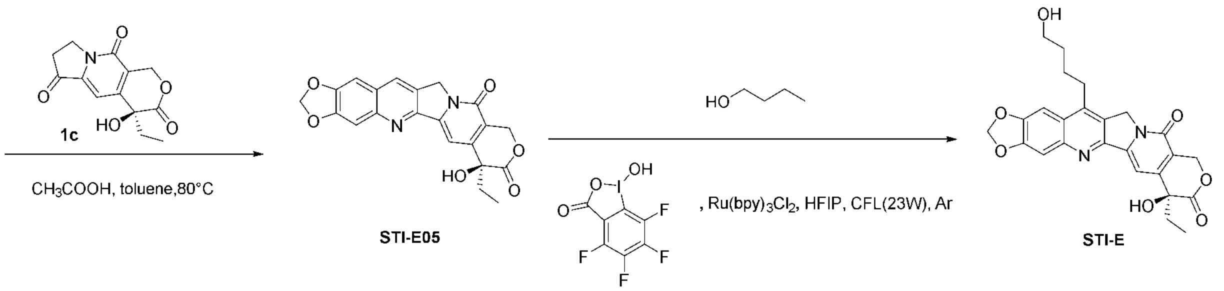


Step 1: Under a nitrogen atmosphere, compound 6a (1 g, 7.24 mmol) was added to a reaction flask and then 15 mL DMF was added for dissolution. Cesium carbonate (3.54 g, 10.86 mmol) and dibromomethane (1.89 g, 10.86 mmol) were added. The mixture was heated to 110°C and stirred for 2.5 hours. The reaction was stopped after TLC (PE:DCM = 1:1, product Rf = 0.7) monitoring showed that the reaction was complete. Purification by prep-TLC afforded 972 mg of compound 6b in 89.4% yield.

Step 2: Compound 6b (972 mg, 6.48 mmol) was added to the reaction flask, followed by 20 mL nitric acid. The reaction was carried out at RT under a nitrogen atmosphere for 0.5 hour. After completion of the reaction confirmed by TLC (PE:EA = 5:1, product Rf = 0.5), ice water was added to the reaction system. The mixture was allowed to stand and then filtered. The filter cake was dried under vacuum at RT to afford 1.053 g of compound 6c as a yellow solid in 83.3% yield. $^1H$ NMR (400 MHz, $CDCl_3$) $\delta$ 10.22 (s, 1H), 7.45 (s, 1H), 7.26 (s, 1H), 6.14 (s, 2H).

Step 3: Compound 6c (7.0 g, 35.87 mmol) was dissolved in 350 mL 50% aqueous ethanol and heated to 100°C; $FeSO_4 \cdot 7H_2O$ (70 g, 251.09 mmol) was dissolved in 350 mL water, heated at 100°C for 2 minutes, and then added to the reaction system. Concentrated aqueous ammonia (91.0 mL) was added slowly. The mixture was heated at 100°C for 15 minutes. After the reaction was confirmed to be complete by TLC (PE:EA = 5:1, product Rf = 0.6) and LCMS, the mixture was allowed to warm to RT, filtered, and cooled in an ice bath, precipitating 4.5 g of a yellow needle-like product (compound 6d) with a yield of 76.0%. LCMS (254 nm) purity 96.61%, Rt = 1.40 min; MS Calculated: 165.0; MS Found: 166.1 $[M+H]^+$. $^1H$ NMR (400 MHz, $CDCl_3$) $\delta$ 9.61 (d, $J$ = 0.6 Hz, 1H), 6.82 (s, 1H), 6.27 (s, 2H), 6.14 (s, 1H), 5.93 (s, 2H).

Step 4: Compound 6d (300 mg, 1.82 mmol) and compound 1c (360 mg, 1.37 mmol) were suspended in 4 mL toluene, and 4 mL acetic acid was added. The mixture was heated at 80°C overnight under a nitrogen atmosphere. Upon confirmation of completion by LCMS, the mixture was cooled to RT, filtered, and washed with acetone to afford 45 mg of compound STI-E05 as a yellowish solid in 45.9% yield. LCMS (254 nm) purity 99.62%, Rt = 1.49 min; MS Calculated: 392.1; MS Found: 393.2 $[M+H]^+$. $^1H$ NMR (400 MHz, DMSO-$d_6$) $\delta$ 8.46 (s, 1H), 7.51 (s, 2H), 7.25 (s, 1H), 6.50 (s, 1H), 6.28 (dd, $J$ = 1.4, 0.7 Hz, 2H), 5.41 (s, 2H), 5.21 (s, 2H), 1.92 - 1.80 (m, 2H), 0.87 (t, $J$ = 7.3 Hz, 3H).

Step 5: Compound STI-E05 (100 mg, 0.26 mmol), n-butanol (58 mg, 0.78 mmol), tris(2,2'-bipyridine)ruthenium(II) chloride hexahydrate (1.6 mg, 0.0026 mmol), and 4,5,6,7-tetrafluoro-1-hydroxy-1$\lambda^3$-benzo[d][1,2]iodoxol-3(1H)-one (175 mg, 0.52 mmol) were added to a reaction flask, suspended in 5 mL hexafluoroisopropanol (HFIP) and purged with argon for 1 hour. Under an argon atmosphere, the reaction was heated to 70°C and stirred under irradiation with a 23 W energy-saving lamp for 65 hours. When LCMS monitoring indicated approximately 20% conversion, the reaction was stopped. The mixture was subjected to low-to-medium pressure reverse-phase prep-LC (basic mobile phase) and lyophilized to afford 20 mg of compound STI-E as a brownish-yellow solid. LCMS (254 nm) purity 94.36%, Rt = 1.75 min; MS Calculated: 464.2; MS Found: 465.2 $[M+H]^+$. $^1H$ NMR (400 MHz, DMSO-$d_6$) $\delta$ 7.55 (s, 1H), 7.50 (s, 1H), 7.30 (s, 1H), 6.51 (s, 1H), 6.36 (t, $J$ = 6.2 Hz, 1H), 6.29 (s, 2H), 5.42 (s, 2H), 5.26 (s, 2H), 3.92 - 3.82 (m, 2H), 3.79 (d, $J$ = 6.7 Hz, 2H), 1.92 - 1.81 (m, 4H), 1.38 - 1.26 (m, 2H), 0.84 (t, $J$ = 6.8 Hz, 3H).

**Example 8: Synthesis of (S)-4-ethyl-4-hydroxy-8-fluoro-9-methyl-1,12-dihydro-14H-pyrano[3',4':6,7]indolizino [1,2-b]q uinoline-3,14(4H)-dione (STI-F-8)**

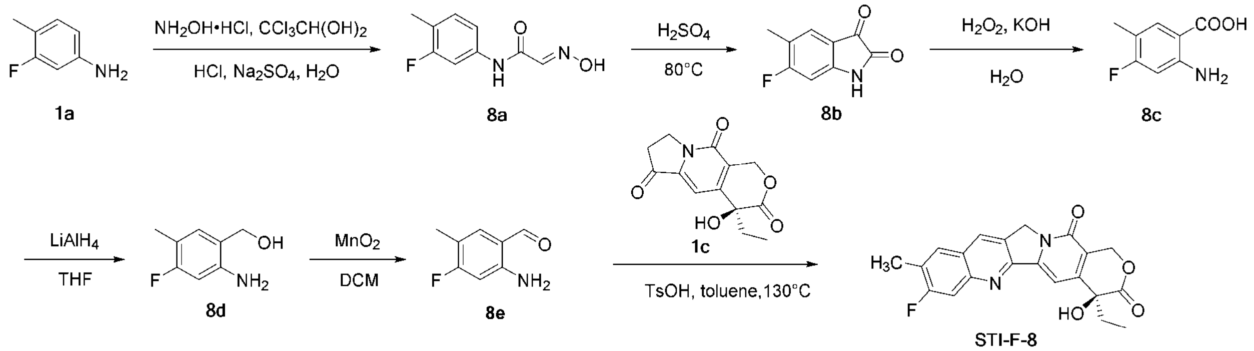


1a 8a 8b 8c 1c 8d 8e STI-F-8

Step 1: $Na_2SO_4$ (22.7 g, 159.81 mmol) and chloral hydrate (3.0 g, 17.58 mmol) were added to a three-necked flask, followed by 67 mL water. Compound 1a (2.0 g, 15.98 mmol), hydroxylamine hydrochloride (4.4 g, 63.92 mmol), and 25 mL 1 M hydrochloric acid were then added. The mixture was heated at 80°C overnight with stirring under a nitrogen atmosphere. After the reaction was complete, the mixture was filtered, and the filter cake was dried at 50°C to afford 2.9 g of compound 8a as a yellow solid in 92.5% yield. LCMS (254 nm) purity 97.76%, Rt = 1.522 min; MS Calculated: 196.1; MS Found: 197.1 $[M+H]^+$. $^1H$ NMR (400 MHz, DMSO- $d_6$) δ 12.21 (s, 1H), 10.28 (s, 1H), 7.64 (s, 1H), 7.61 (dd, $J$ = 12.3, 2.0 Hz, 1H), 7.36 (dd, $J$ = 8.3, 2.0 Hz, 1H), 7.22 (t, $J$ = 8.6 Hz, 1H), 2.19 (d, $J$ = 1.9 Hz, 3H).

Step 2: Compound 8a (2.9 g, 14.79 mmol) was dissolved in 10 mL concentrated sulfuric acid. The mixture was heated at 80°C with stirring for 3 hours, and LCMS monitoring indicated complete consumption of the starting material. The reaction mixture was cooled to RT. Under an ice bath, 200 mL ice water was added to the reaction system. The mixture was filtered, and the filter cake was washed with water until the filtrate reached neutral pH. The filter cake was dried at 50°C to afford 2.07 g of compound 8b as an orange-yellow solid in 78.2% yield. $^1H$ NMR (400 MHz, DMSO-$d_6$) δ 11.06 (s, 1H), 7.51 (d, $J$ = 7.7 Hz, 1H), 6.69 (d, $J$ = 9.8 Hz, 1H), 2.16 (d, $J$ = 2.2 Hz, 3H).

Step 3: Compound 8b (2.07 g, 11.6 mmol) and KOH (1.3 g, 23.2 mmol) were dissolved in 20 mL water. Under an ice bath, 590 mg of $H_2O_2$ was added dropwise, and the mixture was stirred at RT for 3 hours. After the reaction was complete, the pH was adjusted to 4~6 with hydrochloric acid under an ice bath. The mixture was filtered, washed, and dried at 50°C to afford 660 mg of compound 8c as a yellow solid. LCMS (254 nm) purity 94.31%, Rt = 1.065 min; MS Calculated: 169.1; MS Found: 170.1 $[M+H]^+$. $^1H$ NMR (400 MHz, DMSO-$d_6$) δ 7.61 (d, $J$ = 9.2 Hz, 1H), 6.49 (d, $J$ = 12.3 Hz, 1H), 2.07 (s, 3H).

Step 4: To a reaction tube were added 10 mL tetrahydrofuran. Under nitrogen atomsphore cooling to 0°C in an ice bath, $LiAlH_4$ (352 mg, 9.28 mmol) was added. A solution of compound 8c (100 mg, 0.6 mmol) in tetrahydrofuran (10 mL) was then added dropwise. After the addition, the mixture was allowed to warm to RT naturally, and the reaction was carried out overnight. Upon completion of the reaction, the mixture was cooled below 10°C in an ice bath and quenched by adding water slowly. The mixture was extracted with ethyl acetate, washed with saturated sodium chloride solution, dried over anhydrous $Na_2SO_4$, filtered, and subjected to rotary evaporation to afford 510 mg of compound 8d as a brownish-yellow solid in 82.9% yield. LCMS (254 nm) purity 88.5%, Rt = 1.375 min; MS Calculated: 155.1; MS Found: 156.1 $[M+H]^+$. $^1H$ NMR (400 MHz, DMSO-$d_6$) δ 6.90 (d, $J$ = 9.0 Hz, 1H), 6.36 (d, $J$ = 12.2 Hz, 1H), 4.94 (d, $J$ = 5.5 Hz, 3H), 4.32 (d, $J$ = 4.7 Hz, 2H), 2.05 (s, 3H).

Step 5: Compound 8d (510 mg, 3.29 mmol) was dissolved in 10 mL dichloromethane. $MnO_2$ (1.5 g, 17.11 mmol) was added, and the mixture was stirred at RT overnight. After the reaction was complete, the mixture was filtered, washed, subjected to rotary evaporation and purified by column chromatography to afford 464 mg of compound 8e as a yellowish solid in 92.2% yield. LCMS (254 nm) purity 84.82%, Rt = 1.787 min; MS Calculated: 153.1; MS Found: 154.1 $[M+H]^+$. $^1H$ NMR (400 MHz, $CDCl_3$) δ 9.69 (s, 1H), 7.21 (d, $J$ = 8.4 Hz, 1H), 6.23 (d, $J$ = 11.6 Hz, 1H), 6.17 - 5.88 (m, 2H), 2.11 (d, $J$ = 1.8 Hz, 3H).

Step 6: Compound 8e (466 mg, 3.04 mmol) and compound 1c (600 mg, 2.28 mmol) were dissolved in 28 mL toluene. Under a nitrogen atmosphere, the mixture was heated to 130°C and stirred under reflux with a water separator for 30 minutes. Then, TsOH (131 mg, 0.76 mmol) was added, and stirring was continued while monitoring the reaction progress by LCMS. After completion, the system was cooled to RT, filtered, and washed with acetone. The filtrate was concentrated and subjected to low-to-medium pressure reverse-phase prep-LC (basic mobile phase). The target fractions were collected and lyophilized to afford 350 mg of the title compound (STI-F-8) as a yellowish solid in 40.4% yield. LCMS (254 nm) purity 94.07%, Rt = 1.589 min; MS Calculated: 380.1; MS Found: 381.1 $[M+H]^+$. $^1H$ NMR (400 MHz, DMSO-$d_6$) δ 8.61 (s, 1H), 8.05 (d, $J$ = 8.5 Hz, 1H), 7.88 (d, $J$ = 10.9 Hz, 1H), 7.32 (s, 1H), 6.53 (s, 1H), 5.42 (s, 2H), 5.25 (s, 2H), 2.48 (s, 3H), 1.92 - 1.80 (m, 2H), 0.88 (t, $J$ = 7.4 Hz, 3H).

**Example 9: Synthesis of (S)-N-(4-ethyl-4-hydroxy-8-fluoro-9-methyl-3,14-dioxo-3,4,12,14-tetrahydro-1H-pyrano [3',4':6,7]indolizino[1,2-b]quinolin-11-yl)-2-hydroxyacetamide (STI-F2), and**

**Example 10: Synthesis of (S)-4-ethyl-4-hydroxy-8-fluoro-9-methyl-11-amino-1,12-dihydro-14H-pyrano[3',4':6,7] indolizino[1,2-b]quinoline-3,14(4H)-dione (STI-F-03c-1)**

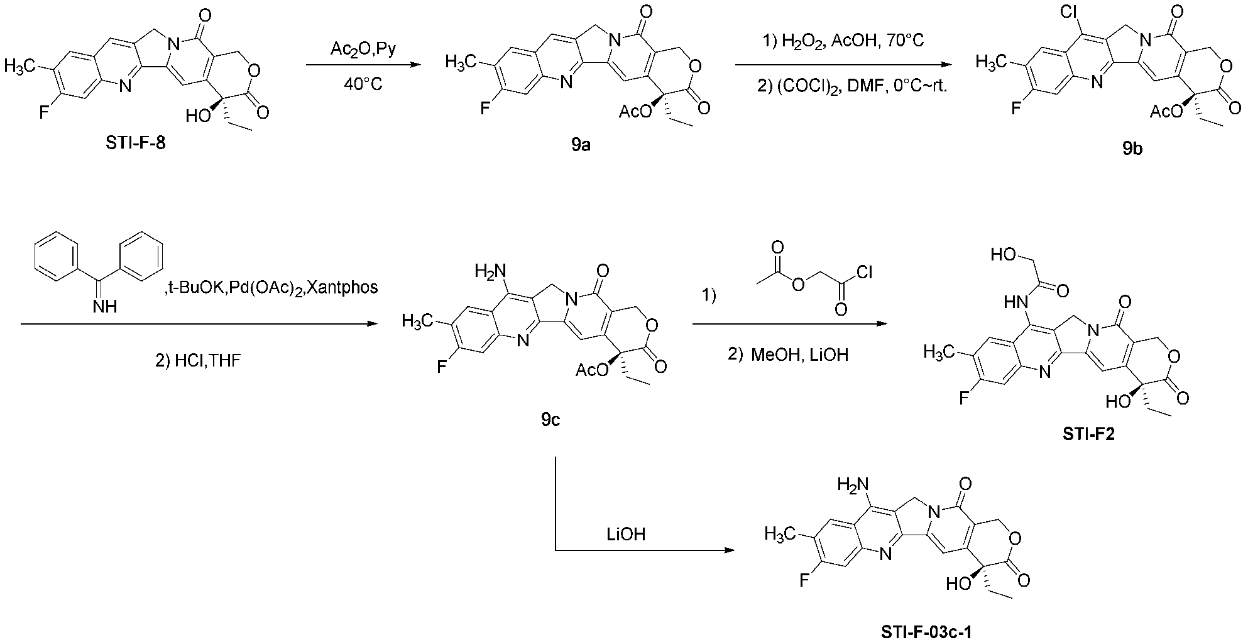


STI-F-8, 9a, 9b, 9c, STI-F2, STI-F-03c-1

Step 1: At RT, 3.0 mL acetic anhydride and 2.4 mL pyridine were mixed uniformly. Compound STI-F-8 (300 mg, 0.79 mmol) was added, and the mixture was heated to 40°C with stirring. The reaction was monitored by LCMS. After completion, saturated NaCl was added, and the mixture was extracted with acetonitrile. The organic phase was concentrated and subjected to low-to-medium pressure reverse-phase prep-LC (basic mobile phase). The target fractions were collected and lyophilized to afford 117 mg of compound 9a as a yellowish-brown solid in 35.1% yield. LCMS (254 nm) purity 97.16%, Rt = 1.698 min; MS Calculated: 422.1; MS Found: 423.2 $[M+H]^+$. $^1$H NMR (400 MHz, $CDCl_3$) δ 8.23 (s, 1H), 7.77 - 7.64 (m, 2H), 7.12 (s, 1H), 5.60 (d, *J* = 17.2 Hz, 1H), 5.33 (d, *J* = 17.2 Hz, 1H), 5.19 (t, *J* = 1.5 Hz, 2H), 2.46 (t, *J* = 1.4 Hz, 3H), 2.21 (dd, *J* = 13.9, 7.5 Hz, 1H), 2.15 (s, 3H), 2.08 (dd, *J* = 13.9, 7.5 Hz, 1H), 0.91 (t, *J* = 7.5 Hz, 3H).

Step 2: Compound 9a (480 mg, 1.14 mmol) was dissolved in 5 mL acetic acid. A 30% aqueous hydrogen peroxide solution (6 mL) was added, and the mixture was heated at 70°C with stirring for 3.5 hours. The mixture was extracted with DCM, dried over anhydrous $Na_2SO_4$, filtered, and subjected to rotary evaporation. 24 mL DMF was added for dissolution. After cooling to 0°C in an ice bath, 1.5 mL oxaloyl chloride was slowly added dropwise. After the addition, the mixture was stirred at RT, with the reaction being monitored by LCMS. After the reaction was complete, the mixture was quenched with water, extracted with DCM, and subjected to rotary evaporation and low-to-medium pressure reverse-phase prep-LC (basic mobile phase). The target fractions were collected and lyophilized to afford 420 mg of compound 9b as a pink solid in 81.0% yield. LCMS (254 nm) purity 97.37%, Rt = 2.014 min; MS Calculated: 456.1; MS Found: 457.0 $[M+H]^+$. $^1$H NMR (400 MHz, DMSO-$d_6$) δ 8.24 (d, *J* = 8.0 Hz, 1H), 7.98 (d, *J* = 10.6 Hz, 1H), 7.05 (s, 1H), 5.50 (d, *J* = 4.2 Hz, 2H), 5.28 (d, *J* = 4.7 Hz, 2H), 2.54 (s, 3H), 2.22 (s, 3H), 2.18 - 2.12 (m, 2H), 0.91 (t, *J* = 7.4 Hz, 3H).

Step 3: Compound 9b (240 mg, 0.52 mmol), t-BuOK (128 mg, 1.04 mmol), Pd(OAc)$_2$ (16 mg, 0.07 mmol), Xantphos (30 mg, 0.05 mmol), and benzophenone imine (104 mg, 0.58 mmol) were added to a reaction tube. The tube was purged with nitrogen three times, then 16 mL super-dry toluene was added. The mixture was stirred at 100°C for 6 hours. The reaction solution was subjected to rotary evaporation, dissolved in added acetonitrile and subjected to low-to-medium pressure reverse-phase prep-LC (basic mobile phase). The target fractions were collected and lyophilized to afford the coupled product. To the coupled product (90 mg) were added with 15 mL THF and 2 mL 6 M hydrochloric acid, and the mixture was stirred at RT overnight. The reaction solution was concentrated and subjected to low-to-medium pressure reverse-phase prep-LC (basic mobile phase). The target fractions were collected and lyophilized to afford 50 mg of compound 9c as a yellowish solid; a two-step total yield of 21.7%. LCMS (254 nm) purity 90.36%, Rt = 1.730 min; MS Calculated: 437.1; MS Found: 438.2 $[M+H]^+$. $^1$H NMR (400 MHz, DMSO-$d_6$) δ 8.25 (d, *J* = 8.3 Hz, 1H), 7.58 (d, *J* = 11.4 Hz, 1H), 7.38 (s, 2H), 6.87 (s, 1H), 5.46 (s, 2H), 5.08 - 4.97 (m, 2H), 2.43 (s, 3H), 2.20 (s, 3H), 2.15 - 2.07 (m, 2H), 0.90 (t, *J* = 7.4 Hz, 3H).

Step 4: Compound 9c (50 mg, 0.011 mmol) and DMAP (1 mg, cat.) were added to a reaction flask under a nitrogen

atmosphere. Super-dry DMF (1.5 mL) was added, followed by the sequential addition of DIPEA (29 mg, 0.023 mmol) and acetoxyacetyl chloride (31 mg, 0.023 mmol). The mixture was reacted at RT for 2 hours, then heated to 55°C for 3 hours, and stirred at RT overnight. When LCMS showed the reaction was approximately 50% complete, the reaction was stopped. The system was concentrated to dryness under reduced pressure. Methanol (4 mL) and LiOH (50 mg) were added, and the mixture was stirred at RT for about 30 minutes. The pH was adjusted to 2-3 with acetic acid, and the system was concentrated to dryness, subjected to low-to-medium pressure reverse-phase prep-LC (basic mobile phase) and lyophilized to afford approximately 7.8 mg of compound STI-F2 as a yellowish solid. The remaining compound 9c in the reaction mixture underwent LiOH-mediated hydrolysis of the acetyl group to yield approximately 8.3 mg of compound STI-F-03c-1 as a yellowish solid.

Compound STI-F2: LCMS (254 nm) purity 97.89%, Rt = 1.51 min; MS Calculated: 453.1; MS Found: 454.2 $[M+H]^+$. 1H NMR (400 MHz, DMSO- $d_6$) δ 10.65 (s, 1H), 8.13 (d, *J* = 8.0 Hz, 1H), 7.90 (d, *J* = 10.8 Hz, 1H), 7.32 (s, 1H), 5.81 (br, 1H), 5.43 (s, 2H), 5.12 (s, 2H), 4.30 (s, 2H), 1.92-1.81 (m, 2H), 0.88 (t, *J* = 7.6 Hz, 3H).

Compound STI-F-03c-1: LCMS (254 nm) purity 97.67%, Rt = 1.60 min; MS Calculated: 395.1; MS Found: 396.2 $[M+H]^+$. $^1$H NMR (400 MHz, DMSO-$d_6$) δ 8.76-8.58 (m, 2H), 8.38 (d, *J* = 8.0 Hz, 1H), 7.60 (d, *J* = 10.84 Hz, 1H), 7.46-7.42 (m, 1H), 6.61 (br, 1H), 5.44 (s, 2H), 5.05(s, 2H), 2.44 (s, 3H), 1.93 - 1.78(m, 2H), 0.87 (t, *J* = 7.2 Hz, 3H).

**Example 11: Synthesis of (S)-4-ethyl-4-hydroxy-8-fluoro-9-amino-11-pentyl-1,12-dihydro-14H-pyrano[3',4':6,7] indolizi no[1,2-b]quinoline-3,14(4H)-dione (STI-G1)**

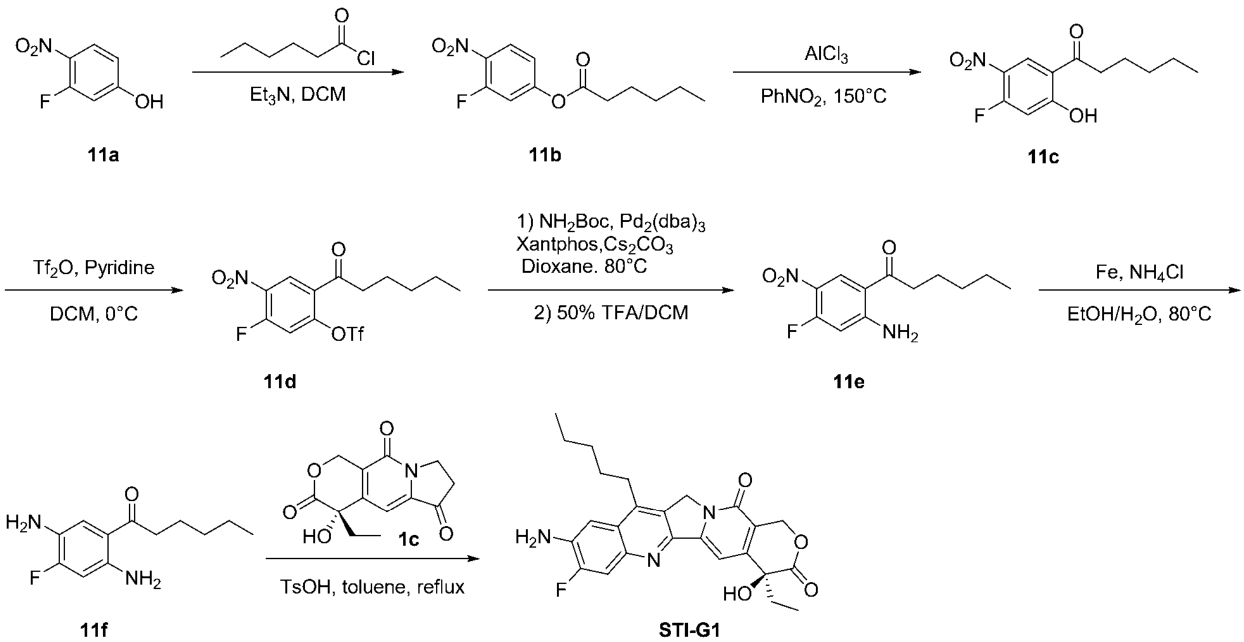


11a 11b 11c 11d 11e 1c 11f STI-G1

Step 1: Under a nitrogen atmosphere and in an ice bath, compound 11a (500 mg, 3.18 mmol) was dissolved in 30 mL DCM. With stirring at 0°C, hexanoyl chloride (0.67 mL, 4.77 mmol) and triethylamine (0.88 mL, 6.36 mmol) were added to a reaction flask. The reaction was continued at this temperature for 30 minutes. After TLC (PE:EA=30:1, product Rf=0.5) confirmed the completion of reaction, 50 mL dichloromethane and 50 mL water were added, and the mixture was extracted twice. The organic phase was dried over anhydrous $Na_2SO_4$ and concentrated under reduced pressure. The resulting brown crude product was purified by silica gel column chromatography (PE:EA=10:1) to afford 650 mg of compound 11b as a colorless liquid in 80.9% yield. $^1$H NMR (400 MHz, DMSO-$d_6$) δ 8.25 (t, *J* = 8.9 Hz, 1H), 7.54 (dd, *J* = 12.1, 2.4 Hz, 1H), 7.27 (dt, *J* = 9.1, 1.8 Hz, 1H), 2.62 (t, *J* = 7.4 Hz, 2H), 1.64 (dd, *J* = 10.0, 4.8 Hz, 2H), 1.33 (q, *J* = 3.7 Hz, 4H), 0.89 (t, *J* = 6.9 Hz, 3H).

Step 2: Compound 11b (500 mg, 1.96 mmol) was dissolved in 4 mL nitrobenzene. Under a nitrogen atmosphere, aluminum trichloride (290 mg, 2.16 mmol) was added, and the reaction was carried out at 150°C for 5 hours. After TLC (PE:EA=30:1, product Rf=0.5) confirmed the completion of reaction, the reaction system was cooled to RT and poured into 50 mL ice water containing 1 N HCl. The mixture was stirred at RT for 30 minutes, and extracted with 50 mL ethyl acetate. The aqueous phase was further extracted twice with ethyl acetate. The organic phases were combined, dried over anhydrous $Na_2SO_4$ and concentrated under reduced pressure. The resulting brown crude product was purified by silica

gel column chromatography (PE:EA=200:1 to remove the nitrobenzene solvent, then PE:EA=20:1 to elute the product), and subjected to rotary evaporation, giving 300 mg of compound 11c as a yellow solid in 60% yield. $^1$H NMR (400 MHz, DMSO-$d_6$) δ 12.66 (s, 1H), 8.54 (d, *J* = 9.0 Hz, 1H), 7.04 (d, *J* = 13.0 Hz, 1H), 3.06 (t, *J* = 7.2 Hz, 2H), 1.60 (dd, *J* = 8.5, 5.9 Hz, 2H), 1.33 - 1.28 (m, 4H), 0.89 - 0.85 (m, 3H).

Step 3: Compound 11c (500 mg, 1.96 mmol) was dissolved in 20 mL dichloromethane. Under an ice bath and a nitrogen atmosphere, pyridine (0.63 mL, 7.84 mmol) and $Tf_2O$ (0.99 mL, 5.88 mmol) were added to the reaction system. The reaction was carried out at 0°C for 30 minutes. After the completion of the reaction was confirmed by TLC (PE:EA=20:1, product Rf=0.5), the reaction system was diluted with 20 mL dichloromethane. Water was added for extraction, and the aqueous phase was further extracted twice with 10 mL dichloromethane. The organic phases were combined, dried over anhydrous $Na_2SO_4$ and concentrated under reduced pressure. The resulting brown crude product was purified by silica gel column chromatography (PE:EA=20:1), and subjected to rotary evaporation, giving 650 mg of compound 11d as a yellow solid in 85.7% yield. $^1$H NMR (400 MHz, $CDCl_3$) δ 8.55 (d, *J* = 7.9 Hz, 1H), 7.36 (d, *J* = 9.9 Hz, 1H), 2.96 (t, *J* = 7.3 Hz, 2H), 1.75 (dd, *J* = 9.1, 5.6 Hz, 2H), 1.36 (q, *J* = 3.9 Hz, 4H), 0.93 (d, *J* = 6.8 Hz, 3H).

Step 4: Compound 11d (1.12 g, 2.90 mmol) was dissolved in 28 mL 1,4-dioxane. Under a nitrogen atmosphere, tert-butyl carbamate (680 mg, 5.80 mmol), Xantphos (336 mg, 0.58 mmol), $Pd_2(dba)_3$ (266 mg, 0.29 mmol), and cesium carbonate (1.89 g, 5.80 mmol) were added to the reaction mixture. The system was purged with nitrogen three times and then reacted at 80°C for 1 hour. After the completion of the reaction was confirmed by TLC (PE:EA=10:1, product Rf=0.6), the reaction was cooled to RT. The reaction system was diluted with 20 mL ethyl acetate, and extracted with water. The aqueous phase was further extracted twice with 10 mL ethyl acetate. The organic phases were combined, dried over anhydrous $Na_2SO_4$ and concentrated under reduced pressure. The resulting brown crude product was purified by silica gel column chromatography (PE:EA=50:1), and subjected to rotary evaporation, giving 440 mg of a yellow liquid. This yellow liquid was dissolved in 12 mL a 50% TFA/DCM solution and reacted at RT for 30 minutes. After the completion of the reaction was confirmed by TLC (PE:EA=5:1, product Rf=0.3), the reaction system was diluted with 20 mL dichloromethane and extracted with water. The aqueous phase was further extracted twice with 10 mL dichloromethane. The organic phases were combined, dried over anhydrous $Na_2SO_4$ and concentrated under reduced pressure. The resulting brown crude product was purified by silica gel column chromatography (PE:EA=5:1), and subjected to rotary evaporation, giving 260 mg of compound 11e as a yellowish solid in 35.3% yield. LCMS (254 nm): Rt = 2.108 min; MS Calculated: 254.1; MS Found: 253.1 $[M-H]^-$. $^1$H NMR (400 MHz, DMSO-$d_6$) δ 8.64 (d, *J* = 8.7 Hz, 1H), 8.53 - 7.43 (m, 2H), 6.67 (d, *J* = 14.4 Hz, 1H), 3.01 (t, *J* = 7.3 Hz, 2H), 1.59 (t, *J* = 7.2 Hz, 2H), 1.31 (dq, *J* = 7.6, 3.4 Hz, 4H), 0.92 - 0.83 (m, 3H).

Step 5: Compound 11e (200 mg, 0.79 mmol) was dissolved in 12 mL a 3:1 ethanol/water mixed solvent. Under a nitrogen atmosphere, iron powder (176 mg, 3.16 mmol) and ammonium chloride (44 mg, 0.79 mmol) were added. The system was purged with nitrogen three times and then heated under reflux at 80°C for 1 hour. After the completion of the reaction was confirmed by TLC (PE:EA=5:1, product Rf=0.2), the reaction was cooled to RT and the reaction solvent was removed by rotary evaporation. The crude product was purified by silica gel column chromatography (PE:EA=2:1), and subjected to rotary evaporation, giving 160 mg of compound 11f as a brown solid in 90% yield. LCMS (254 nm): Rt = 2.189 min; MS Calculated: 224.1; MS Found: 225.2 $[M+H]^+$. $^1$H NMR (400 MHz, DMSO-$d_6$) δ 7.25 (d, *J* = 10.1 Hz, 1H), 6.70 (s, 2H), 6.45 (d, *J* = 13.5 Hz, 1H), 4.39 (s, 2H), 2.78 (t, *J* = 7.4 Hz, 2H), 1.57 (t, *J* = 7.3 Hz, 2H), 1.30 (tt, *J* = 5.9, 3.2 Hz, 4H), 0.87 (t, *J* = 6.8 Hz, 3H).

Step 6: Compound 11f (20 mg, 0.09 mmol) and compound 1c (24 mg, 0.09 mmol) were dissolved in 1 mL anhydrous toluene. Under a nitrogen atmosphere, the mixture was refluxed with a water separator for 30 minutes. Then, TsOH (4 mg, 0.023 mmol) was added, and the mixture was stirred under reflux for 3.5 hours. After the completion of the reaction was confirmed by TLC (DCM:MeOH = 30:1, product Rf=0.4) and LCMS, the reaction system was cooled to RT, filtered, and washed with acetone. The crude product was purified by Prep-TLC to afford 5 mg of the title compound (STI-G1) as a yellow solid. LCMS (254 nm): Rt = 1.839 min; MS Calculated: 451.2; MS Found: 452.3 $[M+H]^+$. $^1$H NMR (400 MHz, DMSO-$d_6$) δ7.74 (d, *J* = 12.3 Hz, 1H), 7.30 (d, *J* = 9.5 Hz, 1H), 7.19 (s, 1H), 6.46 (s, 1H), 6.06 (s, 2H), 5.40 (s, 2H), 5.21 (s, 2H), 3.00 (t, *J* = 8.1 Hz, 2H), 2.05 - 1.94 (m, 2H), 1.91 - 1.79 (m, 2H), 1.73 - 1.62 (m, 2H), 1.48 - 1.40 (m, 2H), 0.90 (t, *J* = 6.0 Hz, 3H), 0.87 (t, *J* = 5.9 Hz, 3H).

**Example 12: Synthesis of (S)-4-ethyl-4-hydroxy-8-fluoro-9-nitro-11-pentyl-1,12-dihydro-14H-pyrano[3',4':6,7] indolizin o[1,2-b]quinoline-3,14(4H)-dione (STI-G03)**

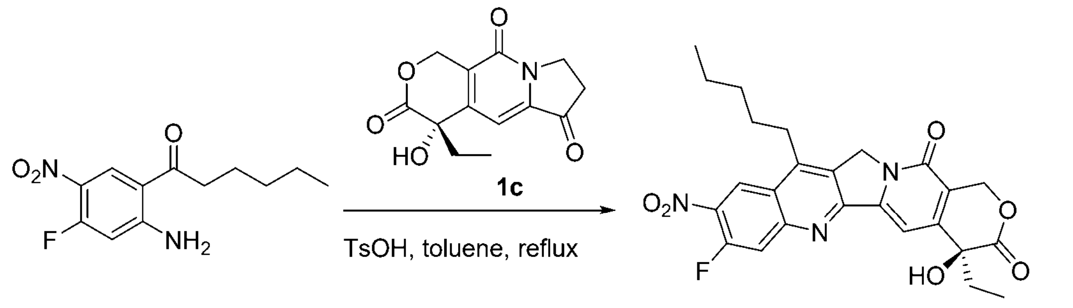


11e

STI-G03

Compound 11e (20 mg, 0.078 mmol) and compound 1c (20 mg, 0.078 mmol) were dissolved in 1 mL anhydrous toluene. Under a nitrogen atmosphere, the mixture was refluxed with a water separator for 30 minutes. Then, TsOH (3.5 mg, 0.02 mmol) was added, and the mixture was stirred under reflux for 3.5 hours. After the completion of the reaction was confirmed by TLC (DCM:MeOH=30:1, product Rf=0.6) and LCMS, the reaction system was cooled to RT, filtered, and washed with acetone. The crude product was purified by Prep-TLC to afford 8 mg of the title compound (STI-G03) as a yellow solid in 21% yield. LCMS (254 nm) purity 93.78%, Rt = 2.01 min; MS Calculated: 481.2; MS Found: 482.2 $[M+H]^+$. $^1H$ NMR (400 MHz, DMSO-$d_6$) δ 9.05 (d, *J* = 7.9 Hz, 1H), 8.26 (d, *J* = 12.2 Hz, 1H), 7.38 (s, 1H), 6.57 (s, 1H), 5.45 (s, 2H), 5.35 (s, 2H), 3.30 - 3.25 (m, 2H), 1.92 - 1.82 (m, 2H), 1.72 (t, *J* = 7.7 Hz, 2H), 1.49 - 1.44 (m, 2H), 1.38 (t, *J* = 7.1 Hz, 2H), 0.88 (d, *J* = 8.1 Hz, 6H).

**Example 13: Synthesis of (S)-9-bromo-4-ethyl-8-fluoro-4-hydroxy-11-((propylthio)methyl)-1,12-dihydro-14H-pyrano[3', 4':6,7]indolizino[1,2-b]quinoline-3,14(4H)-dione (STI-G2-06c)**

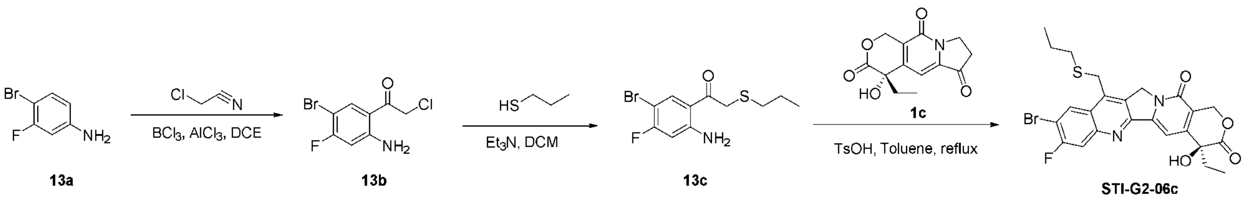


13a 13b 13c STI-G2-06c

Step 1: Under a nitrogen atmosphere and in an ice bath, 70 mL DCE and 1 M boron trichloride in DCM (12.5 mL, 12.5 mmol) were added to a reaction flask, followed by addition of compound 13a (3.0 g, 15.6 mmol). The mixture was stirred at 0°C for 10 minutes. Chloroacetonitrile (1416 mg, 18.7 mmol) and aluminum trichloride (2706 mg, 20.3 mmol) were then added under the ice bath. The reaction system was slowly warmed to RT, stirred for 10 minutes, and then heated under reflux with stirring for 39 hours. After completion, the system was poured into 100 mL ice water, followed by the addition of 40 mL 2 M HCl. The mixture was stirred at RT for 1 hour. The mixture was extracted with 100 mL dichloromethane. The organic phase was dried over anhydrous $Na_2SO_4$ and concentrated under reduced pressure. The residue was subjected to medium-to-high pressure reverse-phase prep-LC (basic mobile phase) and lyophilized to afford 1.1 g of compound 13b as a yellow solid powder in 26.2% yield. LCMS (254 nm) purity 100%, Rt = 1.93 min; MS Calculated: 264.9; MS Found: 263.9 $[M-H]^-$. $^1H$ NMR (400 MHz, DMSO-$d_6$) δ 8.03 (d, *J* = 7.9 Hz, 1H), 7.56 (s, 2H), 6.74 (d, *J* = 11.6 Hz, 1H), 5.05 (s, 2H).

Step 2: Compound 13b (250 mg, 0.94 mmol) was dissolved in 5 mL dichloromethane under a nitrogen atmosphere. Propanethiol (94 μL, 1.03 mmol) and triethylamine (261 μL, 1.88 mmol) were added, and the mixture was stirred at RT overnight. After the completion of the reaction was confirmed by TLC (PE:EA=5:1, product Rf=0.3), the mixture was washed with water and dried. Purification by Pre-TLC afforded 218 mg of compound 13c as a yellowish solid in 75.7% yield.. $^1H$ NMR (400 MHz, DMSO-$d_6$) δ 8.08 (d, *J* = 8.0 Hz, 1H), 7.53 (s, 2H), 6.71 (d, *J* = 11.5 Hz, 1H), 3.90 (s, 2H), 2.47 (d, *J* = 7.3 Hz, 2H), 1.54 (h, *J* = 7.3 Hz, 2H), 0.91 (t, *J* = 7.3 Hz, 3H).

Step 3: Compound 13c (187 mg, 0.61 mmol) and compound 1c (121 mg, 0.46 mmol) were dissolved in 10 mL anhydrous toluene. Under a nitrogen atmosphere, the mixture was refluxed with a water separator for 30 minutes. Then, TsOH (11 mg, 0.06 mmol) was added, and the mixture was stirred under reflux for 4 hours. Upon confirmation of completion by LCMS, the reaction system was cooled to RT, filtered, washed with acetone, and purified by Pre-TLC (DCM:MeOH = 15:1) to afford 230 mg of compound STI-G2-06c as a yellow solid in 94.0% yield. Rt = 2.09 min; MS Calculated: 532.1; MS Found: 533.1 $[M+H]^+$. $^1H$ NMR (400 MHz, DMSO-$d_6$) δ 8.79 (d, *J* = 7.5 Hz, 1H), 8.13 (d, *J* = 9.8 Hz, 1H), 7.33 (s, 1H), 6.54 (s, 1H), 5.44 (s, 2H), 5.33 (s, 2H), 4.45 (s, 2H), 2.05 - 1.95 (m, 2H), 1.92 - 1.81 (m, 2H), 1.63 - 1.52 (m, 2H), 0.93 - 0.88 (m, 3H),

0.87 - 0.82 (m, 3H).

**Example 14: Synthesis of (S)-9-amino-4-ethyl-8-fluoro-4-hydroxy-11-((propylthio)methyl)-1,12-dihydro-14H-pyrano[3', 4':6,7]indolizino[1,2-b]quinoline-3,14(4H)-dione (STI-G2)**

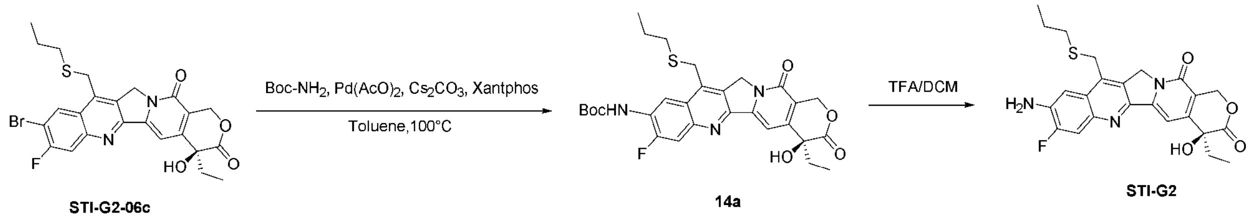


STI-G2-06c 14a STI-G2

Step 1: Under a nitrogen atmosphere, the compound STI-G2-06c (53 mg, 0.10 mmol) from Example 13 and tert-butyl carbamate (23 mg, 0.20 mmol) were dissolved in 5 mL anhydrous toluene. Cesium carbonate (65 mg, 0.20 mmol), Xantphos (5.8 mg, 0.01 mmol), and palladium acetate (2.2 mg, 0.01 mmol) were added under nitrogen. The mixture was stirred thoroughly and heated to 100°C for 2.0 hours. After the completion of the reaction was confirmed by TLC (DCM:MeOH=15:1, product Rf=0.5), the mixture was concentrated to dryness under reduced pressure. The residue was extracted with ethyl acetate and water. The organic phase was concentrated to afford 40 mg of compound 14a as a yellow solid in 70.5% yield. LCMS (254 nm) purity 98.42%, Rt = 2.07 min; MS Calculated: 569.2; MS Found: 570.3 $[M+H]^+$.

Step 2: Compound 14a (65 mg, 0.11 mmol) was dissolved in 2.0 mL DCM, and 0.6 mL TFA was added. The mixture was stirred at RT for 0.5 hour under a nitrogen atmosphere. The reaction was then stopped, and the mixture was concentrated to dryness under reduced pressure. Purification by medium-to-high pressure reverse-phase prep-LC (acidic mobile phase) followed by lyophilization afforded 10 mg of compound STI-G2 as a reddish-brown solid in 18.7% yield. LCMS (254 nm) purity 98.48%, Rt = 1.75 min; MS Calculated: 469.2; MS Found: 470.2 $[M+H]^+$. $^1H$ NMR (400 MHz, DMSO-$d_6$) δ 7.76 (d, *J* = 12.3 Hz, 1H), 7.35 (d, *J* = 9.6 Hz, 1H), 7.20 (s, 1H), 6.48 (s, 1H), 6.16 (s, 2H), 5.41 (s, 2H), 5.24 (s, 2H), 4.19 (s, 2H), 2.58 (t, *J* = 7.2 Hz, 2H), 1.91 - 1.80 (m, 2H), 1.65 - 1.56 (m, 2H), 0.92 (t, J = 7.3 Hz, 3H), 0.87 (t, J = 7.3 Hz, 3H).

**Example 15: Synthesis of (S)-10-cyclopropyl-4-ethyl-8-fluoro-4-hydroxy-11-(4-hydroxybutyl)-9-methyl-1,12-dihydro-14 H-pyrano[3',4':6,7]indolizino[1,2-b]quinoline-3,14(4H)-dione (STI-D)**

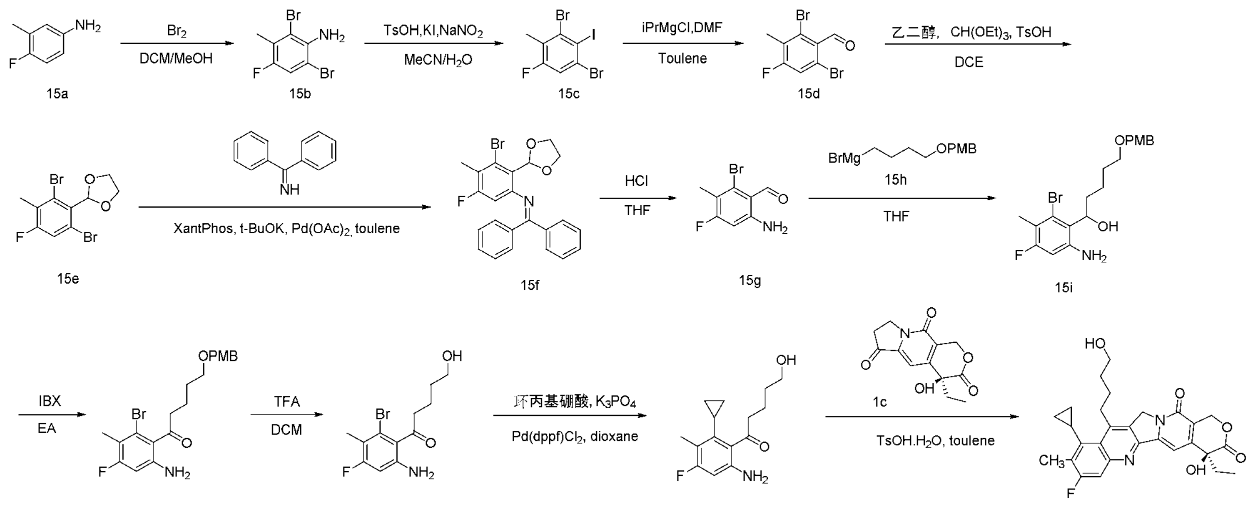


15a 15b 15c 15d 15e 15f 15g 15i 15j 15k 15l STI-D

Step 1: At RT, compound 15a (10.0 g, 80.0 mmol) was dissolved in 100 mL DCM. Then, 100 mL methanol was added, and $Br_2$ (32.0 g, 200.0 mmol) was added dropwise to the mixture. After the addition, the reaction was carried out at RT for 2 hours. Upon completion, the mixture was concentrated to dryness under reduced pressure. Then, 1 M aqueous sodium thiosulfate solution (100 mL) and 100 mL ethyl acetate were added, and the pH was adjusted to 7-8 with saturated

$NaHCO_3$. The aqueous phase was washed with ethyl acetate (100 mL × 2). The organic phases were combined, washed sequentially with 100 mL 1 M aqueous sodium thiosulfate and saturated brine, dried over anhydrous $Na_2SO_4$, and concentrated to dryness. Purification by column chromatography (PE) afforded 20.0 g of compound 15b as a pale purple solid in 89.3% yield. $^1H$ NMR (400 MHz, DMSO-$d_6$) δ 7.44 (d, *J* = 8.8 Hz, 1H), 5.16 (s, 2H), 2.21 (d, *J* = 2.4 Hz, 3H).

Step 2: TsOH (40.0 g, 232.6 mmol) was added in MeCN (240.0 mL) and cooled to 10°C. Compound 15b (20.0 g, 70.8 mmol) was added. $NaNO_2$ (9.8 g, 141.6 mmol) and KI (29.4 g, 141.6 mmol) were dissolved in water (60 mL) and added dropwise to the reaction system. After the addition, the system was allowed to naturally return to RT and stirred for 1 h, then poured into 200 mL water. The pH was adjusted to about 9 with saturated $NaHCO_3$, and 1 M sodium thiosulfate aqueous solution was added until the color no longer faded. The mixture was extracted with EtOAc (3 × 200 mL). The organic phases were combined, dried over anhydrous $Na_2SO_4$, concentrated in vacuo, and purified by column chromatography (PE) to afford 18.6 g of compound 15c as a white solid in 67.1% yield. $^1H$ NMR (400 MHz, DMSO-$d_6$) δ 7.79 (d, *J* = 9.2Hz, 1H), 2.26 (d, *J* = 2.4 Hz, 3H).

Step 3: Compound 15c (5040 mg, 12.8 mmol) was added to a reaction flask under a nitrogen atmosphere. Toluene was added, and the mixture was cooled to approximately -30°C. To the system was added dropwise 2 M solution of iPrMgCl in THF (9.6 mL, 19.2 mmol). The reaction was maintained at -30°C for 1.5 hours. Then, DMF (3083 mg, 42.2 mmol) was added dropwise to the system. After the addition, the system was allowed to warm to RT naturally and stirred for 1.5 hours. After the completion of the reaction was confirmed by TLC (PE:EA = 20:1, product Rf = 0.3), the reaction was quenched by adding 50.0 mL saturated aqueous $NH_4Cl$ solution. The mixture was extracted with ethyl acetate (50.0 mL × 3). The organic phases were combined, dried over anhydrous $Na_2SO_4$ and concentrated to dryness. Purification by column chromatography (PE-PE:EA=100:1) afforded 3.5 g of compound 15d as a yellowish solid in 79.8% yield. $^1H$ NMR (400 MHz, DMSO-$d_6$) δ 10.14 (s, 1H), 7.32 (d, *J*= 12.6Hz, 1H), 2.29 (d, *J* = 2.8 Hz, 3H).

Step 4: Compound 15d (2959 mg, 10.0 mmol) was added to a three-necked flask under a nitrogen atmosphere. DCE (30.0 mL) was added for dissolution, followed by the sequential addition of ethylene glycol (3724 mg, 60.0 mmol), triethyl orthoformate (1630 mg, 11.0 mmol), and TsOH (86 mg, 0.5 mmol). The reaction was refluxed for 3 hours. After the completion of the reaction was confirmed by TLC (PE:EA = 20:1, product Rf = 0.25), the reaction was stopped and allowed to cool to RT naturally. The reaction solution was diluted with 50 mL DCM and extracted with 80 mL saturated aqueous $Na_2CO_3$ solution. The organic phase was then washed with 80 mL saturated brine, dried over anhydrous $Na_2SO_4$, and concentrated to dryness to afford 3.3 g of compound 15e as a yellowish solid in 97.7% yield. $^1H$ NMR (400 MHz, DMSO-$d_6$) δ 7.33 (d, *J* = 8.8 Hz, 1H), 6.42 (s, 1H), 4.35-4.31 (m, 2H), 4.09-4.06(m, 2H), 2.33 (d, *J* = 2.4 Hz, 3H).

Step 5: Compound 15e (3.3 g, 10.0 mmol) was added to a reaction flask under a nitrogen atmosphere. Toluene (20.0 mL) was added, followed by the sequential addition of benzophenone imine (1.45 g, 8.0 mmol), t-BuOK (2.36 g, 21.0 mmol), $Pd(OAc)_2$ (225 mg, 1.0 mmol), and Xantphos (579 mg, 1.0 mmol). The mixture was heated to 100°C and stirred for 1 hour. The reaction was then stopped, and the system was filtered through a pad of Celite. The filter cake was washed with EA. The filtrate was concentrated to dryness under reduced pressure and purified by column chromatography (PE:EA = 400:1) to afford 730 mg of compound 15f as a yellowish solid in 16.6% yield.

Step 6: Compound 15f (730 mg, 1.67 mmol) was dissolved in 4.0 mL THF and cooled to 0-5°C. Concentrated hydrochloric acid (11.0 mL, 116.8 mmol) was added dropwise to the system. After the addition, the reaction was maintained at 0-5 °C for 10 minutes and then stopped. The pH was adjusted to neutral with solid $NaHCO_3$. The mixture was extracted with EA (20.0 mL) and water (20.0 mL). The aqueous phase was further washed with EA (20.0 mL × 2). The organic phases were combined, dried over anhydrous $Na_2SO_4$ and concentrated to dryness. Purification by prep-TLC afforded 130 mg of compound 15g as a yellow solid in 33.7% yield. LCMS (254 nm) purity 92.14%, Rt = 2.024 min; MS Calculated: 231.0; MS Found: 230.0 $[M-H]^-$. $^1H$ NMR (400 MHz, DMSO-$d_6$) δ 10.39 (s, 1H), 6.51(br, 2H) 6.31 (d, *J* = 11.2 Hz, 1H), 2.25 (d, *J*= 2.4 Hz, 3H).

Step 7: Compound 15g (130 mg, 0.56 mmol) was added to a reaction flask under a nitrogen atmosphere. After dissolution in THF, the mixture was cooled to 0-5°C. To the system was added dropwise a solution of compound 15h in THF, and the reaction was carried out at 0-5°C for 1 hour. The reaction was then quenched by adding 20 mL saturated aqueous $NH_4Cl$ solution. The mixture was extracted with EA, dried over anhydrous $Na_2SO_4$, and concentrated to dryness. Purification by prep-TLC (PE:EA = 3:1) afforded 130 mg of compound 15i as a brown oily liquid in 54.6% yield. LCMS (254 nm) purity 98.60%, Rt = 2.174 min; MS Calculated: 425.1; MS Found: 424.1 $[M-H]^-$. $^1H$ NMR (400 MHz, DMSO-$d_6$) δ 7.26-7.17 (m, 2H), 6.93-6.84 (m, 2H), 6.44 (d, *J* = 12.0 Hz, 1H), 5.79 (d, *J* = 3.8 Hz, 1H), 5.66 (s, 2H), 5.16 (dt, *J* = 8.7, 4.2 Hz, 1H), 4.34 (s, 2H), 3.74 (s, 3H), 3.36 (t, *J* = 6.2 Hz, 2H), 2.11 (d, *J* = 2.2 Hz, 3H), 1.87-1.74 (m, 1H), 1.55-1.46 (m, 5H).

Step 8: Compound 15i (130 mg, 0.31 mmol) was dissolved in 4.0 mL ethyl acetate. 2-Iodoxybenzoic acid (IBX, 95 mg, 0.34 mmol) was added, and the mixture was heated under reflux for 4 hours. The reaction was then stopped and allowed to cool to RT naturally. The mixture was filtered through a pad of Celite, and the filter cake was washed with ethyl acetate. The filtrate was concentrated to dryness under reduced pressure and further purified by prep-TLC (PE:EA = 5:2) to afford 130 mg of compound 15j as a brown oily liquid in 100.0% yield. LCMS (214 nm) purity 90.82%, Rt = 2.226 min; MS Calculated: 423.1; MS Found: 422.1 $[M-H]^-$. $^1H$ NMR (400 MHz, DMSO-$d_6$) δ 7.26-7.21 (m, 2H), 6.91-6.87 (m, 2H), 6.52 (d, *J* = 12.0 Hz, 1H), 5.35 (br, 2H), 4.36 (s, 2H), 3.74 (s, 3H), 3.39 (t, *J* = 6.4 Hz, 2H), 2.77 (t, *J* = 7.2 Hz, 2H), 2.10 (d, *J* = 2.4 Hz,

3H), 1.68-1.63 (m, 2H), 1.61-1.55 (m, 2H).

Step 9: Compound 15j (130 mg, 0.31 mmol) was dissolved in 1 mL DCM and cooled to 0-5°C. A 50% solution of TFA in dichloromethane (3.0 mL) was added dropwise to the mixture. After the addition, the reaction was carried out at 0-5 °C for 20 minutes and then stopped. The system was concentrated to dryness under reduced pressure, then redissolved in DCM. The pH was adjusted to approximately 7 with saturated $NaHCO_3$. The mixture was extracted, and the aqueous phase was further washed with DCM (20.0 mL). The organic phases were combined, dried over anhydrous $Na_2SO_4$ and concentrated to dryness. Purification by prep-TLC (PE:EA = 5:2) afforded 80 mg of compound 15k in 86.0% yield. LCMS (254 nm) purity 94.67%, $R_t$ = 1.763 min; MS Calculated: 303.0; MS Found: 302.0 $[M-H]^-$. $^1H$ NMR (400 MHz, DMSO-$d_6$) δ 6.51 (d, *J* = 12.0 Hz, 1H), 5.35 (s, 2H), 4.34 (s, 1H), 3.40 (t, *J* = 6.4 Hz, 2H), 2.77 (t, *J* = 7.2 Hz, 2H), 2.11 (d, *J* = 2.0 Hz, 3H), 1.67-1.60 (m, 2H), 1.50-1.53 (m, 2H).

Step 10: Compound 15k (40 mg, 0.13 mmol) was dissolved in 1,4-dioxane (4.0 mL). Cyclopropylboronic acid (67 mg, 0.78 mmol), $K_3PO_4$ (165 mg, 0.78 mmol), and Pd(dppf)$Cl_2$ (22 mg, 0.03 mmol) were added sequentially. Under a nitrogen atmosphere, the mixture was heated to 90°C and stirred for 1.5 hours. The reaction progress was monitored by LCMS until the starting material was completely consumed. The reaction was then stopped and allowed to cool to RT naturally. The mixture was filtered through a pad of Celite, and the filter cake was washed with ethyl acetate. The filtrate was concentrated to dryness under reduced pressure and further purified by prep-TLC (DCM:MeOH = 20:1) to afford 30 mg of compound 151 as a white solid in 85.8% yield.

Step 11: Compound 151 (21 mg, 0.08 mmol), compound 1c (40 mg, 0.13 mmol), and TsOH·$H_2O$ (40 mg, 0.13 mmol) were added to a reaction flask. Toluene was added, and the mixture was heated under reflux for 1 hour under a nitrogen atmosphere. The reaction was then stopped, and the system was concentrated to dryness under reduced pressure. Medium-to-high pressure reverse-phase prep-LC (acidic mobile phase) followed by lyophilization afforded 6.5 mg of the title compound (STI-D) as a yellowish solid. LCMS (254 nm) purity 98.45%, Rt = 1.990 min; MS Calculated: 492.2; MS Found: 493.1 $[M+H]^+$. $^1H$ NMR (400 MHz, DMSO-$d_6$) δ 6.72 (s, 1H), 6.29 (s, 1H), 6.20 (d, *J* = 11.2 Hz, 1H), 6.13 (s, 1H), 4.56 (dd, *J* = 24.4, 16.0 Hz, 2H), 4.16-3.92 (m, 4H), 2.59-2.54 (m, 2H), 2.13 (d, *J* = 2.0 Hz, 3H), 2.12-2.08 (m, 1H) 1.95-1.85 (m, 2H), 1.84-1.61 (m, 4H), 0.99-0.88 (m, 2H), 0.75 (t, *J* = 7.2 Hz, 3H), 0.54-0.48 (m, 1H), 0.32-0.28 (m, 1H).

Intermediate compound 15h was prepared as follows:

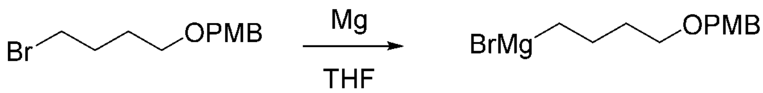


15h-1 15h

Magnesium granules (97 mg, 4.03 mmol) were added to a three-necked flask. Under a nitrogen atmosphere, THF (4.0 mL) was added, followed by 1,2-dibromoethane (2 drops, cat.). The mixture was heated until initiation occurred. 15h-1 (914 mg, 3.36 mmol) was dissolved in THF (2.0 mL), and the solution was added to the reaction system. The reaction was carried out at 50°C for 30 minutes to afford a solution of 15h in THF.

**Example 16: Synthesis of 2-cyclopropyl-N-((S)-4-ethyl-8-fluoro-4-hydroxy-9-methyl-3,14-dioxo-3,4,12,14-tetrahydro-1H -pyrano[3',4':6,7]indolizino[1,2-b]quinolin-11-yl)-2-hydroxyacetamide (STI-F)**

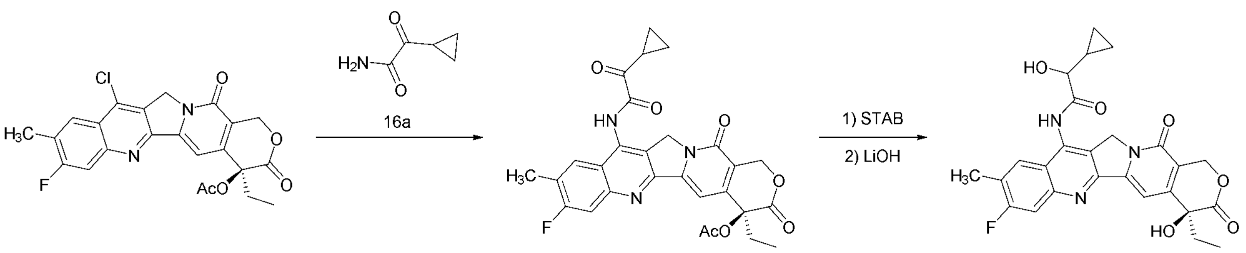


9b 16b STI-F

Step 1: Compound 16a (23 mg, 0.2 mmol), compound 9b (46 mg, 0.1 mmol) from Example 9, Xantphos (6 mg, 0.01 mmol), palladium acetate (2.2 mg, 0.01 mmol), and cesium carbonate (65 mg, 0.20 mmol) were added to a reaction flask. Under a nitrogen atmosphere, 3 mL anhydrous toluene was added, and the mixture was stirred thoroughly. It was then heated to 100°C and reacted for 1.0 hour. After the completion of the reaction was confirmed by TLC (DCM:MeOH=20:1, product Rf=0.5), the system was concentrated to dryness under reduced pressure. Purification by

column chromatography (DCM:MeOH = 100:1) afforded 17 mg of compound 16b as a yellow solid in 31.8% yield. LCMS (254 nm) purity 99.42%, $R_t$ = 1.89 min; MS Calculated: 533.2; MS Found: 534.2 $[M+H]^+$.

Step 2: Compound 16b (17 mg, 0.032 mmol) was dissolved in DCM (1.0 mL) under a nitrogen atmosphere. STAB (28 mg, 0.140 mmol) was added in four portions, and reacted at RT for 1.5 hours. After the completion of the reaction was confirmed by TLC (DCM:MeOH=20:1, product Rf = 0.3), the system was extracted with 10 mL DCM and 10 mL water. The aqueous phase was further washed with DCM (10 mL × 2). The organic phases were combined, and concentrated to dryness under reduced pressure. Methanol (3 mL) and lithium hydroxide (20 mg) were added to the residue, and the mixture was stirred at RT for 10 minutes. The pH was adjusted to 3~4 with acetic acid. A mixture of dichloromethane and methanol (DCM:MeOH=10:1, 10 mL) and water (10 mL) were added for extraction. The aqueous phase was further washed with the DCM:MeOH (10:1) mixture (10 mL × 2). The organic phases were combined, and concentrated to dryness. Low-to-medium pressure reverse-phase prep-LC (acidic mobile phase) followed by lyophilization afforded 6 mg of compound STI-F as a yellowish solid in 37.9% yield. LCMS (254 nm) purity 98.86%, Rt = 1.60 min; MS Calculated: 493.2; MS Found: 494.2 $[M+H]^+$. $^1H$ NMR (400 MHz, DMSO-$d_6$) δ 10. 60(s, 1H), 8.07 (d, *J* =8.0 Hz, 1H), 7.92 (d, *J* = 10.8 Hz, 1H), 7.33 (s, 1H), 6.52 (s, 1H), 5.91 (br, 1H), 5.42 (s, 2H), 5.11 (s, 2H), 3.95 (d, *J* = 6.8 Hz, 1H), 2.50 (s, 3H), 1.92-1.79 (m, 2H), 1.34-1.27 (m, 1H), 0.88 (t, *J* = 7.2 Hz, 3H), 0.62-0.49 (m, 4H).

Intermediate compound 16a was prepared as follows:

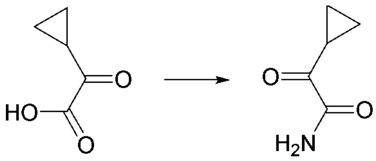

16a-1 16a

Compound 16a-1 (250 mg, 2.19 mmol) was dissolved in THF (5 mL). Triethylamine (0.61 mL, 4.38 mmol) was added. Under a nitrogen atmosphere, the mixture was cooled to -20°C, and a solution of isopropyl chloroacetate in tetrahydrofuran (299 mg, 2.19 mmol) was added dropwise. After stirring at constant temperature for 30 minutes, concentrated aqueous ammonia (1279 mg, 10.95 mmol) was added to the system. The reaction was then allowed to proceed at RT for 2 hours, and then was stopped. The system was concentrated to dryness under reduced pressure. The residue was extracted with saturated sodium bicarbonate aqueous solution and EA. The organic phase was washed with saturated brine, dried over anhydrous $Na_2SO_4$, and concentrated to dryness. Pre-TLC (PE:EA=2:1) afforded 92 mg of compound 16a as a white solid in 37.2% yield. $^1H$ NMR (400 MHz, DMSO-$d_6$) δ 7.91 (s, 1H), 7.69 (s, 1H), 2.86-2.80 (m, 1H), 1.12-1.08 (m, 2H), 0.97-0.94 (m, 2H).

**Example 17: Synthesis of (S)-4-ethyl-8-fluoro-4-hydroxy-9-methyl-11-((4-hydroxybutyl)amino)-1,12-dihydro-14H-pyra no[3',4':6,7]indolizino[1,2-b]quinoline-3,14(4H)-dione (STI-F6)**

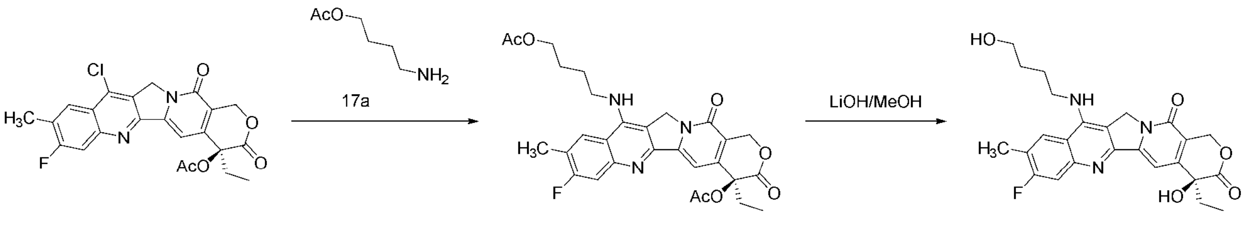


9b 17b STI-F6

Step 1: Compound 9b (91 mg, 0.20 mmol) from Example 9, 17a (52 mg, 0.40 mmol), Xantphos (23 mg, 0.04 mmol), palladium acetate (5 mg, 0.02 mmol), and cesium carbonate (195 mg, 0.60 mmol) were added to a reaction flask. The mixture was dissolved in 6 mL anhydrous toluene. Under a nitrogen atmosphere, the mixture was heated to 100°C and reaction was carried out for 5 hours. The mixture was then concentrated to dryness. Purification by silica gel column chromatography (DCM:MeOH=100:1) afforded 20 mg of compound 17b as an off-white solid in 18.2% yield. LCMS (254 nm) purity 97.1%, Rt= 1.89 min; MS Calculated: 551.2; MS Found: 552.3 $[M+H]^+$. $^1H$ NMR (400 MHz, DMSO-$d_6$) δ 8.30 (d, *J* = 8.1 Hz, 1H), 7.56 (d, *J* = 11.1 Hz, 1H), 7.37 (t, *J* = 5.8 Hz, 1H), 6.87 (s, 1H), 5.46 (s, 2H), 5.50-5.35 (m, 2H), 4.11-4.03 (m, 2H), 3.67-3.61 (m, 2H), 2.43 (s, 3H), 2.20 (s, 3H), 2.20-2.03 (m, 2H), 2.00 (s, 3H), 1.75 (h, *J* = 4.3 Hz, 4H), 0.90 (t, *J* = 7.4 Hz, 3H).

Step 2: Compound 17b (35 mg, 0.06 mmol) was dissolved in a mixture of methanol (2 mL) and dichloromethane (1 mL). LiOH (35 mg, 1.46 mmol) was added. Under a nitrogen atmosphere, the mixture was stirred at RT for 80 minutes. The pH was adjusted to 3-4 with glacial acetic acid. After concentration, low-to-medium pressure reverse-phase prep-LC (acidic mobile phase) and lyophilization afforded 15 mg of compound STI-F6 as a yellowish solid in 50.6% yield. LCMS (254 nm) purity 98.6%, Rt = 1.59 min; MS Calculated: 467.2; MS Found: 468.3 $[M+H]^+$. $^1H$ NMR (400 MHz, DMSO-$d_6$) δ 8.43 (d, *J* = 7.7 Hz, 1H), 7.57 (d, *J* = 10.3 Hz, 1H), 7.45 (s, 1H), 6.61 (s, 1H), 5.46 (s, 2H), 5.44 (s, 2H), 3.74 (q, *J* = 6.7 Hz, 2H), 3.48 (t, *J* = 6.4 Hz, 2H), 2.43 (s, 3H), 1.96 - 1.73 (m, 4H), 1.66 - 1.54 (m, 2H), 0.87 (t, *J* = 7.3 Hz, 3H).

Intermediate compound 17a was prepared as follows:

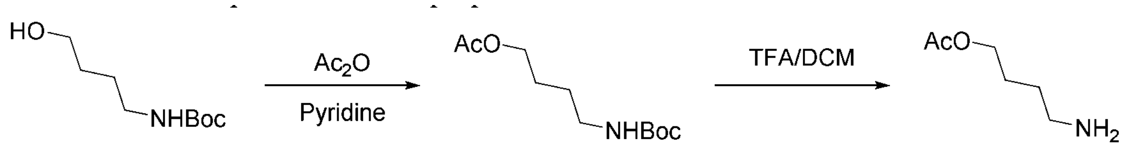


17a-1 17a-2 17a

Step 1: Compound 17a-1 (3.0 g, 15.9 mmol) was dissolved in 15 mL pyridine. Acetic anhydride (15 mL) was added, and the reaction was heated at 40°C for 1 hour under a nitrogen atmosphere. Under ice-bath, water and EA were added for extraction. The organic phase was washed with 2 M HCl to pH ≤ 2 and with brine, and dried over anhydrous $Na_2SO_4$. The solvent was rotary-evaporated to afford 3.56 g of compound 17a-2 as a transparent oil in 97.1% yield. $^1H$ NMR (400 MHz, DMSO-$d_6$) δ 6.81 (t, *J* = 5.8 Hz, 1H), 3.98 (t, *J* = 6.6 Hz, 2H), 2.92 (q, *J* = 6.6 Hz, 2H), 1.99 (s, 3H), 1.59 - 1.49 (m, 2H), 1.45 - 1.33 (m, 11H).

Step 2: Compound 17a-2 (92 mg, 0.4 mmol) was dissolved in 0.5 mL dichloromethane. TFA (0.5 mL) was added, and the reaction was stirred at RT for 20 minutes under a nitrogen atmosphere. After rotary evaporation, crude compound 17a was obtained, which can be directly used in the next reaction.

**Example 18: Synthesis of (S)-11-((4-aminobutyl)amino)-4-ethyl-8-fluoro-4-hydroxy-9-methyl-1,12-dihydro-14H-pyrano[ 3',4':6,7]indolizino[1,2-b]quinoline-3,14(4H)-dione (STI-F7)**

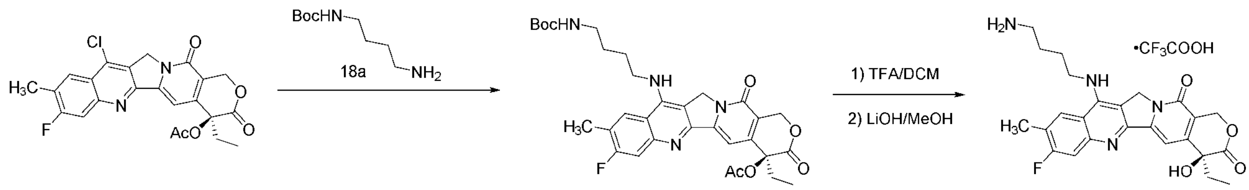


9b 18b STI-F7

Step 1: Compound 9b (91 mg, 0.2 mmol) from Example 9, Xantphos (23 mg, 0.04 mmol), palladium acetate (5 mg, 0.02 mmol), and cesium carbonate (130 mg, 0.4 mmol) were added to a reaction flask. Under a nitrogen atmosphere, 18a (75 mg, 0.4 mmol) was dissolved in 6 mL anhydrous toluene and the solution was added to the system. The mixture was stirred until homogeneous, heated to 100°C, and reacted for 1.0 h. After the completion of the reaction was confirmed by TLC (DCM:MeOH = 30:1, product Rf = 0.3), the system was concentrated to dryness under reduced pressure. Purification by column chromatography (DCM:MeOH = 100:1) afforded 100 mg of compound 18b as a brown solid in 54.8% yield. $^1H$ NMR (400 MHz, DMSO-$d_6$) δ 8.31 (d, *J* =8.4 Hz, 1H), 7.57 (d, *J* = 11.2 Hz, 1H), 7.40 (br, 1H), 6.87 (s, 1H), 6.83 (t, *J* =5.6 Hz, 1H), 5.46 (s, 2H), 5.53 (d, *J* =4.8 Hz, 2H), 3.60 (q, *J* =6.4 Hz, 2H), 2.97 (q, *J* =6.4 Hz, 2H), 2.44 (s, 3H), 2.20 (s, 3H), 2.12-2.07 (m, 2H), 1.70-1.63 (m, 2H), 1.56-1.49 (m, 2H), 1.33 (s, 9H), 0.90 (t, *J* = 7.2 Hz, 3H).

Step 2: Compound 18b (100 mg, 0.16 mmol) was added to DCM (1.5 mL), followed by TFA (0.5 mL). The reaction was carried out at RT for 20 minutes, then the reaction was stopped. The system was concentrated to dryness under reduced pressure, and methanol (2.0 mL) and LiOH (100 mg) were added. The mixture was stirred at RT for another 20 minutes, and then the reaction was stopped. The pH was adjusted to 4~5 with acetic acid. After concentration, the mixture was subjected to medium-to-low pressure reverse-phase prep-LC (acidic mobile phase) and lyophilized to afford 60 mg of compound STI-F7 as a yellowish solid in 83.3% yield. LCMS (254 nm) purity 100.0%, $R_t$ = 1.789 min; MS Calculated: 466.2; MS Found: 467.2 $[M+H]^+$. $^1H$ NMR (400 MHz, DMSO-$d_6$) δ 8.39 (d, *J* =8.0 Hz, 1H), 8.06 (br, 1H), 7.73 (br, 3H), 7.61 (d, *J* =10.8 Hz, 1H), 7.36 (s, 1H), 6.56 (br, 1H), 5.46 (s, 2H), 5.43 (s, 2H), 3.60 (q, *J* =6.4 Hz, 2H), 2.97 (q, *J* =6.4 Hz, 2H), 2.44 (s, 3H), 1.91-1.81 (m, 2H), 1.79 -1.67 (m, 4H), 0.87 (t, *J* = 7.2 Hz, 3H).

**Example 19: Synthesis of (S)-4-ethyl-8-fluoro-4-hydroxy-9-methyl-11-(pentylamino)-1,12-dihydro-14H-pyrano [3',4':6,7] indolizino[1,2-b]quinoline-3,14(4H)-dione (STI-F13)**

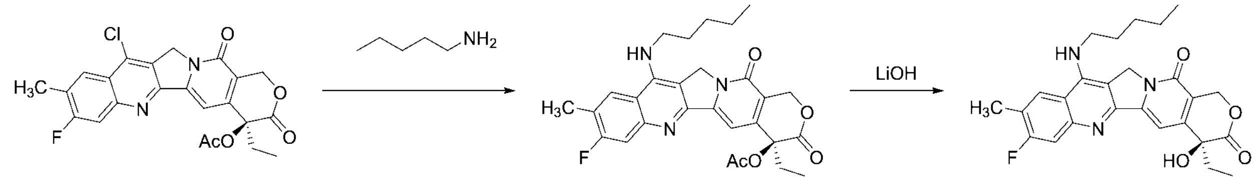


9b 19a STI-F13

Step 1: Compound 9b (91 mg, 0.20 mmol) from Example 9, n-pentylamine (35 mg, 0.40 mmol), Xantphos (23 mg, 0.04 mmol), palladium acetate (5 mg, 0.02 mmol), and cesium carbonate (130 mg, 0.40 mmol) were added to a reaction flask. The mixture was dissolved in 6 mL anhydrous toluene. Under a nitrogen atmosphere, the mixturue was heated to 100°C and reacted for 5 hours. The mixture was then concentrated to dryness. Purification by silica gel column chromatography (DCM:MeOH = 200:1 to 100:1) afforded 30 mg of compound 19a as a pale red solid in 29.6% yield.

Step 2: Compound 19a (23 mg, 0.05 mmol) was dissolved in a mixed solution of methanol (2 mL) and dichloromethane (1 mL). Lithium hydroxide (LiOH, 23 mg, 0.96 mmol) was added. Under a nitrogen atmosphere, the mixture was stirred at RT for 80 minutes. The pH was adjusted to 3~4 with glacial acetic acid. After concentration, low-to-medium pressure reverse-phase prep-LC (acidic mobile phase) and lyophilization afforded 12 mg of compound STI-F13 as a yellowish solid in 56.9% yield. LCMS (254 nm) purity 98.5%, Rt = 1.97 min; MS Calculated: 465.2; MS Found: 466.3 $[M+H]^+$. $^1H$ NMR (400 MHz, DMSO-$d_6$) $\delta$ 8.41 (d, $J$ = 7.7 Hz, 1H), 7.55 (d, $J$ = 10.3 Hz, 1H), 7.42 (s, 1H), 6.60 (s, 1H), 5.50-5.37 (m, 4H), 3.72-3.67 (m, 2H), 2.42 (s, 3H), 1.87 (dq, $J$ = 14.1, 7.1 Hz, 2H), 1.74 (q, $J$ = 7.4 Hz, 2H), 1.41 (td, $J$ = 12.8, 7.1 Hz, 4H), 0.90 (dt, $J$ = 18.2, 7.2 Hz, 6H).

**Example 20: Synthesis of (S)-N-(2-(9-amino-4-ethyl-8-fluoro-4-hydroxy-3,14-dioxo-3,4,12,14-tetrahydro-1H-pyrano[3',4 ':6,7]indolizino[1,2-b]quinolin-11-yl)ethyl)-N-isopropylmethanesulfonamide (STI-G4)**

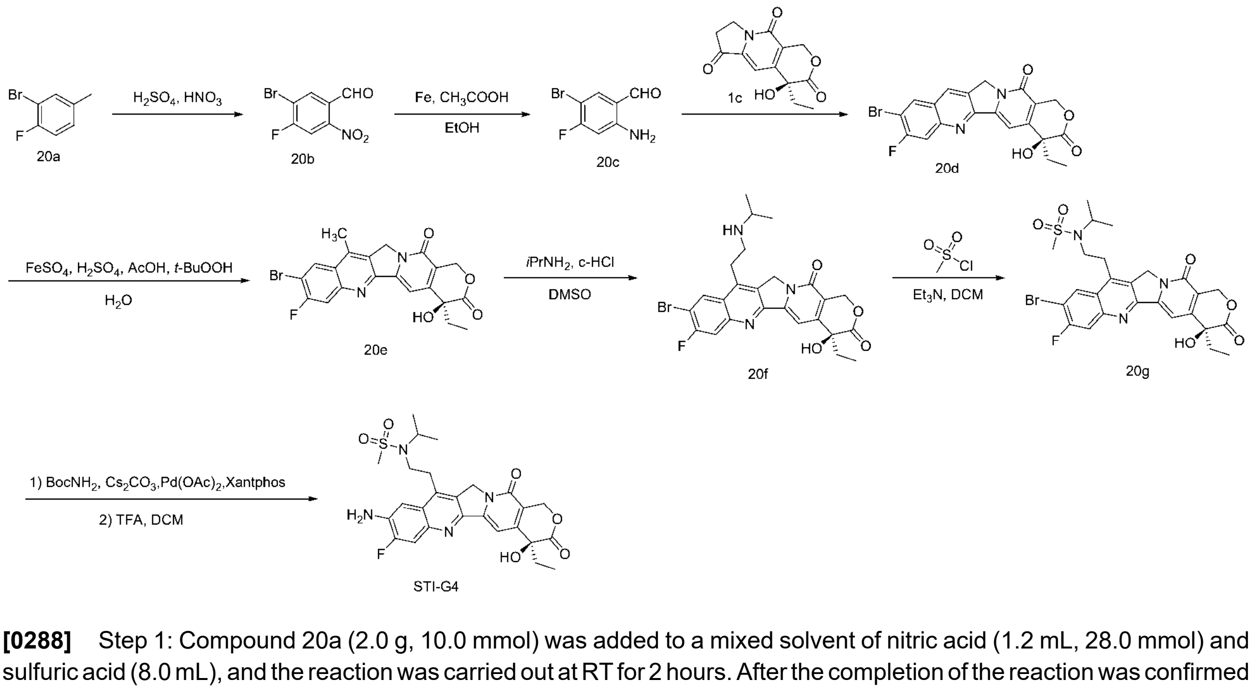


20a 20b 20c 20d 20e 20f 20g STI-G4

Step 1: Compound 20a (2.0 g, 10.0 mmol) was added to a mixed solvent of nitric acid (1.2 mL, 28.0 mmol) and sulfuric acid (8.0 mL), and the reaction was carried out at RT for 2 hours. After the completion of the reaction was confirmed by TLC (PE:EA=10:1, product Rf=0.7), the system was poured into ice water and extracted with ethyl acetate. The organic phase was dried over anhydrous $Na_2SO_4$ and concentrated under reduced pressure. The resulting brown crude product was purified by column chromatography (PE:EA=50:1) to afford 2.2 g of compound 20b as a yellow solid in 89.1% yield. $^1H$ NMR (400 MHz, DMSO-$d_6$) $\delta$ 10.15 (s, 1H), 8.32 (d, $J$ = 8.4 Hz, 1H), 7.26 (d, $J$ = 6.8 Hz, 1H).

Step 2: Compound 20b (247 mg, 1.0 mmol), iron powder (173 mg, 3.1 mmol), and glacial acetic acid (1.0 mL, 18.0 mmol) were added to a mixed solvent of ethanol (5.0 mL) and water (1.0 mL). The reaction was carried out at 80°C for 1 hour. After the completion of the reaction was confirmed by TLC (PE:EA=10:1, product Rf=0.5), the system was allowed to cool to RT naturally and filtered. The filtrate was concentrated under reduced pressure and purified by silica gel column chromatography (PE:EA=50:1) to afford 190 mg of compound 20c as a yellow solid in 81.5% yield. $^1H$ NMR (400 MHz, DMSO-$d_6$) δ 9.75 (s, 1H), 7.92 (d, *J* = 8.0 Hz, 1H), 7.44 (s, 2H), 6.68 (d, *J* = 11.6 Hz, 1H).

Step 3: Compound 20c (233 mg, 1.0 mmol) and compound 1c (121 mg, 1.0 mmol) were dissolved in 5 mL anhydrous toluene. Under a nitrogen atmosphere, the mixture was refluxed with a water separator for 30 minutes. Then, TsOH (11 mg, 0.06 mmol) was added, and the mixture was stirred under reflux for 1 hour. Upon confirmation of completion by LCMS, the reaction system was cooled to RT and concentrated to dryness under reduced pressure. Trituration with acetone afforded 260 mg of compound 20d as a yellow solid in 58.6% yield. $^1H$ NMR (400 MHz, DMSO-$d_6$) δ 8.69 (s, 1H), 8.66 (d, *J* = 8.0 Hz, 1H), 8.12 (d, *J* = 10.0 Hz, 1H), 7.35 (s, 1H), 6.53 (br, 1H), 5.43 (s, 2H), 5.29 (s, 2H), 1.92 - 1.81 (m, 2H), 0.88 (t, *J* = 6.4 Hz, 3H).

Step 4: Compound 20d (222 mg, 0.5 mmol) and ferrous sulfate heptahydrate (52 mg, 0.19 mmol) were successively added to 8 mL water. The mixture was cooled to 0-5°C. Under a nitrogen atmosphere, glacial acetic acid (0.52 mL, 9.2 mmol) and sulfuric acid (3.6 mL) were added. Tert-butyl hydroperoxide (0.34 mL, 3.5 mmol) was then added dropwise to the system. After the addition, reaction was carried out at RT for 3 hours. The system was poured into ice water and extracted with dichloromethane. The organic phase was dried over anhydrous $Na_2SO_4$ and concentrated under reduced pressure. Purification by silica gel column chromatography (DCM:MeOH = 100:1) afforded 130 mg of compound 20e as a yellow-brown solid in 56.8% yield. $^1H$ NMR (400 MHz, DMSO-$d_6$) δ 8.65 (d, *J*= 7.6 Hz, 1H), 8.10 (d, *J* = 10.0 Hz, 1H), 7.33 (s, 1H), 6.53 (s, 1H), 5.43 (s, 2H), 5.29 (s, 2H), 2.79 (s, 3H),1.91 - 1.81 (m, 2H), 0.88 (t, *J* = 7.6 Hz, 3H).

Step 5: Compound 20e (92 mg, 0.2 mmol) was dissolved in 2 mL DMSO. Under a nitrogen atmosphere, hydrochloric acid (0.12 mL, 1.4 mmol) and isopropylamine (0.1 mL, 1.2 mmol) were added to the reaction system, and reaction was carried out at 140°C for 1 hour. Upon confirmation of completion by LCMS, the reaction was concentrated to dryness under reduced pressure. Medium-to-high pressure reverse-phase prep-LC (acidic mobile phase) and lyophilization afforded 35 mg of compound 20f as a yellow solid in 33.0% yield.

Step 6: Compound 20f (30 mg, 0.057 mmol) was dissolved in 2 mL DCM and cooled to 0~5°C. Under a nitrogen atmosphere, methanesulfonyl chloride (8 mg, 0.068 mmol) and triethylamine (7 mg, 0.068 mmol) were added to the reaction system, and reaction was carried out at RT for 1 hour. Upon confirmation of completion by LCMS, the organic layer was washed sequentially with 1 M aqueous hydrochloric acid and saturated brine. The organic phase was dried over anhydrous $Na_2SO_4$ and concentrated under reduced pressure to afford 30 mg of crude compound 20g, which was used directly in the next step.

Step 7: The crude compound 20g from the previous step (30 mg, 0.049 mmol) and tert-butyl carbamate (12 mg, 0.10 mmol) were dissolved in 3 mL anhydrous toluene. Under a nitrogen atmosphere, cesium carbonate (33 mg, 0.10 mmol), Xantphos (1 mg, 0.001 mmol), and palladium acetate (0.2 mg, 0.001 mmol) were added. The mixture was stirred thoroughly and heated to 100°C. Reaction was carried out for 2.0 hours. Upon confirmation of completion by LCMS, the reaction was concentrated to dryness under reduced pressure, and extracted with dichloromethane and water. The organic phase was concentrated. To the residue was added a 25% solution of TFA in dichloromethane (2 mL), and the mixture was stirred at RT for 30 minutes. Upon confirmation of completion by LCMS, the reaction was concentrated to dryness. Medium-to-high pressure reverse-phase prep-LC (acidic mobile phase) and lyophilization afforded 8 mg of compound STI-G4 as a yellow solid in 30.0% yield.

Similarly, the following compounds of examples were synthesized:

| Example No. | Structure |
|---|---|
| 21 | 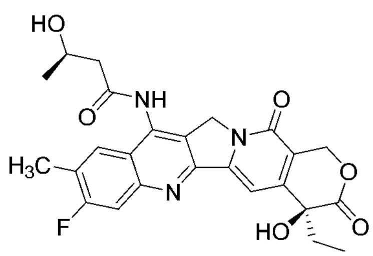 **STI-F12R** |

(continued)

| Example No. | Structure |
|---|---|
| 22 | 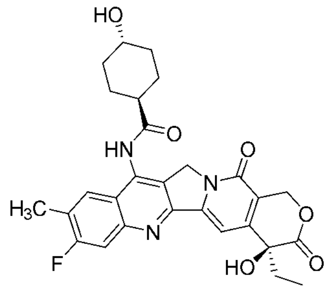 STI-F10 |

**Example P1: Synthesis of (S)-4-ethyl-8-fluoro-4-hydroxy-11-((2-hydroxyethyl)amino)-9-methyl-1,12-dihydro-14H-pyran o[3',4':6,7]indolizino[1,2-b]quinoline-3,14(4H)-dione (STI-F5)**

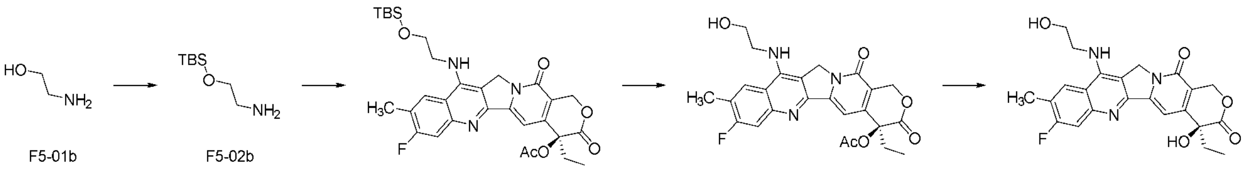

F5-01b F5-02b F5-03b F5-04b STI-F5

Step 1: Compound F5-01b (1.22 g, 20.00 mmol) and triethylamine (4.17 mL, 30.00 mmol) were dissolved in 20 mL dichloromethane. A solution of tert-butylchlorodimethylsilane triflate (6.34 g, 24.00 mmol) in 10 mL dichloromethane was added dropwise over 3 minutes. Under a nitrogen atmosphere, the reaction was carried out at RT for 1.5 hours. Then, the mixture was extracted with 30 mL water and then extracted twice with dichloromethane. The organic phases were combined, dried over anhydrous $Na_2SO_4$, and then sujected to rotary evaporation to remove the solvent, to afford 3.5 g of compound F5-02b as a colorless oil in 100% yield. $^1$H NMR (400 MHz, DMSO-$d_6$) δ 3.57 (t, *J* = 5.8 Hz, 2H), 2.67 (t, *J* = 5.8 Hz, 2H), 0.87 (s, 9H), 0.04 (s, 6H).

Step 2: (S)-11-Chloro-4-ethyl-8-fluoro-9-methyl-3,14-dioxo-3,4,12,14-tetrahydro-1H-pyran [3',4':6,7]indolizino [1,2-b]quinolin-4-yl acetate (184 mg, 0.40 mmol), F5-02b (140 mg, 0.80 mmol), Xantphos (23 mg, 0.04 mmol), palladium acetate (9 mg, 0.04 mmol), and cesium carbonate (391 mg, 0.80 mmol) were added to a reaction flask. The mixture was dissolved in 10 mL anhydrous toluene. Under a nitrogen atmosphere, the mixture was heated to 100°C and the reaction was carried out for 1.5 hours. The system was then concentrated to dryness. Purification by silica gel column chromatography (DCM:MeOH = 200:1) afforded 127 mg of compound F5-03b as a yellowish solid in 52.9% yield. $^1$H NMR (400 MHz, DMSO-$d_6$) δ 8.23 (d, *J* = 8.2 Hz, 1H), 7.56 (d, *J* = 11.1 Hz, 1H), 7.52 (t, *J* = 6.5 Hz, 1H), 6.86 (s, 1H), 5.46 (s, 2H), 5.43 (s, 2H), 3.83 (t, *J* = 5.6 Hz, 2H), 3.70 (t, *J* = 5.5 Hz, 2H), 2.43 (s, 3H), 2.20 (s, 3H), 2.16 - 2.08 (m, 2H), 0.89 (t, *J* = 7.6 Hz, 3H), 0.67 (s, 9H), -0.11 (s, 3H), -0.13 (s, 3H).

Step 3: Compound F5-03b (127 mg, 0.21 mmol) was dissolved in 1 mL dichloromethane. A 4 M HCl solution in 1,4-dioxane was added. Under a nitrogen atmosphere, the mixture was stirred at RT for 40 minutes. Upon confirmation of completion by LCMS, the reaction solution was concentrated to dryness by rotary evaporation. The crude product F5-04b was used directly in the next step.

Step 4: The crude product F5-04b from the previous step was dissolved in 12 mL methanol. Lithium hydroxide (150 mg) was added. Under a nitrogen atmosphere, the mixture was stirred at RT for 1 hour. The pH was adjusted to 6~7 with glacial acetic acid. High-pressure reverse-phase prep-LC (mobile phase: acetonitrile/0.05% formic acid aqueous solution) and lyophilization afforded 16 mg of compound STI-F5 as a yellowish solid in 56.9% yield. LCMS (254 nm) purity 99.7%, $R_t$ = 1.45 min; MS Calculated: 439.2; MS Found: 440.1 $[M+H]^+$. $^1$H NMR (400 MHz, DMSO-$d_6$) δ 8.27 (d, *J* = 8.1 Hz, 1H), 7.57 (d, *J* = 11.1 Hz, 1H), 7.37 (t, *J* = 6.1 Hz, 1H), 7.20 (s, 1H), 6.46 (s, 1H), 5.43 (s, 2H), 5.40 (s, 2H), 5.00 (s, 1H), 3.69 (d, *J* = 2.9 Hz, 4H), 2.43 (s, 3H), 1.94 - 1.76 (m, *J* = 7.2 Hz, 2H), 0.87 (t, *J* = 7.3 Hz, 3H).

**Example P2: Synthesis of (S,E)-4-ethyl-8-fluoro-4-hydroxy-11-((4-hydroxybut-2-en-1-yl)amino)-9-methyl-1,12-dihydro-14H-pyrano[3',4':6,7]indolizino[1,2-b]quinoline-3,14(4H)- dione (STI-F8)**

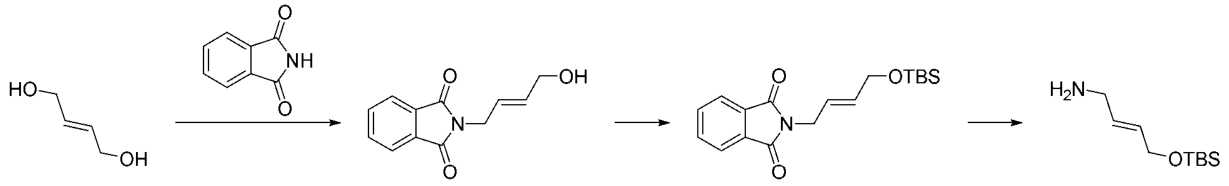

F8-01 F8-02 F8-02c F8-01b

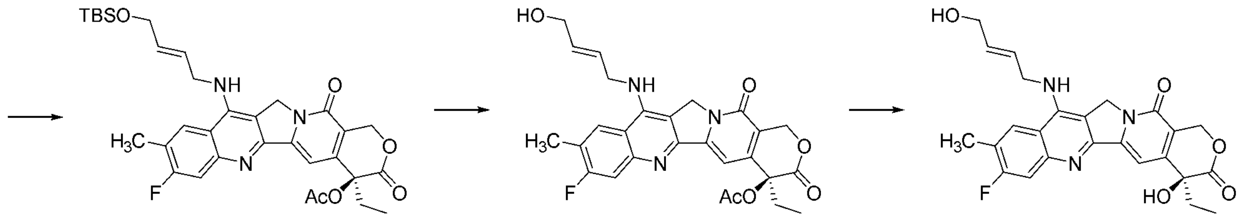

F8-02b F8-03b STI-F8

Step 1: Compound F8-01 (1.2 g, 13.60 mmol) was dissolved in 40 mL tetrahydrofuran and cooled to 0°C. Under a nitrogen atmosphere, phthalimide (1.00 g, 6.80 mmol) was added, followed by a solution of diethyl azodicarboxylate (3.57 g, 13.60 mmol) in 60 mL toluene. The reaction was carried out at RT for 2 hours. The solvent was removed by rotary evaporation. Purification by silica gel column chromatography (PE:EA = 5:1) afforded 500 mg of compound F8-02 as a white solid. $^1$H NMR (400 MHz, DMSO-$d_6$) δ7.93 - 7.80 (m, 4H), 5.74 - 5.60 (m, 2H), 4.71 (t, *J* = 5.5 Hz, 1H), 4.21 - 4.15 (m, 2H), 3.90 (m, 2H).

Step 2: Compound F8-02 (759 mg, 3.5 mmol) and triethylamine (1062 mg, 10.5 mmol) were dissolved in 8 mL dichloromethane. A solution of tert-butylchlorodimethylsilane (633 mg, 4.2 mmol) in 2 mL dichloromethane was added dropwise. Under a nitrogen atmosphere, the mixture was stirred at RT overnight. The solvent was removed by rotary evaporation. Purification by silica gel column chromatography (PE:EA=10:1) afforded 536 mg of compound F8-02c as a colorless oil. $^1$H NMR (400 MHz, DMSO-$d_6$) δ 7.93 - 7.80 (m, 4H), 5.77 - 5.61 (m, 2H), 4.21 - 4.15 (m, 2H), 4.10 (dd, *J* = 2.9, 1.5 Hz, 2H), 0.83 (s, 9H), 0.00 (s, 6H).

Step 3: Compound F8-02c (536 mg, 1.62 mmol) was dissolved in 8 mL anhydrous ethanol. 80% Hydrazine hydrate (112 mg, 1.78 mmol) was added, and the mixture was refluxed for 6 hours. The mixture was filtered, and the filtrate was concentrated to dryness by rotary evaporation. Cold dichloromethane was added, and the mixture was filtered again. The filtrate was concentrated to dryness by rotary evaporation to afford 250 mg of compound F8-01b as a yellow oil.

Step 4: (S)-11-Chloro-4-ethyl-8-fluoro-9-methyl-3,14-dioxo-3,4,12,14-tetrahydro-1H-pyrano [3',4':6,7]indolizino[1,2-b]quinolin-4-yl acetate (184 mg, 0.40 mmol), Xantphos (23 mg, 0.04 mmol), palladium acetate (9 mg, 0.04 mmol), and cesium carbonate (391 mg, 0.80 mmol) were added to a reaction flask. Compound F8-01b (161 mg, 0.80 mmol) was added, and the mixture was dissolved in 10 mL anhydrous toluene. Under a nitrogen atmosphere, the mixture was heated to 100°C and reacted for 1 hour. The system was then concentrated to dryness. Purification by silica gel column chromatography (DCM:MeOH = 200:1 to 150:1) afforded 100 mg of compound F8-02b as a brown solid in 39.9% yield. MS Calculated: 621.2; MS Found: 622.3 [M+H]$^+$.

Step 5: Compound F8-02b (100 mg, 0.16 mmol) was suspended in a mixture of 3 mL dichloromethane and 5 mL 4 M HCl in 1,4-dioxane. Under a nitrogen atmosphere, the mixture was stirred at RT for 1 hour. The mixture was then concentrated to dryness by rotary evaporation. The crude product was used directly in the next step. LCMS (254 nm) purity 85.0%, Rt = 1.67 min; MS Calculated: 507.2; MS Found: 508.2 [M+H]$^+$.

Step 6: The crude product from the previous step was dissolved in 8 mL methanol. Lithium hydroxide (38 mg) was added. Under a nitrogen atmosphere, the reaction was stirred at RT for 1 hour. The pH was adjusted to 5 with glacial acetic acid in an ice bath. High-pressure reverse-phase prep-LC (mobile phase: acetonitrile/0.05% formic acid aqueous solution) and lyophilization afforded 35 mg of compound STI-F8 as a yellowish solid. LCMS (254 nm): Purity 97.5%, Rt = 1.50 min; MS Calculated: 465.2; MS Found: 466.2 [M+H]$^+$. $^1$H NMR (400 MHz, DMSO-$d_6$) δ 8.34 (d, *J* = 8.1 Hz, 1H), 7.77 (t, *J* = 6.3 Hz, 1H), 7.61 (d, *J* = 11.1 Hz, 1H), 7.25 (s, 1H), 6.51 (s, 1H), 5.94 (dt, *J* = 15.8, 4.5 Hz, 1H), 5.71 (dt, *J* = 15.7, 5.1 Hz, 1H),

5.45 (s, 2H), 5.37 (s, 2H), 4.75 (s, 1H), 4.28 (t, *J* = 5.8 Hz, 2H), 3.97 (d, *J* = 4.9 Hz, 2H), 2.47 (s, 3H), 1.97 - 1.85 (m, 2H), 0.93 (t, *J* = 7.3 Hz, 3H).

**Example P3: Synthesis of (S)-4-ethyl-8-fluoro-4-hydroxy-11-(4-(hydroxymethyl)piperidin-1-yl)-9-methyl-1,12-dihydro-1 4H-pyrano[3',4':6,7]indolizino[1,2-b]quinoline-3,14(4H)-dione (STI-F14)**

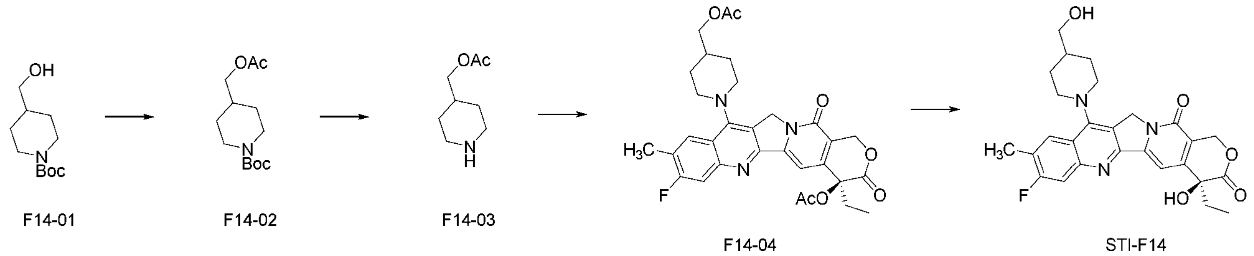


Step 1: Compound F14-01 (1.00 g, 4.60 mmol) was dissolved in 20.0 mL dichloromethane. Pyridine (790 mg, 9.20 mmol) and acetyl chloride (630 mg, 7.40 mmol) were added. Under a nitrogen atmosphere, the reaction was carried out at RT overnight. While cooling in an ice bath, 1 M hydrochloric acid aqueous solution was added to adjust the pH to 1~2. The mixture was extracted with ethyl acetate, washed with saturated sodium bicarbonate and saturated brine, dried over anhydrous $Na_2SO_4$, filtered, and concentrated to dryness by rotary evaporation, giving 1143 mg of compound F14-02 as a yellowish oil in 96.6% yield. $^1H$ NMR (400 MHz, DMSO-$d_6$) δ 3.94 (d, *J* = 13.0 Hz, 2H), 3.86 (d, *J* = 6.5 Hz, 2H), 2.69 (s, 2H), 2.01 (s, 3H), 1.81 - 1.71 (m, 1H), 1.61 (dd, *J* = 12.7, 3.5 Hz, 2H), 1.39 (s, 9H), 1.12 - 0.97 (m, 2H).

Step 2: Compound F14-02 (680 mg, 2.64 mmol) was dissolved in 5 mL dichloromethane. TFA (3 mL) was added. Under a nitrogen atmosphere, the mixture was stirred at RT for 1 hour. The reaction solution was then concentrated to dryness by rotary evaporation. The crude product F14-03 was used directly in the next step.

Step 3: (S)-11-Chloro-4-ethyl-8-fluoro-9-methyl-3,14-dioxo-3,4,12,14-tetrahydro-1H-pyrano [3',4':6,7]indolizino[1,2-b]quinolin-4-yl acetate (184 mg, 0.40 mmol), Xantphos (23 mg, 0.04 mmol), palladium acetate (9 mg, 0.04 mmol), and cesium carbonate (391 mg, 0.80 mmol) were added to a reaction flask. The mixture was dissolved in 10 mL anhydrous toluene. A solution of compound F14-03 (217 mg, 0.80 mmol) in 1 mL tert-butanol was added. Under a nitrogen atmosphere, the mixture was heated to 100°C and reacted for 1 hour. The system was then concentrated to dryness. Purification by silica gel column chromatography (DCM:MeOH = 300:1 to 100:1) afforded 120 mg of compound F14-04 as a yellowish solid in 52.0% yield. LCMS (254 nm) purity 82.5%, Rt = 2.04 min; MS Calculated: 577.2; MS Found: 578.3 $[M+H]^+$.

Step 4: Compound F14-04 (80 mg, 0.14 mmol) was dissolved in 5 mL methanol. Lithium hydroxide (33 mg, 1.40 mmol) was added. Under a nitrogen atmosphere, the mixture was stirred at RT for 40 minutes. The pH was adjusted to 5~6 with glacial acetic acid. High-pressure reverse-phase prep-LC (mobile phase: acetonitrile/0.05% TFA aqueous solution) and lyophilization afforded 15 mg of the compound STI-F14 as a yellowish solid in 21.7% yield. LCMS (254 nm): Purity 99.3%, Rt = 1.67 min; MS Calculated: 493.2; MS Found: 494.3 $[M+H]^+$. $^1H$ NMR (400 MHz, DMSO-$d_6$) δ 7.91 (d, *J* = 8.4 Hz, 1H), 7.74 (d, *J* = 10.8 Hz, 1H), 7.29 (s, 1H), 6.58 (d, *J* = 63.2 Hz, 2H), 5.46 (s, 2H), 5.42 (s, 2H), 3.69 - 3.64 (m, 2H), 3.42 (d, *J* = 6.2 Hz, 2H), 3.25 (t, *J* = 11.5 Hz, 2H), 2.47 (s, 3H), 1.93 - 1.78 (m, 4H), 1.74 - 1.65 (m, 1H), 1.58 - 1.45 (m, 2H), 0.87 (t, *J* = 7.3 Hz, 3H).

**Example P4: Synthesis of (S)-4-ethyl-8-fluoro-4-hydroxy-11-(((1r,4S)-4-(hydroxymethyl) cyclohexyl)amino)-9-methyl-1,12-dihydro-14H-pyrano[3',4':6,7]indolizino[1,2-b]quinoline-3,14(4H)-dione (STI-F15)**

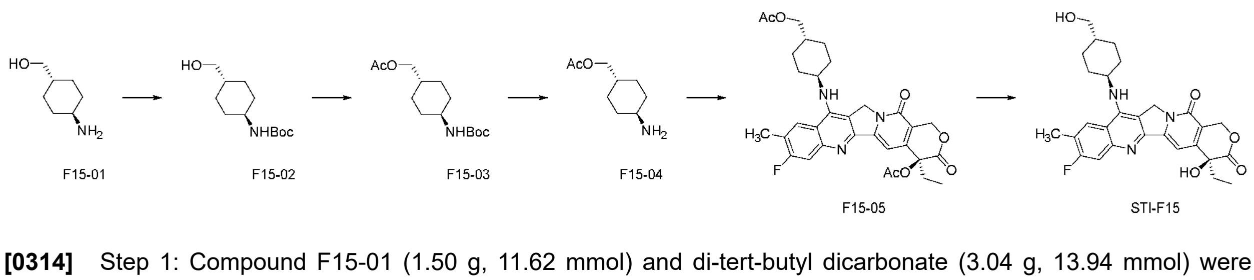


Step 1: Compound F15-01 (1.50 g, 11.62 mmol) and di-tert-butyl dicarbonate (3.04 g, 13.94 mmol) were

dissolved in 20 mL tetrahydrofuran. Under a nitrogen atmosphere, the reaction was carried out at RT overnight. The solvent was removed by rotary evaporation. The residue was extracted with ethyl acetate and water, washed with saturated brine, dried over anhydrous $Na_2SO_4$, filtered, and concentrated to afford 2.74 g of compound F15-02 as a white solid in quantitative yield. $^1$H NMR (400 MHz, DMSO-$d_6$) δ 6.66 (d, $J$ = 8.0 Hz, 1H), 4.36 (t, $J$ = 5.3 Hz, 1H), 3.18 (t, $J$ = 5.8 Hz, 2H), 3.15 - 3.06 (m, 1H), 1.80 - 1.67 (m, 4H), 1.37 (s, 9H), 1.29 - 1.18 (m, 1H), 1.17 - 1.02 (m, 2H), 0.94 - 0.80 (m, 2H).

Step 2: Compound F15-02 (1.10 g, 4.80 mmol) was dissolved in 2.5 mL pyridine. Acetic anhydride (5 mL) was added. Under a nitrogen atmosphere, the reaction was heated at 40°C for 4 hours. While cooling in an ice bath, 1 M hydrochloric acid aqueous solution was added to adjust the pH to 1~2. The mixture was extracted with ethyl acetate, washed with saturated brine, dried over anhydrous $Na_2SO_4$, filtered, and concentrated to dryness by rotary evaporation, giving 979 mg of compound F15-03 as a white solid in 75% yield.

Step 3: Compound F15-03 (740 mg, 2.73 mmol) was dissolved in 5 mL dichloromethane. TFA (3 mL) was added. Under a nitrogen atmosphere, the mixture was stirred at RT for 2 hours. The reaction solution was then concentrated to dryness by rotary evaporation. The crude product F15-04 was used directly in the next step. $^1$H NMR (400 MHz, DMSO-$d_6$) δ 3.83 (d, $J$ = 6.4 Hz, 2H), 3.01 - 2.87 (m, 1H), 2.01 (s, 3H), 1.98 - 1.89 (m, 2H), 1.80 - 1.71 (m, 2H), 1.60 - 1.47 (m, 1H), 1.35 - 1.21 (m, 2H), 1.11 - 0.96 (m, 2H).

Step 4: (S)-11-Chloro-4-ethyl-8-fluoro-9-methyl-3,14-dioxo-3,4,12,14-tetrahydro-1H-pyrano [3',4':6,7]indolizino[1,2-b]quinolin-4-yl acetate (184 mg, 0.40 mmol), Xantphos (23 mg, 0.04 mmol), palladium acetate (9 mg, 0.04 mmol), and cesium carbonate (391 mg, 0.80 mmol) were added to a reaction flask. The mixture was dissolved in 10 mL anhydrous toluene. A solution of F15-04 (114 mg, 0.80 mmol) in 1 mL tert-butanol was added. Under a nitrogen atmosphere, the mixture was heated to 100°C and reacted for 1.5 hours. The system was then concentrated to dryness. Purification by silica gel column chromatography (DCM:MeOH = 300:1 to 100:1) afforded 135 mg of compound F15-05 as a yellowish solid in 57.1% yield. LCMS (254 nm) purity 79.8%, Rt = 1.97 min; MS Calculated: 591.2; MS Found: 592.3 $[M+H]^+$.

Step 5: Compound F15-05 (135 mg, 0.23 mmol) was dissolved in 10 mL methanol. Lithium hydroxide (55 mg, 2.30 mmol) was added. Under a nitrogen atmosphere, the mixture was stirred at RT for 1 hour. The pH was adjusted to 4~5 with glacial acetic acid. High-pressure reverse-phase prep-LC (mobile phase: acetonitrile/0.05% TFA aqueous solution) and lyophilization afforded 10 mg of compound STI-F15 as a yellowish solid in 8.6% yield. LCMS (254 nm) purity 95.8%, Rt = 1.62 min; MS Calculated: 507.2; MS Found: 508.3 $[M+H]^+$. $^1$H NMR (400 MHz, DMSO-$d_6$) δ 8.50 (d, $J$ = 7.9 Hz, 1H), 7.59 (d, $J$ = 10.5 Hz, 1H), 7.41 (s, 1H), 6.56 (s, 1H), 5.43 (s, 2H), 5.40 (s, 2H), 3.77 (s, 1H), 3.31 (d, $J$ = 6.0 Hz, 2H), 2.45 (s, 3H), 2.11 - 1.97 (m, 2H), 1.94 - 1.79 (m, 4H), 1.59 (q, $J$ = 11.8 Hz, 2H), 1.42 (s, 1H), 1.22 - 1.10 (m, 2H), 0.87 (t, $J$ = 7.3 Hz, 3H).

**Example P5: Synthesis of (S)-4-ethyl-8-fluoro-4-hydroxy-11-(((1r,4S)-4-(hydroxymethyl) cyclohexyl)amino)-9-methyl-1,12-dihydro-14H-pyrano[3',4':6,7]indolizino[1,2-b]quinoline-3,14(4H)-dione (STI-F17)**

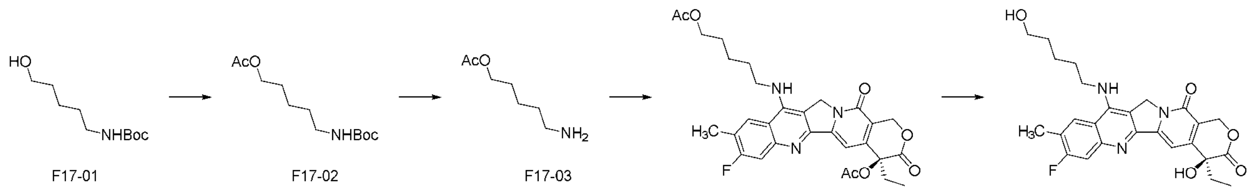

F17-01 F17-02 F17-03 F17-04 STI-F17

Step 1: Compound F17-01 (1.00 g, 4.92 mmol) was dissolved in 5 mL pyridine. Acetic anhydride (5 mL) was added. Under a nitrogen atmosphere, the reaction was heated at 40°C for 4 hours. While cooling in an ice bath, 4 M hydrochloric acid aqueous solution was added to adjust the pH to 1~2. The mixture was extracted with ethyl acetate, washed with saturated brine, dried over anhydrous $Na_2SO_4$, and filtered. The filtrate was concentrated to dryness by rotary evaporation, giving 1.12 g of compound F17-02 as a transparent oil in 92.9% yield. $^1$H NMR (400 MHz, $CDCl_3$) δ 4.56 (s, 1H), 4.06 (t, $J$ = 6.6 Hz, 2H), 3.12 (q, $J$ = 6.7 Hz, 2H), 2.05 (s, 3H), 1.64 (h, $J$ = 6.2 Hz, 2H), 1.55 - 1.49 (m, 2H), 1.45 (s, 9H), 1.42 - 1.34 (m, 2H).

Step 2: Compound F17-02 (1.12 g, 4.57 mmol) was dissolved in 9 mL dichloromethane. TFA (3 mL) was added. Under a nitrogen atmosphere, the mixture was stirred at RT for 3 hours. The reaction solution was then concentrated to dryness by rotary evaporation. The crude product F17-03 was used directly in the next step.

Step 3: (S)-11-Chloro-4-ethyl-8-fluoro-9-methyl-3,14-dioxo-3,4,12,14-tetrahydro-1H-pyrano [3',4':6,7]indolizino[1,2-b]quinolin-4-yl acetate (184 mg, 0.40 mmol), Xantphos (46 mg, 0.08 mmol), palladium acetate (9 mg, 0.04 mmol), and cesium carbonate (521 mg, 1.60 mmol) were added to a reaction flask. The mixture was dissolved in 10 mL anhydrous toluene. A solution of F17-03 (114 mg, 0.80 mmol) in 2 mL 1,4-dioxane was added. Under a nitrogen atmosphere, the mixture was heated to 100°C and reacted for 100 minutes. The system was then concentrated to dryness. Purification by

silica gel column chromatography (DCM:MeOH = 300:1 to 200:1) afforded 300 mg of crude compound F17-04 as a brown solid.

Step 4: Compound F17-04 (140 mg, 0.25 mmol) was dissolved in 11 mL methanol. Lithium hydroxide (70 mg, 2.92 mmol) was added. Under a nitrogen atmosphere, the mixture was stirred at RT for 1 hour. The pH was adjusted to 6 with glacial acetic acid. High-pressure reverse-phase prep-LC (mobile phase: methanol/0.05% formic acid aqueous solution) and lyophilization afforded 15 mg of compound (STI-F17) as a yellowish solid in 12.5% yield. LCMS (254 nm): Purity 99.7%, Rt = 1.55 min; MS Calculated: 481.2; MS Found: 482.1 $[M+H]^+$. $^1H$ NMR (400 MHz, DMSO-$d_6$) δ 8.26 (d, *J* = 8.1 Hz, 1H), 7.54 (d, *J* = 11.1 Hz, 1H), 7.31 (t, *J* = 5.9 Hz, 1H), 7.20 (s, 1H), 6.47 (s, 1H), 5.40 (s, 2H), 5.36 (s, 2H), 4.43 (t, *J* = 5.5 Hz, 1H), 3.60 (q, *J* = 6.7 Hz, 4H), 2.42 (s, 3H), 1.93 - 1.76 (m, *J* = 7.2 Hz, 2H), 1.94 - 1.79 (m, 4H), 1.56 - 1.39 (m, 4H), 0.87 (t, *J* = 7.3 Hz, 3H).

**Example P6: Synthesis of (S)-7-ethyl-7-hydroxy-14-((4-hydroxybutyl)amino)-10,13-dihydro-11H-[1,3]dioxolo [4,5-g]pyrano[3',4':6,7]indolizino[1,2-b]quinoline-8,11(7H)-dione (STI-F18)**

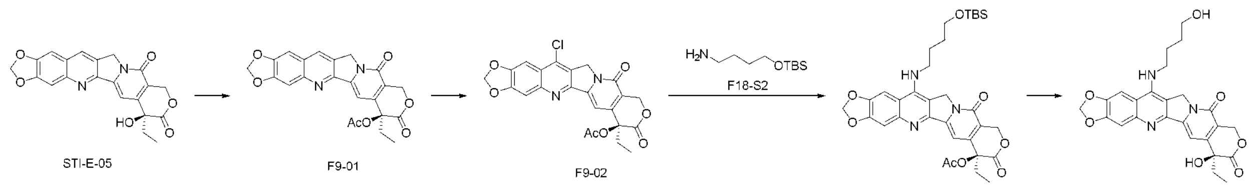


STI-E-05 F9-01 F9-02 F18-01 STI-F18

Step 1: Compound STI-E-05 (1.0 g, 2.6 mmol) was added to 6 mL pyridine, followed by 6 mL acetic anhydride. Under a nitrogen atmosphere, the mixture was heated at 40°C with stirring, and the reaction was monitored by LCMS. After reaction completion, the mixture was concentrated under reduced pressure. Trituration with acetone afforded 360 mg of compound F9-01 as a yellow solid in 33.0% yield.

Step 2: Compound F9-01 (360 mg, 0.83 mmol) was dissolved in 12 mL acetic acid. A 30% hydrogen peroxide aqueous solution (4 mL) was added. Under a nitrogen atmosphere, the mixture was heated at 70°C with stirring for 3.0 hours. Upon confirmation of completion by LCMS, the mixture was concentrated under reduced pressure. The residue was poured into ice water, extracted with DCM, dried over anhydrous $Na_2SO_4$ and concentrated to dryness under reduced pressure. The residue was dissolved in 16 mL DMF and cooled to 0°C in an ice bath. Oxalyl chloride (1.1 mL) was added dropwise. After the addition, the mixture was warmed to RT with stirring and the reaction was monitored by LCMS. Upon completion, water was added to quench the reaction. The mixture was filtered. The filter cake was washed with deionized water and dried under reduced pressure to afford 300 mg of compound F9-02 as a yellow solid in 77.3% yield. LCMS (254 nm): Purity 92.13%, Rt = 1.83 min; MS Calculated: 468.1; MS Found: 469.1 $[M+H]^+$.

Step 3: Compound F9-02 (140 mg, 0.30 mmol), F18-S2 (122 mg, 0.60 mmol), Xantphos (35 mg, 0.06 mmol), palladium acetate (7 mg, 0.03 mmol), and cesium carbonate (392 mg, 0.60 mmol) were added to a reaction flask. The mixture was dissolved in 6 mL anhydrous toluene and 6 mL 1,4-dioxane. Under a nitrogen atmosphere, the mixture was heated to 100°C and reacted for 2 hours. The system was then concentrated to dryness. Purification by silica gel column chromatography (DCM:MeOH = 100:1) afforded 50 mg of compound F18-01 as a red solid in 26.2% yield. LCMS (254 nm) purity 78.92%, Rt = 2.22 min; MS Calculated: 635.3; MS Found: 636.3 $[M+H]^+$.

Step 4: Compound F18-01 (50 mg, 0.08 mmol) was dissolved in 1 mL dichloromethane. A 4 M solution of hydrogen chloride in dioxane was added, and the reaction was stirred at RT for 1 hour. Upon confirmation of completion by LCMS, the reaction was concentrated to dryness under reduced pressure. Methanol (3 mL) and lithium hydroxide (50 mg, 2.08 mmol) were added to the residue, and the mixture was stirred at RT for 20 minutes. The pH was adjusted to 3~4 with glacial acetic acid. After concentration, high-pressure reverse-phase prep-LC (mobile phase: acetonitrile/0.05% TFA aqueous solution) and lyophilization afforded 5 mg of compound STI-F18 as a yellowish solid in 13.3% yield. LCMS (254 nm): Purity 97.86%, Rt = 1.38 min; MS Calculated: 479.2; MS Found: 480.2 $[M+H]^+$. $^1H$ NMR (400 MHz, DMSO-$d_6$) δ 7.94 (s, 1H), 7.67 (brs, 1H), 7.47(s, 1H), 7.29 (s, 1H), 6.63 (d, *J* = 13.0 Hz, 2H), 6.31 (d, 1H), 5.46 (s, 2H), 5.43 (s, 2H), 3.70 (q, *J* = 7.0 Hz, 2H), 3.48 (d, *J* = 6.7 Hz, 2H), 1.92 - 1.82 (m, 2H), 1.81 - 1.71 (m, 2H), 1.61 - 1.54 (m, 2H), 0.88 (t, *J* = 7.3 Hz, 3H).

Intermediate compound F18-S2 was prepared as follows:

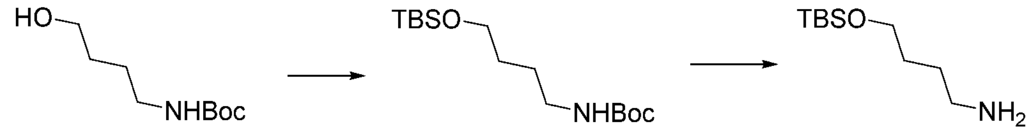

F18-S1 F18-S2

Step 1: 4-(N-tert-butoxycarbonylamino)-1-butanol (3785 mg, 20.0 mmol) was dissolved in 20 mL dichloromethane. Triethylamine (4.17 mL, 30.0 mmol) was added. Under a nitrogen atmosphere, a solution of tert-butyldimethylchlorosilane (3617 mg, 24.00 mmol) in 10 mL dichloromethane was added dropwise. The reaction was carried out at RT overnight. The mixture was extracted with saturated brine, followed by dichloromethane twice. The organic phases were combined, and dried over anhydrous $Na_2SO_4$. The solvent was removed by rotary evaporation to afford 4.49 g of compound F18-S1 as a colorless oil in 74.1% yield. $^1$H NMR (400 MHz, DMSO-$d_6$) δ 6.74 (t, *J* = 5.7 Hz, 1H), 3.54 (t, *J* = 5.6 Hz, 2H), 2.88 (q, *J* = 6.2 Hz, 2H), 1.40 - 1.38 (m, 4H), 1.35 (s, 9H), 0.87 (s, 9H), 0.04 (s, 6H).

Step 2: Compound F18-S1 (712 mg, 2.35 mmol) was dissolved in 5 mL dichloromethane. Under a nitrogen atmosphere, the mixture was cooled to 0~5°C, and iodotrimethylsilane (604 mg, 2.82 mmol) was added. The reaction was maintained at constant temperature for 3 hours. After completion of the reaction was confirmed by TLC (DCM:MeOH = 10:1, product Rf = 0.2), methanol (0.1 mL) was added to the reaction system. The mixture was concentrated to dryness under reduced pressure. Purification by column chromatography (DCM:MeOH = 50:1) afforded 400 mg of compound F18-S2 as a yellowish solid in 83.8% yield. $^1$H NMR (400 MHz, DMSO-$d_6$) δ 7.57 (s, 2H), 3.55 (t, *J* = 5.9 Hz, 2H), 2.76 (t, *J* = 7.5 Hz, 2H), 1.62 - 1.39 (m, 4H), 0.87 (s, 9H), 0.04 (s, 6H).

**Example P7: Synthesis of (S)-7-ethyl-7-hydroxy-14-((4-hydroxybutyl)amino)-10,13-dihydro-11H-[1,3]dioxolo [4,5-g]pyrano[3',4':6,7]indolizino[1,2-b]quinoline-8,11(7H)-dione (STI-G5)**

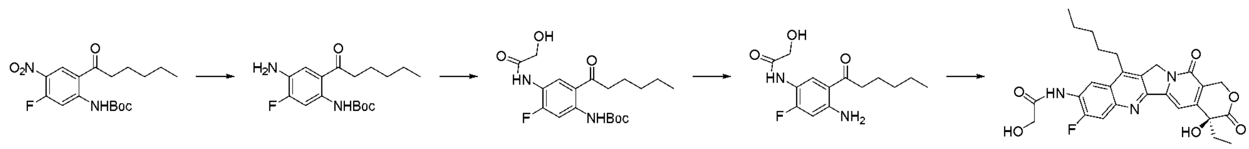

G5-01 G5-02 G5-03 G5-04 STI-G5

Step 1: Compound G5-01 (1.4 g, 4.32 mmol) was dissolved in 40 mL ethanol/water (3:1). Iron powder (965 mg, 17.28 mmol) and ammonium chloride (231 mg, 4.32 mmol) were added. After purging with nitrogen, the reaction was carried out at 80°C for 1 hour. After TLC monitoring indicated complete conversion of the starting material, the mixture was filtered while hot and the reaction solution was concentrated to dryness. The crude product was purified by column chromatography to afford 1.0 g of compound G5-02 as a brown oil in 71.4% yield. LCMS: Rt = 2.31 min; MS Calculated: 324.2; MS Found: 323.2 $[M-H]^-$.

Step 2: Compound G5-02 (1.0 g, 3.09 mmol) and 4-dimethylaminopyridine (38 mg, 0.309 mmol) were dissolved in 30 mL N,N-dimethylformamide. After purging with nitrogen, N,N-diisopropylethylamine (1.2 g, 9.25 mmol) and acetoxyacetyl chloride (842 mg, 6.18 mmol) were added. The reaction was carried out at RT for 2 hours. After TLC monitoring indicated complete conversion of the starting material, the reaction was quenched and extracted with ethyl acetate and water for three times. The organic layers were combined, washed with saturated brine, dried over anhydrous $Na_2SO_4$, and concentrated by rotary evaporation. The crude product was redissolved in 15 mL methanol. Lithium hydroxide (1.0 g) was added, and the reaction was carried out at RT for 1 hour. After TLC monitoring indicated complete conversion of the starting material, the reaction was quenched and extracted with ethyl acetate and water for three times. The organic layers were combined, washed with saturated brine, dried over anhydrous $Na_2SO_4$, and concentrated by rotary evaporation to afford 750 mg of compound G5-03 as a dark yellow oil in 63.4% yield. The crude G5-03 was used directly in the next step. LCMS: Rt = 2.21 min; MS Calculated: 382.2; MS Found: 381.2 $[M-H]^-$.

Step 3: Compound G5-03 (750 mg, 1.96 mmol) was dissolved in 10 mL a 50% solution of TFA in dichloromethane. The mixture was stirred at RT for 1 hour. After TLC confirmed the completion of the reaction, the reaction was quenched and extracted with dichloromethane and water for three times. The organic layers were combined, washed with saturated brine, dried over anhydrous $Na_2SO_4$, and concentrated to dryness by rotary evaporation. The crude product was purified by column chromatography to afford 450 mg of compound G5-04 as a black oil in 81.4% yield. LCMS: Rt = 1.70 min; MS Calculated: 282.1; MS Found: 283.3 $[M+H]^+$.

Step 4: Compound G5-04 (450 mg, 1.60 mmol) was dissolved in 15 mL toluene. (S)-4-ethyl-4-hydroxy-7,8-dihydro-1H-pyrano[3,4-f]indolizine-3,6,10(4H)-one (461 mg, 1.75 mmol) was added. Under a nitrogen atmosphere, the mixture was stirred at 130°C for 30 minutes, then TsOH (69 mg, 0.40 mmol) was added. The reaction was continued at 130°C for 2 hours. Solids precipitated during the reaction. After TLC confirmed the completion of the reaction, the solution was concentrated to dryness by rotary evaporation. The crude product was triturated with acetone to afford 520 mg of compound STI-G5 as a gray solid in 63.8% yield. LCMS: Rt = 1.73 min; MS Calculated: 509.2; MS Found: 510.2 $[M+H]^+$. $^1H$ NMR (400 MHz, DMSO-$d_6$) δ 9.73 - 9.64 (m, 1H), 8.95 (d, *J* = 8.5 Hz, 1H), 8.01 (d, *J* = 12.0 Hz, 1H), 7.29 (s, 1H), 6.51 (s, 1H), 5.98 (s, 1H), 5.43 (s, 2H), 5.27 (s, 2H), 4.15 (s, 2H), 3.11 (d, *J* = 8.1 Hz, 2H), 1.95 - 1.79 (m, 2H), 1.73 (t, *J* = 7.8 Hz, 2H), 1.48 - 1.34 (m, 4H), 0.88 (d, *J* = 6.7 Hz, 6H).

## Synthesis of Linker-payload Conjugates

### Example 23: Synthesis of Linker-payload Conjugate STI-A3

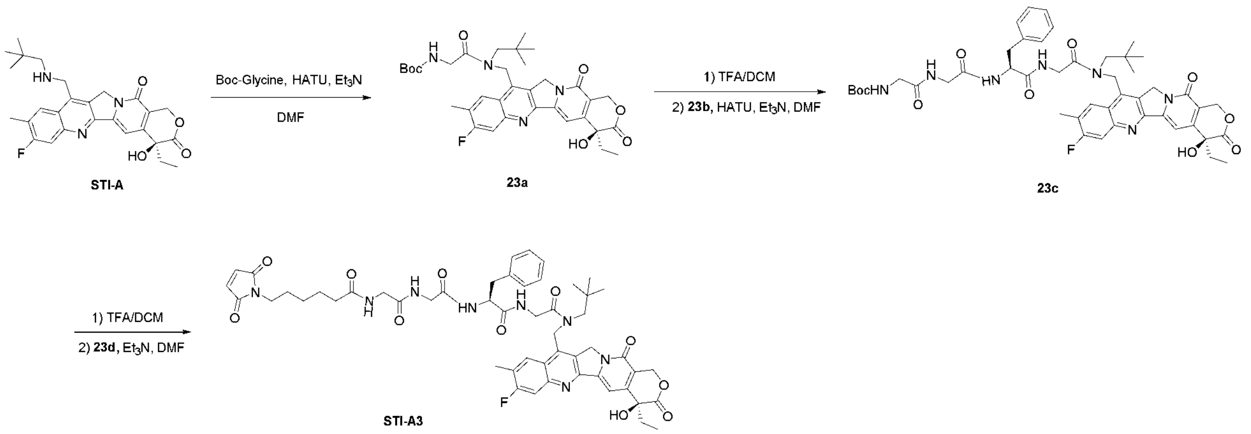

STI-A 23a 23c STI-A3

Step 1: Compound STI-A (60 mg, 0.13 mmol) from Example 3, Boc-Glycine (46 mg, 0.26 mmol), and HATU (99 mg, 0.26 mmol) were added to a reaction flask. The flask was purged with nitrogen three times. The mixture was dissolved in 1.5 mL DMF, and triethylamine (72 μL, 0.13 mmol) was added with stirring at RT for 3.5 hours. LCMS monitoring indicated approximately 30% conversion, and the reaction was stopped. Medium-to-high pressure reverse-phase prep-LC (basic mobile phase) and lyophilization afforded 9 mg of compound 23a as a yellow solid product, with 45 mg of starting material recovered. Yield: 53.6%. LCMS (254 nm) purity 98.32%, Rt = 1.98 min; MS Calculated: 636.3; MS Found: 635.4 $[M-H]^-$. $^1H$ NMR (400 MHz, DMSO-$d_6$) δ 8.23 (d, *J* = 8.0 Hz, 1H), 7.83 (d, *J* = 10.6 Hz, 1H), 7.36 (s, 1H), 5.54 - 4.92 (m, 6H), 3.64 (d, 2H), 3.55 (t, *J* = 13.2 Hz, 2H), 2.51 (s, 3H), 1.87 (qt, *J* = 13.9, 6.8 Hz, 2H), 1.21 (s, 9H), 1.06 (s, 9H), 0.89 (t, *J* = 7.3 Hz, 3H).

Step 2: Compound 23a (6 mg, 0.01 mmol) was dissolved in 1 mL DCM, and 0.3 mL TFA was added. Under a nitrogen atmosphere, the mixture was stirred at RT for 0.5 hour. Upon confirmation of completion by LCMS, the reaction was stopped and concentrated to dryness under reduced pressure. Triethylamine (14 μL, 0.10 mmol), compound 23b (4 mg, 0.01 mmol), and HATU (8 mg, 0.02 mmol) were added to the reaction flask. The flask was purged with nitrogen three times. The mixture was dissolved in 1.0 mL DMF and stirred at RT for 1 hour. LCMS confirmed the completion of the reaction. Medium-to-high pressure reverse-phase prep-LC (basic mobile phase) and lyophilization afforded 3 mg of compound 23c as a yellowish solid in 30.0% yield). LCMS (254 nm) purity 94.25%, Rt = 1.92 min; MS Calculated: 897.4; MS Found: 898.5 $[M+H]^+$. $^1H$ NMR (400 MHz, DMSO-$d_6$) δ 8.29 (d, *J*= 8.2 Hz, 1H), 7.94 (t, *J* = 5.3 Hz, 1H), 7.91 - 7.86 (m, 1H), 7.84 (d, *J* = 8.5 Hz, 1H), 7.65 (t, *J* = 5.8 Hz, 1H), 7.35 (s, 1H), 7.14 (q, *J* = 8.1 Hz, 5H), 6.72 (s, 1H), 6.34 (s, 1H), 5.51 - 5.02 (m, 6H), 4.47 (td, *J* = 9.0, 4.6 Hz, 1H), 4.08 (s, 1H), 3.85 (s, 1H), 3.72 - 3.52 (m, 4H), 3.51 (d, *J* = 6.0 Hz, 2H), 2.95 (dd, *J*= 14.0, 4.6 Hz, 1H), 2.69 (dd, *J* = 13.5, 9.4 Hz, 1H), 2.54 (s, 3H), 1.88 (p, *J* = 6.9 Hz, 2H), 1.36 (s, 9H), 1.05 (d, *J* = 10.3 Hz, 9H), 0.89 (t, *J* = 7.4 Hz, 3H).

Step 3: Compound 23c (16 mg, 0.018 mmol) was dissolved in 1 mL DCM, and 0.3 mL TFA was added. Under a nitrogen atmosphere, the mixture was stirred at RT for 0.5 hour. Upon confirmation of completion by LCMS, the reaction was stopped and concentrated to dryness under reduced pressure. Triethylamine (13 μL, 0.09 mmol) and compound 23d (11 mg, 0.036 mmol) were added. The flask was purged with nitrogen three times. The mixture was dissolved in 1.0 mL DMF and stirred at RT for 1.5 hours. LCMS confirmed the completion of the reaction. Medium-to-high pressure reverse-phase prep-LC (basic mobile phase) and lyophilization afforded 11 mg of the linker-payload conjugate STI-A3 as a white solid in 62.3% yield. LCMS (254 nm) purity 96.87%, Rt = 1.81 min; MS Calculated: 990.4; MS Found: 991.1 $[M+H]^+$. $^1H$

NMR (400 MHz, DMSO-$d_6$) δ 8.28 (d, 1H), 7.87 - 7.81 (m, 3H), 7.35 (s, 1H), 7.22 (s, 2H), 7.21 - 7.13 (m, 6H), 6.94 (s, 1H), 6.34 - 6.29 (m, 1H), 5.42 - 5.30 (m, 6H), 4.47 - 4.45 (m, 1H), 3.69 - 3.64 (m, 4H), 3.57 (d, *J* = 6.1 Hz, 2H), 3.49 (d, *J* = 6.1 Hz, 2H), 3.37 (t, *J* = 7.1 Hz, 2H), 2.94 (s, 1H), 2.54 (s, 3H), 2.21 - 2.16 (m, 2H), 1.90 - 1.85 (m, 2H), 1.50 - 1.46 (m, 6H), 1.05 (s, 9H), 0.92 (t, *J* = 7.8 Hz, 3H).

Intermediate compound 23b was prepared as follows:

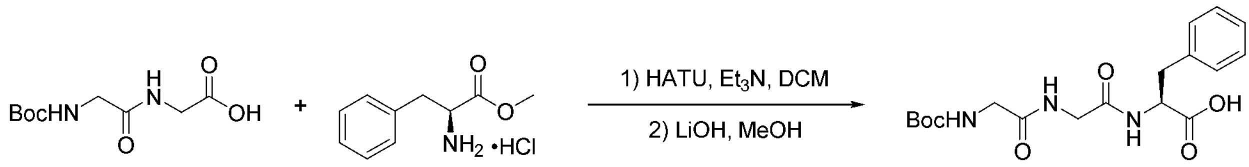


**23b-1** **23b-2** **23b**

Compounds 23b-1 (1.0 g, 4.31 mmol), 23b-2 (930 mg, 4.31 mmol), and HATU (2.458 g, 6.47 mmol) were added to a reaction flask. The flask was purged with nitrogen three times. The mixture was dissolved in 20 mL DCM, and triethylamine (2.4 mL, 17.24 mmol) was added. The reaction was stirred at RT for 7.0 hours. Upon confirmation of completion by LCMS, the reaction was stopped. The mixture was concentrated under reduced pressure, extracted with EA and water, and triturated with PE and DCM. The mixture was filtered. The resulting solid was dissolved in methanol, and cooled in an ice bath. LiOH (310 mg, 12.93 mmol) was added. The mixture was stirred at 0°C for 30 minutes, then warmed to RT and stirred for 3 hours. LCMS confirmed the completion of the reaction. The mixture was concentrated to dryness by rotary evaporation, and the pH was adjusted to 2~3 with 1 M hydrochloric acid. The mixture was extracted with EA and water, dried over anhydrous $Na_2SO_4$, and concentrated to dryness by rotary evaporation, giving 694 mg of compound 23b as a white solid in 53.6% yield. LCMS (214 nm) purity 97.25%, Rt = 1.25 min; MS Calculated: 379.2; MS Found: 378.2 [M-H]-. $^1$H NMR (400 MHz, DMSO-$d_6$) δ 12.70 (s, 1H), 8.15 (d, *J* = 8.1 Hz, 1H), 7.93 (t, *J*= 5.7 Hz, 1H), 7.28 (t, *J* = 7.4 Hz, 2H), 7.21 (d, *J* = 7.5 Hz, 3H), 6.99 (t, *J* = 6.0 Hz, 1H), 4.42 (td, *J* = 8.5, 5.0 Hz, 1H), 3.69 (qd, *J* = 16.8, 5.6 Hz, 2H), 3.55 (d, *J* = 6.0 Hz, 2H), 3.05 (dd, *J* = 13.8, 5.1 Hz, 1H), 2.87 (dd, *J* = 13.8, 9.0 Hz, 1H), 1.38 (s, 9H).

Intermediate compound 23d was prepared as follows:

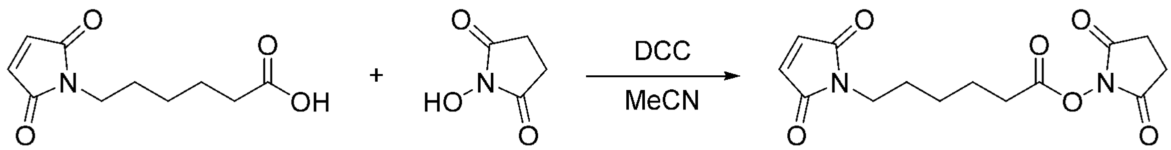


**23d-1** **23d-2** **23d**

In an ice bath, compound 23d-1 (500 mg, 2.37 mmol) and compound 23d-2 (273 mg, 2.37 mmol) were dissolved in acetonitrile. DCC (489 mg, 2.37 mmol) was added. Under a nitrogen atmosphere, the mixture was stirred at 0°C for 2 hours and then allowed to warm to RT. Reaction was carried out overnight. After TLC (PE:EA = 1:1, product Rf = 0.5) confirmed the completion of the reaction, the reaction was stopped. The mixture was filtered, and the filtrate was subjected to silica gel column chromatography (PE:EA = 4:1 to 1:1). The product was concentrated to dryness under reduced pressure to afford 548 mg of compound 23d as a white solid in 75.1% yield. $^1$H NMR (400 MHz, DMSO-$d_6$) δ 7.00 (s, 2H), 3.39 (t, *J* = 7.0 Hz, 2H), 2.81 (s, 4H), 2.65 (t, *J* = 7.3 Hz, 2H), 1.62 (p, *J* = 7.4 Hz, 2H), 1.51 (h, *J* = 7.2 Hz, 2H), 1.31 (q, *J* = 8.0 Hz, 2H).

**Example 24: Synthesis of Linker-payload Conjugate STI-F3**

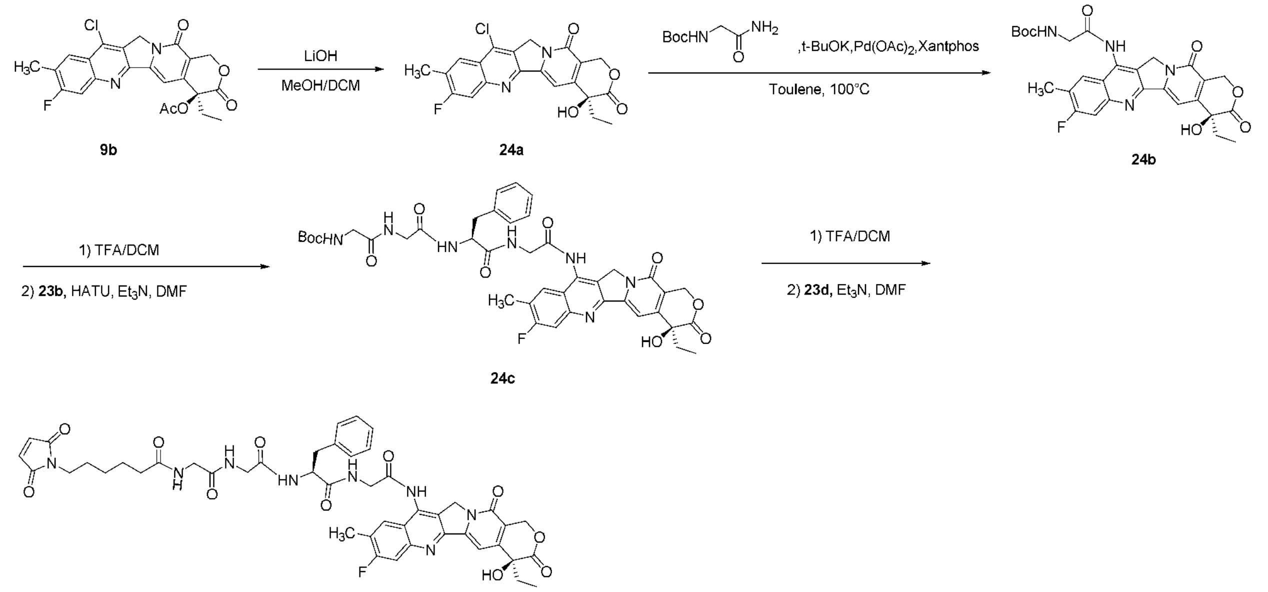


9b 24a 24b 24c STI-F3

Step 1: Compound 9b (1046 mg, 2.3 mmol) from Example 9 was dissolved in a mixed solvent of 60 mL dichloromethane and 60 mL methanol. Lithium hydroxide (166 mg, 6.9 mmol) was added, and the reaction was carried out at RT for 1 hour. The system was concentrated to dryness under reduced pressure. The pH was adjusted to 3~4 with 1 M hydrochloric acid, and the mixture was extracted four times with dichloromethane. The organic phase was concentrated to afford approximately 1.0 g of compound 24a as a brownish-red solid. LCMS (254 nm): Purity 93.42%, Rt = 1.88 min; MS Calculated: 414.1; MS Found: 415.0 $[M+H]^+$. $^1H$ NMR (400 MHz, DMSO-$d_6$) δ 8.22 (d, *J* = 8.0 Hz, 1H), 7.98 (d, *J* = 10.6 Hz, 1H), 7.31 (s, 1H), 6.56 (s, 1H), 5.44 (s, 2H), 5.25 (s, 2H), 2.54 (s, 3H), 1.87 (hept, *J* = 7.0 Hz, 2H), 0.88 (t, *J* = 7.3 Hz, 3H).

Step 2: Compound 24a (200 mg, 0.48 mmol) and Boc-glycinamide (126 mg, 0.72 mmol) were dissolved in 6 mL anhydrous toluene. Under a nitrogen atmosphere, potassium tert-butoxide (108 mg, 0.96 mmol), Xantphos (28 mg, 0.048 mmol), and palladium acetate (11 mg, 0.048 mmol) were added. The mixture was stirred thoroughly and heated to 100°C for 3.5 hours. After completion was confirmed by TLC (DCM:MeOH = 15:1, product Rf = 0.2), the mixture was concentrated to dryness under reduced pressure. The residue was extracted with 1 M hydrochloric acid and dichloromethane. The organic phase was concentrated and purified by silica gel column chromatography to afford 39 mg of compound 24b as a red solid, with 40 mg of starting material 24a recovered. Yield: 67.0%. $^1H$ NMR (400 MHz, DMSO-$d_6$) δ 10.80 (s, 1H), 8.17 (d, *J* = 8.1 Hz, 1H), 7.85 (d, *J* = 10.7 Hz, 1H), 7.30 (s, 2H), 6.52 (s, 1H), 5.42 (s, 2H), 5.03 (s, 2H), 4.04 (d, *J* = 6.0 Hz, 2H), 2.50 (s, 3H), 1.91 - 1.81 (m, 2H), 1.44 (s, 9H), 0.88 (t, *J* = 7.3 Hz, 3H).

Step 3: Compound 24b (39 mg, 0.07 mmol) was dissolved in 1 mL dichloromethane. Under a nitrogen atmosphere, 0.3 mL TFA was added, and the mixture was stirred at RT for 30 minutes. Upon confirmation of completion by LCMS, the reaction was concentrated to dryness under reduced pressure. The crude product was dissolved in 1 mL anhydrous DMF. Under a nitrogen atmosphere, triethylamine (1 mL, 0.71 mmol), 23b (27 mg, 0.07 mmol), and HATU (41 mg, 0.11 mmol) were added. The reaction was carried out at RT for 1.5 hours. Upon confirmation of completion by LCMS, the DMF was removed by rotary evaporation. The residue was extracted three times with ethyl acetate and water, washed with saturated brine, dried over anhydrous $Na_2SO_4$, and subjected to rotary evaporation, giving 50 mg of compound 24c as a pale pink product in 87.2% yield. LCMS (254 nm) purity 85.45%, Rt = 1.71 min; MS Calculated: 813.3; MS Found: 814.1 $[M+H]^+$. $^1H$ NMR (400 MHz, DMSO-$d_6$) δ 10.80 (s, 1H), 8.56 (t, *J* = 5.8 Hz, 1H), 8.53 (dd, *J* = 8.4, 1.4 Hz, 1H), 8.24 (s, 1H), 8.21 (d, *J* = 8.6 Hz, 1H), 7.91 (d, *J* = 10.7 Hz, 2H), 7.32 (s, 1H), 7.29 - 7.23 (m, 5H), 6.52 (s, 1H), 5.42 (s, 2H), 5.10 (s, 2H), 4.66 - 4.58 (m, 1H), 4.22 (d, *J* = 6.4 Hz, 2H), 3.78 (dd, *J* = 16.6, 5.7 Hz, 1H), 3.61 (dd, *J* = 17.4, 4.7 Hz, 1H), 3.53 (d, *J* = 5.7 Hz, 2H), 2.52 (s, 3H), 1.89 - 1.82 (m, 2H), 1.36 (s, 9H), 0.87 (s, 3H).

Step 4: Compound 24c (45 mg, 0.055 mmol) was dissolved in 1.0 mL DCM, and 0.4 mL TFA was added. Under a nitrogen atmosphere, the mixture was stirred at RT for 0.5 hour. Upon confirmation of completion by LCMS, the reaction was stopped and concentrated to dryness under reduced pressure. Triethylamine (76 μL, 0.550 mmol) and compound 23d (34 mg, 0.110 mmol) were added. The flask was purged with nitrogen three times. The mixture was dissolved in 1.0 mL DMF and stirred at RT for 2.5 hours. LCMS confirmed the completion of the reaction. Medium-to-high pressure reverse-phase prep-LC (basic mobile phase) afforded 11 mg of the linker-payload conjugate STI-F3 as a yellow solid. LCMS (254 nm) purity 100%, Rt = 1.64 min; MS Calculated: 906.3; MS Found: 907.0 $[M+H]^+$. $^1H$ NMR (400 MHz, DMSO-$d_6$) δ 10.79 (s, 1H), 8.53 (t, *J* = 5.7 Hz, 1H), 8.20 (dd, *J* = 8.5, 2.7 Hz, 2H), 8.06 (t, *J* = 5.8 Hz, 1H), 8.01 (t, *J* = 5.4 Hz, 1H), 7.90 (d, *J* = 10.8 Hz, 1H), 7.32 (s, 1H), 7.31 - 7.21 (m, 5H), 6.97 (s, 2H), 6.52 (s, 1H), 5.42 (s, 2H), 5.09 (s, 2H), 4.60 (td, *J* = 8.9, 4.3 Hz, 1H), 4.24

(d, *J* = 7.9 Hz, 2H), 3.77 (dd, *J* = 16.7, 5.8 Hz, 2H), 3.71 - 3.55 (m, 6H), 2.51 (s, 3H), 2.09 (t, *J* = 7.5 Hz, 2H), 1.94 - 1.78 (m, 2H), 1.46 (t, *J* = 8.0 Hz, 6H), 0.88 (t, *J* = 7.4 Hz, 3H).

**Example 25: Synthesis of Linker-payload Conjugate STI-F4**

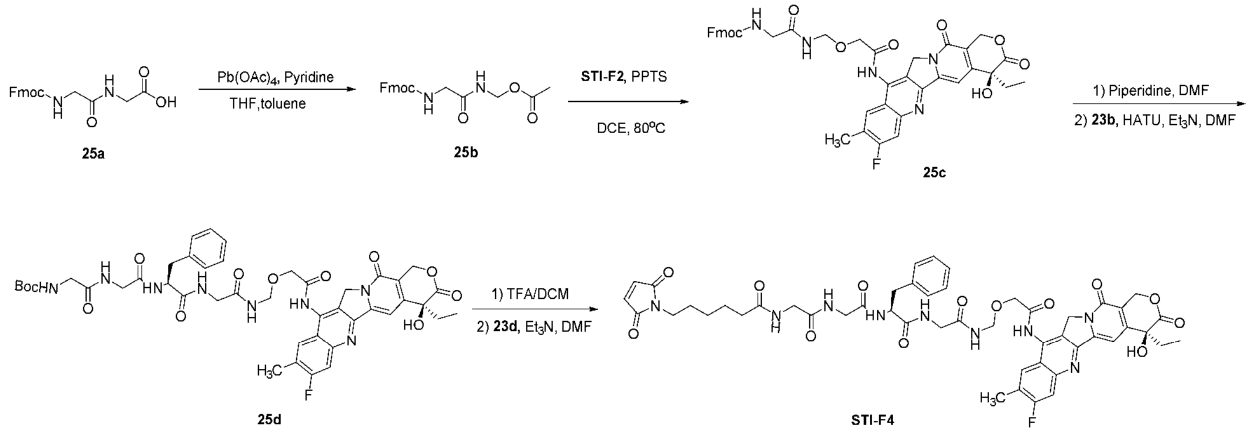

25a 25b 25c 25d STI-F4

Step 1: Compound 25a (4.3 g, 12.2 mmol) and lead tetraacetate (6.8 g, 14.7 mmol) were added to a reaction flask. Under a nitrogen atmosphere, the substances were dissolved in tetrahydrofuran (120.0 mL) and toluene (40.0 mL), followed by the addition of pyridine (1.16 mL, 14.7 mmol). The mixture was heated to 85°C and reacted for 3 hours, then stopped. The mixture was filtered through a pad of Celite and extracted with EA and water. The organic phase was dried over anhydrous $Na_2SO_4$ and concentrated to dryness under reduced pressure. Purification by column chromatography (DCM:MeOH = 50:1) afforded 4.4 g of compound 25b as a white solid in 98.0% yield. LCMS (254 nm) purity 97.2%, Rt = 1.789 min; MS Calculated: 368.1; MS Found: 386.1 $[M+NH_4]^+$.

Step 2: Compound STI-F2 (60 mg, 0.13 mmol) from Example 9, 25b (150 mg, 0.39 mmol), and pyridinium p-toluenesulfonate (2 mg, 0.01 mmol) were added to a reaction flask. DCE (6.0 mL) was added. Under a nitrogen atmosphere, the mixture was heated to 80°C and reacted overnight. The system was then concentrated to dryness under reduced pressure and purified by pre-TLC (DCM:MeOH = 15:1) to afford 40 mg of compound 25c as a yellow solid in 34.1% yield. LCMS (254 nm) purity 93.2%, Rt = 1.811 min; MS Calculated: 761.2; MS Found: 762.3 $[M+H]^+$.

Step 3: Compound 25c (40 mg, 0.053 mmol) was dissolved in DMF (0.8 mL). Piperidine (0.2 mL) was added, and the reaction was carried out at RT for 20 minutes. The system was concentrated to dryness, diluted with water, and lyophilized. To the residue, compound 23b (21 mg, 0.056 mmol), HATU (30 mg, 0.08 mmol), and DMF (1.0 mL) were added. Under a nitrogen atmosphere, triethylamine (16 mg, 0.159 mmol) was added, and the reaction was carried out at RT for 1 hour. The reaction was then stopped, and the system was concentrated to dryness. EA and saturated brine were added for extraction. The aqueous phase was further washed with EA (10.0 mL × 5). The organic phases were combined, dried over anhydrous $Na_2SO_4$ and concentrated to dryness under reduced pressure to afford 30 mg of compound 25d as a yellowish solid in 62.9% yield. LCMS (254 nm) purity 97.2%, Rt = 1.662 min; MS Calculated: 900.3; MS Found: 901.4 $[M+H]^+$.

Step 4: Compound 25d (30 mg, 0.033 mmol) was dissolved in 1.0 mL DCM. Under a nitrogen atmosphere, 0.3 mL TFA was added, and the reaction was carried out at RT for 20 minutes. Upon confirmation of completion by LCMS, the reaction was stopped. The system was concentrated to dryness under reduced pressure. To the residue was added 1.0 mL DMF. Under a nitrogen atmosphere, triethylamine (22 mg, 0.22 mmol) and 23d (10 mg, 0.033 mmol) were added sequentially. The reaction was carried out at RT for 20 minutes and then stopped. The system was concentrated to dryness under reduced pressure. Medium-to-high pressure reverse-phase prep-LC (acidic mobile phase) and lyophilization afforded 6 mg of the linker-payload conjugate STI-F4 as a yellow oily liquid in 27.9% yield. LCMS (254 nm) purity 98.65%, Rt = 1.591 min; MS Calculated: 993.4; MS Found: 994.4 $[M+H]^+$. $^1H$ NMR (400 MHz, DMSO-$d_6$) $\delta$ 10. 74(s, 1H), 8.75 (t, J = 6.4 Hz, 1H), 8.37 (t, J = 6.0 Hz, 1H), 8.15 (d, J = 8.0 Hz, 1H), 8.07 (t, J = 6.0 Hz, 1H), 8.02 (t, J = 5.6 Hz, 1H), 7.90 (d, J = 10.8 Hz, 1H), 7.32 (s, 1H), 7.27 - 7.14 (m, 6H), 6.98 (s, 2H), 6.52 (s, 1H), 5.42 (s, 2H), 5.15 (s, 2H), 4.84 - 4.77 (m, 2H), 4.52 - 4.47 (m, 1H), 4.38 (s, 2H), 3.80 (t, J = 6.0 Hz, 2H), 3.73 - 3.57 (m, 6H), 3.04 (dd, J = 14.0, 4.4 Hz, 1H), 2.82 - 2.79 (m, 1H), 2.50 (s, 3H), 2.09 (t, J = 7.6 Hz, 2H), 1.92 - 1.80 (m, 2H), 1.48 - 1.41 (m, 4H), 1.20 - 1.32 (m, 2H), 0.88 (t, J = 7.2 Hz, 3H).

**Example 26: Synthesis of Linker-payload Conjugate STI-G**

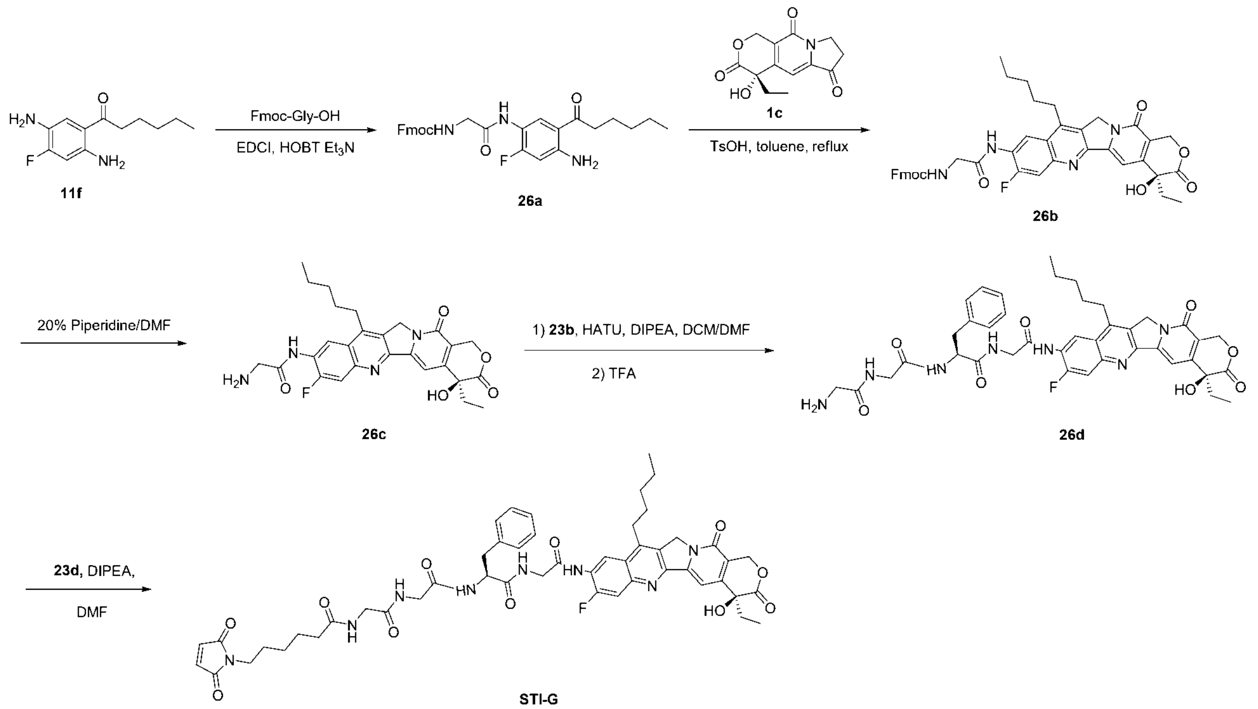


Step 1: Compound 11f (200 mg, 0.89 mmol) and Fmoc-Gly-OH (530 mg, 1.78 mmol) were dissolved in 5 mL tetrahydrofuran. $Et_3N$ (360 mg, 3.56 mmol), HOBt (240 mg, 1.78 mmol), and EDCI (340 mg, 1.78 mmol) were added to the reaction mixture. The reaction mixture was stirred at RT. After TLC monitoring indicated the completion of reaction, 10 mL ethyl acetate was added to dilute the mixture. The mixture was extracted with water, and the aqueous phase was further extracted twice with 10 mL ethyl acetate. The organic phases were combined, dried over anhydrous $Na_2SO_4$ and concentrated under reduced pressure. The resulting brown crude product was purified by silica gel column chromatography (PE:EA = 2:1). After rotary evaporation, 300 mg of compound 26a was obtained as a pink solid in 67% yield. LCMS: Rt = 2.112 min; MS Calculated: 503.2; MS Found: 504.2 $[M+H]^+$ . $^1H$ NMR (400 MHz, DMSO-$d_6$) $\delta$ 9.45 (s, 1H), 8.07 (d, *J* = 8.8 Hz, 1H), 7.90 (d, *J* = 7.6 Hz, 2H), 7.73 (d, *J* = 7.5 Hz, 1H), 7.61 (s, 1H), 7.42 (t, *J* = 7.5 Hz, 2H), 7.34 (d, *J* = 7.1 Hz, 3H), 6.57 (d, *J* = 12.9 Hz, 1H), 4.34 - 4.18 (m, 3H), 3.82 (d, *J* = 6.1 Hz, 2H), 2.81 (t, *J* = 7.4 Hz, 2H), 1.58 (t, *J*= 7.3 Hz, 2H), 1.35 - 1.19 (m, 6H), 0.90 - 0.82 (m, 3H).

Step 2: Compound 26a (200 mg, 0.40 mmol) and compound 1c (105 mg, 0.40 mmol) were dissolved in 4 mL anhydrous toluene. Under a nitrogen atmosphere, the mixture was refluxed with a water separator for 30 minutes. Then, TsOH (18 mg, 0.1 mmol) was added, and the mixture was stirred under reflux for 3.5 hours. After the completion of the reaction was confirmed by TLC (DCM:MeOH = 10:1, product Rf = 0.5) and LCMS, the reaction system was cooled to RT and concentrated to dryness by rotary evaporation. The residue was triturated with acetone and filtered to afford 200 mg of compound 26b as a yellow solid in 70% yield. LCMS (254 nm): Purity 96.96%, $R_t$ = 2.084 min; MS Calculated: 730.3; MS Found: 731.3$[M+H]^+$. $^1H$ NMR (400 MHz, DMSO-$d_6$) $\delta$ 10.21 (s, 1H), 9.00 (d, *J* = 8.4 Hz, 1H), 8.01 (d, *J* = 12.0 Hz, 1H), 7.90 (d, *J* = 7.6 Hz, 2H), 7.75 (d, *J* = 7.8 Hz, 2H), 7.49 - 7.41 (m, 2H), 7.37 - 7.28 (m, 3H), 7.11 (d, *J* = 7.9 Hz, 1H), 5.43 (s, 2H), 5.28 (s, 2H), 4.37 - 4.24 (m, 3H), 4.02 (d, *J* = 6.0 Hz, 2H), 3.11 (t, *J* = 8.0 Hz, 2H), 2.28 (s, 1H), 1.86 (p, *J* = 7.1 Hz, 2H), 1.72 (t, *J* = 7.7 Hz, 2H), 1.42 (d, *J* = 7.4 Hz, 2H), 1.34 (d, *J* = 7.1 Hz, 2H), 0.86 (d, *J* = 7.6 Hz, 6H).

Step 3: Compound 26b (50 mg, 0.068 mmol) was dissolved in 2 mL a 20% piperidine/DMF solution. The reaction was carried out with stirring at RT for 30 minutes. After the completion of the reaction was confirmed by TLC (DCM:MeOH = 10:1, product Rf = 0.2) and LCMS, the reaction solution was directly purified by silica gel column chromatography (DCM:MeOH = 20:1). After rotary evaporation, 20 mg of compound 26c was obtained as a white solid in 57.8% yield. LCMS: Rt = 1.670 min; MS Calculated: 508.2; MS Found: 509.3 $[M+H]^+$.

Step 4: Compound 26c (20 mg, 0.039 mmol) was dissolved in 1 mL a DCM/DMF (9:1) mixed solvent. Compound 23b (30 mg, 0.078 mmol), HATU (30 mg, 0.078 mmol), and DIPEA (25 mg, 0.195 mmol) were added. The reaction was carried out at RT for 30 minutes. After the completion of the reaction was confirmed by TLC (DCM:MeOH = 10:1, product Rf = 0.4) and LCMS, 1 mL TFA was directly added to the reaction solution. The reaction was carried out at RT for 4 hours. Upon

confirmation of completion by LCMS, the reaction solution was concentrated to dryness by rotary evaporation. The crude product was subjected to medium-to-high pressure reverse-phase prep-LC (acidic mobile phase) followed by lyophilization to afford 10 mg of compound 26d as a yellow solid in 33.3% yield. LCMS: Rt = 1.616 min; MS Calculated: 769.3; MS Found: 770.4 $[M+H]^+$. $^1H$ NMR (400 MHz, DMSO-$d_6$) δ 10.23 (s, 1H), 8.99 (d, *J* = 8.4 Hz, 1H), 8.58 (t, *J* = 6.0 Hz, 1H), 8.50 (t, *J* = 5.6 Hz, 1H), 8.36 (d, *J* = 8.4 Hz, 1H), 8.02 (d, *J* = 12.1 Hz, 1H), 7.98 (s, 2H), 7.28 (t, *J* = 7.7 Hz, 5H), 7.18 (t, *J* = 6.9 Hz, 1H), 6.52 (s, 1H), 5.43 (s, 2H), 5.29 (s, 2H), 4.65 (td, *J* = 9.2, 4.3 Hz, 1H), 4.12 (d, *J* = 5.7 Hz, 2H), 3.89 (dd, *J* = 16.8, 5.8 Hz, 1H), 3.71 (dd, *J* = 16.8, 5.3 Hz, 1H), 3.57 (s, 2H), 3.12 (q, *J* = 5.7, 4.9 Hz, 3H), 2.81 (dd, *J* = 13.8, 9.8 Hz, 1H), 1.86 (p, *J* = 7.2 Hz, 2H), 1.74 (t, *J* = 7.7 Hz, 2H), 1.41 (ddd, *J* = 21.6, 11.3, 5.2 Hz, 4H), 0.87 (td, *J* = 7.1, 3.8 Hz, 6H).

Step 5: Compound 26d (20 mg, 0.026 mmol) was dissolved in 0.2 mL DMF. Compound 23d (16 mg, 0.052 mmol) and DIPEA (14 mg, 0.104 mmol) were added. The reaction was carried out at RT for 30 minutes. Upon confirmation of completion by LCMS, the reaction solution was directly purified by medium-to-high pressure reverse-phase prep-LC (acidic mobile phase) followed by lyophilization to afford 6 mg of the linker-payload conjugate STI-G as a white solid in 23.9% yield. LCMS (254 nm): Purity 99.08%, Rt = 1.693 min; MS Calculated: 962.4; MS Found: 963.1 $[M+H]^+$. $^1H$ NMR (400 MHz, DMSO-$d_6$) δ 10.19 (s, 1H), 9.00 (dd, *J* = 8.5, 3.4 Hz, 1H), 8.45 (dt, *J* = 9.2, 5.8 Hz, 3H), 8.14 (d, *J* = 8.3 Hz, 1H), 8.07 (t, *J* = 5.7 Hz, 1H), 8.04 - 7.98 (m, 2H), 7.31 - 7.24 (m, 5H), 7.21 - 7.15 (m, 1H), 6.98 (s, 1H), 6.53 (d, *J* = 7.8 Hz, 1H), 5.41 (d, *J* = 14.1 Hz, 2H), 5.31 (d, *J* = 11.2 Hz, 2H), 4.62 - 4.50 (m, 2H), 4.11 (d, *J* = 5.8 Hz, 2H), 3.74 (d, *J* = 6.2 Hz, 1H), 3.67 (d, *J* = 5.7 Hz, 2H), 3.60 (d, *J* = 11.3 Hz, 1H), 3.11 (dd, *J* = 13.1, 5.2 Hz, 3H), 2.84 (dd, *J* = 13.8, 9.8 Hz, 1H), 2.10 (q, *J* = 6.1, 4.7 Hz, 2H), 2.06 - 1.93 (m, 2H), 1.86 (p, *J* = 7.0 Hz, 2H), 1.77 - 1.70 (m, 2H), 1.50 (d, *J* = 7.0 Hz, 4H), 1.45 (dt, *J* = 7.7, 3.8 Hz, 4H), 0.89 - 0.84 (m, 6H).

**Example 27: Synthesis of Linker-payload Conjugate STI-G3**

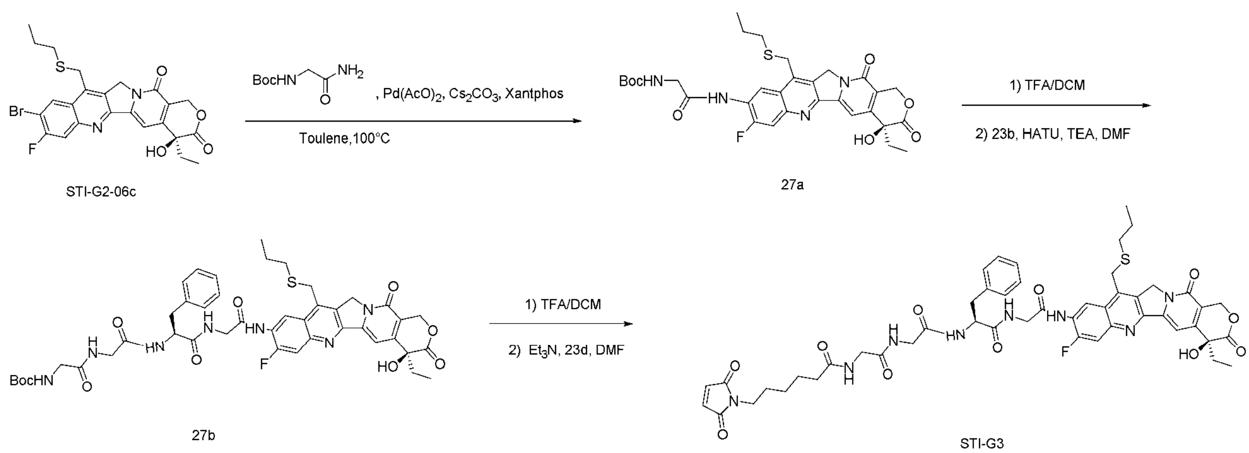

STI-G2-06c

27a

27b

STI-G3

Step 1: Under a nitrogen atmosphere, compound STI-G2-06c (176 mg, 0.33 mmol) from Example 13 and Boc-glycinamide (115 mg, 0.66 mmol) were dissolved in 5 mL anhydrous toluene. Under a nitrogen atmosphere, cesium carbonate (215 mg, 0.66 mmol), Xantphos (19 mg, 0.033 mmol), and palladium acetate (7 mg, 0.033 mmol) were added. The mixture was stirred till uniform, and heated to 100 °C for 4.0 hours. Upon confirmation of completion by LCMS, the reaction was concentrated to dryness under reduced pressure, and then dissolved in methanol. Low-to-medium pressure reverse-phase prep-LC (acidic mobile phase) afforded 45 mg of compound 27a as a brown solid in 21.7% yield. LCMS (254 nm) purity 96.8%, Rt = 1.86 min; MS Calculated: 626.2; MS Found: 627.3 $[M+H]^+$. $^1H$ NMR (400 MHz, DMSO-$d_6$) δ 10.14 (s, 1H), 9.04 (d, *J* = 8.9 Hz, 1H), 8.07 (s, 1H), 8.04 (d, *J* = 11.9 Hz, 1H), 7.30 (s, 1H), 6.52 (s, 1H), 5.43 (s, 2H), 5.31 (s, 2H), 4.66 (s, 2H), 4.30 (s, 2H), 2.59 (t, *J* = 7.2 Hz, 2H), 1.87 (hept, *J* = 7.1 Hz, 2H), 1.59 (q, *J* = 7.2 Hz, 2H), 1.42 (s, 9H), 0.93 - 0.89 (m, 3H), 0.89 - 0.86 (m, 3H).

Step 2: Compound 27a (45 mg, 0.072 mmol) was dissolved in 2 mL DCM, and 0.6 mL TFA was added. Under a nitrogen atmosphere, the mixture was stirred at RT for 1 hour. Upon confirmation of completion by LCMS, the reaction was stopped and the mixture was concentrated to dryness under reduced pressure. Triethylamine (73 mg, 0.72 mmol), compound 23b (27 mg, 0.072 mmol), and HATU (41 mg, 0.108 mmol) were added to the reaction flask. The flask was purged with nitrogen three times. The mixture was dissolved in 1.0 mL DMF and stirred at RT for 1.5 hours. LCMS confirmed the completion of the reaction. Medium-to-high pressure reverse-phase prep-LC (acidic mobile phase) and lyophilization afforded 20 mg of compound 27b as a yellow solid in 31.4% yield. LCMS (254 nm) purity 98.2%, Rt = 1.73 min; MS Calculated: 887.3; MS Found: 888.3 $[M+H]^+$. $^1H$ NMR (400 MHz, MeOD) δ 8.94 (d, *J* = 8.3 Hz, 1H), 7.65 (d, *J* = 11.8 Hz, 1H), 7.42 (s, 1H), 7.19 (dd, *J* = 4.5, 2.7 Hz, 6H), 7.10 (d, *J* = 6.3 Hz, 2H), 5.47 (d, *J* = 16.3 Hz, 1H), 5.26 (d, *J* = 16.2 Hz,

1H), 5.12 (d, *J* = 4.3 Hz, 1H), 4.85 (s, 1H), 4.65 - 4.60 (m, 2H), 4.49 (s, 2H), 4.13 (s, 2H), 4.05 (d, *J* = 11.3 Hz, 2H), 3.80 (s, 1H), 3.74 (s, 1H), 2.93 (dd, *J* = 14.0, 9.5 Hz, 2H), 2.52 (t, *J* = 7.3 Hz, 2H), 1.92 - 1.82 (m, 2H), 1.56 (p, *J* = 7.4 Hz, 2H), 1.33 (s, 11H), 0.94 - 0.88 (m, 3H), 0.88 - 0.83 (m, 3H).

Step 3: Compound 27b (17 mg, 0.019 mmol) was dissolved in 1.0 mL DCM, and 0.4 mL TFA was added. Under a nitrogen atmosphere, the mixture was stirred at RT for 0.5 hour. Upon confirmation of completion by LCMS, the reaction was stopped and the mixture was concentrated to dryness under reduced pressure. Triethylamine (26 μL, 0.190 mmol) and compound 23d (12 mg, 0.038 mmol) were added. The flask was purged with nitrogen three times. The mixture was dissolved in 1.0 mL DMF and stirred at RT for 1 hour. LCMS confirmed the completion of the reaction. Medium-to-high pressure reverse-phase prep-LC (acidic mobile phase) afforded 11 mg of the linker-payload conjugate STI-G3 as a yellow solid. LCMS (254 nm) purity 100%, Rt = 1.65 min; MS Calculated: 980.4; MS Found: 981.3 $[M+H]^+$. $^1H$ NMR (400 MHz, DMSO-$d_6$) δ 10.33 (s, 1H), 8.98 (d, *J* = 8.5 Hz, 1H), 8.10 (q, *J* = 7.8 Hz, 2H), 8.05 (d, *J* = 11.6 Hz, 1H), 7.95 (s, 1H), 7.33 - 7.23 (m, 5H), 7.20 (s, 2H), 7.01 (s, 2H), 6.67 (s, 1H), 6.54 (d, *J* = 2.2 Hz, 1H), 6.40 (d, *J* = 2.4 Hz, 1H), 5.39 (s, 2H), 4.52 (d, *J* = 23.6 Hz, 2H), 4.32 - 4.25 (m, 1H), 3.89 (t, *J* = 12.3 Hz, 2H), 3.83 - 3.73 (m, 2H), 3.63 (t, *J* = 12.4 Hz, 2H), 3.14 (d, *J* = 11.8 Hz, 1H), 2.94 (d, *J* = 12.9 Hz, 1H), 2.89 (s, 2H), 2.73 (d, *J* = 2.3 Hz, 2H), 2.10 (d, *J* = 7.7 Hz, 2H), 2.00 (t, *J* = 7.4 Hz, 2H), 1.84 (q, *J* = 7.2 Hz, 2H), 1.57 (dd, *J* = 7.2, 3.7 Hz, 2H), 1.18 (t, *J* = 8.0 Hz, 4H), 0.87 (t, *J*= 7.3 Hz, 6H).

### Example 28: Synthesis of Linker-payload Conjugate STI-F1002

Linker-payload conjugate STI-F1002 was prepared analogously to STI-F4, as shown in the following scheme:

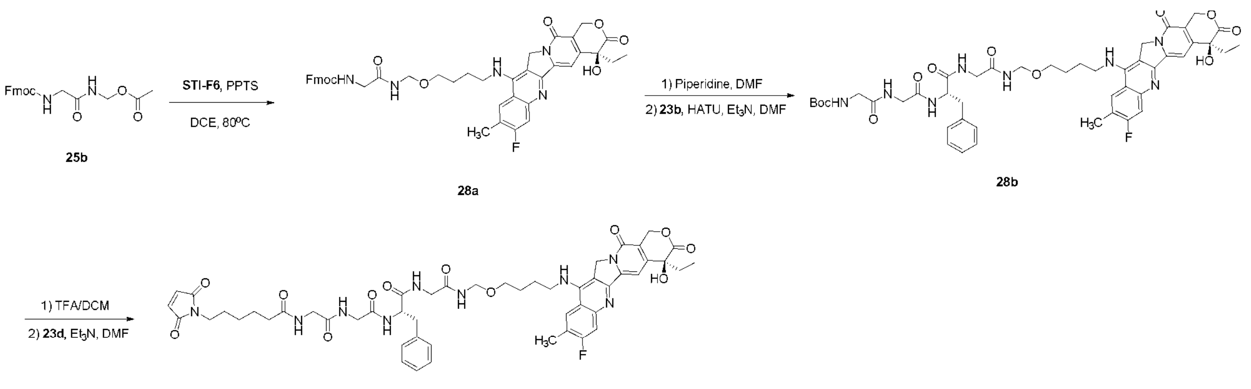

25b
28a
28b
STI-F1002

Step 1: Compound STI-F6 (40 mg, 0.09 mmol), 25b (40 mg, 0.10 mmol), and pyridinium p-toluenesulfonate (13 mg, 0.5 mmol) were added to a reaction flask. 1,2-Dichloroethane (2.0 mL) was added. Under a nitrogen atmosphere, the mixture was heated to 80°C and reacted overnight. The system was then concentrated to dryness under reduced pressure and purified by prep-TLC (DCM:MeOH = 15:1) to afford 46 mg of compound 28a as a yellow solid in 69.7% yield. LCMS (254 nm) purity 87.90%, Rt = 1.90 min; MS Calculated: 775.3; MS Found: 776.2 $[M+H]^+$.

Step 2: Compound 28a (46 mg, 0.06 mmol) was dissolved in DMF (1.8 mL). Piperidine (0.4 mL) was added, and the reaction was stirred at RT for 20 minutes. The system was concentrated to dryness. To the residue were added (tert-butoxycarbonyl)glycyl-L-phenylalanine (23b) (27 mg, 0.07 mmol), 2-(7-azabenzotriazol-1-yl)-N,N,N',N'-tetramethyluronium hexafluorophosphate (34 mg, 0.09 mmol), and N,N-dimethylformamide (1.0 mL) were added. Under a nitrogen atmosphere, triethylamine (16 mg, 0.159 mmol) was added, and the reaction was carried out at RT for 1 hour. The reaction was then stopped, and the mixture was concentrated to dryness. Dichloromethane and saturated brine were added for extraction. The aqueous phase was further washed with dichloromethane (10.0 mL × 3). The organic phases were combined, dried over anhydrous $Na_2SO_4$ and concentrated to dryness under reduced pressure to afford 86 mg of crude compound 28b as a yellow oily liquid in 62.9% yield. LCMS (254 nm) purity 93.41%, Rt = 1.54 min; MS Calculated: 914.4; MS Found: 915.4 $[M+H]^+$.

Step 3: Compound 28b (86 mg, 0.10 mmol) was dissolved in dichloromethane (1.8 mL). Under a nitrogen atmosphere, TFA (0.1 mL) was added, and the reaction was stirred at RT for 1 hour. Upon confirmation of completion by LCMS, the reaction was stopped. The mixture was concentrated to dryness under reduced pressure. To the residue was added N,N-dimethylformamide (2.0 mL). Under a nitrogen atmosphere, triethylamine (111 mg, 1.10 mmol) and N-succinimidyl 6-maleimidohexanoate (EMCS, 52 mg, 0.17 mmol) were added sequentially. The reaction was carried out at RT for approximately 1 hour and then stopped. The system was concentrated to dryness under reduced pressure and directly purified by high-pressure reverse-phase prep-LC (mobile phase: acetonitrile/0.05% TFA aqueous solution) followed by lyophilization to afford 12 mg of compound STI-F1002 as a yellow solid in 12.6% yield. LCMS (254 nm): Purity 98.5%, Rt = 1.58 min; MS Calculated: 1007.4; MS Found: 1008.4 $[M+H]^+$. $^1H$ NMR (400 MHz, DMSO-$d_6$) δ10.05 (s,

1H), 8.50 (t, *J* = 6.6 Hz, 1H), 8.41 (d, *J* = 5.9 Hz, 1H), 8.29 (t, *J* = 5.9 Hz, 1H), 8.11 (d, *J* = 8.0 Hz, 1H), 8.07 (t, *J* = 5.8 Hz, 1H), 8.01 (t, *J* = 5.8 Hz, 1H), 7.59 (d, *J* = 10.5 Hz, 1H), 7.27 - 7.16 (m, 5H), 7.00 (s, 1H), 6.99 (s, 2H), 6.61 (br, 1H), 5.49 (s, 2H), 5.43 (s, 2H), 4.54 - 4.44 (m, 1H), 3.76 - 3.56 (m, 10H), 3.39 - 3.33 (m, 4H), 3.04 (d, *J* = 4.8 Hz, 1H), 3.01 (d, *J* = 4.7 Hz, 1H), 2.44 (s, 3H), 2.13 (t, *J* = 7.4 Hz, 2H), 1.91 - 1.80 (m, 2H), 1.69 - 1.60 (m, 2H), 1.50 - 1.41 (m, 4H), 1.26 - 1.14 (m, 4H), 0.87 (t, *J* = 7.3 Hz, 3H).

**Example 29: Synthesis of Linker-payload Conjugate STI-F1001**

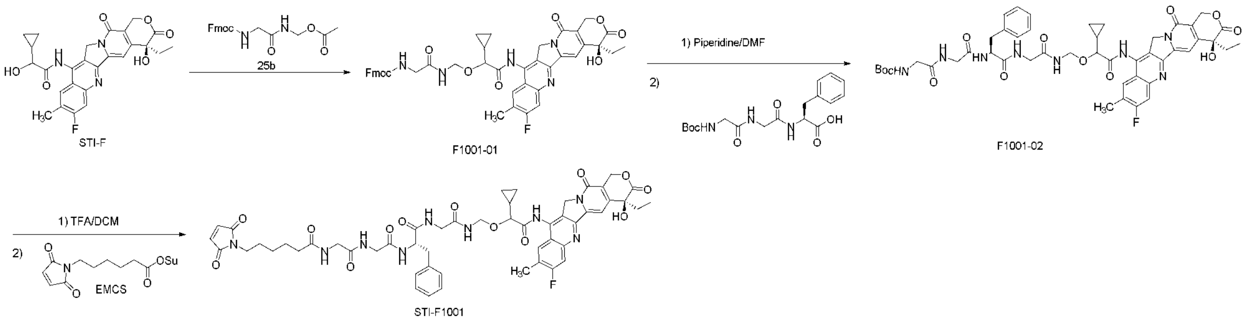

STI-F F1001-01 F1001-02 STI-F1001

Step 1: Compound STI-F (80 mg, 0.16 mmol), 25b (61 mg, 0.16 mmol), and pyridinium p-toluenesulfonate (4 mg, 0.01 mmol) were added to a reaction flask. 1,2-Dichloroethane (5.0 mL) was added. Under a nitrogen atmosphere, the mixture was heated to 75°C and reacted for 48 hours. The system was then concentrated to dryness under reduced pressure and purified by prep-TLC (DCM:MeOH = 15:1) to afford 40 mg of compound F1001-01 as an orange-yellow solid in 31.3% yield. LCMS (254 nm) purity 92.16%, Rt = 1.905 min; MS Calculated: 801.3; MS Found: 802.1 $[M+H]^+$.

Step 2: Compound F1001-01 (40 mg, 0.05 mmol) was dissolved in N,N-dimethylformamide (0.8 mL). Piperidine (0.2 mL) was added, and the reaction was carried out at RT for 20 minutes. The system was then concentrated to dryness. To the crude product were added (tert-butoxycarbonyl)glycyl-L-phenylalanine (23b) (27 mg, 0.07 mmol), 2-(7-azabenzotriazol-1-yl)-N,N,N',N'-tetramethyluronium hexafluorophosphate (34 mg, 0.09 mmol), and N,N-dimethylformamide (1.0 mL). Under a nitrogen atmosphere, triethylamine (15 mg, 0.15 mmol) was added, and the reaction was carried out at RT for 1 hour. The system was concentrated to dryness. Dichloromethane and saturated brine were added for extraction. The aqueous phase was further extracted with dichloromethane (10.0 mL × 3). The organic phases were combined, dried over anhydrous $Na_2SO_4$ and concentrated to dryness under reduced pressure to afford 46 mg of crude compound F1001-02. LCMS (254 nm): Purity 72.13%, $R_t$ = 1.708 min; MS Calculated: 940.4; MS Found: 941.2 $[M+H]^+$.

Step 3: Compound F1001-02 (46 mg, 0.05 mmol) was dissolved in dichloromethane (1.8 mL). Under a nitrogen atmosphere, TFA (0.1 mL) was added, and the reaction was carried out at RT for 1 hour. Upon confirmation of completion by LCMS, the reaction was stopped. The system was concentrated to dryness under reduced pressure. To the residue was added N,N-dimethylformamide (2.0 mL). Under a nitrogen atmosphere, triethylamine (0.07 mL, 0.50 mmol) and 6-(maleimido)hexanoic acid succinimide ester (EMCS, 52 mg, 0.17 mmol) were added sequentially. The reaction was carried out at RT for approximately 1 hour and then stopped. The system was concentrated to dryness under reduced pressure, then subjected to high-pressure reverse-phase prep-LC (mobile phase: acetonitrile/0.05% TFA aqueous solution) and lyophilization to afford 12 mg of compound STI-F1001 as a yellow solid in 23.7% yield. LCMS (254 nm) purity 96.79%, Rt = 1.58 min; MS Calculated: 1033.4; MS Found: 1034.1 $[M+H]^+$. $^1$H NMR (400 MHz, DMSO-$d_6$) δ 10.69 (s, 1H), 8.72 (t, *J* = 6.6 Hz, 1H), 8.35 (t, *J* = 5.9 Hz, 1H), 8.12 (dd, *J* = 8.3, 4.3 Hz, 2H), 8.06 (t, *J* = 5.9 Hz, 1H), 8.00 (t, *J* = 5.8 Hz, 1H), 7.91 (d, *J* = 10.7 Hz, 1H), 7.33 (s, 1H), 7.26 - 7.11 (m, 5H), 6.98 (s, 2H), 6.53 (br, 1H), 5.41 (s, 2H), 5.14 (s, 2H), 4.86 - 4.71 (m, 2H), 4.48 (td, *J* = 8.9, 4.6 Hz, 1H), 3.83 (d, *J* = 7.5 Hz, 1H), 3.77 (d, *J* = 6.4 Hz, 2H), 3.75 - 3.53 (m, 6H), 3.02 (dd, *J* = 14.0, 4.4 Hz, 1H), 2.77 (dd, *J* = 13.9, 9.7 Hz, 1H), 2.50 (s, 3H), 2.09 (t, *J* = 7.6 Hz, 2H), 1.92 - 1.81 (m, 2H), 1.50 - 1.41 (m, 4H), 1.31 (q, *J* = 6.4 Hz, 1H), 1.18 (dd, *J* = 15.4, 7.8 Hz, 2H), 0.88 (t, *J* = 7.3 Hz, 3H), 0.65 - 0.56 (m, 4H).

Similarly, the linker-payload conjugates as described below were prepared:

| Example No. | Structure |
|---|---|
| 30 | 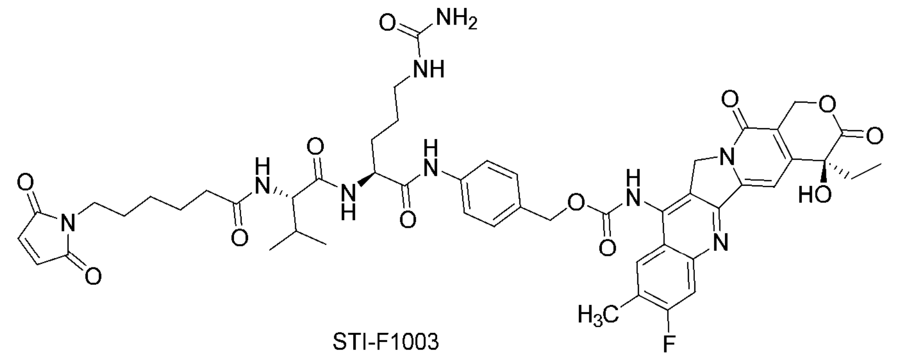 STI-F1003 |
| 31 | 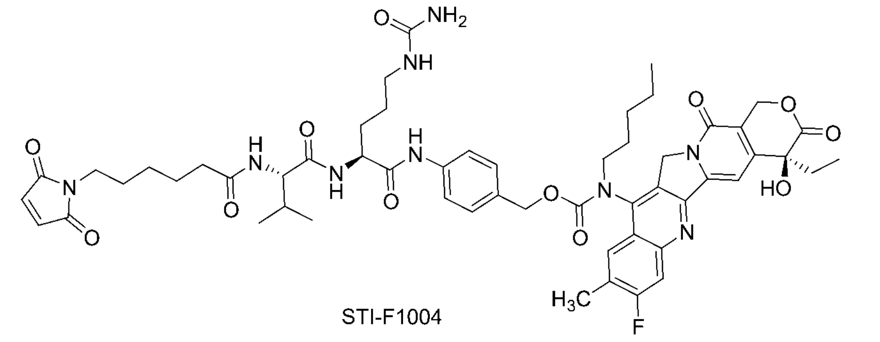 STI-F1004 |

**Example 32: Synthesis of Linker-payload Conjugate STI-F1006**

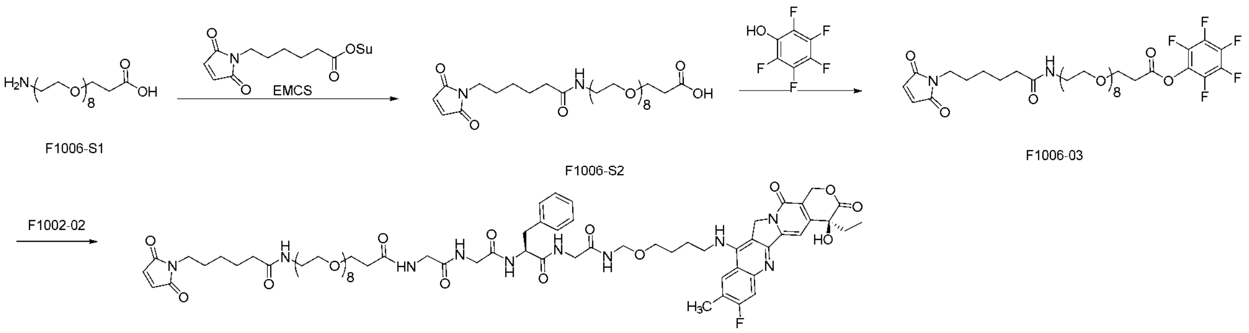

F1006-S1
F1006-S2
F1006-03
STI-F1006

Step 1: Compound F1006-S1 (500 mg, 1.13 mmol) was dissolved in N,N-dimethylformamide (10.0 mL). Under a nitrogen atmosphere, EMCS (382 mg, 1.24 mmol) and N,N-diisopropylethylamine (219 mg, 1.70 mmol) were added. The reaction was carried out at RT for 2 hours, then quenched with water. Direct reverse-phase prep-LC (mobile phase: acetonitrile/0.05% TFA in water) followed by lyophilization afforded 587 mg of compound F1006-S2 as a yellowish oily liquid in 80.7% yield. LCMS (254 nm) purity 98.8%, Rt = 1.38 min; MS Calculated: 634.3; MS Found: 635.2 $[M+H]^+$.

Step 2: Compound F1006-S2 (578 mg, 0.93 mmol) and N,N'-dicyclohexylcarbodiimide (210 mg, 1.02 mmol) were dissolved in tetrahydrofuran (12.0 mL). Under a nitrogen atmosphere, pentafluorophenol (188 mg, 1.02 mmol) was added. The reaction was carried out at RT overnight. Upon confirmation of completion by LCMS, the reaction was filtered, and the filtrate was concentrated to dryness under reduced pressure to afford 587 mg of crude compound F1006-S3 as a yellowish oily liquid in 80.6% yield. LCMS (254 nm) purity 80.0%, Rt = 1.85 min; MS Calculated: 800.3; MS Found: 801.3 $[M+H]^+$.

Step 3: Compound 28b (100 mg, 0.11 mmol) was dissolved in dichloromethane (1.8 mL). Under a nitrogen atmosphere, TFA (0.2 mL) was added, and the reaction was carried out at 0~5 °C for 3 hours. Upon confirmation of completion by LCMS, the reaction was stopped. The system was concentrated to dryness under reduced pressure. To the residue, N,N-dimethylformamide (2.0 mL) was added. Under a nitrogen atmosphere, the mixture was cooled to 0~5°C,

and N,N-diisopropylethylamine (111 mg, 1.10 mmol) and F1006-S3 (80 mg, 0.10 mmol) were added sequentially. The reaction was carried out for approximately 0.5 hour and then stopped. The system was concentrated to dryness under reduced pressure. Direct high-pressure reverse-phase prep-LC (mobile phase: acetonitrile/0.05% TFA in water) followed by lyophilization afforded 16 mg of compound STI-F1006 as a yellow solid in 10.2% yield. LCMS (254 nm) purity 99.8%, Rt = 1.59 min; MS Calculated: 1430.7; MS Found: 1429.5 $[M-H]^-$. $^1$H NMR (400 MHz, DMSO-*d*$_6$) δ 8.52 (t, *J* = 6.7 Hz, 1H), 8.30 (d, *J* = 7.5 Hz, 2H), 8.17 (d, *J* = 5.7 Hz, 1H), 8.07 (t, *J* = 5.7 Hz, 1H), 8.11 (d, *J* = 8.0 Hz, 1H), 8.01 (t, *J* = 5.8 Hz, 1H), 7.82 (t, *J* = 5.7 Hz, 1H), 7.58 (d, *J* = 11.0 Hz, 1H), 7.38 (t, *J* = 5.6 Hz, 1H), 7.23 - 7.18 (m, 5H), 6.99 (s, 2H), 6.48 (s, 1H), 5.44 (s, 2H), 5.41 (s, 2H), 4.51 - 4.45 (m, 1H), 3.77 - 3.68 (m, 6H), 3.66 - 3.57 (m, 8H), 3.50 (s, 30H), 3.19 - 3.16 (m, 4H), 3.05 (d, *J* = 4.8 Hz, 1H), 3.01 (d, *J* = 3.6Hz, 1H), 2.43 (s, 3H), 2.38 (t, *J* = 6.5 Hz, 2H), 2.03 (t, *J* = 7.4 Hz, 2H), 1.88 - 1.81 (m, 2H), 1.66 - 1.64 (m, 2H), 1.50 - 1.43 (m, 4H), 1.24 - 1.18 (m, 4H), 0.87 (t, *J* = 7.3 Hz, 3H).

**Example 33: Synthesis of Linker-payload Conjugate STI-F1010**

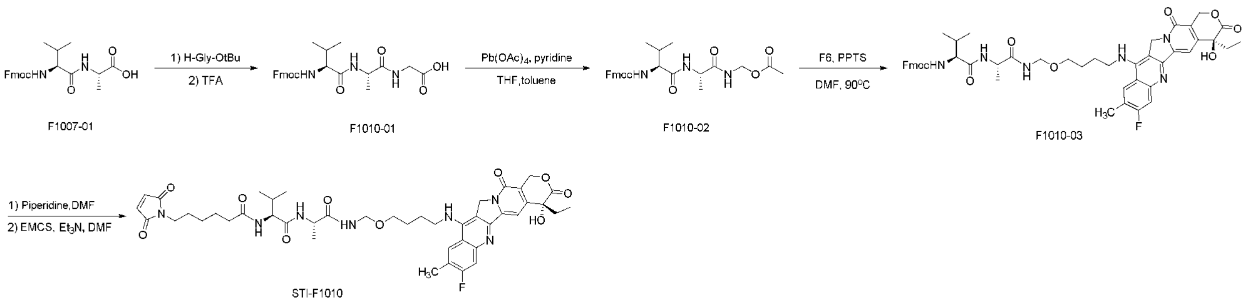


Step 1: Under an ice bath, compound F1007-01 (1.64 g, 4.00 mmol) was added to 50 mL dichloromethane, followed by N,N-diisopropylethylamine (1551 mg, 12.00 mmol), glycine tert-butyl ester (H-Gly-OtBu, 525 mg, 4.00 mmol), and 2-(1H-benzotriazol-1-yl)-1,1,3,3-tetramethyluronium tetrafluoroborate (1349 mg, 4.20 mmol). After nitrogen protection, the reaction was transferred to RT for 1 hour. Upon confirmation of completion by LCMS, the reaction was filtered and washed several times with dichloromethane. The filter cake was added to 8 mL dichloromethane, followed by 4 mL TFA. The reaction was carried out at RT for 3 hours. Upon confirmation of completion by LCMS, the reaction was concentrated to dryness by rotary evaporation. The residue was triturated with dichloromethane/acetonitrile, filtered, and washed with acetonitrile to afford 1280 mg of compound F1010-01 as a white solid in 68.5% yield. LCMS (254 nm): Purity 100%, Rt = 1.695 min; MS Calculated: 467.2; MS Found: 468.3 $[M+H]^+$.

Step 2: Compound F1010-01 (545 mg, 1.17 mmol) was dissolved in tetrahydrofuran (15.0 mL) and toluene (5.0 mL) to dissolve the substances. Under a nitrogen atmosphere, lead tetraacetate (620 mg, 1.40 mmol) and pyridine (111 mg, 1.40 mmol) were added. The mixture was heated to 85°C and reacted for 3 hours, then stopped. The mixture was filtered through a pad of Celite, and the filtrate was concentrated to dryness under reduced pressure. Purification by column chromatography (DCM:MeOH = 100:1) afforded 400 mg of compound F1010-02 as a white solid in 71.0% yield. LCMS (254 nm) purity 89.0%, Rt = 1.811 min; MS Calculated: 481.2; MS Found: 499.7 $[M+NH_4]^+$.

Step 3: Compound STI-F6 (160 mg, 0.34 mmol) from Example 17, F1010-02 (197 mg, 0.41 mmol), and pyridinium p-toluenesulfonate (PPTS, 10 mg) were added to a reaction flask. DMF (5.0 mL) was added. Under a nitrogen atmosphere, the mixture was heated to 95°C and reacted for approximately 3 hours, then stopped. The system was concentrated to dryness under reduced pressure and purified by column chromatography (DCM:MeOH = 50:1) to afford 110 mg of compound F1010-03 as an orange-red solid in 25.5% yield. LCMS (254 nm) purity 84.0%, Rt = 1.920 min; MS Calculated: 888.4; MS Found: 889.2 $[M+H]^+$.

Step 4: Compound F1010-03 (55 mg, 0.062 mmol) was dissolved in DMF (0.5 mL). Piperidine (0.05 mL) was added, and the reaction was carried out at RT for 20 minutes. The system was concentrated to dryness, and the residue was washed with petroleum ether and dried under reduced pressure. To the residue, EMCS (29 mg, 0.093 mmol) and DMF (1.0 mL) were added. Under a nitrogen atmosphere, triethylamine (19 mg, 0.19 mmol) was added, and the reaction was carried out at RT for 1 hour. The reaction was then stopped, and the system was concentrated to dryness. Medium-to-high pressure reverse-phase prep-LC (mobile phase: acetonitrile/0.05% formic acid aqueous solution) followed by lyophilization afforded 16 mg of compound STI-F1010 as a yellowish solid in 30.2% yield. LCMS (254 nm) purity 96.38%, Rt = 1.663 min; MS Calculated: 859.4; MS Found: 860.4 $[M+H]^+$. $^1$H NMR (400 MHz, DMSO-*d*$_6$) δ 8.79 (t, *J* = 6.4 Hz, 1H), 8.59 (t, *J* = 6.6 Hz, 1H), 8.42 (d, *J* = 7.8 Hz, 1H), 8.00 (d, *J* = 7.2 Hz, 1H), 7.76 (t, *J* = 8.7 Hz, 1H), 7.59 (d, *J* = 10.5 Hz, 1H) , 7.41 (s, 1H), 7.00 (s, 2H), 6.57 (br, 1H), 5.47 (s, 2H), 5.44 (s, 2H), 4.58 - 4.56 (m, 2H), 4.23 (q, *J* = 7.1 Hz, 1H), 4.14 - 4.09 (m, 1H), 3.71 (d, *J* = 6.8 Hz, 2H), 3.45 - 3.43 (m, 2H), 3.35 (t, *J* = 7.0 Hz, 2H), 2.44 (s, 3H), 2.16 - 2.05 (m, 2H), 1.88 - 1.84 (m, 1H), 1.77 - 1.72

(m, 2H), 1.67 - 1.61 (m, 2H), 1.47 - 1.42 (m, 4H), 1.24 - 1.12 (m, 7H), 0.88 (t, *J* = 6.0 Hz, 3H), 0.79 (dd, *J* = 11.3, 6.7 Hz, 6H).

**Example 34: Synthesis of Linker-payload Conjugate STI-F1011**

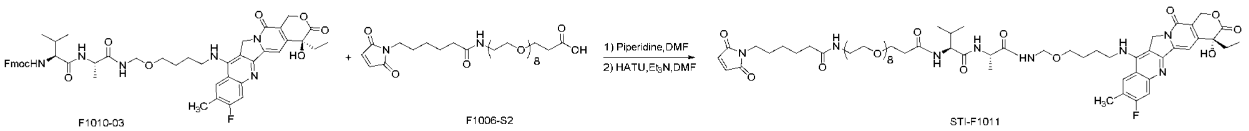

F1010-03 F1006-S2 STI-F1011

Compound F1010-03 (55 mg, 0.062 mmol) from Example 33 was dissolved in DMF (0.5 mL). Piperidine (0.05 mL) was added, and the reaction was carried out at RT for 10 minutes. The system was concentrated to dryness. The residue was washed with petroleum ether and dried under reduced pressure. To the residue, HATU (57 mg, 0.19 mmol), compound F1006-S2 (29 mg, 0.093 mmol) from Example 32, and DMF (1.0 mL) were added. Under a nitrogen atmosphere, triethylamine (19 mg, 0.19 mmol) was added, and the reaction was carried out at RT for 0.5 hour. The reaction was then stopped, and the system was concentrated to dryness. Medium-to-high pressure reverse-phase prep-LC (mobile phase: acetonitrile/0.05% formic acid aqueous solution) followed by lyophilization afforded 28 mg of compound STI-F1011 as a yellowish viscous solid in 35.0% yield. LCMS (254 nm) purity 96.07%, Rt = 1.597 min; MS Calculated: 1282.6; MS Found: 1283.6 $[M+H]^+$. $^1$H NMR (400 MHz, DMSO-$d_6$) δ 8.60 - 8.56 (m, 2H), 8.49 - 8.45 (m, 1H), 8.03 (d, *J* = 7.3 Hz, 1H), 7.90 - 7.78 (m, 2H), 7.60 (d, *J* = 10.3, 1H), 7.49 (s, 1H) , 7.00 (s, 2H), 6.53 (br, 1H), 5.51 (s, 2H), 5.45(s, 2H), 4.62 - 4.52 (m, 2H), 4.23 (t, *J* = 7.2 Hz, 1H), 4.15 (dd, *J* = 8.7, 6.6 Hz, 1H), 3.75 - 3.73 (m, 2H), 3.60 - 3.52 (m, 4H), 3.51 - 3.46 (m, 30H), 3.39 - 3.36 (m, 4H), 3.16 (q, *J* = 5.9 Hz, 2H), 2.45 (s, 3H), 2.03 (t, *J* = 7.4 Hz, 2H), 1.88 - 1.84 (m, 1H), 1.78 - 1.73 (m, 2H), 1.68 - 1.62 (m, 2H), 1.50 - 1.42 (m, 4H), 1.23 - 1.12 (m, 7H), 0.87 (t, *J* = 7.4 Hz, 3H), 0.79 (dd, *J* = 11.5, 6.8 Hz, 6H).

**Example 35: Synthesis of Linker-payload Conjugate STI-F1012**

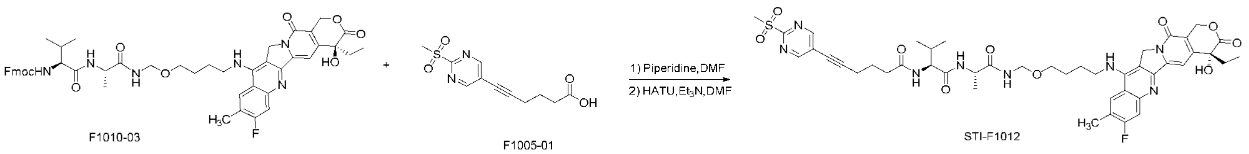

F1010-03 F1005-01 STI-F1012

Compound F1010-03 (70 mg, 0.078 mmol) from Example 33 was dissolved in DMF (0.5 mL). Piperidine (0.05 mL) was added, and the reaction was carried out at RT for 10 minutes. The system was concentrated to dryness. The residue was washed with petroleum ether and dried under reduced pressure. To the residue, HATU (87 mg, 0.23 mmol), 6-(2-(methylsulfonyl)pyrimidin-5-yl)hex-5-ynoic acid (25 mg, 0.094 mmol), and DMF (2.0 mL) were added. Under a nitrogen atmosphere, triethylamine (23 mg, 0.23 mmol) was added, and the reaction was carried out at RT for 0.5 hour. The reaction was then stopped, and the system was concentrated to dryness. Medium-to-high pressure reverse-phase prep-LC (mobile phase: acetonitrile/0.05% TFA aqueous solution) followed by lyophilization afforded 30 mg of compound STI-F1012 as a yellowish solid in 41.9% yield. LCMS (254 nm) purity 98.69%, Rt = 1.646 min; MS Calculated: 916.4; MS Found: 917.3 $[M+H]^+$. $^1$H NMR (400 MHz, DMSO-$d_6$) δ 9.11 (s, 2H), 8.60 (t, *J* = 6.6 Hz, 1H), 8.45 (d, *J* = 7.7 Hz, 1H), 8.06 (d, *J* = 7.2 Hz, 1H), 7.89 (d, *J* = 8.7 Hz, 1H), 7.58 (dd, *J* = 10.3, 2.3 Hz, 1H), 7.41 (s, 1H), 6.65 (br, 1H), 5.49 (s, 2H), 5.45 (s, 2H), 4.64 - 4.51 (m, 2H), 4.24 (q, *J* = 7.1 Hz, 1H), 4.16 (dd, *J* = 8.7, 6.7 Hz, 1H), 3.76 - 3.69 (m, 4H), 3.45 - 3.42 (m, 1H), 3.40 (s, 3H), 2.54 (d, *J* = 7.3 Hz, 2H), 2.44 (s, 3H), 2.16 - 2.05 (m, 2H), 1.89 - 1.83 (m, 2H), 1.81 - 1.76 (m, 4H), 1.67 - 1.61 (m, 2H), 1.21 (d, *J* = 7.1 Hz, 3H), 0.87 (t, *J* = 7.4 Hz, 3H), 0.81 (dd, *J* = 11.8, 6.7 Hz, 6H).

**Example 36: Synthesis of Linker-payload Conjugate STI-F1013**

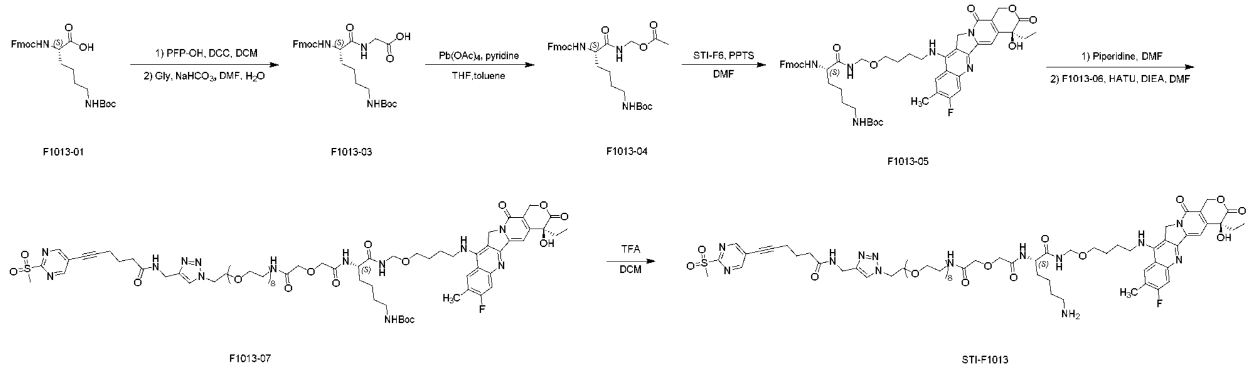


F1013-01 F1013-03 F1013-04 F1013-05

F1013-07 STI-F1013

Step 1: F1013-01 (2.0 g, 4.27 mmol) was dissolved in dichloromethane (20.0 mL). Under a nitrogen atmosphere, pentafluorophenol (943 mg, 5.12 mmol) and DCC (1.05 g, 5.12 mmol) were added, and the reaction was carried out at RT for 1 hour. After the reaction was completed as monitored by TLC, the reaction solution was filtered, and the filter cake was washed with dichloromethane (5 mL × 3). The filtrate was concentrated to dryness at RTand used directly in the next step. The above product was dissolved in acetone/water (5 mL/15 mL), followed by sequential addition of sodium bicarbonate (1.07 g, 12.8 mmol) and glycine (320 mg, 4.27 mmol). The mixture was stirred at RT for 2 hours. After the reaction was completed as monitored by LCMS, 1M hydrochloric acid was added to adjust the pH to 3~4. The mixture was extracted with ethyl acetate (10 mL × 2), washed with saturated brine (10 mL × 2), and dried over anhydrous $Na_2SO_4$. After concentration at 45°C, direct low-to-medium pressure reverse-phase prep-LC (mobile phase: acetonitrile/0.05% TFA aqueous solution) followed by lyophilization afforded 2.0 g of compound F1013-03 as a white solid; yield 90.9%. LCMS (254 nm) purity 95.6%, Rt = 2.16 min; MS calculated: 525.2; MS found: 526.3 $[M+H]^+$.

Step 2: F1013-03 (2.0 g, 3.80 mmol) was dissolved in tetrahydrofuran (40 mL) and toluene (10 mL). Under a nitrogen atmosphere, $Pb(OAc)_4$ (2.03 g 4.57 mmol) and pyridine (361 mg, 4.57 mmol) were added, and the reaction was carried out at 85°C for 1 hour. After the reaction was confirmed to be complete by LCMS monitoring, the reaction solution was concentrated to dryness under reduced pressure at 45°C. The crude product was purified by column chromatography (petroleum ether/ethyl acetate = 2/3) to yield 1.80 g of compound F1013-04 as a yellowish solid; yield 87.8%. LCMS (254 nm) purity: 92.1%, Rt = 2.36 min; MS calculated: 539.2; MS found: 557.3 $[M+NH_4]^+$.

Step 3: Compound F1013-04 (260 mg, 0.43 mmol) was dissolved in DMF (5 mL). Under a nitrogen atmosphere, STI-F6 (170 mg, 0.36 mmol) from Example 17 and PPTS (17 mg) were added sequentially. The reaction was carried out at 95 °C for 2 hours. The reaction solution was then concentrated to dryness under reduced pressure at 45°C. The crude product was purified by column chromatography (dichloromethane/methanol = 50/3) to afford 70 mg of compound F1013-05 as a black solid; yield 14.2%. LCMS (254 nm) purity: 70.1%, Rt = 2.05 min; MS calculated: 946.4; MS found: 947.5 $[M+H]^+$.

Step 4: Compound F1013-05 (70 mg, 0.07 mmol) was dissolved in DMF (0.7 mL), and piperidine (70 μL) was added with stirring under a nitrogen atmosphere. Reaction was carried out for 0.5 hour. Completion of the reaction was monitored by LCMS. The reaction solution was concentrated to dryness using an oil pump at 45°C, then triturated with petroleum ether (2 mL*3) and concentrated again for use directly in the next step. F1013-06 (64 mg, 0.07 mmol) was dissolved in DMF (2 mL), followed by sequential addition of HATU (42 mg, 0.11 mmol) and N,N-diisopropylethylamine (19 mg, 0.148 mmol). The mixture was stirred under a nitrogen atmosphere for 5 minutes, after which the above concentrate was added. The reaction was stirred at RT under a nitrogen atmosphere for 1 hour. Upon confirmation of completion by LCMS, the reaction was concentrated to dryness using an oil pump at 45°C. The crude product was purified by column chromatography (dichloromethane/methanol=50/7) to yield 60 mg of compound F1013-07 as a yellow solid; yield 52.1%. LCMS (254 nm) purity: 93.0%, Rt = 1.78 min; MS calculated: 1565.1; MS found: 1566.2 $[M+H]^+$.

Step 5: Compound F1013-07 (60 mg, 0.03 mmol) was dissolved in 10% TFA/DCM (2 mL) and stirred under a nitrogen atmosphere in an ice bath for 1 hour. The completion of the reaction was monitored by LCMS. The solution mixture was adjusted to pH 7~8 using triethylamine. After purging with nitrogen, high-pressure reverse-phase prep-LC (mobile phase: acetonitrile/0.05% TFA aqueous solution) followed by lyophilization afforded 6.2 mg of compound STI-F1013 as a yellow solid; yield 11.8%. LCMS (254 nm) purity: 99.0%, Rt = 1.51 min; MS calculated: 1465.6; MS found: 1466.8 $[M+H]^+$. $^1$H NMR (400 MHz, MeOD) δ 8.95 (s, 2H), 8.32 (d, $J$ = 7.6 Hz, 1H), 7.96 (s, 1H), 7.70 (s, 1H), 7.56 (d, $J$ = 10.0 Hz, 1H), 5.66-5.58 (m, 2H), 4.77 (q, $J_1$ = 10.4Hz, $J_2$ = 26.4Hz, 2H), 4.56 (t, $J$ = 4.8 Hz, 2H), 4.45 - 4.41 (m, 3H), 4.03 (s, 2H), 3.98 (d, $J$ = 2.0 Hz, 2H), 3.89 - 3.83 (m, 4H), 3.64 - 3.57(m, 28H), 3.52 - 3.49 (m, 2H), 3.37 (s, 3H), 3.37 - 3.31 (m, 6H), 2.97 (t, $J$ = 7.2 Hz, 2H), 2.60 (t, $J$ = 7.2 Hz, 2H), 2.52 (s, 3H), 2.45 (t, $J$ = 7.2 Hz, 2H), 2.03 - 1.68 (m, 12H), 1.51-1.47 (m, 2H), 1.02 (t, $J$ = 7.2 Hz, 3H).

Intermediate compound F1013-06 was prepared as follows:

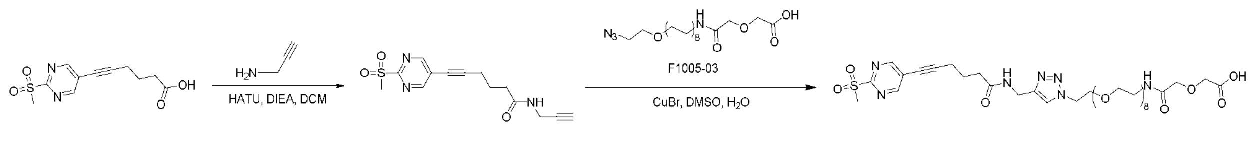


F1005-01 F1005-02 F1013-06

Step 1: Compound 6-(2-(methylsulfonyl)pyrimidin-5-yl)hex-5-ynoic acid (200 mg, 0.74 mmol) was dissolved in dichloromethane (2 mL). HATU (425 mg, 1.12 mmol) and N,N-diisopropylethylamine (193 mg, 1.49 mmol) were added sequentially. The reaction mixture was stirred under a nitrogen atmosphere for 10 minutes. Propargylamine (49 mg, 0.89 mmol) was then added to the reaction solution, and stirring was continued for 1 hour. The completion of the reaction was monitored by LCMS. The reaction solution was concentrated to dryness under reduced pressure at RT. The crude product was purified by column chromatography (petroleum ether/ethyl acetate = 1/3) to afford 210 mg of compound F1005-02 as a yellow solid; yield 92.5%. LCMS (254 nm) purity: 92.3%, Rt = 2.20 min; MS calculated: 305.0; MS found: 306.1 [M+H]$^+$.

Step 2: Compound F1005-02 (100 mg, 0.32 mmol) was dissolved in a mixed solvent of dimethyl sulfoxide (1 mL) and water (0.1 mL). F1005-03 (144 mg, 0.32 mmol) and CuBr (94 mg, 0.65 mmol) were added sequentially. The mixture was stirred under a nitrogen atmosphere and reacted for 1 hour. The completion of the reaction was monitored by LCMS. The reaction solution was then subjected to low-to-medium pressure reverse-phase prep-LC (mobile phase: acetonitrile/0.05% TFA aqueous solution) and lyophilized to afford 350 mg of compound F1013-06 as a green oil. LCMS (254 nm) purity: 85.2%, Rt = 1.95 min; MS calculated: 859.3; MS found: 860.4 [M+H]$^+$.

**Example 37: Synthesis of Linker-payload Conjugate STI-F1014**

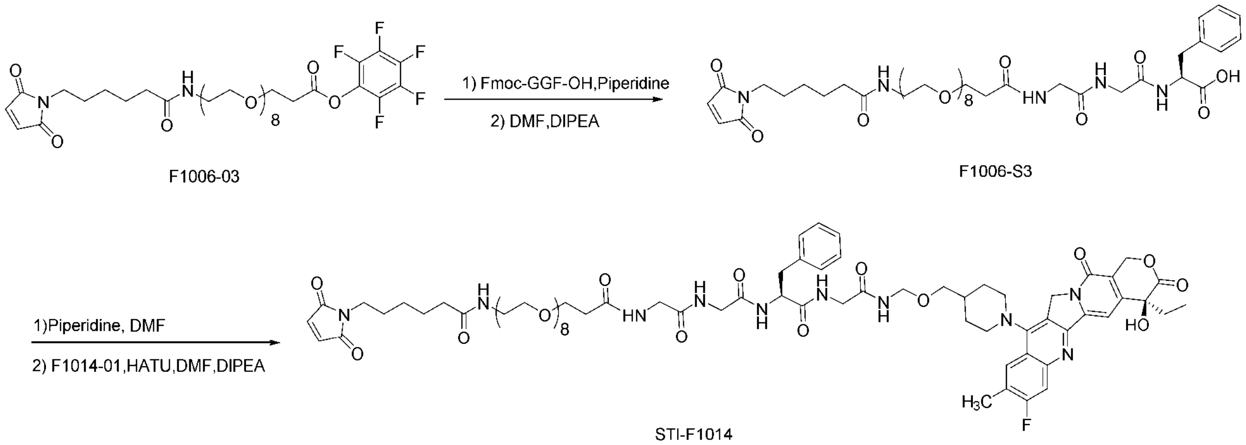


F1006-03 F1006-S3 STI-F1014

Step 1: The compound ((9H-fluorene-9-yl)methoxy)carbonyl)glycyl-L-phenylalanine (Fmoc-GGF-OH, 400 mg, 0.80 mmol) was dissolved in DMF (5.4 mL). Piperidine (0.6 mL) was added, and the system was reacted at RT for 30 minutes. The mixture was then concentrated to dryness, and triturated with petroleum ether. The residue was redissolved in DMF (8.0 mL). Under a nitrogen atmosphere, the mixture was cooled to 0~5°C, and F1006-03 (832 mg, 1.04 mmol) and DIPEA (154 mg, 1.20 mmol) were added. The mixture was reacted at 0~5 °C for about 1 hour. Upon confirmation of completion by LCMS, the reaction was stopped. The system was concentrated to dryness under reduced pressure. Acidic reverse-phase purification afforded 380 mg of compound F1006-S3 as a colorless oily liquid; yield 40.8%. LCMS (254 nm) purity: 95.96%, Rt = 1.648 min; MS calculated: 895.4; MS found: 896.5 [M+H]$^+$.

Step 2: Compound F1014-01 (180 mg, 0.22 mmol) was dissolved in DMF (2.7 mL). Piperidine (0.3 mL) was added, and the reaction was carried out at RT for 30 minutes. The system was concentrated to dryness, triturated with acetone, and filtered. The filter cake was redissolved in DMF (3.0 mL). To this solution, F1006-S3 (63 mg, 0.07 mmol) and HATU (125 mg, 0.33 mmol) were added. Under a nitrogen atmosphere, DIPEA (43 mg, 0.33 mmol) was added, and the reaction was carried out at RT for 0.5 hour. Upon confirmation of completion by LCMS, the reaction was stopped. The system was concentrated to dryness. Medium-to-high pressure reverse-phase prep-LC (mobile phase: acetonitrile/0.05% TFA aqueous solution) followed by lyophilization afforded 14 mg of compound STI-F1014 as a yellowish solid; yield 4.4%. LCMS (254 nm) purity: 95.8%, Rt = 1.654 min; MS calculated: 1456.7; MS found: 1457.8 [M+H]$^+$. $^1$H NMR (400 MHz, DMSO-$d_6$) δ 8.52 (t, $J$ = 6.7 Hz, 1H), 8.37 (t, $J$ = 6.0 Hz, 1H), 8.19 - 8.12 (m, 2H), 8.02 (t, $J$ = 5.7 Hz, 1H), 7.93 (t, $J$ = 8.6 Hz, 1H), 7.80 - 7.75 (m, 2H), 7.28 - 7.24 (m, 6H), 7.00 (s, 2H), 6.51 (br, 1H), 5.48 (s, 2H), 5.42 (s, 2H), 4.61 - 4.60 (m, 2H), 4.51(q,

*J* = 8.3, 7.8 Hz, 1H), 3.77 - 3.69 (m, 6H), 3.61 - 3.59 (m, 6H), 3.49 - 3.47 (m, 28H), 3.28 - 3.22 (m, 6H), 3.18 - 3.16 (m, 2H), 3.09 - 3.08 (m, 1H), 3.06 - 3.05 (m, 1H), 2.49 (s, 3H), 2.39 (t, *J* = 6.6 Hz, 2H), 2.03 (t, *J* = 7.3 Hz, 2H), 1.88 - 1.82 (m, 2H), 1.58 - 1.52 (m, 1H), 1.48 - 1.42 (m, 4H), 1.25 - 1.17 (m, 6H), 0.88 (t, *J* = 6.0 Hz, 3H).

Intermediate compound F1014-01 was prepared as follows:

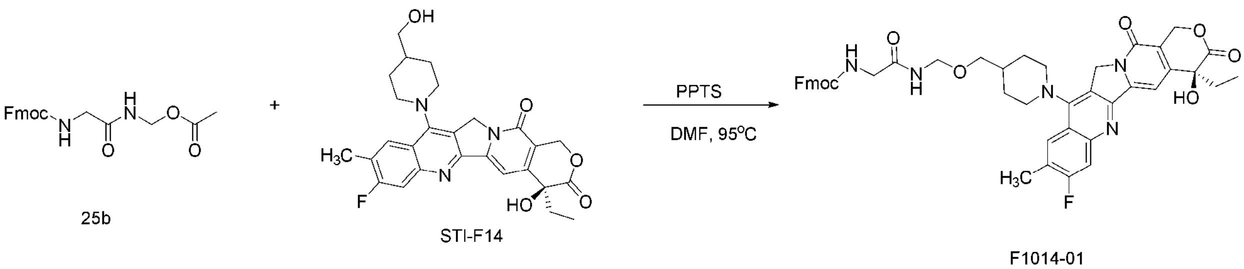


25b STI-F14 F1014-01

Step: Compound STI-F14 (170 mg, 0.34 mmol), 25b (151 mg, 0.41 mmol), and pyridinium p-toluenesulfonate (20 mg) were added to a reaction flask, followed by addition of DMF (10.0 mL). Under a nitrogen atmosphere, the temperature was raised to 95°C and the reaction was carried out for approximately 3 hours before being stopped. The system was concentrated to dryness under reduced pressure and purified by column chromatography (DCM:MeOH = 50:1) to afford 180 mg of compound F1014-01 as a yellow solid; yield 63.8%. LCMS (254 nm) purity: 51.39%, Rt = 1.992 min; MS calculated: 801.3; MS found: 802.3 $[M+H]^+$.

**Example 38: Synthesis of Linker-payload Conjugate STI-F1015**

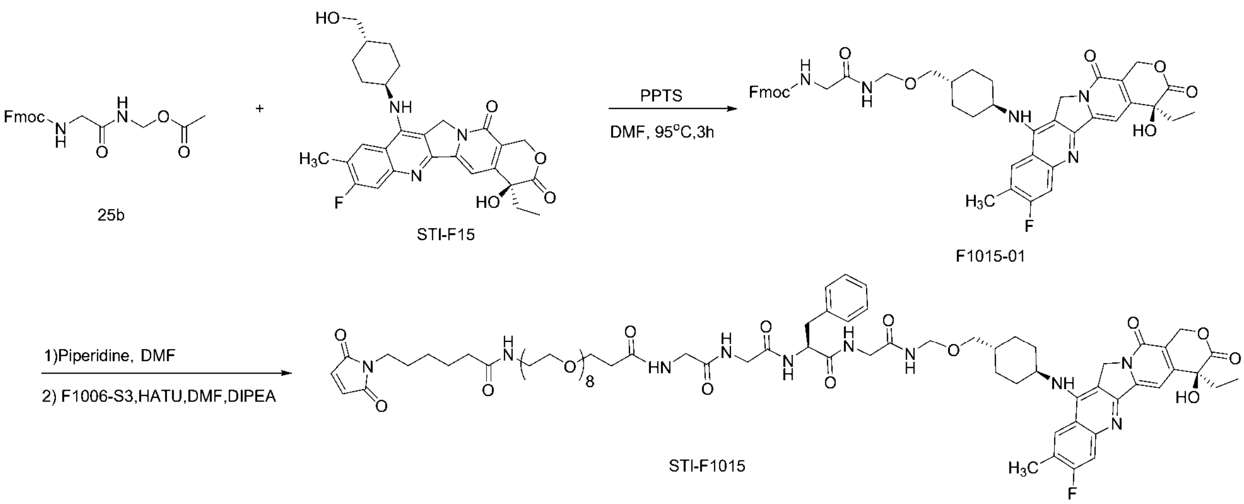


25b STI-F15 F1015-01 STI-F1015

Step 1: Compound STI-F15 (390 mg, 0.77 mmol), 25b (339 mg, 0.92 mmol), and pyridinium p-toluenesulfonate (20 mg) were added to a reaction flask, followed by addition of DMF (12.0 mL). Under a nitrogen atmosphere, the temperature was raised to 95°C and the reaction was carried out for approximately 3 hours before being stopped. The system was concentrated to dryness under reduced pressure and purified by column chromatography (DCM:MeOH = 50:1) to afford 180 mg of compound F1015-01 as a yellow solid; yield 18.5%. LCMS (254 nm) purity: 47%, Rt = 1.891 min; MS calculated: 815.3; MS found: 816.4 $M+H]^+$.

Step 2: Compound F1015-01 (47% purity, 180 mg, 0.11 mmol) was dissolved in DMF (2.7 mL). Piperidine (0.3 mL) was added, and the reaction was carried out at RT for 30 minutes. The system was concentrated to dryness, triturated with acetone, and filtered. The filter cake and HATU (125 mg, 0.30 mmol) were added to the reaction flask. Under a nitrogen atmosphere, DMF (3.0 mL), F1006-S3 (107 mg, 0.12 mmol) from Example 37, and DIPEA (43 mg, 0.30 mmol) were added. The reaction was carried out at RT for 0.5 hour. Upon confirmation of completion by LCMS, the reaction was stopped. The system was concentrated to dryness. Medium-to-high pressure reverse-phase prep-LC (acidic mobile phase) and lyophilization afforded 15 mg of compound STI-F1015 as a yellowish solid; yield 9.3%. LCMS (254 nm) purity: 97.56%, Rt = 1.655 min; MS calculated: 1470.7; MS found: 1469.6 $[M-H]^-$. $^1H$ NMR (400 MHz, DMSO-$d_6$) $\delta$ 8.52-8.47 (m, 2H), 8.31 (t, *J* = 5.7 Hz, 1H), 8.17 (t, *J* = 5.7 Hz, 1H), 8.12 (d, *J* = 8.1 Hz, 1H), 8.02 (t, *J* = 5.8 Hz, 1H), 7.81 (t, *J* = 5.7 Hz,

1H), 7.60 (d, *J* = 10.7 Hz, 1H) , 7.37 (s, 1H),7.28 - 7.20 (m, 5H), 7.00 (s, 2H), 6.54 (br, 1H), 5.42 (s, 2H), 5.38 (s, 2H), 4.57 - 4.55 (m, 2H), 4.53 - 4.48 (m, 1H), 3.78 - 3.69 (m, 7H), 3.63 - 3.58 (m, 7H), 3.49 - 3.47 (m, 28H), 3.29 (d, *J* = 6.1 Hz, 2H), 3.19 - 3.14 (m, 2H), 3.09 - 3.08 (m, 1H), 3.06 - 3.05 (m, 1H), 2.45 (s, 3H), 2.39 (t, *J* = 6.6 Hz, 2H), 2.07 - 1.97 (t, *J* = 7.3 Hz, 4H), 1.88 - 1.82 (m, 2H), 1.64 - 1.54 (m, 2H), 1.50 - 1.42 (m, 5H), 1.21 - 1.15 (m, 6H), 0.88 (t, *J* = 6.0 Hz, 3H).

**Example 39: Synthesis of Linker-payload Conjugate STI-F1018**

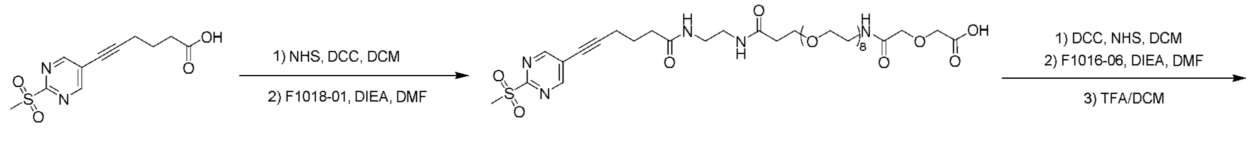


F1005-01 F1018-02

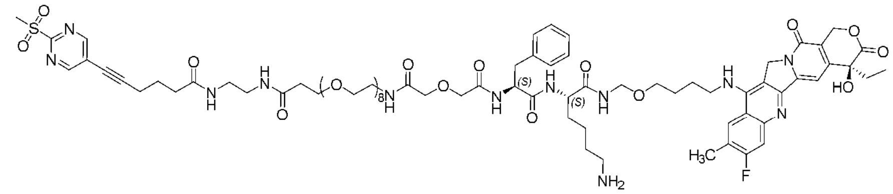

STI-F1018

Step 1: F1005-01 (65 mg, 0.24 mmol) was dissolved in dichloromethane (2.0 mL). Under a nitrogen atmosphere, NHS (34 mg, 0.29 mmol) and DCC (60 mg, 0.29 mmol) were added, and the reaction was carried out in an ice bath for 1 hour. Upon confirmation of completion by TLC monitoring, the reaction solution was filtered, and the filter cake was washed with DCM (5 mL × 3). The filtrate was concentrated to dryness at RT and used directly in the next step. The above crude product and F1018-01 (145 mg, 0.24 mmol) were dissolved in DMF (2 mL). DIEA (130 μL, 0.72 mmol) was added, and the mixture was stirred in an ice bath for 2 hours. After the reaction was complete as monitored by LCMS, acetic acid was added to quench the reaction until the pH turned acidic. The mixture was concentrated using an oil pump at 45°C. The crude product was subjected to low-to-medium pressure reverse-phase prep-LC (mobile phase: acetonitrile/0.05% TFA aqueous solution) and lyophilization to afford 200 mg of compound F1018-02 as a transparent oil; yield 96.9%. LCMS (254 nm) purity: 92.1%, Rt = 1.95 min; MS calculated: 849.3; MS found: 850.5 $[M+H]^+$.

Step 2: F1018-02 (53 mg, 0.06 mmol) was dissolved in dichloromethane (2.0 mL). Under a nitrogen atmosphere, NHS (9.0 mg, 0.07 mmol) and DCC (16 mg, 0.07 mmol) were added, and the reaction was carried out in an ice bath for 1 hour. Upon confirmation of completion by TLC monitoring, the reaction solution was filtered, and the filter cake was washed with DCM (5 mL × 3). The filtrate was concentrated to dryness at RT and used directly in the next step. The resulting concentrate was dissolved in DMF (2.0 mL). F1016-06 (43 mg, 0.05 mmol) and DIEA (49 mg, 0.37 mmol) were added, and the reaction was carried out in an ice bath for 2 hours. Upon confirmation of completion by LCMS, the mixture was concentrated using an oil pump at 45°C. The crude product was purified by column chromatography (dichloromethane/methanol=10/3) to yield 46 mg of the crude compound, which was dissolved in 10% TFA/DCM (2 mL) under a nitrogen atmosphere and stirred in an ice bath for 1 hour. Upon confirmation of completion by LCMS, the reaction solution was adjusted to pH 7~8. After purging with nitrogen, high-pressure reverse-phase prep-LC (mobile phase: acetonitrile/0.05% TFA aqueous solution) followed by lyophilization afforded 5.69 mg of compound STI-F1018 as a yellow solid. LCMS (254 nm) purity: 93.5%, Rt = 1.57 min; MS calculated: 1602.7; MS found: 1603.6 $[M+H]^+$. $^1$H NMR (400 MHz, DMSO-$d_6$) δ 8.95 (s, 2H), 8.32 (t, *J* = 6.0 Hz, 1H), 8.36 - 8.25 (m, 2H), 8.10 (d, *J* = 8.0 Hz, 1H), 7.99 (t, *J* = 6.0 Hz, 1H), 7.87 (s, 2H), 7.61-7.58 (m, 3H), 7.31 (d, *J* = 2.0 Hz, 1H), 7.21 - 7.08 (m, 5H), 6.53 (brs, 1H), 5.41 (d, *J* = 16.8 Hz, 4H), 4.59 - 4.54 (m, 3H), 4.24 - 4.23(m, 1H), 3.88 - 3.79 (m, 3H), 3.67-3.59 (m, 2H), 3.57 (t, *J* = 6.8 Hz, 3H), 3.47-3.42 (m, 34H), 3.28-3.23 (m, 4H), 3.16 - 3.12(m, 6H), 2.86 - 2.74 (m, 4H), 2.55 (t, *J* = 7.2 Hz 2H), 2.32 - 2.22 (m, 5H), 1.88 - 1.78 (m, 4H), 1.77 - 1.62 (m, 5H), 1.57 - 1.50 (m, 3H), 1.34 - 1.27 (m, 2H), 0.86 (t, *J* = 7.6 Hz, 3H).

Intermediate compound F1018-01 was prepared as follows:

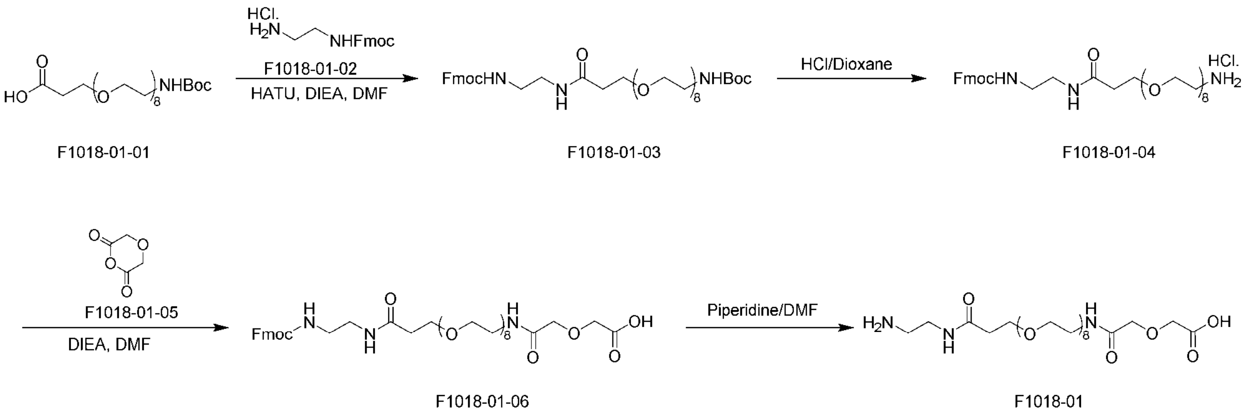


F1018-01-01 F1018-01-03 F1018-01-04 F1018-01-06 F1018-01

Step 1: F1018-01-01 (541 mg, 1.0 mmol) was dissolved in dichloromethane (10.0 mL). Under a nitrogen atmosphere, HATU (380 mg, 1.0 mmol) and DIEA (258 mg, 2.0 mmol) were added, and the mixture was stirred at RT for 5 minutes. F1018-01-02 (318 mg, 1.0 mmol, HCl salt) was then added. After the reaction was complete as monitored by LCMS, the reaction solution was concentrated to dryness at 45°C. The crude product was subjected to low-to-medium reverse-phase prep-LC (mobile phase: acetonitrile/0.05% TFA aqueous solution) and lyophilization to afford 680 mg of compound F1018-01-03 as a transparent oil; yield 84.4%. LCMS (254 nm) purity: 94.5%, Rt = 1.89 min; MS calculated: 805.4; MS found: 806.5 $[M+H]^+$.

Step 2: F1018-01-03 (200 mg, 0.24 mmol) was dissolved in 4M HCl/EA (4.0 mL), and the reaction was carried out at RT for 1.0 hour. Upon confirmation of completion by TLC monitoring, the reaction solution was concentrated at 45°C. Petroleum ether (5 mL*3) was used to remove part of the EA, yielding 174 mg of crude F1018-01-04, which was used directly in the next step.

Step 3: F1018-01-04 (174 mg, crude product) was dissolved in DMF. F1018-01-05 (143 mg, 1.24 mmol) and DIEA (160 mg, 1.24 mmol) were added, and the mixture was stirred at RT for 0.5 hour. After LCMS confirmed complete consumption of the starting material, acetic acid was added to quench the reaction until the pH turned acidic. The mixture was concentrated using an oil pump at 45°C and then subjected to low-to-medium pressure reverse-phase prep-LC (mobile phase: acetonitrile/0.05% TFA aqueous solution) followed by lyophilization to afford 180 mg of compound F1018-01-06 as a transparent oil; yield 88.4%. LCMS (254 nm) purity: 93.1%, Rt = 1.95 min; MS calculated: 821.3; MS found: 822.5 $[M+H]^+$.

Step 4: Compound F1018-01-06 (180 mg, 0.22 mmol) was dissolved in DMF (2.0 mL), followed by addition of piperidine (200 μL). Under a nitrogen atmosphere, the mixture was stirred and reacted for 0.5 hour. Upon confirmation of completion by LCMS, the reaction solution was concentrated to dryness using an oil pump at 45°C. The residue was triturated with petroleum ether (2 mL*3), and the supernatant was decanted to yield 145 mg of crude compound F1018-01, which was used directly in the next step.

Intermediate compound F1016-06 was prepared as follows:

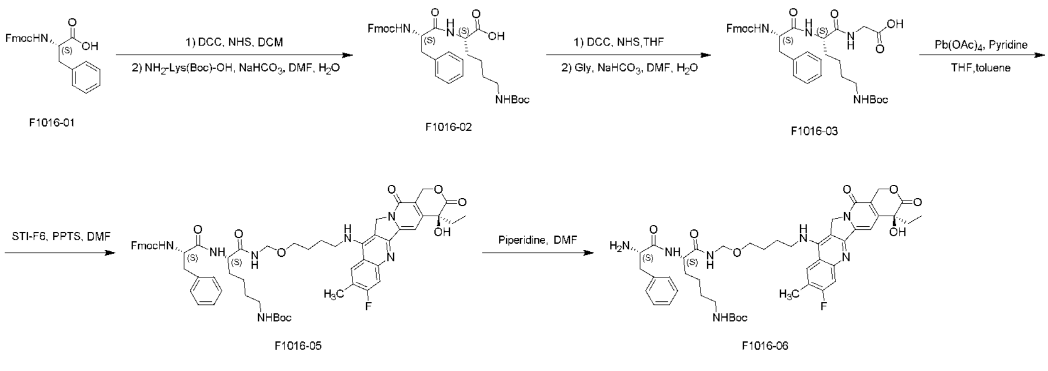


F1016-01 F1016-02 F1016-03 F1016-04 F1016-05 F1016-06

Step 1: F1016-01 (1.0 g, 2.58 mmol) was dissolved in dichloromethane (20.0 mL). Under a nitrogen atmosphere, NHS (357 mg, 3.1 mmol) and DCC (640 mg, 3.1 mmol) were added, and the reaction was carried out in an ice bath for 1 hour. Upon confirmation of completion by TLC monitoring, the reaction solution was filtered, and the filter cake was washed with DCM (5 mL*3). The filtrate was concentrated to dryness at RT, and the crude product was used directly in the next step. The above crude product was dissolved in DMF/$H_2O$=1:1 (20 mL). $NH_2$-Lys(Boc)-OH (635 mg, 2.58 mmol) and $NaHCO_3$ (665 mg, 7.75 mmol) were added, and the reaction was carried out in an ice bath for 2 hours. Upon confirmation of

completion by LCMS, 1M hydrochloric acid was added to adjust the pH to 1~2. The mixture was extracted with ethyl acetate (10 mL*3), washed with saturated brine (10 mL*2), dried over anhydrous $Na_2SO_4$, and concentrated. The crude product was subjected to low-to-medium pressure reverse-phase prep-LC (mobile phase: acetonitrile/0.05% TFA aqueous solution) and lyophilization to afford 900 mg of compound F1016-02 as a white solid; yield 56.7%. LCMS (254 nm) purity: 95.1%, Rt = 2.12 min; MS calculated: 615.2; MS found: 616.3 $[M+H]^+$.

Step 2: F1016-02 (900 mg, 1.46 mmol) was dissolved in tetrahydrofuran (10.0 mL). Under a nitrogen atmosphere, NHS (168 mg, 1.46 mmol) and DCC (300 mg, 1.46 mmol) were added, and the reaction was carried out in an ice bath for 1 hour. Upon confirmation of completion by TLC monitoring, the reaction solution was filtered, and the filter cake was washed with THF (5 mL*3). The mother liquor was concentrated to dryness at RT and used directly in the next step, yielding 1.1 g of crude product. The above concentrate was dissolved in a mixture of DMF/$H_2O$/acetone=1:1:1 (10 mL). Glycine (110 mg, 1.46 mmol) and $NaHCO_3$ (369 mg, 4.38 mmol) were added, and the reaction was carried out in an ice bath for 2 hours. Upon confirmation of completion by LCMS, 1N hydrochloric acid was added to adjust the pH to 1~2. The mixture was extracted with ethyl acetate (10 mL*3), washed with saturated brine (10 mL*2), dried over anhydrous $Na_2SO_4$, and concentrated. The crude product was subjected to low-to-medium pressure reverse-phase prep-LC (mobile phase: acetonitrile/0.05% TFA aqueous solution) and lyophilization to afford 800 mg of compound F1016-03 as a white solid; yield 81.5%. LCMS (254 nm) purity: 96.1%, Rt = 2.02 min; MS calculated: 672.3; MS found: 673.4 $[M+H]^+$.

Step 3: F1016-03 (800 mg, 1.19 mmol) was dissolved in a mixture of tetrahydrofuran (16 mL) and toluene (4 mL). $Pb(OAc)_4$ (633 mg, 1.42 mmol) and pyridine (113 mg, 1.42 mmol) were added. Under a nitrogen atmosphere, the reaction mixture was stirred at 85°C for 2.5 hours. After LCMS confirmed complete consumption of the starting material, the mixture was concentrated to dryness at 45°C. The crude product was purified by column chromatography (petroleum ether/ethyl acetate=2/3) to afford 460 mg of compound F1016-04 as a white solid; yield 56.3%. LCMS (254 nm) purity: 93.2%, Rt = 1.85 min; MS calculated: 686.3; MS found: 704.1 $[M+NH_4]^+$. $^1H$ NMR (400 MHz, DMSO-$d_6$) $\delta$ 8.91 (t, $J$ = 6.8 Hz, 1H), 8.10 (d, $J$ = 8.0 Hz, 1H), 7.87 (d, $J$ = 7.2 Hz, 2H), 7.63 - 7.58 (m, 3H), 7.42-7.38 (m, 2H), 7.32-7.26 (m, 7H), 6.72 (t, $J$ = 4.8 Hz, 1H), 5.11-5.06 (m, 2H), 4.31 - 4.13(m, 5H), 3.03-2.73 (m, 4H), 1.98 (s, 3H), 1.63-1.49 (m, 2H), 1.34-1.18 (m, 13H).

Step 4: Compound F1016-04 (176 mg, 0.25 mmol) was dissolved in DMF (5 mL). Under a nitrogen atmosphere, STI-F6 (120 mg, 0.25 mmol) and PPTS (12 mg, catalytic amount) were added sequentially, and the reaction was carried out at 95 °C for 2 hours. The reaction solution was concentrated to dryness under reduced pressure at 45 °C. The crude product was purified by column chromatography (dichloromethane/methanol=50/1) to afford 50 mg of compound F1016-05 as a yellow solid, with a purity of 50%. MS calculated: 1093.5; MS found: 1094.6 $[M+H]^+$.

Step 5: Compound F1016-05 (60 mg, 0.05 mmol) was dissolved in DMF (2.0 mL), followed by addition of piperidine (200 μL). Under a nitrogen atmosphere, the mixture was stirred and reacted for 0.5 hour. Upon confirmation of completion by LCMS, the reaction solution was concentrated to dryness using an oil pump at 45°C. The residue was triturated with petroleum ether (2 mL*3), and the supernatant was decanted. The resulting material was concentrated again to yield 43 mg of crude compound F1016-06, which was used directly in the next step.

### Example 40: Synthesis of Linker-payload Conjugate STI-F1019

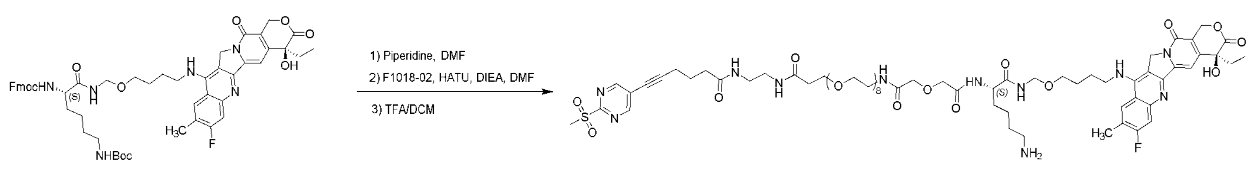


F1013-05 STI-F1019

Compound F1013-05 (30 mg, 0.03 mmol) from Example 36 was dissolved in DMF (2.0 mL), followed by addition of piperidine (200 μL). Under a nitrogen atmosphere, the mixture was stirred and reacted for 0.5 hour. Upon confirmation of completion by LCMS, the reaction solution was concentrated to dryness using an oil pump at 45°C. The residue was triturated with petroleum ether (2 mL*3), and the supernatant was decanted. The resulting material was concentrated again to yield 24 m of crude Fmoc-deprotected product.

Compound F1018-02 (32 mg, 0.03 mmol) from Example 39 was dissolved in DMF (2.0 mL). Under a nitrogen atmosphere, HATU (22 mg, 0.05 mmol) and DIEA (15 mg, 0.11 mmol) were added. After stirring for 5 minutes, the aforementioned Fmoc-deprotected crude product was added, and the reaction was continued for 1 hour. Upon confirmation of completion by LCMS, the reaction solution was concentrated to dryness at 45°C. The resulting crude product was purified by column chromatography (DCM/MeOH = 20:3) to afford a condensation product.

The resulting condensation product was dissolved in 10% TFA/DCM (2 mL) and stirred under a nitrogen

atmosphere in an ice bath for 1 hour. The completion of the reaction was monitored by LCMS. The reaction solution was quenched with triethylamine to adjust the pH to 7~8, purged dry with nitrogen, and then subjected to high-pressure reverse-phase prep-LC (mobile phase: acetonitrile/0.05% TFA aqueous solution) and lyophilisation to afford 2.87 mg of compound STI-F1019 as a white solid. LCMS (254 nm) purity: 99.6%, Rt = 1.51 min; MS calculated: 1455.6; MS found: 1456.7 [M+H]+. $^1$H NMR (400 MHz, DMSO-$d_6$) δ 9.11 (s, 2H), 8.78 (t, $J$ = 6.4 Hz, 1H), 8.41 - 8.39 (d, $J$ = 7.6 Hz, 1H), 8.11 (d, $J$ = 8.0 Hz, 1H), 8.03(t, $J$ = 4.2 Hz, 1H), 7.88 (s, 2H), 7.65-7.59 (m, 3H), 7.39 (s, 1H), 6.57 (brs, 1H), 5.48 (d, $J$ = 38 Hz, 4H), 4.59 - 4.57 (m, 2H), 4.29 - 4.26(m, 1H), 4.01 - 3.90 (m, 3H), 3.70 - 3.59 (m, 7H), 3.45 - 3.30 (m, 30H), 3.32 - 3.16 (m, 4H), 3.09 - 3.07 (m, 3H), 2.77 - 2.73 (m, 2H), 2.63-2.53 (m, 4H),2.44 (s, 3H), 2.32 - 2.22 (m, 5H), 1.89 - 1.64 (m, 12H), 1.30 - 1.23(m, 2H), 0.87 (t, $J$= 7.2 Hz, 3H).

**Example 41: Synthesis of Linker-payload Conjugate STI-G1001**

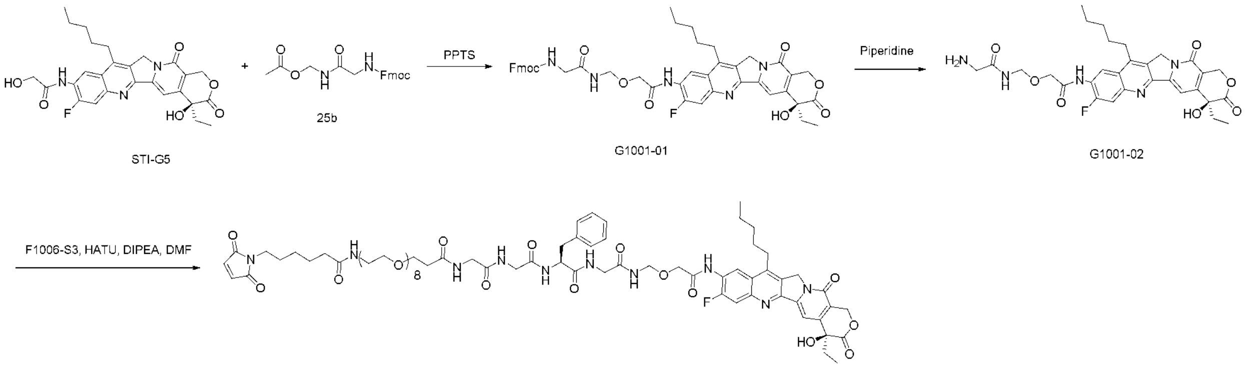


Step 1: Compound STI-G5 (140 mg, 0.275 mmol) was dissolved in 2.8 mL N,N-dimethylacetamide. Compound 25b (152 mg, 0.42 mmol) and PPTS (7 mg, 0.0275 mmol) were added. Under a nitrogen atmosphere, the reaction was carried out at 80°C for 4 hours. After completion, the reaction solution was concentrated to dryness. The residue was triturated with acetone and filtered. The filtrate was concentrated and purified by column chromatography to afford approximately 100 mg of crude compound G1001-01 as a yellow solid; yield approximately 40%. LCMS (254 nm) Rt = 1.918 min; MS calculated: 817.3; MS found: 816.3 [M-H]$^-$.

Step 2: Compound G1001-01 (100 mg, 0.122 mmol) was placed in a reaction flask, and 2 mL a 10% piperidine/dichloromethane solution was added. The reaction was carried out at RT for 30 minutes. After completion, the reaction solution was concentrated to dryness. The crude product was triturated with acetone and filtered. The filter cake afforded approximately 50 mg of compound G1001-02 as a yellow solid; yield approximately 68%. LCMS (254 nm) Rt = 1.539 min; MS calculated: 595.24; MS found: 596.30 [M+H]$^+$.

Step 3: Compound G1001-02 (50 mg, 0.084 mmol) was placed in a reaction flask. F1006-S3 (75 mg, 0.084 mmol) from Example 37 was added, followed by 1 mL DMF to dissolve the mixture. DIPEA (30 μL, 0.168 mmol) and HATU (60 mg, 0.168 mmol) were then added, and the reaction was carried out at RT for 30 minutes. Upon confirmation of completion by LCMS, the reaction solution was concentrated to dryness by rotary evaporation. The resulting material was separated by high-pressure preparation (mobile phase: acetonitrile/0.05% formic acid aqueous solution) and lyophilized to afford approximately 15 mg of compound STI-G1001 as a yellow solid. LCMS (254 nm) purity: 97.5%, Rt = 1.620 min; MS calculated: 1472.68; MS found: 737.50 [M/2+H]$^+$. $^1$H NMR (400 MHz, DMSO-$d_6$) δ 9.78 (s, 1H), 9.70 (s, 1H), 8.95 (d, $J$ = 8.4 Hz, 1H), 8.87 (d, $J$ = 8.4 Hz, 1H), 8.72 (t, $J$ = 6.8 Hz, 1H), 8.33 (t, $J$ = 5.6 Hz, 1H), 8.18 - 8.11 (m, 2H), 8.04-7.99 (m, 2H), 7.81 - 7.99 (m, 1H), 7.30 (s, 2H), 7.22 (s, 2H), 6.99 (s, 1H), 6.52 (brs, 1H), 5.43 (s, 2H), 5.29 (s, 2H), 4.74 (d, $J$ = 6.8 Hz, 1H), 4.51 - 4.47 (m, 1H), 4.22 (s, 2H), 4.15 (s, 2H), 3.80 - 3.76(m, 2H), 3.72 - 3.67 (m, 2H), 3.64 - 3.57 (m, 2H), 3.49 (s, 34 H), 3.24 - 3.15 (m, 2H), 3.14 - 3.10 (m, 2H), 3.05 - 3.01 (m, 2H), 2.38 (t, $J$ = 6.4 Hz, 2H), 2.04 - 1.99 (m, 2H), 1.90 - 1.81 (m, 2H), 1.73 (s, 2H), 1.47 - 1.44 (m, 4H), 1.40 - 1.36 (m, 2H), 1.23(s, 4H), 0.91 - 0.87 (m, 6H)

**Example 42: Synthesis of Linker-payload Conjugate STI-F1031**

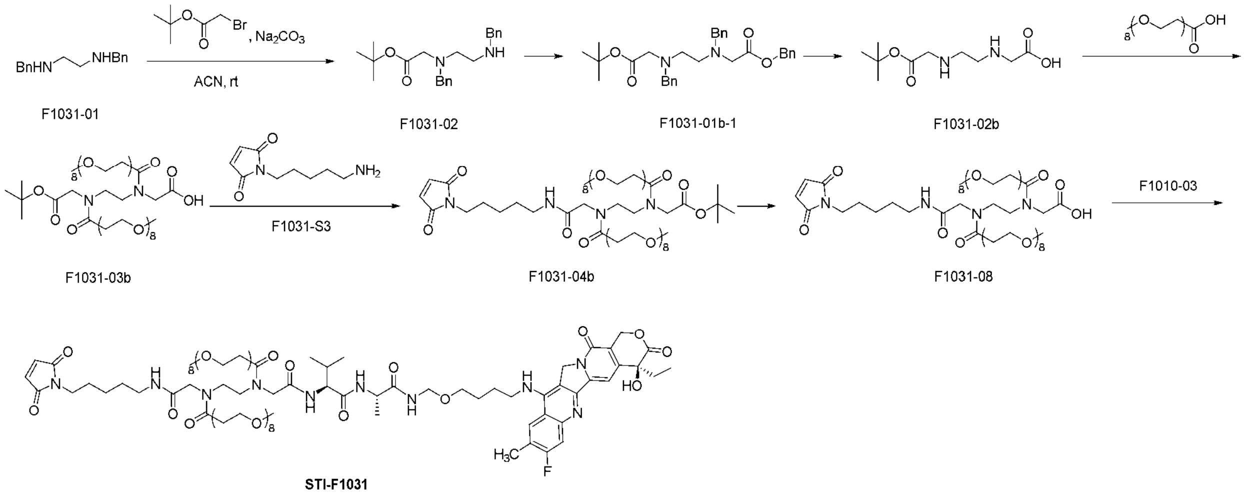


F1031-01 F1031-02 F1031-01b-1 F1031-02b

F1031-03b F1031-04b F1031-08

STI-F1031

Step 1: Compound F1031-01 (2.88 g, 12.0 mmol) was dissolved in acetonitrile (48.0 mL), followed by addition of sodium carbonate (1272 mg, 12.0 mmol). Under a nitrogen atmosphere, a solution of tert-butyl bromoacetate (585 mg, 3.0 mmol) in acetonitrile was added dropwise to the system. The reaction was carried out at RT for 24 hours. After completion, the mixture was filtered, and the filtrate was concentrated under reduced pressure. The residue was extracted with ethyl acetate and water. The organic phase was dried over anhydrous $Na_2SO_4$ and concentrated under reduced pressure. The crude product was purified by column chromatography (PE:EA = 1:1) to afford 1.1 g of compound F1031-02 as a yellow solid; yield 25.9%. LCMS (254 nm) purity: 93.2%, Rt = 1.816 min; MS calculated: 354.2; MS found: 355.3 $[M+H]^+$.

Step 2: Compound F1031-02 (1200 mg, 3.39 mmol) was dissolved in acetonitrile (20.0 mL), followed by addition of sodium carbonate (1030 mg, 10.0 mmol). Under a nitrogen atmosphere, a solution of benzyl bromoacetate (1214 mg, 5.3 mmol) in acetonitrile was added dropwise to the system. The reaction was carried out at RT for 1 hour. After completion, the mixture was filtered, and the filtrate was concentrated under reduced pressure. The crude product was purified by column chromatography (PE:EA = 30:1) to afford 1.5 g of compound F1031-01b-1 as a yellow oily liquid; yield 88.2%. LCMS (254 nm) purity: 93.2%, Rt = 2.421 min; MS calculated: 502.2; MS found: 503.6 $[M+H]^+$.

Step 3: Compound F1031-01b-1 (1.0 g, 2.0 mmol) was dissolved in methanol (20.0 mL). 10% Palladium on carbon (100 mg) was added, and the atmosphere was replaced with hydrogen. The reaction was carried out at RT overnight. After LCMS confirmed completion, the reaction was stopped. The mixture was filtered, and the filtrate was concentrated to dryness under reduced pressure to afford 445 mg of compound F1031-02b as a colorless oily liquid; yield 95.9%. LCMS (254 nm) purity: 91.0%, Rt = 1.910 min; MS calculated: 232.1; MS found: 233.2 $[M+H]^+$.

Step 4: 4,7,10,13,16,19,22,25-Octaoxahexacosanoic acid (412 mg, 1.0 mmol) and pentafluorophenol (221 mg, 1.2 mmol) were dissolved in tetrahydrofuran (5.0 mL). Under a nitrogen atmosphere, the mixture was cooled to 0~5°C, and N,N'-dicyclohexylcarbodiimide (247 mg, 1.2 mmol) was added. The reaction was carried out at RT for 4 hours. The mixture was filtered, and the filtrate was concentrated to dryness under reduced pressure. Compound F1031-02b (126 mg, 0.5 mmol) and DMF (5.0 mL) were added to the residue. Under a nitrogen atmosphere, DIPEA (258 mg, 2.0 mmol) was added, and the reaction was carried out at RT overnight. After LCMS confirmed completion, the system was concentrated to dryness under reduced pressure. The crude product (F1031-03b) was used directly in the next step.

Step 5: The crude product from the previous step was redissolved in DMF (5.0 mL). F1031-S3, HATU (570 mg, 1.5 mmol), and DIPEA (387 mg, 3.0 mmol) were added. Under a nitrogen atmosphere, the reaction was carried out at RT for 1 hour. After LCMS confirmed completion, the reaction was stopped. The system was concentrated to dryness, then subjected to reverse-phase prep-LC (mobile phase: acetonitrile/0.05% TFA aqueous solution) and lyophilization to afford 240 mg of compound F1031-04b as a colorless oily liquid; yield 40.2%. LCMS (254 nm) purity: 94.1%, Rt = 1.526 min; MS calculated: 1184.7; MS found: 1183.35 $[M-H]^-$.

Step 6: Compound F1031-04b (100 mg, 0.084 mmol) was dissolved in a solution of 25% TFA in dichloromethane and stirred at RT for approximately 2 hours. Upon confirmation of completion by LCMS, the reaction was concentrated to dryness under reduced pressure to afford 130 mg of compound F1031-08 as a colorless oily liquid; yield 100.0%. LCMS (254 nm) purity: 95.1%, Rt = 1.513 min; MS calculated: 1128.6; MS found: 1127.7 $[M-H]^-$.

Step 7: Compound F1010-03 (40 mg, 0.045 mmol) from Example 33 was dissolved in DMF (0.9 mL). Piperidine (0.1 mL) was added, and the reaction was carried out at RT for 20 minutes. The mixture was concentrated to dryness, and the residue was washed with PE and dried under reduced pressure. To the residue, DMF (2.0 mL), HATU (34 mg, 0.09 mmol), F1031-08 (56 mg, 0.05 mmol), and triethylamine (23 mg, 0.23 mmol) were added. Under a nitrogen atmosphere, the reaction was carried out at RT for 1.0 hour. After completion, the system was concentrated to dryness, and then subjected to medium-to-high pressure reverse-phase prep-LC (mobile phase: acetonitrile/0.05% TFA aqueous solution)

and lyophilization to afford 17 mg of compound STI-F1031 as a yellowish solid; yield 21.3%. LCMS (254 nm) purity: 98.58%, Rt = 1.623 min; MS calculated: 1776.9; MS found: 1776.0. $[M-H]^-$. $^1H$ NMR (400 MHz, DMSO-$d_6$) δ 8.63 - 8.56 (m, 1H), 8.48 - 8.35 (m, 2H), 8.15 (t, *J* = 6.8 Hz, 1H), 8.10 - 8.04 (m, 1H), 8.00 (d, *J* = 6.5 Hz, 1H), 7.60 (d, *J* = 10.6, 1H), 7.37 (s, 1H) , 6.98 (s, 2H), 6.55 (br, 1H), 5.48 (s, 2H), 5.43 (s, 2H), 4.72 - 4.69 (m, 1H), 4.56 - 4.46 (m, 2H), 4.25 - 4.18 (m, 3H), 4.12 (t, *J* = 4.6 Hz, 1H), 4.08 - 3.96 (m, 2H), 3.70 - 3.65 (m, 3H), 3.62 - 3.58 (m, 4H), 3.51 - 3.46 (m, 63H), 3.23 (s, 6H), 3.05 - 2.99 (m, 4H), 2.61 - 2.56 (m, 4H), 2.44 (s, 3H), 2.04 - 1.97 (m, 2H), 1.88 - 1.83 (m, 2H), 1.74 (q, *J* = 7.4 Hz, 2H), 1.64 (q, *J* = 6.8 Hz, 2H), 1.51 - 1.42 (m, 2H), 1.21 (d, *J* = 7.1 Hz, 3H), 0.87 (t, *J* = 7.2 Hz, 3H), 0.82 (dd, *J* = 11.2, 4.9 Hz, 6H).

Intermediate compound F1031-S3 was prepared as follows:

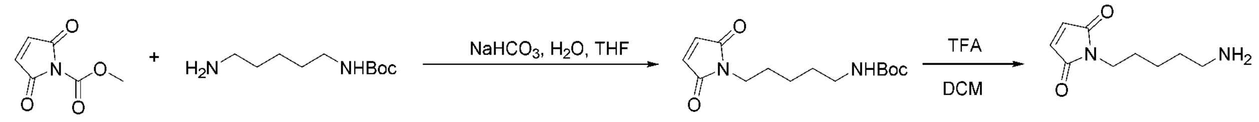


F1031-S1 F1031-S2 F1031-S3

Step 1: Tert-butyl N-(5-Aminopentyl)carbamate (1414 mg, 7.0 mmol) was added to a saturated sodium bicarbonate solution (35.0 mL) and cooled to 0~5 °C. F1031-S1 (1085 mg, 7.0 mmol) was added, and the mixture was allowed to warm to RT and stirred for 15 minutes. Tetrahydrofuran (55.0 mL) was added, and the reaction was continued at RT for 30 minutes before being stopped. To the system were added 50 mL water and EA. The mixture was extracted, and the organic phase was dried over anhydrous $Na_2SO_4$. Purification by column chromatography (DCM:EA = 20:1) afforded 1.1 g of compound F1031-S2 as a white solid; yield 55.7%. LCMS (254 nm) purity: 93.2%, Rt = 1.723 min; MS calculated: 282.2; MS found: 283.1 $[M+H]^+$.

Step 2: Compound F1031-S2 (423 mg, 1.5 mmol) was dissolved in a solution of 25% TFA in dichloromethane and stirred at RT for approximately 2 hours. Upon confirmation of completion by LCMS, the mixture was concentrated to dryness under reduced pressure to afford F1031-S3, which was used directly in the next step.

**Example 43: Synthesis of Linker-payload Conjugate STI-F1034**

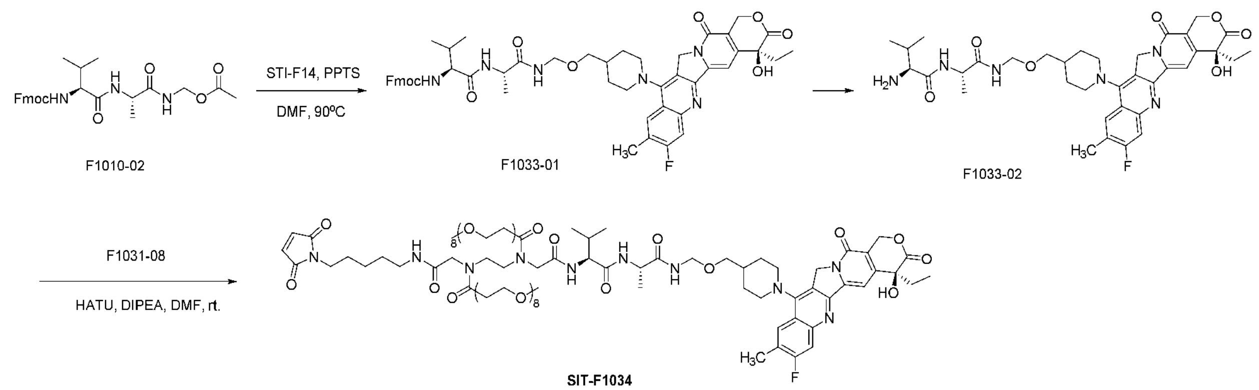


F1010-02 F1033-01 F1033-02

SIT-F1034

Step 1: Compound STI-F14 (276 mg, 0.56 mmol), compound F1010-02 (269 mg, 0.56 mmol) from Example 33, and PPTS (20 mg) were added to a reaction flask, followed by the addition of DMF (10 mL). Under a nitrogen atmosphere, the reaction mixture was heated to 90°C and reacted for 5 hours before being stopped. The system was then concentrated to dryness under reduced pressure and purified by column chromatography (DCM:MeOH=100:1~60:1) to afford 154 mg of compound F1033-01 as a yellowish solid in 30.0% yield. LCMS (254 nm) purity: 76.8%, Rt = 2.043 min; MS calculated: 914.4; MS found: 915.4 $[M+H]^+$.

Step 2: Compound F1033-01 (154 mg, 0.17 mmol) was dissolved in DMF (3 mL), followed by addition of piperidine (300 μL). Under a nitrogen atmosphere, the mixture was stirred and reacted for 0.5 hour. Upon confirmation of completion by LCMS, the reaction solution was concentrated to dryness using an oil pump at 45°C. The residue was triturated with petroleum ether and filtered to afford 100 mg of compound F1033-02 as a yellowish solid; yield 85.0%. LCMS (254 nm) purity: 98.7%, Rt = 1.692 min; MS calculated: 692.3; MS found: 693.4 $[M+H]^+$. $^1H$ NMR (400 MHz, DMSO-$d_6$) δ 8.74 (t, J = 6.6 Hz, 1H), 8.13 (s, 1H), 7.91 (d, J = 8.4 Hz, 1H), 7.76 (d, J = 10.7 Hz, 1H), 7.27 (s, 1H), 6.51 (s, 1H), 5.46 (s, 2H), 5.43 (s, 2H), 4.67 - 4.55 (m, 2H), 4.37 - 4.29 (m, 1H), 3.63 (d, J = 11.2 Hz, 2H), 3.44 - 3.37 (m, 4H), 3.07 - 3.01 (m, 1H), 2.48 (s, 3H), 1.98 - 1.88 (m, 2H), 1.89 - 1.79 (m, 4H), 1.60 - 1.46 (m, 2H), 1.26 (d, J = 7.0 Hz, 3H), 1.24 - 1.14 (m, 2H), 0.90 - 0.86

(m, 6H), 0.79 (d, J = 6.8 Hz, 3H).

Step 3: Compound F1033-02 (67 mg, 0.097 mmol), F1031-08 (120 mg, 0.106 mmol) from Example 42, and HATU (74 mg, 0.194 mmol) were dissolved in DMF (3 mL), followed by addition of DIPEA (82 μL, 0.485 mmol). Under a nitrogen atmosphere, the reaction was carried out at RT for 1 hour. The system was concentrated to dryness, then subjected to medium-to-high pressure reverse-phase prep-LC (mobile phase: acetonitrile/0.05% TFA aqueous solution) and lyophilization to afford 12.5 mg of compound STI-F1034 as a yellow oil; yield 6.9%. LCMS (254 nm) purity: 100%, Rt = 1.684 min; MS calculated: 1802.9; MS found: 1803.7 $[M+H]^+$. $^1H$ NMR (400 MHz, DMSO-$d_6$) δ 8.47 (s, 1H), 8.25 - 8.17 (m, 1H), 8.13 - 7.99 (m, 2H), 7.92 (d, *J* = 8.5 Hz, 1H), 7.77 (d, *J* = 10.8 Hz, 1H), 7.28 (s, 1H), 6.99 (s, 2H), 6.50 (s, 1H), 5.48 (s, 2H), 5.42 (s, 2H), 4.66 - 4.50 (m, 7H), 4.32 - 4.19 (m, 2H), 3.67 - 3.44 (m, 66H), 3.23 (s, 6H), 3.09 - 2.93 (m, 3H), 2.70 - 2.57 (m, 4H), 2.48 (s, 3H), 2.44 - 2.36 (m, 4H), 2.36 - 2.30 (m, 1H), 2.05 - 1.94 (m, 2H), 1.93 - 1.78 (m, 5H), 1.44 - 1.34 (m, 2H), 1.31 - 1.21 (m, 5H), 1.23 - 1.14 (m, 2H), 0.93 - 0.80 (m, 9H).

**Example 44: Synthesis of Linker-payload Conjugate STI-F1035**

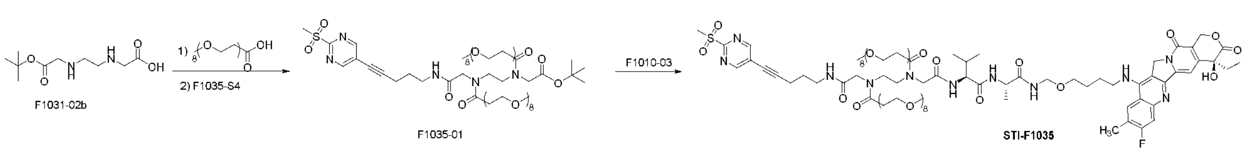


F1031-02b F1035-01 STI-F1035

Step 1: 4,7,10,13,16,19,22,25-Octaoxahexacosanoic acid (412 mg, 1.0 mmol) and pentafluorophenol (221 mg, 1.2 mmol) were dissolved in tetrahydrofuran (5.0 mL). Under a nitrogen atmosphere, the mixture was cooled to 0~5°C, and N,N'-dicyclohexylcarbodiimide (247 mg, 1.2 mmol) was added. The reaction was carried out at RT for 4 hours. The mixture was filtered, and the filtrate was concentrated to dryness under reduced pressure. Compound F1031-02b (100 mg, 0.43 mmol) and DMF (3.0 mL) were added to the residue. Under a nitrogen atmosphere, DIPEA (111 mg, 0.86 mmol) was added, and the reaction was carried out at RT overnight. After LCMS confirmed completion, the system was concentrated to dryness under reduced pressure. The residue was redissolved in DMF (5.0 mL). F1035-S4, HATU (327 mg, 0.86 mmol), and DIPEA (166 mg, 1.29 mmol) were added. Under a nitrogen atmosphere, the reaction was carried out at RT for 1 hour. After LCMS confirmed completion, the reaction was stopped. The system was concentrated to dryness, then subjected to reverse-phase prep-LC (mobile phase: acetonitrile/0.05% TFA aqueous solution) and lyophilization to afford 179 mg of compound F1035-01 as a colorless oily liquid; yield 33.5%. LCMS (254 nm) purity: 98.0%, Rt = 1.537 min; MS calculated: 1241.6; MS found: 1240.6 $[M-H]^-$.

Step 2: Compound F1035-01 (59 mg, 0.05 mmol) was dissolved in a solution of 25% TFA in dichloromethane and stirred at RT for approximately 5 hours. Upon confirmation of completion by LCMS, the mixture was concentrated to dryness under reduced pressure to afford the deprotected product of F1035-01. Compound F1010-03 (40 mg, 0.045 mmol) from Example 33 was dissolved in DMF (0.9 mL). Piperidine (0.1 mL) was added, and the reaction was carried out at RT for 20 minutes. The system was concentrated to dryness, and the residue was washed with petroleum ether and dried under reduced pressure. To the residue, the deprotected product of F1035-01, DMF (2.0 mL), HATU (34 mg, 0.09 mmol), and triethylamine (23 mg, 0.23 mmol) were added. Under a nitrogen atmosphere, the reaction was carried out at RT for 0.5 hour. After the reaction was stopped, the system was concentrated to dryness, then subjected to medium-to-high pressure reverse-phase prep-LC (mobile phase: acetonitrile/0.05% TFA aqueous solution) and lyophilization to afford 20 mg of compound STI-F1035 as a yellow solid; yield 24.2%. LCMS (254 nm) purity: 95.23%, Rt = 1.633 min; MS calculated: 1833.9; MS found: 1833.0 $[M-H]^-$. $^1H$ NMR (400 MHz, DMSO-$d_6$) δ 9.10 (s, 2H), 8.60 (t, *J* = 6.0 Hz, 1H), 8.45 - 8.40 (m, 2H), 8.16 (t, *J* = 6.7 Hz, 1H), 8.08 (d, *J* = 8.5 Hz, 1H), 7.91 - 7.80 (m, 1H), 7.58 (d, *J* = 10.5 Hz, 1H), 7.41 (s, 1H), 6.58 (br, 1H), 5.47 (s, 2H), 5.44 (s, 2H), 4.56 - 4.49 (m, 2H), 4.26 - 4.18 (m, 3H), 4.13 - 4.11 (m, 1H), 4.05 - 3.95(m, 2H), 3.73 - 3.68 (m, 2H), 3.61 - 3.56 (m, 4H), 3.51 - 3.46 (m, 65H), 3.40 (s, 6H), 3.23 (s, 3H), 2.63 - 2.55 (m, 4H), 2.43 (s, 3H), 2.41 - 2.39 (m, 2H), 2.01 - 1.91 (m, 2H), 1.90 - 1.83 (m, 2H), 1.76 - 1.73 (m, 2H), 1.68 - 1.60 (m, 2H), 1.23 (d, *J* = 4.6 Hz, 3H), 0.87 (t, *J* = 7.4 Hz, 3H), 0.82 (dd, *J* = 12.7, 6.5 Hz, 6H).

Intermediate compound F1035-S4 was prepared as follows:

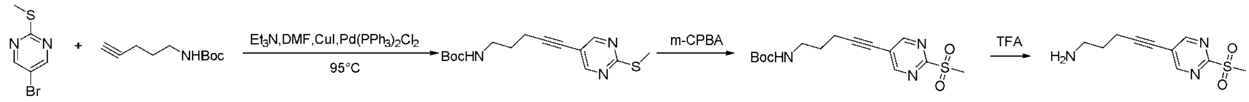


F1035-S1 F1035-S2 F1035-S3 F1035-S4

Step 1: F1035-S1 (303 mg, 1.5 mmol), N-Boc-4-pentyn-1-amine (330 mg, 1.8 mmol), cuprous iodide (30 mg, 0.3

mmol), and bis(triphenylphosphine)palladium dichloride (35 mg, 0.3 mmol) were added to a reaction flask. Under a nitrogen atmosphere, DMF (2.0 mL) and triethylamine (223 mg, 2.25 mmol) were added. The reaction was heated to 95°C and stirred for 5 hours. After the reaction was stopped, water and EA were added for extraction. The EA layer was washed with saturated brine, dried over anhydrous $Na_2SO_4$, and concentrated to dryness under reduced pressure. The crude product was purified by column chromatography (PE:EA=100:1 to PE:EA=10:1) to afford 330 mg of compound F1035-S2 as a yellow solid; yield 71.6%. LCMS (254 nm) purity: 95.0%, Rt = 1.968 min; MS calculated: 307.1; MS found: 308.6 $[M+H]^+$.

Step 2: F1035-S2 (300 mg, 0.98 mmol) was dissolved in dichloromethane. Under a nitrogen atmosphere, meta-chloroperoxybenzoic acid (507 mg, 2.94 mmol) was added, and the reaction was carried out at RT overnight. After LCMS confirmed completion, the reaction was stopped. The mixture was concentrated to dryness under reduced pressure and purified by column chromatography (PE:EA=20:1 to PE:EA=2:1) to afford 260 mg of compound F1035-S3 as a yellow solid; yield 78.3%. LCMS (254 nm) purity: 96.0%, Rt = 1.532 min; MS calculated: 339.1; MS found: 338.2 $[M-H]^-$.

Step 3: Compound F1035-S3 (146 mg, 0.43 mmol) was dissolved in a solution of 25% TFA in dichloromethane and stirred at RT for approximately 2 hours. Upon confirmation of completion by LCMS, the mixture was concentrated to dryness under reduced pressure to afford F1035-S4 as a crude product, which was used directly in the next step.

**Example 45: Synthesis of Linker-payload Conjugate STI-F1043**

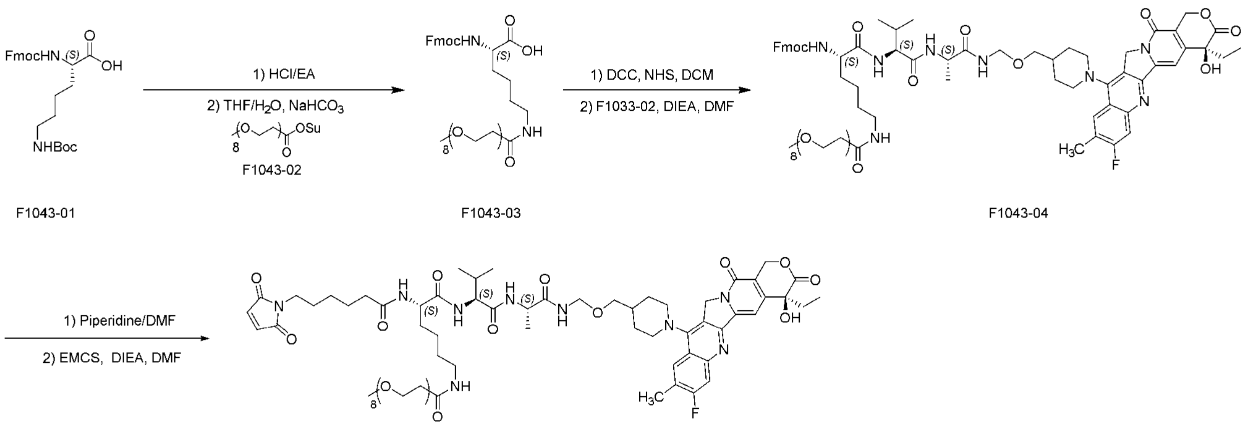


STI-F1043

Step 1: F1043-01 (1.0 g, 2.13 mmol) was dissolved in 4M HCl/EA (10.0 mL) and the reaction was carried out at RT for 1.5 hours. After TLC confirmed completion, the reaction solution was concentrated at 45°C to obtain the crude Boc-deprotected product. F1043-02 (220 mg, crude product) and the above Boc-deprotected product (178 mg, 0.48 mmol) were dissolved in $THF/H_2O$ (2 mL:2 mL). $NaHCO_3$ (203 mg, 2.42 mmol) was added, and the reaction was stirred in an ice bath for 2 hours. Upon confirmation of completion by LCMS, 1M HCl was added to adjust the pH to 3~4. The mixture was extracted with ethyl acetate (10mL*2), washed with saturated brine (10mL*2), and dried over anhydrous $Na_2SO_4$. After concentration at 45°C, direct low-to-medium pressure reverse-phase prep-LC (mobile phase: acetonitrile/0.05% TFA aqueous solution) followed by lyophilization afforded 277 mg of compound F1043-03 as a transparent oil; yield 74.9%. LCMS (254 nm) purity: 93.6%, Rt = 1.96 min; MS calculated: 762.3; MS found: 763.5 $[M+H]^+$.

Step 2: F1043-03 (100 mg, 0.13 mmol) was dissolved in dichloromethane (20.0 mL). Under a nitrogen atmosphere, NHS (18 mg, 0.15 mmol) and DCC (32 mg, 0.15 mmol) were added, and the reaction was carried out in an ice bath for 1 hour. Upon confirmation of completion by TLC, the reaction solution was filtered, and the filter cake was washed with DCM (5 mL*3). The filtrate was concentrated at RT. The crude product and F1033-02 (90 mg, 0.13 mmol) were dissolved in DMF (4 mL). DIEA (33 mg, 0.26 mmol) was added, and the reaction was stirred in an ice bath for 2 hours. Upon confirmation of completion by LCMS, the mixture was concentrated using an oil pump at 45°C. The crude product was purified by column chromatography (dichloromethane/methanol=25/3) to afford 110 mg of compound F1043-04 as a yellow solid; yield 58.9%. LCMS (254 nm) purity: 91.2%, Rt = 1.85 min; MS calculated: 1436.7; MS found: 1437.9 $[M+H]^+$.

Step 3: Compound F1043-04 (110 mg, 0.07 mmol) was dissolved in DMF (2.0 mL). Piperidine (200 μL) was added. The mixture was stirred under a nitrogen atmosphere for 0.5 hour. Upon confirmation of completion by LCMS, the reacton solution was concentrated using an oil pump at 45°C. The residue was triturated with petroleum ether and filtered. The filter cake and EMCS (16 mg, 0.07 mmol) were dissolved in DMF (2 mL). DIEA (19 mg, 0.15 mmol) was added, stirred at RT under a nitrogen atmosphere for 2 hours. Upon confirmation of completion by LCMS, the mixture was concentrated

using an oil pump at 45°C. The crude product was subjected to high-pressure reverse-phase prep-LC (mobile phase: acetonitrile/0.05% TFA aqueous solution) and lyophilization to afford 7.18 mg of compound STI-F1043 as a yellow solid; yield 6.6%. LCMS (254 nm) purity: 99.0%, Rt = 1.71 min; MS calculated: 1407.7; MS found: 1408.6 $[M+H]^+$. $^1H$ NMR (400 MHz, DMSO). δ8.81 (s, 1H), 8.64 (t, *J* = 6.4 Hz, 1H), 8.06 (d, *J* = 6.8 Hz, 1H), 7.95-7.88 (m, 2H), 7.80-7.72 (m, 2H), 7.64 (d, *J* = 8.8 Hz, 1H), 7.26 (s, 1H), 6.99 (s, 2H), 5.43 (d, *J* = 9.6 Hz, 4H), 4.64-4.55 (m, 2H), 4.29-4.15 (m, 3H), 3.58-3.55 (m, 22H), 3.49-3.43 (m, 10H), 3.41-3.33 (m, 5H), 3.23 (s, 4H), 3.00-2.98 (m, 2H), 2.47 (s, 4H), 2.28 (t, *J* = 6.4 Hz, 2H), 1.89-1.80 (m, 4H), 1.65-1.13 (m, 20H), 0.89-0.81 (m, 9H).

Intermediate compound F1043-02 was prepared as follows:

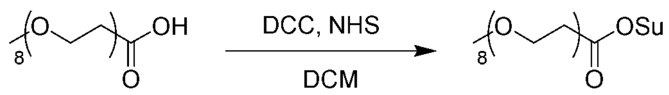


F1043-02a F1043-02

F1043-02a (200 mg, 0.48 mmol) was dissolved in dichloromethane (20.0 mL). Under a nitrogen atmosphere, NHS (67 mg, 0.58 mmol) and DCC (120 mg, 0.58 mmol) were added, and the reaction was carried out in an ice bath for 2 hours. Upon confirmation of completion by TLC monitoring, the reaction solution was filtered and washed with dichloromethane. The filtrate was concentrated to dryness at RT and used directly in the next step.

**Example 46: Preparation of Antibodies**

Farletuzumab is an anti-folate receptor α (FolRα, FRα) antibody, with its sequence information as follows:

Heavy chain sequence of Farletuzumab:

EVQLVESGGGVVQPGRSLRLSCSASGFTFSGYGLSWVRQAPGKGLEWVAMISSGGSYTYY
ADSVKGRFAISRDNAKNTLFLQMDSLRPEDTGVYFCARHGDDPAWFAYWGQGTPVTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKSRWQQGN
VFSCSVMHEALHNHYTQKSLSLSPGK

Light chain sequence of Farletuzumab:

DIQLTQSPSSLSASVGDRVTITCSVSSSISSNNLHWYQQKPGKAPKPWIYGTSNLASGVPSRF
SGSGSGTDYTFTISSLQPEDIATYYCQQWSSYPYMYTFGQGTKVEIKRTVAAPSVFIFPPSDE
QLKSGTASVVCLLNNFYPREAKVQWKVDNALQSGNSQESVTEQDSKDSTYSLSSTLTLSK
ADYEKHKVYACEVTHQGLSSPVTKSFNRGEC

Trastuzumab is an anti-HER2 antibody, with its sequence information as follows:

Light chain sequence of Trastuzumab:

DIQMTQSPSSLSASVGDRVTITCRASQDVNTAVAWYQQKPGKAPKLLIYSASFLYSGVPSR
FSGSRSGTDFTLTISSLQPEDFATYYCQQHYTTPPTFGQGTKVEIKRTVAAPSVFIFPPSDEQL
KSGTASVVCLLNNFYPREAKVQWKVDNALQSGNSQESVTEQDSKDSTYSLSSTLTLSKAD
YEKHKVYACEVTHQGLSSPVTKSFNRGEC

Heavy chain sequence of Trastuzumab:

EVQLVESGGGLVQPGGSLRLSCAASGFNIKDTYIHWVRQAPGKGLEWVARIYPTNGYTRY
ADSVKGRFTISADTSKNTAYLQMNSLRAEDTAVYYCSRWGGDGFYAMDYWGQGTLVTV
SSASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKKVEPKSCDKTHTCPPCPAPELLGGPS
VFLFPPKPKDTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVYTLPPSREEMT
KNQVSLTCLVKGFYPSDIAVEWESNGQPENNYKTTPPVLDSDGSFFLYSKLTVDKSRWQQ
GNVFSCSV MHEALHNHYT QKSLSLSPGK

Isotype is an anti-HEL human IgG1 antibody, with its sequence information as follows:

Light chain sequence of the anti-HEL human IgG1 monoclonal antibody:

DIQMTQSPASLSASVGETVTITCRASGNIHNYLAWYQQKQGKSPQLLVYNAKTLADGVPS
RFSGSGSGTQYSLKINSLQPEDFGSYYCQHFWSTPRTFGGGTKLEIKRTVAAPSVFIFPPSDE
QLKSGTASVVCLLNNFYPREAKVQWKVDNALQSGNSQESVTEQDSKDSTYSLSSTLTLSK

ADYEKHKVYACEVTHQGLSSPVTKSFNRGEC

Heavy chain sequence of the anti-HEL human IgG1 monoclonal antibody:

QVQLQESGPGLVRPSQTLSLTCTVSGSTFSGYGVNWVRQPPGRGLEWIGMIWGDGNT
DYNSALKSRVTMLVDTSKNQFSLRLSSVTAADTAVYYCARERDYRLDYWGQGSLVTVSS
ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKVEPKSCDKTHTCPPCPAPELLGGPSV
FLFPPKPKDTLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPPSRDELTKN
QVSLTCLVKGFYPSDIAVEWESNGQPENNYKTTPPVLDSDGSFFLYSKLTVDKSRWQQGN
VFSCSVMHEALHNHYTQKSLSLSPGK

After the construction of the above sequence vectors, sequencing confirmed that the constructed sequences were consistent with the described sequences. Expi293F cells (Thermo Fisher, Cat. No.: A1452) were used for expression, and Protein A (Cytiva, Cat. No.: 17549801) was used for purification.

**Example 47: Preparation and Assay of Antibody-Drug Conjugates**

The antibody-drug conjugates (ADCs) were synthesized with the camptothecin derivatives of the present invention, and the DARs were determined.

Specifically, the purified antibody (with SEC-HPLC purity >95%) was added to 10 mM PBS buffer. Then, 500 mM EDTA was added to the reaction system to achieve an EDTA concentration of 2 mM and an antibody concentration of approximately 1-5 mg/mL. After addition of 5~8 equivalents of TCEP (TCEP/mAb), the mixture was stirred thoroughly and reacted at 37°C and 600 rpm for 2 hours. After completion of the reaction, the solution was cooled to 4°C, and a solution of the linker-payload conjugate in DMSO (10-16eq, drug/mAb) was added. The mixture was mixed thoroughly, and reacted at 4°C and 600 rpm for 1 hour. Upon completion of the reaction, the solution was transferred to a 30K ultrafiltration tube, concentrated to an appropriate volume, and then loaded onto a pre-equilibrated desalting column. After centrifugation, a purified conjugated sample was obtained. The concentration, purity, and DAR of the sample were then determined. The DAR value can be measured and calculated using the following method:

1. RP-HPLC Assay Method

75 μL of 8 M guanidine hydrochloride and 5 μL of 1 M Tris were added to a centrifuge tube, followed by an

appropriate amount of sample (40 μg, with the volume calculated based on the concentration). Then, water was added to the final volume of 100 μL. Subsequently, 2 μL of 1 M DTT were added. The mixture was vortexed, and then incubated at 30°C for 30 minutes. After incubation, the mixture was allowed to return to RT and centrifuged at 13,000 rpm for 5 minutes. The supernatant was transferred to an HPLC vial. The analysis was performed using a Vanquish high-performance liquid chromatography system equipped with a BioResolve™ RP mAb, Polyphenyl, 450 Å, 2.7 μm, 2.1*100 mm column (Part No: 186008945, Serial No: 01293033515811); column temperature: 80°C; DAD detector wavelength: 280 nm; flow rate: 1 mL/min; and injection volume: 20 μL. The positions of the heavy and light chains were identified by comparing the sample with a reference of the unconjugated antibody. The sample chromatogram was then integrated to calculate the DAR value.

Mobile Phase:

Reversed-phase Column Mobile Pase A: 0.05% TFA+0.05%FA in $H_2O$
Reversed-phase Column Mobile Pase B: 0.05% TFA+0.05%FA in ACN
Data Analysis: Based on the positions of the light and heavy chains, the chromatogram of the test sample was integrated to calculate the DAR value.

The calculation equation is as follows:

| Designation | Number of loaded drugs |
|---|---|
| LC | 0 |
| LC+1 | 2 |
| HC | 0 |
| HC+1 | 2 |
| HC+2 | 4 |
| HC+3 | 6 |

$$\text{Total LC peak area} = \text{LC peak area} + \text{LC+1 peak area}$$

$$\text{Total HC peak area} = \text{HC peak area} + \text{HC+1 peak area} + \text{HC+2 peak areas} + \text{HC+3 peak areas}$$

$$\text{LC DAR} = \Sigma\,(\text{number of loaded drugs} \times \text{Peak area percentage}) / \text{Total LC peak area}$$

$$\text{HC DAR} = \Sigma\,(\text{number of loaded drugs} \times \text{Peak area percentage}) / \text{Total HC peak area}$$

$$\text{DAR} = \text{LC DAR} + \text{HC DAR}$$

2. SEC-HPLC Assay Method

The sample was diluted to 1 mg/mL with ultrapure water. Injection volume: 20 μL; Vanquish high-performance liquid chromatography system equipped with an XBridge® BEH200Å SEC 3.5 μm, 7.8 × 150 mm column (Part No. 186007639, Serial No. 01783201716107); column temperature: 25°C; DAD detector wavelength: 280 nm; flow rate: 0.5 mL/min.

3. Mass Spectrometric Assay Method

Approximately 25 μg of protein was diluted with 50 mM $NH_4HCO_3$ to 0.5 μg/μL. Then, 0.5 μL PNGase F and 1 μL 1 M DTT were added, followed by incubation at 37°C for 60 minutes. An amount of 0.5 μg was injected for mass spectrometry analysis. Analysis was performed using a Waters Xevo G2-XS Q-TOF system equipped with an ACQUITY UPLC BEH C4, 1.7 μm, 2.1 × 50 mm, 300 Å (186004495); column temperature: 80 °C; DAD detector wavelength: 280 nm; flow rate: 0.3 mL/min. The DAR value was calculated based on the MS response signals of the sample.

The antibody-drug conjugates of the present invention were synthesized according to the afore-mentioned

methods, and the DAR values were determined, with the results shown in Table 1.

Table 1. Prepared ADCs and Their Characterization

| ADC Name | Involved Linker-payload Conjugate | ADC Structure | Average DAR (RP-HPLC) |
|---|---|---|---|
| Fa-ADC1 | STI-G | 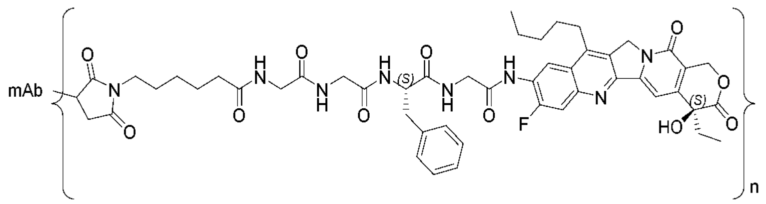 | 7.0 |
| Fa-ADC2 | STI-A3 | 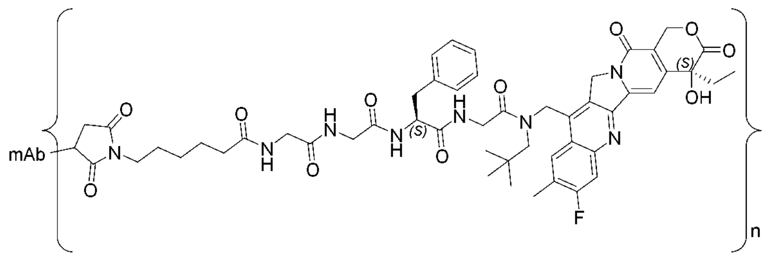 | 7.2 |
| Fa-ADC3 | STI-F3 | 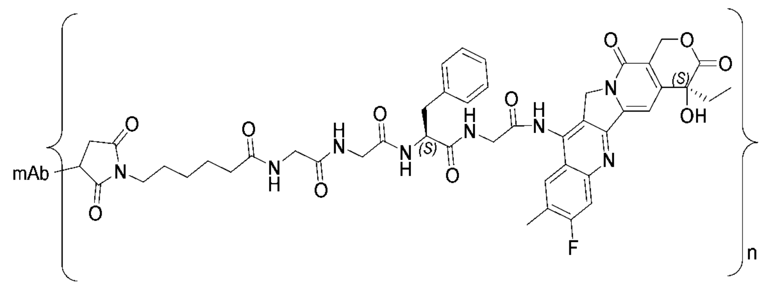 | 7.1 |
| Fa-ADC4 | STI-F4 | 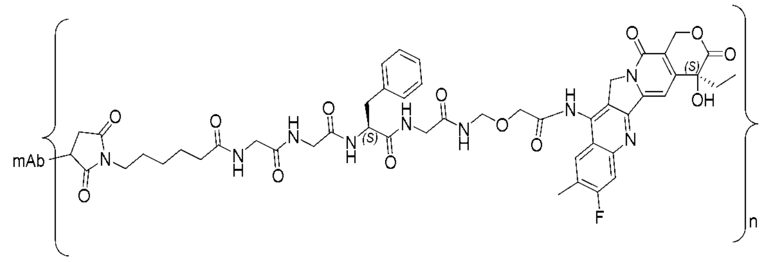 | 7.1 |
| Fa-ADC5 | STI-F1002 | 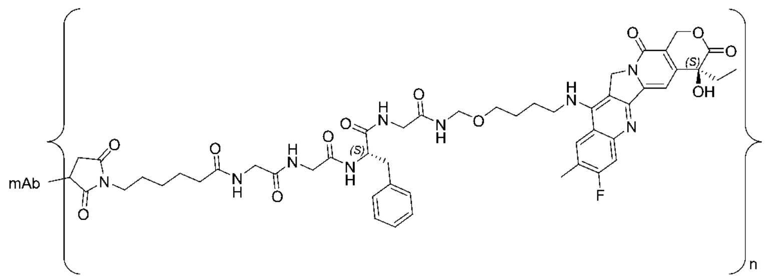 | 5.8 |

(continued)

| ADC Name | Involved Linker-payload Conjugate | ADC Structure | Average DAR (RP-HPLC) |
|---|---|---|---|
| Fa-ADC6 | STI-F1001 | 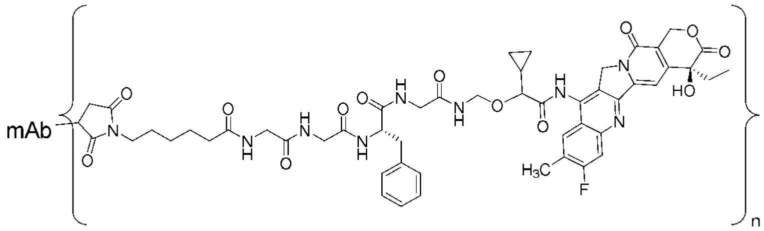 | 7.2 |
| Tra-ADC7 | STI-F1006 | 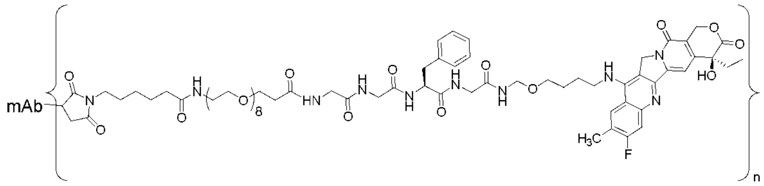 | 7.9 |
| Tra-ADC8 | STI-F1010 | 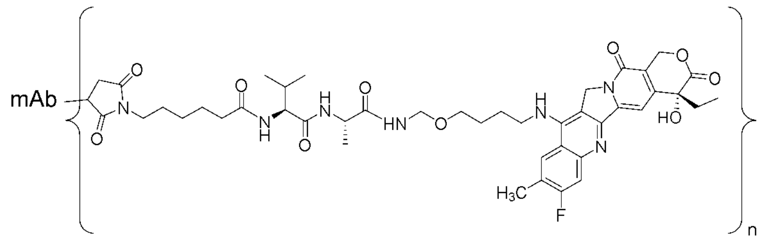 | 8.0 |
| Tra-ADC9 | STI-F1011 | 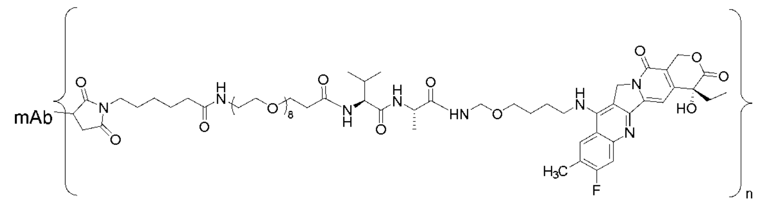 | 8.0 |
| Tra-ADC10 | STI-F1012 | 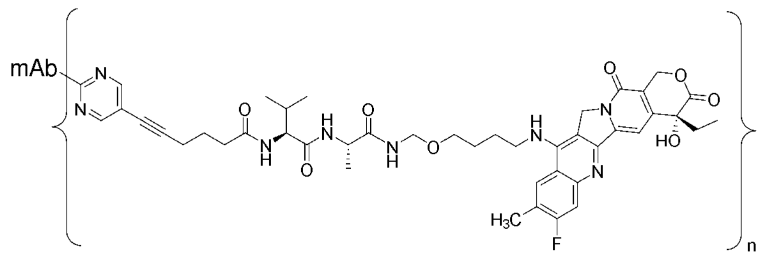 | 7.5 |
| Tra-ADC11 | STI-F1013 | 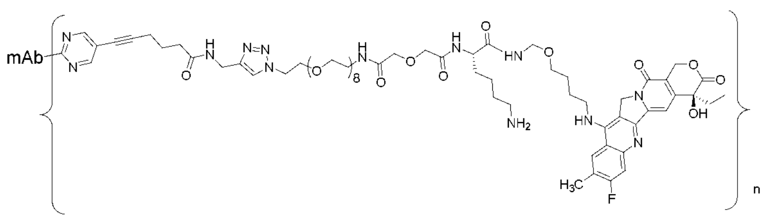 | 8.0 |

(continued)

| ADC Name | Involved Linker-payload Conjugate | ADC Structure | Average DAR (RP-HPLC) |
|---|---|---|---|
| Tra-ADC12 | STI-F1014 | 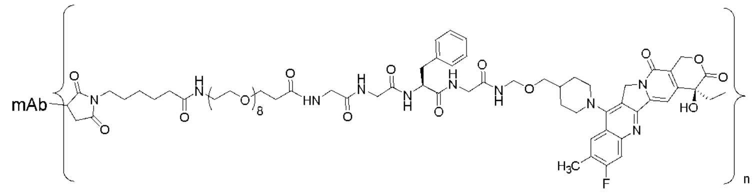 | 7.6 |
| Tra-ADC13 | STI-F1015 | 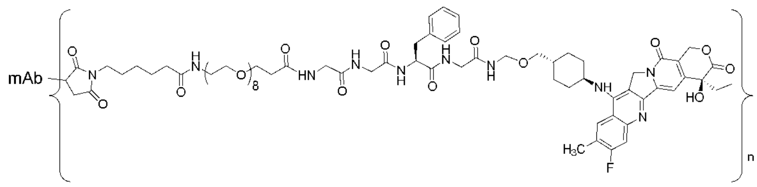 | 7.7 |
| Tra-ADC14 | STI-F1018 | 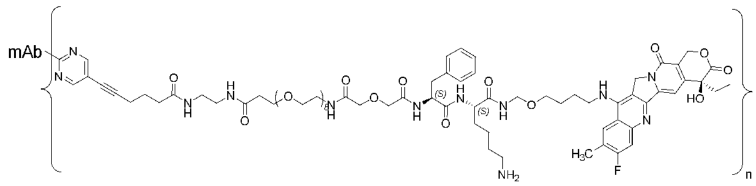 | 8.0 |
| Tra-ADC15 | STI-F1019 | 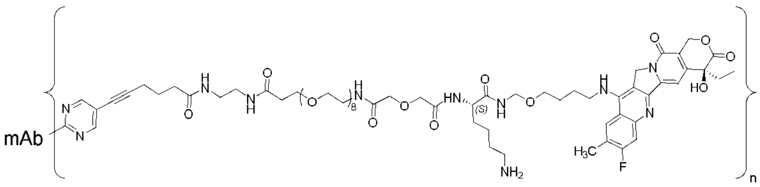 | 8.0 |
| Tra-ADC16 | STI-G1001 | 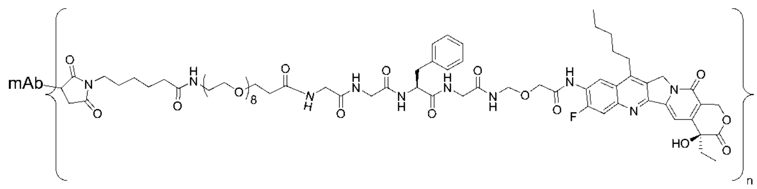 | 7.8 |
| Tra-ADC17 | STI-F1031 | 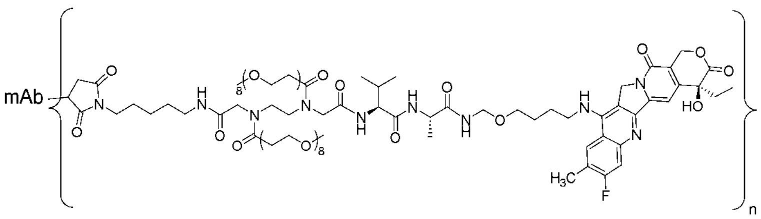 | 7.4 |
| Tra-ADC18 | STI-F1034 | 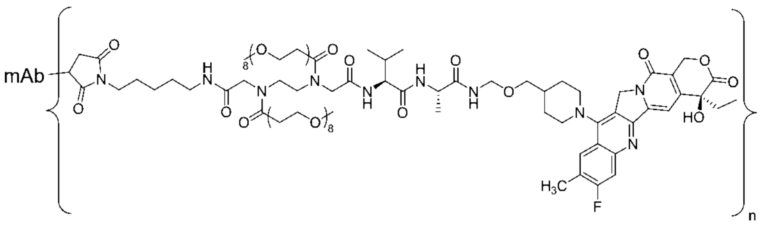 | 7.5 |

(continued)

| ADC Name | Involved Linker-payload Conjugate | ADC Structure | Average DAR (RP-HPLC) |
|---|---|---|---|
| Tra-ADC19 | STI-F1035 | 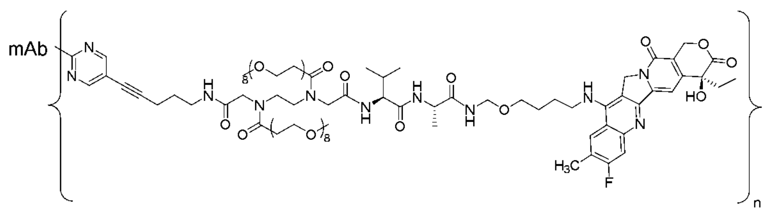 | 7.8 |
| Tra-ADC20 | STI-F1043 | 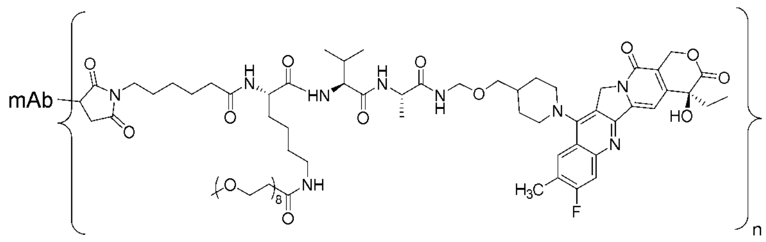 | 7.5 |
| Iso-ADC21 | STI-F1006 | 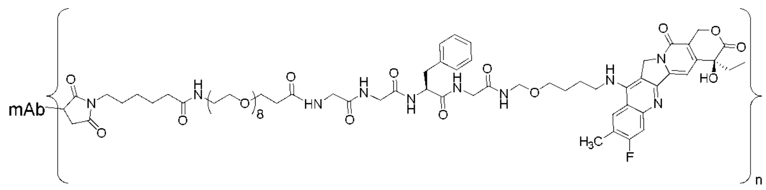 | 7.8 |
| Iso-ADC22 | STI-F1011 | 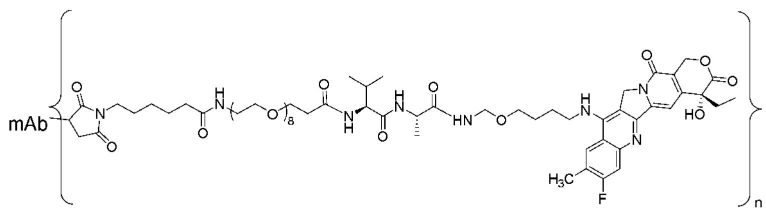 | 7.5 |
| Iso-ADC23 | STI-F1012 | 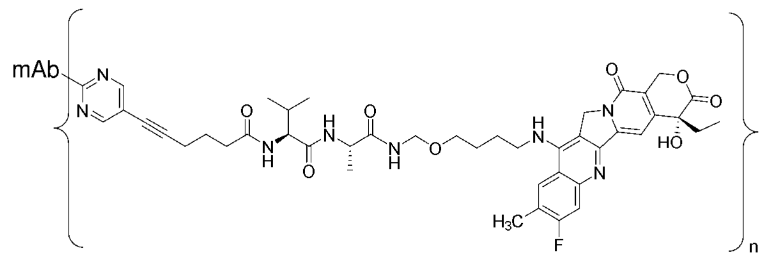 | 7.3 |
| Iso-ADC24 | STI-F1014 | 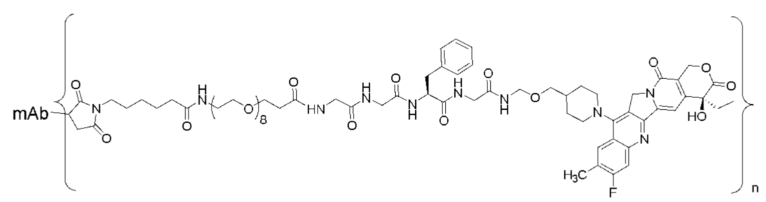 | 8.0 |

(continued)

| ADC Name | Involved Linker-payload Conjugate | ADC Structure | Average DAR (RP-HPLC) |
|---|---|---|---|
| Iso-ADC25 | STI-F1031 | 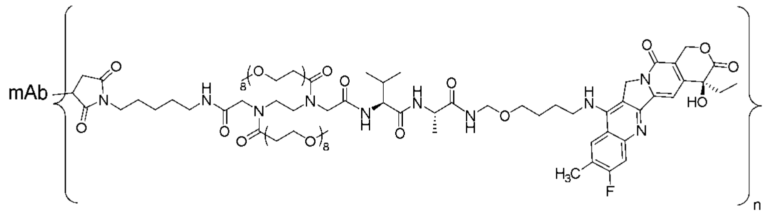 | 7.8 |
| Fa-ADC reference | (MC-GGFC-DXD) | 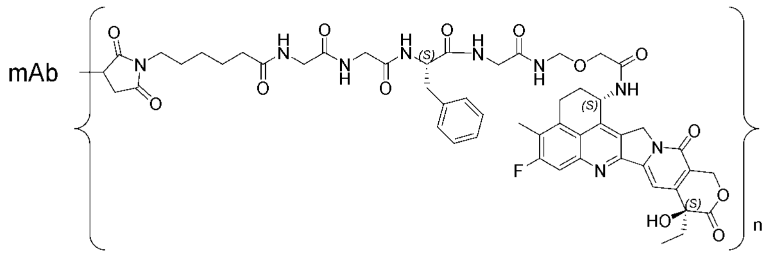 | 7.0 |
| Tra-ADC reference | (MC-GGFC-DXD) | 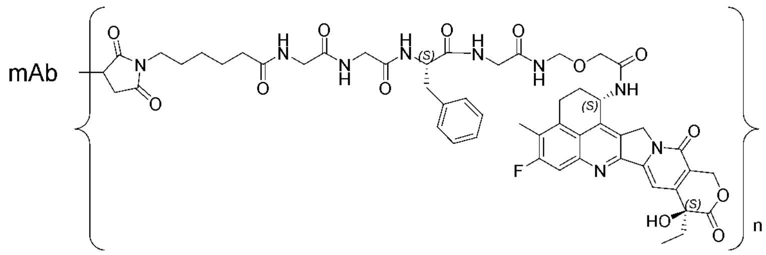 | 8.0 |
| Iso-ADC 参比 | (MC-GGFC-DXD) | 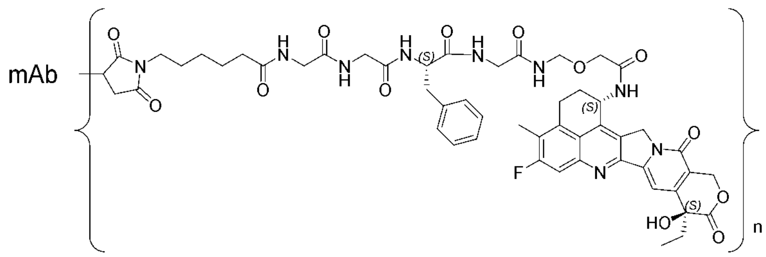 | 7.7 |
| Note:<br>1) In Fa-ADCs, mAb denotes farletuzumab (Fa); in Tra-ADCs, mAb denotes trastuzumab (Tra); in Iso-ADCs, mAb denotes an Anti-HEL Human IgG1 antibody, and n represents an integer from 0 to 8, such as 0, 1, 2, 3, 4, 5, 6, 7, or 8.<br>2) The linker-payload conjugate MC-GGFC-DXD in the ADC reference was purchased from Shanghai Haoyuan Biopharmaceutical Technology Co., Ltd. | | | |

**Biological Section**

**Example A. Cytotoxicity Assay of Camptothecin Derivative Toxins**

Tumor cells (human ovarian cancer cell line SK-OV-3; human colon cancer cell line HCT116; human lung adenocarcinoma cell line A549) in logarithmic growth phase were digested and adjusted to a density of approximately $3\times10^4$ cells/mL. The cells were plated on a white-walled 96-well plate at approximately 100 μL/well. The camptothecin derivative toxins of the Examples of the present application were serially diluted with the corresponding cell culture medium, and then mixed with the cells for incubation at 37°C in a 5% $CO_2$ incubator for 3 days. The growth inhibitory effect of the camptothecin derivative toxins on tumor cells was assessed by the Cell Titer Turbo 2.0 Luminescent Cell Viability Assay (Damas life, RA-GL11). Relative light unit (RLU) values were read from the cell culture plate using a microplate

reader. Experimental data were analyzed using GraphPad Prism 8.0 software to calculate $IC_{50}$ values. The above experiment was repeated using the following compounds as positive controls:

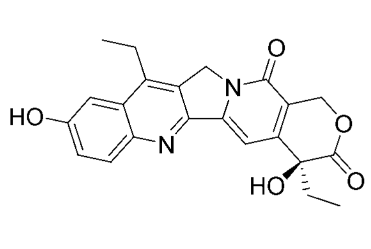

SN-38

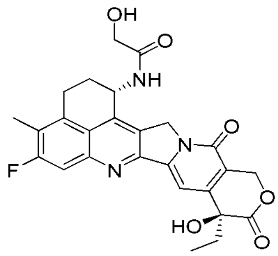

DXD

The results are shown in Table 2. As shown, the compounds of the Examples of the present application demonstrate favorable tumor cell killing activity. In particular, the tumor cell killing activity of the compounds of Example 3 (STI-A), Example 4 (STI-A2), Example 6 (STI-E-05), Example 8 (STI-F-8), Example 10 (STI-F-03C-1), Example 13 (STI-G2-06C), and Example 16 (STI-F) was comparable to that of SN-38 and Dxd. Further, the tumor cell killing activity of the compounds of Example 11 (STI-G1), Example 14 (STI-G2), Example 17 (STI-F6), and Example 19 (STI-F-13) was even significantly superior to that of SN-38 and Dxd.

Table 2 Cytotoxic Activity of Camptothecin Derivatives Against Tumor Cells

| Example No. | Compound Code | $IC_{50}$ (nM) | | |
|---|---|---|---|---|
| | | SK-OV-3 | HCT116 | A549 |
| Control | SN-38 | 30.875 | 60.55 | 522.9 |
| Control | DXD | 41.38 | 49.34 | 413.1 |
| 1 | STI-A-4 | 71.80 | 221.9 | 756.9 |
| 3 | STI-A | 29.33 | 39.89 | 114.8 |
| 4 | STI-A2 | 30.80 | 52.46 | 107.4 |
| 5 | STI-F-01 | 131.2 | 321.6 | 1047 |
| 6 | STI-E-05 | 12.71 | 32.85 | 70.33 |
| 7 | STI-E | 402.8 | 1735 | 1059 |
| 8 | STI-F-8 | 12.38 | 26.57 | 52.84 |
| 9 | STI-F2 | 317.3 | 944.6 | 2368 |
| 10 | STI-F-03C-1 | 19.905 | 38.2 | 1081 |
| 11 | STI-G1 | 3.704 | 10.97 | 9.517 |
| 12 | STI-G03 | 137.3 | 267.9 | 322.4 |
| 13 | STI-G2-06C | 57.77 | 81.26 | 216.2 |
| 14 | STI-G2 | 2.189 | 11.03 | 14.95 |
| 16 | STI-F | 45.45 | NT | NT |
| 17 | STI-F6 | 2.65 | NT | NT |
| 18 | STI-F7 | 285.3 | NT | NT |
| 19 | STI-F13 | 2.37 | NT | NT |
| NT: not tested. | | | | |

**Example A': Cytotoxicity Assay of Camptothecin Derivative Toxins**

The camptothecin derivatives of the Examples of the present application were serially diluted with DMSO and the corresponding cell culture medium, and were added into a white-walled 96-well plate. Tumor cells (human ovarian cancer

cell line SK-OV-3; human colon cancer cell line HCT116; human lung adenocarcinoma cell line A549; human lung adenocarcinoma cell line NCI-H441; human oral epidermoid carcinoma cell line KB) in logarithmic growth phase were digested and adjusted to a density of approximately $3.33\times10^4$ cells/mL. The cells were plated at 90 μL/well on the white-walled 96-well plate that was pre-loaded with the samples, followed by incubation at 37°C in a 5% $CO_2$ incubator for 3 days. The growth inhibitory effect of the small molecule compounds on tumor cells was evaluated by the Cell Titer Turbo 2.0 Luminescent Cell Viability Assay (Damas life, RA-GL11). Relative light unit (RLU) values were read from the cell culture plates using a microplate reader. Experimental data were analyzed with GraphPad Prism 8.0 software to calculate $IC_{50}$ values. The results are shown in the Table below.

Table 2' Cytotoxic Activity of Camptothecin Derivatives Against Tumor Cells

| Example No. | Compound Code | $IC_{50}$ (nM) | | | |
|---|---|---|---|---|---|
| | | SK-OV-3 | HCT116 | A549 | NCI-H441 |
| P1 | STI-F5 | 16.86 | 19.02 | 387.2 | 32.29 |
| P2 | STI-F8 | 12.35 | 11.43 | 277.6 | 24.89 |
| P3 | STI-F14 | NT | 12.36 | 155.8 | 11.09 |
| P4 | STI-F15 | 28.74 | 22.62 | 541.9 | 50.85 |
| P5 | STI-F17 | 20.01 | 17.22 | 377 | 28.73 |
| P6 | STI-F18 | 44.64 | 58.67 | 454.4 | 42.02 |
| P7 | STI-G5 | 10.31 | NT | NT | 2.224 |
| NT: not tested. | | | | | |

**Example B. ADC Cytotoxicity Assay**

Tumor cells in the logarithmic growth phase were digested and adjusted to a cell density of approximately $1\text{-}10\times10^4$ cells/mL. The cells were seeded into white-walled 96-well plates at 50 μL/well and incubated overnight at 37°C in a 5% $CO_2$ incubator. The ADCs of the Example or the reference ADC were serially diluted in the cell culture medium corresponding to those for cell incubation, and then mixed with the cells for incubation at 37°C in a 5% $CO_2$ incubator for 5-7 days. The growth inhibitory effect of the ADCs on tumor cells was assessed by the Cell Titer Turbo 2.0 Luminescent Cell Viability Assay (Damas life, RA-GL11). Relative light unit (RLU) values were read from the cell culture plates using a microplate reader. Experimental data were analyzed using GraphPad Prism 8.0 software to calculate the $IC_{50}$ values. The results are shown in Table 3. Fa-ADC1 demonstrated significantly superior cytotoxic activity against oral epidermoid carcinoma KB tumor cells compared to the positive control Fa-ADC reference.

Table 3. Cytotoxic Activity of ADCs Against Human Oral Epidermoid Carcinoma KB Cells

| ADC Sample | EC50 nM |
|---|---|
| Fa-ADC1 | 0.26 |
| Fa-ADC reference | 1.84 |
| hIgG-DXD | N/A |
| N/A: not applicable | |

Additionally, HER2-endogenously expressing tumor cell lines NCI-N87 (gastric cancer cell line, Nanjing Cobioer, CBP60491) and Capan-1 (pancreatic cancer cell line, Nanjing Cobioer, CBP60543) were used for *in vitro* drug cytotoxicity assays.

NCI-N87 and Capan-1 cells were harvested by digestion with Trypsin (Gibco, 25200072). NCI-N87 cells were resuspended in RPMI-1640 medium containing 10% Fetal Bovine Serum (FBS, Gibco, 10091-148) and adjusted to a cell concentration of $2\times10^4$ cells/mL. The cells were seeded into white-walled clear-bottom 96-well microplates (PerkinElmer, 6005181) at 100 μL/well (2,000 cells/well). Capan-1 cells were resuspended in RPMI-1640 medium containing 20% FBS and adjusted to a concentration of $4\times10^4$ cells/mL, then seeded into white 96-well microplates at 100 μL/well (4,000 cells/well). The peripheral wells of the 96-well microplates were filled with 200 μL of DPBS per well. The 96-well plates were incubated at 37°C in a $CO_2$ incubator for 1 day.

The ADCs of the Example or reference ADCs, serially diluted in RPMI-1640 medium containing 10% FBS, were added. The NCI-N87 cells (the initial concentration 600 nM (6*)) was diluted in a 5-fold gradient, and added at 20 μL/well to the 96-well plates from the previous step; the final volume is 120 μL per well. Capan-1 cells (the initial concentration 3000 nM (6*)), was diluted in a 5-fold gradient, and added at 20 μL/well to the 96-well plates from the previous step; the final volume is 120 μL per well. The 96-well plates were then incubated at 37°C in a $CO_2$ incubator for 4-5 days.

On day 5, the 96-well plates containing Capan-1 cells were removed from the incubator, and Cell Titer Turbo 2.0 reagent (Adamas life, RA-GL11) was taken out from the refrigerator, allowed to equilibrate to RT. Then, 60 μL of the reagent was added to each well of the 96-well plates, and allowed to react at RT for 10 minutes. After 10 minutes, the luminescence signal was measured using a Microplate Reader (BMG, CLARIOstar Plus).

On day 6, the 96-well plates containing NCI-N87 cells were removed from the incubator, and Cell Titer Turbo 2.0 reagent was taken out from the refrigerator, allowed to equilibrate to RT. Then, 60 μL of the reagent was added to each well of the 96-well plates, and allowed to react at RT for 10 minutes. After 10 minutes, the luminescence signal was measured using a Microplate Reader.

As shown in Figs. 1a-1d (cytotoxicity assay of ADC drugs on Capan-1 cells) and Figs. 2a-2d (cytotoxicity assay of ADC drugs on NCI-N87 cells), the ADC drugs of the present invention demonstrated *in vitro* cytotoxic activity against both NCI-N87 and Capan-1 cells.

**Example C. Bystander Effect of ADCs**

Antigen-positive cells (folate receptor alpha-positive KB cells) and antigen-negative cells (293T cells) in the logarithmic growth phase were digested and adjusted to appropriate cell densities. The antigen-positive and antigen-negative cells were added to white-walled, clear-bottom 96-well plates at varying ratios and cultured overnight at 37°C in a 5% $CO_2$ incubator. The following day, ADCs were serially diluted in the corresponding cell culture medium and added to the cell-containing 96-well plates at 100 μL/well, followed by incubation at 37°C in a 5% $CO_2$ incubator for 5-7 days. The growth inhibitory effect of ADCs on tumor cells was assessed using the Cell Titer Turbo 2.0 Luminescent Cell Viability Assay (Adamas Life, RA-GL11-A). Relative light unit (RLU) values were read from the cell culture plates using a microplate reader. The ADCs of the Examples exhibited a potent bystander effect.

CHOK1-EGFP cells (a subline of Chinese hamster ovary cells, target-negative, constructed in-house) and OE19 cells (human esophageal cancer cells, target-positive, Cobioer CBP60495) were harvested by trypsin digestion, resuspended, and counted. OE19 cells were seeded into 96-well plates at densities ranging from 0 to 8,000 cells/well, and CHOK1-EGFP cells were seeded at adensity of 500 cells/well. The ADCs of the Examples or reference ADCs were added to the 96-well plates at a concentration of 1 μg/mL. The 96-well plates were placed in an Incucyte detector and incubated for 7 days, with real-time monitoring of green fluorescent signals. Data were collected after 7 days for graphing and evaluation of the ADC bystander effect, as shown in Figs 3a-3e. The results demonstrated that: when the target-negative CHOK1-EGFP cells were added alone, the ADC drugs showed no cytotoxic effect; as the number of co-cultured target-positive OE19 cells increased, the ADC drugs exhibited enhanced killing of CHOK1-EGFP cells, suggesting that the ADC drugs possess a bystander effect.

**Example D. Pharmacodynamic Evaluation of ADCs in Mouse Tumor Models**

**Example D.1. Efficacy Assessment in a Human Lung Cancer Cell NCI-H441 Immunodeficient Mouse (SCID Beige) Model**

An NCI-H441 human lung adenocarcinoma aminal model was established in immunodeficient mice (SCID Beige, CB17.Cg-PrkdcscidLystbg-J/Crl, Beijing Vital River Laboratory Animal Technology Co., Ltd, Strain Code: 405) to evaluate the antitumor efficacy of the ADCs of the present Examples in this mouse model. NCI-H441 cells were diluted with serum-free medium (RPMI 1640). An appropriate volume of Matrigel (Product No.: 354234, BD) was added and mixed homogeneously with the cell suspension (RPMI 1640 medium : Matrigel = 7:3). The mixture was then inoculated subcutaneously into the right flank of SCID Beige mice at $2.5 \times 10^7$ cells/mL in a volume of 200 μL per mouse. When the average tumor volume reached approximately 150 $mm^3$, the mice were randomized into groups of five each. The test samples or an equal volume of PBS vehicle control were administered via tail vein injection at a dosing volume of 10 mL/kg. The administered dose ranged from 1 to 5 mg/kg. Following administration, the mice were maintained under standard housing conditions. Survival status was monitored, and body weight and tumor volume were recorded twice weekly.

Fa-ADC1 significantly suppressed the growth of NCI-H441 tumors, compared with the vehicle control, IgG1-Dxd, and the Fa-ADC reference.

**Example D.2. Efficacy Evaluation of ADCs in a Human Ovarian Cancer Cell SK-OV-3 Immunodeficient Mice (BALB/c Nude) Model**

An SK-OV-3 human ovarian cancer animal model was established using immunodeficient mice (BALB/c Nude, BALB/cNj-Foxnlnu/Gpt, GemPharmatech Co., Ltd., Jiangsu, Strain Code: D000521) to evaluate the *in vivo* efficacy of the ADCs of the present Example. SK-OV-3 cells were diluted in serum-free medium (RPMI 1640). An appropriate volume of Matrigel (Product No.: 354234, BD; RPMI 1640 medium to Matrigel ratio = 7:3) was added and mixed homogeneously with the cell suspension. The mixture was then inoculated subcutaneously into the right flank of the immunodeficient mice at $2.5\times10^7$ cells/mL in a volume of 200 μL per mouse. When the average tumor volume reached approximately 150 $mm^3$, the mice were randomized into groups of five each. The test samples or an equal volume of PBS vehicle control were administered via tail vein injection at a dosing volume of 10 mL/kg. Two doses were administered, with a 9-day interval between the first and second doses (at 1 mg/kg and 5 mg/kg, respectively). Following administration, the mice were maintained under standard housing conditions. Survival status was monitored, and body weight and tumor volume were recorded twice weekly. The results are presented in Figs. 4-5. As illustrated in Fig. 4, Fa-ADC1 significantly suppressed the growth of SK-OV-3 tumors compared with the vehicle control, IgG1-Dxd (Iso-ADC reference), and the Fa-ADC reference. Throughout the observation period, no body weight loss was observed in the tumor-bearing mice, suggesting good tolerability at the administered dose levels (see Fig. 5).

In addition, the antitumor efficacy of various ADC molecules conjugating to anti-HER2 antibody trastuzumab was evaluated in the following tumor cell models: NCI-N87 (human gastric cancer cells), Capan-1 (human pancreatic cancer cells), JIMT-1 (human breast cancer cells), Calu-3 (human lung adenocarcinoma cells), and OE19 (human esophageal cancer cells).

**Example D.3. Efficacy Evaluation of HER2-ADCs in JIMT-1, Capan-1, and NCI-N87 Tumor Models Established in BALB/c Nude Mice**

JIMT-1, Capan-1, and NCI-N87 tumor models were established in immunodeficient BALB/c Nude mice (GemPharmatech Co., Ltd., Jiangsu) to evaluate the antitumor efficacy of the moleculars conjugating to anti-HER2 antibodies. NCI-N87, JIMT-1, and Capan-1 cells were separately diluted in serum-free (RPMI-1640) medium. An appropriate volume of Matrigel (Product No.: 354234, BD; RPMI-1640 medium to Matrigel ratio = 1:1) was added and mixed homogeneously with the cell suspension. The mixture was then inoculated subcutaneously into the right flank of BALB/c Nude mice at $5\times10^6$ cells/mL in a volume of 200 μL per mouse. When the average tumor volume reached approximately 150 $mm^3$, the mice were grouped and administered according to the dosing regimen. The drug was administered as a solution of the test ADCs in 10 mM histidine-HCl buffer (pH 6.0) as vehicle (0.3 mg/mL). Mice received a single intravenous (i.v.) injection via the tail vein at a dosing volume of 10 mL/kg. Following administration, the survival status of the mice was monitored, and body weight (twice weekly) and tumor volume (twice weekly) data were collected throughout the study period.

Notes:

1. Tumor Volume Measurement in Mice: Tumor length and width were measured using a digital caliper (Model 500-151-30, Mitutoyo), and tumor volumn was calculated according to the formula: TV = $1/2 \times L \times W^2$ $mm^3$ (TV: tumor volume; L: tumor length; W: tumor width).
2. Tumor Growth Inhibition TGI % = [1-T/C] $\times$100. T/C% =$(T_i-T_0) / (C_i-C_0)\times100\%$; $T_i$ is the mean tumor volume of the treatment group at a given measurement time point; $T_0$ is the mean tumor volume of the treatment group at the time of grouping; $C_i$ is the mean tumor volume of the control group at a given measurement time point; and $C_0$ is the mean tumor volume of the control group at the time of grouping.
3. Body Weight Change Formula: RCBW %= $(BW_i-BW_0)BW_0\times100\%$, where $BW_i$ is the body weight of a mouse at a given time point, and $BW_0$ is the body weight of the mouse on the day of grouping.

**Table 4.** Grouping and Dosing Regimen for JIMT-1, Capan-1, and NCI-N87 Tumor Mice Models

| Model | Group | Treatment | Dose | N | Dosing Volume | Route of dosing | Dosing Frequency | Study Duration |
|---|---|---|---|---|---|---|---|---|
| JIMT-1 | G1 | His-HCl solution | NA | 3 | 10 ml/kg | 1.V. | Single | 28 days |
| | G2 | Tra-ADC7 | 3mg/kg | 3 | 10 ml/kg | 1.V. | Single | 28 days |
| | G3 | Tra-ADC12 | 3mg/kg | 3 | 10 ml/kg | 1.V. | Single | 28 days |
| | G4 | Tra-ADC13 | 3mg/kg | 3 | 10 ml/kg | 1.V. | Single | 28 days |
| | G5 | Tra-ADC 16 | 3mg/kg | 3 | 10 ml/kg | i.v. | Single | 28 days |
| | G6 | Tra-ADC Reference | 3mg/kg | 3 | 10 ml/kg | 1.V. | Single | 28 days |
| Capan-1 | G7 | His-HCl solution | NA | 3 | 10 ml/kg | 1.V. | Single | 31 days |
| | G8 | Tra-ADC7 | 3mg/kg | 3 | 10 ml/kg | i.v. | Single | 31 days |
| | G9 | Tra-ADC12 | 3mg/kg | 3 | 10 ml/kg | i.v. | Single | 31 days |
| | G10 | Tra-ADC13 | 3mg/kg | 3 | 10 ml/kg | i.v. | Single | 31 days |
| | G11 | Tra-ADC16 | 3mg/kg | 3 | 10 ml/kg | 1.V. | Single | 31 days |
| | G12 | Tra-ADC参比 | 3mg/kg | 3 | 10 ml/kg | i.v. | Single | 31 days |
| NCI-N87 | G13 | His-HCl solution | NA | 3 | 10 ml/kg | 1.V. | Single | 29 days |
| | G14 | Tra-ADC7 | 3mg/kg | 3 | 10 ml/kg | 1.V. | Single | 29 days |
| | G15 | Tra-ADC12 | 3mg/kg | 3 | 10 ml/kg | i.v. | Single | 29 days |
| | G16 | Tra-ADC13 | 3mg/kg | 3 | 10 ml/kg | i.v. | Single | 29 days |
| | G17 | Tra-ADC16 | 3mg/kg | 3 | 10 ml/kg | 1.V. | Single | 29 days |
| | G18 | Tra-ADC Reference | 3mg/kg | 3 | 10 ml/kg | 1.V. | Single | 29 days |

The results are shown in Figs. 6a/6b to 8a/8b. As shown in Figs. 6a, 7a, and 8a, the ADC drugs of the present invention demonstrated significant antitumor activity in all three models compared to the vehicle control, with tumor growth being markedly suppressed. As shown in Figs. 6b, 7b, and 8b, the tumor-bearing mice exhibited a consistent increase in body weight throughout the observation period following administration, suggesting favorable tolerability to the test products.

In the JIMT-1 model, at a dose of 3 mg/kg, the tested ADC molecules exhibited the following TGI% (Tumor Growth Inhibition) (on Day 28 post-dosing): Tra-ADC7, 71.3%; Tra-ADC12, 55.1%; Tra-ADC13, 25.8%; Tra-ADC16, 49.7%; Tra-ADC reference, 106.1% (Fig. 6a).

In the Capan-1 model, at a dose of 3 mg/kg, the tested ADC molecules exhibited the following TGI% (on Day 31 post-dosing): Tra-ADC7, 106.2%; Tra-ADC12, 72.4%; Tra-ADC13, 41.1%; Tra-ADC16, 77%; Tra-ADC reference, 70.2% (Fig. 7a).

In the NCI-N87 model, at a dose of 3 mg/kg, the tested ADC molecules exhibited the following TGI% (on Day 29 post-dosing): Tra-ADC7, 111.7%; Tra-ADC12, 96.6%; Tra-ADC13, 108.2%; Tra-ADC16, 106.8%; Tra-ADC reference, 96.7% (Fig. 8a).

**Example D.4. Efficacy Evaluation of HER2-ADCs in JIMT-1, Capan-1, and NCI-N87 Tumor BALB/c Nude Mice Models**

This study was conducted following the procedures described in Example D.3, except that the dosing regimen was as specified in Table 5.

**Table 5.** Grouping and Dosing Regimen in JIMT-1, Capan-1, and NCI-N87 Tumor Mice Models

| Model | Group | Treatment | Dose | N | Dosing Volume | Route of Dosing | Dosing Frequency | Study Duration |
|---|---|---|---|---|---|---|---|---|
| JIMT-1 | G1 | His-HCl solution | NA | 3 | 10 ml/kg | i.v. | Single | 28 days |
| | G3 | Iso-ADC reference | 3mg/kg | 3 | 10 ml/kg | i.v. | Single | 28 days |
| | G5 | Tra-ADC9 | 3mg/kg | 3 | 10 ml/kg | i.v. | Single | 28 days |
| | G6 | Tra-ADC10 | 3mg/kg | 3 | 10 ml/kg | i.v. | Single | 28 days |
| | G7 | Tra-ADC11 | 3mg/kg | 3 | 10 ml/kg | i.v. | Single | 28 days |
| | G8 | Tra-ADC reference | 3mg/kg | 3 | 10 ml/kg | i.v. | Single | 28 days |
| Capan-1 | G10 | His-HCl solution | NA | 3 | 10 ml/kg | i.v. | Single | 42 days |
| | G12 | Iso-ADC reference | 3mg/kg | 3 | 10 ml/kg | i.v. | Single | 42 days |
| | G14 | Tra-ADC9 | 3mg/kg | 3 | 10 ml/kg | i.v. | Single | 42 days |
| | G15 | Tra-ADC10 | 3mg/kg | 3 | 10 ml/kg | i.v. | Single | 42 days |
| | G16 | Tra-ADC11 | 3mg/kg | 3 | 10 ml/kg | i.v. | Single | 42 days |
| | G17 | Tra-ADC reference | 3mg/kg | 3 | 10 ml/kg | i.v. | Single | 42 days |
| NCI-N87 | G19 | His-HCl solution | NA | 3 | 10 ml/kg | i.v. | Single | 48 days |
| | G21 | Iso-ADC reference | 3mg/kg | 3 | 10 ml/kg | i.v. | Single | 48 days |
| | G23 | Tra-ADC9 | 3mg/kg | 3 | 10 ml/kg | i.v. | Single | 48 days |
| | G24 | Tra-ADC10 | 3mg/kg | 3 | 10 ml/kg | i.v. | Single | 48 days |
| | G25 | Tra-ADC11 | 3mg/kg | 3 | 10 ml/kg | i.v. | Single | 48 days |
| | G26 | Tra-ADC reference | 3mg/kg | 3 | 10 ml/kg | i.v. | Single | 48 days |

The results are presented in Figures 9a/9b to 11a/11b. As shown in Figures 9a, 10a, and 11a, the Tra-ADCs of the present invention demonstrated significant antitumor activity in all three models compared to the vehicle control and the Iso-ADC reference, with tumor growth being markedly suppressed. As shown in Figures 9b, 10b, and 11b, the tumor-bearing mice exhibited an overall increasing trend in body weight during the experimental observation period, suggesting good tolerability to the ADC conjugates at the administered dose levels.

In the JIMT-1 model, at a dose of 3 mg/kg, the tested ADC molecules showed the following TGI% (on Day 28 post-dosing): Tra-ADC9, 65.1%; Tra-ADC10, 50.6%; Tra-ADC11, 57.5%; Tra-ADC reference, 44.4%; Iso-ADC reference, 29.5% (Figure 9a).

In the Capan-1 model, at a dose of 3 mg/kg, the tested ADC molecules showed the following TGI% (on Day 35 post-dosing): Tra-ADC9, 80.2%; Tra-ADC10, 103.9%; Tra-ADC11, 106.7%; Tra-ADC reference, 96.9%; Iso-ADC reference, 41.4% (Figure 10a).

In the NCI-N87 model, at a dose of 3 mg/kg, the tested ADC molecules showed the following TGI% (on Day 35 post-dosing): Tra-ADC9, 111.7%; Tra-ADC10, 111.4%; Tra-ADC11, 111.7%; Tra-ADC reference, 101.4%; Iso-ADC reference, 16.2% (Figure 11a).

**Example D.5. Efficacy Evaluation of HER2-ADCs in BALB/c Nude Mice Calu-3 and OE-19 Tumor Models**

Calu-3 (human lung adenocarcinoma cells) and OE-19 (human esophageal cancer cells) tumor models were established in immunodeficient BALB/c nude mice (GemPharmatech Co., Ltd., Jiangsu) to evaluate the antitumor efficacy of the moleculars conjugating to anti-HER2 antibodies. Calu-3 cells were diluted in serum-free (RPMI-1640) medium. An appropriate volume of Matrigel (Product No.: 354234, BD; RPMI-1640 medium to Matrigel ratio = 5:5) was added and mixed homogeneously with the cell suspension. The mixture was then inoculated subcutaneously into the right flank of BALB/c Nude mice at $5\times10^6$ cells/mL in a volume of 200 $\mu$L per mouse. When the average tumor volume reached approximately 150 $mm^3$, the mice were grouped and administered according to the dosing regimen. The drug was administered as a solution of the test ADCs in 10 mM histidine-HCl buffer (pH 6.0) as vehicle (0.3 mg/mL). Mice received a single intravenous (i.v.) injection via the tail vein at a dosing volume of 10 mL/kg. Following administration, body weight and tumor volume data were collected twice weekly throughout the study period, and the body weight change was calculated until Day 42 post-dosing.

OE-19 cells were diluted in serum-free (RPMI-1640) medium. An appropriate volume of Matrigel (Product No.: 354234, BD; RPMI-1640 medium to Matrigel ratio = 5:5) was added and mixed homogeneously with the cell suspension. The mixture was then inoculated subcutaneously into the right flank of BALB/c Nude mice at $5\times10^6$ cells/mL in a volume of 200 μL per mouse. When the average tumor volume reached approximately 150 $mm^3$, the mice were grouped and administered according to the dosing regimen. Mice received a single intravenous (i.v.) injection via the tail vein at a standard dosing volume of 10 mL/kg. Tumor volume and body weight data of the mice were collected twice weekly, and the body weight change was calculated until Day 44 post-dosing.

The methods for measuring tumor volume, calculating the tumor growth inhibition rate, and calculating the body weight change rate of mice were as described in Example D.3.

**Table 6.** Grouping and Dosing Regimen for Calu-3 and OE-19 Tumor Mice Models

| Model | Group | Treatment | Dose | N | Dosing Volume | Route of Dosing | Dosing Frequency | Study Duration |
|---|---|---|---|---|---|---|---|---|
| Calu-3 | G1 | His-HCl solution | NA | 3 | 10 ml/kg | i.v. | Single | 35 days |
| | G2 | Tra-ADC10 | 1mg/kg | 3 | 10 ml/kg | 1.V. | Single | 35 days |
| | G3 | Tra-ADC7 | 1mg/kg | 3 | 10 ml/kg | i.v. | Single | 35 days |
| | G4 | Tra-ADC reference | 1mg/kg | 3 | 10 ml/kg | i.v. | Single | 35 days |
| | G5 | Tra-ADC9 | 1mg/kg | 3 | 10 ml/kg | 1.V. | Single | 35 days |
| OE-19 | G7 | His-HCl solution | NA | 3 | 10 ml/kg | i.v. | Single | 26 days |
| | G8 | Tra-ADC10 | 1mg/kg | 3 | 10 ml/kg | i.v. | Single | 26 days |
| | G10 | Tra-ADC reference | 1mg/kg | 3 | 10 ml/kg | 1.V. | Single | 26 days |
| | G11 | Tra-ADC9 | 1mg/kg | 3 | 10 ml/kg | i.v. | Single | 26 days |

The results are shown in Figures 12a/12b and 13a/13b. As shown in Figures 12a and 13a, the Tra-ADCs of the present invention exhibited potent antitumor activity in both models compared to the vehicle control, with significant inhibition of tumor growth. As shown in Figures 12b and 13b, the tumor-bearing mice showed an overall increasing trend in body weight during the experimental observation period, suggesting good tolerability to the ADC conjugates at the administered doses.

In the Calu-3 model, at a dose of 1 mg/kg, the tested ADC molecules demonstrated the following TGI% (on Day 35 post-dosing): Tra-ADC10, 103.4%; Tra-ADC7, 76.4%; Tra-ADC9, 91.1%; Tra-ADC reference, 56.9% (Figure 12a).

In the OE-19 model, the tested ADC molecules demonstrated the following TGI% (on Day 26 post-dosing): Tra-ADC10, 97.9%; Tra-ADC9, 94.8%; Tra-ADC reference, 84.9% (Figure 13a).

**Example D.6. Efficacy Evaluation of HER2-ADCs in a Capan-1 Tumor BALB/c Nude Mice Model**

Capan-1 and NCI-N87 tumor models were established in immunodeficient BALB/c Nude mice (GemPharmatech Co., Ltd., Jiangsu) to evaluate the antitumor efficacy of various moleculars conjugating to anti-HER2 antibodies. Capan-1 cells were diluted in serum-free (RPMI-1640) medium. An appropriate volume of Matrigel (Product No.: 354234, BD; RPMI-1640 medium to Matrigel ratio = 5:5) was added and mixed homogeneously with the cell suspension. The mixture was then inoculated subcutaneously into the right flank of BALB/c Nude mice at $5\times10^6$ cells/mL in a volume of 200 μL per mouse. When the average tumor volume reached approximately 150 $mm^3$, the mice were grouped and administered according to the dosing regimen. The drug was administered as a solution of the test ADCs in 10 mM histidine-HCl buffer (pH 6.0) as vehicle (0.3 mg/mL). Mice received a single intravenous (i.v.) injection via the tail vein at a dosing volume of 10 mL/kg. The survival status of the mice was monitored until Day 55 post-dosing, during which body weight and tumor volume data were collected twice weekly. The methods for measuring tumor volume, calculating the tumor growth inhibition rate, and calculating the body weight change rate were as described in Example D.3.

**Table 7.** Grouping and Dosing Regimen for the Capan-1 Tumor Mice Model

| Model | Group | Treatment | Dose | N | Dosing Volume | Route of Dosing | Dosing Frequency | Study Duration |
|---|---|---|---|---|---|---|---|---|
| | G1 | His-HCl solution | NA | 3 | 10 ml/kg | i.v. | Single | 29 days |
| | G3 | Tra-ADC9 | 3mg/kg | 4 | 10 ml/kg | 1.V. | Single | 29 days |

(continued)

| Model | Group | Treatment | Dose | N | Dosing Volume | Route of Dosing | Dosing Frequency | Study Duration |
|---|---|---|---|---|---|---|---|---|
| Capan-1 | G5 | Tra-ADC10 | 3mg/kg | 4 | 10 ml/kg | 1.V. | Single | 29 days |
| | G6 | Iso-ADC reference | 3mg/kg | 4 | 10 ml/kg | i.v. | Single | 29 days |
| | G7 | Tra-ADC reference | 3mg/kg | 4 | 10 ml/kg | 1.V. | Single | 29 days |
| | G8 | Tra-ADC17 | 3mg/kg | 3 | 10 ml/kg | 1.V. | Single | 29 days |
| | G9 | Tra-ADC18 | 3mg/kg | 3 | 10 ml/kg | i.v. | Single | 29 days |
| | G10 | Tra-ADC20 | 3mg/kg | 3 | 10 ml/kg | 1.V. | Single | 29 days |
| | G11 | Tra-ADC19 | 3mg/kg | 3 | 10 ml/kg | 1.V. | Single | 29 days |
| | G12 | Tra-ADC14 | 3mg/kg | 3 | 10 ml/kg | 1.V. | Single | 29 days |
| | G13 | Tra-ADC15 | 3mg/kg | 3 | 10 ml/kg | 1.V. | Single | 29 days |

The results are shown in Figures 14a/14b. As shown in Figure 14a, the Tra-ADCs of the present invention demonstrated significant antitumor activity compared to the vehicle control and Iso-ADCs, with marked suppression of tumor growth. As shown in Figure 14b, the tumor-bearing mice exhibited an overall increasing trend in body weight during the experimental observation period, suggesting favorable tolerability to the ADC conjugates at the administered dose levels.

In the Capan-1 model, at a dose of 3 mg/kg, the tested ADC molecules exhibited the following TGI% (on Day 29 post-dosing): Tra-ADC9, 104.1%; Tra-ADC17, 111.8%; Tra-ADC20, 102%; Tra-ADC19, 102.7%; Tra-ADC14, 110.4%; Tra-ADC15, 103.5%; Tra-ADC reference, 90.0% (Figure 14a).

In summary, the ADCs of the present invention demonstrated favorable tolerability in tumor mice models and exhibited potent, and even superior, antitumor efficacy in multiple cancer cell models.

**Example E. Tolerability of ADCs in Mice**

The tolerability of the ADCs of the present application was evaluated by daily body weight measurements and clinical observations following intravenous tail vein injection at doses ranging from 10 to 300 mg/kg. In this experiment, all mice in the administered groups exhibited no behavioral abnormalities or body weight loss, suggesting favorable tolerability to the ADCs at the tested dose levels.

All references cited herein are incorporated by reference in their entireties, as if each individual reference were specifically and individually present herein. It should be understood that, after reading the foregoing disclosure, those skilled in the art may make various changes or modifications to the present invention, and such equivalent forms shall also fall within the scope defined by the appended claims of the present application.

**Claims**

**1.** An antibody-drug conjugate of formula (I):

$$Ab\left[L_1-L_2-L_3-L_4-D\right]_n \qquad (I)$$

or a pharmaceutically acceptable salt or ester, solvate, tautomer, stereoisomer, prodrug, or isotopically labeled form thereof,
wherein:

Ab is an antibody or antigen-binding fragment thereof;
$L_1$ is each independently a linker unit;
$L_2$ is each independently a linking unit selected from the group consisting of:

$-(CR_mR_n)_t-(L_M)_{0-1}-(CH_2CH_2O)_s-(CR_mR_n)_t-CO-$,

$-(CR_mR_n)_t-L_M-(CR_mR_n)_t-(L_M)_{0-1}-L_N-L_M-(CH_2O)_s-(CR_mR_n)_t-CO-$,

$-(CR_mR_n)_t-L_M-(CR_mR_n)_t-N(R_p)-(CR_mR_n)_t-N(R_p)-(CR_mR_n)_t-CO-$,

and
$-(CR_mR_n)_t-L_M-CH(R_p)-CO-$, wherein the -CO- terminus of $L_2$ is connected to $L_3$, and the other terminus of $L_2$ is connected to $L_1$, and
wherein,

$R_m$ and $R_n$ are each independently H or $C_{1-6}$ alkyl,
$L_M$ is each independently -NH-CO- or -CO-NH-,
$L_N$ is each independently $-(CH_2CH_2O)_m-(CH_2)_t-$, $-(CH_2)_t-(OCH_2CH_2)_m-$, -(3- to 8-membered heteroarylene)$-(CH_2)_t-(OCH_2CH_2)_m$, or $-(OCH_2CH_2)_m-(CH_2)_t-$(3- to 8-membered heteroarylene)-,
$R_p$ is each independently $-CO-(CH_2CH_2O)_m-CH_3$ or $-(CH_2)_t-L_M-(CH_2CH_2O)_m-CH_3$, and
s and t are each independently 0, 1, 2, 3, 4, 5, 6, 7, or 8, and
m is each independently 2, 3, 4, 5, 6, 7, or 8;

$L_3$ is each independently an amino acid residue, a short peptide chain consisting of 2-10 amino acid residues, or $-NH-(CH_2)_p-CO-$;
$L_4$ is each independently a bond or a spacer unit;
D is each independently a small molecule drug moiety derived from a compound of formula (II);

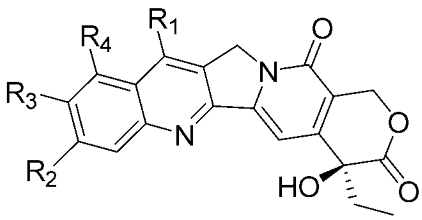

(II)

wherein,

$R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from the group consisting of halogen, CN, OH, SH and amino; $-(CH_2)_p-(C_{3-8}$ cycloalkyl) or $-(CH_2)_p-(C_{3-8}$ heterocycloalkyl), wherein the cycloalkyl and heterocycloalkyl are each optionally substituted with one or more substituents independently selected from the group consisting of halogen, CN, OH, SH, amino, $C_{1-6}$ alkyl, $C_{1-6}$ haloalkyl, $C_{1-6}$ hydroxyalkyl, $C_{1-6}$ cyanoalkyl and $C_{1-6}$ aminoalkyl; $-(CH_2)_p-(C_{1-6}$ alkoxy); $-(CH_2)_p-(C_{1-6}$ alkylthio); $-(CH_2)_p-NR_aR_b$; $-(CH_2)_p-CH=NR_a$; $-NH-CO-R_a$; $-NH-CO-(CH_2)_p-CH(OH)-R_a$; $-NH-(CH_2)_p-(CH=CH)-R_a$; or -COOH;
$R_2$ and $R_3$ are each independently halogen, cyano, $NO_2$, $C_{1-6}$ alkyl, $C_{1-6}$ haloalkyl, $C_{1-6}$ hydroxyalkyl, $C_{1-6}$ alkoxy, -NH-CO-($C_{1-6}$ hydroxyalkyl), or $NR_aR_b$; or $R_2$ and $R_3$, together with the atoms to which they are attached, form a 4- to 8-membered heterocyclic group;
$R_4$ is H or $C_{3-8}$ cycloalkyl, preferably H;
$R_a$ and $R_b$ are each independently H, $C_{1-6}$ alkyl, -CO-($C_{1-6}$ alkyl), -SO-($C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino, and wherein the cycloalkyl is further optionally substituted with one or more substituents independently selected from the group consisting of $C_{1-6}$ alkyl, $C_{1-6}$ haloalkyl, $C_{1-6}$ hydroxyalkyl, $C_{1-6}$ cyanoalkyl and $C_{1-6}$ aminoalkyl; and
p and q are each independently 0, 1, 2, 3, 4, 5, or 6;
wherein D is linked to $L_4$ via $R_1$ or $R_3$, or via the oxygen from the hydroxy shown in formula (II); and
n is an integer from 0 to 10, preferably an integer from 0 to 8.

**2.** The antibody-drug conjugate of formula (I) according to claim 1:

$$Ab\left[L_1-L_2-L_3-L_4-D\right]_n \quad (I)$$

or a pharmaceutically acceptable salt or ester, solvate, tautomer, stereoisomer, prodrug, or isotopically labeled form thereof,
wherein:

Ab is an antibody or antigen-binding fragment thereof;
$L_1$ is each independently a linker unit;
$L_2$ is each independently a linking unit of the formula $-(CH_2CH_2O)_x-(CR_mR_n)_y-CO-$, wherein the -CO-terminus is connected to $L_3$ and the other terminus is connected to $L_1$, and wherein $R_m$ and $R_n$ are each independently H or $C_{1-6}$ alkyl, x is an integer from 0 to 5, y is an integer from 1 to 5; and the sum of x and y is $\leq 6$;
$L_3$ is each independently an amino acid residue, a short peptide chain consisting of 2-10 amino acid residues, or $-NH-(CH_2)_p-CO-$;
$L_4$ is each independently a bond or a spacer unit;
D is each independently a small molecule drug moiety derived from a compound of formula (II);

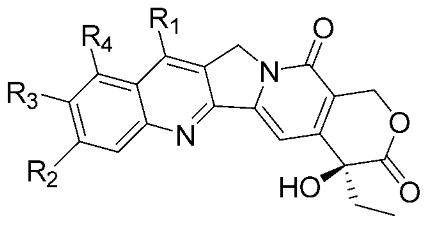

(II)

wherein,

$R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from the group consisting of halogen, CN, OH, SH and amino; $-(CH_2)_p-(C_{3-8}$ cycloalkyl) or $-(CH_2)_p-(C_{3-8}$ heterocycloalkyl), wherein the cycloalkyl and heterocycloalkyl are each optionally substituted with one or more substituents independently selected from the group consisting of halogen, CN, OH, SH, amino, $C_{1-6}$ alkyl, $C_{1-6}$ haloalkyl, $C_{1-6}$ hydroxyalkyl, $C_{1-6}$ cyanoalkyl and $C_{1-6}$ aminoalkyl; $-(CH_2)_p-(C_{1-6}$ alkoxy); $-(CH_2)_p-(C_{1-6}$ alkylthio); $-(CH_2)_p-NR_aR_b$; $-(CH_2)_p-CH=NR_a$; $-NH-CO-R_a$; $-NH-CO-(CH_2)_p-CH(OH)-R_a$; $-NH-(CH_2)_p-(CH=CH)-R_a$; or -COOH;
$R_2$ and $R_3$ are each independently halogen, cyano, $NO_2$, $C_{1-6}$ alkyl, $C_{1-6}$ haloalkyl, $C_{1-6}$ hydroxyalkyl, $C_{1-6}$ alkoxy, $-NH-CO-(C_{1-6}$ hydroxyalkyl), or $NR_aR_b$; or $R_2$ and $R_3$, together with the atoms to which they are attached, form a 4- to 8-membered heterocyclic group;
$R_4$ is H or $C_{3-8}$ cycloalkyl, preferably H;
$R_a$ and $R_b$ are each independently H, $C_{1-6}$ alkyl, $-CO-(C_{1-6}$ alkyl), $-SO-(C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino, and wherein the cycloalkyl is further optionally substituted with one or more substituents independently selected from the group consisting of $C_{1-6}$ alkyl, $C_{1-6}$ haloalkyl, $C_{1-6}$ hydroxyalkyl, $C_{1-6}$ cyanoalkyl and $C_{1-6}$ aminoalkyl; and
p and q are each independently 0, 1, 2, 3, 4, 5, or 6;
wherein D is linked to $L_4$ via $R_1$ or $R_3$, or via the oxygen from the hydroxyl shown in formula (II); and
n is an integer from 0 to 10, preferably an integer from 0 to 8.

**3.** The antibody-drug conjugate according to claim 1 or 2, or a pharmaceutically acceptable salt or ester, solvate, tautomer, stereoisomer, prodrug, or isotopically labeled form thereof, wherein

$R_1$ is: H; halogen; CN; OH; SH; $C_{1-6}$ alkyl; $C_{1-6}$ haloalkyl; $C_{1-6}$ hydroxyalkyl; $-(CH_2)_p-(C_{3-8}$ cycloalkyl) or

$-(CH_2)_p-(C_{3-8}$ heterocycloalkyl), wherein the cycloalkyl and the heterocycloalkyl are each optionally substituted with one or more substituents independently selected from the group consisting of OH, SH, $C_{1-6}$ alkyl, and $C_{1-6}$ hydroxyalkyl; $-(CH_2)_p-(C_{1-6}$ alkoxy); $-(CH_2)_p-(C_{1-6}$ alkylthio); $-(CH_2)_p-NR_aR_b$; $-(CH_2)_p-CH=NR_a$; $-NH-CO-R_a$; $-NH-CO-(CH_2)_p-CH(OH)-R_a$; $-NH-(CH_2)_p-(CH=CH)-R_a$; or -COOH; wherein $R_a$ and $R_b$ are each independently H, $C_{1-6}$ alkyl, $C_{1-6}$ hydroxyalkyl, $C_{1-6}$ aminoalkyl, $-SO_2-(C_{1-6}$ alkyl), or $-(CH_2)_q-(C_{3-8}$ cycloalkyl optionally substituted with one or more substituents independently selected from the group consisting of hydroxy and $C_{1-6}$ hydroxyalkyl);

preferably, $R_1$ is H; $C_{1-6}$ alkyl; $C_{1-6}$ haloalkyl; $C_{1-6}$ hydroxyalkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-(C_{1-6}$ alkoxy); $(CH_2)_p-(C_{1-6}$ alkylthio); $-(CH_2)_p-NH_2$; $-(CH_2)_p-NH(C_{1-6}$ alkyl); $-(CH_2)_p-NH(C_{1-6}$ hydroxyalkyl); $-(CH_2)_p-NH(C_{1-6}$ aminoalkyl); $-(CH_2)_p-NH-(CH_2)_q-(C_{3-8}$ cycloalkyl), wherein the cycloalkyl is optionally substituted with one or more substituents selected from $C_{1-6}$ hydroxyalkyl; $-(CH_2)_p-N(C_{1-6}$ alkyl)($-SO_2-C_{1-6}$ alkyl); $-CH=N(C_{1-6}$ alkyl); $-NH-CO-(C_{1-6}$ hydroxyalkyl); $-NH-CO-(C_{3-8}$ hydroxycycloalkyl); $-NH-CO-(CH_2)_p-CH(OH)-(C_{1-6}$ alkyl); $-NH-CO-(CH_2)_p-CH(OH)-(C_{3-8}$ cycloalkyl); $-NH-(CH_2)_p-(CH=CH)-(C_{1-6}$ hydroxyalkyl); or -COOH;

or

$R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from the group consisting of halogen, CN, OH, SH and amino; $-(CH_2)_p-(C_{3-8}$ cycloalkyl), wherein the cycloalkyl is optionally substituted with one or more substituents independently selected from the group consisting of halogen, CN, OH, SH and amino; $-(CH_2)_p-(C_{1-6}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH; wherein $R_a$ and $R_b$ are each independently H, $C_{1-6}$ alkyl, $-SO-(C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino;

preferably, $R_1$ is: H; halogen; CN; OH; SH; $C_{1-6}$ alkyl; $C_{1-6}$ haloalkyl; $C_{1-6}$ hydroxyalkyl; $-(CH_2)_p-(C_{3-8}$ cycloalkyl); $-(CH_2)_p-(C_{3-8}$ hydroxycycloalkyl); $-(CH_2)_p-(C_{3-8}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH;

more preferably, $R_1$ is H; $C_{1-6}$ alkyl; $C_{1-6}$ haloalkyl; $C_{1-6}$ hydroxyalkyl; $-(CH_2)_p-(C_{1-6}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH; preferably wherein $R_a$ and $R_b$ are each independently H, $C_{1-6}$ alkyl, $C_{1-6}$ hydroxyalkyl, $C_{1-6}$ aminoalkyl, $-SO_2-(C_{1-6}$ alkyl), $-(CH_2)_q-(C_{3-8}$ cycloalkyl), or $-(CH_2)_q-(C_{3-8}$ hydroxycycloalkyl);

further more preferably, $R_1$ is H; $C_{1-6}$ alkyl; $C_{1-6}$ haloalkyl; $C_{1-6}$ hydroxyalkyl; $-(CH_2)_p-(C_{1-6}$ alkoxy); $-(CH_2)_p-(C_{1-6}$ alkylthio); $-(CH_2)_p-NH_2$; $-(CH_2)_p-NH(C_{1-6}$ alkyl); $-(CH_2)_p-NH(C_{1-6}$ hydroxyalkyl); $-(CH_2)_p-NH(C_{1-6}$ aminoalkyl); $-(CH_2)_p-NH-(CH_2)_q-(C_{3-8}$ cycloalkyl); $-(CH_2)_p-N(C_{1-6}$ alkyl)($-SO_2-C_{1-6}$ alkyl); $-CH=N(C_{1-6}$ alkyl); $-NH-CO-(C_{1-6}$ hydroxyalkyl); $-NH-CO-(C_{3-8}$ hydroxycycloalkyl); $-NH-CO-(CH_2)_p-CH(OH)-(C_{1-6}$ alkyl); $-NH-CO-(CH_2)_p-CH(OH)-(C_{3-8}$ cycloalkyl); or -COOH.

**4.** The antibody-drug conjugate according to claim 1 or 2, or a pharmaceutically acceptable salt or ester, solvate, tautomer, stereoisomer, prodrug, or isotopically labeled form thereof, wherein $R_1$ is H, $C_{1-6}$ alkyl, $-(CH_2)_p-(C_{1-6}$ alkylthio), $-(CH_2)_p-(NH_2)$, $-(CH_2)_p-NH(C_{1-6}$ alkyl), $-(CH_2)_p-NH(C_{1-6}$ hydroxyalkyl), $-(CH_2)_p-NH-(CH_2)_q-(C_{3-8}$ cycloalkyl), or $-NH-CO-(CH_2)_p-CH(OH)-(C_{3-8}$ cycloalkyl);

preferably, $R_1$ is H, $C_{1-6}$ alkyl, $-(CH_2)_p-(C_{1-6}$ alkylthio), $-NH_2$, $-(CH_2)_p-NH(C_{1-6}$ alkyl), $-NH(C_{1-6}$ hydroxyalkyl), $-(CH_2)_p-NH-(CH_2)_q-(C_{3-8}$ cycloalkyl), or $-NH-CO-CH(OH)-(C_{3-8}$ cycloalkyl);

more preferably, $R_1$ is H, $C_{1-6}$ alkyl, $-CH_2-(C_{1-6}$ alkylthio), $-NH_2$, $-CH_2-NH(C_{1-6}$ alkyl), $-NH(C_{1-6}$ hydroxyalkyl), $-CH_2-NH-CH_2-(C_{3-8}$ cycloalkyl), or $-NH-CO-CH(OH)-(C_{3-8}$ cycloalkyl).

**5.** The antibody-drug conjugate according to claim 1 or 2, or a pharmaceutically acceptable salt or ester, solvate, tautomer, stereoisomer, prodrug, or isotopically labeled form thereof, wherein $R_1$ is $C_{1-6}$ alkyl, $-(CH_2)_p-(C_{1-6}$ alkylthio), $-(CH_2)_p-NH(C_{1-6}$ alkyl), or $-(CH_2)_p-NH(C_{1-6}$ hydroxyalkyl); preferably, $R_1$ is $C_{1-6}$ alkyl, $-CH_2-(C_{1-6}$ alkylthio), $-NH(C_{1-6}$ alkyl), or $-NH(C_{1-6}$ hydroxyalkyl).

**6.** The antibody-drug conjugate according to claim 1 or 2, or a pharmaceutically acceptable salt or ester, solvate, tautomer, stereoisomer, prodrug, or isotopically labeled form thereof, wherein $R_1$ is $C_{1-6}$ alkyl, $-(CH_2)_p-(C_{1-6}$ alkylthio), $-NH_2$, $-(CH_2)_p-NH(C_{1-6}$ alkyl), $-(CH_2)_p-NH(C_{1-6}$ hydroxyalkyl), $-NH-CO-(C_{1-6}$ hydroxyalkyl), or $-NH-CO-(CH_2)_p-CH(OH)-(C_{3-8}$ cycloalkyl); preferably, $R_1$ is $C_{1-6}$ alkyl, $-NH_2$, $-(CH_2)_p-NH(C_{1-6}$ alkyl), $-(CH_2)_p-NH(C_{1-6}$ hydroxyalkyl), or $-NH-CO-(C_{1-6}$ hydroxyalkyl).

**7.** The antibody-drug conjugate according to claim 1 or 2, or a pharmaceutically acceptable salt or ester, solvate,

tautomer, stereoisomer, prodrug, or isotopically labeled form thereof, wherein

$R_1$ is $C_{1-6}$ alkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-NH(C_{1-6}$ hydroxyalkyl); $-(CH_2)_p-NH-(CH_2)_q-(C_{3-8}$ cycloalkyl), wherein the cycloalkyl is optionally substituted with one or more substituents selected from $C_{1-6}$ hydroxyalkyl; or $-NH-(CH_2)_p-(CH=CH)-(C_{1-6}$ hydroxyalkyl);
or, $R_1$ is $C_{1-6}$ alkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-(C_{1-6}$ alkylthio); $-(CH_2)_p-NH_2$; $-(CH_2)_p-NH(C_{1-6}$ alkyl); $-(CH_2)_p-NH(C_{1-6}$ hydroxyalkyl); $-(CH_2)_p-NH-(CH_2)_q-(C_{3-8}$ cycloalkyl), wherein the cycloalkyl is optionally substituted with one or more substituents selected from $C_{1-6}$ hydroxyalkyl; $-NH-CO-(C_{1-6}$ hydroxyalkyl); or $-NH-CO-(CH_2)_p-CH(OH)-(C_{3-8}$ cycloalkyl);
or, $R_1$ is $C_{1-6}$ alkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-NH_2$; $-(CH_2)_p-NH(C_{1-6}$ alkyl); $-(CH_2)_p-NH(C_{1-6}$ hydroxyalkyl); $-(CH_2)_p-NH-(CH_2)_q-(C_{3-8}$ cycloalkyl), wherein the cycloalkyl is optionally substituted with one or more substituents selected from $C_{1-6}$ hydroxyalkyl; $-NH-CO-(C_{1-6}$ hydroxyalkyl); or $-NH-CO-(CH_2)_p-CH(OH)-(C_{3-8}$ cycloalkyl);;
or, $R_1$ is $C_{1-6}$ alkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-NH(C_{1-6}$ hydroxyalkyl); $-(CH_2)_p-NH-(CH_2)_q-(C_{3-8}$ cycloalkyl), wherein the cycloalkyl is optionally substituted with one or more substituents selected from $C_{1-6}$ hydroxyalkyl; or $-NH-CO-(CH_2)_p-CH(OH)-(C_{3-8}$ cycloalkyl);
or, $R_1$ is $C_{1-6}$ alkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-NH(C_{1-6}$ hydroxyalkyl); or $-(CH_2)_p-NH-(CH_2)_q-(C_{3-8}$ cycloalkyl), wherein the cycloalkyl is optionally substituted with one or more substituents selected from $C_{1-6}$ hydroxyalkyl.

**8.** The antibody-drug conjugate according to any one of the preceding claims, or a pharmaceutically acceptable salt or ester, solvate, tautomer, stereoisomer, prodrug, or isotopically labeled form thereof, wherein $R_2$ is a halogen, preferably fluorine.

**9.** The antibody-drug conjugate according to any one of the preceding claims, or a pharmaceutically acceptable salt or ester, solvate, tautomer, stereoisomer, prodrug, or isotopically labeled form thereof, wherein $R_3$ is $C_{1-6}$ alkyl, $C_{1-6}$ alkoxy, halogen, cyano, $-NH_2$, $-NH-CO-(C_{1-6}$ hydroxyalkyl), or $NO_2$; preferably, $R_3$ is $C_{1-6}$ alkyl, halogen, $-NH_2$, $-NH-CO-(C_{1-6}$ hydroxyalkyl), or $NO_2$;

or, $R_3$ is halogen, cyano, $NO_2$, $C_{1-6}$ alkyl, $C_{1-6}$ haloalkyl, $C_{1-6}$ hydroxyalkyl, $C_{1-6}$ alkoxy, or $NR_aR_b$, wherein $R_a$ and $R_b$ are each independently H, $C_{1-6}$ alkyl, $-SO-(C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino; preferably, $R_3$ is $C_{1-6}$ alkyl, $C_{1-6}$ alkoxy, halogen, cyano, $-NH_2$ or $NO_2$; more preferably, $R_3$ is $C_{1-6}$ alkyl, halogen, $-NH_2$ or $NO_2$;
or, $R_3$ is $C_{1-6}$ alkyl or $NR_aR_b$, wherein $R_a$ and $R_b$ are each independently H or $-CO-(C_{1-6}$ alkyl), wherein the alkyl is optionally substituted with one or more OH groups;
or, $R_3$ is $C_{1-6}$ alkyl, $-NH_2$ or $-NH-CO-(CH_2OH)$, preferably $R_3$ is $C_{1-6}$ alkyl or $-NH-CO-(CH_2OH)$;
or $R_3$ is $C_{1-6}$ alkyl.

**10.** The antibody-drug conjugate according to any one of the preceding claims, or a pharmaceutically acceptable salt or ester, solvate, tautomer, stereoisomer, prodrug, or isotopically labeled form thereof, wherein $R_2$ and $R_3$, together with the atoms to which they are attached, form a 4- to 8-membered heterocyclic group containing N, O or S as a heteroatom, such as dioxolane.

**11.** The antibody-drug conjugate according to any one of the preceding claims, or a pharmaceutically acceptable salt or ester, solvate, tautomer, stereoisomer, prodrug, or isotopically labeled form thereof, wherein D is connected to $L_4$ via a nitrogen or oxygen atom in $R_1$ or $R_3$, or via the oxygen atom from hydroxy shown in formula (II), for example, through an amide bond, an aminomethyl ether bond, or a carbamate bond.

**12.** The antibody-drug conjugate according to any one of the preceding claims, or a pharmaceutically acceptable salt or ester, solvate, tautomer, stereoisomer, prodrug, or isotopically labeled form thereof, wherein D is a moiety selected from the following:

| No. | Structure | No. | Structure |
|---|---|---|---|
| 1’ | 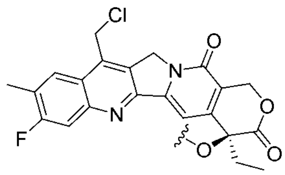 STI-A-4' | 2’ | 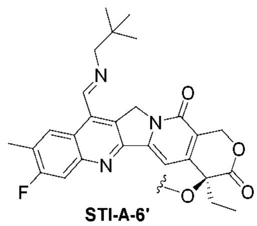 STI-A-6' |
| 3’ | 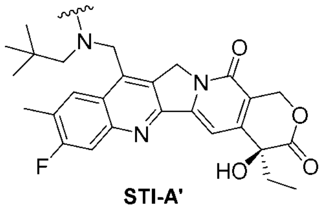 STI-A' | 4’ | 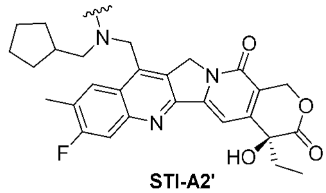 STI-A2' |
| 5’ | 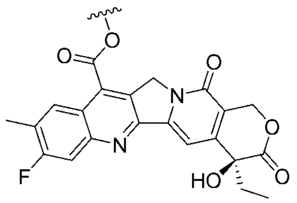 STI-F-01' | 6’ | 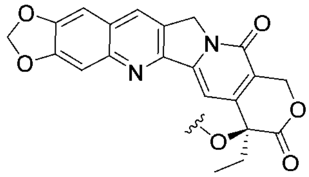 STI-E-05' |
| 7’ | 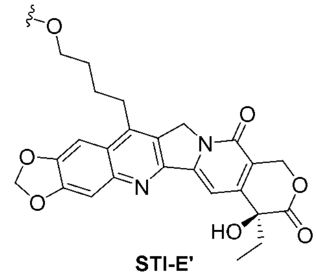 STI-E' | 8’ | 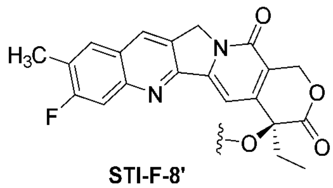 STI-F-8' |
| 9’ | 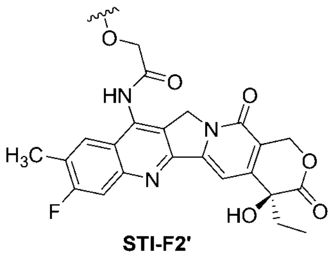 STI-F2' | 10’ | 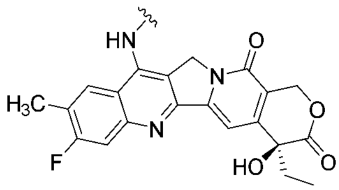 STI-F-03c-1' |
| 11’ | 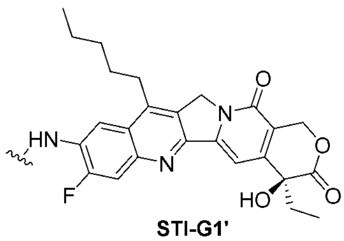 STI-G1' | 12’ | 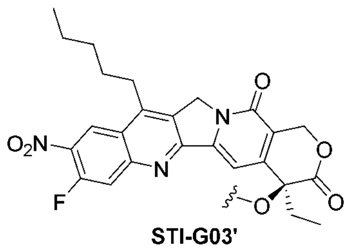 STI-G03' |

(continued)

| No. | Structure | No. | Structure |
|---|---|---|---|
| 13' | 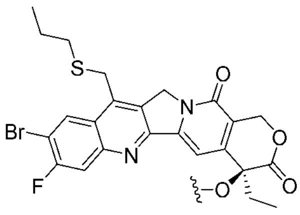 STI-G2-06c' | 14' | 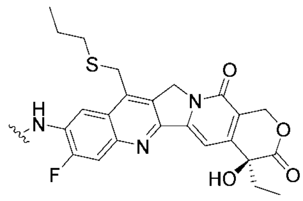 STI-G2' |
| 15' | 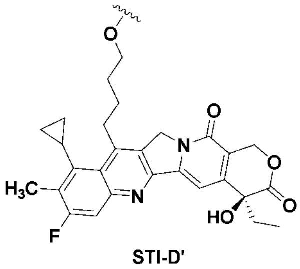 STI-D' | 16' | 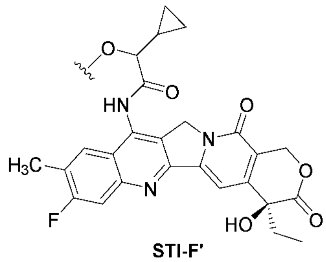 STI-F' |
| 17' | 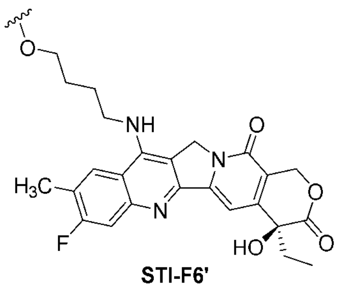 STI-F6' | 18' | 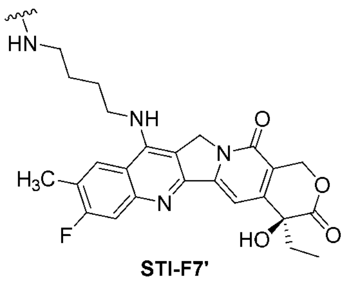 STI-F7' |
| 19' | 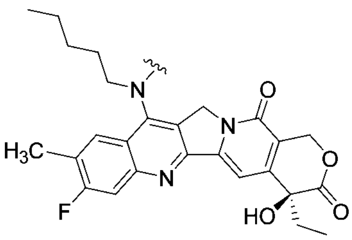 STI-F13' | 20' | 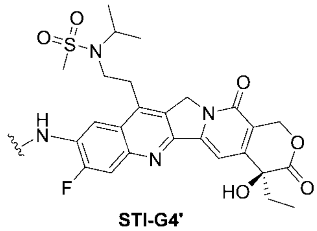 STI-G4' |
| 21' | 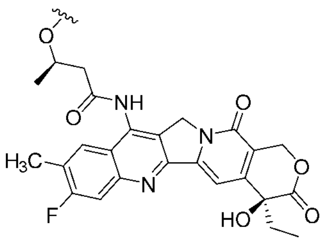 SIT-F12R' | 22' | 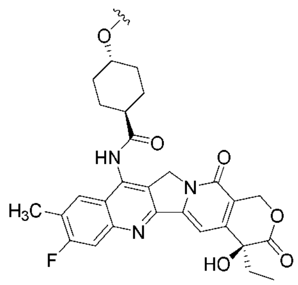 STI-F10' |

(continued)

| No. | Structure | No. | Structure |
|---|---|---|---|
| P1' | 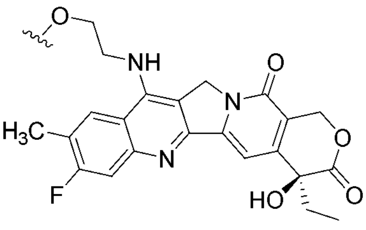 STI-F5' | P2' | 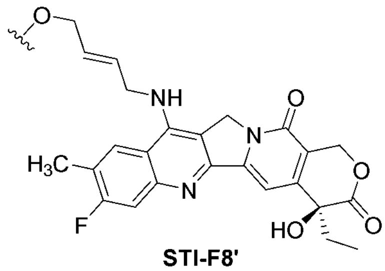 STI-F8' |
| P3' | 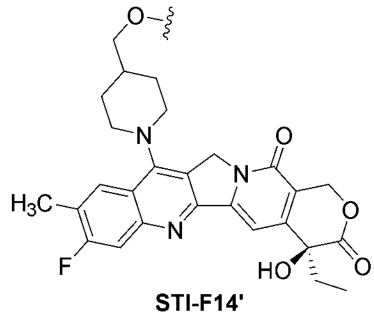 STI-F14' | P4' | 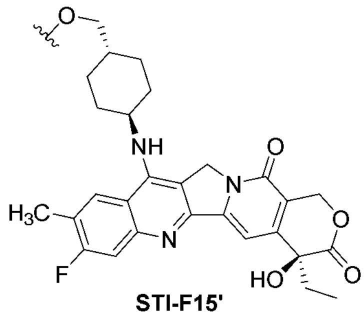 STI-F15' |
| P5' | 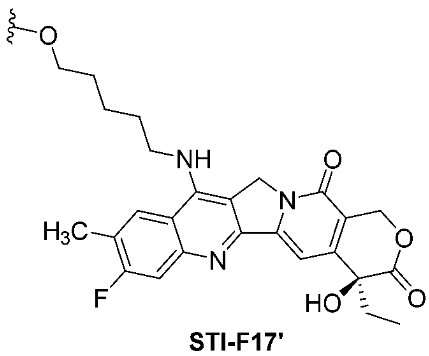 STI-F17' | P6' | 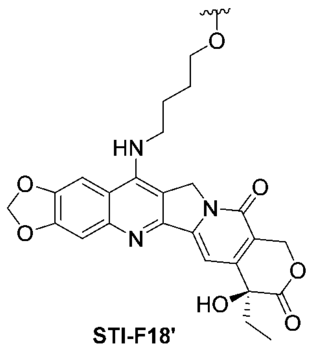 STI-F18' |
| P7' | 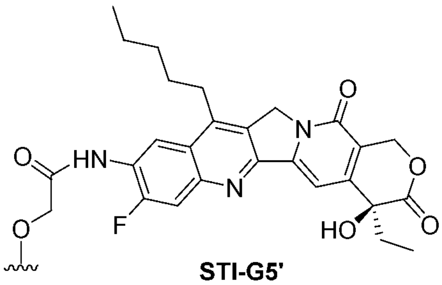 STI-G5' | | |

**13.** The antibody-drug conjugate according to any one of the preceding claims, or a pharmaceutically acceptable salt or ester, solvate, tautomer, stereoisomer, prodrug, or isotopically labeled form thereof, wherein $L_1$ is each independently:

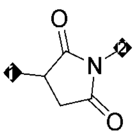 , 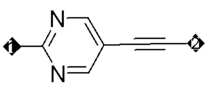 , 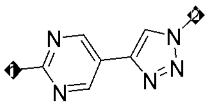 , 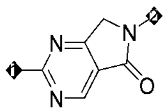 , or 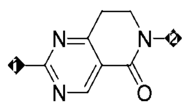 ,

preferably

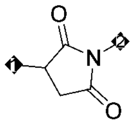 or 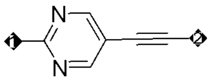,

more preferably

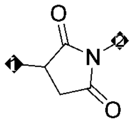,

wherein position 1 is connected to Ab, and position 2 is connected to $L_2$.

**14.** The antibody-drug conjugate according to any one of the preceding claims, or a pharmaceutically acceptable salt or ester, solvate, tautomer, stereoisomer, prodrug, or isotopically labeled form thereof, wherein $L_2$ is each independently a linking unit selected from the group consisting of:

$-(CH_2)_t-(L_M)_{0-1}-(CH_2CH_2O)_s-(CH_2)_t-CO-$, $-(CH_2)_t-L_M-(CH_2)_t-(L_M)_{0-1}-L_N-L_M-(CH_2O)_s-(CH_2)_t-CO-$, $-(CH_2)_t-L_M-(CH_2)_t-N(R_p)-(CH_2)_t-N(R_p)-(CH_2)_t-CO-$, and $(CH_2)_t-L_M-CH(R_p)-CO-$,

wherein the -CO- terminus of $L_2$ is connected to $L_3$, and the other terminus of $L_2$ is connected to $L_1$.

**15.** The antibody-drug conjugate according to any one of the preceding claims, or a pharmaceutically acceptable salt or ester, solvate, tautomer, stereoisomer, prodrug, or isotopically labeled form thereof, wherein $L_2$ is each independently a linking unit selected from the group consisting of:

$-(CH_2)_t-(CH_2CH_2O)_s-(CH_2)_t-CO-$,

$-(CH_2)_t-CO-NH-(CH_2CH_2O)_s-(CH_2)_t-CO-$, $-(CH_2)_t-NH-CO-(CH_2CH_2O)_s-(CH_2)_t-CO-$,

$-(CH_2)_t-CO-NH-(CH_2)_t-$(3- to 8-membered heteroarylene)$-(CH_2)_t-(OCH_2CH_2)_m-NH-CO-(CH_2O)-(CH_2)_t-CO-$,

$-(CH_2)_t-CO-NH-(CH_2)_t-NH-CO-(CH_2CH_2O)_m-(CH_2)_t-NH-CO-(CH_2O)-(CH_2)_t-CO-$,

$-(CH_2)_t-CO-NH-(CH_2)_t-NH-CO-(CH_2)_t-(OCH_2CH_2)_m-NH-CO-(CH_2O)-(CH_2)_t-CO-$,

$-(CH_2)_t-NH-CO-(CH_2)_t-N(R_p)-(CH_2)_t-N(R_p)-(CH_2)_t-CO-$,

$-(CH_2)_t-CO-NH-(CH_2)_t-N(R_p)-(CH_2)_t-N(R_p)-(CH_2)_t-CO-$, or

$-(CH_2)_t-CO-NH-CH(R_p)-CO-$ or $-(CH_2)_t-NH-CO-CH(R_p)-CO-$,

wherein the -CO- terminus of $L_2$ is connected to $L_3$, and the other terminus of $L_2$ is connected to $L_1$.

**16.** The antibody-drug conjugate according to any one of the preceding claims, or a pharmaceutically acceptable salt or ester, solvate, tautomer, stereoisomer, prodrug, or isotopically labeled form thereof, wherein $L_3$ is each independently an amino acid residue, a short peptide chain consisting of 2-6 amino acid residues, or $-NH-(CH_2)_p-CO-$, wherein p is 0, 1, 2, 3, 4, 5, or 6; preferably, the amino acid is selected from the group consisting of glycine (Gly), alanine (Ala), valine (Val), phenylalanine (Phe), citrulline (Cit), lysine (Lys) and asparagine (Asn); for example, $L_3$ is Gly-Gly-Phe-Gly, Val-Ala, Phe-Lys, or Lys.

**17.** The antibody-drug conjugate according to any one of the preceding claims, or a pharmaceutically acceptable salt or ester, solvate, tautomer, stereoisomer, prodrug, or isotopically labeled form thereof, wherein $L_4$ is each independently a bond,

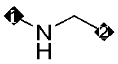, 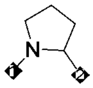, 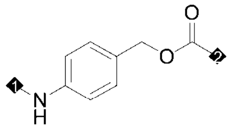, 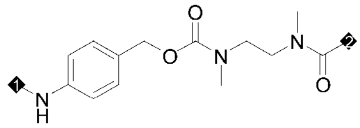 and 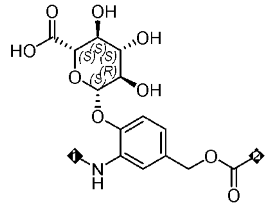,

preferably a bond,

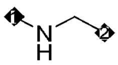, 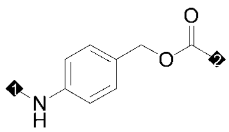 or 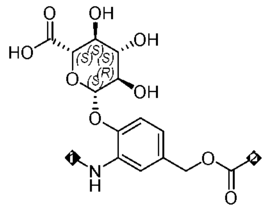,

more preferably

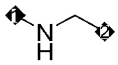,

wherein position 1 is connected to $L_3$ and position 2 is connected to D.

**18.** The antibody-drug conjugate according to any one of the preceding claims, or a pharmaceutically acceptable salt or ester, solvate, tautomer, stereoisomer, prodrug, or isotopically labeled form thereof, wherein:

$L_1$ is each independently:

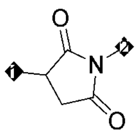, 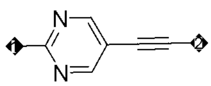, 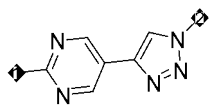, 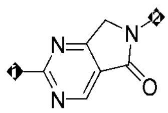,

or

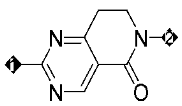,

preferably

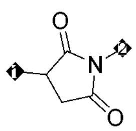 or 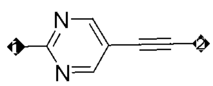,

more preferably

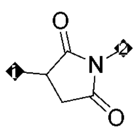,

wherein position 1 is connected to Ab, and position 2 is connected to $L_2$;

$L_2$ is each independently a linking unit of the formula $-(CH_2CH_2O)_x-(CH_2)_y-CO-$, wherein the -CO- terminus is connected to $L_3$ and the other terminus is connected to $L_1$, and wherein x is an integer from 0 to 5, y is an integer from 1 to 5, and $x + y \leq 6$; preferably, $L_2$ is $-(CH_2)-CO-$, $-(CH_2)_2-CO-$, $-(CH_2)_3-CO-$, $-(CH_2)_4-CO-$, $-(CH_2)_5-CO-$, $-(CH_2)_6-CO-$, $-(CH_2CH_2O)-(CH_2)-CO-$, $-(CH_2CH_2O)-(CH_2)_2-CO-$, $-(CH_2CH_2O)-(CH_2)_3-CO-$, $-(CH_2CH_2O)-(CH_2)_4-CO-$, $-(CH_2CH_2O)-(CH_2)_5-CO-$, $-(CH_2CH_2O)_2-(CH_2)-CO-$, $-(CH_2CH_2O)_2-(CH_2)_2-CO-$, $-(CH_2CH_2O)_2-(CH_2)_3-CO-$, $-(CH_2CH_2O)_2-(CH_2)_4-CO-$, $-(CH_2CH_2O)_3-(CH_2)-CO-$, $-(CH_2CH_2O)_3-(CH_2)_2-CO-$, $-(CH_2CH_2O)_3-(CH_2)_3-CO-$; more preferably, $L_2$ is each independently $-(CH_2)_5-CO-$ or $-(CH_2CH_2O)_3-(CH_2)_2-CO-$;

$L_3$ is each independently an amino acid residue, a short peptide chain consisting of 2-6 amino acid residues, or $-NH-(CH_2)_p-CO-$, wherein p is 0, 1, 2, 3, 4, 5, or 6; preferably, the amino acid is selected from the group consisting of glycine (Gly), alanine (Ala), valine (Val), phenylalanine (Phe), citrulline (Cit), lysine (Lys) and asparagine (Asn); for example, $L_3$ is Gly-Gly-Phe-Gly, Val-Cit, Val-Ala, Phe-Lys, Val-Lys, Ala-Ala-Ala, Ala-Ala-Asn, or $-NH-(CH_2)_2-CO-$; and

$L_4$ is each independently a bond ,

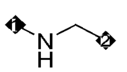 , 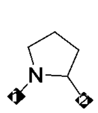 , 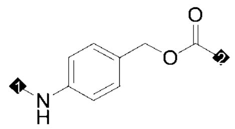 ,

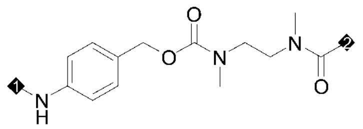 and 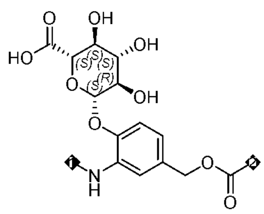 ,

wherein position 1 is connected to $L_3$ and position 2 is connected to D.

**19.** The antibody-drug conjugate according to any one of the preceding claims, or a pharmaceutically acceptable salt or ester, solvate, tautomer, stereoisomer, prodrug, or isotopically labeled form thereof, wherein the $-L_1-L_2-L_3-L_4-$ moiety is each independently selected from the group consisting of:

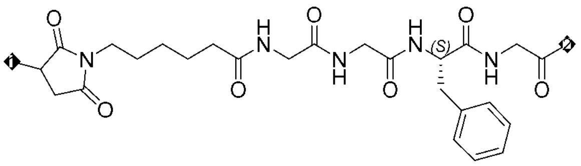 ; 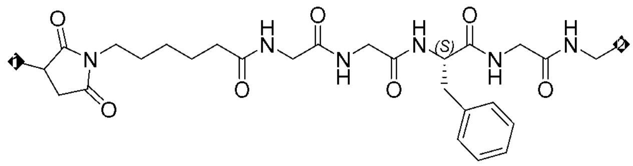 ;

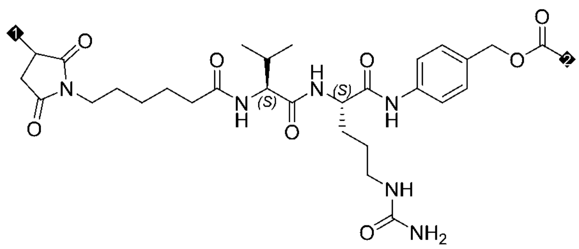 ; 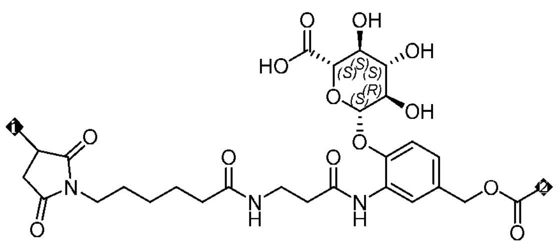 ;

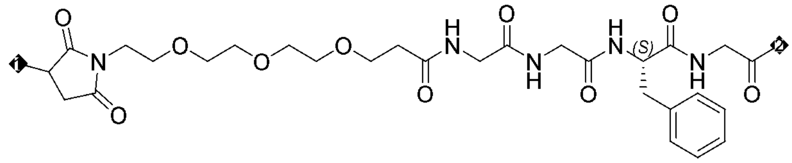 ;

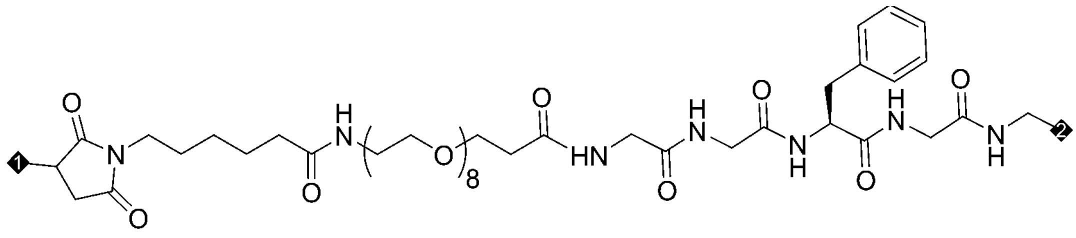

,

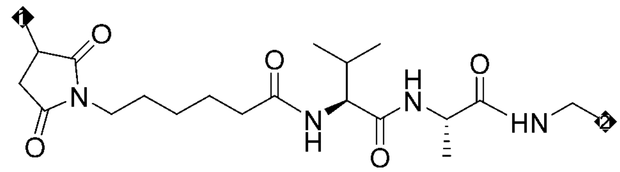

,

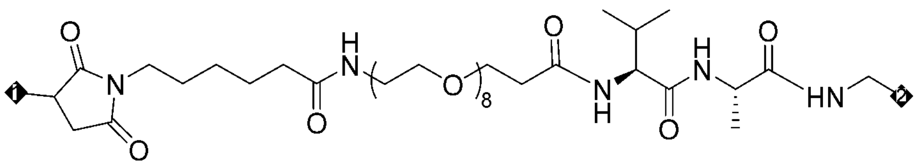

,

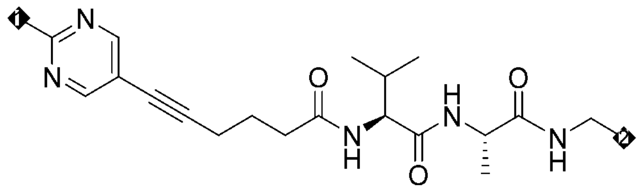

,

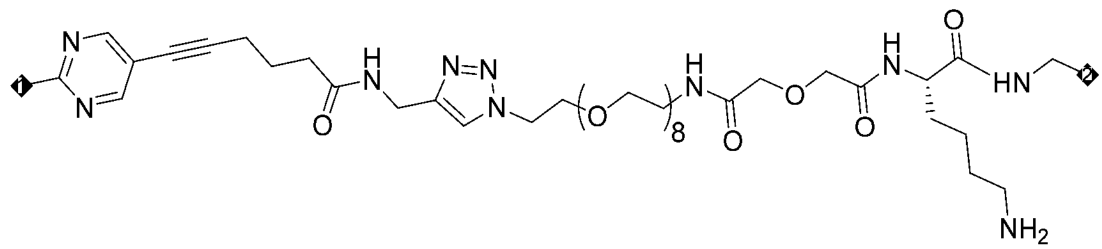

,

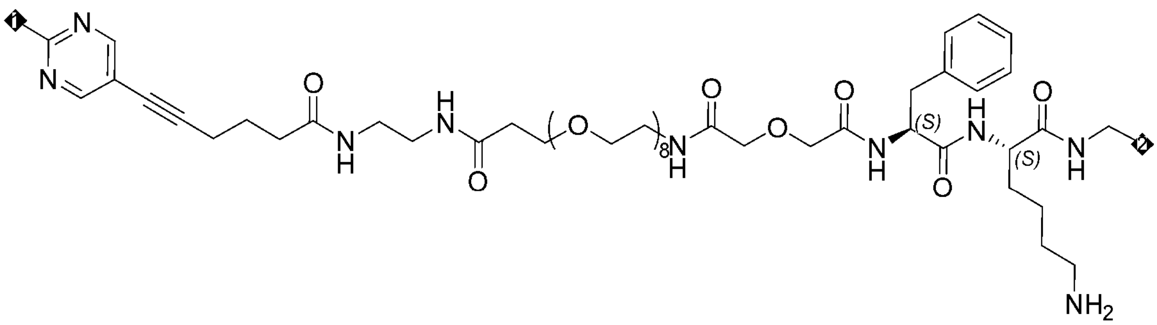

,

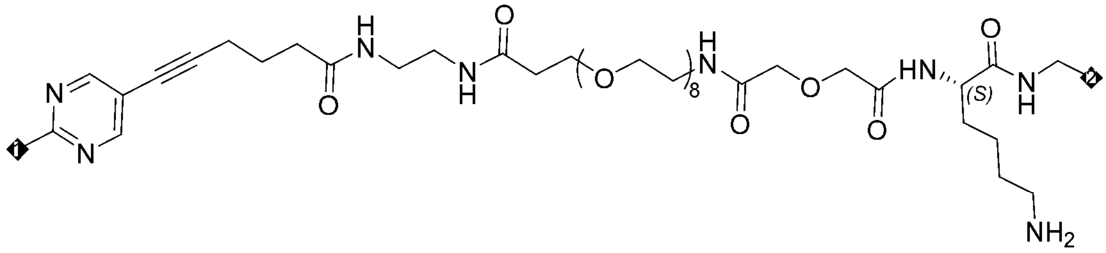

,

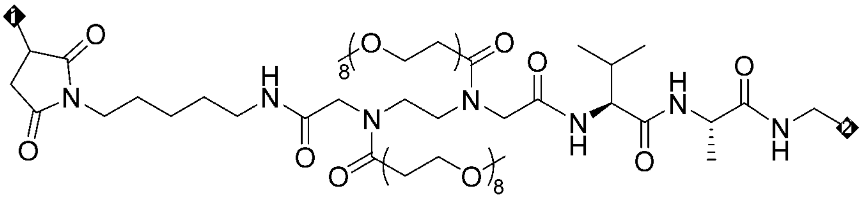

,

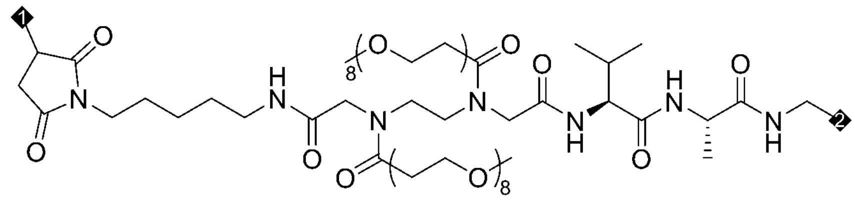

,

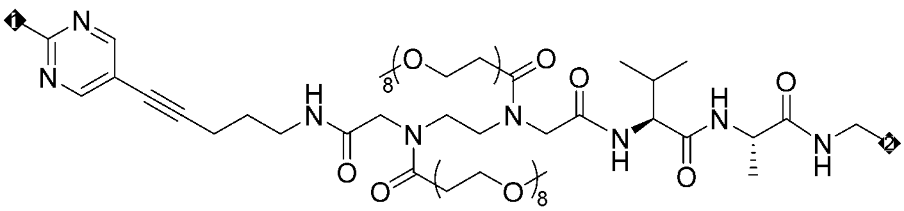

,

and

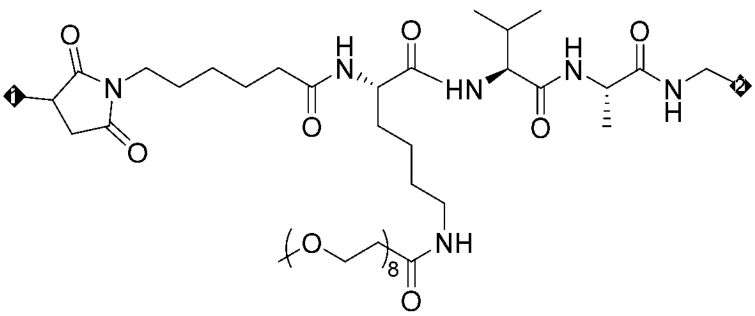

.

wherein position 1 is connected to Ab, and position 2 is connected to D.

**20.** The antibody-drug conjugate according to any one of the preceding claims, or a pharmaceutically acceptable salt or ester, solvate, tautomer, stereoisomer, prodrug, or isotopically labeled form thereof, wherein the $-L_1-L_2-L_3-L_4-D$ moiety is each independently selected from:

| No. | Structure |
|---|---|
| 23' | 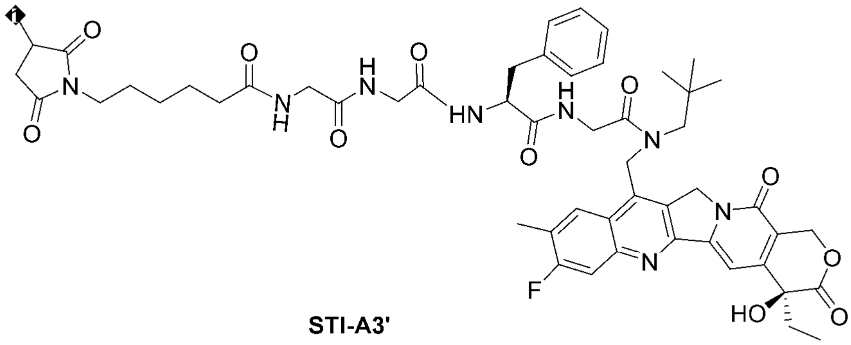 STI-A3' |
| 24' | 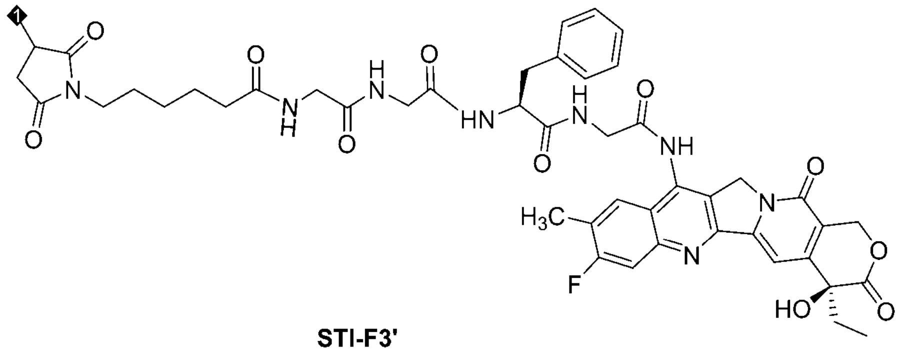 STI-F3' |
| 25' | 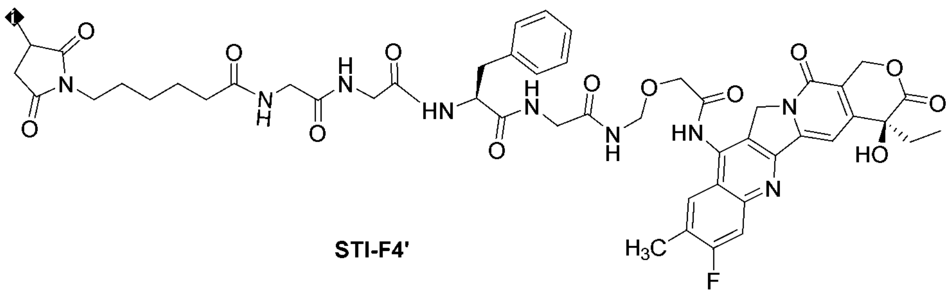 STI-F4' |
| 26' | 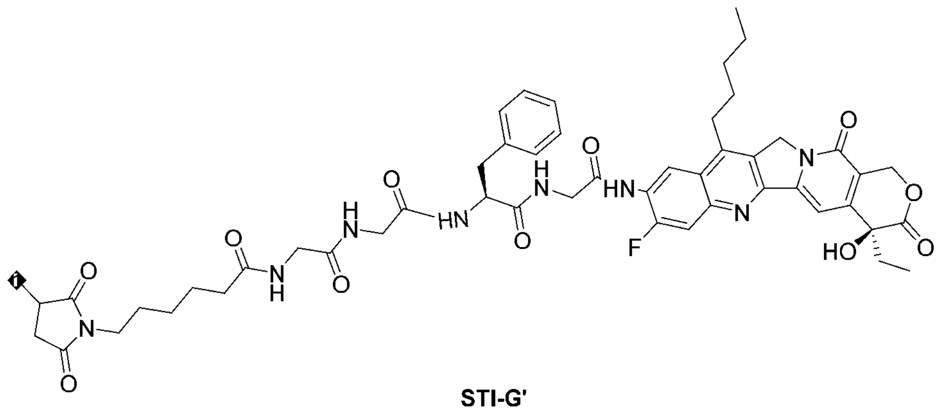 STI-G' |

(continued)

| No. | Structure |
|---|---|
| 27' | 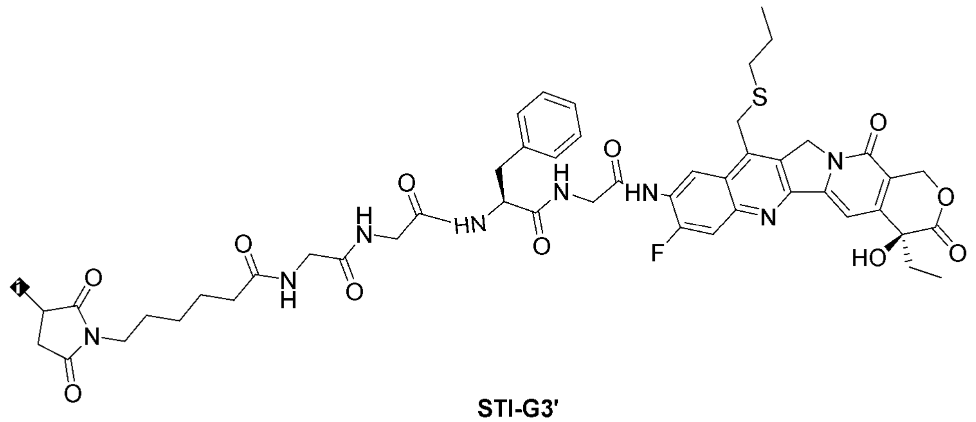<br>**STI-G3'** |
| 28' | 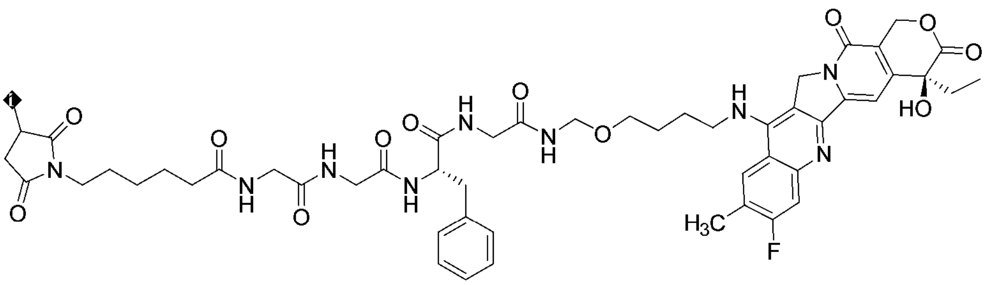<br>**STI-F1002'** |
| 29' | 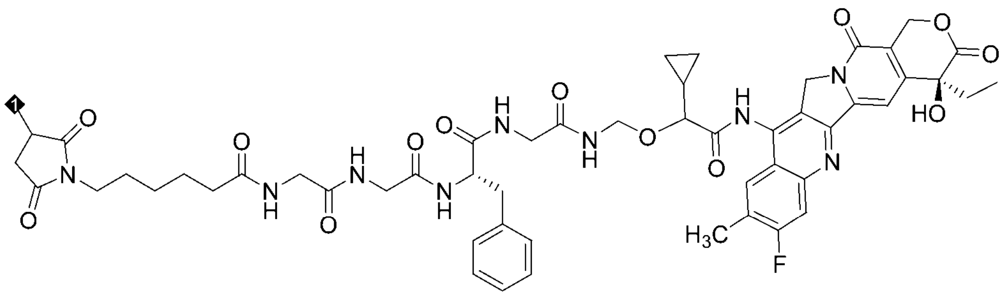<br>**STI-F1001'** |
| 30' | 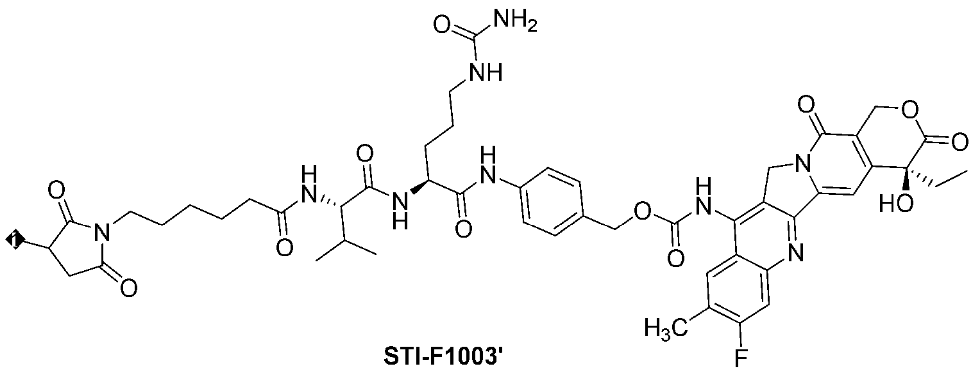<br>**STI-F1003'** |

(continued)

| No. | Structure |
|---|---|
| 31' | 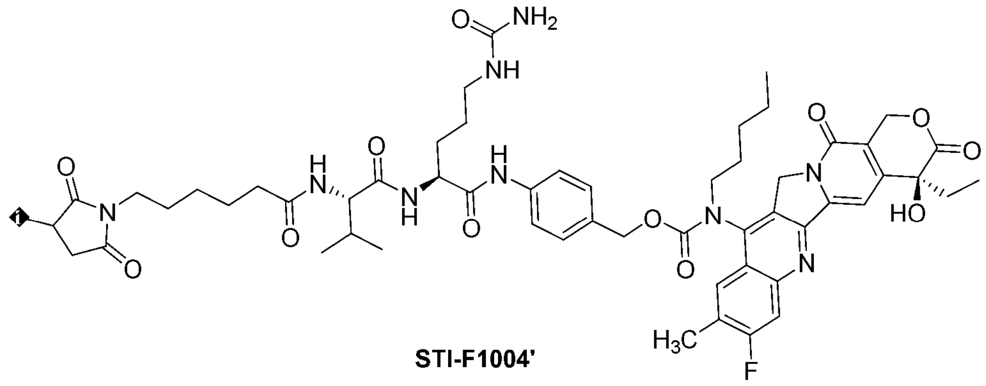 STI-F1004' |
| 32' | 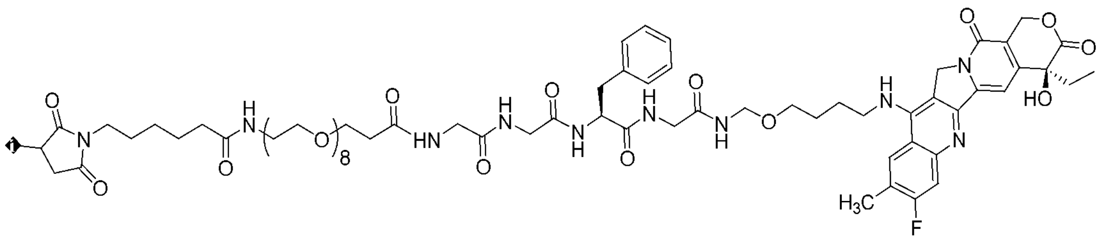 STI-F1006 |
| 33' | 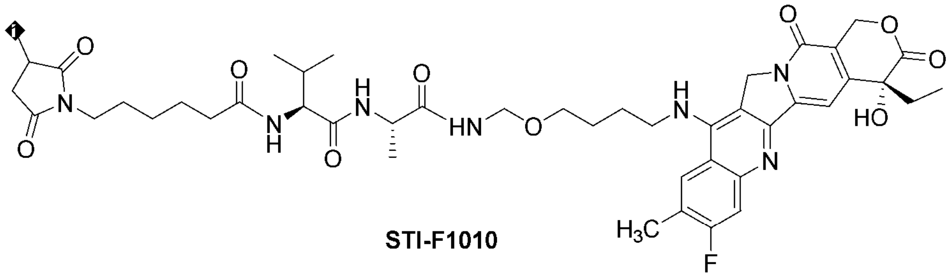 STI-F1010 |
| 34' | 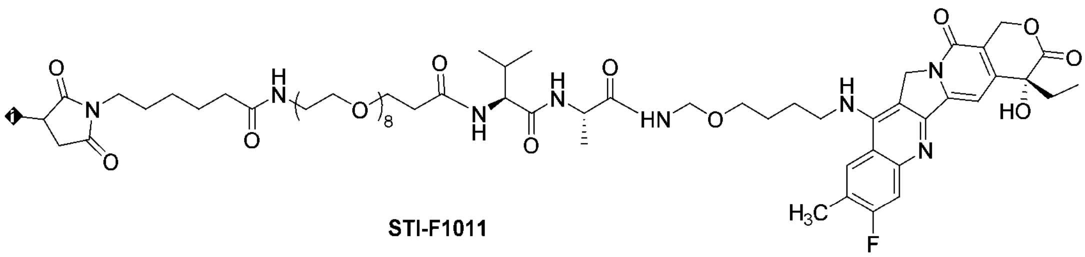 STI-F1011 |
| 35' | 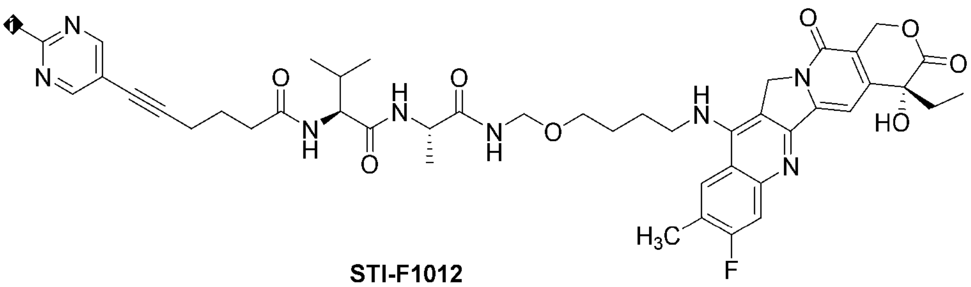 STI-F1012 |

(continued)

| No. | Structure |
|---|---|
| 36' | 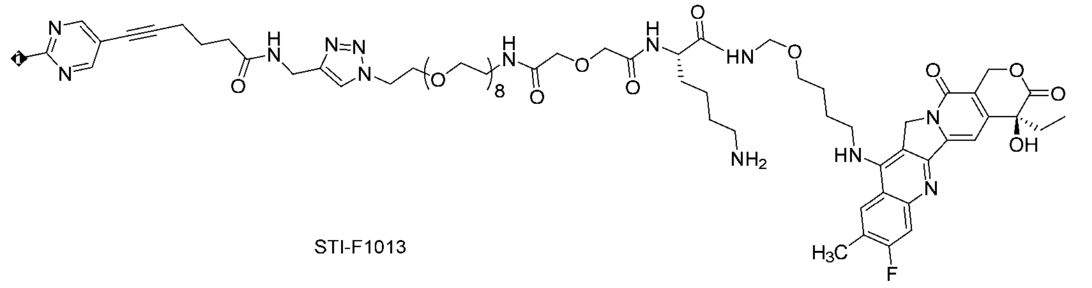 STI-F1013 |
| 37' | 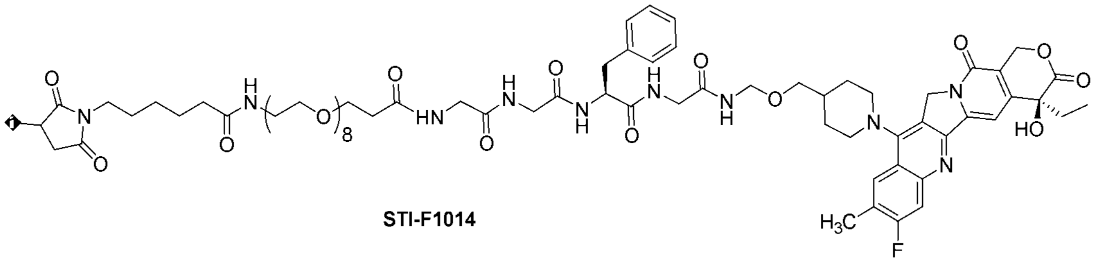 **STI-F1014** |
| 38' | 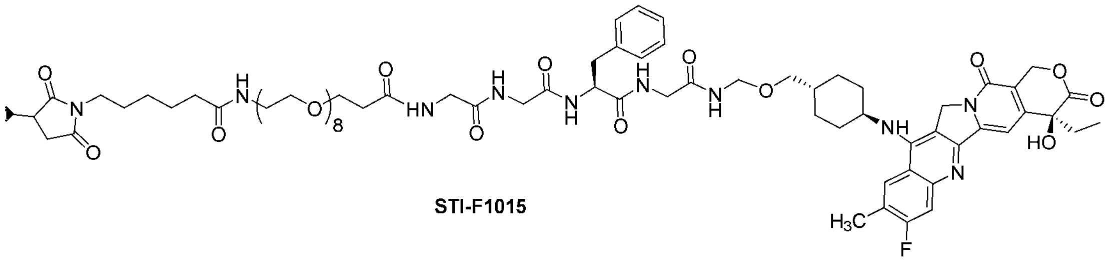 **STI-F1015** |
| 39' | 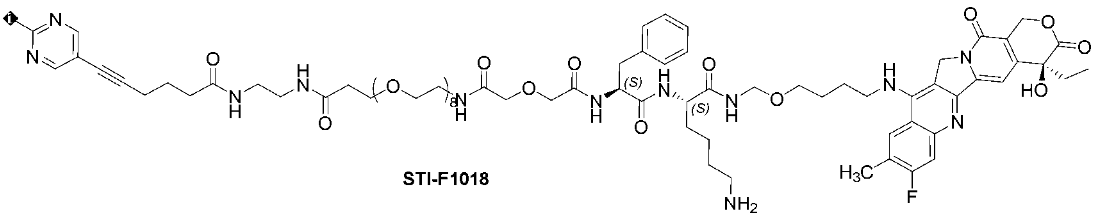 **STI-F1018** |
| 40' | 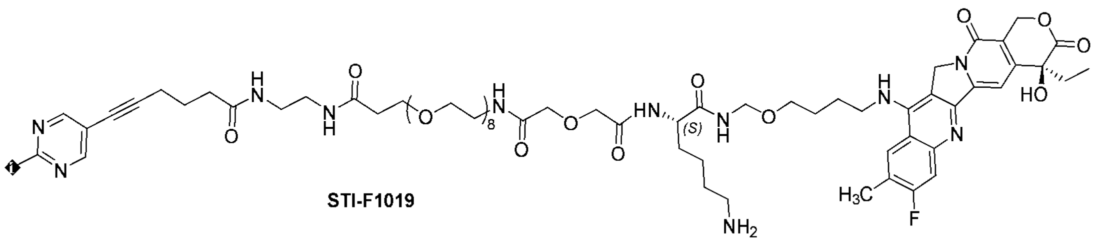 **STI-F1019** |
| 41' | 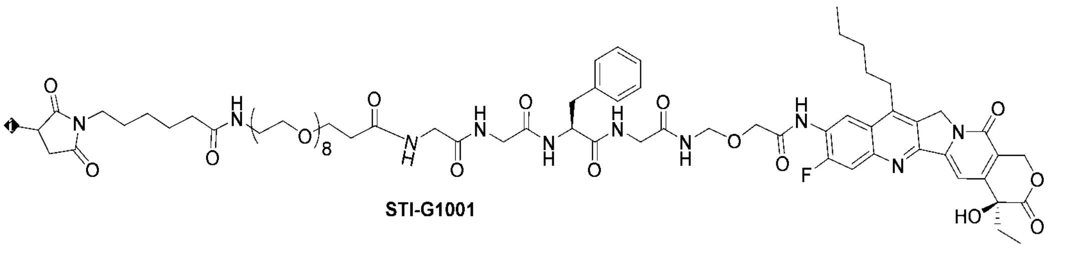 **STI-G1001** |

(continued)

| No. | Structure |
|---|---|
| 42' | 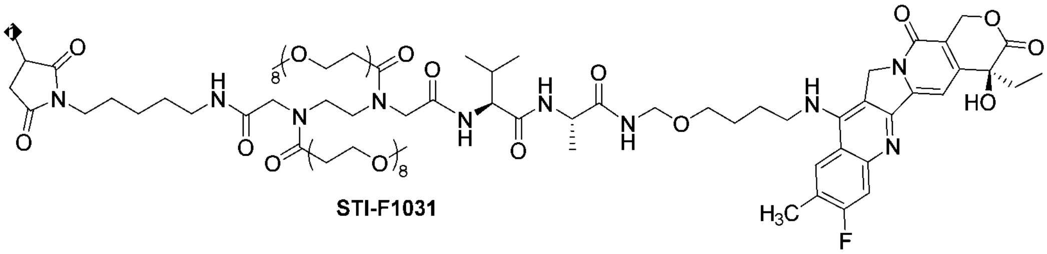 STI-F1031 |
| 43' | 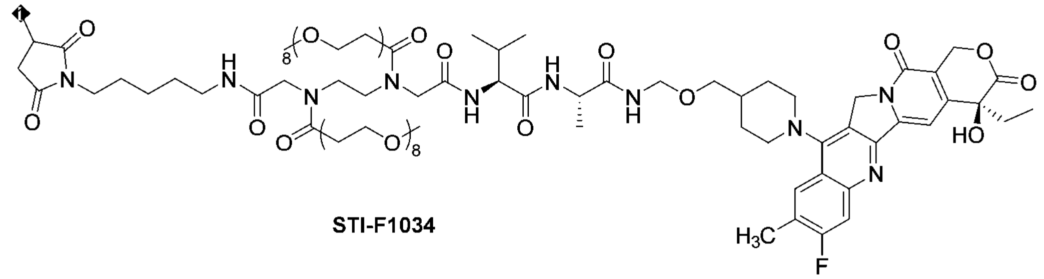 STI-F1034 |
| 44' | 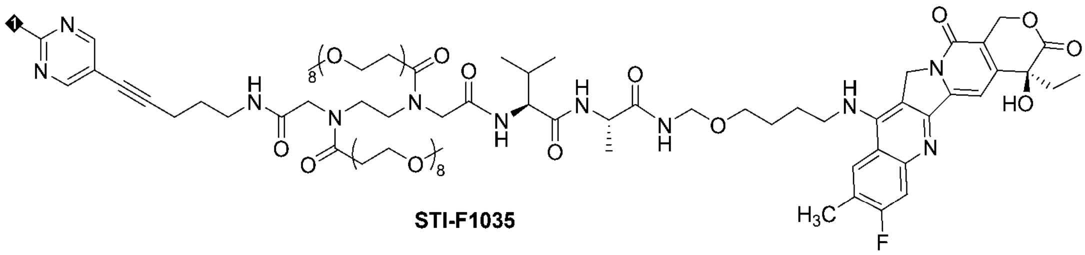 STI-F1035 |
| 45' | 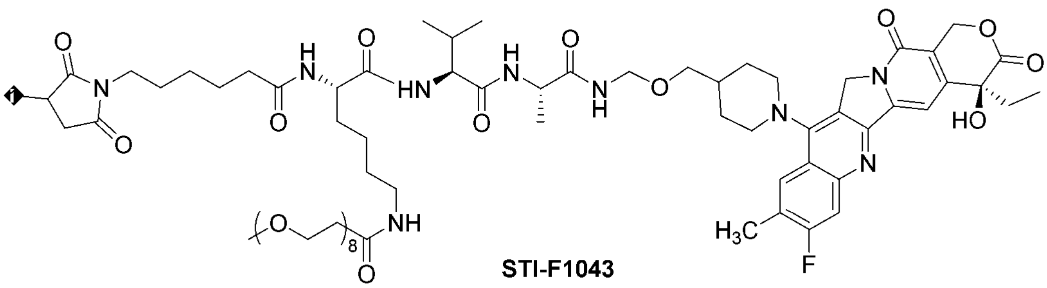 STI-F1043 |

wherein position 1 represents the point of attachment to Ab.

**21.** The antibody-drug conjugate according to any one of the preceding claims, or a pharmaceutically acceptable salt or ester, solvate, tautomer, stereoisomer, prodrug, or isotopically labeled form thereof, wherein Ab is an antibody or an antigen-binding fragment thereof that binds to a tumor cell surface antigen; for example, an anti-Her2 antibody or an antigen-binding fragment thereof and/or an anti-FRα antibody or an antigen-binding fragment thereof; in particular, Ab is farletuzumab or an antigen-binding fragment thereof and/or trastuzumab or an antigen-binding fragment thereof.

**22.** The antibody-drug conjugate according to any one of the preceding claims, or a pharmaceutically acceptable salt or ester, solvate, tautomer, stereoisomer, prodrug, or isotopically labeled form thereof, wherein the antibody-drug conjugate is selected from the group consisting of:

| Name | Structure |
|---|---|
| Fa-ADC1 | 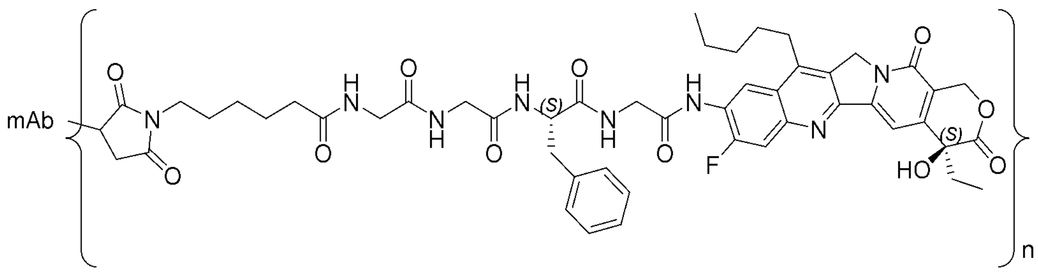 |
| Fa-ADC2 | 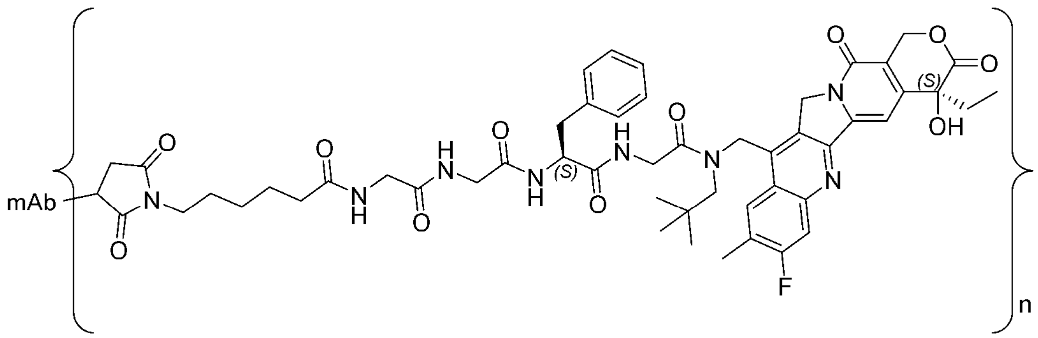 |
| Fa-ADC3 | 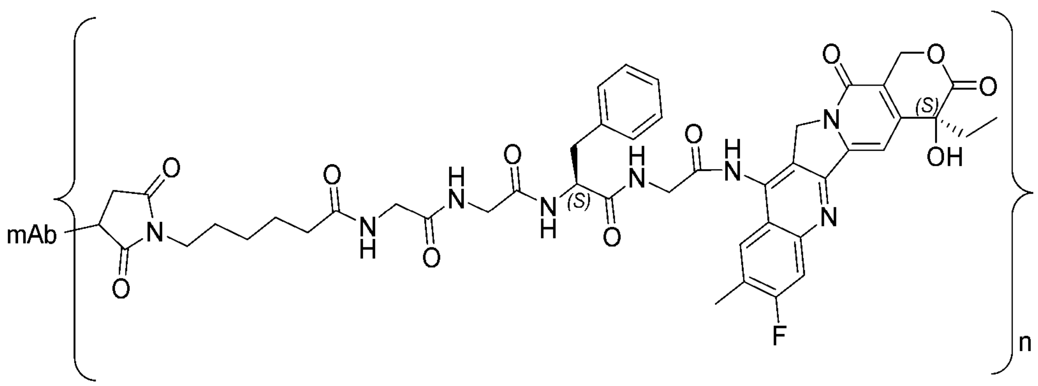 |
| Fa-ADC4 | 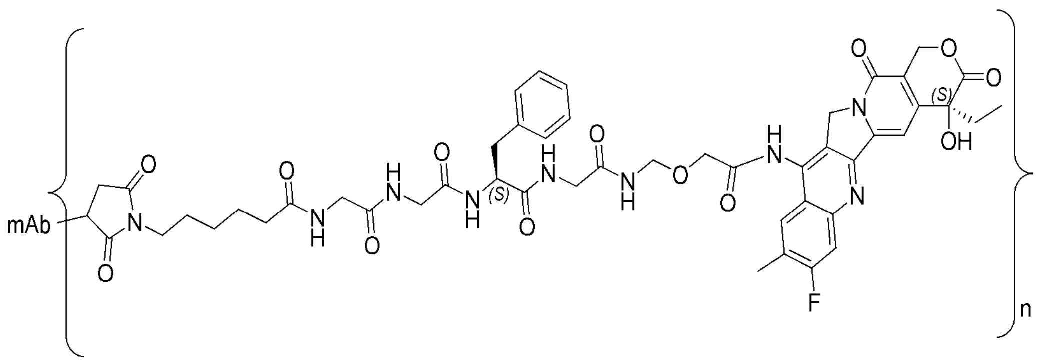 |

(continued)

| Name | Structure |
|---|---|
| Fa-ADC5 | 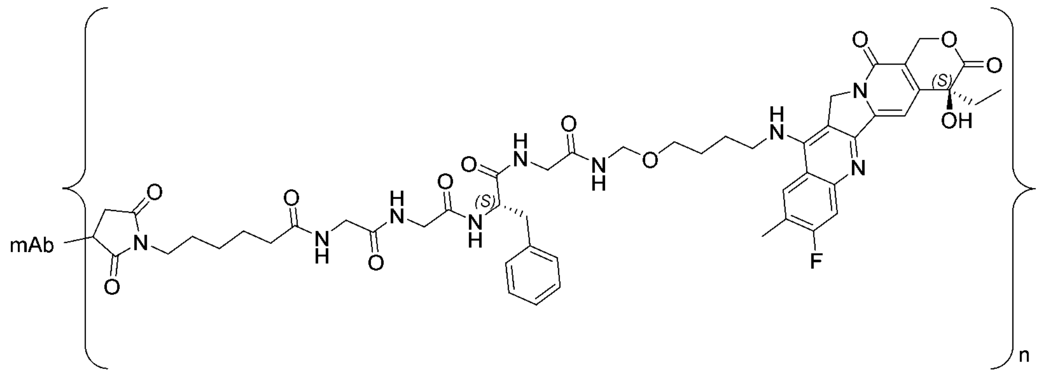 |
| Fa-ADC6 | 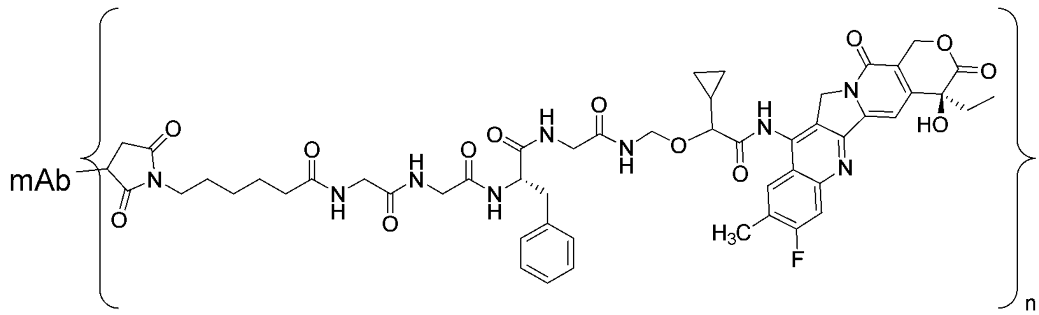 |
| Tra-ADC7 | 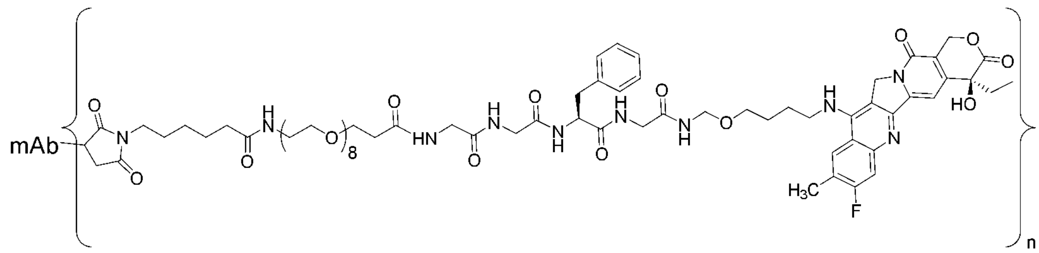 |
| Tra-ADC8 | 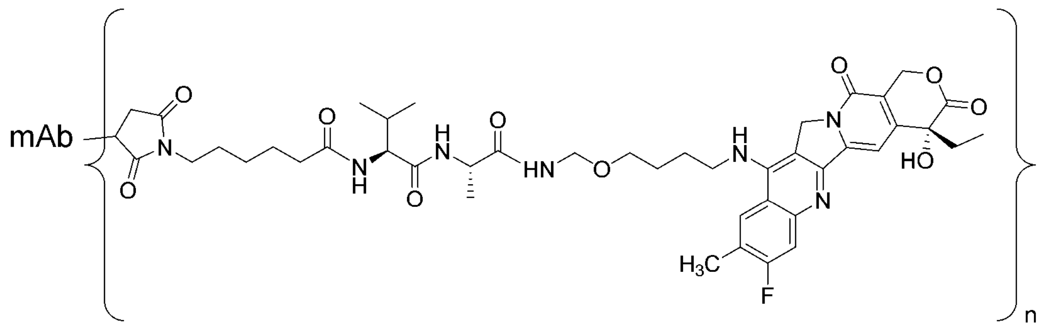 |
| Tra-ADC9 | 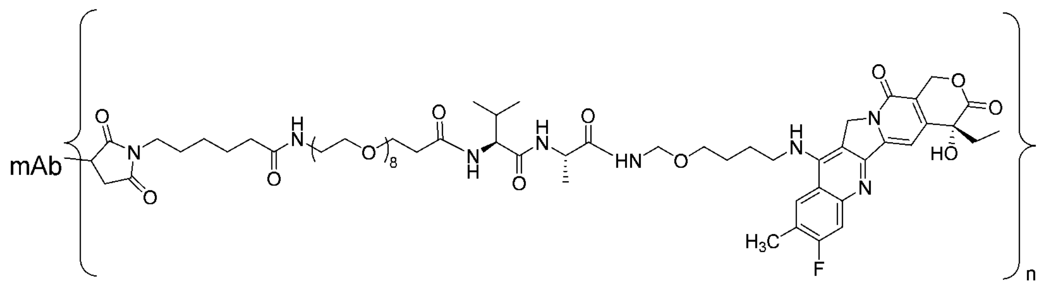 |

(continued)

| Name | Structure |
|---|---|
| Tra-ADC10 | 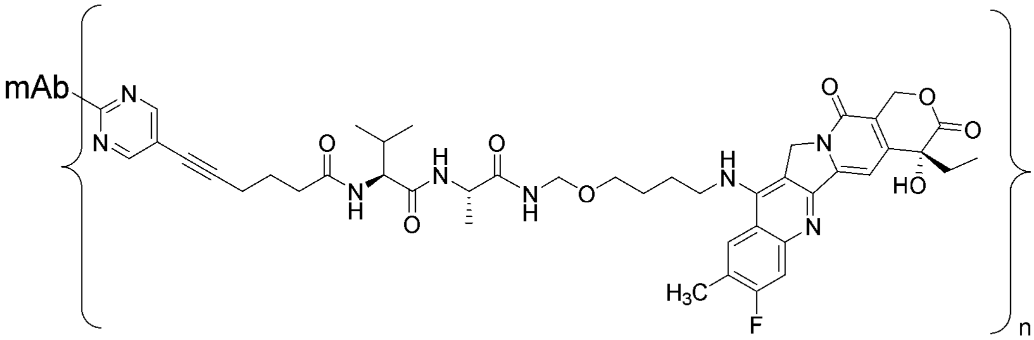 |
| Tra-ADC11 | 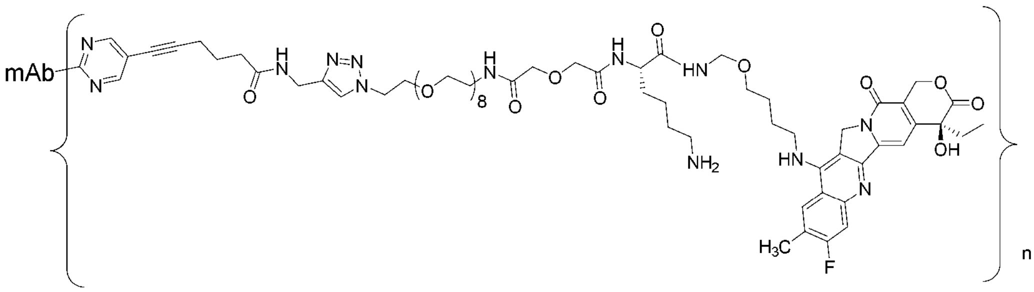 |
| Tra-ADC12 | 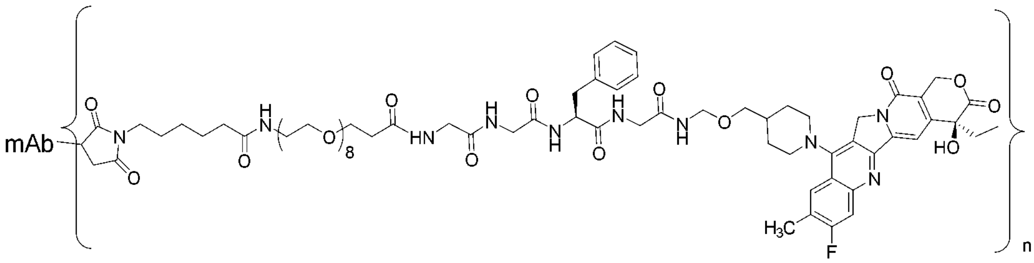 |
| Tra-ADC13 | 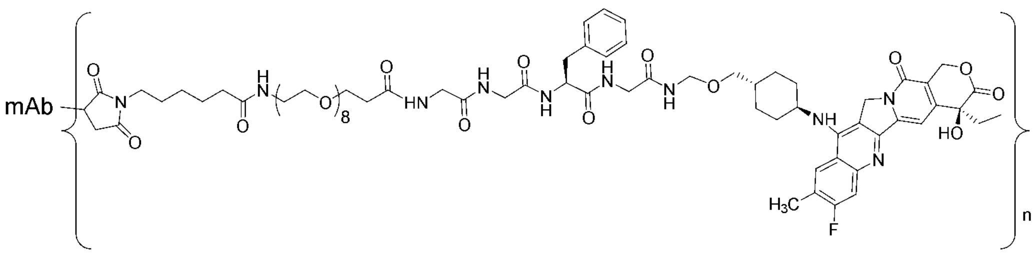 |
| Tra-ADC14 | 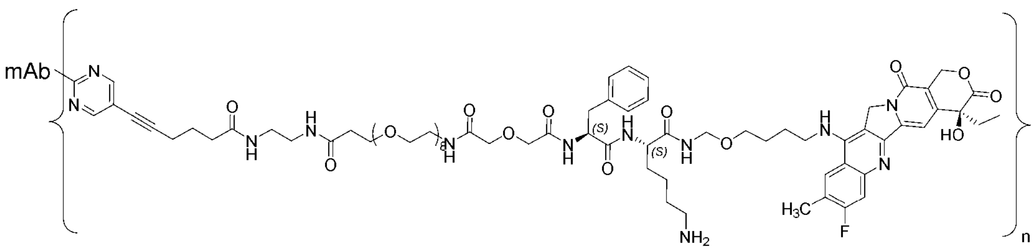 |

(continued)

| Name | Structure |
|---|---|
| Tra-ADC15 | 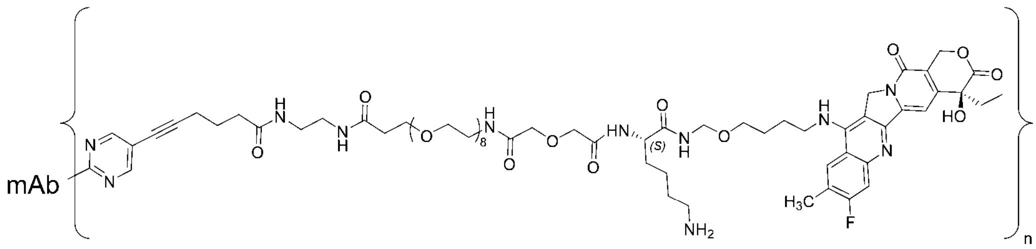 |
| Tra-ADC16 | 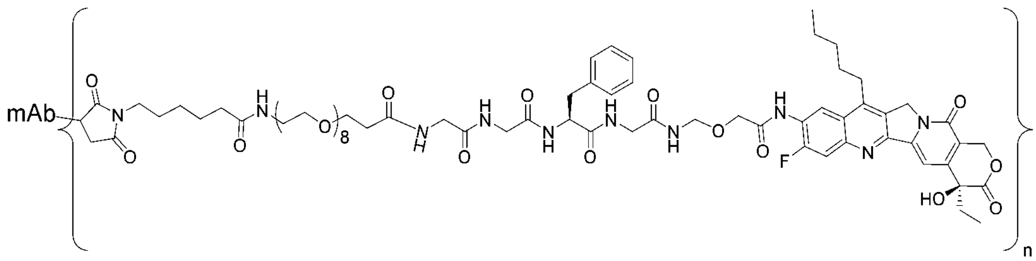 |
| Tra-ADC17 | 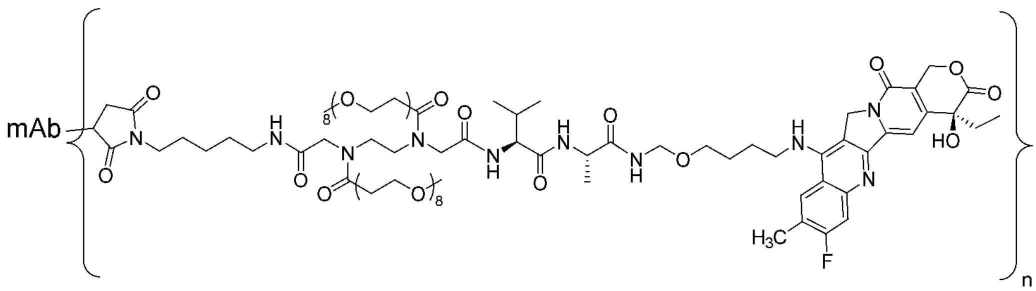 |
| Tra-ADC18 | 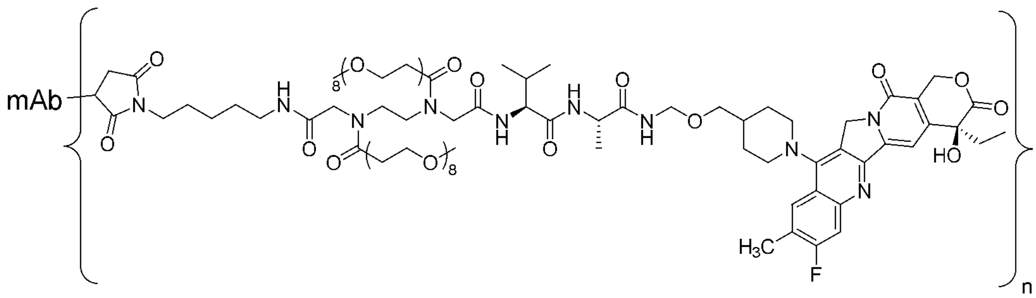 |
| Tra-ADC19 | 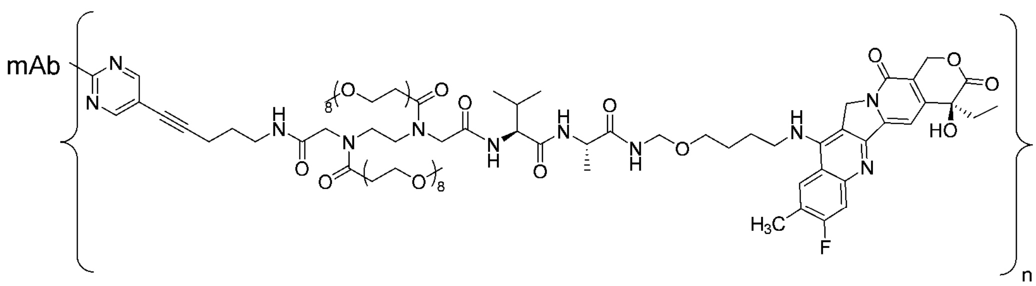 |

(continued)

| Name | Structure |
|---|---|
| Tra-ADC20 | 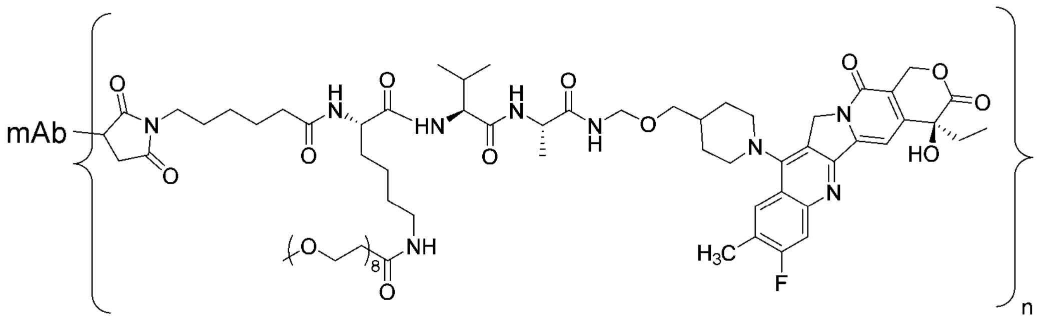 |

wherein, in Fa-ADCs, mAb represents farletuzumab; in Tra-ADCs, mAb represents trastuzumab, and n is 0, 1, 2, 3, 4, 5, 6, 7, or 8.

**23.** The antibody-drug conjugate according to any one of the preceding claims, or a pharmaceutically acceptable salt or ester, solvate, tautomer, stereoisomer, prodrug, or isotopically labeled form thereof, which has an average DAR selected from any value within the range from 1.0 to 10.0, preferably has an average DAR selected from any value within the range from 2.0 to 8.0, and more preferably has an average DAR selected from any value within the range from 6.0 to 8.0.

**24.** A compound of formula (II):

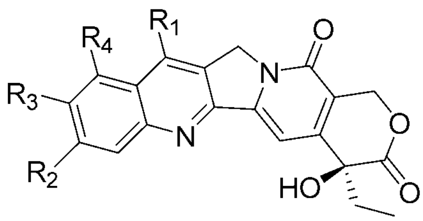

(II)

or a pharmaceutically acceptable salt or ester, solvate, tautomer, stereoisomer, prodrug, or isotopically labeled form thereof,
wherein,

$R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from the group consisting of halogen, CN, OH, SH and amino; $-(CH_2)_p-(C_{3-8}$ cycloalkyl) or $-(CH_2)_p-(C_{3-8}$ heterocycloalkyl), wherein the cycloalkyl and heterocycloalkyl are each optionally substituted with one or more substituents independently selected from the group consisting of halogen, CN, OH, SH, amino, $C_{1-6}$ alkyl, $C_{1-6}$ haloalkyl, $C_{1-6}$ hydroxyalkyl, $C_{1-6}$ cyanoalkyl and $C_{1-6}$ aminoalkyl; $-(CH_2)_p-(C_{1-6}$ alkoxy); $-(CH_2)_p-(C_{1-6}$ alkylthio); $-(CH_2)_p-NR_aR_b$; $-(CH_2)_p-CH=NR_a$; $-NH-CO-R_a$; $-NH-CO-(CH_2)_p-CH(OH)-R_a$; $-NH-(CH_2)_p-(CH=CH)-R_a$; or -COOH;

$R_2$ and $R_3$ are each independently halogen, cyano, $NO_2$, $C_{1-6}$ alkyl, $C_{1-6}$ haloalkyl, $C_{1-6}$ hydroxyalkyl, $C_{1-6}$ alkoxy, $-NH-CO-(C_{1-6}$ hydroxyalkyl), or $NR_aR_b$; or $R_2$ and $R_3$, together with the atoms to which they are attached, form a 4- to 8-membered heterocyclic group;

$R_4$ is H or $C_{3-8}$ cycloalkyl, preferably H;

$R_a$ and $R_b$ are each independently H, $C_{1-6}$ alkyl, $-CO-(C_{1-6}$ alkyl), $-SO-(C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino, and wherein the cycloalkyl is further optionally substituted with one or more substituents independently selected from the group consisting of $C_{1-6}$ alkyl, $C_{1-6}$ haloalkyl, $C_{1-6}$ hydroxyalkyl, $C_{1-6}$ cyanoalkyl and $C_{1-6}$ aminoalkyl; and

p and q are each independently 0, 1, 2, 3, 4, 5, or 6.

**25.** The compound according to claim 24, or a pharmaceutically acceptable salt or ester, solvate, tautomer, stereoisomer, prodrug, or isotopically labeled form thereof, wherein:

$R_1$ is: H; halogen; CN; OH; SH; $C_{1-6}$ alkyl; $C_{1-6}$ haloalkyl; $C_{1-6}$ hydroxyalkyl; $-(CH_2)_p-(C_{3-8}$ cycloalkyl) or $-(CH_2)_p-(C_{3-8}$ heterocycloalkyl), wherein the cycloalkyl and the heterocycloalkyl are each optionally substituted with one or more substituents independently selected from the group consisting of OH, SH, $C_{1-6}$ alkyl, and $C_{1-6}$ hydroxyalkyl; $-(CH_2)_p-(C_{1-6}$ alkoxy); $-(CH_2)_p-(C_{1-6}$ alkylthio); $-(CH_2)_p-NR_aR_b$; $-(CH_2)_p-CH=NR_a$; $-NH-CO-R_a$; $-NH-CO-(CH_2)_p-CH(OH)-R_a$; $-NH-(CH_2)_p-(CH=CH)-R_a$; or -COOH; wherein $R_a$ and $R_b$ are each independently H, $C_{1-6}$ alkyl, $C_{1-6}$ hydroxyalkyl, $C_{1-6}$ aminoalkyl, $-SO_2-(C_{1-6}$ alkyl), or $-(CH_2)_q-(C_{3-8}$ cycloalkyl optionally substituted with one or more substituents independently selected from the group consisting of hydroxy and $C_{1-6}$ hydroxyalkyl);

preferably, $R_1$ is H; $C_{1-6}$ alkyl; $C_{1-6}$ haloalkyl; $C_{1-6}$ hydroxyalkyl; $-(CH_2)_p-(C_{3-8}$ heterocycloalkyl), wherein the heterocycloalkyl is optionally substituted with one or more $C_{1-6}$ hydroxyalkyl; $-(CH_2)_p-(C_{1-6}$ alkoxy); $(CH_2)_p-(C_{1-6}$ alkylthio); $-(CH_2)_p-NH_2$; $-(CH_2)_p-NH(C_{1-6}$ alkyl); $-(CH_2)_p-NH(C_{1-6}$ hydroxyalkyl); $-(CH_2)_p-NH(C_{1-6}$ aminoalkyl); $-(CH_2)_p-NH-(CH_2)_q-(C_{3-8}$ cycloalkyl), wherein the cycloalkyl is optionally substituted with one or more substituents selected from $C_{1-6}$ hydroxyalkyl; $-(CH_2)_p-N(C_{1-6}$ alkyl)($-SO_2-C_{1-6}$ alkyl); $-CH=N(C_{1-6}$ alkyl); $-NH-CO-(C_{1-6}$ hydroxyalkyl); $-NH-CO-(C_{3-8}$ hydroxycycloalkyl); $-NH-CO-(CH_2)_p-CH(OH)-(C_{1-6}$ alkyl); $-NH-CO-(CH_2)_p-CH(OH)-(C_{3-8}$ cycloalkyl); $-NH-(CH_2)_p-(CH=CH)-(C_{1-6}$ hydroxyalkyl); or -COOH;

or

$R_1$ is: H; halogen; CN; OH; SH; $C_{1-6}$ alkyl, $C_{2-6}$ alkenyl, or $C_{2-6}$ alkynyl, each of which is optionally substituted with one or more substituents independently selected from the group consisting of halogen, CN, OH, SH and amino; $-(CH_2)_p-(C_{3-8}$ cycloalkyl), wherein the cycloalkyl is optionally substituted with one or more substituents independently selected from the group consisting of halogen, CN, OH, SH and amino; $-(CH_2)_p-(C_{1-6}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH; wherein $R_a$ and $R_b$ are each independently H, $C_{1-6}$ alkyl, $-SO-(C_{1-6}$ alkyl), $-SO_2-(C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino;

preferably, $R_1$ is: H; halogen; CN; OH; SH; $C_{1-6}$ alkyl; $C_{1-6}$ haloalkyl; $C_{1-6}$ hydroxyalkyl; $-(CH_2)_p-(C_{3-8}$ cycloalkyl); $-(CH_2)_p-(C_{3-8}$ hydroxycycloalkyl); $-(CH_2)_p-(C_{1-6}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH;

more preferably, $R_1$ is H; $C_{1-6}$ alkyl; $C_{1-6}$ haloalkyl; $C_{1-6}$ hydroxyalkyl; $-(CH_2)_p-(C_{1-6}$ alkoxy); $-(CH_2)_p-(C_{1-6}$ alkylthio); $-(CH_2)_p-NR_aR_b$; $-CH=NR_a$; $-NH-CO-R_a$; $-NH-CO-(CH_2)_p-CH(OH)-R_a$; or -COOH; preferably wherein $R_a$ and $R_b$ are each independently H, $C_{1-6}$ alkyl, $C_{1-6}$ hydroxyalkyl, $C_{1-6}$ aminoalkyl, $-SO_2-(C_{1-6}$ alkyl), $-(CH_2)_q-(C_{3-8}$ cycloalkyl), or $-(CH_2)_q-(C_{3-8}$ hydroxycycloalkyl);

further more preferably, $R_1$ is H; $C_{1-6}$ alkyl; $C_{1-6}$ haloalkyl; $C_{1-6}$ hydroxyalkyl; $-(CH_2)_p-(C_{1-6}$ alkoxy); $-(CH_2)_p-(C_{1-6}$ alkylthio); $-(CH_2)_p-NH_2$; $-(CH_2)_p-NH(C_{1-6}$ alkyl); $-(CH_2)_p-NH(C_{1-6}$ hydroxyalkyl); $-(CH_2)_p-NH(C_{1-6}$ aminoalkyl); $-(CH_2)_p-NH-(CH_2)_q-(C_{3-8}$ cycloalkyl); $-(CH_2)_p-N(C_{1-6}$ alkyl)($-SO_2-C_{1-6}$ alkyl); $-CH=N(C_{1-6}$ alkyl); $-NH-CO-(C_{1-6}$ hydroxyalkyl); $-NH-CO-(C_{3-8}$ hydroxycycloalkyl); $-NH-CO-(CH_2)_p-CH(OH)-(C_{1-6}$ alkyl); $-NH-CO-(CH_2)_p-CH(OH)-(C_{3-8}$ cycloalkyl); or -COOH.

**26.** The compound according to claim 24, or a pharmaceutically acceptable salt or ester, solvate, tautomer, stereoisomer, prodrug, or isotopically labeled form thereof, wherein $R_1$ is H, $C_{1-6}$ alkyl, $-(CH_2)_p-(C_{1-6}$ alkylthio), $-(CH_2)_p-(NH_2)$, $-(CH_2)_p-NH(C_{1-6}$ alkyl), $-(CH_2)_p-NH(C_{1-6}$ hydroxyalkyl), $-(CH_2)_p-NH-(CH_2)_q-(C_{3-8}$ cycloalkyl), or $-NH-CO-(CH_2)_p-CH(OH)-(C_{3-8}$ cycloalkyl);

preferably, $R_1$ is H, $C_{1-6}$ alkyl, $-(CH_2)_p-(C_{1-6}$ alkylthio), $-NH_2$, $-(CH_2)_p-NH(C_{1-6}$ alkyl), $-NH(C_{1-6}$ hydroxyalkyl), $-(CH_2)_p-NH-(CH_2)_q-(C_{3-8}$ cycloalkyl), or $-NH-CO-CH(OH)-(C_{3-8}$ cycloalkyl);

more preferably, $R_1$ is H, $C_{1-6}$ alkyl, $-CH_2-(C_{1-6}$ alkylthio), $-NH_2$, $-CH_2-NH(C_{1-6}$ alkyl), $-NH(C_{1-6}$ hydroxyalkyl), $-CH_2-NH-CH_2-(C_{3-8}$ cycloalkyl), or $-NH-CO-CH(OH)-(C_{3-8}$ cycloalkyl).

**27.** The compound according to claim 24, or a pharmaceutically acceptable salt or ester, solvate, tautomer, stereoisomer, prodrug, or isotopically labeled form thereof, wherein $R_1$ is $C_{1-6}$ alkyl, $-(CH_2)_p-(C_{1-6}$ alkylthio), $-(CH_2)_p-NH(C_{1-6}$ alkyl), or $-(CH_2)_p-NH(C_{1-6}$ hydroxyalkyl); preferably, $R_1$ is $C_{1-6}$ alkyl, $-CH_2-(C_{1-6}$ alkylthio), $-NH(C_{1-6}$ alkyl), or $-NH(C_{1-6}$ hydroxyalkyl).

**28.** The compound according to any one of claims 24-27, or a pharmaceutically acceptable salt or ester, solvate, tautomer, stereoisomer, prodrug, or isotopically labeled form thereof, wherein $R_2$ is a halogen, preferably fluorine.

**29.** The compound according to any one of claims 24-28, or a pharmaceutically acceptable salt or ester, solvate, tautomer, stereoisomer, prodrug, or isotopically labeled form thereof, wherein $R_3$ is $C_{1-6}$ alkyl, $C_{1-6}$ alkoxy, halogen, cyano, $-NH_2$, -NH-CO-($C_{1-6}$ hydroxyalkyl), or $NO_2$; preferably, $R_3$ is $C_{1-6}$ alkyl, halogen, $-NH_2$, -NH-CO-($C_{1-6}$ hydroxyalkyl), or $NO_2$; or, $R_3$ is halogen, cyano, $NO_2$, $C_{1-6}$ alkyl, $C_{1-6}$ haloalkyl, $C_{1-6}$ hydroxyalkyl, $C_{1-6}$ alkoxy, or $NR_aR_b$, wherein $R_a$ and $R_b$ are each independently H, $C_{1-6}$ alkyl, -SO-($C_{1-6}$ alkyl), $-SO_2$-($C_{1-6}$ alkyl), or $-(CH_2)_q-(C_{3-8}$ cycloalkyl), wherein the alkyl and the cycloalkyl are each optionally substituted with one or more substituents independently selected from the group consisting of halogen, CN, OH, SH and amino; preferably, $R_3$ is $C_{1-6}$ alkyl, $C_{1-6}$ alkoxy, halogen, cyano, $-NH_2$ or $NO_2$; more preferably, $R_3$ is $C_{1-6}$ alkyl, halogen, $-NH_2$ or $NO_2$.

**30.** The compound according to any one of claims 24-27, or a pharmaceutically acceptable salt or ester, solvate, tautomer, stereoisomer, prodrug, or isotopically labeled form thereof, wherein $R_2$ and $R_3$, together with the atoms to which they are attached, form a 4- to 8-membered heterocyclic group containing an oxygen, nitrogen or sulfur atom as a heteroatom, such as dioxolane.

**31.** The compound according to claim 24, or a pharmaceutically acceptable salt or ester, solvate, tautomer, stereoisomer, prodrug, or isotopically labeled form thereof, wherein the compound is selected from the group consisting of:

| No. | Structure | No. | Structure |
|---|---|---|---|
| 1 | 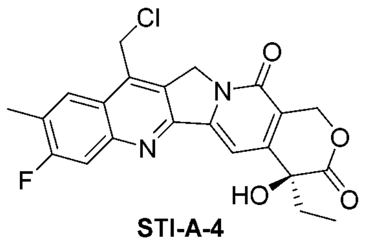 **STI-A-4** | 2 | 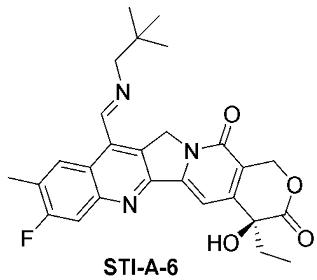 **STI-A-6** |
| 3 | 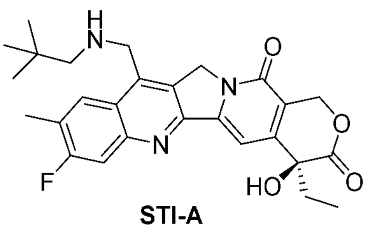 **STI-A** | 4 | 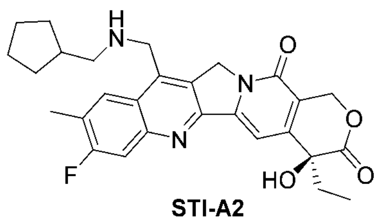 **STI-A2** |
| 5 | 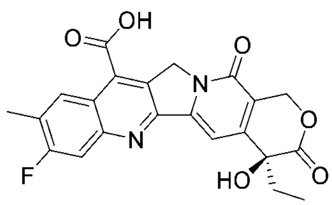 **STI-F-01** | 6 | 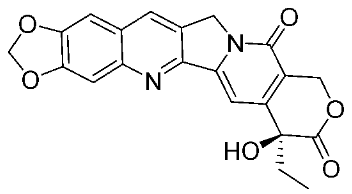 **STI-E05** |
| 7 | 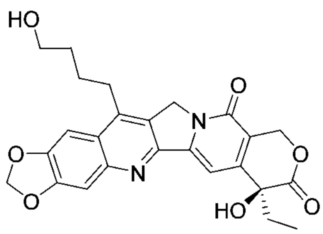 **STI-E** | 8 | 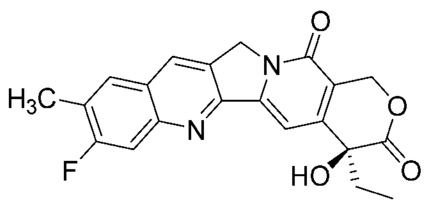 **STI-F-8** |

(continued)

| No. | Structure | No. | Structure |
|---|---|---|---|
| 9 | 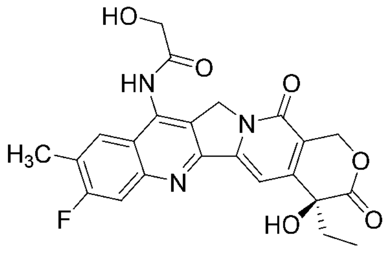 STI-F2 | 10 | 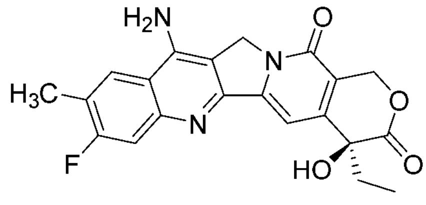 STI-F-03c-1 |
| 11 | 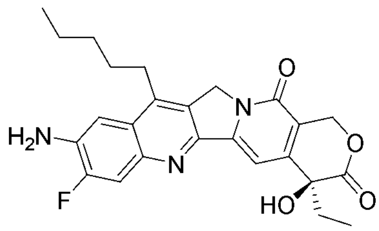 STI-G1 | 12 | 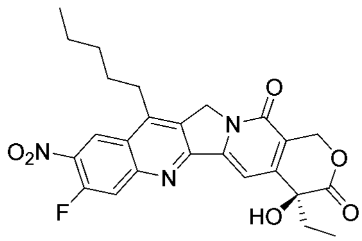 STI-G03 |
| 13 | 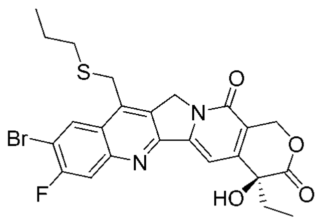 STI-G2-06c | 14 | 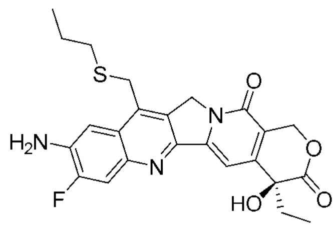 STI-G2 |
| 15 | 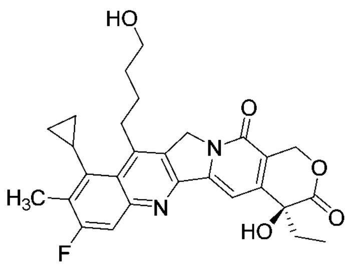 STI-D | 16 | 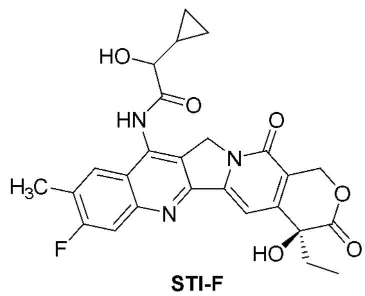 STI-F |
| 17 | 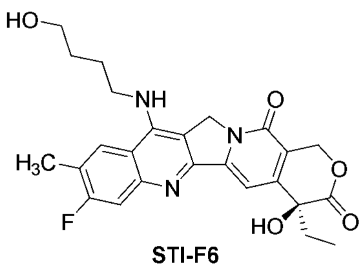 STI-F6 | 18 | 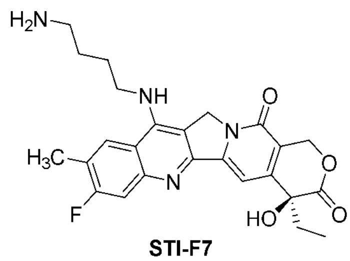 STI-F7 |

(continued)

| No. | Structure | No. | Structure |
|---|---|---|---|
| 19 | 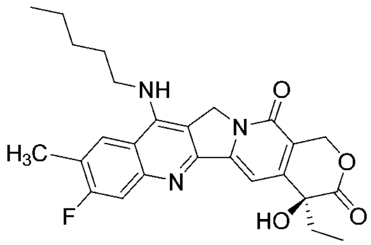 **STI-F13** | 20 | 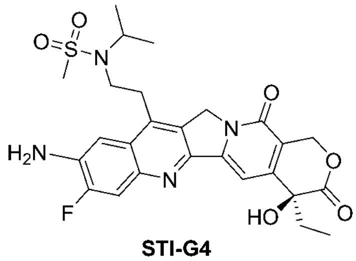 **STI-G4** |
| 21 | 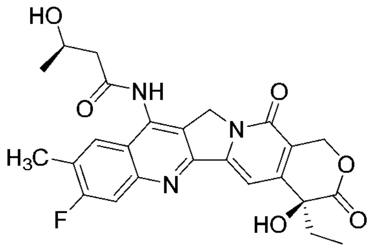 **STI-F12R** | 22 | 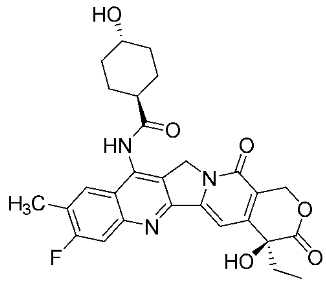 **STI-F10** |
| P1 | 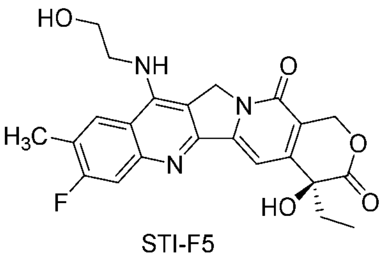 STI-F5 | P2 | 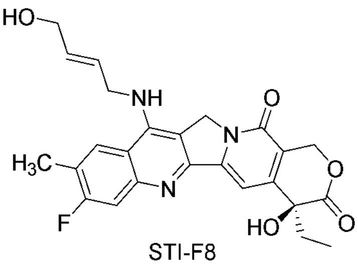 STI-F8 |
| P3 | 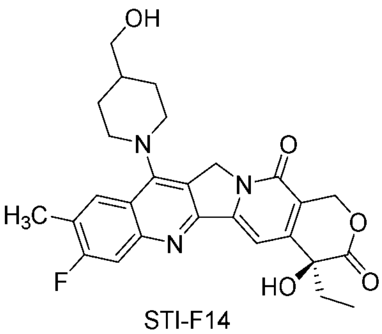 STI-F14 | P4 | 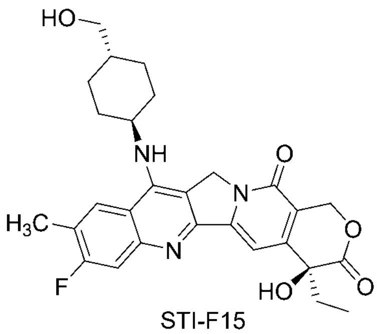 STI-F15 |
| P5 | 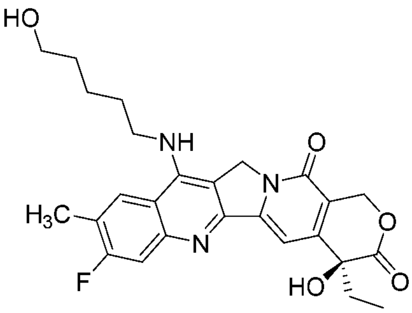 STI-F17 | P6 | 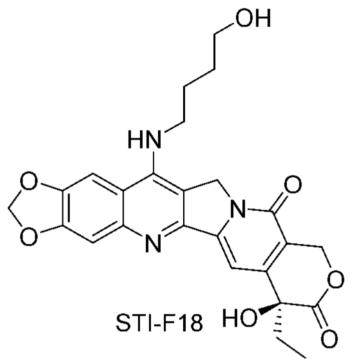 STI-F18 |

(continued)

| No. | Structure | No. | Structure |
|---|---|---|---|
| P7 | 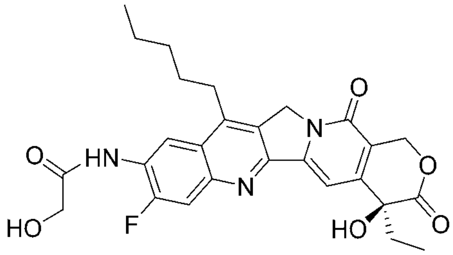 STI-G5 | | |

**32.** A linker-payload conjugate of formula (III):

$$Lg\text{-}L_1\text{-}L_2\text{-}L_3\text{-}L_4\text{-}D \qquad (III)$$

or a pharmaceutically acceptable salt or ester, solvate, tautomer, stereoisomer, prodrug, or isotopically labeled form thereof, wherein $L_1$, $L_2$, $L_3$, $L_4$ and D are as defined in any one of claims 1-20, and wherein

when $L_1$ is

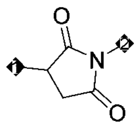 ,

Lg and $L_1$ together form

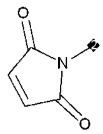 ;

when $L_1$ is not

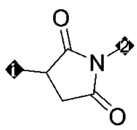 ,

Lg is a leaving group; preferably, Lg is halogen, sulfonyl (e.g., methanesulfonyl, p-toluenesulfonyl), sulfonyloxy (e.g., methanesulfonyloxy, $CF_3SO_3$-, p-toluenesulfonyloxy), a tertiary-ammonium group (e.g., $Me_3N^+$ or $Et_3N^+$), or a diazonium group; more preferably, Lg is F, Cl, Br, or methanesulfonyl; especially preferably, Lg is methanesulfonyl.

**33.** The linker-payload conjugate according to claim 32, or a pharmaceutically acceptable salt or ester, solvate, tautomer, stereoisomer, prodrug, or isotopically labeled form thereof, wherein the linker-payload conjugate is selected from the group consisting of:

➽Too large Table

| No. | Structure |
|---|---|
| 23 | 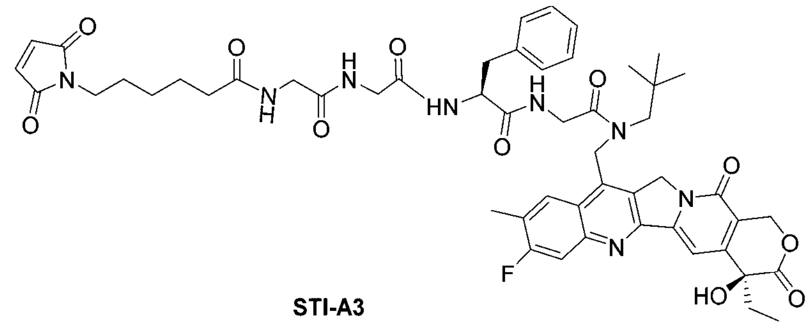 STI-A3 |
| 24 | 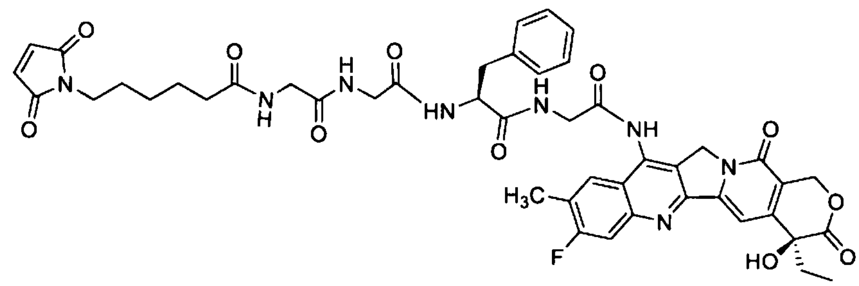 STI-F3 |
| 25 | 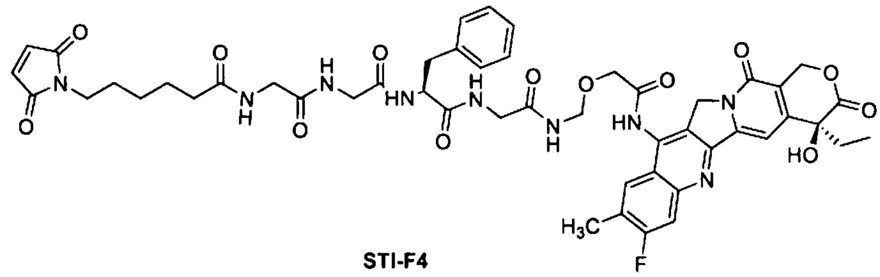 STI-F4 |
| 26 | 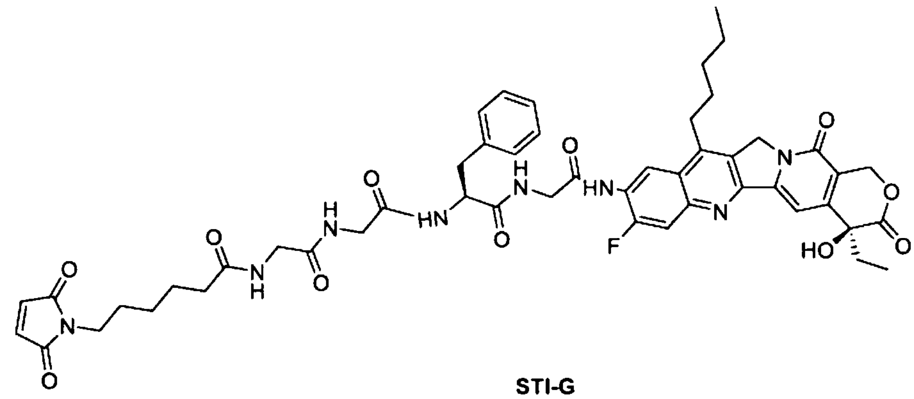 STI-G |

(continued)

| No. | Structure |
|---|---|
| 27 | 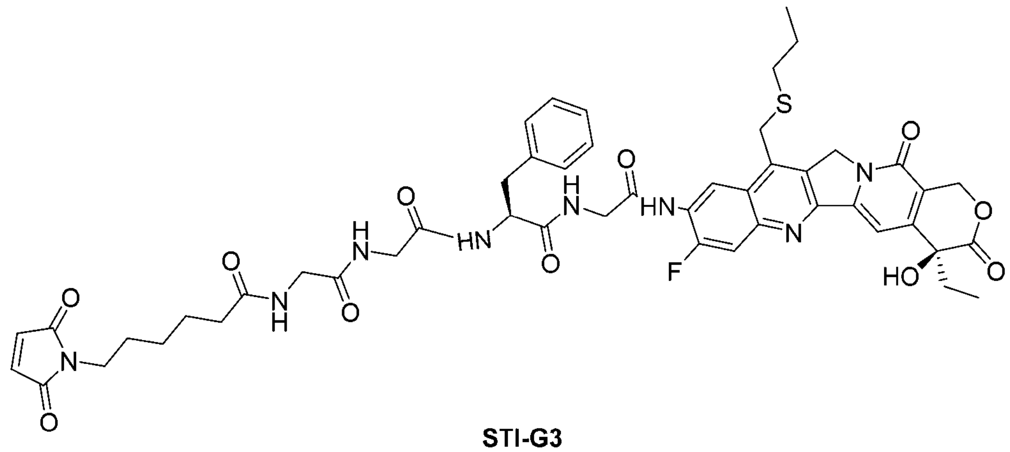 **STI-G3** |
| 28 | 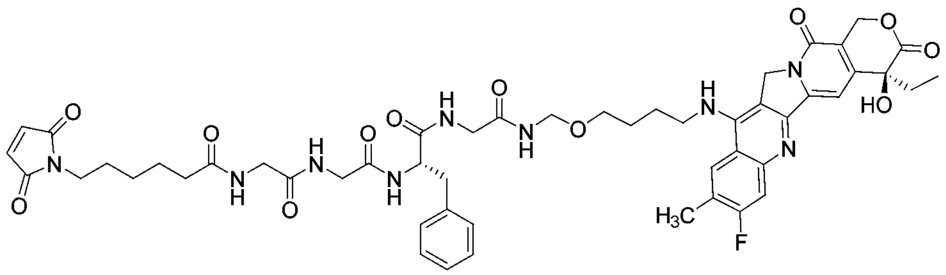 STI-F1002 |
| 29 | 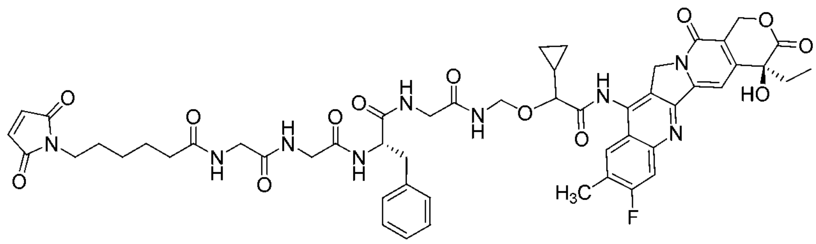 STI-F1001 |
| 30 | 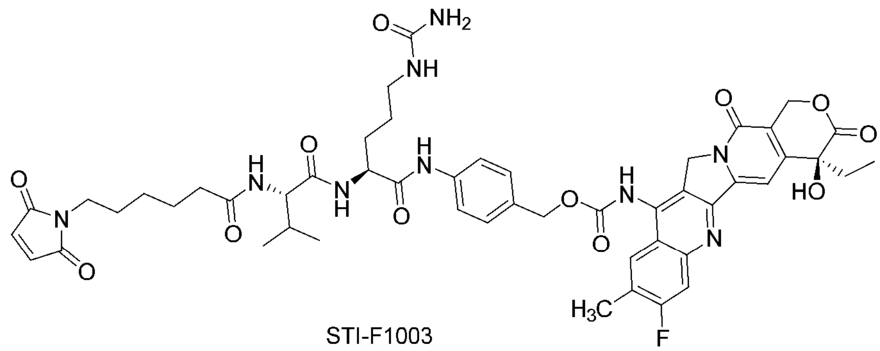 STI-F1003 |

(continued)

| No. | Structure |
|---|---|
| 31 | 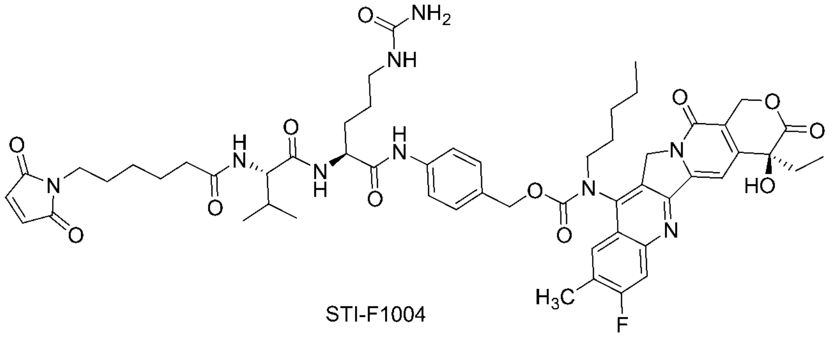 STI-F1004 |
| 32 | 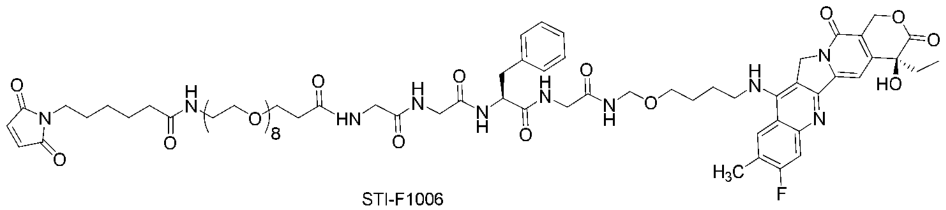 STI-F1006 |
| 33 | 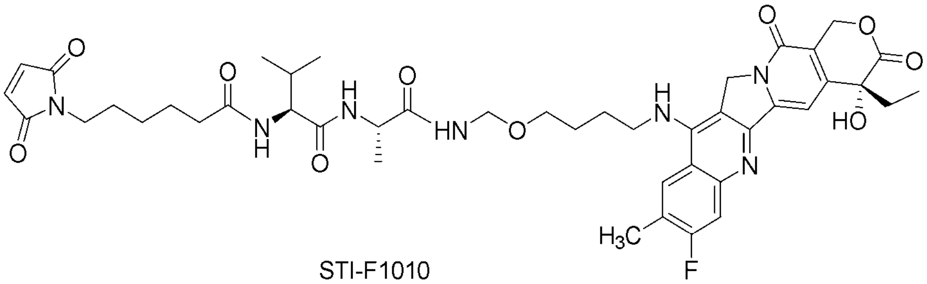 STI-F1010 |
| 34 | 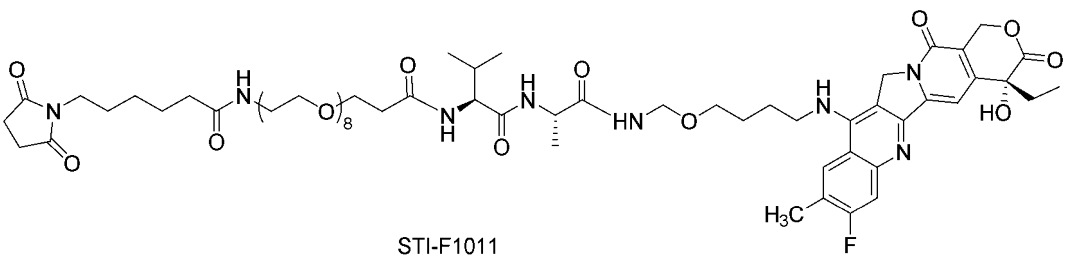 STI-F1011 |
| 35 | 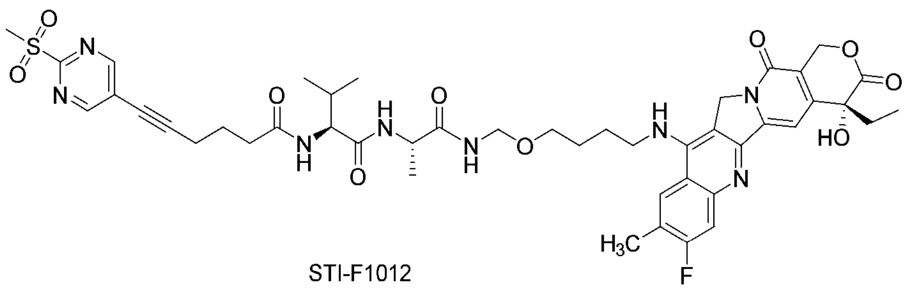 STI-F1012 |

(continued)

| No. | Structure |
|---|---|
| 36 | 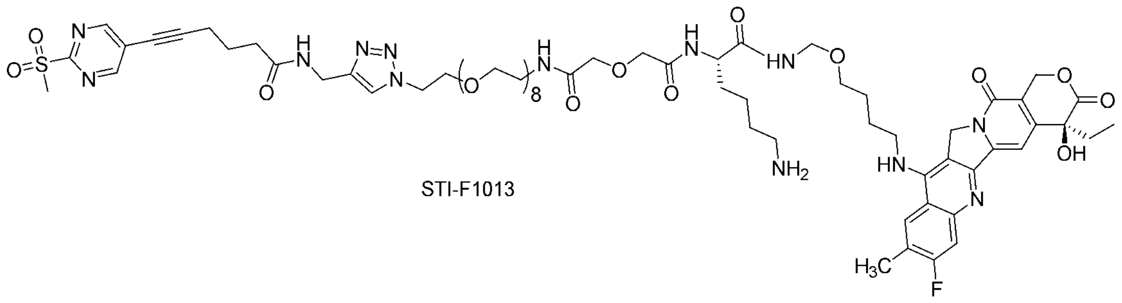 STI-F1013 |
| 37 | 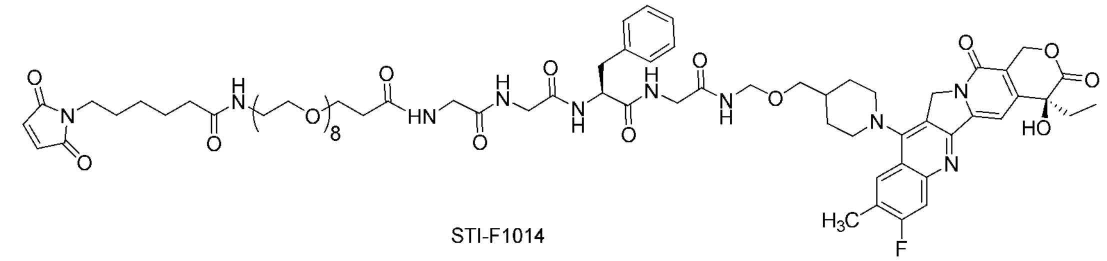 STI-F1014 |
| 38 | 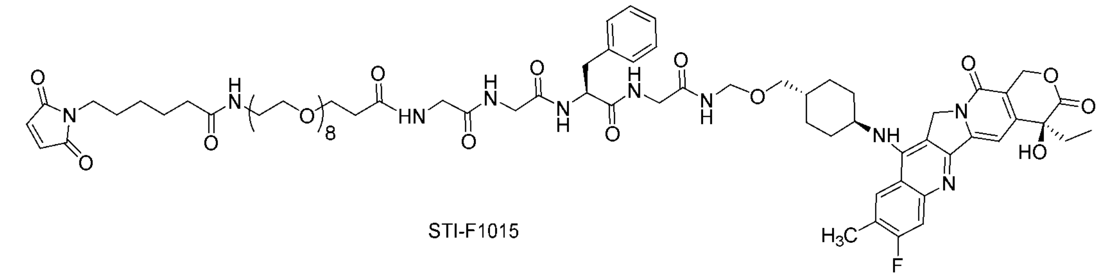 STI-F1015 |
| 39 | 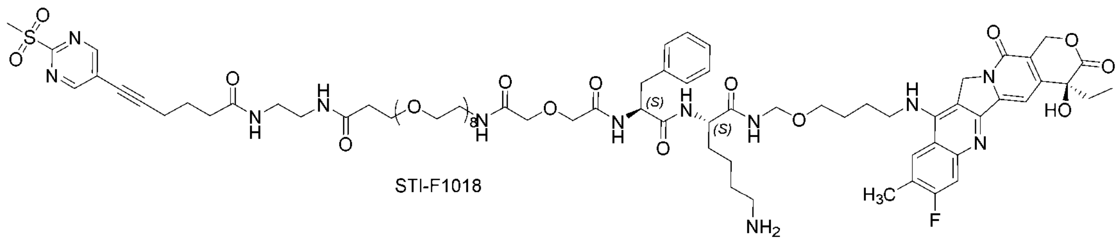 STI-F1018 |
| 40 | 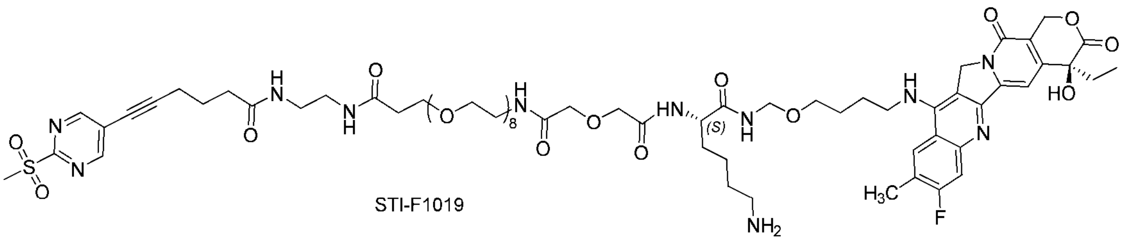 STI-F1019 |
| 41 | 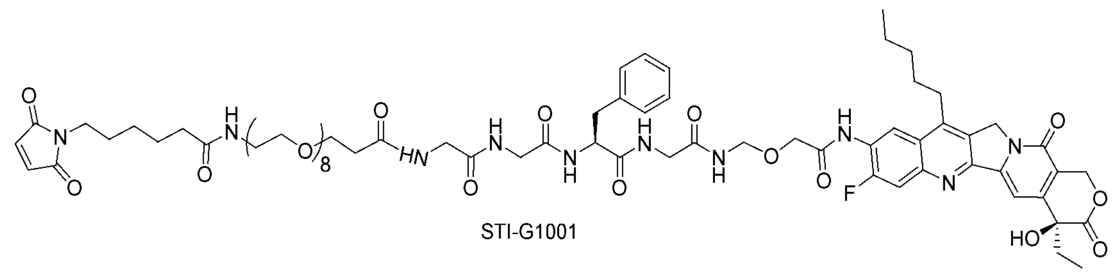 STI-G1001 |

(continued)

| No. | Structure |
|---|---|
| 42 | 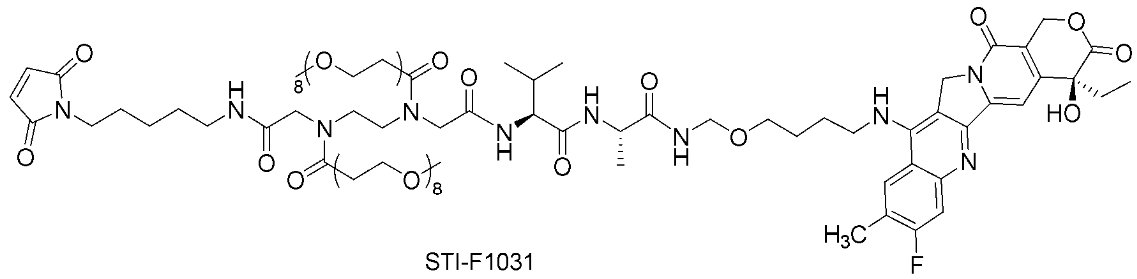 STI-F1031 |
| 43 | 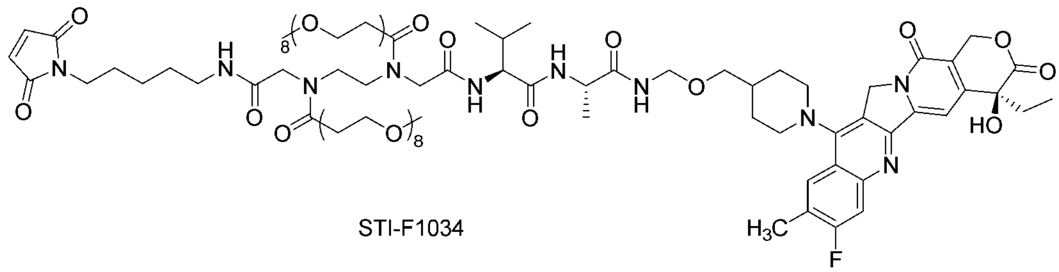 STI-F1034 |
| 44 | 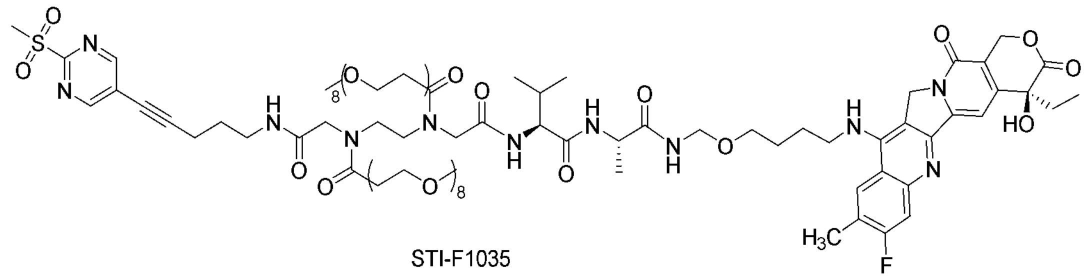 STI-F1035 |
| 45 | 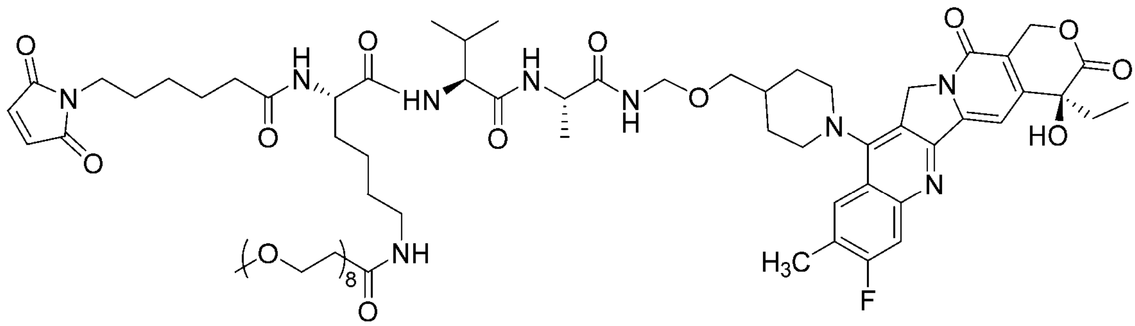 STI-F1043 . |

**34.** A pharmaceutical composition comprising the antibody-drug conjugate according to any one of claims 1-23, or a pharmaceutically acceptable salt or ester, solvate, tautomer, stereoisomer, prodrug, or isotopically labeled form thereof; or the compound according to any one of claims 21-31, or a pharmaceutically acceptable salt or ester, solvate, tautomer, stereoisomer, prodrug, or isotopically labeled form thereof; or the linker-payload conjugate according to any one of claims 32-33, or a pharmaceutically acceptable salt or ester, solvate, tautomer, stereoisomer, prodrug, or isotopically labeled form thereof; together with a pharmaceutically acceptable carrier, diluent or excipient.

**35.** The antibody-drug conjugate according to any one of claims 1-23, or a pharmaceutically acceptable salt or ester, solvate, tautomer, stereoisomer, prodrug, or isotopically labeled form thereof; or the compound according to any one of claims 21-31, or a pharmaceutically acceptable salt or ester, solvate, tautomer, stereoisomer, prodrug, or isotopically labeled form thereof; or the linker-payload conjugate according to any one of claims 32-33, or a pharmaceutically acceptable salt or ester, solvate, tautomer, stereoisomer, prodrug, or isotopically labeled form thereof, for use in the treatment or prevention of cancer.

**36.** A method for preventing or treating cancer in a patient, comprising administering to the patient a therapeutically effective amount of the antibody-drug conjugate according to any one of claims 1-23, or a pharmaceutically acceptable salt or ester, solvate, tautomer, stereoisomer, prodrug, or isotopically labeled form thereof; or the compound according to any one of claims 21-31, or a pharmaceutically acceptable salt or ester, solvate, tautomer, stereoisomer, prodrug, or isotopically labeled form thereof; or the linker-payload conjugate according to any one of claims 32-33, or a pharmaceutically acceptable salt or ester, solvate, tautomer, stereoisomer, prodrug, or isotopically labeled form thereof.

**37.** Use of the antibody-drug conjugate according to any one of claims 1-23, or a pharmaceutically acceptable salt or ester, solvate, tautomer, stereoisomer, prodrug, or isotopically labeled form thereof; or of the compound according to any one of claims 21-31, or a pharmaceutically acceptable salt or ester, solvate, tautomer, stereoisomer, prodrug, or isotopically labeled form thereof; or of the linker-payload conjugate according to any one of claims 32-33, or a pharmaceutically acceptable salt or ester, solvate, tautomer, stereoisomer, prodrug, or isotopically labeled form thereof, in the manufacture of a medicament for the treatment or prevention of cancer.

**38.** The antibody-drug conjugate or compound or linker-payload conjugate according to claim 35, or a pharmaceutically acceptable salt or ester, solvate, tautomer, stereoisomer, prodrug, or isotopically labeled form thereof, or the method according to claim 36, or the use according to claim 37, wherein the cancer is a solid tumor, particularly lung cancer (e.g., small cell lung cancer, non-small cell lung cancer), liver cancer, digestive system cancers (e.g., intestinal cancer, gastric cancer, cardia cancer, esophageal cancer, appendiceal adenocarcinoma, colon cancer, rectal cancer, colorectal cancer, pancreatic cancer), bladder cancer, melanoma, breast cancer, ovarian cancer, cervical cancer, endometrial cancer, prostate cancer, basal cell carcinoma, cholangiocarcinoma, squamous cell carcinoma, thyroid cancer, brain cancer, head and neck cancer, peritoneal cancer, kidney cancer, urothelial carcinoma, testicular cancer, central nervous system tumors (e.g., glioma, glioblastoma such as glioblastoma multiforme, glioma, or sarcoma), choriocarcinoma, oral epidermoid carcinoma; or hematological malignancies, particularly leukemias (e.g., acute or chronic myeloid leukemia, acute or chronic granulocytic leukemia), lymphomas (e.g., Hodgkin's lymphoma, non-Hodgkin's lymphoma, diffuse large B-cell lymphoma, acute B-cell lymphoma, follicular lymphoma), multiple myeloma; preferably, the cancer is ovarian cancer, colon cancer, lung adenocarcinoma, or oral epidermoid carcinoma.

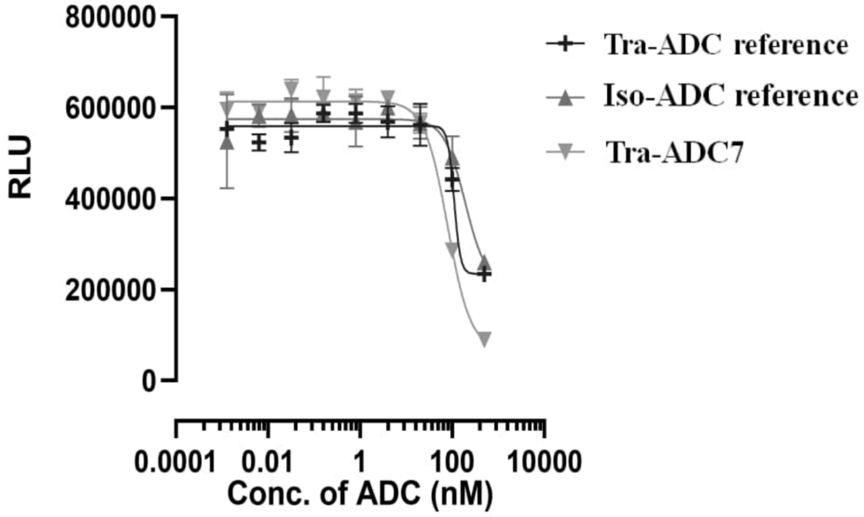


| | IC50 |
|---|---|
| Tra-ADC reference | ~109.7 |
| Iso-ADC reference | 190.5 |
| Tra-ADC7 | 79.01 |

Fig. 1a

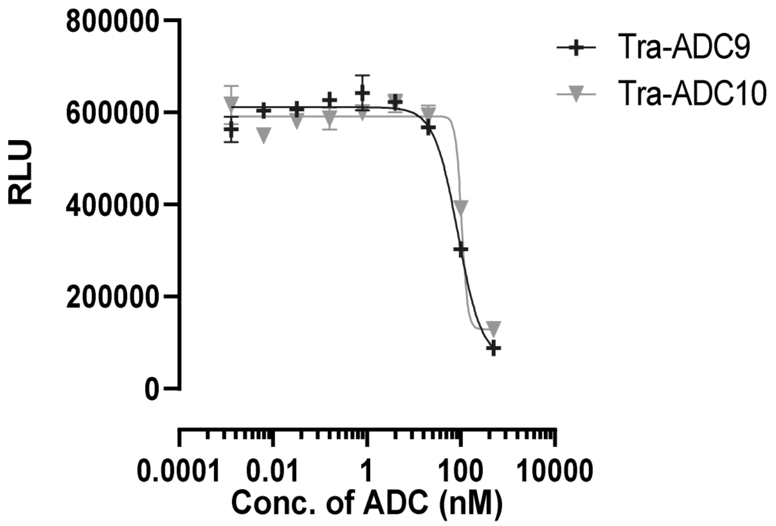


| | IC50 |
|---|---|
| Tra-ADC9 | 86.02 |
| Tra-ADC10 | ~ 103.9 |

Fig. 1b

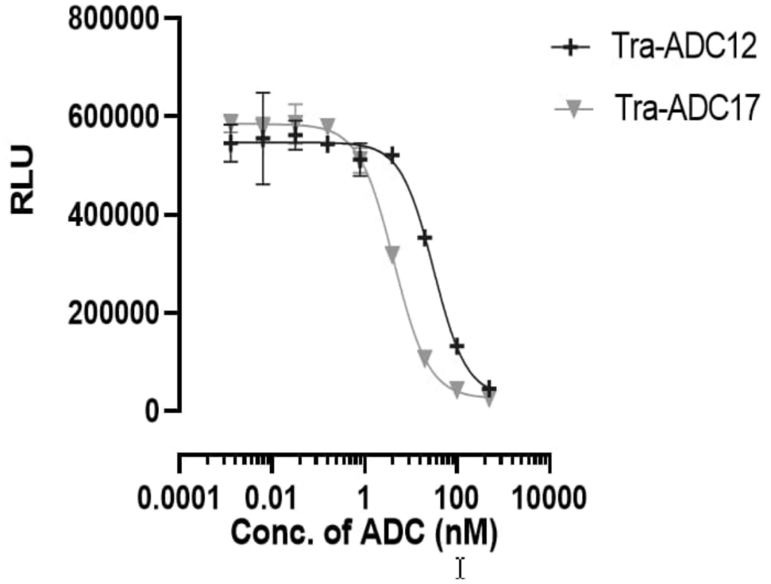


| | IC50 |
|---|---|
| Tra-ADC12 | 31.15 |
| Her2-F1031 | 4.297 |

Fig. 1c

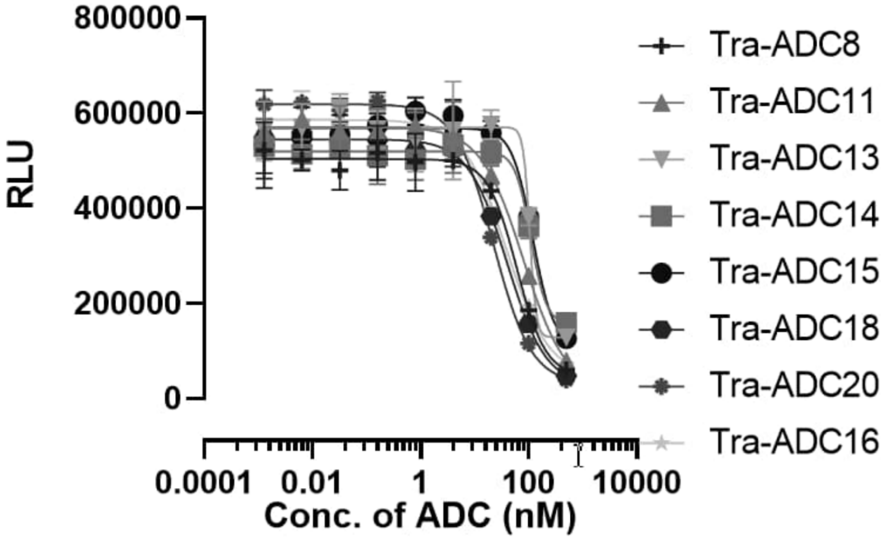


| | IC50 |
|---|---|
| Tra-ADC8 | 59.91 |
| Tra-ADC11 | 79.41 |
| Tra-ADC13 | ~ 104.3 |
| Tra-ADC14 | 109.3 |
| Tra-ADC15 | 118.3 |
| Tra-ADC18 | 39.93 |
| Tra-ADC20 | 22.18 |
| Tra-ADC16 | 38.07 |

Fig. 1d

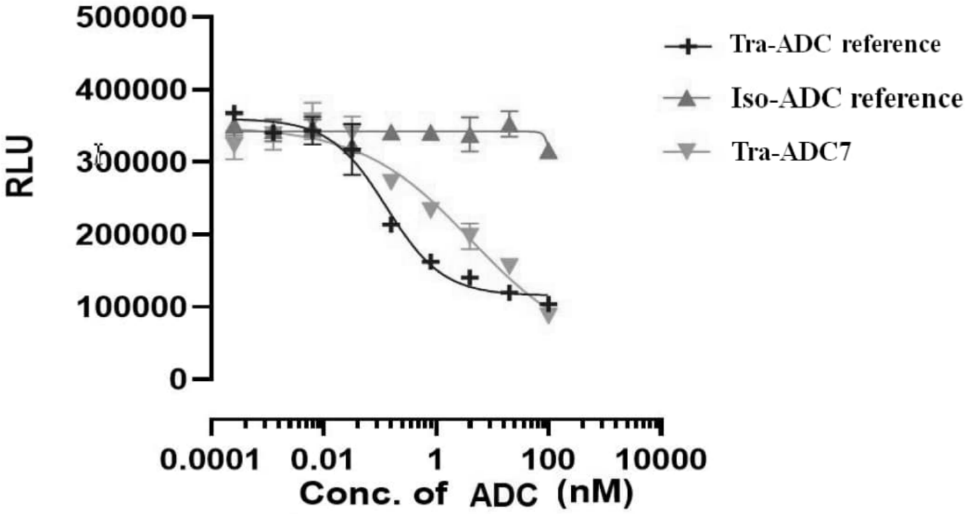


| | IC50 |
|---|---|
| Tra-ADC reference | 0.1309 |
| Iso-ADC reference | 7374 |
| Tra-ADC7 | 4.189 |

Fig. 2a

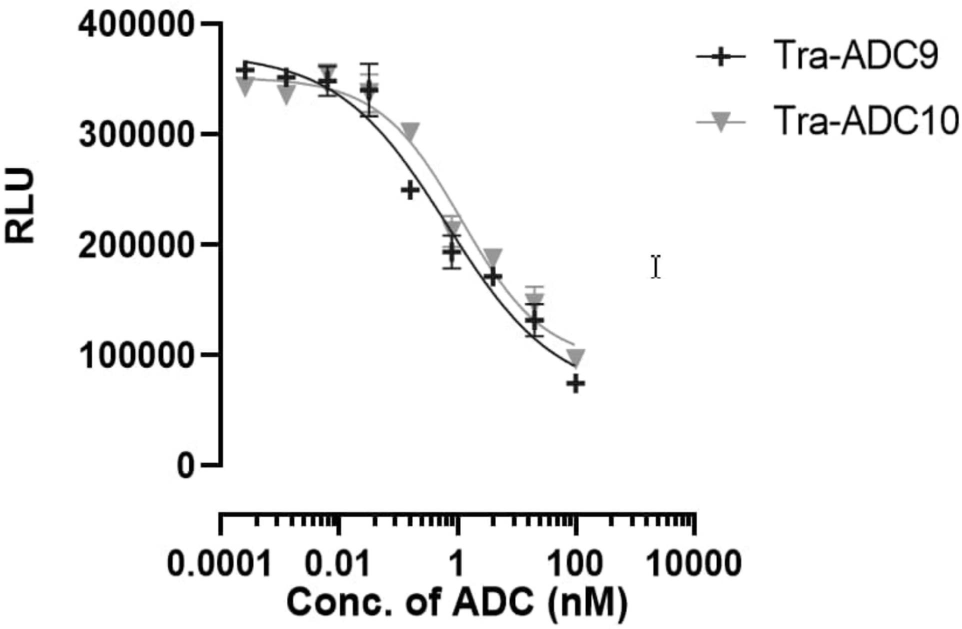


| | IC50 |
|---|---|
| Tra-ADC9 | 0.6644 |
| Tra-ADC10 | 1.166 |

Fig. 2b

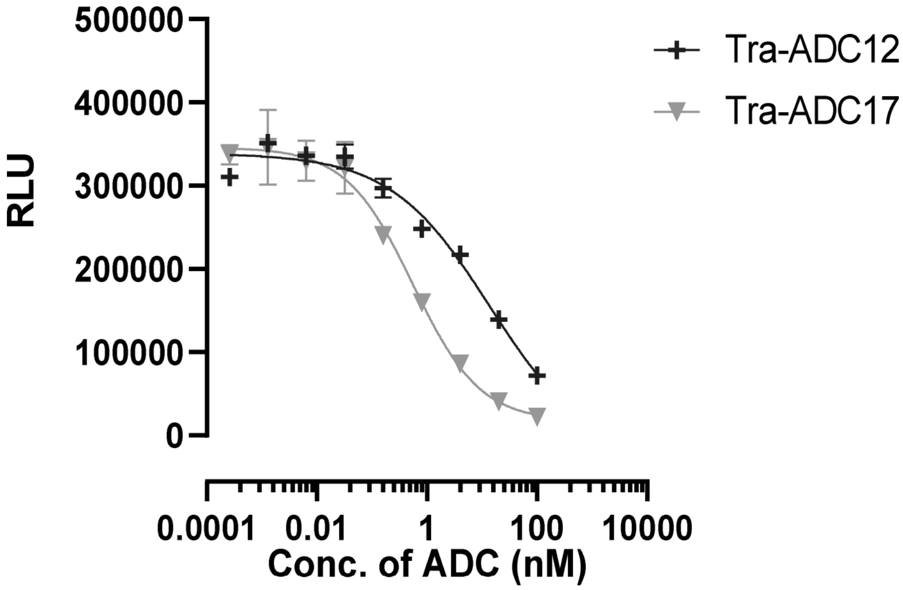


| | IC50 |
|---|---|
| Tra-ADC12 | 13.82 |
| Tra-ADC17 | 0.5519 |

Fig. 2c

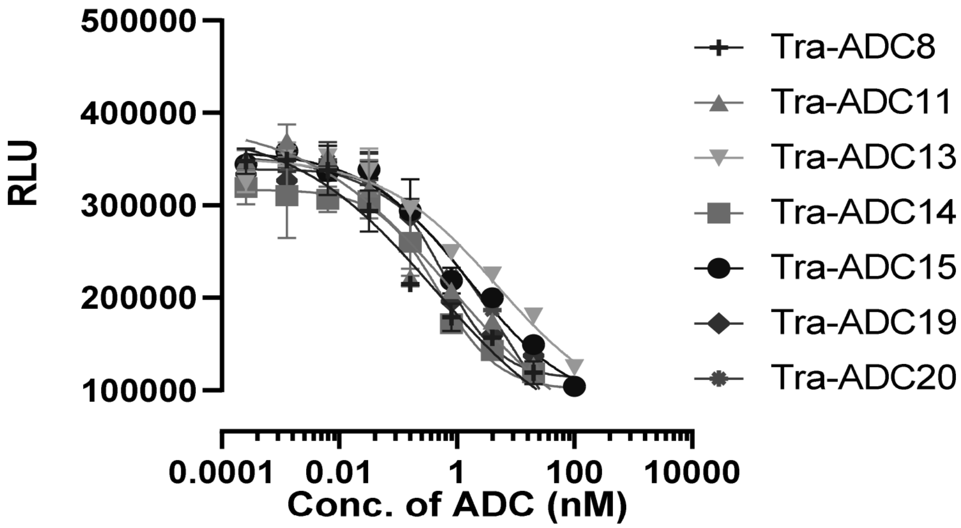


| | IC50 |
|---|---|
| Tra-ADC8 | 0.4230 |
| Tra-ADC11 | 0.8583 |
| Tra-ADC13 | 4.907 |
| Tra-ADC14 | 0.4796 |
| Tra-ADC15 | 1.572 |
| Tra-ADC19 | 0.5944 |
| Tra-ADC20 | 8.473 |

Fig. 2d

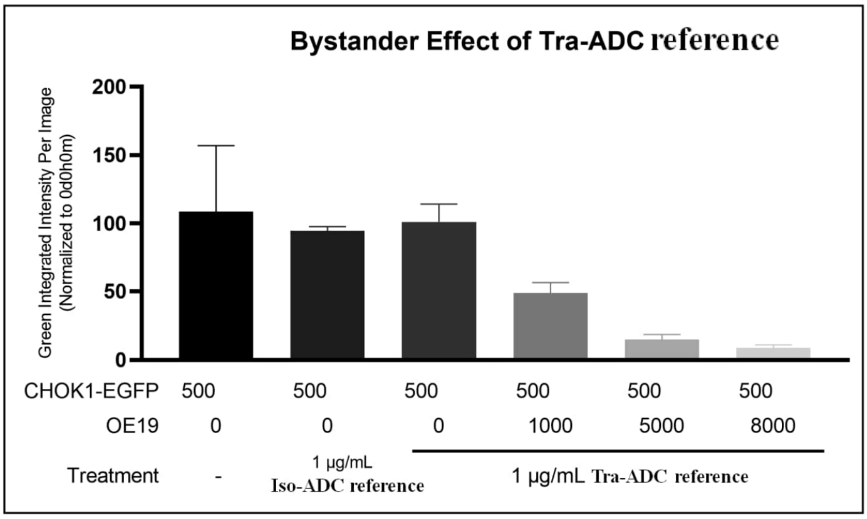


Fig. 3a

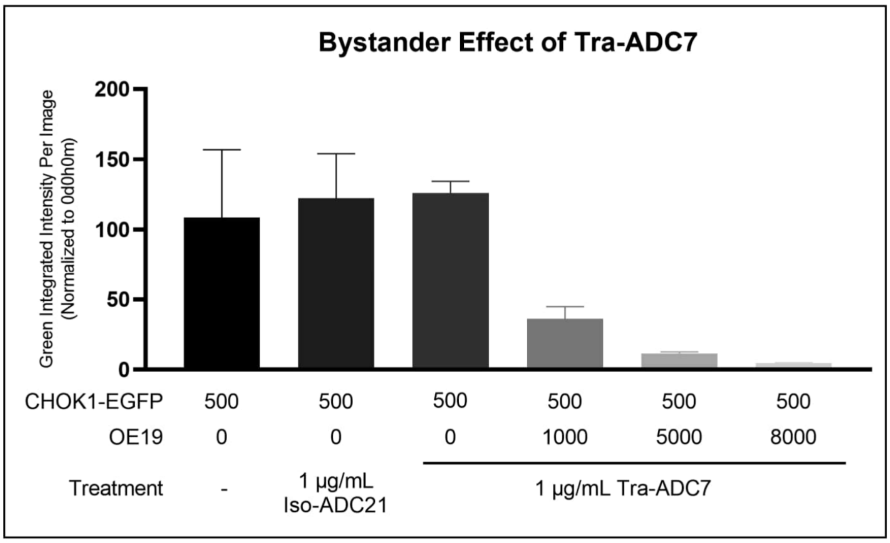


Fig. 3b

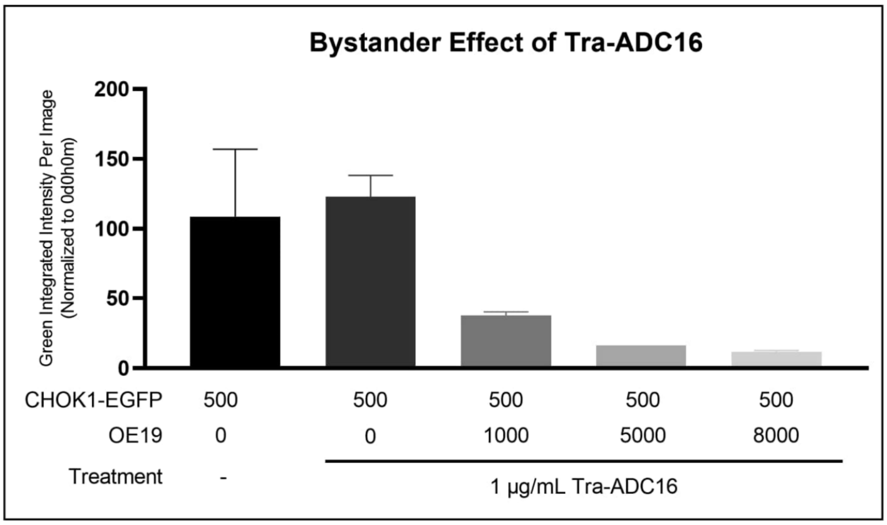


Fig. 3c

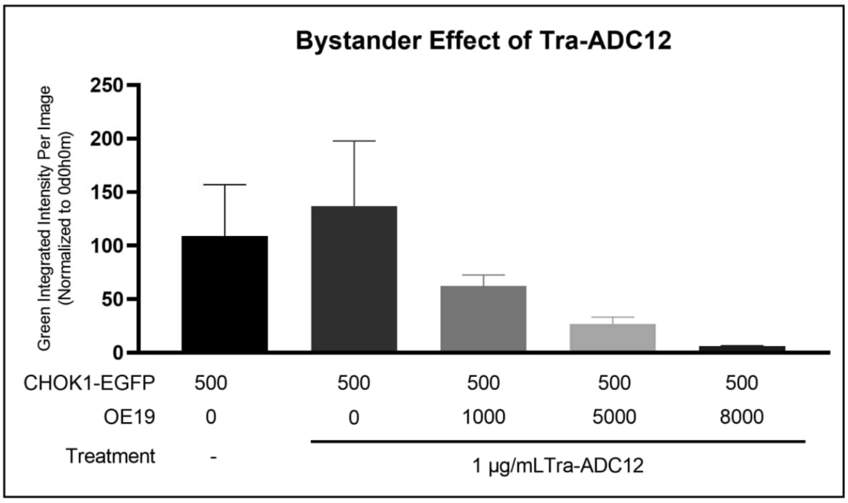
Bystander Effect of Tra-ADC12
Green Integrated Intensity Per Image (Normalized to 0d0h0m)
250
200
150
100
50
0
CHOK1-EGFP 500 500 500 500 500
OE19 0 0 1000 5000 8000
Treatment -
1 µg/mLTra-ADC12

Fig. 3d

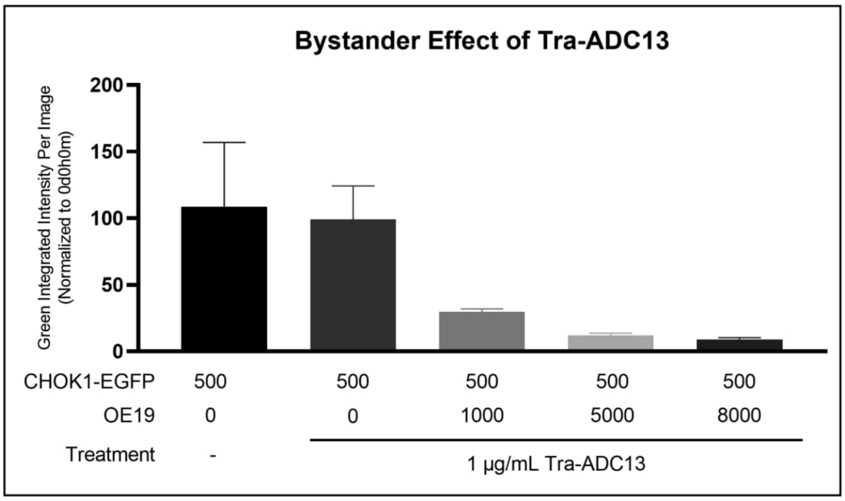
Bystander Effect of Tra-ADC13
Green Integrated Intensity Per Image (Normalized to 0d0h0m)
200
150
100
50
0
CHOK1-EGFP 500 500 500 500 500
OE19 0 0 1000 5000 8000
Treatment -
1 µg/mL Tra-ADC13

Fig. 3e

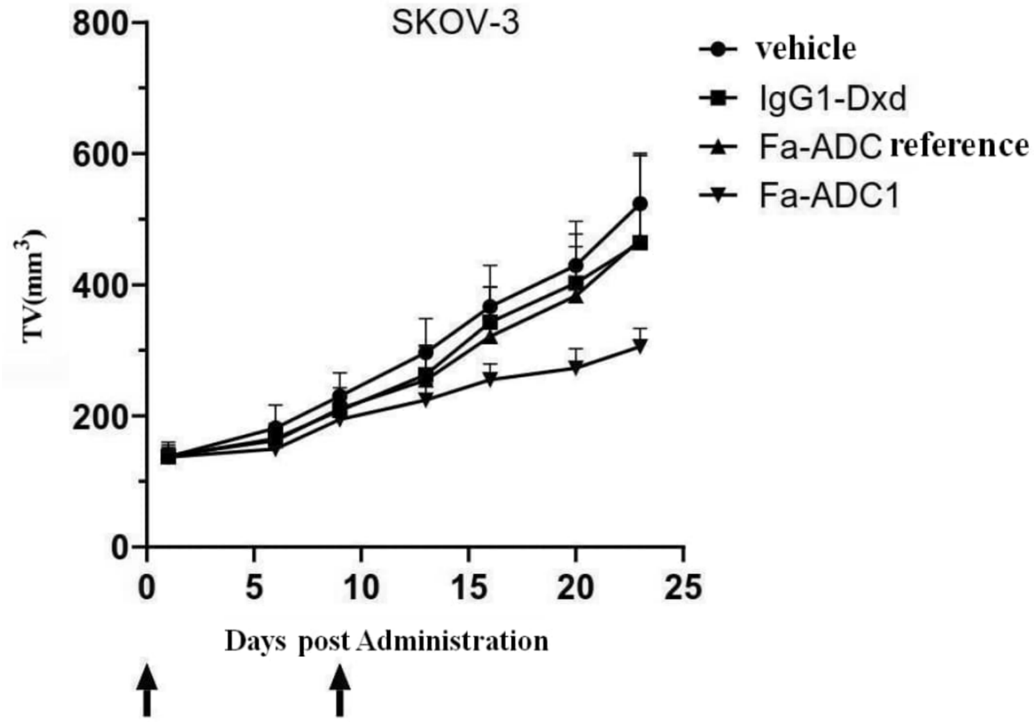
SKOV-3
800
600
400
200
0
TV(mm3)
0
5
10
15
20
25
Days post Administration
vehicle
IgG1-Dxd
Fa-ADC reference
Fa-ADC1

Fig. 4

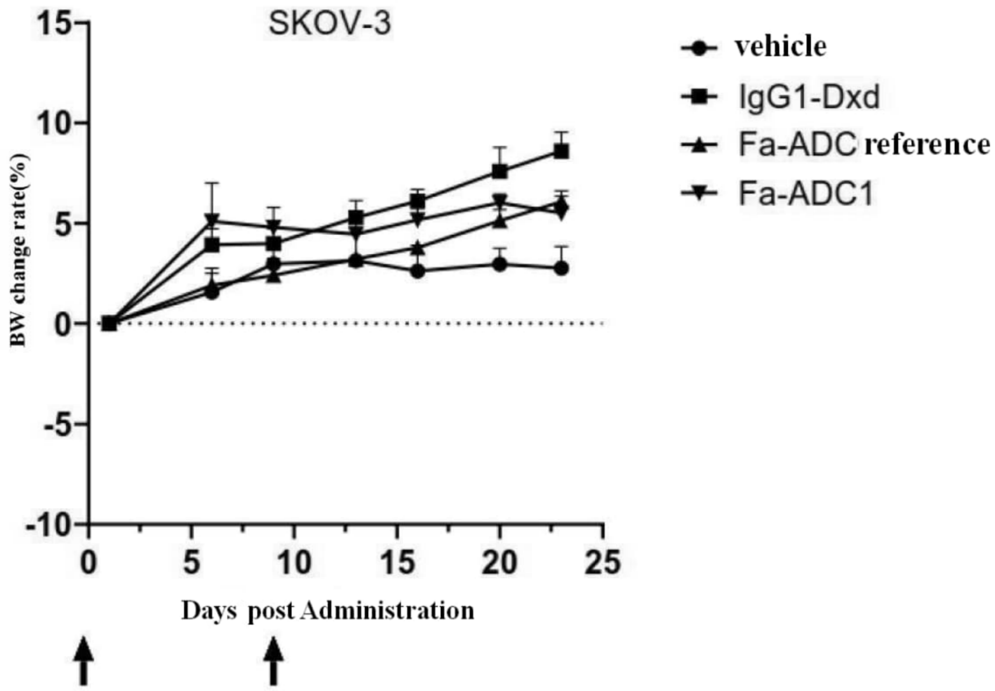
SKOV-3
15
10
5
0
-5
-10
BW change rate(%)
0
5
10
15
20
25
Days post Administration
vehicle
IgG1-Dxd
Fa-ADC reference
Fa-ADC1

Fig. 5

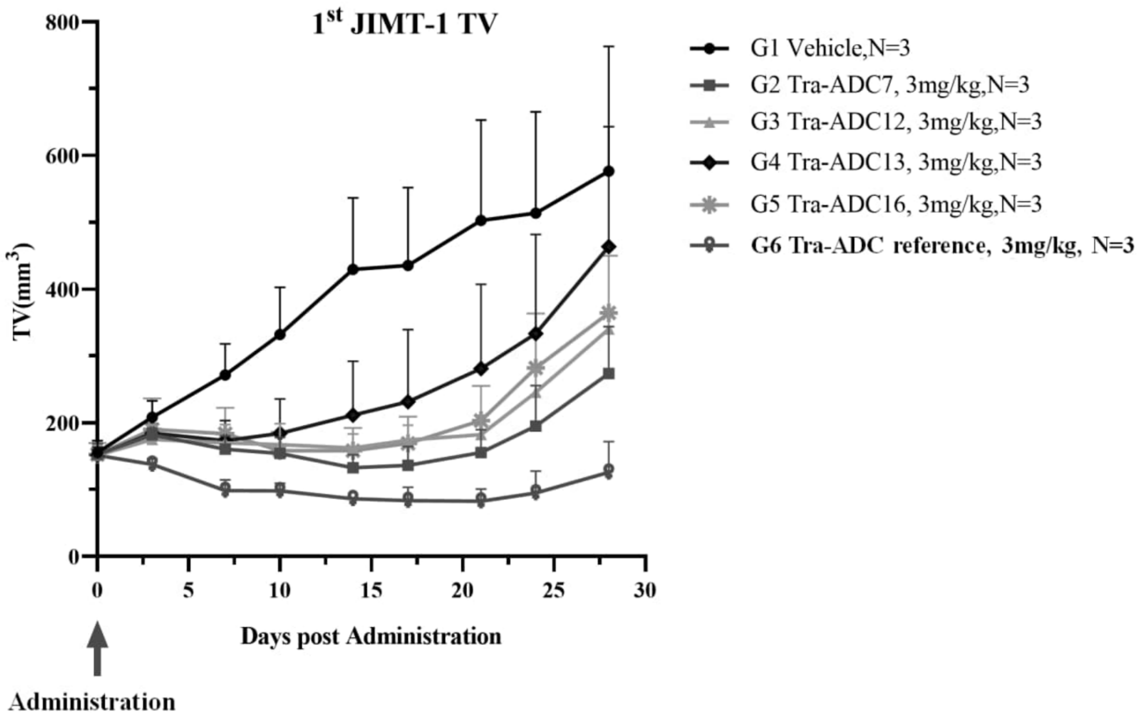

1st JIMT-1 TV
TV(mm3)
800
600
400
200
0
0
5
10
15
20
25
30
Days post Administration
Administration
G1 Vehicle,N=3
G2 Tra-ADC7, 3mg/kg,N=3
G3 Tra-ADC12, 3mg/kg,N=3
G4 Tra-ADC13, 3mg/kg,N=3
G5 Tra-ADC16, 3mg/kg,N=3
G6 Tra-ADC reference, 3mg/kg, N=3


Fig. 6a

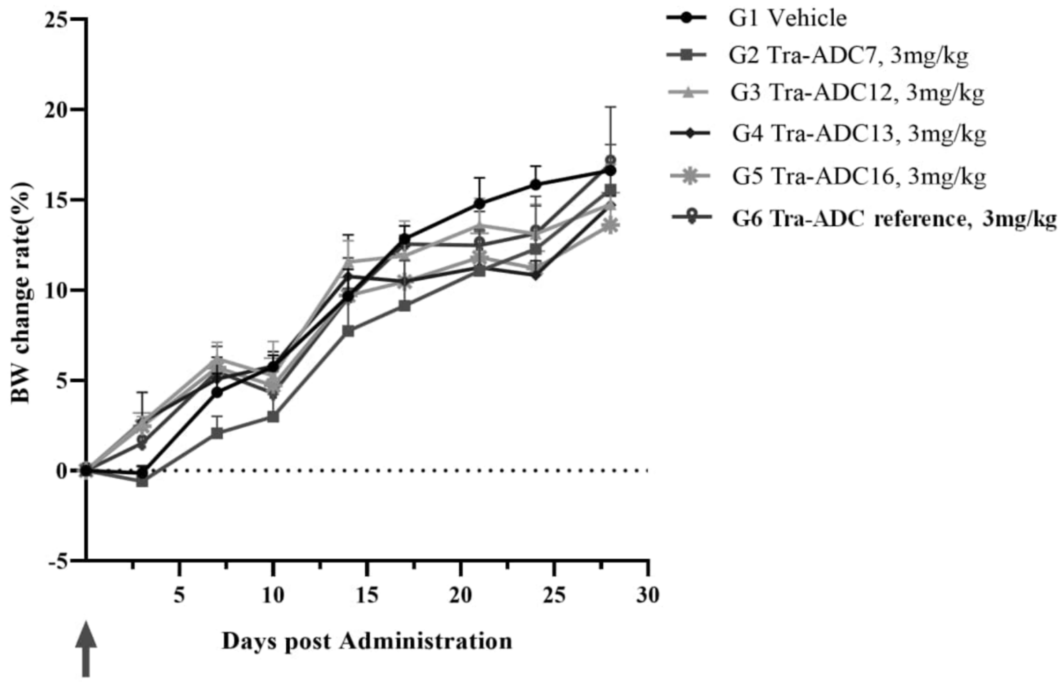

1st JIMT-1 BW change rate(%)
BW change rate(%)
25
20
15
10
5
0
-5
5
10
15
20
25
30
Days post Administration
G1 Vehicle
G2 Tra-ADC7, 3mg/kg
G3 Tra-ADC12, 3mg/kg
G4 Tra-ADC13, 3mg/kg
G5 Tra-ADC16, 3mg/kg
G6 Tra-ADC reference, 3mg/kg


Fig. 6b

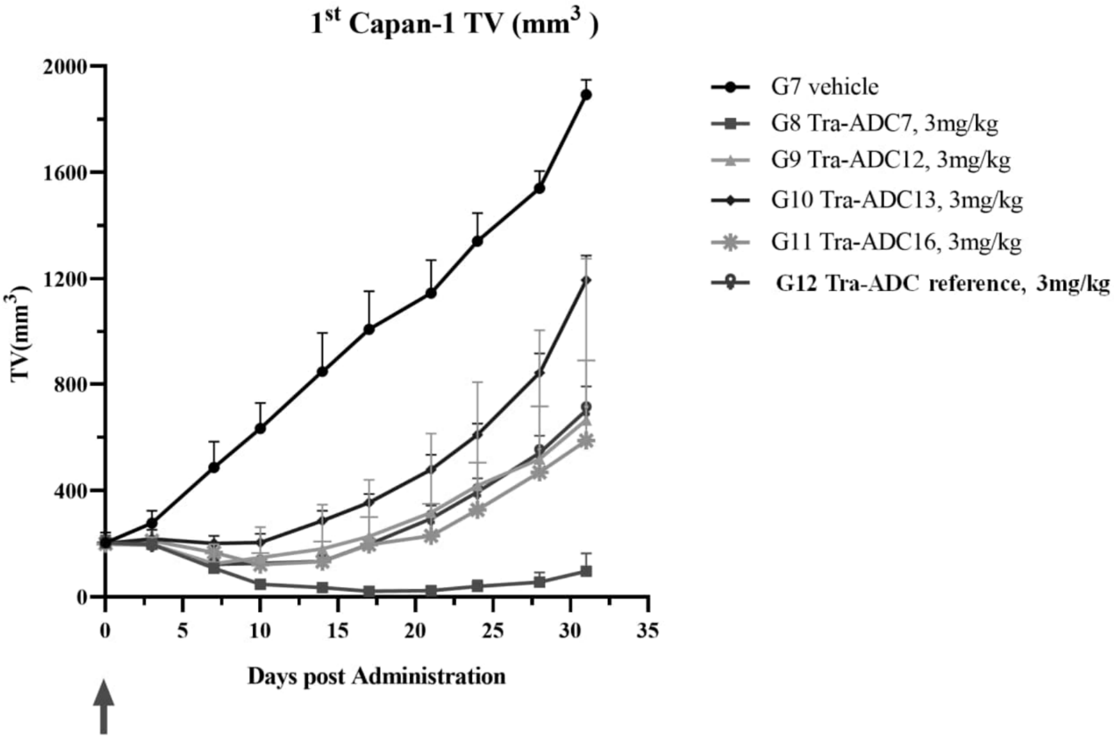
1st Capan-1 TV (mm3 )
TV(mm3)
2000
1600
1200
800
400
0
0
5
10
15
20
25
30
35
Days post Administration
G7 vehicle
G8 Tra-ADC7, 3mg/kg
G9 Tra-ADC12, 3mg/kg
G10 Tra-ADC13, 3mg/kg
G11 Tra-ADC16, 3mg/kg
G12 Tra-ADC reference, 3mg/kg

Fig. 7a

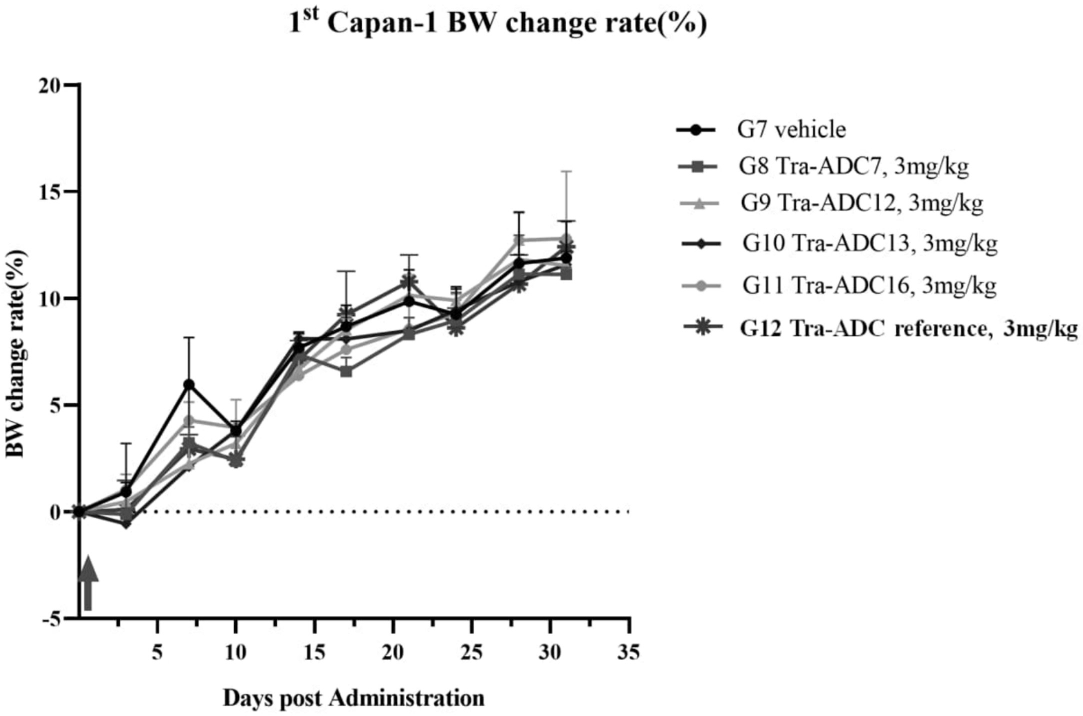
1st Capan-1 BW change rate(%)
BW change rate(%)
20
15
10
5
0
-5
5
10
15
20
25
30
35
Days post Administration
G7 vehicle
G8 Tra-ADC7, 3mg/kg
G9 Tra-ADC12, 3mg/kg
G10 Tra-ADC13, 3mg/kg
G11 Tra-ADC16, 3mg/kg
G12 Tra-ADC reference, 3mg/kg

Fig. 7b

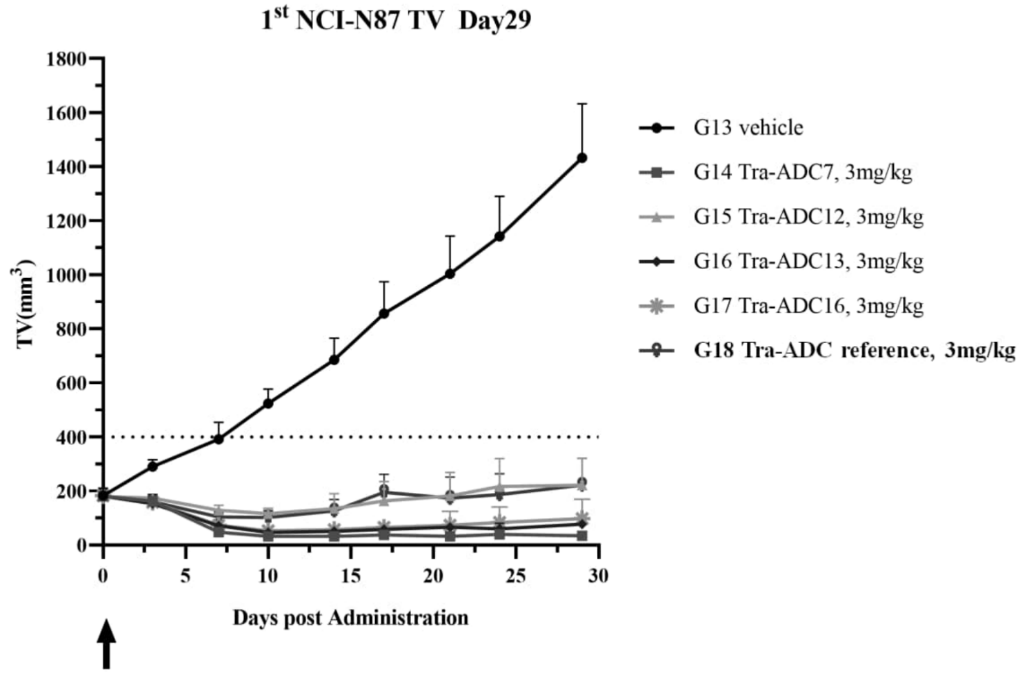
1st NCI-N87 TV Day29
TV(mm3)
1800
1600
1400
1200
1000
800
600
400
200
0
0
5
10
15
20
25
30
Days post Administration
G13 vehicle
G14 Tra-ADC7, 3mg/kg
G15 Tra-ADC12, 3mg/kg
G16 Tra-ADC13, 3mg/kg
G17 Tra-ADC16, 3mg/kg
G18 Tra-ADC reference, 3mg/kg

Fig. 8a

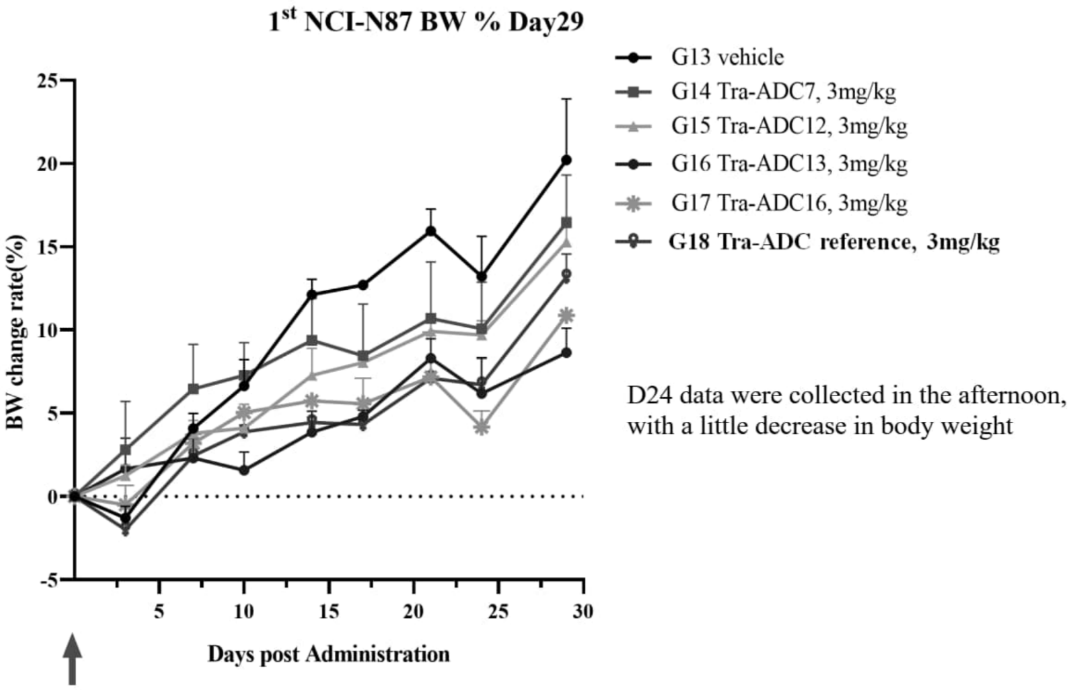
1st NCI-N87 BW % Day29
BW change rate(%)
25
20
15
10
5
0
-5
5
10
15
20
25
30
Days post Administration
G13 vehicle
G14 Tra-ADC7, 3mg/kg
G15 Tra-ADC12, 3mg/kg
G16 Tra-ADC13, 3mg/kg
G17 Tra-ADC16, 3mg/kg
G18 Tra-ADC reference, 3mg/kg
D24 data were collected in the afternoon, with a little decrease in body weight

Fig. 8b

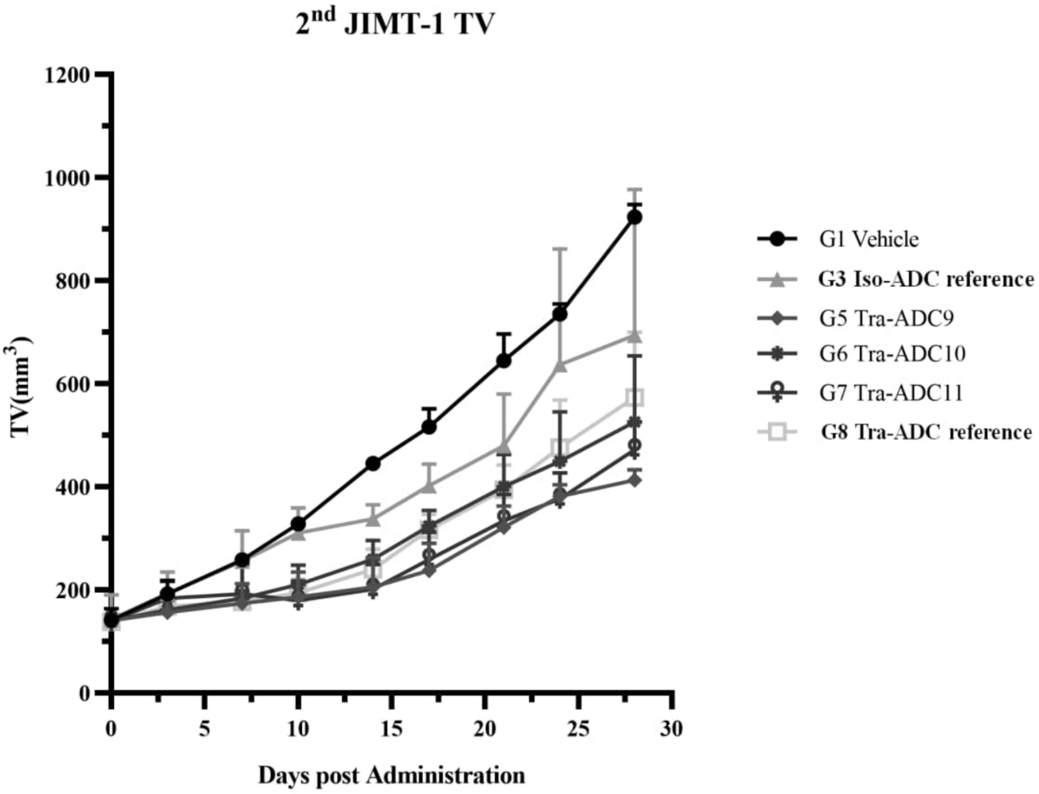

2nd JIMT-1 TV
TV(mm3)
Days post Administration
G1 Vehicle
G3 Iso-ADC reference
G5 Tra-ADC9
G6 Tra-ADC10
G7 Tra-ADC11
G8 Tra-ADC reference


Fig. 9a

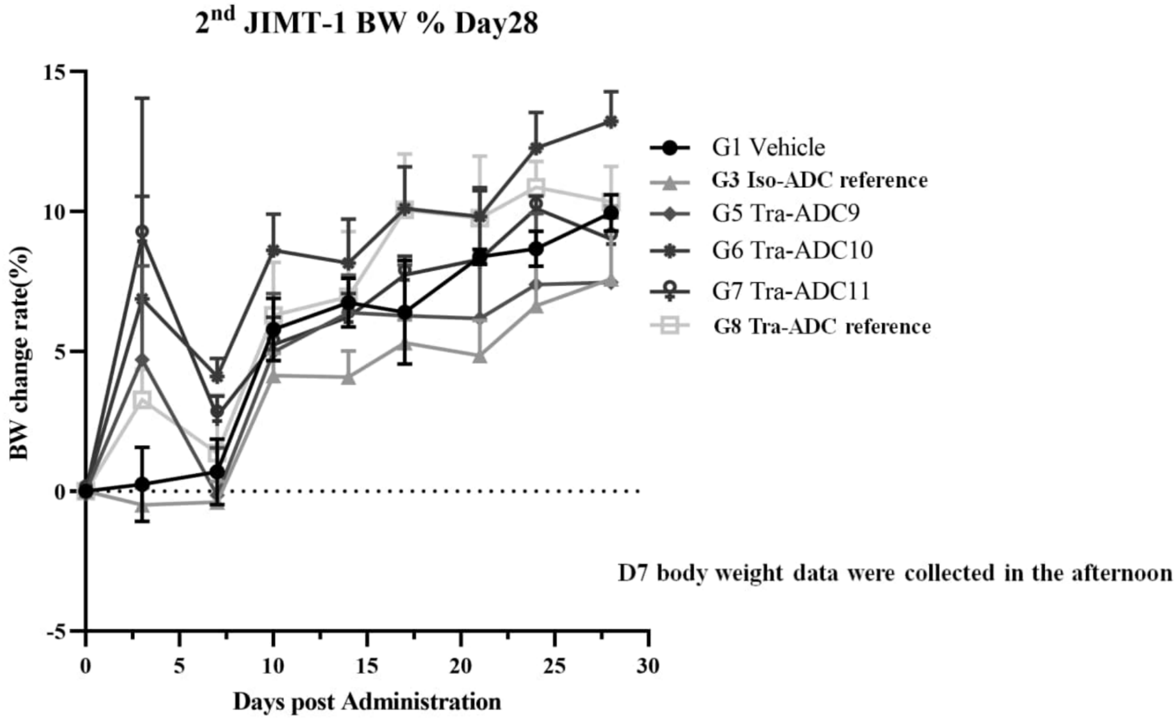

2nd JIMT-1 BW % Day28
BW change rate(%)
Days post Administration
G1 Vehicle
G3 Iso-ADC reference
G5 Tra-ADC9
G6 Tra-ADC10
G7 Tra-ADC11
G8 Tra-ADC reference
D7 body weight data were collected in the afternoon


Fig. 9b

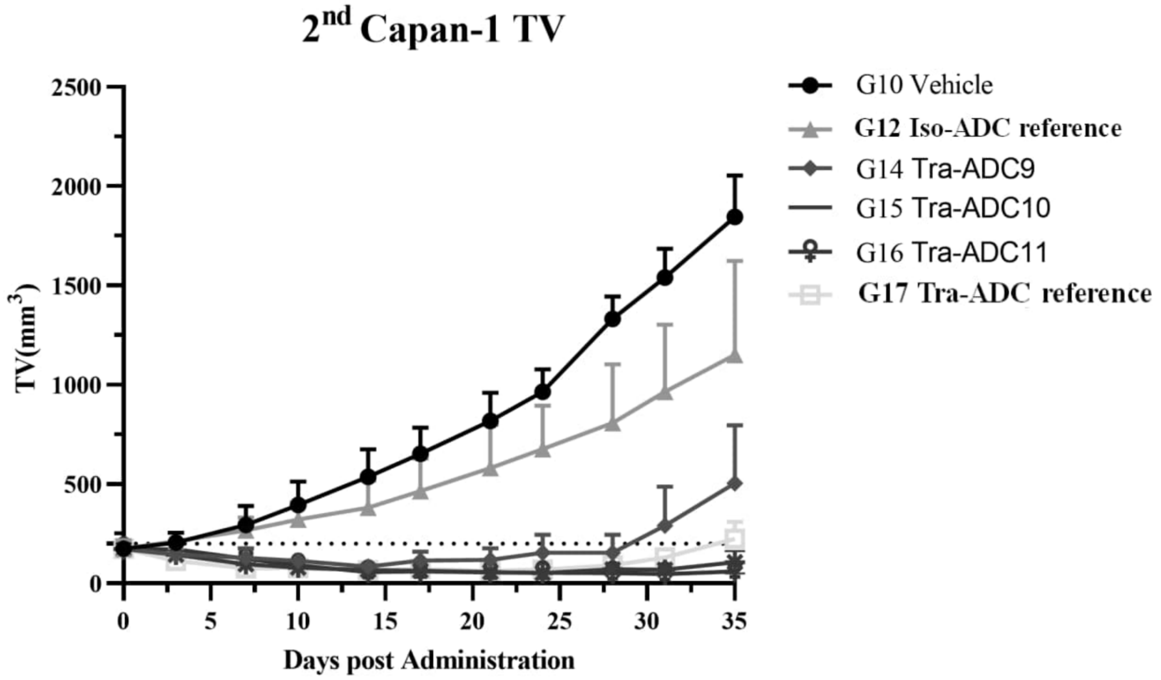


Fig. 10a

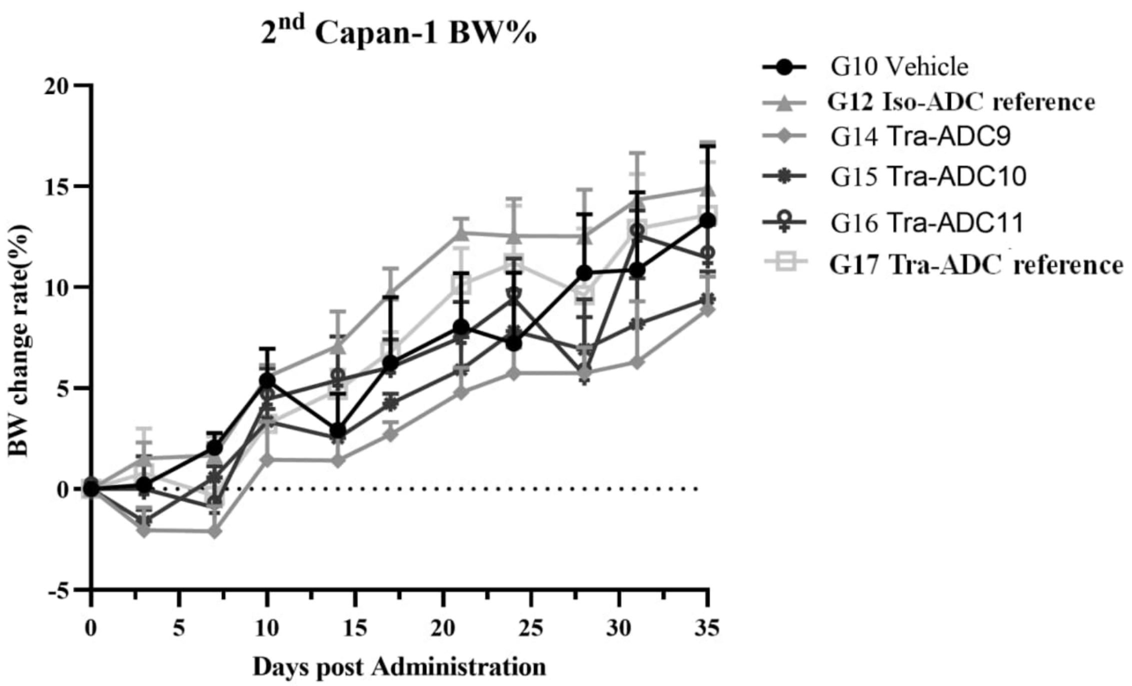


Fig. 10b

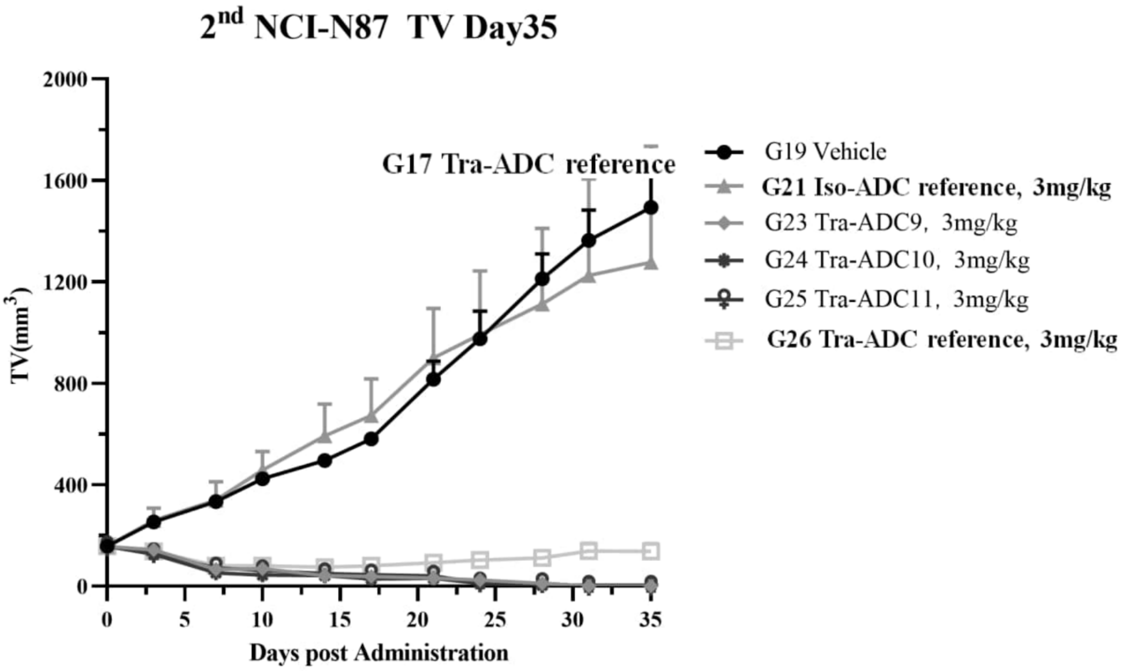


Fig. 11a

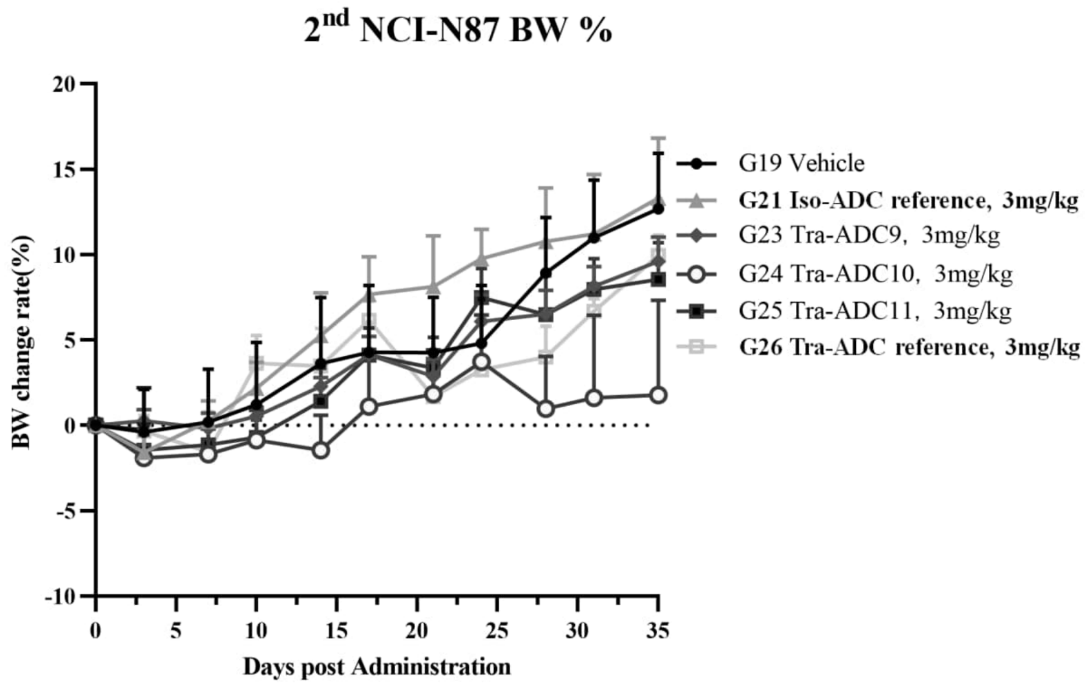


Fig. 11b

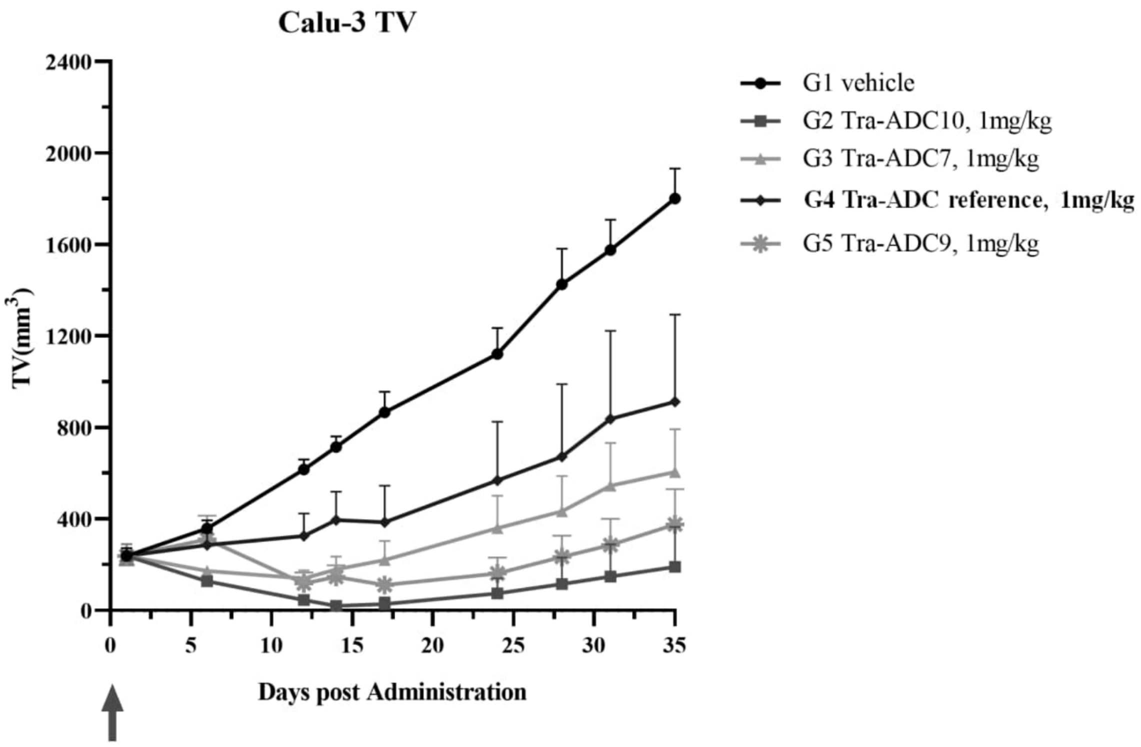


Fig. 12a

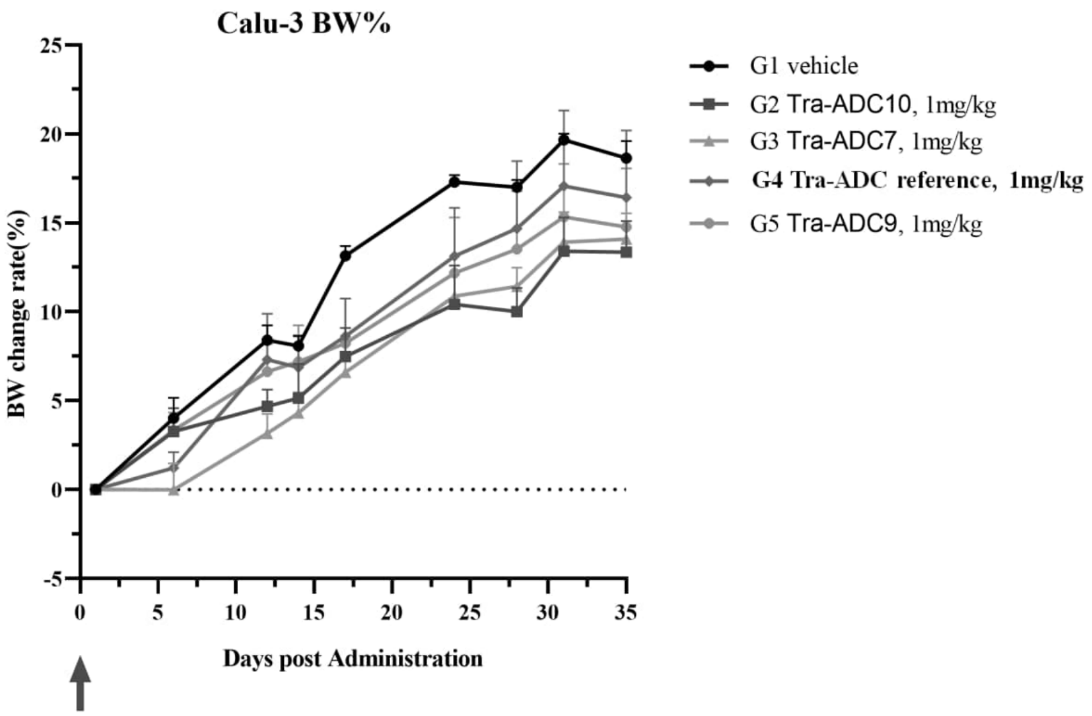


Fig. 12b

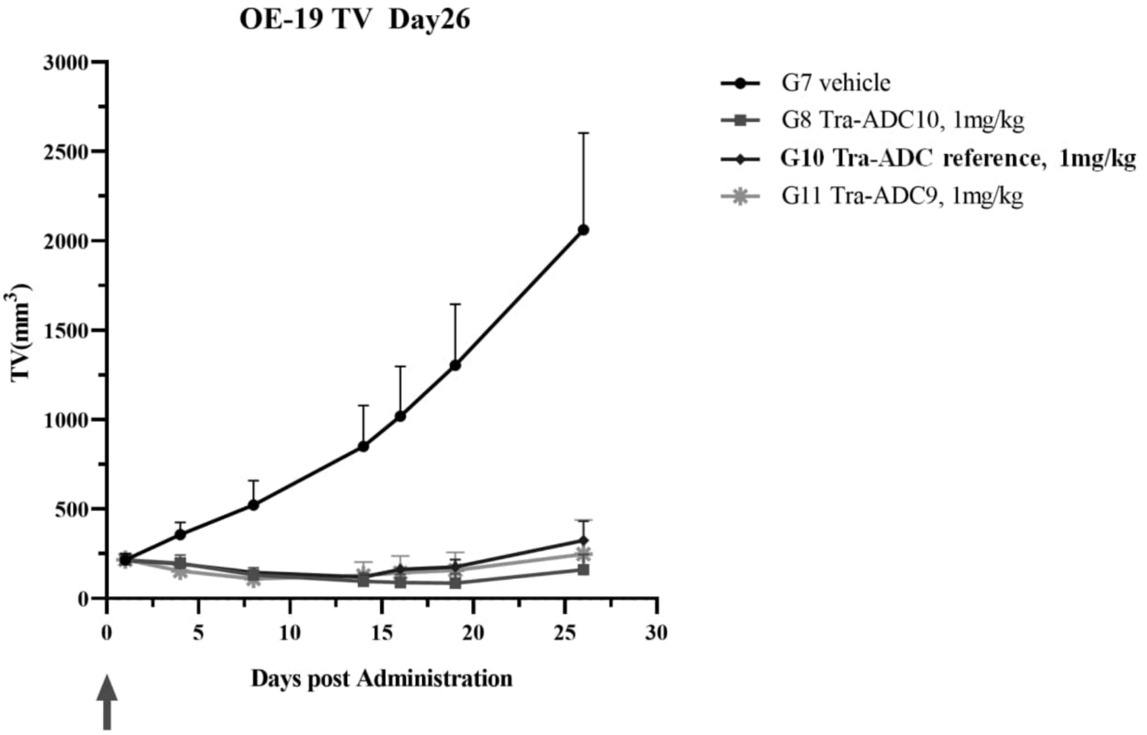


Fig. 13a

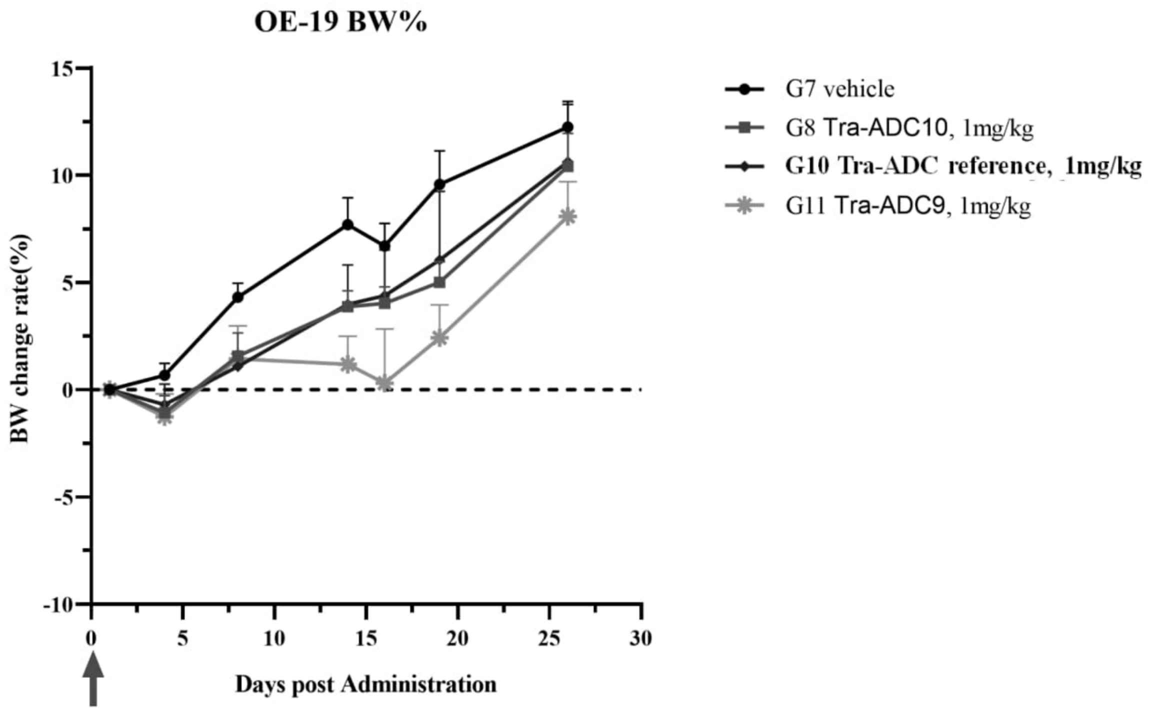


Fig. 13b

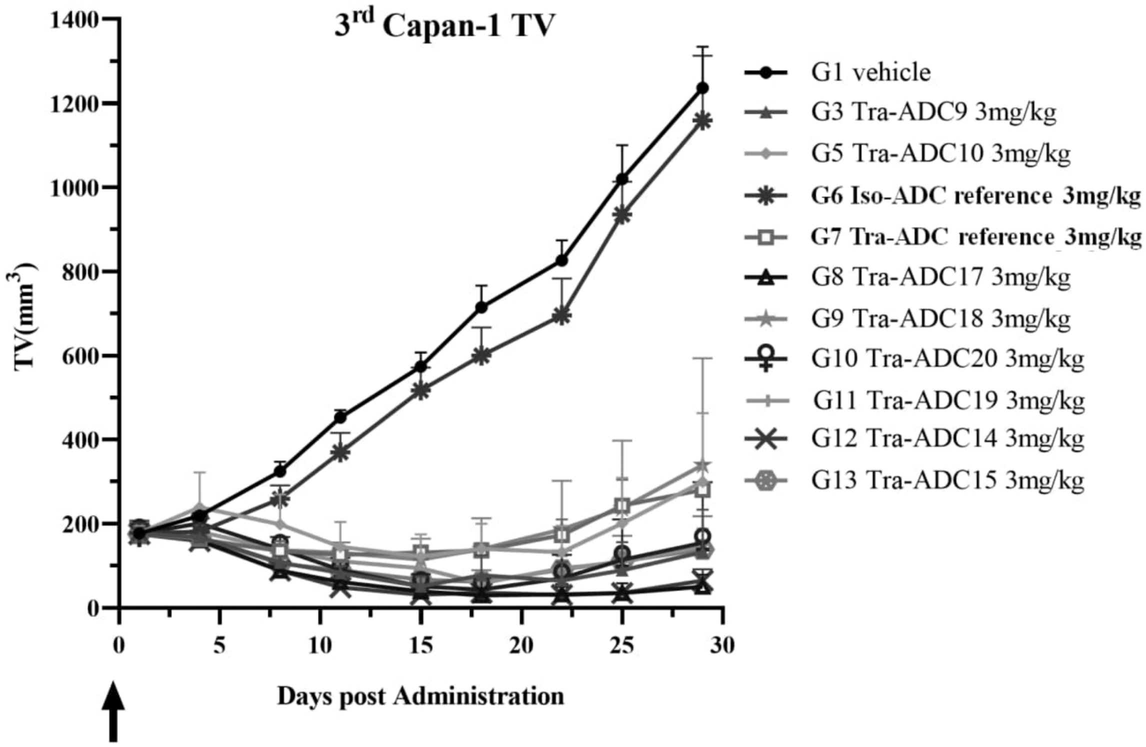


Fig. 14a

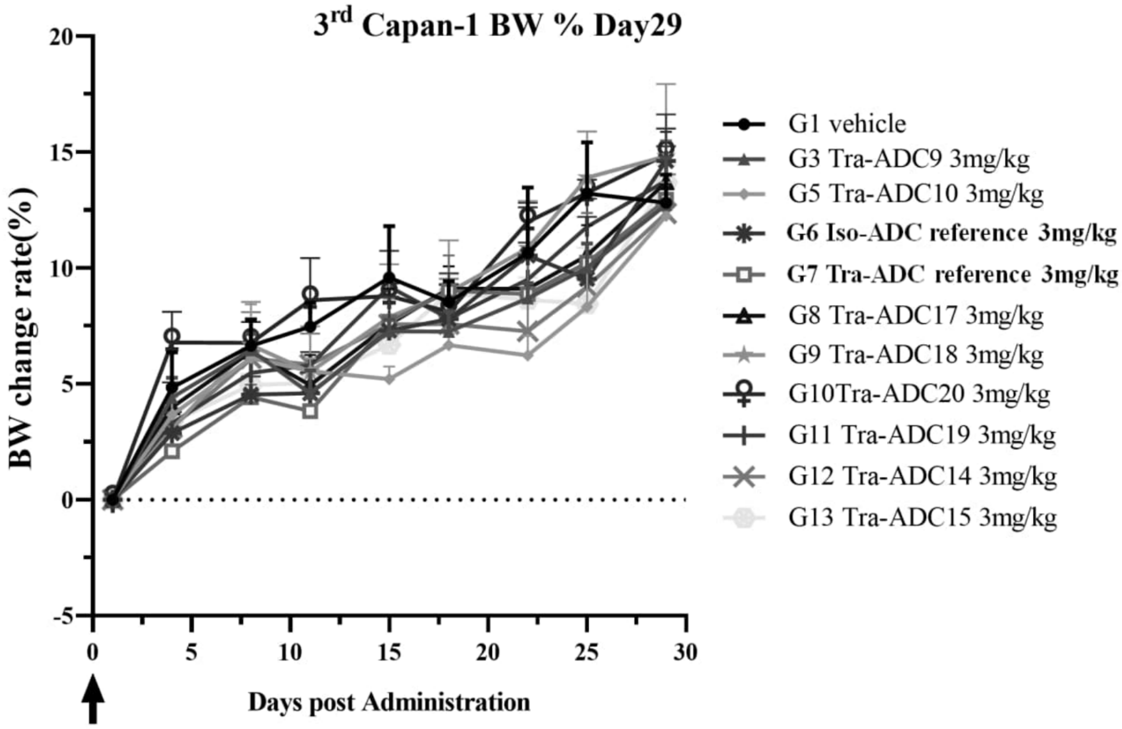


Fig. 14b

**INTERNATIONAL SEARCH REPORT**

International application No. **PCT/CN2024/098491**

**A. CLASSIFICATION OF SUBJECT MATTER**

C07D491/22(2006.01)i; A61K47/68(2017.01)i; A61K31/541(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNABS; CNTXT; ENTXT; ENTXTC; DWPI; STNext Registry; STNext Caplus; CNKI: 基于式(II)及具体化合物的结构检索, structure search based on formula (II) and specific compounds, 喜树碱, 抗体药物偶联物, camptothecin, antibody drug conjugate, ADC

**C. DOCUMENTS CONSIDERED TO BE RELEVANT**

| Category* | Citation of document, with indication, where appropriate, of the relevant passages | Relevant to claim No. |
|---|---|---|
| PX | WO 2023178452 A1 (ZYMEWORKS BC INC.) 28 September 2023 (2023-09-28) claims 1-46 | 1-38 |
| PX | WO 2024082055 A1 (ZYMEWORKS BC INC.) 25 April 2024 (2024-04-25) claims 1-48 | 1-38 |
| PX | WO 2024082051 A1 (ZYMEWORKS BC INC.) 25 April 2024 (2024-04-25) claims 1-55 | 1-38 |
| PX | WO 2023207773 A1 (SHANGHAI MICURX PHARMACEUTICAL CO., LTD.) 02 November 2023 (2023-11-02) claims 1-18 | 1-38 |
| X | WO 2022246576 A1 (ZYMEWORKS INC.) 01 December 2022 (2022-12-01) claims 20, 27, 32 and 50-58, and description, paragraphs 00797, 00807 and 00244, table 1, figures 4-9, and embodiment 1 | 1-38 |
| X | WO 2022170971 A1 (MEDILINK THERAPEUTICS (SUZHOU) CO., LTD.) 18 August 2022 (2022-08-18) claims 1-51 | 1-38 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 September 2024** | **24 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/098491**

**C. DOCUMENTS CONSIDERED TO BE RELEVANT**

| Category* | Citation of document, with indication, where appropriate, of the relevant passages | Relevant to claim No. |
|---|---|---|
| X | WO 2021212638 A1 (HANGZHOU DAC BIOTECH CO., LTD.) 28 October 2021 (2021-10-28)<br>claims 1-13 | 1-38 |
| X | CN 110430901 A (SEATTLE GENETICS INC.) 08 November 2019 (2019-11-08)<br>embodiment 3, and claim 39 | 1-38 |
| X | CN 113766954 A (IMMUNOGEN, INC.) 07 December 2021 (2021-12-07)<br>claims 1-119 | 1-38 |
| X | WO 2022140504 A1 (SHANGHAI RUOTUO BIOSCIENCES CO., LTD.) 30 June 2022 (2022-06-30)<br>claims 1-68 | 1-38 |

Form PCT/ISA/210 (second sheet) (July 2022)

**INTERNATIONAL SEARCH REPORT**

International application No. **PCT/CN2024/098491**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **36, 38**
because they relate to subject matter not required to be searched by this Authority, namely:

Claims 36 and 38 relate to a method for preventing or treating cancer in a patient, and thus do not comply with PCT Rule 39.1(iv). The search is provided on the basis of the corresponding pharmaceutical use of the compound in the claims.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/098491**

| Patent document cited in search report | Publication date (day/month/year) | Patent family member(s) | Publication date (day/month/year) |
|---|---|---|---|
| WO 2023178452 A1 | 28 September 2023 | None | |
| WO 2024082055 A1 | 25 April 2024 | None | |
| WO 2024082051 A1 | 25 April 2024 | None | |
| WO 2023207773 A1 | 02 November 2023 | None | |
| WO 2022246576 A1 | 01 December 2022 | BR 112023024433 A2 | 20 February 2024 |
| | | JP 2024519140 A | 08 May 2024 |
| | | MX 2023013738 A | 07 February 2024 |
| | | AU 2022282813 A1 | 30 November 2023 |
| | | CA 3177067 A1 | 01 December 2022 |
| | | IL 308734 A | 01 January 2024 |
| | | KR 20240031235 A | 07 March 2024 |
| | | US 2024269309 A1 | 15 August 2024 |
| | | EP 4347601 A1 | 10 April 2024 |
| WO 2022170971 A1 | 18 August 2022 | CA 3206117 A1 | 18 August 2022 |
| | | MX 2023008895 A | 09 August 2023 |
| | | AU 2022220512 A1 | 13 July 2023 |
| | | KR 20230145038 A | 17 October 2023 |
| | | JP 2024508081 A | 22 February 2024 |
| | | EP 4257154 A1 | 11 October 2023 |
| | | IL 304804 A | 01 September 2023 |
| | | US 2024108745 A1 | 04 April 2024 |
| | | TW 202241521 A | 01 November 2022 |
| WO 2021212638 A1 | 28 October 2021 | TW 202216206 A | 01 May 2022 |
| | | KR 20230034957 A | 10 March 2023 |
| | | MX 2022015598 A | 01 February 2023 |
| | | CL 2022003648 A1 | 11 August 2023 |
| | | BR 112022025583 A2 | 03 January 2023 |
| | | ZA 202212768 B | 26 July 2023 |
| | | AU 2020444233 A1 | 02 February 2023 |
| | | CA 3181660 A1 | 28 October 2021 |
| | | IL 299001 A | 01 February 2023 |
| | | US 2023241241 A1 | 03 August 2023 |
| | | JP 2023530128 A | 13 July 2023 |
| | | EP 4168009 A1 | 26 April 2023 |
| CN 110430901 A | 08 November 2019 | IL 292648 A | 01 July 2022 |
| | | JP 2020512312 A | 23 April 2020 |
| | | JP 7527787 B2 | 05 August 2024 |
| | | MX 2019010769 A | 11 December 2019 |
| | | JP 2023105175 A | 28 July 2023 |
| | | EP 3600443 A1 | 05 February 2020 |
| | | AU 2018237683 A1 | 31 October 2019 |
| | | US 2020222553 A1 | 16 July 2020 |
| | | US 11730822 B2 | 22 August 2023 |
| | | SG 11201908325 PA | 30 October 2019 |
| | | US 2024207435 A1 | 27 June 2024 |
| | | CA 3056134 A1 | 27 September 2018 |
| | | KR 20190133014 A | 29 November 2019 |
| | | KR 102648564 B1 | 19 March 2024 |
| | | WO 2018175994 A1 | 27 September 2018 |

Form PCT/ISA/210 (patent family annex) (July 2022)

**INTERNATIONAL SEARCH REPORT**
**Information on patent family members**

International application No.
**PCT/CN2024/098491**

| Patent document cited in search report | | | Publication date (day/month/year) | Patent family member(s) | | | Publication date (day/month/year) |
|---|---|---|---|---|---|---|---|
| | | | | IL | 269398 | A | 28 November 2019 |
| | | | | IL | 269398 | B1 | 01 January 2024 |
| | | | | IL | 269398 | B2 | 01 May 2024 |
| CN | 113766954 | A | 07 December 2021 | EP | 4316524 | A2 | 07 February 2024 |
| | | | | EP | 4316524 | A3 | 24 April 2024 |
| | | | | SI | 3958977 | T1 | 31 July 2024 |
| | | | | TW | 202106691 | A | 16 February 2021 |
| | | | | PL | 3958977 | T3 | 11 March 2024 |
| | | | | DK | 3958977 | T3 | 11 December 2023 |
| | | | | AU | 2020263231 | A1 | 18 November 2021 |
| | | | | JP | 2022529854 | A | 24 June 2022 |
| | | | | PT | 3958977 | T | 15 December 2023 |
| | | | | FI | 3958977 | T3 | 12 December 2023 |
| | | | | HUE | 065484 | T2 | 28 May 2024 |
| | | | | ES | 2966473 | T3 | 22 April 2024 |
| | | | | KR | 20220027828 | A | 08 March 2022 |
| | | | | HRP | 20231619 | T1 | 26 April 2024 |
| | | | | US | 2021077482 | A1 | 18 March 2021 |
| | | | | US | 11229639 | B2 | 25 January 2022 |
| | | | | CA | 3137125 | A1 | 29 October 2020 |
| | | | | SG | 11202110922 | QA | 28 October 2021 |
| | | | | LT | 3958977 | T | 27 December 2023 |
| | | | | WO | 2020219287 | A1 | 29 October 2020 |
| | | | | RS | 64961 | B1 | 31 January 2024 |
| | | | | US | 2022133711 | A1 | 05 May 2022 |
| | | | | EP | 3958977 | A1 | 02 March 2022 |
| | | | | EP | 3958977 | B1 | 13 September 2023 |
| | | | | IL | 286846 | A | 31 October 2021 |
| WO | 2022140504 | A1 | 30 June 2022 | US | 2024066138 | A1 | 29 February 2024 |
| | | | | EP | 4267140 | A1 | 01 November 2023 |
| | | | | TW | 202241897 | A | 01 November 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **JOSHUA Z DRAGO** ; **SHANU MODI** ; **SARAT CHANDARLAPATY et al.** Unlocking the potential of antibody-drug conjugates for cancer therapy.. *Nat Rev Clin Oncol.*, 08 February 2021, vol. 18 (6), 327-344
- **G L BERETTA** ; **L GATTI** ; **P PEREGO et al.** Camptothecin resistance in cancer: insights into the molecular mechanisms of a DNA-damaging drug.. *Curr Med Chem.*, 2013, vol. 20 (12), 1541-65
- **MICHAEL TAGEN** ; **YANLI ZHUANG** ; **FAN ZHANG et al.** P-glycoprotein, but not multidrug resistance protein 4, plays a role in the systemic clearance of irinotecan and SN-38 in mice.. *Drug Metab Lett.*, December 2010, vol. 4 (4), 195-201
- **CHOTHIA et al.** *Nature*, 1989, vol. 342, 877-883
- **AL-LAZIKANI et al.** Standard conformations for the canonical structures of immunoglobulins. *Journal of Molecular Biology*, 1997, vol. 273, 927-948
- **KABAT et al.** Sequences of Proteins of Immunological Interest. U.S. Department of Health and Human Services, National Institutes of Health, 1987
- **NORTH et al.** A New Clustering of Antibody CDR Loop Conformations. *Journal of Molecular Biology*, 2011, vol. 406, 228-256
- **T. W. GREENE**. Protective Groups in Organic Synthesis. John Wiley & Sons, 1991